(19) Europäisches Patentamt
European Patent Office
Office européen des brevets

(11) EP 1 962 854 B1

(12) EUROPÄISCHE PATENTSCHRIFT

(45) Veröffentlichungstag und Bekanntmachung des Hinweises auf die Patenterteilung:
**06.05.2015 Patentblatt 2015/19**

(21) Anmeldenummer: **06849272.7**

(22) Anmeldetag: **10.11.2006**

(51) Int Cl.:
***A61K 31/4985*** *(2006.01)* ***A61P 35/00*** *(2006.01)*
***C07D 471/04*** *(2006.01)*

(86) Internationale Anmeldenummer:
**PCT/EP2006/068323**

(87) Internationale Veröffentlichungsnummer:
**WO 2007/079999 (19.07.2007 Gazette 2007/29)**

(54) **PYRIDOPYRAZIN-DERIVATE UND DEREN VERWENDUNG ALS MODULATOREN DER SIGNALTRANSDUKTIONSWEGE**

PYRIDOPYRAZINE DERIVATIVES AND USE THEREOF AS MODULATORS OF THE SIGNAL TRANSDUCTION PATHS

DERIVES DE LA PYRIDOPYRAZINE ET LEUR UTILISATION COMME MODULATEUR DES VOIES DE TRANSDUCTION DE SIGNAUX

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK RS**

(30) Priorität: **11.11.2005 EP 05024692**
**11.11.2005 US 735707 P**

(43) Veröffentlichungstag der Anmeldung:
**03.09.2008 Patentblatt 2008/36**

(73) Patentinhaber: **Æterna Zentaris GmbH**
**60314 Frankfurt am Main (DE)**

(72) Erfinder:
- **SEIPELT, Irene**
  **63069 Offenbach (DE)**
- **CLAUS, Eckhard**
  **60388 Frankfurt (DE)**
- **GÜNTHER, Eckhard**
  **63477 Maintal (DE)**
- **SCHUSTER, Tilmann**
  **63762 Grossostheim (DE)**
- **CZECH, Michael**
  **60435 Frankfurt (DE)**
- **POLYMEROPOULOS, Emmanuel**
  **60325 Frankfurt (DE)**

(56) Entgegenhaltungen:
**WO-A-2004/104002 WO-A-2004/104003**
**WO-A-2005/007099 WO-A-2007/054556**
**US-A1- 2004 102 360**

- **ENGELMAN JEFFREY A ET AL: "The evolution of phosphatidylinositol 3-kinases as regulators of growth and metabolism", NATURE REVIEWS GENETICS, vol. 7, no. 8, August 2006 (2006-08), pages 606-619, ISSN: 1471-0056**
- **BRADER SHARON ET AL: "Phosphoinositide 3-kinase signalling pathways in tumor progression, invasion and angiogenesis", TUMORI, vol. 90, no. 1, January 2004 (2004-01), pages 2-8, ISSN: 0300-8916**
- **CHUGH PAULINE ET AL: "Akt inhibitors as an HIV-1 infected macrophage-specific anti-viral therapy", RETROVIROLOGY, vol. 5, January 2008 (2008-01), page Article No.: 11, ISSN: 1742-4690 (print)**
- **FARRAR CHRISTINE ET AL: "Activation of the PI3K/Akt signal transduction pathway and increased levels of insulin receptor in protein repair-deficient mice.", AGING CELL FEB 2005, vol. 4, no. 1, February 2005 (2005-02), pages 1-12, ISSN: 1474-9718**
- **LEE HAN-KYU ET AL: "The Insulin/Akt Signaling Pathway Is Targeted by Intracellular beta-Amyloid", MOLECULAR BIOLOGY OF THE CELL, vol. 20, no. 5, March 2009 (2009-03), pages 1533-1544, ISSN: 1059-1524**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **MARONE ROMINA ET AL: "Targeting phosphoinositide 3-kinase - Moving towards therapy", BIOCHIMICA ET BIOPHYSICA ACTA, vol. 1784, no. 1, January 2008 (2008-01), pages 159-185, ISSN: 1570-9639**
- **WYMANN M P ET AL: "Phosphoinositide 3-kinase gamma: A key modulator in inflammation and allergy.", BIOCHEMICAL SOCIETY TRANSACTIONS, vol. 31, no. 1, February 2003 (2003-02), pages 275-280, ISSN: 0300-5127**


Bemerkungen:

Die Akte enthält technische Angaben, die nach dem Eingang der Anmeldung eingereicht wurden und die nicht in dieser Patentschrift enthalten sind.

## Beschreibung

Technisches Gebiet

Die vorliegende Erfindung betrifft Pyridopyrazinderivate mit neuer biologischer Wirkung und deren Verwendung zur Behandlung von durch Signaltransduktionswege vermittelte und/oder modulierte physiologischen und/oder pathophysiologischen Zuständen in Säugetieren und insbesondere im Menschen.
Die Erfindung bezieht sich spezifisch auf den in den Ansprüchen definierten Gegenstand; die folgende Darstellung unterliegt dieser Definition.

Stand der Technik

Die Signaltransduktionskaskaden ras-Raf-Mek-Erk und PI3K-Akt spielen eine zentrale Rolle bei Zellwachstum, Zellproliferation, Apoptose, Adhäsion, Migration und Glukose Stoffwechsel. So ist die fundamentale Beteiligung an der Pathogenese von Erkrankungen wie Krebs, Neurodegeneration und endzündlichen Erkrankungen sowohl für den ras-Raf-Mek-Erk- als auch für den PI3K-Akt-Signalweg belegt. Daher stellen die Einzelkomponenten dieser Signalkaskaden wichtige therapeutische Angriffspunkte für die Intervention der verschiedenen Krankheitsprozesse dar (Weinstein-Oppenheimer C.R. et al 2000, Chang F. et al 2003, Katso R. et al 2001 und Lu Y. et al 2003).
Im Folgenden sind die molekularen und biochemischen Eigenschaften beider Signalwege zunächst gesondert beschrieben.
Eine Vielzahl von Wachstumsfaktoren, Zytokinen und Onkogenen transduziert ihre wachstumsfördernden Signale über die Aktivierung von G-Protein gekuppeltem ras, welches zur Aktivierung der Serin-Threonin-Kinase Raf und zur Aktivierung der Mitogen aktivierten Protein Kinase Kinase 1 und 2 (MAPKK1/2 oder Mek1/2) führt und in der Phosphorylierung und Aktivierung der MAPK 1 und 2 - auch bekannt als Extrazellulär Signal Regulierte Kinase (Erk1 und 2) - resultiert. Verglichen mit anderen Signalwegen vereint der ras-Raf-Mek-Erk-Signalweg eine große Anzahl an Proto-Onkogenen, eingeschlossen Liganden, Tyrosinkinase-Rezeptoren, G-Proteinen, Kinasen und nukleären Transkriptionsfaktoren. Tyrosin-Kinasen, wie z.B. der EGFR (Mendelsohn J. et al., 2000) vermitteln im Tumorgeschehen, bedingt durch Überexpression und Mutation, häufig konstitutiv aktive Signale an den nachgeschalteten ras-Raf-Mek-Erk-Signalweg. Ras Mutationen sind in 30% aller humanen Tumore mutiert (Khleif S.N. et al., 1999, Marshall C., 1999) wobei die höchste Inzidenz mit 90% bei Pankreaskarzinomen liegt (Friess H. et al., 1996, Sirivatanauksorn V. et al., 1998). Für c-Raf wurde bei verschiedenen Tumoren eine deregulierte Expression und/oder Aktivierung beschrieben (Hoshino R. et al., 1999, McPhillips F. et al., 2001). B-Raf-Punktmutanten wurden in 66% aller humanen malignen Melanome, 14% aller ovarialen und 12% aller Kolonkarzinomes detektiert (Davies H. et al., 2002). Daher ist es nicht überraschend, dass Erk1/2 an vielen zellulären Prozessen wie Zellwachstum, Zellproliferation und Zelldifferenzierung primär beteiligt ist (Lewis T.S. et al., 1998, Chang F. et al., 2003).
Darüberhinaus haben die Mitglieder der Raf-Kinasen auch Mek-Erk unabhängige, anti-apoptotische Funktionen, deren molekulare Schritte noch nicht vollständig beschrieben sind. Als mögliche Interaktionspartner für die Mek-Erk-unabhängige Raf-Aktivität wurden Ask1, Bcl-2, Akt und Bag1 beschrieben (Chen J et al., 2001, Troppmaier J. et al., 2003, Rapp U.R. et al., 2004, Gotz R. et al., 2005). Man geht heute davon aus, daß sowohl Mek-Erk-abhängige als auch Mek-Erk-unabhängige Signaltransduktions-Mechanismen die Aktivierung der oberhalb gelegenen Stimuli ras und Raf steuern.
Die Isoenzyme der Phosphatidylinositol 3-Kinasen (PI3Ks) fungieren vorwiegend als Lipidkinasen und katalysieren die D3-Phosphorylierung der Second-Messenger Lipide Ptdlns (Phosphatidylinositol) zu Ptdlns(3)P, PtdIns(3,4)$P_2$, PtdIns(3,4,5)$P_3$ Phosphatidylinositolphosphaten. Die PI3Ks der Klasse I setzen sich strukturell aus der katalytischen (p110alpha, beta, gamma, delta) und der regulatorischen (p85alpha, beta oder p101gamma) Untereinheit zusammen. Weiterhin gehören die Klasse II-(PI3K-C2alpha, PI3K-C2beta) und Klasse III-(Vps34p)Enzyme zur Familie der PI3-Kinasen (Wymann M.P. et al., 1998, VanHaesebroeck B. et al., 2001). Der durch die PI3Ks ausgelöste PIP-Anstieg aktiviert einerseits den proliferativen ras-Raf-Mek-Erk Signalweg über die Kupplung von ras (Rodriguez-Viciana P. et al., 1994) und stimuliert andererseits den anti-apoptotischen Signalweg durch Rekrutierung von Akt an die Zellmembran und folglicher Überaktivierung dieser Kinase (Alessi D.R. et al., 1996, Chang H.W. et al., 1997, Moore S.M. et al., 1998). Somit erfüllt die Aktivierung der PI3Ks mindestens 2 entscheidende Mechanismen der Tumorentstehung, nämlich die Aktivierung von Zellwachstum und Zelldifferenzierung und die Inhibition der Apoptose. Darüberhinaus verfügen die PI3K auch über Protein-phosphorylierende Eigenschaften (Dhand et al., 1994, Bondeva T. et al., 1998, Bondev A. et al., 1999, VanHaesebroeck B. et al., 1999), die z.B. eine die PI3Ks intrinsisch regulierende Serin-Autophosphorylierung auslösen kann. Außerdem ist bekannt, dass PI3Ks auch Kinase-unabhängige, regulierende Effektor-Eigenschaften haben z.B. bei der Kontrolle der Herzkontraktion (Crackower M.A. et al., 2002, Patrucco et al., 2004). Ferner ist belegt, dass PI3Kdelta und PI3Kgamma auf hematopoietischen Zellen spezifisch exprimiert werden und damit potenzielle Angriffpunkte für Isoenzym-spezifische PI3Kdelta- und PI3Kgamma-Inhibitoren bei der Behandlung von inflammatorischen Erkrankungen wie Rheuma, Asthma und Allergien und bei der Behandlung von B- und T-Zelllymphomen (Okkenhaug K. et al., 2003, Ali K. et al., 2004, Sujobert P. et al., 2005) darstellen. Die PI3Kalpha, welche kürzlich als Proto-Onkogen identifiziert wurde (Shayesteh L. et al., 1999, Ma Y.Y. et al., 2000, Samuels Y. et al., 2004, Campbell I.G. et al., 2004, Levine D.A., 2005), gilt als wichtiges Target bei der Therapie von Tumorerkrankungen. Die Bedeutung der PI3K-Spezies als Target für die Wirkstoffentwicklung ist daher äußerst vielfältig (Chang F. & Lee J.T. et al, 2003).

Von ebenso großem Interesse sind die PI3K verwandten Kinasen (PIKK), welche die Serin/Threonin-Kinasen mTOR, ATM, ATR, h-SMG-1 und DNA-PK (Chiang G.G. et al 2004) einschließen. Ihre katalytischen Domänen haben eine hohe Sequenz-Homologie zur katalytische Domäne der PI3Ks.

Überdies trägt der Verlust des Tumorsuppressor-Proteins PTEN (Li J. et al., 1997, Steck P.A. et al., 1997) - dessen Funktion die Reversion der durch die PI3K initiierte Phosphorylierung ist - zu einer Überaktivierung von Akt und deren abwärts gelegenen Kaskade-Komponenten bei und unterstreicht damit die kausale Bedeutung von PI3K als Zielmolekül für die Tumortherapie.

Diverse Inhibitoren von Einzelkomponenten der ras-Raf-Mek-Erk und PI3K-Akt-Signalwege sind bereits publiziert und patentiert worden.

Der gegenwärtige Entwicklungsstand auf dem Gebiet der Kinase-Inhibitoren, besonders des ras-Raf-Mek-Erk- und des PI3K-Akt-Weges wird in den Übersichten von

J.S. Sebolt-Leopold et al., 2004, und R. Wetzker et al., 2004 dargestellt. In den benannten Publikation finden sich umfassende Auflistungen der offengelegten Patentschriften, die die Synthese und Anwendung niedermolekularer ras-Raf-Mek-Erk- und PI3K-Inhibitoren beschreiben.

Der bereits in klinischer Erprobung befindliche Kinaseinhibitor Bay 43-9006 (WO 99/32111, WO 03/068223) zeigt ein relativ unspezifisches Inhibitionsmuster von Serin/Threonin- und von Tyrosin-Kinasen wie Raf, VEGFR2/3, Flt-3, PDGFR, c-Kit und weiteren Kinasen. Diesem Inhibitor wird bei durch Angiogenese-induzierten fortgeschrittenen Tumorerkrankungen (z.B. beim Nierenzellkarzinom), aber auch bei Melanomen mit hoher B-Raf-Mutationsrate eine große Bedeutung zugemessen. Die klinische Wirkung von Bay 43-9006 wird zurzeit außerdem an Patienten mit refraktären soliden Tumoren in Kombination z.B. mit Docetaxel ermittelt. Bislang wurden milde Nebenwirkungen und vielversprechende anti-tumorale Effekte beschrieben. Eine Inhibition der Kinasen im PI3K-Akt-Signalweg ist für Bay 43-9006 nicht beschrieben.

Der Mek1/2-Inhibitor PD0325901 (WO 02/06213) befindet sich derzeit in klinischer Erprobung der Phase I. Die Vorläufersubstanz CI-1040 (WO 00/35435, WO 00/37141) fiel durch ihre hohe Mek-Spezifität und Target-Affinität auf. Jedoch erwies sich diese Verbindung in Phase I/II-Studien als metabolisch instabil. Klinische Daten der aktuellen Nachfolgersubstanz PD0325901 stehen noch aus. Jedoch ist für diesen Mek-Inhibitor weder eine Wechselwirkung mit Erk1 oder Erk2 noch eine den PI3K-Akt-Signalweg inhibierende Funktion oder deren gleichzeitige Modulation offenbart.

Die Patentschriften WO 04/104002 und WO 04/104003 beschrieben Pyrido[2,3-b]pyrazine, die in 6- oder 7-Position mit Harnstoff-, Thioharnstoff-, Amidin- oder Guanidingruppen substituiert sein können. Diese Verbindungen besitzen Eigenschaften als Inhibitoren bzw. Modulatoren von Kinasen, insbesondere von Tyrosin- und Serin/Threoninkinasen, und es ist eine Verwendung als Arzneimittel angegeben. Demgegenüber ist eine Verwendung dieser Verbindungen als Modulatoren von Lipidkinasen, alleine oder in Kombination mit Tyrosin- und Serin/Threoninkinasen, nicht beschrieben.

Weiterhin werden in der Patentschrift WO 99/17759 Pyrido[2,3-b]pyrazine beschrieben, die in 6-Position unter anderem Alkyl-, Aryl- und Heteroarylsubstituierte Carbamate tragen. Diese Verbindungen sollen dazu verwendet werden, die Funktion von Serin-Threonin-Proteinkinasen zu modulieren.

In der Patentschrift WO 05/007099 werden unter anderem auch Harnstoffsubstituierte Pyrido[2,3-b]pyrazine als Inhibitoren der Serin/Threoninkinase PKB beschrieben. Für diese Verbindungen wird eine Verwendung bei der Behandlung von Krebserkrankungen angegeben. Allerdings werden keine konkreten Beispiele von Harnstoff-substituierten Pyridopyrazinen mit diesen biologischen Eigenschaften aufgeführt.

Weitere Beispiele für in 6- und 7-Position Harnstoff-substitutierte Pyrido[2,3-b]pyrazine werden in der Patentschrift WO 05/056547 angegeben. Die Verbindungen in dieser Patentschrift werden als Inhibitoren von Protein-Kinasen, insbesondere von GSK-3, Syk und JAK-3, beschrieben. Für diese Verbindungen wird unter anderem eine Verwendung bei der Behandlung von proliferativen Erkrankungen angegeben. Eine Verwendung dieser Verbindungen als Modulatoren von Lipidkinasen, alleine oder in Kombination mit Serin/Threoninkinasen, ist nicht beschrieben.

In der Patentanmeldung WO 04/005472 werden unter anderem in 6-Position Carbamat-substituierte Pyrido[2,3-b]pyrazine beschrieben, die als antibakterielle Substanzen das Wachstum von Bakterien hemmen. Eine Antitumorwirkung ist nicht beschrieben.

Bestimmte Diphenylchinoxaline und -pyrido[2,3-b]pyrazine mit speziellen Alkylpyrrolidin-, Alkylpiperidin- oder Alkylsulfonamid-Resten an einem Phenylring, die zusätzlich auch Harnstoff- oder Carbamat-Substitutionen in 6- oder 7-Position tragen können, werden in den Patentschriften WO 03/084473, WO 03/086394 und WO 03/086403 als Inhibitoren der Aktivität der Serin/Threonin-Kinase Akt beschrieben. Für diese Verbindungen wird eine Verwendung bei der Behandlung von Krebserkrankungen angegeben. Für die dort beschriebenen Pyrido[2,3-b]pyrazin-Beispiel-Verbindungen ist kein definierter Hinweis auf eine biologische Wirkung angegeben.

Weiterhin werden in der Patentschrift WO 03/024448 Amid- und Acrylamidsubstituierte Pyrido[2,3-b]pyrazine beschrieben, die als zusätzliche Substituenten auch Carbamate enthalten und als Histon Deacetylase-Inhibitoren zur Behandlung von Zellproliferationserkrankungen verwendet werden können.

In einer weiteren Publikation (Temple C. et al, 1990) wird an einem Beispiel die Synthese eines 6-Ethylcarbamat-substituierten Pyrido[2,3-b]pyrazin-Derivates beschrieben. Eine Antitumorwirkung ist weder offenbart noch nahegelegt.

Die Synthese von weiteren Derivaten des 6-Ethylcarbamat-substituierten Pyrido[2,3-b]pyrazins wird in einer Veröffentlichung von R. D. Elliott beschrieben (J. Org. Chem. 1968). Eine biologische Wirkung dieser Verbindungen ist weder beschrieben noch nahegelegt.

In der Publikation von C. Temple (1968) wird die Synthese und Untersuchung von 6-Ethylcarbamat-substituierten Pyrido[2,3-b]pyrazinen als potentielle Antimalaria-Wirkstoffe beschrieben. Eine Antitumorwirkung ist weder offenbart noch nahegelegt.

Die bislang publizierten PI3K-Inhibitoren befinden sich in präklinischer Erprobung. ICOS offenbarte einen PI3K-Inhibitor IC87114 mit hoher PI3Kdelta Isoenzymspezifität (WO01/81346). Für PI103 (WO 04/017950) beschreiben Yamanouchi/Piramed eine Selektivität versus der PI3Kalpha Isoform. In der frühen Entwicklung von PI3K-Inhibitoren existiert darüberhinaus ein stark beachtetes Forschungs-Umfeld (s. Übersicht R. Wetzker et al., 2004).

Inhibitoren des SAPK-Signalweges, entweder von Jnk oder von p38, sind in der Literatur beschrieben (Gum R.J., 1998, Bennett B.L. et al 2001, Davies S.P. et al 2000). Jedoch ist für diese SAPK-Inhibitoren keine die PI3Ks inhibierende Funktion und auch keine spezifische Inhibition der Erk1 oder Erk2 oder auch gleichzeitige Inhibition von SAPKs, Erk1, Erk2, oder PI3Ks offenbart.

Darstellung der Erfindung

Die vorliegende Erfindung hat die Aufgabe, neue Verbindungen bereitzustellen, die zur Behandlung oder Prophylaxe von physiologischen und/oder pathophysiologischen Zuständen in Säugetieren, insbesondere dem Menschen, verwendet werden können, die durch Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" vermittelt werden. Eine weitere Aufgabe der Erfindung ist die Bereitstellung neuer Verbindungen für o.g. Verwendungen unter Modulation der genannten Signaltransduktionswege. Die vorliegende Erfindung hat weiterhin die Aufgabe, neue Verbindungen bereitzustellen, die zur Behandlung oder Prophylaxe von physiologischen und/oder pathophysiologischen Zuständen in Säugetieren, insbesondere dem Menschen, verwendet werden können, die durch Enzyme ausgewählt aus der Gruppe bestehend aus: "ATM, ATR, mTOR, DNA-PK, hSMG-1" vermittelt werden. Eine weitere Aufgabe der Erfindung ist die Bereitstellung neuer Verbindungen für o.g. Verwendungen unter Modulation der genannten Enzyme.

Die erfinderische Aufgabe wurde in einem Aspekt überraschender Weise dadurch gelöst, dass Verbindung gemäß der allgemeinen Formel (I),

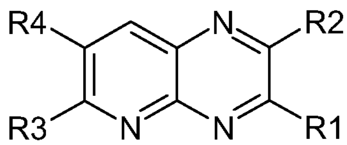
(I)

worin die Substituenten R1, R2, R3, R4 folgende Bedeutung haben:

R1 und R2 können unabhängig voneinander:

(i) Wasserstoff

(ii) Hydroxyl

(iii) Halogen

(iv) Alkyl, wobei der Alkylrest gesättigt ist und aus 1 bis 8 C-Atomen bestehen kann,

(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl,

NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, $O-(CH_2)_n-O$, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, $C(O)-NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, S03H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, n den Wert 1, 2 oder 3 annehmen kann, und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum substituiert sein können,

(vi) unsubstituiertes oder substituiertes Heteroaryl, wobei der Heteroarylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $NH-Alkyl-NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, $C(O)-NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2NH_2$, $SO_2NH$-Alkyl, $SO_2NH$-Aryl, $SO_2NH$-Heteroaryl, $SO_2NH$-Alkyl-Aryl, S03H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, und die Alkyl-, Cycloalkyl-, Heterocyclyl-, Aryl- und Heteroarylsubstituenten ihrerseits wiederum substituiert sein können,

(vii) OR5, wobei R5 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl-, Heteroaryl-, Alkyl-Cycloalkyl, Alkyl-Heterocyclyl, Alkyl-Aryl oder Alkyl-Heteroarylsubstituenten ihrerseits wiederum substituiert sein können,

(viii) SR6, wobei R6 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl-, Al-kyl-Heterocyclyl-, Alkyl-Aryl- oder Alkyl-Heteroarylsubstituenten ihrerseits wiederum substituiert sein können,

(ix) NR7R8, wobei R7 und R8 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können, oder R7 und R8 zusammen Cycloalkyl oder Heterocyclyl bedeuten, wobei Cycloalkyl und Heterocyclyl ihrerseits wiederum substituiert sein können, bedeuten.

R3 und R4 können unabhängig voneinander Wasserstoff oder NR9R10 bedeuten, unter der Voraussetzung, dass, wenn R3 = NR9R10 ist, R4 = H ist, und wenn R4 = NR9R10 ist, R3 = H ist,
wobei R9 Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können,
und R10:

- C(Y)NR11R12 bedeuten kann, wobei Y = O, S und R11 und R12 unabhängig voneinander

(i) Wasserstoff,

(ii) unsubstituiertes oder substituiertes Alkyl, wobei der Alkylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Cycloalkyl, Heterocyclyl, Aryl oder Heteroaryl, ein- oder mehrfach, gleich oder verschieden substituiert sein kann,

(iii) unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, Alkyl, oder Aryl ein- oder mehrfach, gleich oder verschieden substituiert sein kann,

(iv) unsubstituiertes oder substituiertes Heterocyclyl, wobei der Heterocyclylrest mit OH, O-Alkyl, O-Aryl, $NH_2$, NH-Alkyl, NH-Aryl, Alkyl, Alkyl-Aryl oder Aryl ein- oder mehrfach, gleich oder verschieden substituiert sein kann,

(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, $O-(CH_2)_n-O$, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, S03H,

$SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(vi) unsubstituiertes oder substituiertes Heteroaryl, wobei der Heteroarylrest F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2NH_2$, $SO_2NH$-Alkyl, $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Alkyl, Cycloalkyl, Heterocyclyl, Aryl oder Heteroaryl, ein-oder mehrfach, gleich oder verschieden substituiert sein kann,

(vii) -C(O)-R17, wobei R17 Alkyl, Aryl oder Heteroaryl sein kann, und die Alkyl und Arylsubstituenten ihrerseits wiederum substituiert sein können,

(viii) oder R11 und R12 zusammen Cycloalkyl oder Heterocyclyl bedeuten können,

- C(Y)NR13R14 bedeuten kann, wobei Y = NH und R13 und R14 unabhängig voneinander

(i) Wasserstoff,

(ii) unsubstituiertes oder substituiertes Alkyl, wobei der Alkylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_3H$, Alkyl, Cycloalkyl, Heterocyclyl, Aryl oder Heteroaryl, ein- oder mehrfach, gleich oder verschieden substituiert sein kann,

(iii) unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, $CO_2H$, $CO_2$-Alkyl, $CO_2$-Cycloalkyl, $CO_2$-Heterocyclyl, $CO_2$-Aryl, $CO_2$-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, $C(O)N(Alkyl)_2$, Alkyl, oder Aryl, ein- oder mehrfach, gleich oder verschieden substituiert sein kann,

(iv) unsubstituiertes oder substituiertes Heterocyclyl, wobei der Heterocyclylrest mit OH, O-Alkyl, O-Aryl, $NH_2$, NH-Alkyl, NH-Aryl, Alkyl, oder Aryl ein- oder mehrfach, gleich oder verschieden substituiert sein kann,

(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, S03H, $SO_2O$-Alkyl, $SO_2O$-Aryl, $SO_2O$-Heteroaryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(vi) unsubstituiertes oder substituiertes Heteroaryl, wobei der Heteroarylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Aryl, OH, $OCF_3$, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2$-Alkyl, $SO_2$-Aryl, $SO_2NH_2$, $SO_2NH$-Alkyl, $SO_2NH$-Aryl, $SO_2NH$-Heteroaryl, S03H, $SO_2O$-Alkyl, $SO_2O$-Aryl, $SO_2O$-Heteroaryl, Alkyl, Cycloalkyl, Heterocyclyl, Aryl oder Heteroaryl, ein- oder mehrfach, gleich oder verschieden substituiert sein kann,

(vii) oder R13 und R14 zusammen Cycloalkyl oder Heterocyclyl bedeuten können,

- C(NR15)R16 bedeuten kann, wobei R15 = H und R16

(i) unsubstituiertes oder substituiertes Alkyl, wobei der Alkylrest mit F, Cl, Br, I, $CF_3$, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, O-$SO_2$-Heteroaryl, C(O)-Alkyl, C(O)-Aryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_3H$, Alkyl, Cycloalkyl, Heterocyclyl, Aryl oder Heteroaryl, ein- oder mehrfach, gleich oder verschieden substituiert sein kann,

(ii) unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, $CO_2H$, $CO_2$-Alkyl, $CO_2$-Cycloalkyl, $CO_2$-Heterocyclyl, $CO_2$-Aryl, $CO_2$-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, $C(O)N(Alkyl)_2$, Alkyl, oder Aryl, ein- oder mehrfach, gleich oder verschieden substituiert

sein kann,

(iii) unsubstituiertes oder substituiertes Heterocyclyl, wobei der Heterocyclylrest mit OH, O-Alkyl, O-Aryl, $NH_2$, NH-Alkyl, NH-Aryl, Alkyl oder Aryl ein- oder mehrfach, gleich oder verschieden substituiert sein kann,

(iv) unsubstituiertes oder substituiertes Aryl, wobei der Arylrest mit F, Cl, Br, I, $CF_3$, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, SO3H, $SO_2O$-Alkyl, $SO_2O$-Aryl, $SO_2O$-Heteroaryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(v) unsubstituiertes oder substituiertes Heteroaryl, wobei der Heteroarylrest mit F, Cl, Br, I, $CF_3$, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Aryl, OH, $OCF_3$, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2$-Alkyl, $SO_2$-Aryl, $SO_2NH_2$, $SO_2NH$-Alkyl, $SO_2NH$-Aryl, $SO_2NH$-Heteroaryl, S03H, $SO_2O$-Alkyl, $SO_2O$-Aryl, $SO_2O$-Heteroaryl, Alkyl, Cycloalkyl, Heterocyclyl, Aryl oder Heteroaryl, ein- oder mehrfach, gleich oder verschieden substituiert sein kann, bedeuten können;

bereitgestellt werden, die zur Herstellung eines Arzneimittels zur Behandlung oder Prophylaxe von durch Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" vermittelte physiologischen und/oder pathophysiologischen Zuständen in Säugetieren verwendet werden können.

In einer bevorzugten Ausführungsform werden Verbindungen gemäß der allgemeinen Formel (I) bereitgestellt, wobei der Alkylrest ausgewählt ist aus der Gruppe bestehend aus: "Methyl, Ethyl, n-Propyl, 2-Propyl, n-Butyl, sec.-Butyl, tert.-Butyl, n-Pentyl, iso-Pentyl, neo-Pentyl, n-Hexyl, 2-Hexyl, n-Octyl, Ethylenyl (Vinyl), Ethinyl, Propenyl (-CH2CH=CH2; -CH=CH-CH3, -C(=CH2)-CH3), Propinyl (-CH2-C≡CH, -C≡C-CH3), Butenyl, Butinyl, Pentenyl, Pentinyl, Hexenyl, Hexinyl, Heptenyl, Heptinyl, Octenyl, Octinyl", die zur Herstellung eines Arzneimittels zur Behandlung oder Prophylaxe von durch Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" vermittelte physiologischen und/oder pathophysiologischen Zuständen in Säugetieren verwendet werden können.

In einer weiteren bevorzugten Ausführungsform werden Verbindungen gemäß der allgemeinen Formel (I) zur oben dargestellten Verwendung bereitgestellt, wobei der Heterocyclyl-Rest ausgewählt ist aus der Gruppe bestehend aus: "Tetrahydrofuryl, Tetrahydropyranyl, Pyrrolidinyl, Piperidinyl, Piperazinyl, Morpholinyl".

In einer weiteren bevorzugten Ausführungsform werden Verbindungen gemäß der allgemeinen Formel (I) zur oben dargestellten Verwendung bereitgestellt, wobei der Heteroaryl-Rest ausgewählt ist aus der Gruppe bestehend aus: "Pyrrolyl, Furyl, Thienyl, Thiazolyl, Oxazolyl, Isoxazolyl, Pyrazolyl, Imidazolyl, Pyridinyl, Pyrimidinyl, Pyridazinyl, Pyrazinyl, Phthalazinyl, Indolyl, Indazolyl, Indolizinyl, Chinolinyl, Isochinolinyl, Chinoxalinyl, Chinazolinyl, Carbazolyl, Phenazinyl, Phenothiazinyl, Acridinyl".

In einer weiteren bevorzugten Ausführungsform werden Verbindungen gemäß der allgemeinen Formel (I) zur

oben dargestellten Verwendung bereitgestellt, wobei der Alkylrest ausgewählt ist aus der Gruppe bestehend aus: "Methyl, Ethyl, n-Propyl, 2-Propyl, n-Butyl, sec.-Butyl, tert.-Butyl, n-Pentyl, iso-Pentyl, neo-Pentyl, n-Hexyl, 2-Hexyl, n-Octyl, Ethylenyl (Vinyl), Ethinyl, Propenyl (-CH2CH=CH2; -CH=CH-CH3, -C(=CH2)-CH3), Propinyl (-CH2-C≡CH, -C≡C-CH3), Butenyl, Butinyl, Pentenyl, Pentinyl, Hexenyl, Hexinyl, Heptenyl, Heptinyl, Octenyl, Octinyl" und/oder der Heterocyclyl-Rest ausgewählt ist aus der Gruppe bestehend aus: "Tetrahydrofuryl, Tetrahydropyranyl, Pyrrolidinyl, Piperidinyl, Piperazinyl, Morpholinyl" und/oder der Heteroaryl-Rest ausgewählt ist aus der Gruppe bestehend aus: "Pyrrolyl, Furyl, Thienyl, Thiazolyl, Oxazolyl, Isoxazolyl, Pyrazolyl, Imidazolyl, Pyridinyl, Pyrimidinyl, Pyridazinyl, Pyrazinyl, Phthalazinyl, Indolyl, Indazolyl, Indolizinyl, Chinolinyl, Isochinolinyl, Chinoxalinyl, Chinazolinyl, Carbazolyl, Phenazinyl, Phenothiazinyl, Acridinyl".

Die erfinderische Aufgabe wurde in einem weiteren Aspekt überraschender Weise dadurch gelöst, dass Pyridopyrazin-Verbindungen ausgewählt aus der Gruppe bestehend aus:

Verbindung 1 1-Allyl-3-(3-phenyl-pyrido[2,3-b]pyrazin-6-yl)-thioharnstoff

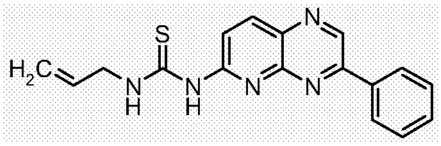

Verbindung 2 1-(2-Methyl-allyl)-3-(3-naphth-2-yl-pyrido[2,3-b]pyrazin-6-yl)-thioharnstoff

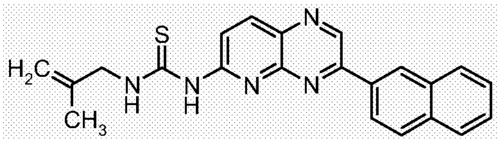

Verbindung 3 1-[3-(4-Methoxy-phenyl)-pyrido[2,3-b]pyrazin-6-yl]-3-(2-methyl-allyl)-thioharnstoff

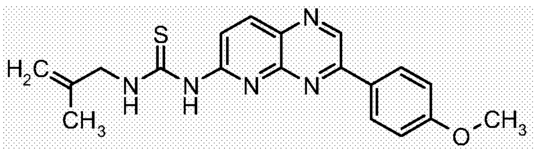

Verbindung 4 1-(2-Methyl-allyl)-3-(3-phenyl-pyrido[2,3-b]pyrazin-6-yl)-thioharnstoff

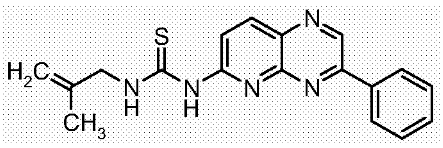

Verbindung 5 1-Allyl-3-(3-naphth-2-yl-pyrido[2,3-b]pyrazin-6-yl)-thioharnstoff

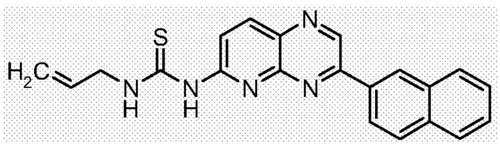

Verbindung 6 1-Allyl-3-[3-(4-methoxy-phenyl)-pyrido[2,3-b]pyrazin-6-yl]-thioharnstoff

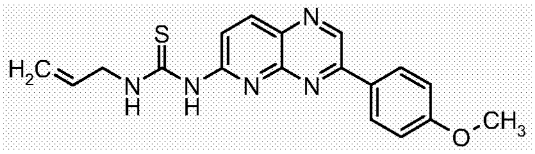

Verbindung 7 1-Allyl-3-[3-(4-hydroxy-phenyl)-pyrido[2,3-b]pyrazin-6-yl]-thioharnstoff Hydrochlorid

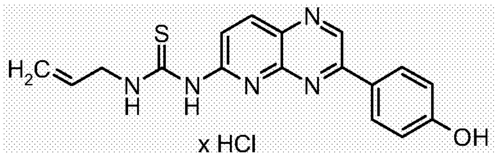

Verbindung 8 1-(3-Naphth-2-yl-pyrido[2,3-b]pyrazin-6-yl)-3-(4-nitro-phenyl)-thioharnstoff

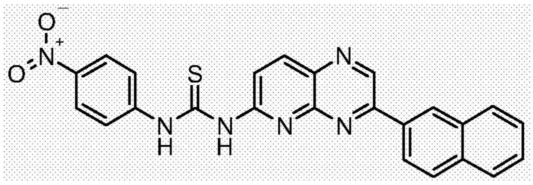

Verbindung 9 1-[3-(4-Methoxy-phenyl)-pyrido[2,3-b]pyrazin-6-yl]-3-(4-nitro-phenyl)-thioharnstoff

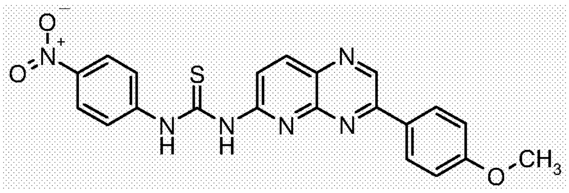

Verbindung 10 1-tert-Butyl-3-(3-phenyl-pyrido[2,3-b]pyrazin-6-yl)-thioharnstoff

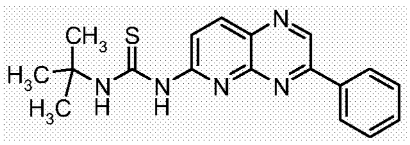

Verbindung 11 1-Cyclopropyl-3-(3-phenyl-pyrido[2,3-b]pyrazin-6-yl)-thioharnstoff

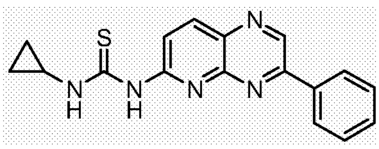

Verbindung 12 1-Methyl-3-(3-phenyl-pyrido[2,3-b]pyrazin-6-yl)-thioharnstoff

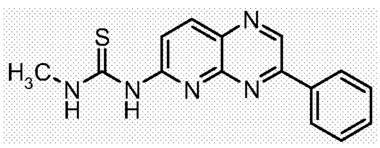

Verbindung 13 1-Benzyl-3-(3-phenyl-pyrido[2,3-b]pyrazin-6-yl)-thioharnstoff

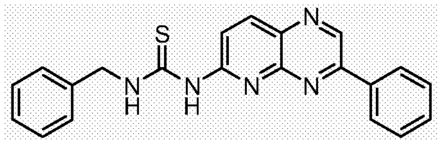

Verbindung 14 1-(4-Fluor-phenyl)-3-(3-phenyl-pyrido[2,3-b]pyrazin-6-yl)-thioharnstoff

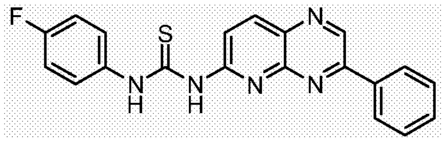

Verbindung 15 1-(3-Phenyl-pyrido[2,3-b]pyrazin-6-yl)-3-p-tolyl-harnstoff

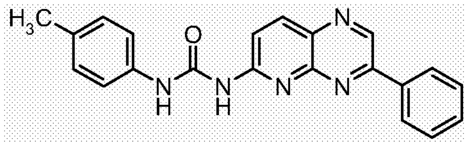

Verbindung 16 1-(4-Chlor-3-trifluormethyl-phenyl)-3-(3-phenyl-pyrido[2,3-b]pyrazin-6-yl)-harnstoff

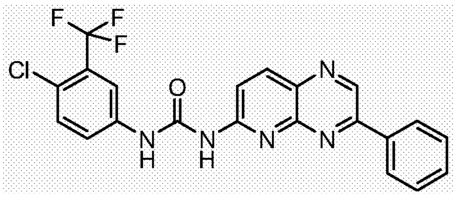

Verbindung 17 1-(2-Morpholin-4-yl-ethyl)-3-(3-phenyl-pyrido[2,3-b]pyrazin-6-yl)-harnstoff

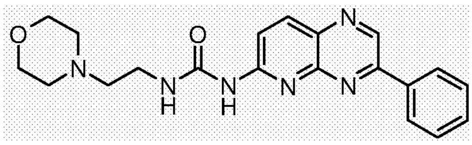

Verbindung 18 1-Cyclohexyl-3-(3-phenyl-pyrido[2,3-b]pyrazin-6-yl)-thioharnstoff

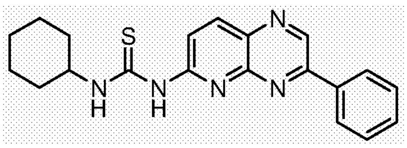

Verbindung 19 1-Isopropyl-3-(3-phenyl-pyrido[2,3-b]pyrazin-6-yl)-thioharnstoff

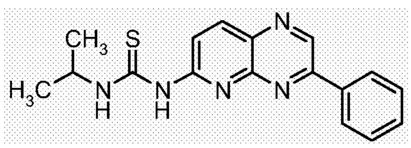

Verbindung 20 1-Furan-2-ylmethyl-3-(3-phenyl-pyrido[2,3-b]pyrazin-6-yl)-thioharnstoff

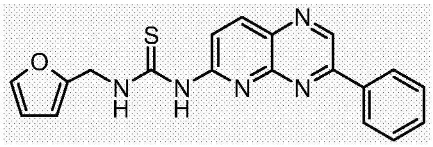

Verbindung 21 1-Cyclopropyl-3-(3-phenyl-pyrido[2,3-b]pyrazin-6-yl)-harnstoff

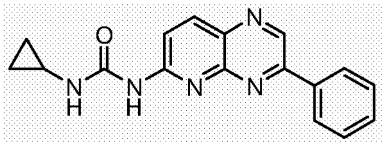

Verbindung 22 1-Methyl-3-[3-(4-nitro-phenyl)-pyrido[2,3-b]pyrazin-6-yl]-thioharnstoff

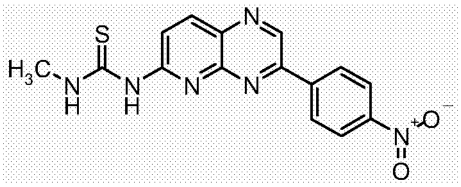

Verbindung 23 1-[3-(4-Hydroxy-phenyl)-pyrido[2,3-b]pyrazin-6-yl]-3-methyl-thioharnstoff

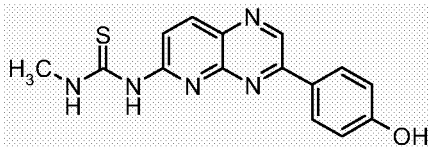

Verbindung 24 1-Allyl-3-[3-(4-nitro-phenyl)-pyrido[2,3-b]pyrazin-6-yl]-thioharnstoff

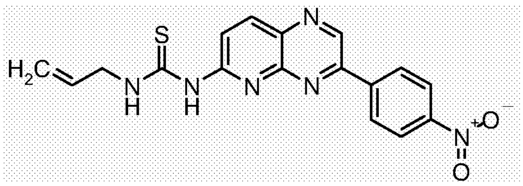

Verbindung 25 1-Allyl-3-[3-(4-hydroxy-phenyl)-pyrido[2,3-b]pyrazin-6-yl]-harnstoff

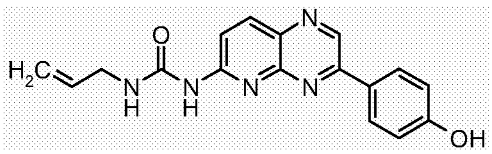

Verbindung 26 4-[6-(3-Allyl-thioureido)-pyrido[2,3-b]pyrazin-3-yl]-benzoesäure-ethyl-ester

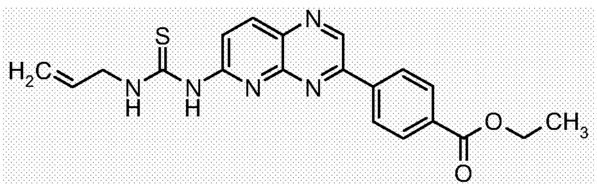

Verbindung 27 1-Allyl-3-[3-(3-hydroxy-phenyl)-pyrido[2,3-b]pyrazin-6-yl]-thioharnstoff

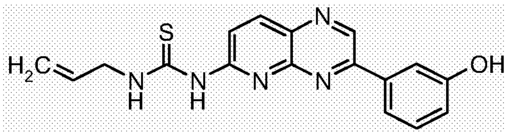

Verbindung 28 1-Allyl-3-(3-benzo[1,3]dioxol-5-yl-pyrido[2,3-b]pyrazin-6-yl)-thioharnstoff

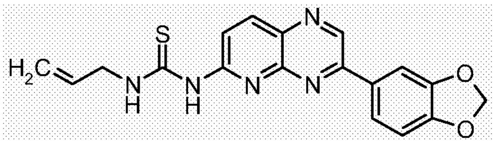

Verbindung 29 1-[3-(4-Hydroxy-phenyl)-pyrido[2,3-b]pyrazin-6-yl]-3-prop-2-inyl-thioharnstoff

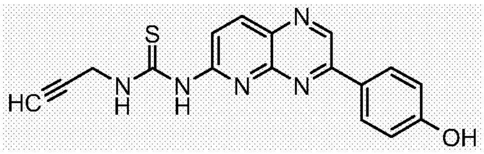

Verbindung 30 1-Allyl-3-[3-(4-hydroxy-phenyl)-pyrido[2,3-b]pyrazin-6-yl]-thioharnstoff

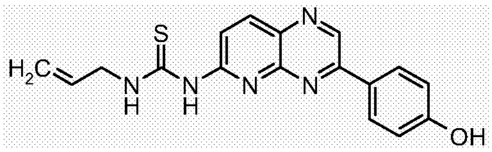

Verbindung 31 1-[3-(4-Hydroxy-phenyl)-pyrido[2,3-b]pyrazin-6-yl]-3-((E)-propenyl)-thioharnstoff

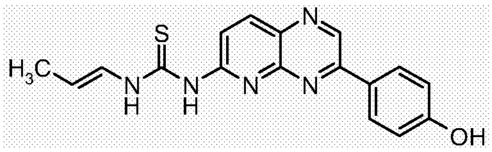

Verbindung 32 1-Allyl-3-[2,3-bis-(4-hydroxy-phenyl)-pyrido[2,3-b]pyrazin-6-yl]-thioharnstoff

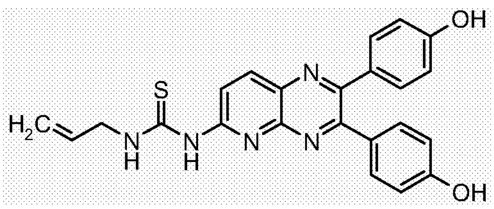

Verbindung 33 1-[2,3-Bis-(4-hydroxy-phenyl)-pyrido[2,3-b]pyrazin-6-yl]-3-((E)-propenyl)-thioharnstoff

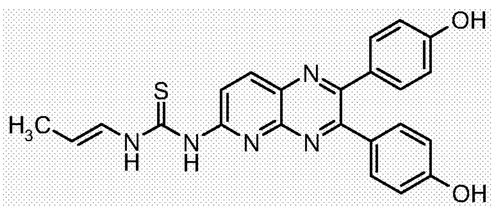

Verbindung 34 1-Allyl-3-[2-(4-hydroxy-phenyl)-pyrido[2,3-b]pyrazin-6-yl]-thioharnstoff

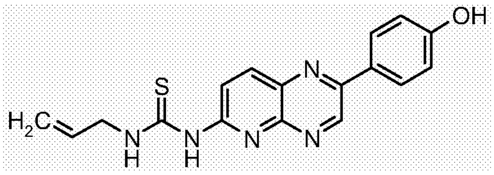

Verbindung 35 1-Phenethyl-3-(3-phenyl-pyrido[2,3-b]pyrazin-6-yl)-harnstoff

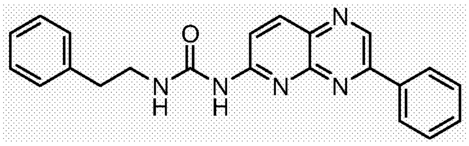

Verbindung 36 1-(2,3-Di-pyridin-2-yl-pyrido[2,3-b]pyrazin-6-yl)-3-ethyl-harnstoff

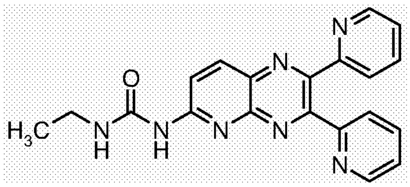

Verbindung 37 1-(2,3-Dimethyl-pyrido[2,3-b]pyrazin-6-yl)-3-ethyl-harnstoff

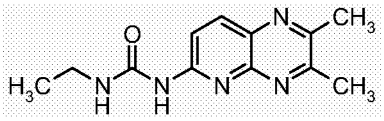

Verbindung 38 1-[3-(4-Hydroxy-phenyl)-pyrido[2,3-b]pyrazin-6-yl]-3-methyl-harnstoff

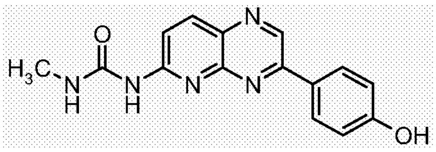

Verbindung 39 1-Allyl-3-[3-(4-phenoxy-phenyl)-pyrido[2,3-b]pyrazin-6-yl]-harnstoff

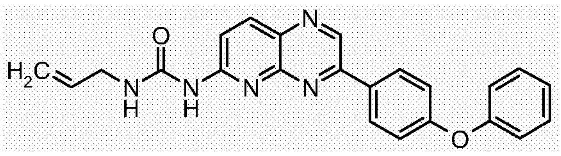

Verbindung 40 Methansulfonsäure-4-[6-(3-allyl-ureido)-pyrido[2,3-b]pyrazin-3-yl]-phenyl-ester

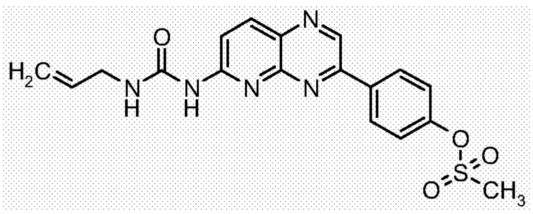

Verbindung 41 4-Dimethylamino-benzoesäure-4-[6-(3-allyl-ureido)-pyrido[2,3-b]pyrazin-3-yl]-phenyl-ester

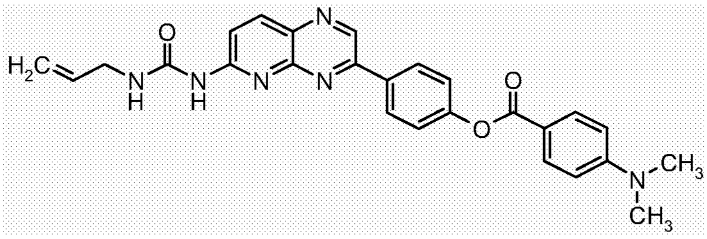

Verbindung 42 Essigsäure-4-[6-(3-allyl-ureido)-pyrido[2,3-b]pyrazin-3-yl]-phenyl-ester

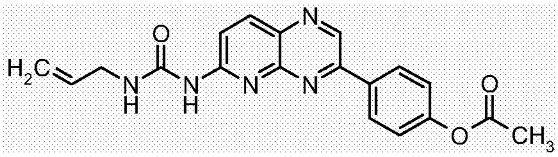

Verbindung 43 1-Ethyl-3-[3-(4-hydroxy-phenyl)-pyrido[2,3-b]pyrazin-6-yl]-harnstoff

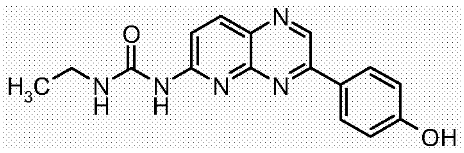

Verbindung 44 1-[3-(4-Amino-phenyl)-pyrido[2,3-b]pyrazin-6-yl]-3-methyl-thioharnstoff

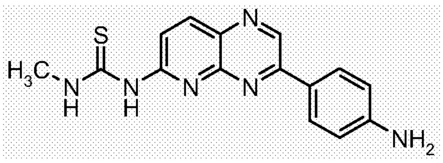

Verbindung 45 1-Ethyl-3-(3-phenyl-pyrido[2,3-b]pyrazin-6-yl)-harnstoff

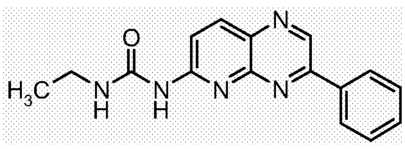

Verbindung 46 1-Acetyl-1-ethyl-3-(3-phenyl-pyrido[2,3-b]pyrazin-6-yl)-harnstoff

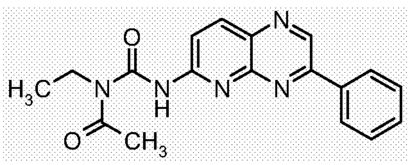

Verbindung 47 1-Allyl-3-[3-(4-fluoro-phenyl)-pyrido[2,3-b]pyrazin-6-yl]-harnstoff

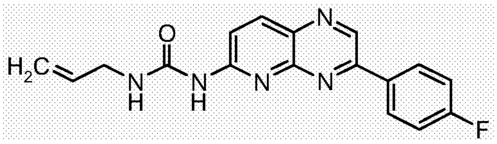

Verbindung 48 1,1-Diethyl-3-(3-phenyl-pyrido[2,3-b]pyrazin-6-yl)-harnstoff

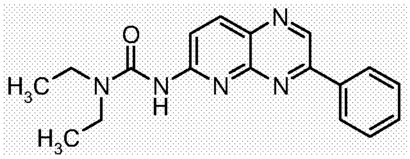

Verbindung 49 1-(2-Chlor-ethyl)-3-[3-(4-fluor-phenyl)-pyrido[2,3-b]pyrazin-6-yl]-harnstoff

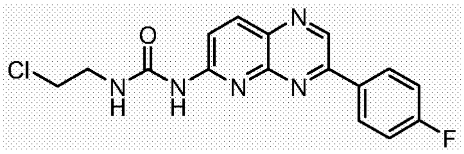

Verbindung 50 1-Ethyl-3-[3-(4-methoxy-phenyl)-pyrido[2,3-b]pyrazin-6-yl]-harnstoff

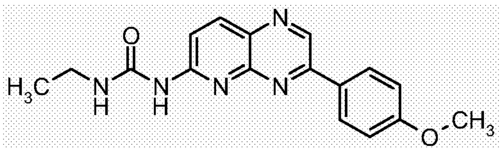

Verbindung 51 1-(3-Phenyl-pyrido[2,3-b]pyrazin-6-yl)-3-propyl-harnstoff

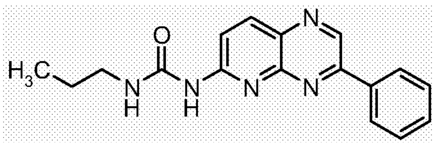

Verbindung 52 [3-(3-Phenyl-pyrido[2,3-b]pyrazin-6-yl)-ureido]-essigsäure-ethyl-ester

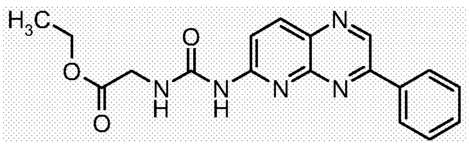

Verbindung 53 1-(3-Chlor-pyrido[2,3-b]pyrazin-6-yl)-3-ethyl-harnstoff

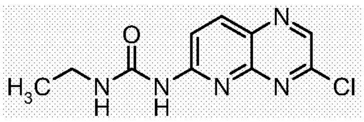

Verbindung 54 1-Ethyl-3-[3-(4-fluor-phenyl)-pyrido[2,3-b]pyrazin-6-yl]-harnstoff

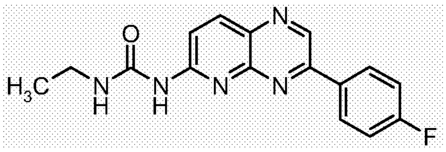

Verbindung 55 1-[3-(3-Chlor-4-fluor-phenyl)-pyrido[2,3-b]pyrazin-6-yl]-3-ethyl-harnstoff

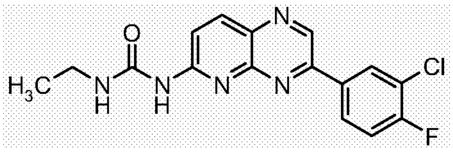

Verbindung 56 4-[6-(3-Ethyl-ureido)-pyrido[2,3-b]pyrazin-3-yl]-benzoesäure

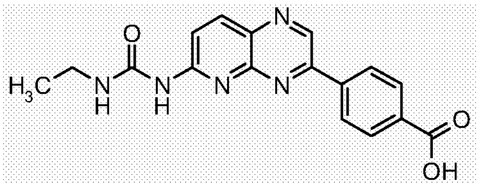

Verbindung 57 N-{4-[6-(3-Ethyl-ureido)-pyrido[2,3-b]pyrazin-3-yl]-phenyl}-acetamide

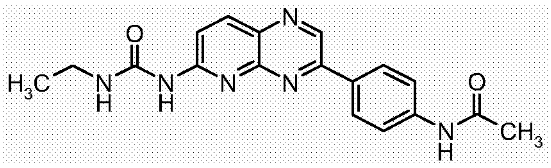

Verbindung 58 1-[3-(2,4-Difluor-phenyl)-pyrido[2,3-b]pyrazin-6-yl]-3-ethyl-harnstoff

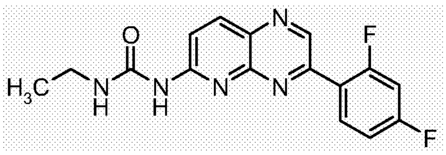

Verbindung 59 1-Ethyl-3-(3-morpholin-4-yl-pyrido[2,3-b]pyrazin-6-yl)-harnstoff

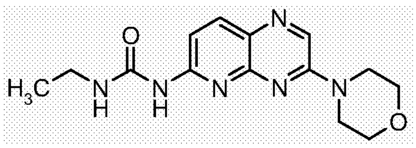

Verbindung 60 1-Ethyl-3-[3-(4-methyl-piperazin-1-yl)-pyrido[2,3-b]pyrazin-6-yl]-harnstoff

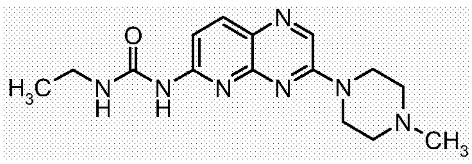

Verbindung 61 1-Ethyl-3-[3-(2-hydroxy-phenyl)-pyrido[2,3-b]pyrazin-6-yl]-harnstoff

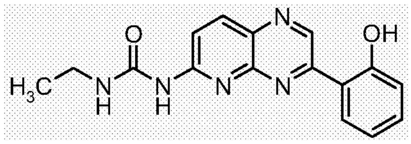

Verbindung 62 1-Ethyl-3-[3-(2-methoxy-ethylamino)-pyrido[2,3-b]pyrazin-6-yl]-harnstoff

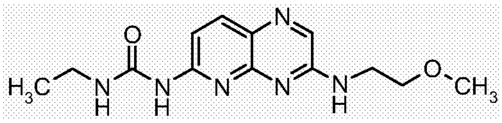

Verbindung 63 1-[3-(4-Chlor-3-trifluormethyl-phenyl)-pyrido[2,3-b]pyrazin-6-yl]-3-ethyl-harnstoff

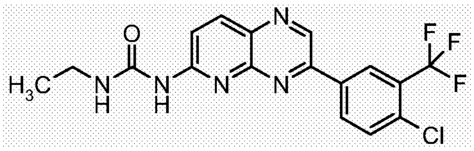

Verbindung 64 1-Ethyl-3-(3-phenoxy-pyrido[2,3-b]pyrazin-6-yl)-harnstoff

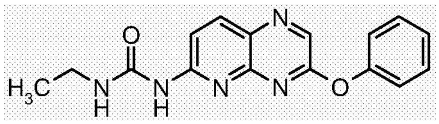

Verbindung 65 1-[3-(Cyclopropylmethyl-amino)-pyrido[2,3-b]pyrazin-6-yl]-3-ethyl-harnstoff

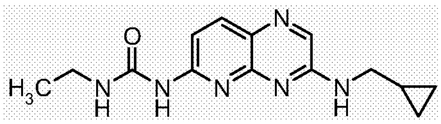

Verbindung 66 1-Ethyl-3-{3-[(pyridin-4-ylmethyl)-amino]-pyrido[2,3-b]pyrazin-6-yl}-harnstoff

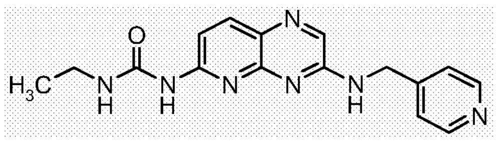

Verbindung 67 1-Ethyl-3-[3-(4-fluor-benzyloxy)-pyrido[2,3-b]pyrazin-6-yl]-harnstoff

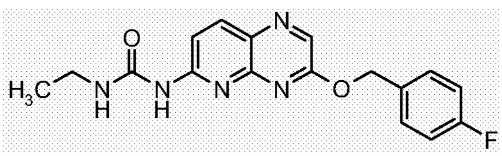

Verbindung 68 1-Ethyl-3-(3-pyridin-4-yl-pyrido[2,3-b]pyrazin-6-yl)-harnstoff

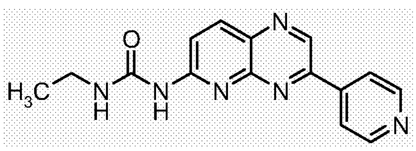

Verbindung 69 1-Ethyl-3-[3-(pyridin-3-yloxy)-pyrido[2,3-b]pyrazin-6-yl]-harnstoff

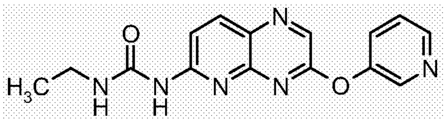

Verbindung 70 1-Ethyl-3-[3-(tetrahydro-furan-2-ylmethoxy)-pyrido[2,3-b]pyrazin-6-yl]-harnstoff

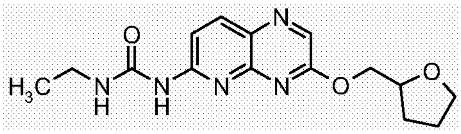

Verbindung 71 1-Ethyl-3-[3-(4-morpholin-4-yl-phenyl)-pyrido[2,3-b]pyrazin-6-yl]-harnstoff

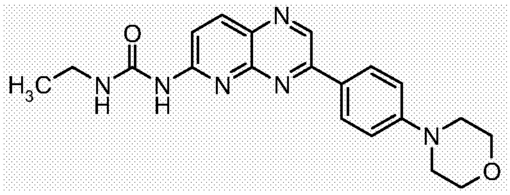

Verbindung 72 1-Ethyl-3-(3-hydroxy-pyrido[2,3-b]pyrazin-6-yl)-harnstoff

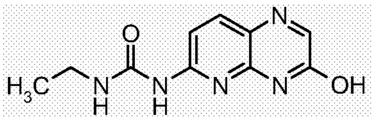

Verbindung 73 1-Ethyl-3-[3-(3-methoxy-phenylsulfanyl)-pyrido[2,3-b]pyrazin-6-yl]-harnstoff

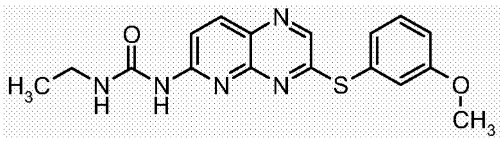

Verbindung 74 1-Ethyl-3-(3-quinolin-3-yl-pyrido[2,3-b]pyrazin-6-yl)-harnstoff

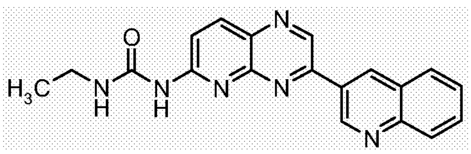

Verbindung 75 1-(3-Benzo[b]thiophen-3-yl-pyrido[2,3-b]pyrazin-6-yl)-3-ethyl-harnstoff

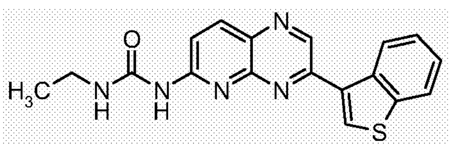

Verbindung 76 1-Ethyl-3-[3-(pyridin-2-ylsulfanyl)-pyrido[2,3-b]pyrazin-6-yl]-harnstoff

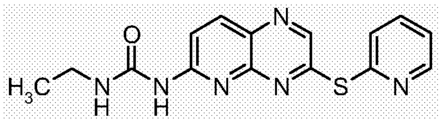

Verbindung 77 1-[3-(4-Dimethylamino-phenyl)-pyrido[2,3-b]pyrazin-6-yl]-3-ethyl-harnstoff

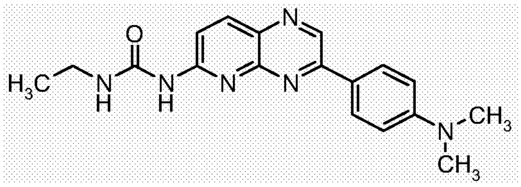

Verbindung 78 N-{4-[6-(3-Ethyl-ureido)-pyrido[2,3-b]pyrazin-3-yl]-phenyl}-methansulfonamid

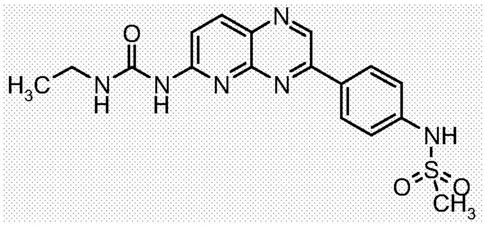

Verbindung 79 1-Ethyl-3-[3-(1H-pyrazol-4-yl)-pyrido[2,3-b]pyrazin-6-yl]-harnstoff

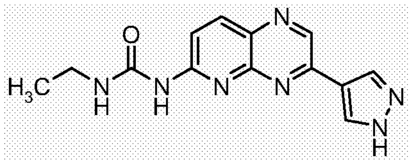

Verbindung 80 1-(3-Benzylsulfanyl-pyrido[2,3-b]pyrazin-6-yl)-3-ethyl-harnstoff

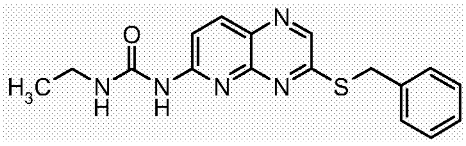

Verbindung 81 1-Ethyl-3-[3-(4-methyl-[1,4]diazepan-1-yl)-pyrido[2,3-b]pyrazin-6-yl]-harnstoff

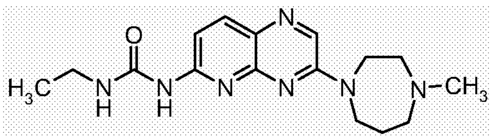

Verbindung 82 1-[3-(4-Amino-phenyl)-pyrido[2,3-b]pyrazin-6-yl]-3-ethyl-harnstoff

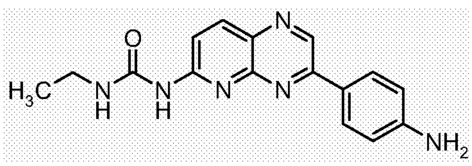

Verbindung 83 1-(3-Amino-pyrido[2,3-b]pyrazin-6-yl)-3-ethyl-harnstoff

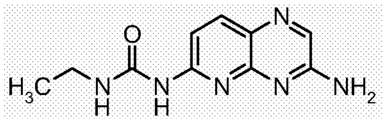

Verbindung 84 1-Ethyl-3-pyrido[2,3-b]pyrazin-6-yl-harnstoff

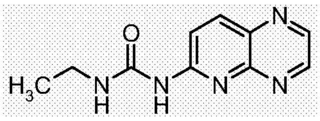

Verbindung 85 1-Ethyl-3-(3-imidazol-1-yl-pyrido[2,3-b]pyrazin-6-yl)-harnstoff

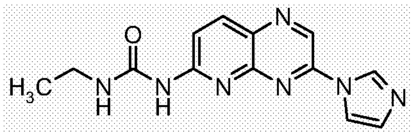

Verbindung 86 1-Ethyl-3-[3-(4-fluor-2-hydroxy-phenyl)-pyrido[2,3-b]pyrazin-6-yl]-harnstoff

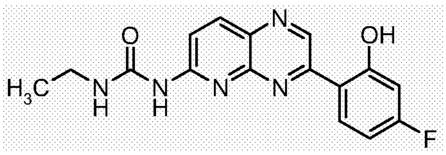

Verbindung 87 1-(3-Cyclopentyloxy-pyrido[2,3-b]pyrazin-6-yl)-3-ethyl-harnstoff

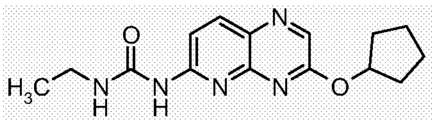

Verbindung 88 1-Ethyl-3-[3-(4-hydroxy-piperidin-1-yl)-pyrido[2,3-b]pyrazin-6-yl]-harnstoff

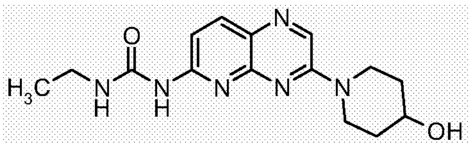

Verbindung 89 (3-Phenyl-pyrido[2,3-b]pyrazin-6-yl)-harnstoff

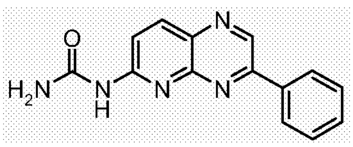

Verbindung 90 1-Ethyl-3-(3-pyrimidin-5-yl-pyrido[2,3-b]pyrazin-6-yl)-harnstoff

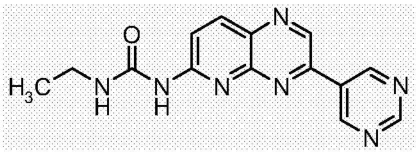

Verbindung 91 1-Ethyl-3-(3-pyridin-3-yl-pyrido[2,3-b]pyrazin-6-yl)-harnstoff

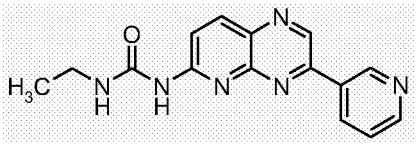

Verbindung 92 1-Allyl-3-(3-phenyl-pyrido[2,3-b]pyrazin-6-yl)-harnstoff

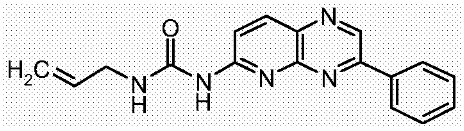

Verbindung 93 1-Ethyl-3-(3-piperazin-1-yl-pyrido[2,3-b]pyrazin-6-yl)-harnstoff

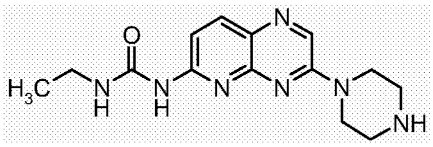

Verbindung 94 1-[3-(3-Chlor-pyridin-4-ylmethyl)-pyrido[2,3-b]pyrazin-6-yl]-3-ethyl-harnstoff

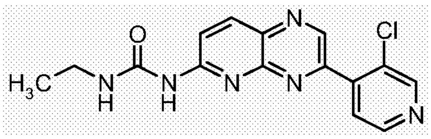

Verbindung 95 1-Ethyl-3-[3-(6-methoxy-pyridin-3-yl)-pyrido[2,3-b]pyrazin-6-yl]-harnstoff

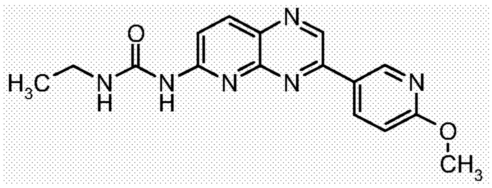

Verbindung 96 1-[3-(3,5-Dimethyl-isoxazol-4-yl)-pyrido[2,3-b]pyrazin-6-yl]-3-ethyl-harnstoff

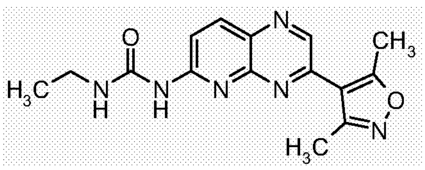

Verbindung 97 1-Ethyl-3-[3-(4-trifluormethoxy-phenyl)-pyrido[2,3-b]pyrazin-6-yl]-harnstoff

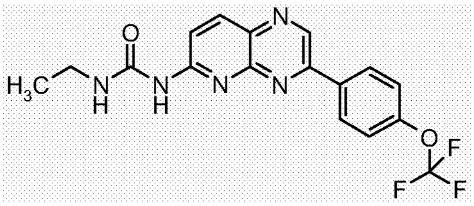

Verbindung 98 1-Ethyl-3-(3-furan-2-yl-pyrido[2,3-b]pyrazin-6-yl)-harnstoff

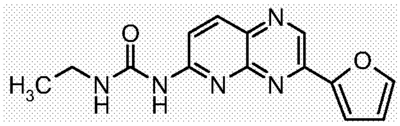

Verbindung 99 1-Ethyl-3-[3-(2-methoxy-pyridin-3-yl)-pyrido[2,3-b]pyrazin-6-yl]-harnstoff

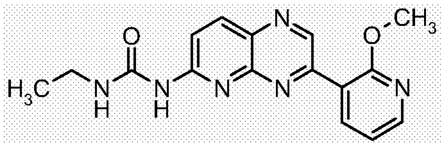

Verbindung 100 1-[3-(2,4-Dimethoxy-phenyl)-pyrido[2,3-b]pyrazin-6-yl]-3-ethyl-harnstoff

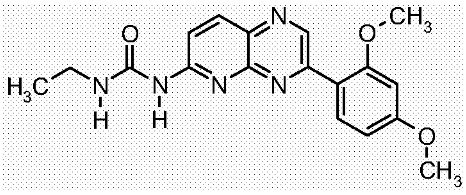

Verbindung 101 1-Ethyl-3-[3-(1H-pyrrol-2-yl)-pyrido[2,3-b]pyrazin-6-yl]-harnstoff

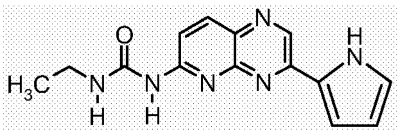

Verbindung 102 1-Ethyl-3-[3-(6-morpholin-4-yl-pyridin-3-yl)-pyrido[2,3-b]pyrazin-6-yl]-harnstoff

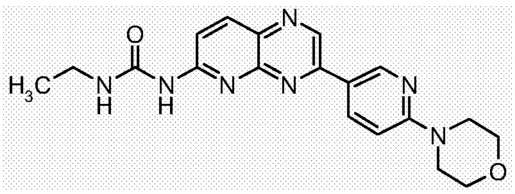

Verbindung 103 1-Benzyl-3-ethyl-1-(3-phenyl-pyrido[2,3-b]pyrazin-6-yl)-harnstoff

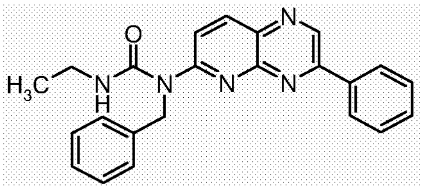

Verbindung 104 1-[3-(2-Amino-phenyl)-pyrido[2,3-b]pyrazin-6-yl]-3-ethyl-harnstoff

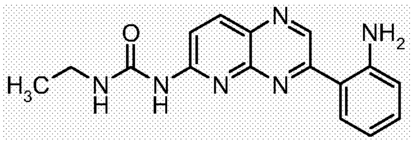

Verbindung 105 1-Ethyl-3-[3-(4-hydroxymethyl-phenyl)-pyrido[2,3-b]pyrazin-6-yl]-harnstoff

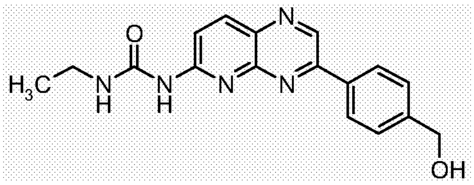

Verbindung 106 1-[3-(3-Amino-phenyl)-pyrido[2,3-b]pyrazin-6-yl]-3-ethyl-harnstoff

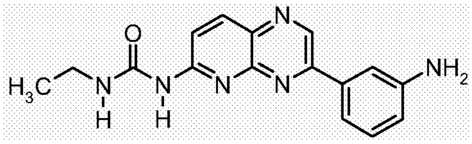

Verbindung 107 1-[3-(4-Acetyl-phenyl)-pyrido[2,3-b]pyrazin-6-yl]-3-ethyl-harnstoff

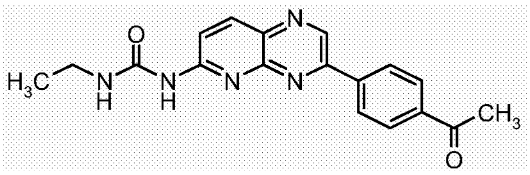

Verbindung 108 1-[3-(2,3-Dihydro-benzofuran-5-yl)-pyrido[2,3-b]pyrazin-6-yl]-3-ethyl-harnstoff

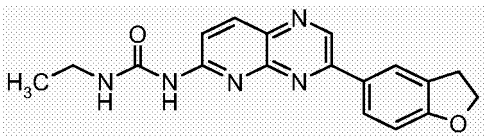

Verbindung 109 1-[3-(4-Benzyloxy-3-fluor-phenyl)-pyrido[2,3-b]pyrazin-6-yl]-3-ethyl-harnstoff

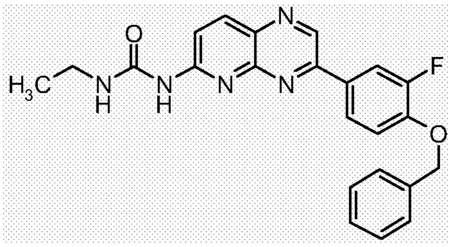

Verbindung 110 1-(2,3-Dihydroxy-propyl)-3-(3-phenyl-pyrido[2,3-b]pyrazin-6-yl)-harnstoff

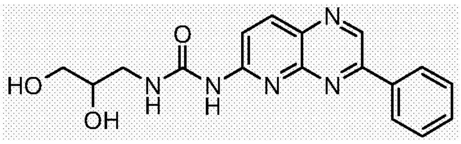

Verbindung 111 1-Ethyl-3-[3-(3-formyl-4-methoxy-phenyl)-pyrido[2,3-b]pyrazin-6-yl]-harnstoff

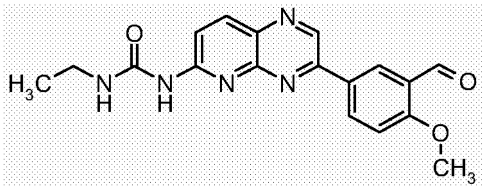

Verbindung 112 1-Ethyl-3-[3-(4-methansulfonyl-phenyl)-pyrido[2,3-b]pyrazin-6-yl]-harnstoff

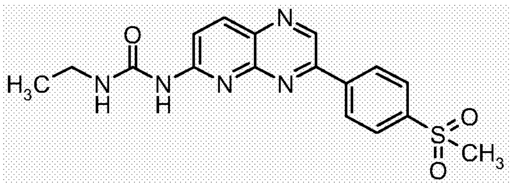

Verbindung 113 N-{4-[6-(3-Ethyl-ureido)-pyrido[2,3-b]pyrazin-3-yl]-phenyl}-succinamidsäure

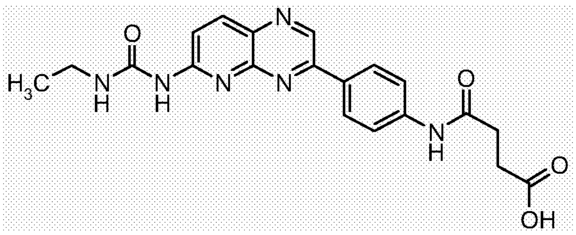

Verbindung 114 1-Ethyl-3-(3-pyridin-4-yl-pyrido[2,3-b]pyrazin-6-yl)-harnstoff Methansulfonsäuresalz (freie Base)

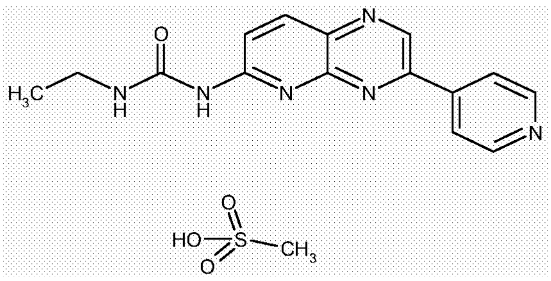

Verbindung 115 1-[3-(2,6-Dimethoxy-pyridin-3-yl)-pyrido[2,3-b]pyrazin-6-yl]-3-ethyl-harnstoff

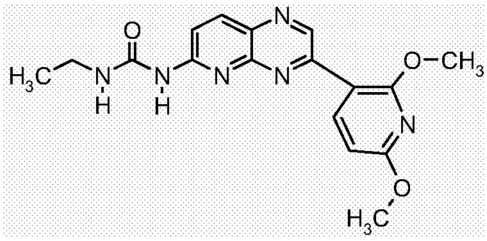

Verbindung 116 1-[3-(2,6-Dimethoxy-pyrimidin-4-yl)-pyrido[2,3-b]pyrazin-6-yl]-3-ethyl-harnstoff

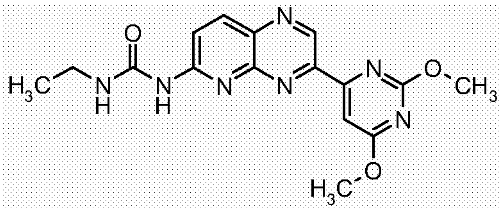

Verbindung 117 1-[3-(2,4-Dioxo-1,2,3,4-tetrahydro-pyrimidin-5-yl)-pyrido[2,3-b]pyrazin-6-yl]-3-ethyl-harnstoff

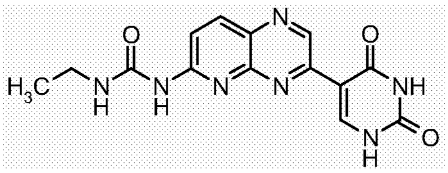

Verbindung 118 1-Ethyl-3-[3-(1H-indol-5-yl)-pyrido[2,3-b]pyrazin-6-yl]-harnstoff

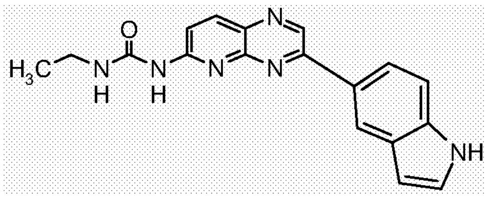

Verbindung 119 1-(3-Chloro-pyrido[2,3-b]pyrazin-6-yl)-3-ethyl-thioharnstoff

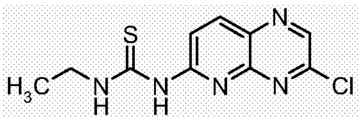

Verbindung 120 1-Ethyl-3-{3-[4-(2-methoxy-ethoxy)-phenyl]-pyrido[2,3-b]pyrazin-6-yl}-harnstoff

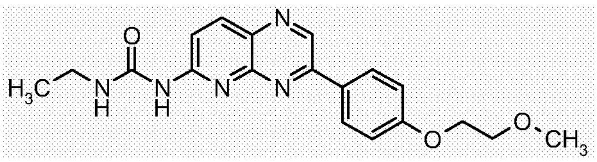

Verbindung 121 Acrylsäure-4-[6-(3-ethyl-ureido)-pyrido[2,3-b]-pyrazin-3-yl]-phenyl-ester

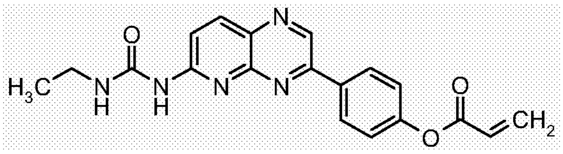

Verbindung 122 1-[3-(4-Cyano-phenyl)-pyrido[2,3-b]pyrazin-6-yl]-3-ethyl-harnstoff

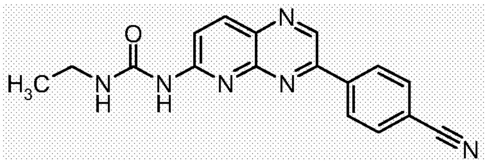

Verbindung 123 1-(3-Benzo[1,2,5]oxadiazol-4-yl-pyrido[2,3-b]pyrazin-6-yl)-3-ethyl-harnstoff

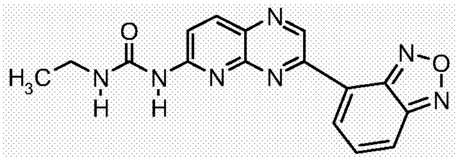

Verbindung 124 1-Ethyl-3-[3-(4-hydroxy-3-methoxy-phenyl)-pyrido[2,3-b]pyrazin-6-yl]-harnstoff

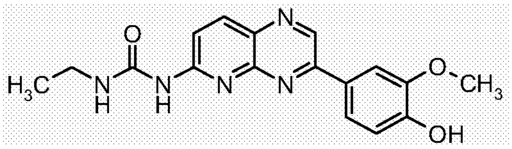

Verbindung 125 1-[3-(2,6-Dimethoxy-phenyl)-pyrido [2,3-b]pyrazin-6-yl]-3-ethyl-harnstoff

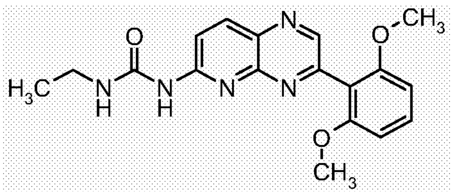

Verbindung 126 1-[3-(3-Acetyl-phenyl)-pyrido[2,3-b]pyrazin-6-yl]-3-ethyl-harnstoff

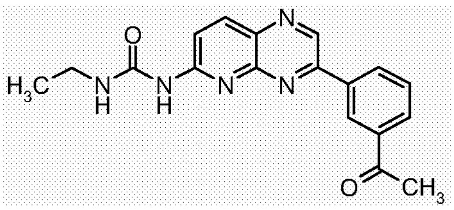

Verbindung 127 1-Ethyl-3-[3-(3-morpholin-4-yl-phenyl)-pyrido[2,3-b]pyrazin-6-yl]-harnstoff

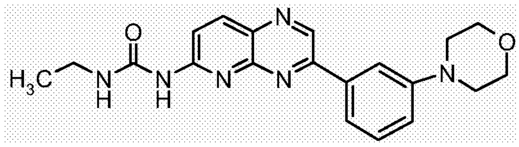

Verbindung 128 1-[3-(6-Amino-pyridin-3-yl)-pyrido[2,3-b]pyrazin-6-yl]-3-ethyl-harnstoff

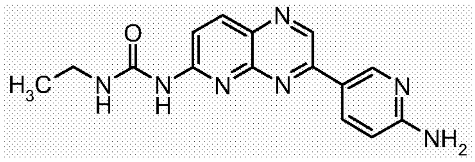

Verbindung 129 3-{4-[6-(3-Ethyl-ureido)-pyrido[2,3-b]-pyrazin-3-yl]-phenoxy}-propionsäure

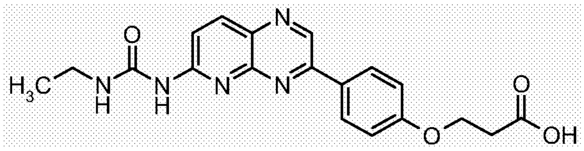

Verbindung 130 1-Isopropyl-3-(3-phenyl-pyrido[2,3-b]pyrazin-6-yl)-harnstoff

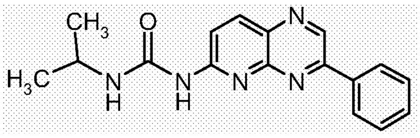

Verbindung 131 1-Cyclopentyl-3-(3-phenyl-pyrido[2,3-b]pyrazin-6-yl)-harnstoff

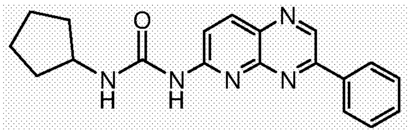

Verbindung 132 1-Pentyl-3-(3-phenyl-pyrido[2,3-b]pyrazin-6-yl)-harnstoff

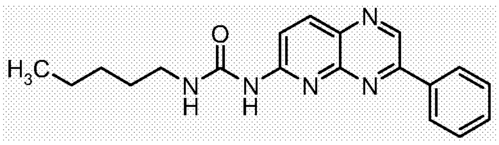

Verbindung 133 N-{4-[6-(3-Ethyl-ureido)-pyrido[2,3-b]pyrazin-3-yl]-phenyl}-acrylamid

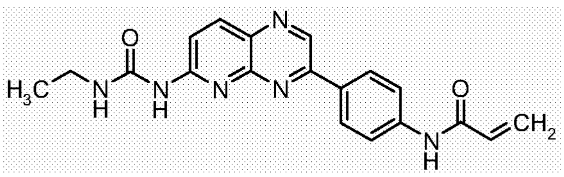

Verbindung 134 1-tert-Butyl-3-(3-phenyl-pyrido[2,3-b]pyrazin-6-yl)-harnstoff

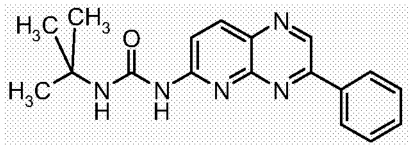

Verbindung 135 1-(2-Hydroxy-ethyl)-3-(3-phenyl-pyrido[2,3-b]pyrazin-6-yl)-harnstoff

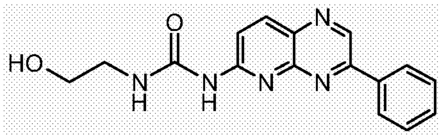

Verbindung 136 1-Cyclobutyl-3-(3-phenyl-pyrido[2,3-b]pyrazin-6-yl)-harnstoff

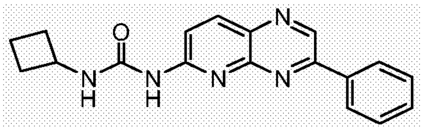

Verbindung 137 1-Allyl-3-[3-(4-nitro-phenyl)-pyrido[2,3-b]pyrazin-7-yl]-thioharnstoff

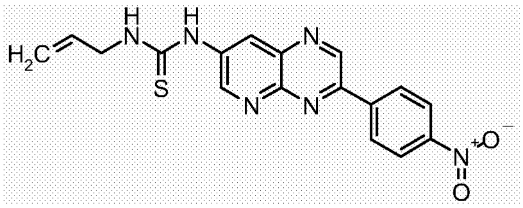

Verbindung 138 1-Ethyl-1-(Ethylcarbamoyl)-3-(3-phenyl-pyrido[2,3-b]pyrazin-6-yl)-harnstoff

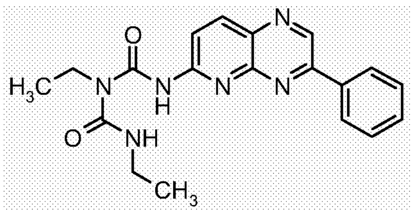

Verbindung 139 [3-(4-Hydroxy-phenyl)-pyrido[2,3-b]pyrazin-6-yl]-carbaminsäure-allyl-ester

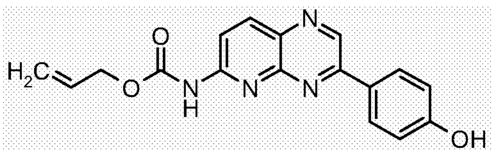

Verbindung 140 (3-Phenyl-pyrido[2,3-b]pyrazin-6-yl)-carbaminsäure-ethyl-ester

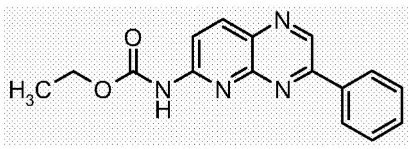

Verbindung 141 1-Ethyl-3-[3-(4-phenyl-piperazin-1-yl)-pyrido[2,3-b]pyrazin-6-yl]-harnstoff

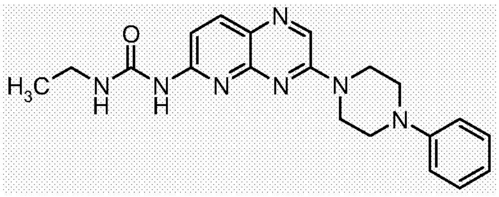

Verbindung 142 1-(2,2-Dimethyl-[1,3]dioxolan-4-ylmethyl)-3-(3-phenyl-pyrido[2,3-b]pyrazin-6-yl)-harnstoff

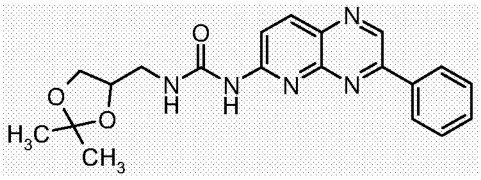

Verbindung 143 1,3-Bis-(3-phenyl-pyrido[2,3-b]pyrazin-6-yl)-harnstoff

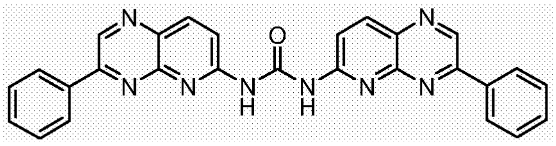

Verbindung 144 N-(3-Phenyl-pyrido[2,3-b]pyrazin-6-yl)-acetamidin

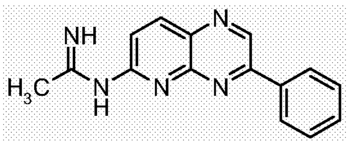

Verbindung 145 1-Ethyl-3-[3-(2-methoxy-pyridin-3-yl)-pyrido[2,3-b]pyrazin-6-yl]-thioharnstoff

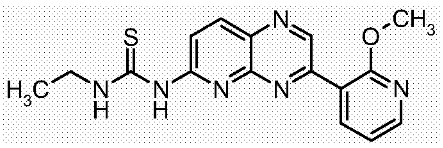

Verbindung 146 1-(4-Hydroxy-butyl)-3-(3-phenyl-pyr ido[2,3-b]pyrazin-6-yl)-harnstoff

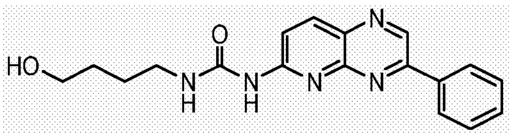

Verbindung 147 1-(3-Hydroxy-propyl)-3-(3-phenyl-py rido[2,3-b]pyrazin-6-yl)-harnstoff

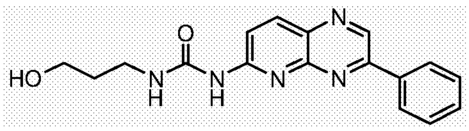

Verbindung 148 1-Ethyl-3-{3-[4-(3-morpholin-4-yl- propoxy)-phenyl]-pyrido[2,3-b]pyrazin-6-yl}-harnstoff

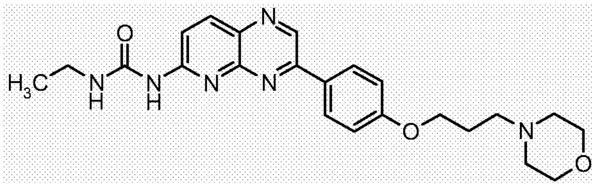

Verbindung 149 1-Ethyl-3-[3-(4-hydroxy-phenyl)- pyrido[2,3-b]pyrazin-6-yl]-thioharnstoff

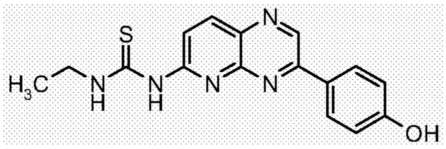

Verbindung 150 1-Ethyl-3-(3-p-tolylamino-pyrido[2,3-b]pyrazin-6-yl)-harnstoff

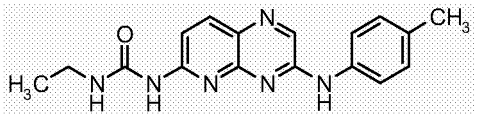

Verbindung 151 1-Ethyl-3-[3-(3-hydroxy-phenyl)- pyrido[2,3-b]pyrazin-6-yl]-harnstoff

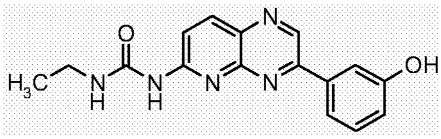

Verbindung 152 3-[6-(3-Ethyl-ureido)-pyrido[2,3-b]pyrazin-3-yl]-benzoesäure

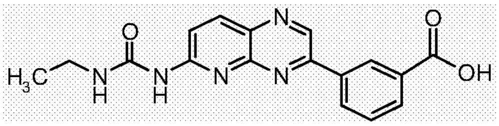

Verbindung 153 1-[3-(3,4-Dimethoxy-phenyl)-pyrido [2,3-b]pyrazin-6-yl]-3-ethyl-harnstoff

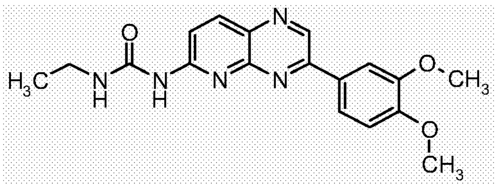

Verbindung 154 1-[3-(4-Amino-3-methoxy-phenyl)-pyrido[2,3-b]pyrazin-6-yl]-3-ethyl-harnstoff

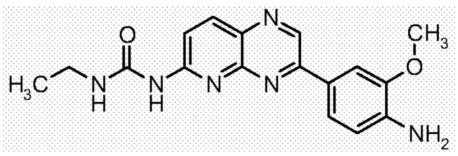

Verbindung 155 3-[6-(3-Ethyl-ureido)-pyrido[2,3-b]-pyrazin-3-yl]-benzamid

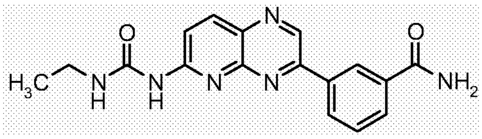

Verbindung 156 1-[3-(3-Chlor-4-hydroxy-5-methoxy-phenyl)-pyrido[2,3-b]pyrazin-6-yl]-3-ethyl-harnstoff

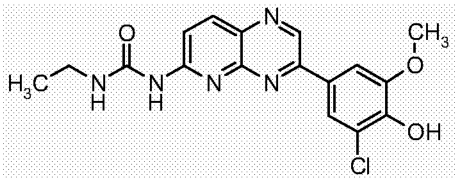

Verbindung 157 1-Ethyl-3-(3-m-tolylamino-pyrido-[2,3-b]pyrazin-6-yl)-harnstoff

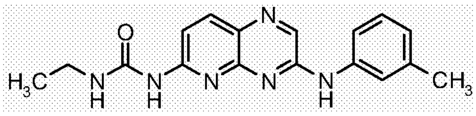

Verbindung 158 1-Ethyl-3-[3-(4-methoxy-phenylamino)-pyrido[2,3-b]pyrazin-6-yl]-harnstoff

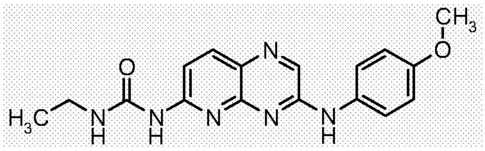

Verbindung 159 1-[3-(4-Chloro-phenylamino)-pyrido-[2,3-b]pyrazin-6-yl]-3-ethyl-harnstoff

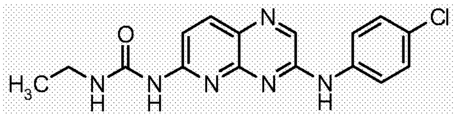

Verbindung 160 1-Ethyl-3-(3-o-tolylamino-pyrido[2,3-b]-pyrazin-6-yl)-harnstoff

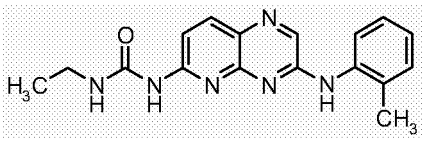

Verbindung 161 1-Ethyl-3-[3-(pyridin-3-ylamino)-pyrido-[2,3-b]pyrazin-6-yl]-harnstoff

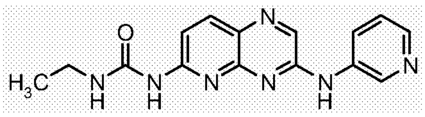

Verbindung 162 1-Ethyl-3-[3-(4-ethyl-phenylamino)-pyrido[2,3-b]pyrazin-6-yl]-harnstoff

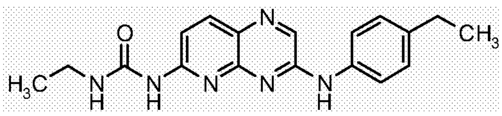

Verbindung 163 1-Ethyl-3-[3-(3-methoxy-4-methyl-phenylamino)-pyrido[2,3-b]pyrazin-6-yl]-harnstoff

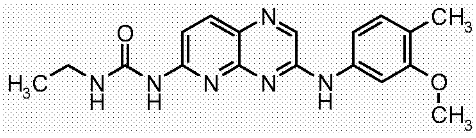

Verbindung 164 1-Ethyl-3-[3-(4-hydroxy-phenylamino)-pyrido[2,3-b]pyrazin-6-yl]-harnstoff

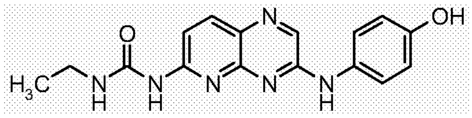

Verbindung 165 1-Ethyl-3-[3-(5-methyl-pyridin-2-yl-amino)-pyrido[2,3-b]pyrazin-6-yl]-harnstoff

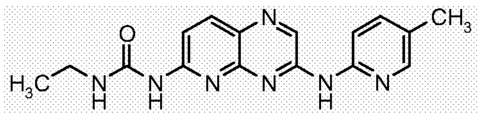

Verbindung 166 1-Ethyl-3-[3-(1-methyl-1H-pyrazol-3-ylamino)-pyrido[2,3-b]pyrazin-6-yl]-harnstoff

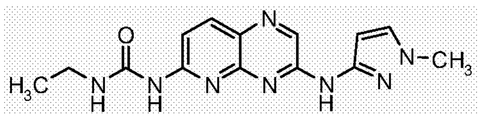

Verbindung 167 1-Ethyl-3-[3-(4-fluor-phenylamino)-pyrido[2,3-b]pyrazin-6-yl]-harnstoff

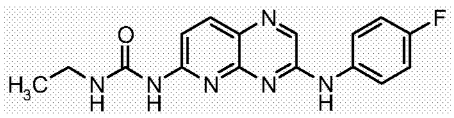

Verbindung 168 1-(4-Hydroxy-butyl)-3-[3-(4-hydroxy-3-methoxy-phenyl)-pyrido[2,3-b]pyrazin-6-yl]-harnstoff

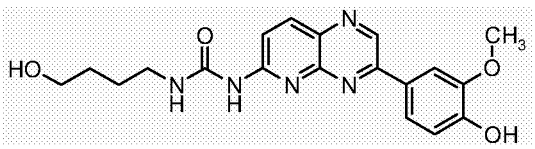

Verbindung 169 Phosphorsäure-mono-{4-[6-(3-ethyl-ureido)-pyrido-[2,3-b]pyrazin-3-yl]-2-methoxy-phenyl}-ester

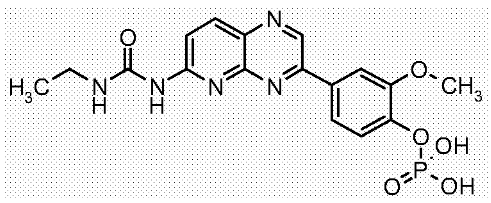

Verbindung 170 1-[3-(2-Chlor-4-hydroxy-phenyl)-pyrido[2,3-b]pyrazin-6-yl]-3-ethyl-harnstoff

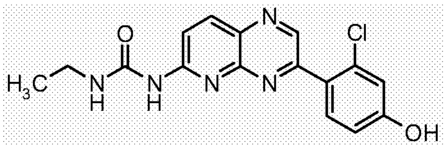

Verbindung 171 1-[3-(4-Chlor-2-methoxy-phenyl)-pyrido[2,3-b]pyrazin-6-yl]-3-ethyl-harnstoff

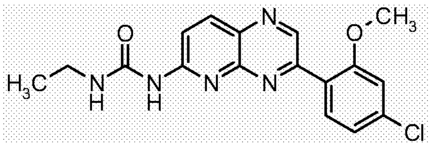

Verbindung 172 1-[3-(3,5-Dimethyl-1 H-pyrazol-4-yl)-pyrido[2,3-b]pyrazin-6-yl]-3-ethyl-harnstoff

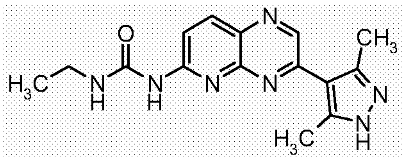

Verbindung 173 1-Ethyl-3-[3-(1-methyl-1H-pyrazol-4-yl)-pyrido[2,3-b]pyrazin-6-yl]-harnstoff

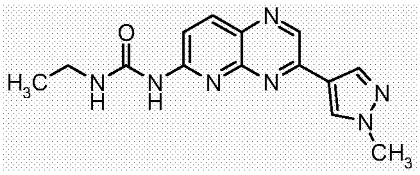

Verbindung 174 1-[3-(5-Cyano-thiophen-2-yl)-pyrido[2,3-b]pyrazin-6-yl]-3-ethyl-harnstoff

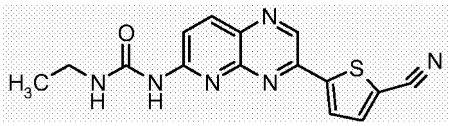

Verbindung 175 Natrium 2-chlor-4-[6-(3-ethyl-ureido)-pyrido[2,3-b]pyrazin-3-yl]-6-methoxy-phenolat

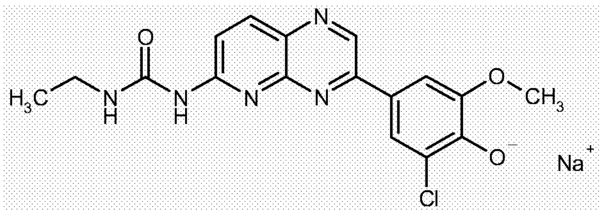

Verbindung 176 1-[3-(4-Hydroxy-3-methoxy-phenyl)-pyrido[2,3-b]pyrazin-6-yl]-3-(4-morpholin-4-yl-butyl)-harnstoff

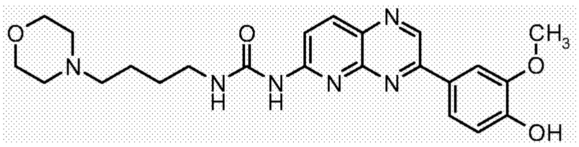

Verbindung 177 1-[3-(3-Chlor-4-methoxy-phenylamino)-pyrido[2,3-b]pyrazin-6-yl]-3-ethyl-harnstoff

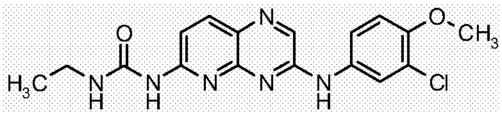

Verbindung 178 1-Ethyl-3-[3-(naphthalin-2-ylamino)-pyrido[2,3-b]pyrazin-6-yl]-harnstoff

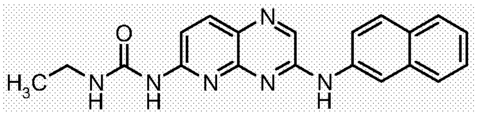

Verbindung 179 1-Ethyl-3-[3-(chinolin-3-ylamino)-pyrido[2,3-b]pyrazin-6-yl]-harnstoff

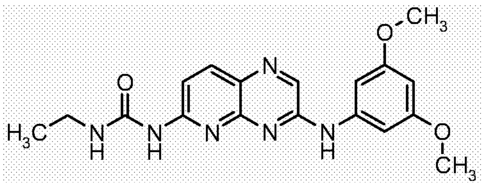

Verbindung 180 1-[3-(3,5-Dimethoxy-phenylamino)-pyrido[2,3-b]pyrazin-6-yl]-3-ethyl-harnstoff

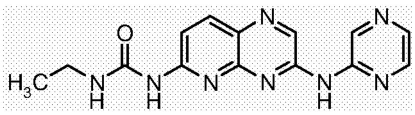

Verbindung 181 1-Ethyl-3-[3-(pyrazin-2-ylamino)-pyrido[2,3-b]pyrazin-6-yl]-harnstoff

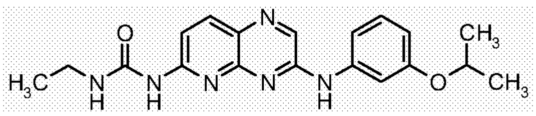

Verbindung 182 1-Ethyl-3-[3-(3-isopropoxy-phenylamino)-pyrido[2,3-b]pyrazin-6-yl]-harnstoff

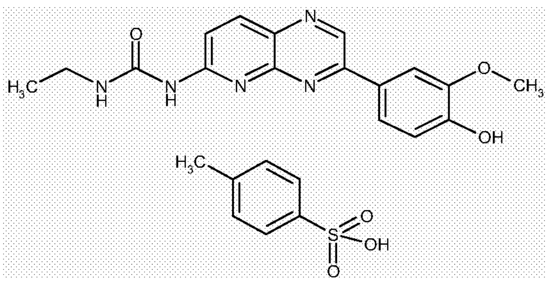

Verbindung 183 1-Ethyl-3-[3-(4-hydroxy-3-methoxy-phenyl)-pyrido[2,3-b]pyrazin-6-yl]-harnstoff p-Toluolsulfonat

Verbindung 184 1-[3-(2-Chlor-pyridin-4-ylamino)-pyrido[2,3-b]pyrazin-6-yl]-3-ethyl-harnstoff

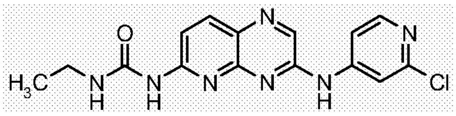

Verbindung 185 1-[3-(3,5-Dichlor-4-hydroxy-phenylamino)-pyrido[2,3-b]pyrazin-6-yl]-3-ethyl-harnstoff

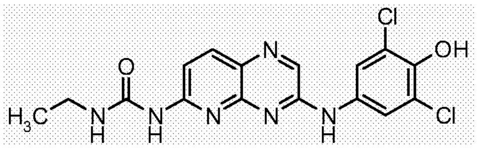

Verbindung 186 1-[3-(3,5-Dichlor-4-hydroxy-phenyl)-pyrido[2,3-b]pyrazin-6-yl]-3-ethyl-harnstoff

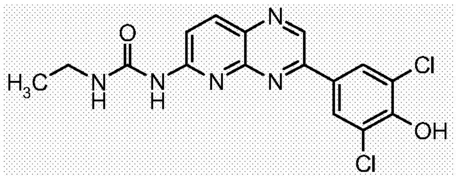

Verbindung 187 1-[3-(3,4-Dimethoxy-phenylamino)-pyrido[2,3-b]pyrazin-6-yl]-3-ethyl-harnstoff

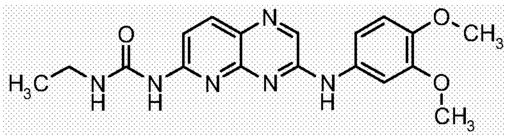

Verbindung 188 1-Ethyl-3-[3-(3-hydroxy-4-methyl-phenylamino)-pyrido[2,3-b]pyrazin-6-yl]-harnstoff

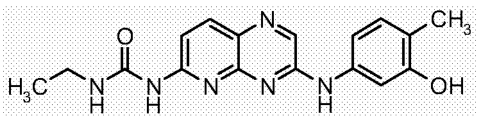

Verbindung 189 3-[6-(3-Ethyl-ureido)-pyrido[2,3-b]-pyrazin-3-ylamino]-5-trifluormethyl-benzoesäure

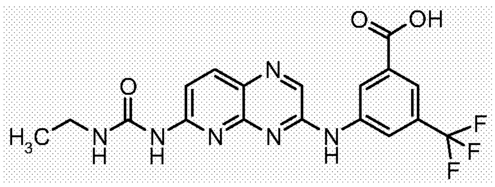

Verbindung 190 1-Ethyl-3-[3-(6-methoxy-pyridin-3-ylamino)-pyrido[2,3-b]pyrazin-6-yl]-harnstoff

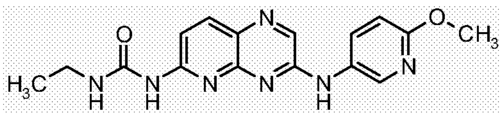

Verbindung 191 1-[3-(3,5-Dimethyl-phenylamino)-pyrido[2,3-b]pyrazin-6-yl]-3-ethyl-harnstoff

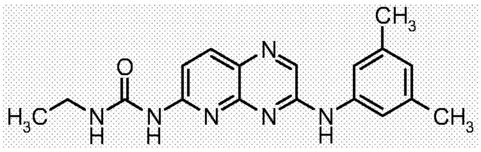

Verbindung 192 1-[3-(4-Cyano-phenylamino)-pyrido-[2,3-b]pyrazin-6-yl]-3-ethyl-harnstoff

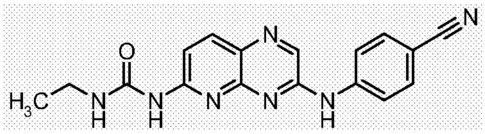

Verbindung 193 1-Ethyl-3-[3-(4-hydroxy-3-methoxy-phenyl)-pyrido[2,3-b]pyrazin-6-yl]-harnstoff (Z)-but-2-endicarbonsäure-Salz

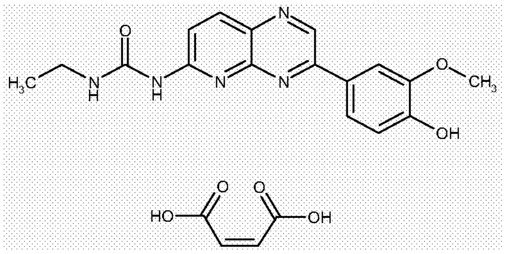

Verbindung 194 1-Ethyl-3-[3-(4-hydroxy-3-methoxy-phenyl)-pyrido[2,3-b]pyrazin-6-yl]-harnstoff Hydrochlorid

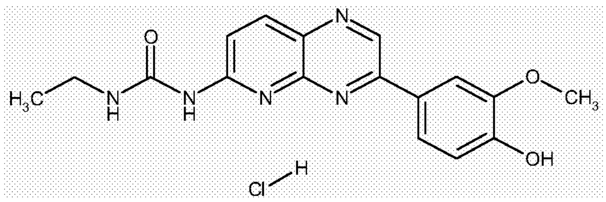

Verbindung 195 1-Ethyl-3-(3-phenyl-pyrido[2,3-b]pyrazin-6-yl)-thioharnstoff

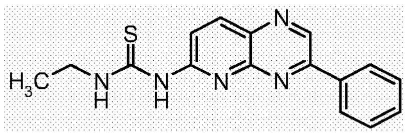

Verbindung 196 1-(3-Chlor-pyrido[2,3-b]pyrazin-6-yl)-3-cyclohexyl-harnstoff

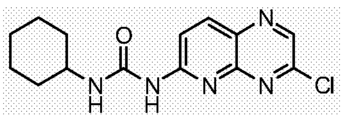

Verbindung 197 1-Ethyl-3-[3-(4-hydroxy-3-methoxy-phenylamino)-pyrido[2,3-b]pyrazin-6-yl]-harnstoff

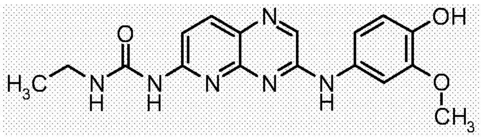

Verbindung 198 1-Ethyl-3-[3-(4-hydroxy-3,5-dimethyl-phenyl)-pyrido[2,3-b]pyrazin-6-yl]-harnstoff

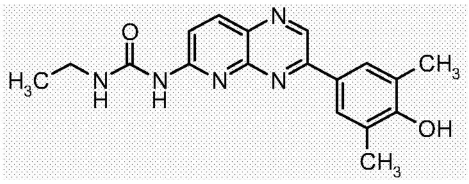

Verbindung 199 3-Ethyl-1-phenethyl-1-(3-phenyl- pyrido[2,3-b]pyrazin-6-yl)-harnstoff

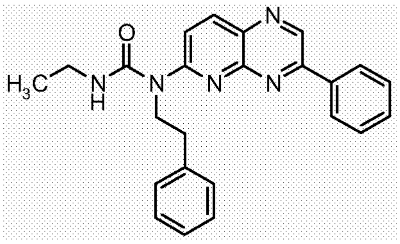

Verbindung 200 1-Allyl-3-{3-[4-(tetrahydro-pyran-2-yloxy)-phenyl]-pyrido[2,3-b]pyrazin-6-yl}-thioharnstoff

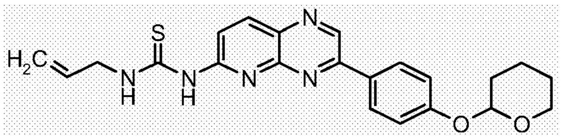

Verbindung 201 3-Ethyl-1-[3-(4-methoxy-phenyl)- pyrido[2,3-b]pyrazin-6-yl]-1-propyl- harnstoff

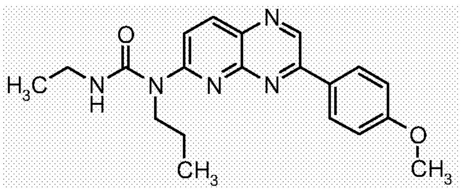

Verbindung 202 3-Ethyl-1-(3-phenyl-pyrido[2,3-b]pyrazin-6-yl)-1-(2-piperidin-1-yl-ethyl)-harnstoff Hydrochlorid

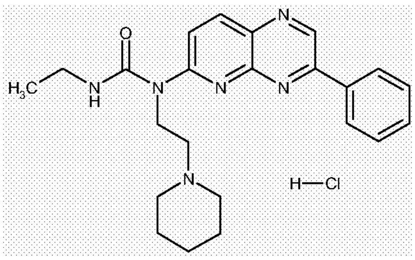

Verbindung 203 N-{4-[6-(3-Ethyl-ureido)-pyrido[2,3-b]- pyrazin-3-yl]-phenyl}-2-phenyl- acetamid

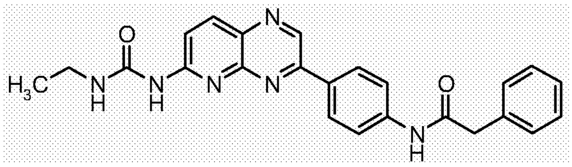

Verbindung 204 1-(4-Hydroxy-butyl)-3-(3-phenyl-pyrido[2,3-b]pyrazin-6-yl)-harnstoff Hydrochlorid

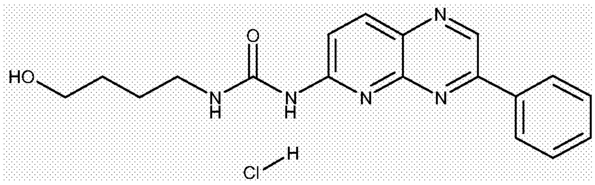

Verbindung 205 Essigsäure-4-[3-(3-phenyl-pyrido[2,3-b]pyrazin-6-yl)-ureido]-butyl ester

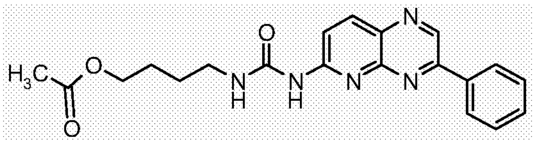

Verbindung 206 1-(4-Amino-butyl)-3-(3-phenyl-pyrido[2,3-b]pyrazin-6-yl)-harnstoff

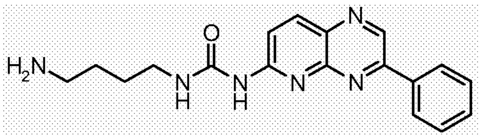

Verbindung 207 1-(5-Hydroxy-pentyl)-3-(3-phenyl-pyrido[2,3-b]pyrazin-6-yl)-harnstoff

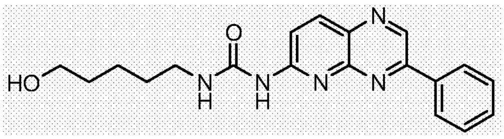

Verbindung 208 3-[6-(3-Ethyl-ureido)-pyrido[2,3-b]-pyrazin-3-yl]-N-methyl-benzamid

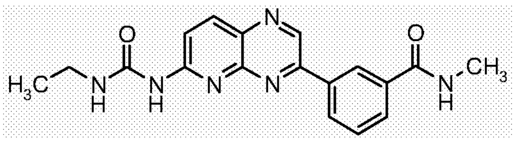

Verbindung 209 1-Ethyl-3-[3-(2-methoxy-5-methyl-phenyl)-pyrido[2,3-b]pyrazin-6-yl]-harnstoff

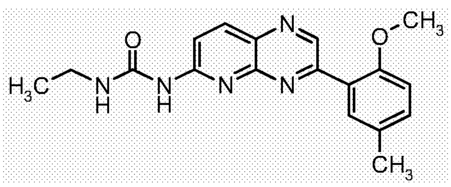

Verbindung 210 1-Ethyl-3-(3-p-tolyl-pyrido[2,3-b] pyrazin-6-yl)-harnstoff

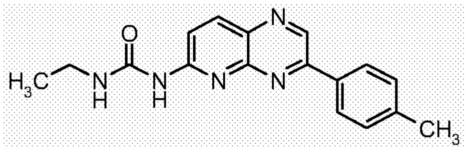

Verbindung 211 1-Ethyl-3-[3-(methyl-p-tolyl-amino)-pyrido[2,3-b]pyrazin-6-yl]-harnstoff

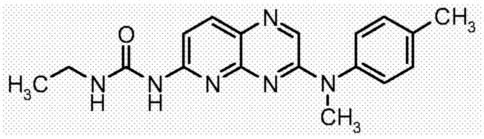

Verbindung 212 1-Ethyl-3-[3-(2-p-tolyl-ethylamino)-pyrido[2,3-b]pyrazin-6-yl]-harnstoff

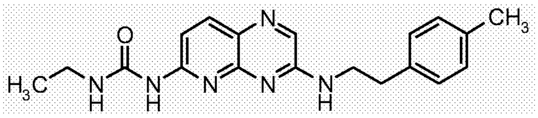

Verbindung 213 1-Ethyl-3-[3-(4-methyl-benzylamino)-pyrido[2,3-b]pyrazin-6-yl]-harnstoff

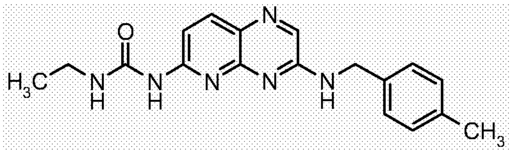

Verbindung 214 1-Ethyl-3-[3-(3-fluor-4-methyl-phenyl-amino)-pyrido[2,3-b]pyrazin-6-yl]-harnstoff

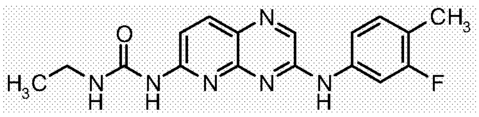

Verbindung 215 1-[3-(3,4-Dimethyl-phenylamino)-pyrido[2,3-b]pyrazin-6-yl]-3-ethyl-harnstoff

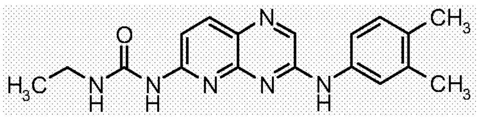

Verbindung 216 1-Ethyl-3-[3-(4-isopropyl-phenylamino)-pyrido[2,3-b]pyrazin-6-yl]-harnstoff

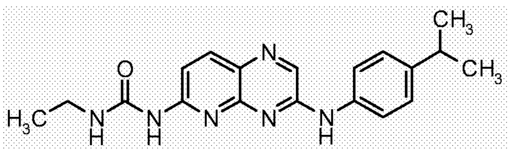

Verbindung 217 1-(4-Morpholin-4-yl-butyl)-3-(3-phenyl-pyrido[2,3-b]pyrazin-6-yl)-harnstoff

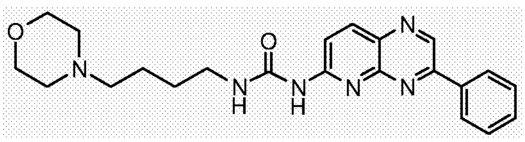

Verbindung 218 N-{4-[3-(3-Phenyl-pyrido[2,3-b]pyrazin-6-yl)-ureido]-butyl}-acetamid

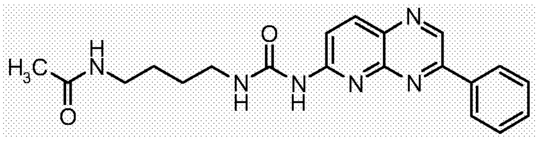

Verbindung 219 1-[3-(3-Amino-4-methyl-phenyl)-pyrido[2,3-b]pyrazin-6-yl]-3-ethyl-harnstoff

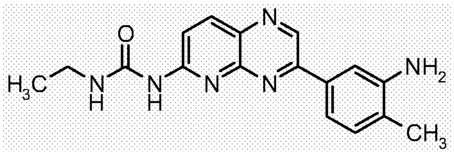

Verbindung 220 1-[3-(3-Acetyl-2-fluor-phenyl)-pyrido[2,3-b]pyrazin-6-yl]-3-ethyl-harnstoff

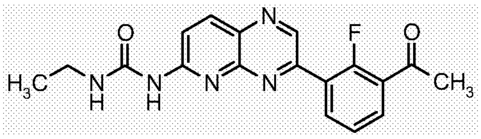

Verbindung 221 1-Ethyl-3-[3-(4-methoxy-3-methyl-phenyl)-pyrido[2,3-b]pyrazin-6-yl]-harnstoff

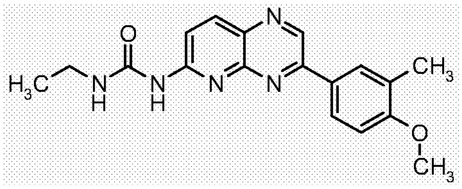

Verbindung 222 1-[3-(6-Ethoxy-pyridin-3-yl)-pyrido[2,3-b]pyrazin-6-yl]-3-ethyl-harnstoff

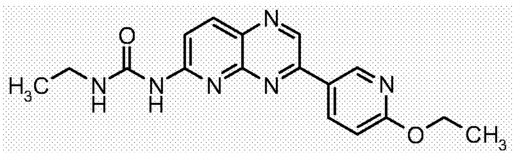

Verbindung 223 1-Ethyl-3-[3-(2-fluor-4-methyl-phenyl)-pyrido[2,3-b]pyrazin-6-yl]-harnstoff

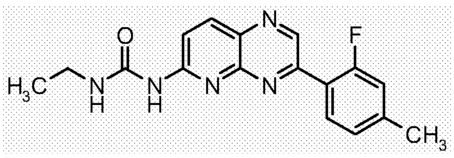

Verbindung 224 1-Ethyl-3-[3-(3-fluor-4-methoxy-phenyl)-pyrido[2,3-b]pyrazin-6-yl]-harnstoff

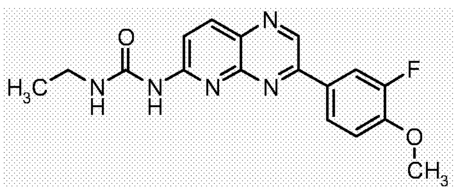

Verbindung 225 1-Ethyl-3-[3-(2-fluor-3-methoxy-phenyl)-pyrido[2,3-b]pyrazin-6-yl]-harnstoff

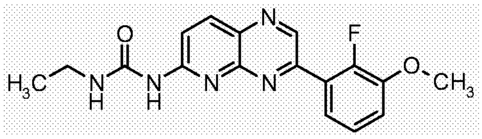

Verbindung 226 1-Ethyl-3-[3-(3,4,5-trimethoxy-phenyl)-pyrido[2,3-b]pyrazin-6-yl]-harnstoff

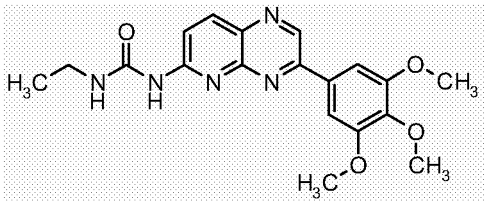

Verbindung 227 1-[3-(3,5-Difluor-2-methoxy-phenyl)-pyrido[2,3-b]pyrazin-6-yl]-3-ethyl-harnstoff

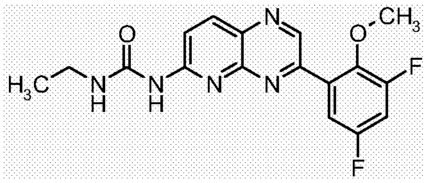

Verbindung 228 1-Ethyl-3-[3-(4-trifluormethyl-phenyl)-pyrido[2,3-b]pyrazin-6-yl]-harnstoff

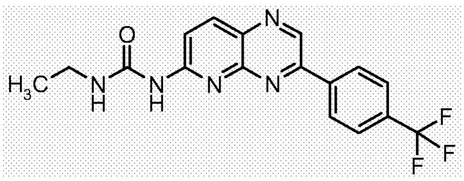

Verbindung 229 1-Ethyl-3-[3-(2,3,4-trimethoxy-phenyl)-pyrido[2,3-b]pyrazin-6-yl]-harnstoff

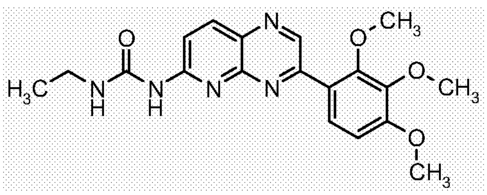

Verbindung 230 1-[3-(3-Chlor-4-methoxy-phenyl)-pyrido[2,3-b]pyrazin-6-yl]-3-ethyl-harnstoff

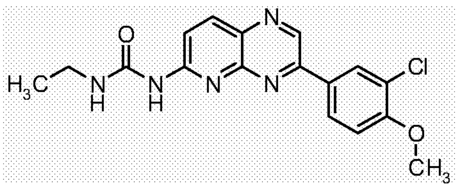

Verbindung 231 1-Ethyl-3-[3-(3-fluor-4-hydroxy-phenyl)-pyrido[2,3-b]pyrazin-6-yl]-harnstoff

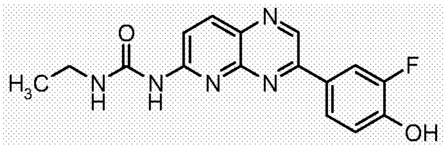

Verbindung 232 1-Ethyl-3-[3-(6-fluor-pyridin-3-yl)-pyrido[2,3-b]pyrazin-6-yl]-harnstoff

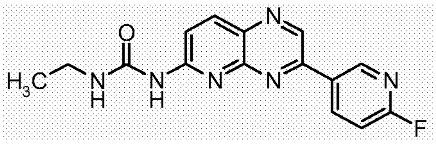

Verbindung 233 1-[3-(2,4-Dimethyl-thiazol-5-yl)-pyrido-[2,3-b]pyrazin-6-yl]-3-ethyl-harnstoff

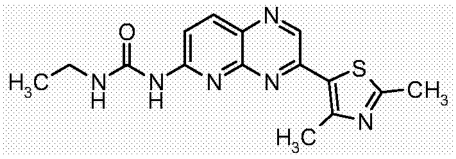

Verbindung 234 1-Ethyl-3-[3-(2-methoxy-phenyl)-pyrido[2,3-b]pyrazin-6-yl]-harnstoff

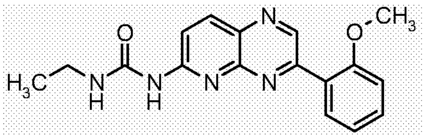

Verbindung 235 1-[3-(2-Chlor-pyridin-4-yl)-pyrido[2,3-b]pyrazin-6-yl]-3-ethyl-harnstoff

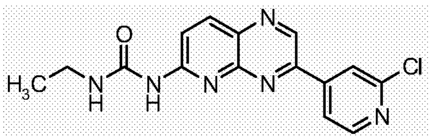

Verbindung 236 1-[3-(5-Acetyl-thiophen-2-yl)-pyrido[2,3-b]pyrazin-6-yl]-3-ethyl-harnstoff

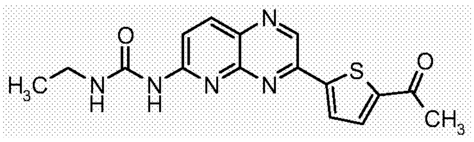

Verbindung 237 1-[3-(5-Chlor-thiophen-2-yl)-pyrido[2,3-b]pyrazin-6-yl]-3-ethyl-harnstoff

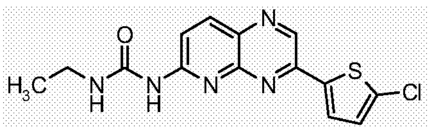

Verbindung 238 1-Ethyl-3-[3-(3-methoxy-phenyl)-pyrido[2,3-b]pyrazin-6-yl]-harnstoff

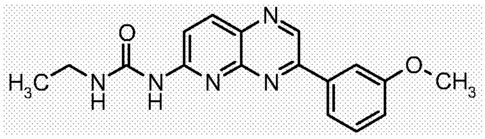

Verbindung 239 1-[3-(3-Brom-5-methoxy-phenyl)-pyrido[2,3-b]pyrazin-6-yl]-3-ethyl-harnstoff

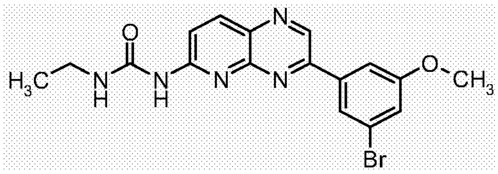

Verbindung 240 1-[3-(Benzothiazol-6-ylamino)-pyrido[2,3-b]pyrazin-6-yl]-3-ethyl-harnstoff

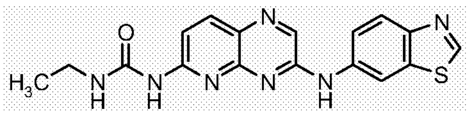

Verbindung 241 1-Ethyl-3-[3-(4-methyl-3-trifluoromethyl-phenylamino)-pyrido[2,3-b]pyrazin-6-yl]-harnstoff

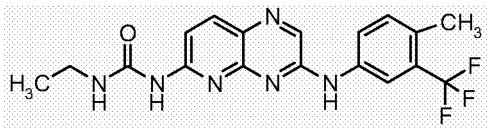

Verbindung 242 1-[3-(3-Cyano-4-methyl-phenylamino)-pyrido[2,3-b]pyrazin-6-yl]-3-ethyl-harnstoff

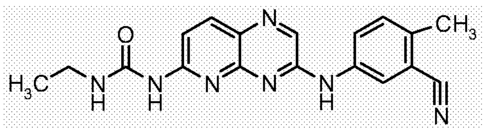

Verbindung 243 1-Ethyl-3-[3-(4-phenoxy-phenylamino)-pyrido[2,3-b]pyrazin-6-yl]-harnstoff

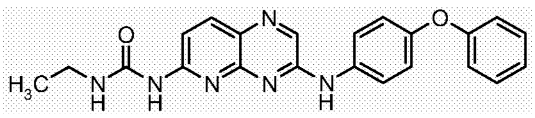

Verbindung 244 1-[3-(4-Chlor-3-methyl-phenylamino)-pyrido[2,3-b]pyrazin-6-yl]-3-ethyl-harnstoff

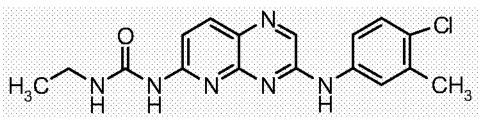

Verbindung 245 1-[3-(2-Chlor-4-methyl-phenylamino)-pyrido[2,3-b]pyrazin-6-yl]-3-ethyl-harnstoff

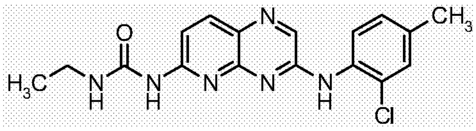

Verbindung 246 1-Ethyl-3-[3-(3-trifluoromethyl-phenylamino)-pyrido[2,3-b]pyrazin-6-yl]-harnstoff

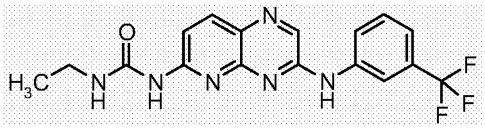

Verbindung 247 1-[3-(2-Chlor-4-trifluormethoxy-phenylamino)-pyrido[2,3-b]pyrazin-6-yl]-3-ethyl-harnstoff

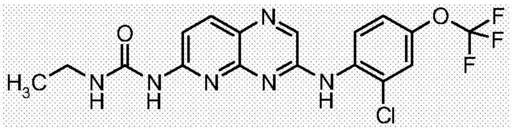

Verbindung 248 1-[3-(4-Chlor-2-methoxy-5-methyl-phenylamino)-pyrido[2,3-b]pyrazin-6-yl]-3-ethyl-harnstoff

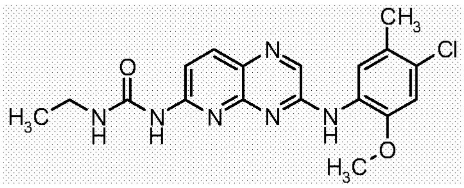

Verbindung 249 1-Ethyl-3-[3-(4-methylsulfanyl-phenyl)-pyrido[2,3-b]pyrazin-6-yl]-harnstoff

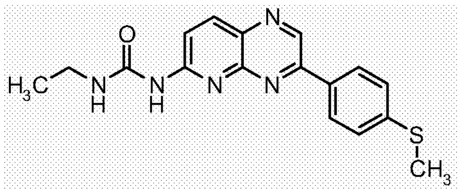

Verbindung 250 3-[6-(3-Ethyl-ureido)-pyrido[2,3-b]pyrazin-3-yl]-benzolsulfonamid

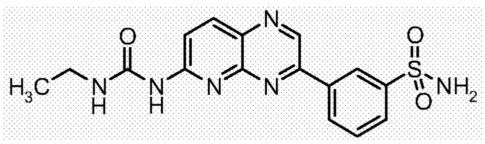

Verbindung 251 N-{4-[3-(3-Phenyl-pyrido[2,3-b]pyrazin-6-yl)-ureido]-butyl}-methansulfonamid

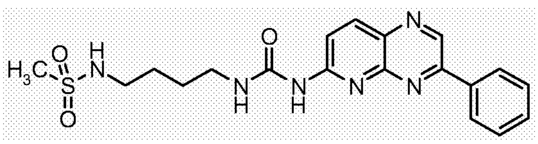

Verbindung 252 1-[3-(Benzo[1,3]dioxol-5-ylamino)-pyrido[2,3-b]pyrazin-6-yl]-3-ethyl-harnstoff

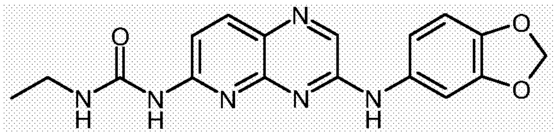

Verbindung 253 3-[6-(3-Ethyl-ureido)-pyrido[2,3-b]pyrazin-3-ylamino]-benzoesäure

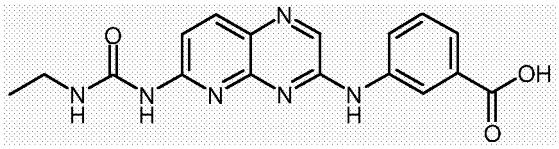

Verbindung 254 2-Chlor-4-[6-(3-ethyl-ureido)-pyrido[2,3-b]pyrazin-3-ylamino]-

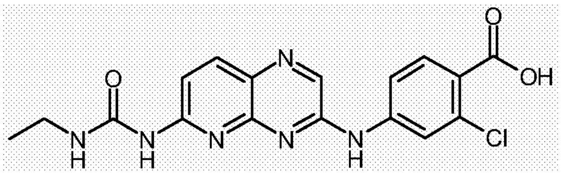

Verbindung 255 1-Ethyl-3-[3-(3-methoxy-5-trifluormethyl-phenylamino)-pyrido[2,3-b]pyrazin-6-yl]-harnstoff

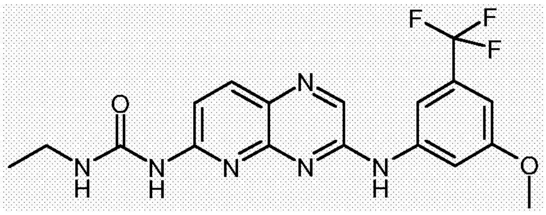

Verbindung 256 1-Ethyl-3-[3-(pyrimidin-2-ylamino)-pyrido[2,3-b]pyrazin-6-yl]-harnstoff

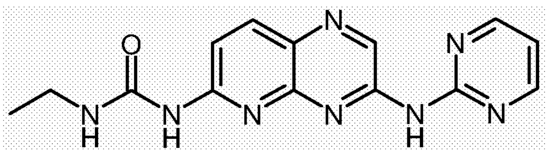

Verbindung 257 6-[6-(3-Ethyl-ureido)-pyrido[2,3-b]pyrazin-3-ylamino]-naphthalin-2-carbonsäure

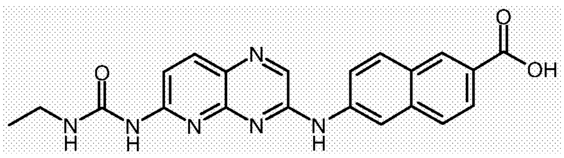

Verbindung 258 1-Ethyl-3-[3-(4-hydroxy-chinolin-2-ylamino)-pyrido[2,3-b]pyrazin-6-yl]-harnstoff

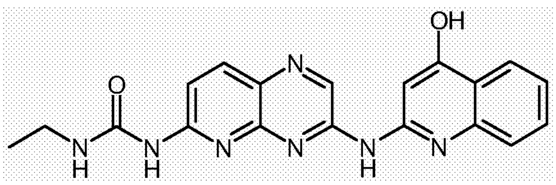

Verbindung 259 1-Ethyl-3-[3-(chinolin-6-ylamino)-pyrido[2,3-b]pyrazin-6-yl]-harnstoff

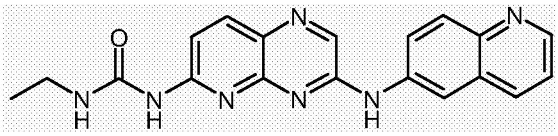

Verbindung 260 1-Ethyl-3-[3-(3,4,5-trimethoxy-phenylamino)-pyrido[2,3-b]pyrazin-6-yl]-harnstoff

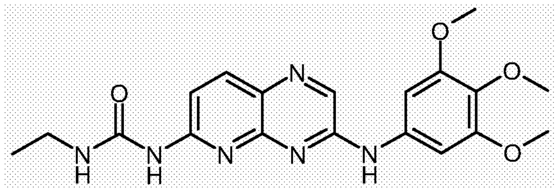

Verbindung 261 1-Ethyl-3-[3-(1H-indol-6-ylamino)-pyrido[2,3-b]pyrazin-6-yl]-harnstoff

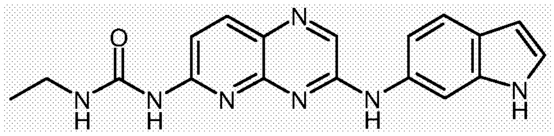

Verbindung 262 1-Ethyl-3-[3-(3-oxo-3,4-dihydro-2H-benzo[1,4]oxazin-7-ylamino)-pyrido[2,3-b]pyrazin-6-yl]-harnstoff

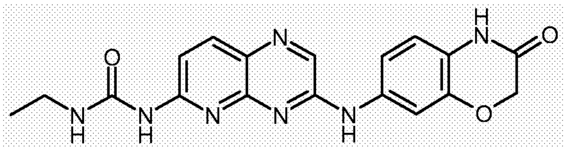

Verbindung 263 1-Ethyl-3-[3-(chinoxalin-6-ylamino)-pyrido[2,3-b]pyrazin-6-yl]-harnstoff

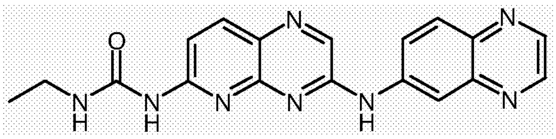

Verbindung 264 1-Ethyl-3-[3-(3-trifluormethoxy-phenylamino)-pyrido[2,3-b]pyrazin-6-yl]-harnstoff

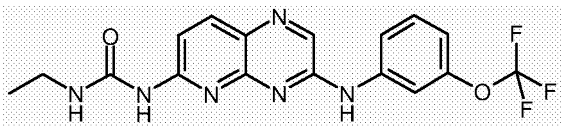

Verbindung 265 1-Ethyl-3-[3-(4-isopropoxy-phenylamino)-pyrido[2,3-b]pyrazin-6-yl]-harnstoff

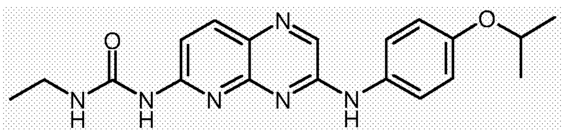

Verbindung 266 1-[3-(Dibenzofuran-2-ylamino)-pyrido[2,3-b]pyrazin-6-yl]-3-ethyl-harnstoff

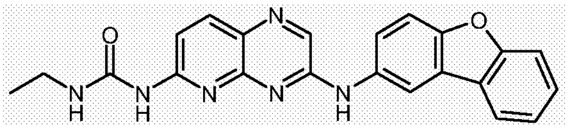

Verbindung 267 1-(6-Dimethylamino-hexyl)-3-[3-(4-hydroxy-3-methoxy-phenyl)-pyrido[2,3-b]pyrazin-6-yl]-harnstoff

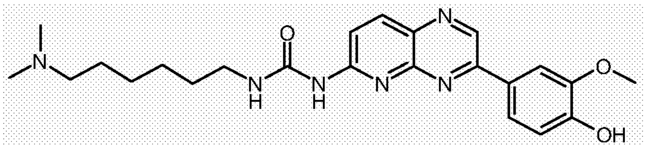

Verbindung 268 1-(4-Dimethylamino-butyl)-3-[3-(4-hydroxy-3-methoxy-phenyl)-pyrido[2,3-b]pyrazin-6-yl]-harnstoff

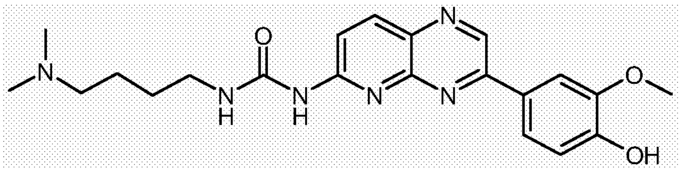

Verbindung 269 1-[3-(4-Hydroxy-3-methoxy-phenyl)-pyrido[2,3-b]pyrazin-6-yl]-3-(5-morpholin-4-yl-pentyl)-harnstoff

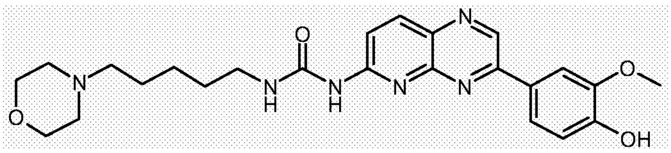

bereitgestellt werden, die zur Herstellung eines Arzneimittels zur Behandlung oder Prophylaxe von durch Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" vermittelte physiologischen und/oder pathophysiologischen Zuständen in Säugetieren verwendet werden können.

Um Unklarheiten zu vermeiden: wenn chemische Struktur und chemischer Name der oben dargestellten expliziten Verbindungen fehlerhafterweise nicht miteinander übereinstimmen, so soll die chemische Struktur die jeweilige explizite Verbindung eindeutig definieren.

Die oben dargestellten generischen Verbindungen der allgemeinen Formel (I) und bevorzugten Ausführungsformen sowie die explizit genannten Pyridopyrazin-Verbindungen 1 bis 269 werden im folgenden gemeinsam als "erfindungsgemäße Verbindungen" bezeichnet.

Die zur Erläuterung der erfindungsgemäßen Verbindungen angegebenen Ausdrücke und Begriffe haben grundsätzlich, sofern in der Beschreibung oder in den Ansprüchen nichts anderes angegeben ist, folgende Bedeutungen:

Der Ausdruck "Alkyl" umfasst im Sinne dieser Erfindung acyclische gesättigte oder ungesättigte Kohlenwasserstoffreste, die verzweigt oder geradkettig sein können, mit 1 bis 8 C-Atomen, d.h. $C_{1-8}$-Alkanyle, $C_{2-8}$-Alkenyle und $C_{2-8}$-Alkinyle. Dabei weisen Al-kenyle mindestens eine C-C-Doppelbindung und Alkinyle mindestens eine C-C-Dreifachbindung auf. Alkinyle können aber zusätzlich auch mindestens eine C-C-Doppelbindung aufweisen. Bevorzugte Alkyl-Reste sind Methyl, Ethyl, n-Propyl, 2-Propyl, n-Butyl, *sec*.-Butyl, *tert*.-Butyl, n-Pentyl, *iso*-Pentyl, neo-Pentyl, n-Hexyl, 2-Hexyl, n-Heptyl, n-Octyl, , n-Nonyl, n-Decyl, n-Undecyl, n-Dodecyl, Ethylenyl (Vinyl), Ethinyl, Propenyl ($-CH_2CH=CH_2$; $-CH=CH-CH_3$, $-C(=CH_2)-CH_3$), Propinyl ($-CH_2-C\equiv CH$, - $C\equiv C-CH_3$), Butenyl, Butinyl, Pentenyl, Pentinyl, Hexenyl, Hexinyl, Heptenyl, Heptinyl, Octenyl, Octadienyl und Octinyl.

Der Ausdruck "Cycloalkyl" bedeutet für die Zwecke dieser Erfindung cyclische, nicht-aromatische Kohlenwasserstoffe mit 1 bis 3 Ringen mit 3 bis 20, bevorzugt 3 bis 12 Kohlenstoffen, die gesättigt oder ungesättigt sein können,

besonders bevorzugt ($C_3$-$C_8$)-cycloalkyl. Der Cycloalkyl-Rest kann auch Teil eines bi- oder polycyclischen Systems sein, wo bspw. der Cycloalkyl-Rest mit einem Aryl-, Heteroaryl- oder Heterocyclyl-Rest wie hierin definiert durch jede(s) mögliche und gewünschte Ringmitglied(er) kondensiert ist. Die Bindung an die Verbindungen der allgemeinen Formel (I) kann über jedes beliebige und mögliche Ringglied des Cycloalkyl-Restes erfolgen. Bevorzugte Cycloalkyl-Reste sind Cyclopropyl, Cyclobutyl, Cyclopentyl, Cyclohexyl, Cycloheptyl, Cyclooctyl, Cyclodecyl, Cyclohexenyl, Cyclopentenyl und Cyclooctadienyl.

Der Ausdruck "Heterocyclyl" steht für einen 3- bis 14-gliedrigen, bevorzugt 3-, 4-, 5-, 6-, 7- oder 8-gliedrigen cyclischen organischen Rest, der mindestens 1, ggf. 2, 3, 4 oder 5 Heteroatome enthält, insbesondere Stickstoff, Sauerstoff und/oder Schwefel, wobei die Heteroatome gleich oder verschieden sind und der cyclische Rest gesättigt oder ungesättigt, aber nicht aromatisch ist. Der Heterocyclyl-Rest kann auch Teil eines bi- oder polycyclischen Systems sein, wo bspw. der Heterocyclyl-Rest mit einem Aryl-, Heteroaryl- oder Cycloalkyl-Rest wie hierin definiert durch jede(s) mögliche und gewünschte Ringmitglied(er) kondensiert ist. Die Bindung an die Verbindungen der allgemeinen Formel (I) kann über jedes beliebige und mögliche Ringglied des Heterocyclyl-Restes erfolgen. Bevorzugte Heterocyclyl-Reste sind Tetrahydrofuryl, Pyrrolidinyl, Imidazolidinyl, Thiazolidinyl, Tetrahydropyranyl, Piperidinyl, Piperazinyl, Morpholinyl, Thiapyrrolidinyl, Oxapiperazinyl, Oxapiperidinyl und Oxadiazolyl.

Der Ausdruck "Aryl" bedeutet im Sinne dieser Erfindung aromatische Kohlenwasserstoffe mit 3 bis 14 C-Atomen, bevorzugt 5 bis 14 C-Atomen, besonders bevorzugt 6 bis 14 C-Atomen. Der Aryl-Rest kann auch Teil eines bi- oder polycyclischen Systems sein, wo bspw. der Aryl-Rest mit einem Heterocyclyl-, Heteroaryl- oder Cycloalkyl-Rest wie hierin definiert durch jede(s) mögliche und gewünschte Ringmitglied(er) kondensiert ist, bspw. mit Tetrahydrofuran, Tetrahydrothiophen, Pyrrolidin, Imidazolidin, Thiazolidin, Tetrahydropyran, Dihydropyran, Piperidin, Furan, Thiophen, Imidazol, Thiazol, Oxazol, Isoxazol. Die Bindung an die Verbindungen der allgemeinen Formel (I) kann über jedes beliebige und mögliche Ringglied des Aryl-Restes erfolgen. Bevorzugte Aryl-Reste sind Phenyl, Biphenyl, Naphthyl und Anthracenyl, aber ebenso Indanyl, Indenyl oder 1,2,3,4-Tetrahydronaphthyl.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome enthält, insbesondere Stickstoff, Sauerstoff und/oder Schwefel, wobei die Heteroatome gleich oder verschieden sind. Die Anzahl der Stickstoffatome ist bevorzugt 0 bis 3, die der Sauerstoff und Schwefelatome bevorzugt 0 oder 1. Der Heteroaryl-Rest kann auch Teil eines bi- oder polycyclischen Systems sein, wo bspw. der Heteroaryl-Rest mit einem Heterocyclyl-, Aryl- oder Cycloalkyl-Rest wie hierin definiert durch jede(s) mögliche und gewünschte Ringmitglied(er) kondensiert ist. Die Bindung an die Verbindungen der allgemeinen Formel (I) kann über jedes beliebige und mögliche Ringglied des HeteroarylRestes erfolgen. Bevorzugte Heteroaryl-Reste sind Pyrrolyl, Furyl, Thienyl, Thiazolyl, Isothiazolyl, Oxazolyl, Oxadiazolyl, Isoxazolyl, Pyrazolyl, Imidazolyl, Triazol, Tetrazol, Pyridinyl, Pyrimidinyl, Pyridazinyl, Pyrazinyl, Triazin, Phthalazinyl, Indolyl, Indazolyl, Indolizinyl, Chinolinyl, Isochinolinyl, Chinoxalinyl, Chinazolinyl, Pteridinyl, Carbazolyl, Phenazinyl, Phenoxazinyl, Phenothiazinyl, und Acridinyl.

Die Ausdrücke "Alkyl-Cycloalkyl", "Cycloalkylalkyl", "Alkyl-Heterocyclyl", "Heterocyclylalkyl", "Alkyl-Aryl", "Arylalkyl", "Alkyl-Heteroaryl" und "Heteroarylalkyl" bedeuten für die Zwecke der vorliegenden Erfindung, daß Alkyl, Cycloalkyl, Heterocyclyl, Aryl und Heteroaryl die oben definierten Bedeutungen haben und der Cycloalkyl-, Heterocyclyl-, Aryl- und Heteroaryl-Rest über einen Alkyl-Rest, bevorzugt $C_1$-$C_8$-Alkyl-Rest, besonders bevorzugt $C_1$-$C_4$-Alkyl-Rest, an die Verbindungen der allgemeinen Formel (I) gebunden ist.

Im Zusammenhang mit "Alkyl", "Cycloalkyl", "Heterocyclyl", "Aryl", "Heteroaryl", "Alkyl-Cycloalkyl", "Alkyl-Heterocyclyl", "Alkyl-Aryl" und "Alkyl-Heteroaryl" versteht man unter dem Begriff substituiert im Sinne dieser Erfindung, insofern oben in der Beschreibung oder den Ansprüchen nicht explicit definiert, die Substitution eines oder mehrerer Wasserstoffreste durch F, Cl, Br, I, CN, $CF_3$, $NH_2$, NH-Alkyl, NH-Aryl, $N(Alkyl)_2$, $NO0_2$, SH, S-Alkyl, OH, $OCF_3$, O-Alkyl, O-Aryl, $OSO_3H$, $OP(O)(OH)_2$, CHO, $CO_2H$, $SO_3H$ oder Alkyl. Die Substituenten können gleich oder verschieden sein und die Substitution kann in jeder beliebigen und möglichen Position des Alkyl-, Cycloalkyl-, Heterocyclyl-, Aryl- und Heteroarylrestes vorkommen.

Der Ausdruck "Halogen" umfasst im Sinne dieser Erfindung die Halogenatome Fluor, Chlor, Brom und lod.

Unter mehrfach substituierten Resten sind solche zu verstehen, die entweder an verschiedenen oder an gleichen Atomen mehrfach, z. B. zwei- oder dreifach substituiert sind, beispielsweise dreifach am gleichen C-Atom wie im Falle von $CF_3$, $-CH_2CF_3$ oder an verschiedenen Stellen wie im Falle von $-CH(OH)-CH=CH-CHCl_2$. Die Mehrfachsubstitution kann mit dem gleichen oder verschiedenen Substituenten erfolgen.

Sofern die erfindungsgemäßen Verbindungen mindestens ein Asymmetriezentrum aufweisen, können sie in Form ihrer Racemate, in Form der reinen Enantiomeren und/oder Diastereomeren oder in Form von Mischungen dieser Enantiomeren und/oder Diastereomeren vorliegen. Die Mischungen können in jedem beliebigen Mischungsverhältnis der Stereoisomeren vorliegen.

So lassen sich beispielsweise die erfindungsgemäßen Verbindungen, welche ein oder mehrere Chiralitätszentren aufweisen und die als Racemate auftreten, nach an sich bekannten Methoden in ihre optischen Isomeren, also Enantiomere oder Diastereomere auftrennen. Die Trennung kann durch Säulentrennung an chiralen Phasen oder durch Umkristallisation aus einem optisch aktiven Lösungsmittel oder unter Verwendung einer optisch aktiven Säure oder

Base oder durch Derivatisierung mit einem optisch aktiven Reagenz, wie beispielsweise einem optisch aktiven Alkohol, und anschließender Abspaltung des Restes erfolgen.

Die erfindungsgemäßen Verbindungen können in Form ihrer Doppelbindungsisomere als "reine" E- oder Z-Isomere oder in Form von Mischungen dieser Doppelbindungsisomere vorliegen.

Sofern möglich, können die erfindungsgemäßen Verbindungen in Form der Tautomeren vorliegen.

Die erfindungsgemäßen Verbindungen können, falls sie eine ausreichend basische Gruppe, wie zum Beispiel ein primäres, sekundäres oder tertiäres Amin besitzen, mit anorganischen und organischen Säuren in ihre physiologisch verträglichen Salze überführt werden. Vorzugsweise werden die pharmazeutisch annehmbaren Salze der erfindungsgemäßen Verbindungen mit Salzsäure, Bromwasserstoffsäure, Schwefelsäure, Phosphorsäure, Methansulfonsäure, p-Toluolsulfonsäure, Kohlensäure, Ameisensäure, Essigsäure, Trifluoressigsäure, Sulfoessigsäure, Oxalsäure, Malonsäure, Maleinsäure, Bernsteinsäure, Weinsäure, Traubensäure, Äpfelsäure, Embonsäure, Mandelsäure, Fumarsäure, Milchsäure, Citronensäure, Glutaminsäure oder Asparaginsäure gebildet. Bei den gebildeten Salzen handelt es sich u.a. um Hydrochloride, Hydrobromide, Sulfate, Hydrogensulfate, Phosphate, Methansulfonate, Tosylate, Carbonate, Hydrogencarbonate, Formiate, Acetate, Triflate, Sulfoacetate, Oxalate, Malonate, Maleate, Succinate, Tartrate, Malate, Embonate, Mandelate, Fumarate, Lactate, Citrate, Glutaminate und Aspartate. Die Stöchiometrie der gebildeten Salze der erfindungsgemäßen Verbindungen kann dabei ganzzahlige oder nicht ganzzahlige Vielfache von eins betragen.

Die erfindungsgemäßen Verbindungen können, falls sie eine ausreichend saure Gruppe, wie zum Beispiel die Carboxygruppe enthalten, mit anorganischen und organischen Basen in ihre physiologisch verträglichen Salze überführt werden. Als anorganische Basen kommen beispielsweise Natriumhydroxid, Kaliumhydroxid, Calciumhydroxid, als organische Basen Ethanolamin, Diethanolamin, Triethanolamin, Cyclohexylamin, Dibenzylethylendiamin und Lysin in Betracht. Die Stöchiometrie der gebildeten Salze der erfindungsgemäßen Verbindungen kann dabei ganzzahlige oder nicht ganzzahlige Vielfache von eins betragen.

Ebenfalls bevorzugt sind Solvate und insbesondere Hydrate der erfindungsgemäßen Verbindungen, die z. B. durch Kristallisation aus einem Lösungsmittel oder aus wässriger Lösung erhalten werden können. Es können sich dabei ein, zwei, drei oder beliebig viele Solvat- oder Wasser-Moleküle mit den erfindungsgemäßen Verbindungen zu Solvaten und Hydraten verbinden.

Es ist bekannt, dass chemische Substanzen Festkörper ausbilden, die in verschiedenen Ordnungszuständen vorliegen, die man als polymorphe Formen oder Modifikationen bezeichnet. Die verschiedenen Modifikationen einer polymorphen Substanz können sich in ihren physikalischen Eigenschaften stark unterscheiden. Die erfindungsgemäßen Verbindungen können in verschiedenen polymorphen Formen vorliegen, dabei können bestimmte Modifikationen metastabil sein.

Ebenfalls können die erfindungsgemäßen Verbindungen in Form beliebiger Prodrugs wie beispielsweise Ester, Carbonate, Carbamate, Harnstoffe, Amide oder Phosphate vorkommen, bei denen die tatsächlich biologisch aktive Form erst durch Verstoffwechselung freigesetzt wird.

Es ist ferner bekannt, dass chemische Substanzen im Körper zu Metaboliten umgewandelt werden die gegebenenfalls ebenfalls die erwünschte biologische Wirkung - unter Umständen sogar in stärker ausgeprägter Form - hervorrufen können.

Entsprechende Prodrugs und Metaboliten der erfindungsgemäßen Verbindungen sind als zur Erfindung gehörig anzusehen.

Es wurde nun überraschend und vorteilhaft festgestellt, dass die erfindungsgemäßen Verbindungen auch gleichzeitig auf zwei oder mehrere Signaltransduktionswege bzw. Enzyme solcher Wege einwirken bzw. modulierend oder hemmend wirken können. Dabei hat sich herausgestellt, dass die erfindungsgemäßen Verbindungen mit hoher Selektivität einwirken bzw. modulierend oder hemmend wirken.

Eine solche gleichzeitige, bspw. duale, Modulation oder Inhibition von zwei oder mehreren Signaltransduktionswegen, z.B. ras-Raf-Mek-Erk-Signaltweg, PI3K-Akt-Signalweg und/oder SAPK-Signalweg, spezieller Erk1/Erk2 und/oder PI3K und/oder Jnk und/oder p38, ist von Vorteil gegenüber der nur einfachen Modulation oder Inhibition eines Signaltransduktionsweges, da synergistische therapeutische Effekte bewirkt werden können, wie bspw. eine verstärkerte Apoptose und schnellere und effizientere Tumorregression.

Die überraschenden vorteilhaften Wirkungen der erfindungsgemäßen Verbindungen ermöglichen die Verfolgung multipler Therapieansätze in den physiologischen und/oder pathophysiologischen Zuständen oder Krankheitsbildern, die sensitiv sind für die Behandlung oder Modulation von bzw. vermittelt werden durch zwei oder mehrere Signaltransduktionswege.

Ferner wurde überraschend und vorteilhaft festgestellt, dass die erfindungsgemäßen Verbindungen auch mit hoher Selektivität einwirken bzw. modulierend oder hemmend wirken können auf den PI3K-Akt-Signaltransduktionsweg bzw. Enzyme dessen und dass die oben dargestellten multiplen Wirkmechansimen und Therapieansätze auch mit diesem Signalweg bzw. Enzymen Anwendung finden können.

Weiterhin wurde überraschend und vorteilhaft festgestellt, dass die erfindungsgemäßen Verbindungen auch mit hoher Selektivität einwirken bzw. modulierend oder hemmend wirken können auf den SAPK-Signaltransduktionsweg

bzw. Enzyme dessen und dass die oben dargestellten multiplen Wirkmechansimen und Therapieansätze auch mit diesem Signalwegn bzw. Enzymen Anwendung finden können.

Desweiteren wurde überraschend und vorteilhaft festgestellt, dass die erfindungsgemäßen Verbindungen auch mit hoher Selektivität einwirken bzw. modulierend oder hemmend wirken können auf Enzyme, wie ATM, ATR, mTOR, DNA-PK und/oder hSMG-1, und dass die oben dargestellten multiplen Wirkmechansimen und Therapieansätze auch mit diesen Enzymen Anwendung finden können.

Unter dem Begriff "Modulation" wird erfindungsgemäß folgendes verstanden: "Aktivierung, partielle Aktivierung, Inhibierung, partielle Inhibierung". Hierbei liegt es im Fachwissen des durchschnittlichen Fachmanns, eine solche Aktivierung, partielle Aktivierung, Inhibierung oder partielle Inhibierung mittels der üblichen Mess- und Bestimmungsverfahren zu messen und zu bestimmen. So kann eine partielle Aktivierung bspw. in Relation zu einer vollständigen Aktivierung gemessen und bestimmt werden; ebenso eine partielle Inhibierung in Relation zu einer vollständigen Inhibierung.

Unter den Begriffen "Inhibierung, Inhibition und/oder Hemmung" wird erfindungsgemäß folgendes verstanden: "partielle oder vollständige Inhibierung, Inhibition und/oder Hemmung". Hierbei liegt es im Fachwissen des durchschnittlichen Fachmanns, eine solche partielle oder vollständige Inhibierung, Inhibition und/oder Hemmung mittels der üblichen Mess- und Bestimmungsverfahren zu messen und zu bestimmen. So kann eine partielle Inhibierung, Inhibition und/oder Hemmung bspw. in Relation zu einer vollständigen Inhibierung, Inhibition und/oder Hemmung gemessen und bestimmt werden.

Die Begriffe "Modulation" und "Inhibierung, Inhibition und/oder Hemmung" beziehen sich im Zusammenhang mit "Enzymen" und/oder "Kinasen" im Rahmen dieser Erfindung sowohl auf die inaktive Form (enzymatisch inaktiv) und/oder aktive Form (enzymatisch aktiv) des jeweiligen Enzyms und/oder Kinase. Dies bedeutet im Rahmen dieser Erfindung, dass eine erfindungsgemäße Verbindung ihre modulierende Wirkung an der inaktiven Form, aktiven Form oder beiden Formen des Enzyms und/oder Kinase entfalten kann.

Die erfinderische Aufgabe wurde in einem weiteren Aspekt überraschender Weise dadurch gelöst, dass die erfindungsgemäßen Verbindungen bereitgestellt werden, die die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation des oder der Signaltransduktionswege ausgewählt aus der Gruppe bestehend aus: "ras-Raf-Mek-Erk-Signaltransduktionsweg, PI3K-Akt-Signaltransduktionsweg und/oder SAPK-Signaltransduktionsweg" bewirkt wird.

In einem weiteren Aspekt wurde die erfinderische Aufgabe überraschender Weise dadurch gelöst, dass die erfindungsgemäßen Verbindungen bereitgestellt werden, die zur Herstellung eines Arzneimittels zur Behandlung oder Prophylaxe von durch Enzyme ausgewählt aus der Gruppe bestehend aus: "ATM, ATR, mTOR, DNA-PK, hSMG-1" vermittelte physiologischen und/oder pathophysiologischen Zuständen in Säugetieren verwendet werden können.

In einem weiteren Aspekt wurde die erfinderische Aufgabe überraschender Weise dadurch gelöst, dass die erfindungsgemäßen Verbindungen bereitgestellt werden, die die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation eines oder mehrerer Enzyme ausgewählt aus der Gruppe bestehend aus: "ATM, ATR, mTOR, DNA-PK, hSMG-1" bewirkt wird.

In einer bevorzugten Ausführungsform werden die erfindungsgemäßen Verbindungen bereitgestellt zur Verwendung zur Herstellung eines Arzneimittels zur Behandlung und/oder Prophylaxe von durch den ras-Raf-Mek-Erk-Signaltransduktionsweg und den PI3K-Akt-Signaltransduktionsweg vermittelten physiologischen und/oder pathophysiologischen Zuständen in Säugetieren und/oder zur Herstellung eines Arzneimittels zur Behandlung oder Prophylaxe von physiologischen und/oder pathophysiologischen Zuständen in Säugetieren, wobei die Behandlung oder Prophylaxe durch Modulation des ras-Raf-Mek-Erk-Signaltransduktionswegs und des PI3K-Akt-Signaltransduktionswegs bewirkt wird.

In einem weiteren Aspekt wurde die erfinderische Aufgabe überraschender Weise dadurch gelöst, dass die erfindungsgemäßen Verbindungen bereitgestellt werden, die zur Herstellung eines Arzneimittels zur Behandlung oder Prophylaxe von durch den PI3K-Akt-Signaltransduktionsweg vermittelte physiologischen und/oder pathophysiologischen Zuständen in Säugetieren verwendet werden können.

In einem weiteren Aspekt wurde die erfinderische Aufgabe überraschender Weise dadurch gelöst, dass die erfindungsgemäßen Verbindungen bereitgestellt werden, die die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation des PI3K-Akt-Signaltransduktionswegs bewirkt wird.

In einer bevorzugten Ausführungsform werden die erfindungsgemäßen Verbindungen bereitgestellt zur Verwendung zur Herstellung eines Arzneimittels zur Behandlung und/oder Prophylaxe von durch den SAPK-Signaltransduktionsweg und den PI3K-Akt-Signaltransduktionsweg vermittelten physiologischen und/oder pathophysiologischen Zuständen in Säugetieren und/oder zur Herstellung eines Arzneimittels zur Behandlung oder Prophylaxe von physiologischen und/oder pathophysiologischen Zuständen in Säugetieren, wobei die Behandlung oder Prophylaxe durch Modulation des SAPK-Signaltransduktionswegs und des PI3K-Akt-Signaltransduktionswegs bewirkt wird.

In einem weiteren Aspekt wurde die erfinderische Aufgabe überraschender Weise dadurch gelöst, dass die erfindungsgemäßen Verbindungen bereitgestellt werden, die zur Herstellung eines Arzneimittels zur Behandlung oder Prophylaxe von durch den SAPK-Signaltransduktionsweg vermittelte physiologischen und/oder pathophysiologischen Zuständen in Säugetieren verwendet werden können.

In einem weiteren Aspekt wurde die erfinderische Aufgabe überraschender Weise dadurch gelöst, dass die erfindungsgemäßen Verbindungen bereitgestellt werden, die die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation des SAPK-Signaltransduktionswegs bewirkt wird.

In einer bevorzugten Ausführungsform werden die erfindungsgemäßen Verbindungen bereitgestellt zu den oben dargestellten Verwendungen, wobei die Modulation des ras-Raf-Mek-Erk-Signaltransduktionsweges bewirkt wird durch Modulation eines oder mehrerer Enzyme ausgewählt aus der Gruppe bestehend aus: "Tyrosinkinase, Serin/Threoninkinase, Rezeptor-Tyrosinkinase, cytoplasmatische Tyrosinkinase, cytoplasmatische Serin/Threoninkinase" und bevorzugt ausgewählt aus der Gruppe bestehend aus: "Erk, Erk1, Erk2".

In einer weiteren bevorzugten Ausführungsform werden die erfindungsgemäßen Verbindungen bereitgestellt zu den oben dargestellten Verwendungen, wobei die Modulation des PI3K-Akt-Signaltransduktionsweges bewirkt wird durch Modulation eines oder mehrerer Enzyme ausgewählt aus der Gruppe bestehend aus: "Lipidkinasen" und bevorzugt ausgewählt aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

In einer weiteren bevorzugten Ausführungsform werden die erfindungsgemäßen Verbindungen bereitgestellt zu den oben dargestellten Verwendungen, wobei die Modulation des SAPK-Signaltransduktionsweges bewirkt wird durch Modulation eines oder mehrerer Enzyme ausgewählt aus der Gruppe bestehend aus: "Tyrosinkinase, Serin/Threoninkinase, Rezeptor-Tyrosinkinase, cytoplasmatische Tyrosinkinase, cytoplasmatische Serin/Threoninkinase"und bevorzugt ausgewählt aus der Gruppe bestehend aus: "Jnk, Jnk1, Jnk2, Jnk3, p38, p38alpha, p38beta, p38gamma, p38delta".

In einem weiteren Aspekt wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, die zur Herstellung eines Arzneimittels zur Behandlung oder Prophylaxe von physiologischen und/oder pathophysiologischen Zuständen in Säugetieren verwendet werden können, wobei die Behandlung oder Prophylaxe durch Modulation von zwei oder mehrerer Enzyme bewirkt wird.

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "Erk, Erk1, Erk2" und mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "Jnk, Jnk1, Jnk2, Jnk3, p38, p38alpha, p38beta, p38gamma, p38delta" und mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "Erk, Erk1, Erk2" und mindestens ein Enzym ausgewählt ist aus der Gruppe bestehend aus: "ATM, ATR, mTOR, DNA-PK, hSMG-1".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "Jnk, Jnk1, Jnk2, Jnk3, p38, p38alpha, p38beta, p38gamma, p38delta" und mindestens ein Enzym ausgewählt ist aus der Gruppe bestehend aus: "ATM, ATR, mTOR, DNA-PK, hSMG-1".

In einer weiter bevorzugten Ausführungsform werden die erfindungsgemäßen Verbindungen bereitgestellt zu den oben dargestellten Verwendungen, wobei bei der durch Modulation von zwei oder mehrerer Enzyme bewirkten Behandlung oder Prophylaxe mindestens ein Enzym ausgewählt ist aus der Gruppe bestehend aus: "PI3K, PI3Kalpha, PI3Kbeta, PI3Kgamma, PI3Kdelta, PI3K-C2alpha, PI3K-C2beta, PI3K-Vps34p" und mindestens ein Enzym ausgewählt ist aus der Gruppe bestehend aus: "ATM, ATR, mTOR, DNA-PK, hSMG-1".

In einer ferner bevorzugten Ausführungsform werden die erfindungsgemäßen Verbindungen bereitgestellt zu den oben dargestellten Verwendungen, wobei die Modulation eine Inhibition ist.

Die erfindungsgemäßen Verbindungen können im Rahmen dieser Erfindung allen bekannten Säugetieren, insbesondere dem Menschen, zur Behandlung und/oder Prophylaxe verabreicht werden.

In einer anderen bevorzugten Ausführungsform werden die erfindungsgemäßen Verbindungen bereitgestellt

zu den oben dargestellten Verwendungen, wobei das Säugetier ausgewählt ist aus der Gruppe bestehend aus: "Mensch, Nutztier, Vieh, Haustier, Rind, Kuh, Schaf, Schwein, Ziege, Pferd, Pony, Esel, Maulesel, Maultier, Hase, Kaninchen, Katze, Hund, Meerschweinchen, Hamster, Ratte, Maus" und bevorzugt ein Mensch ist.

Die erfindungsgemäßen Verbindungen können im Rahmen dieser Erfindung zur Behandlung und/oder Prophylaxe aller bekannten physiologischen und/oder pathophysiologischen Zustände verwendet warden.

In einer bevorzugten Ausführungsform werden die erfindungsgemäßen Verbindungen bereitgestellt zu den oben dargestellten Verwendungen, wobei die physiologischen und/oder pathophysiologischen Zustände ausgewählt sind aus der Gruppe bestehend aus: "maligne Tumore, benigne Tumore, entzündliche Erkrankungen, Entzündungen, Schmerzen, rheumatische Erkrankungen, arthritische Erkrankungen, HIV Infektionen, neurologische oder neurodegenerative Erkrankungen, Rheuma, Arthritis, AIDS, ARC (AIDS related complex), Kaposi-Sarkom, Tumore ausgehend vom Hirn und/oder Nervensystem und/oder Hirnhäuten, Demenz, Alzheimer, hyperproliferative Erkrankungen, Psoriasis, Endometriose, Narbenbildung, benigne Prostatahyperpläsie (BPH), Erkrankungen des Immunsystems, Autoimmunerkrankungen, Immundefizienzerkrankungen, Kolontumor, Magentumor, Darmtumor, Lungentumor, Pankreastumor, Ovarialtumor, Prostatatumor, Leukämie, Melanom, Lebertumor, Nierentumor, Kopftumor, Halstumor, Gliom, Brust-Tumor, Gebärmutterkrebs, Endometriumkrebs, Gebärmutterhalskrebs, Hirntumor, Adenokanthom, Blasenkrebs, Magentumor, Kolorectalrumor, Speiseröhrenkrebs, gynokologischer Tumor, Ovartumor, Schilddrüsenkrebs, Lymphom, chronische Leukämie, akute Leukämie, Restenose, Diabetis, diabetische Nephropathie, fibrotische Erkrankungen, cystische Fibrose, maligne Nephrosklerose, thrombotische Microangiopathie Syndrom, Organtransplantat-Abstoßung, Glomerulpathien, Erkrankungen des Stoffwechsels, solide/feste Tumore, rheumatische Arthritis, diabetische Retinopathie, Asthma, Allergien, allergische Erkrankungen, chronische obstruktive pulmonare Erkrankungen, inflammatorische Bowel-Erkrankung, Fibrose, Atheriosklerose, Herzerkrankungen, cardiovaskuläre Erkrankungen, Erkrankungen des Herzmuskels, vaskuläre Erkrankungen, angiogenetische Erkrankungen, Nierenerkrankungen, Rhinitis, Grave Erkrankung, fokale Ischemie, Herzversagen, Ischemie, kardische Hypertrophie, Nierenversagen, kardische Myozyten Fehlfunktion, Bluthochdruck, Vasoconstriktion, Schlaganfall, anaphylaktischer Schock, Blutplättchen-Verklumpung, Skelettmuskelatrophie, Fettleibigkeit, Übergewicht, Glukose Homeostase, kongestive Herzinsuffizienz, Angina, Herzanfall, Herzinfarkt, Hyperglykämie, Hypoglykämie, Hypertension".

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels zur Behandlung oder Prophylaxe von physiologischen und/oder pathophysiologischen Zuständen in Säugetieren, wobei das Arzneimittel mindestens eine weitere pharmakologisch aktive Substanz umfasst.

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels zur Behandlung oder Prophylaxe von physiologischen und/oder pathophysiologischen Zuständen in Säugetieren, wobei das Arzneimittel vor und/oder während und/oder nach der Behandlung mit mindestens einer weiteren pharmakologisch aktiven Substanz verabreicht wird.

In einem weiteren Aspekt der vorliegenden Erfindung wurde die erfinderische Aufgabe überraschender Weise dadurch gelöst, dass die erfindungsgemäßen Verbindungen gemäß der oben dargestellten Aspekte, bevorzugten Ausführungsformen und Verwendungen bereitgestellt werden, zur Verwendung zur Herstellung eines Arzneimittels zur Behandlung oder Prophylaxe von physiologischen und/oder pathophysiologischen Zuständen in Säugetieren, wobei das Arzneimittel vor und/oder während und/oder nach der Behandlung Strahlentherapie und/oder Chirurgie verabreicht wird.

Die erfindungsgemäßen Verbindungen können dabei Rahmen dieser Erfindung mit allen bekannten pharmakologisch aktiven Substanzen in einer Kombinationstherapie wie dargestellt verabreicht werden.

In einer bevorzugten Ausführungsform werden die erfindungsgemäßen Verbindungen bereitgestellt zu den oben dargestellten Verwendungen, wobei die weitere pharmakologisch aktive Substanz ausgewählt ist aus der Gruppe bestehend aus: "DNA Topoisomerase I und/oder II Inhibitoren, DNA Interkalatoren, alkylierende Agentien, Microtubuli Destabilisatoren, Hormon- und/oder Wachstumsfaktor-Rezeptor-Agonisten und/oder -Antagonisten, Antikörper gegen Wachstumsfaktoren und deren Rezeptoren, Kinase Inhibitoren, Antimetabolite".

In einer bevorzugten Ausführungsform werden die erfindungsgemäßen Verbindungen bereitgestellt zu den oben dargestellten Verwendungen, wobei die weitere pharmakologisch aktive Substanz ausgewählt ist aus der Gruppe bestehend aus: "Asparaginase, Bleomycin, Carboplatin, Carmustin, Chlorambucil, Cisplatin, Colaspase, Cyclophosphamid, Cytarabin, Dacarbazin, Dactinomycin, Daunorubicin, Doxorubicin(Adriamycin), Epirubicin, Etoposide, 5-Fluorouracil, Hexamethylmelamin, Hydroxharnstoff, Ifosfamid, Irinotecan, Leucovorin, Lomustin, Mechlorethamin,6-Mercaptopurin, Mesna, Methotrexat, Mitomycin C, Mitoxantrone, Prednisolon, Prednison, Procarbazin, Raloxifen, Streptozocin, Tamoxifen, Thioguanin, Topotecan, Vinblastin, Vincristin, Vindesin, Aminoglutethimid, L-Asparaginase, Azathioprin, 5-Azacytidin cladribin, Busulfan, Diethylstilbestrol, 2', 2'-Difluorodeoxycytidin, Docetaxel, Erythrohydroxynonyladenin, Ethinylestradiol, 5-Fluorodeoxyuridin, 5- Fluorodeoxyuridin Monophosphat, Fludarabin Phosphate, Fluoxymesteron, Flutamid, Hydroxyprogesteron Caproat, Idarubicin, Interferon, Medroxyprogesteron Acetat, Megestrol Acetat, Melphalan, Mitotan, Paclitaxel, Oxaliplatin, Pentostatin, N-Phosphonoacetyl-L-aspartat (PA-LA), Plicamycin, Semustin, Teniposide, Testosteron Propionat, Thiotepa, Trimethylmelamin, Uridine, Vinorelbin, Epothilon, Gemcitabin, Taxotere, BCNU, CCNU, DTIC, 5-Fluorouarcil, Herceptin, Avastin, Erbitux, Sorafenib, Gleevec, Iressa, Tarceva, Rapamycin, Actinomycin D".

In einem weiteren Aspekt der vorliegenden Erfindung wurde die erfinderische Aufgabe überraschender Weise gelöst durch die Bereitstellung der Pyridopyrazine ausgewählt aus der Gruppe bestehend aus:

Verbindung 38 1-[3-(4-Hydroxy-phenyl)-pyrido[2,3-b]pyrazin-6-yl]-3-methyl-harnstoff

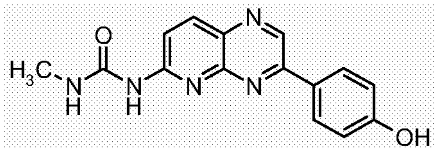

Verbindung 39 1-Allyl-3-[3-(4-phenoxy-phenyl)-pyrido[2,3-b]pyrazin-6-yl]-harnstoff

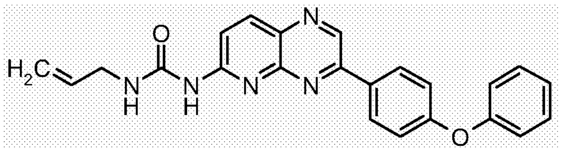

Verbindung 40 Methansulfonsäure-4-[6-(3-allyl-ureido)-pyrido[2,3-b]pyrazin-3-yl]-phenyl-ester

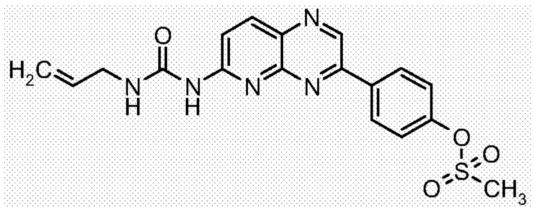

Verbindung 41 4-Dimethylamino-benzoesäure-4-[6-(3-allyl-ureido)-pyrido[2,3-b]pyrazin-3-yl]-phenyl-ester

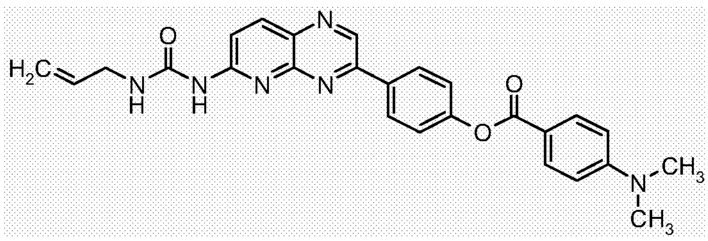

Verbindung 42 Essigsäure-4-[6-(3-allyl-ureido)-pyrido[2,3-b]pyrazin-3-yl]-phenyl-ester

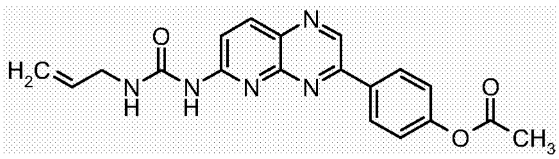

Verbindung 43 1-Ethyl-3-[3-(4-hydroxy-phenyl)-pyrido[2,3-b]pyrazin-6-yl]-harnstoff

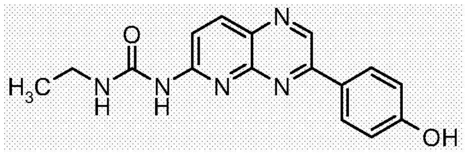

Verbindung 44 1-[3-(4-Amino-phenyl)-pyrido[2,3-b]pyrazin-6-yl]-3-methyl-thioharnstoff

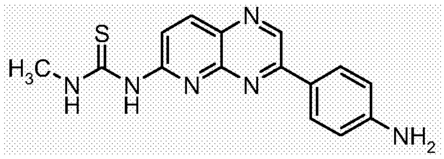

Verbindung 45 1-Ethyl-3-(3-phenyl-pyrido[2,3-b]pyrazin-6-yl)-harnstoff

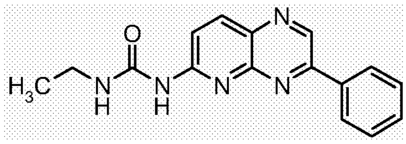

Verbindung 46 1-Acetyl-1-ethyl-3-(3-phenyl-pyrido[2,3-b]pyrazin-6-yl)-harnstoff

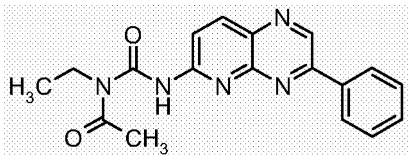

Verbindung 47 1-Allyl-3-[3-(4-fluoro-phenyl)-pyrido[2,3-b]pyrazin-6-yl]-harnstoff

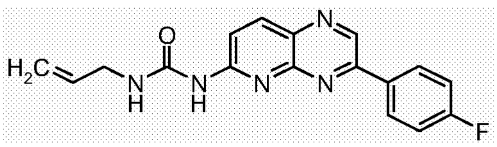

Verbindung 48 1,1-Diethyl-3-(3-phenyl-pyrido[2,3-b]pyrazin-6-yl)-harnstoff

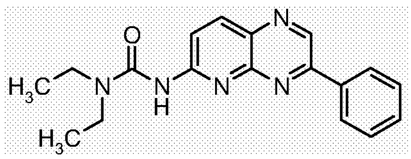

Verbindung 49 1-(2-Chlor-ethyl)-3-[3-(4-fluor-phenyl)-pyrido[2,3-b]pyrazin-6-yl]-harnstoff

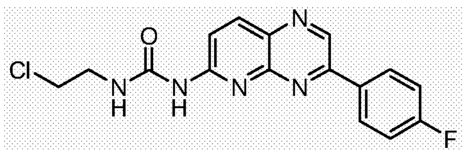

Verbindung 50 1-Ethyl-3-[3-(4-methoxy-phenyl)-pyrido[2,3-b]pyrazin-6-yl]-harnstoff

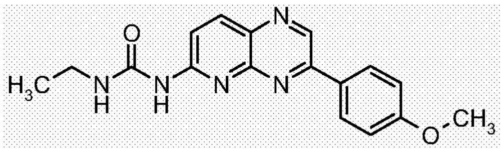

Verbindung 51 1-(3-Phenyl-pyrido[2,3-b]pyrazin-6-yl)-3-propyl-harnstoff

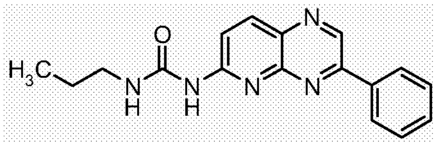

Verbindung 52 [3-(3-Phenyl-pyrido[2,3-b]pyrazin-6-yl)-ureido]-essigsäure-ethyl-ester

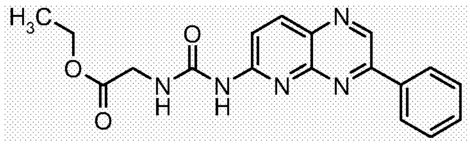

Verbindung 53 1-(3-Chlor-pyrido[2,3-b]pyrazin-6-yl)-3-ethyl-harnstoff

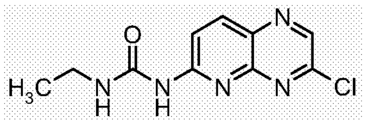

Verbindung 54 1-Ethyl-3-[3-(4-fluor-phenyl)-pyrido[2,3-b]pyrazin-6-yl]-harnstoff

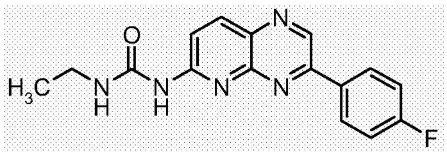

Verbindung 55 1-[3-(3-Chlor-4-fluor-phenyl)-pyrido[2,3-b]pyrazin-6-yl]-3-ethyl-harnstoff

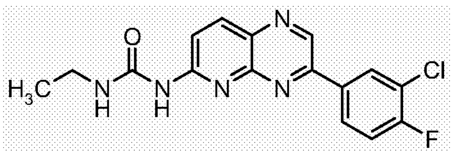

Verbindung 56 4-[6-(3-Ethyl-ureido)-pyrido[2,3-b]pyrazin-3-yl]-benzoesäure

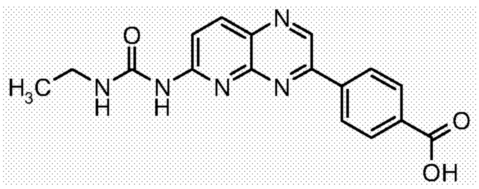

Verbindung 57 N-{4-[6-(3-Ethyl-ureido)-pyrido[2,3-b]pyrazin-3-yl]-phenyl}-acetamide

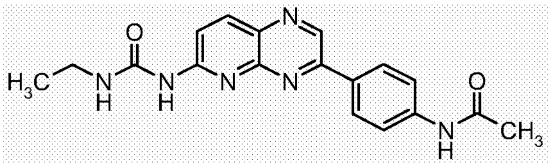

Verbindung 58 1-[3-(2,4-Difluor-phenyl)-pyrido[2,3-b]pyrazin-6-yl]-3-ethyl-harnstoff

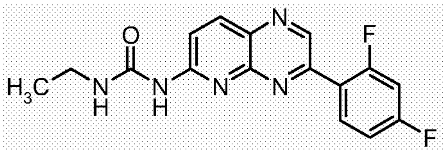

Verbindung 59 1-Ethyl-3-(3-morpholin-4-yl-pyrido[2,3-b]pyrazin-6-yl)-harnstoff

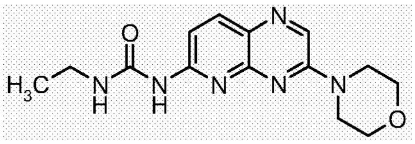

Verbindung 60 1-Ethyl-3-[3-(4-methyl-piperazin-1-yl)-pyrido[2,3-b]pyrazin-6-yl]-harnstoff

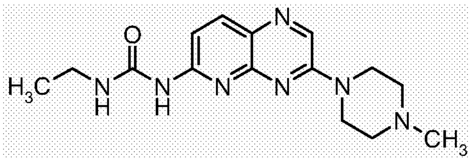

Verbindung 61 1-Ethyl-3-[3-(2-hydroxy-phenyl)-pyrido[2,3-b]pyrazin-6-yl]-harnstoff

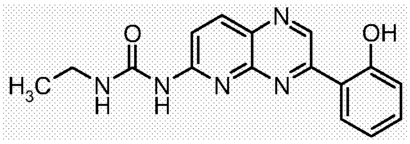

Verbindung 62 1-Ethyl-3-[3-(2-methoxy-ethylamino)-pyrido[2,3-b]pyrazin-6-yl]-harnstoff

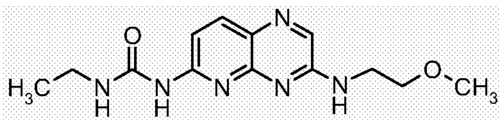

Verbindung 63 1-[3-(4-Chlor-3-trifluormethyl-phenyl)-pyrido[2,3-b]pyrazin-6-yl]-3-ethyl-harnstoff

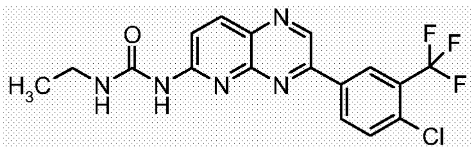

Verbindung 64 1-Ethyl-3-(3-phenoxy-pyrido[2,3-b]pyrazin-6-yl)-harnstoff

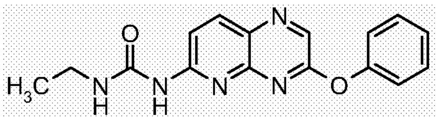

Verbindung 65 1-[3-(Cyclopropylmethyl-amino)-pyrido[2,3-b]pyrazin-6-yl]-3-ethyl-harnstoff

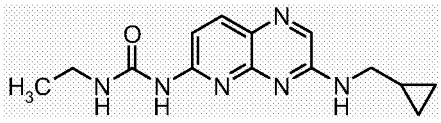

Verbindung 66 1-Ethyl-3-{3-[(pyridin-4-ylmethyl)-amino]-pyrido[2,3-b]pyrazin-6-yl}-harnstoff

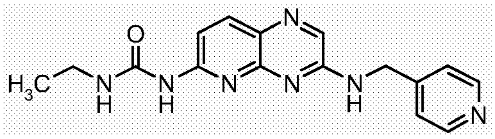

Verbindung 67 1-Ethyl-3-[3-(4-fluor-benzyloxy)-pyrido[2,3-b]pyrazin-6-yl]-harnstoff

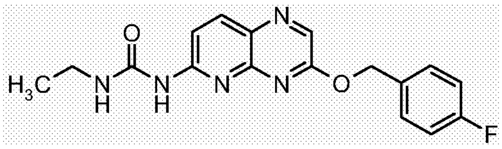

Verbindung 68 1-Ethyl-3-(3-pyridin-4-yl-pyrido[2,3-b]pyrazin-6-yl)-harnstoff

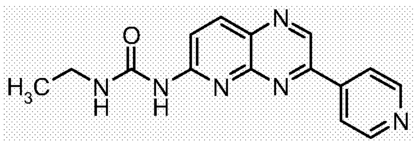

Verbindung 69 1-Ethyl-3-[3-(pyridin-3-yloxy)-pyrido[2,3-b]pyrazin-6-yl]-harnstoff

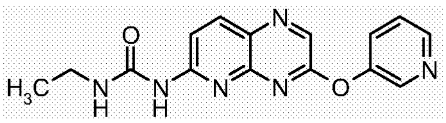

Verbindung 70 1-Ethyl-3-[3-(tetrahydro-furan-2-ylmethoxy)-pyrido[2,3-b]pyrazin-6-yl]-harnstoff

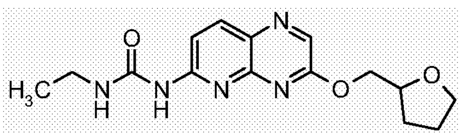

Verbindung 71 1-Ethyl-3-[3-(4-morpholin-4-yl-phenyl)-pyrido[2,3-b]pyrazin-6-yl]-harnstoff

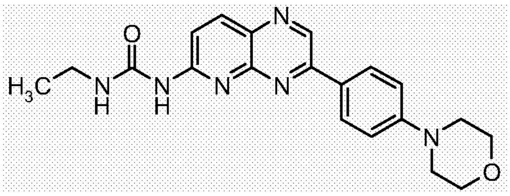

Verbindung 72 1-Ethyl-3-(3-hydroxy-pyrido[2,3-b]pyrazin-6-yl)-harnstoff

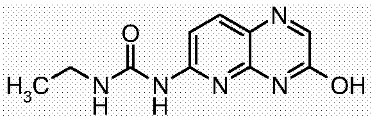

Verbindung 73 1-Ethyl-3-[3-(3-methoxy-phenylsulfanyl)-pyrido[2,3-b]pyrazin-6-yl]-harnstoff

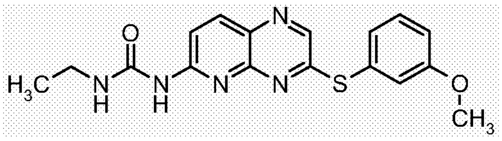

Verbindung 74 1-Ethyl-3-(3-quinolin-3-yl-pyrido[2,3-b]pyrazin-6-yl)-harnstoff

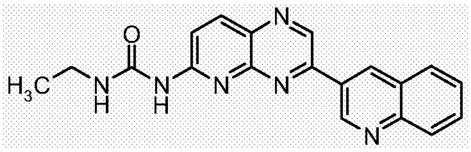

Verbindung 75 1-(3-Benzo[b]thiophen-3-yl-pyrido[2,3-b]pyrazin-6-yl)-3-ethyl-harnstoff

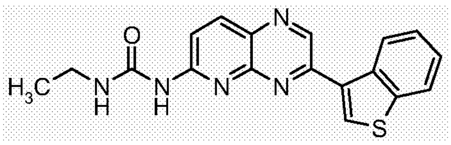

Verbindung 76 1-Ethyl-3-[3-(pyridin-2-ylsulfanyl)-pyrido[2,3-b]pyrazin-6-yl]-harnstoff

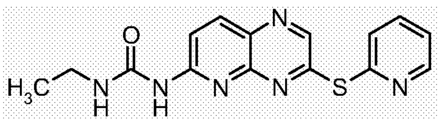

Verbindung 77 1-[3-(4-Dimethylamino-phenyl)-pyrido[2,3-b]pyrazin-6-yl]-3-ethyl-harnstoff

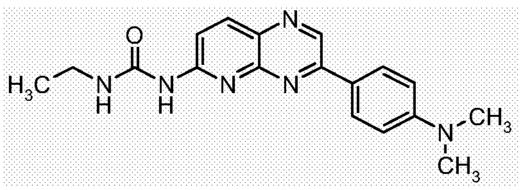

Verbindung 78 N-{4-[6-(3-Ethyl-ureido)-pyrido[2,3-b]pyrazin-3-yl]-phenyl}-methansulfonamid

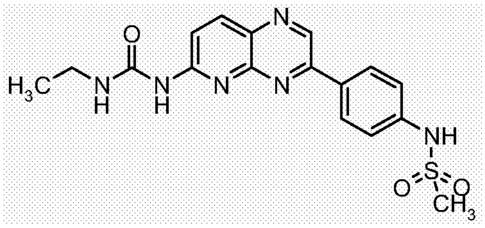

Verbindung 79 1-Ethyl-3-[3-(1H-pyrazol-4-yl)-pyrido[2,3-b]pyrazin-6-yl]-harnstoff

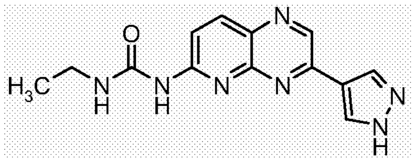

Verbindung 80 1-(3-Benzylsulfanyl-pyrido[2,3-b]pyrazin-6-yl)-3-ethyl-harnstoff

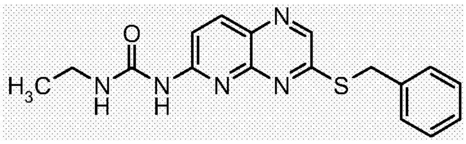

Verbindung 81 1-Ethyl-3-[3-(4-methyl-[1,4]diazepan-1-yl)-pyrido[2,3-b]pyrazin-6-yl]-harnstoff

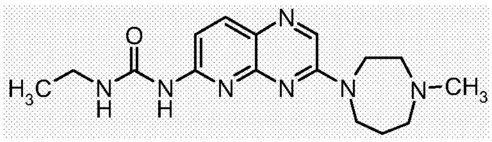

Verbindung 82 1-[3-(4-Amino-phenyl)-pyrido[2,3-b]pyrazin-6-yl]-3-ethyl-harnstoff

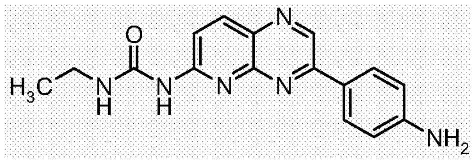

Verbindung 83 1-(3-Amino-pyrido[2,3-b]pyrazin-6-yl)-3-ethyl-harnstoff

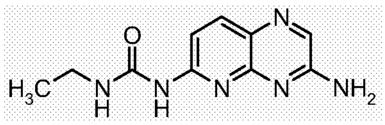

Verbindung 84 1-Ethyl-3-pyrido[2,3-b]pyrazin-6-yl-harnstoff

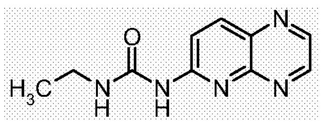

Verbindung 85 1-Ethyl-3-(3-imidazol-1-yl-pyrido[2,3-b]pyrazin-6-yl)-harnstoff

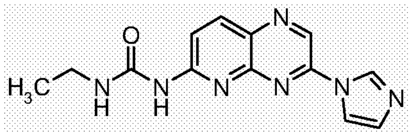

Verbindung 86 1-Ethyl-3-[3-(4-fluor-2-hydroxy-phenyl)-pyrido[2,3-b]pyrazin-6-yl]-harnstoff

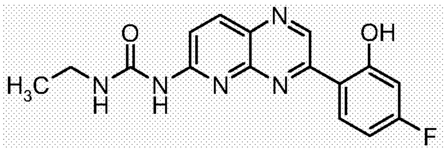

Verbindung 87 1-(3-Cyclopentyloxy-pyrido[2,3-b]pyrazin-6-yl)-3-ethyl-harnstoff

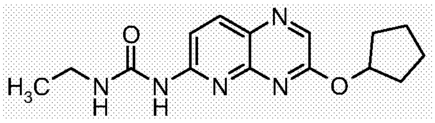

Verbindung 88 1-Ethyl-3-[3-(4-hydroxy-piperidin-1-yl)-pyrido[2,3-b]pyrazin-6-yl]-harnstoff

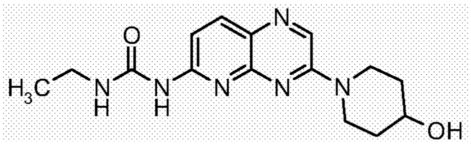

Verbindung 89 (3-Phenyl-pyrido[2,3-b]pyrazin-6-yl)-harnstoff

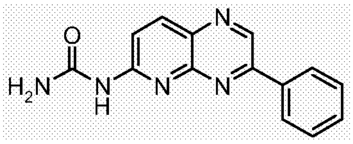

Verbindung 90 1-Ethyl-3-(3-pyrimidin-5-yl-pyrido[2,3-b]pyrazin-6-yl)-harnstoff

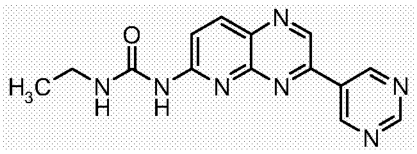

Verbindung 91 1-Ethyl-3-(3-pyridin-3-yl-pyrido[2,3-b]pyrazin-6-yl)-harnstoff

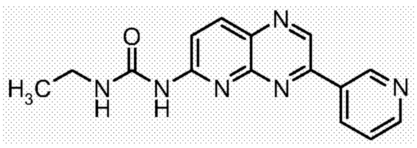

Verbindung 92 1-Allyl-3-(3-phenyl-pyrido[2,3-b]pyrazin-6-yl)-harnstoff

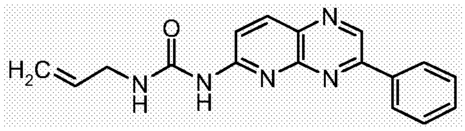

Verbindung 93 1-Ethyl-3-(3-piperazin-1-yl-pyrido[2,3-b]pyrazin-6-yl)-harnstoff

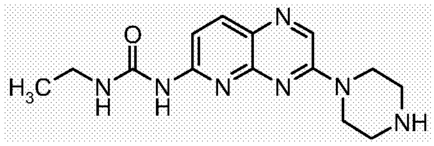

Verbindung 94 1-[3-(3-Chlor-pyridin-4-ylmethyl)-pyrido[2,3-b]pyrazin-6-yl]-3-ethyl-harnstoff

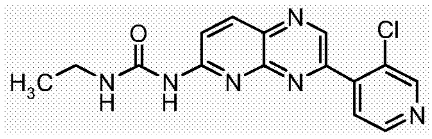

Verbindung 95 1-Ethyl-3-[3-(6-methoxy-pyridin-3-yl)-pyrido[2,3-b]pyrazin-6-yl]-harnstoff

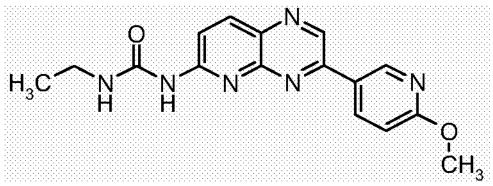

Verbindung 96 1-[3-(3,5-Dimethyl-isoxazol-4-yl)-pyrido[2,3-b]pyrazin-6-yl]-3-ethyl-harnstoff

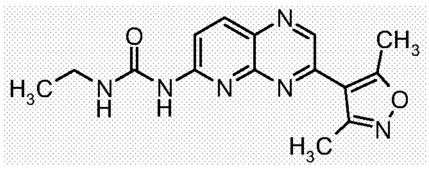

Verbindung 97 1-Ethyl-3-[3-(4-trifluormethoxy-phenyl)-pyrido[2,3-b]pyrazin-6-yl]-harnstoff

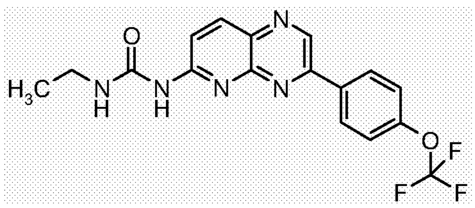

Verbindung 98 1-Ethyl-3-(3-furan-2-yl-pyrido[2,3-b]pyrazin-6-yl)-harnstoff

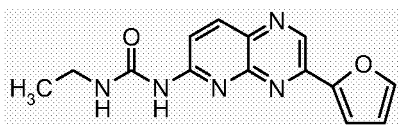

Verbindung 99 1-Ethyl-3-[3-(2-methoxy-pyridin-3-yl)-pyrido[2,3-b]pyrazin-6-yl]-harnstoff

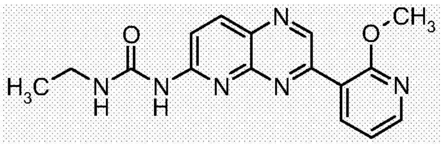

Verbindung 100 1-[3-(2,4-Dimethoxy-phenyl)-pyrido[2,3-b]pyrazin-6-yl]-3-ethyl-harnstoff

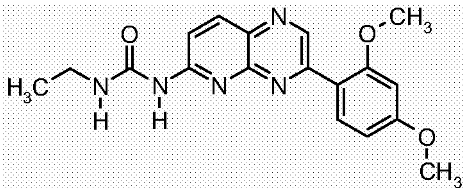

Verbindung 101 1-Ethyl-3-[3-(1H-pyrrol-2-yl)-pyrido[2,3-b]pyrazin-6-yl]-harnstoff

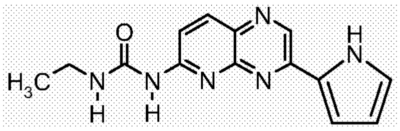

Verbindung 102 1-Ethyl-3-[3-(6-morpholin-4-yl-pyridin-3-yl)-pyrido[2,3-b]pyrazin-6-yl]-harnstoff

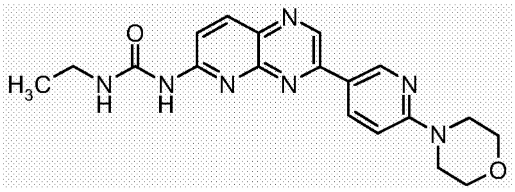

Verbindung 103 1-Benzyl-3-ethyl-1-(3-phenyl-pyrido[2,3-b]pyrazin-6-yl)-harnstoff

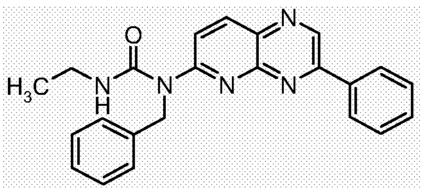

Verbindung 104 1-[3-(2-Amino-phenyl)-pyrido[2,3-b]pyrazin-6-yl]-3-ethyl-harnstoff

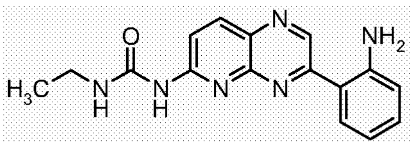

Verbindung 105 1-Ethyl-3-[3-(4-hydroxymethyl-phenyl)-pyrido[2,3-b]pyrazin-6-yl]-harnstoff

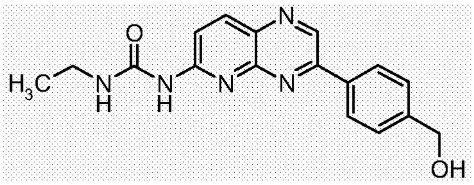

Verbindung 106 1-[3-(3-Amino-phenyl)-pyrido[2,3-b]pyrazin-6-yl]-3-ethyl-harnstoff

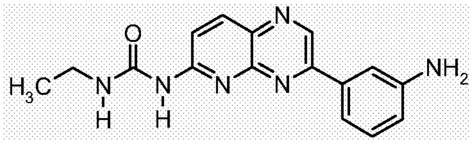

Verbindung 107 1-[3-(4-Acetyl-phenyl)-pyrido[2,3-b]pyrazin-6-yl]-3-ethyl-harnstoff

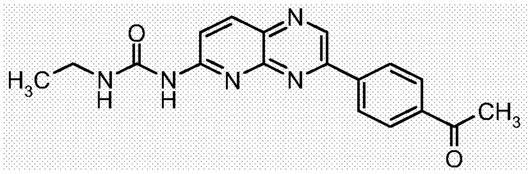

Verbindung 108 1-[3-(2,3-Dihydro-benzofuran-5-yl)-pyrido[2,3-b]pyrazin-6-yl]-3-ethyl-harnstoff

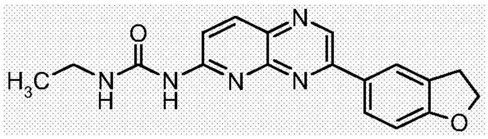

Verbindung 109 1-[3-(4-Benzyloxy-3-fluor-phenyl)-pyrido[2,3-b]pyrazin-6-yl]-3-ethyl-harnstoff

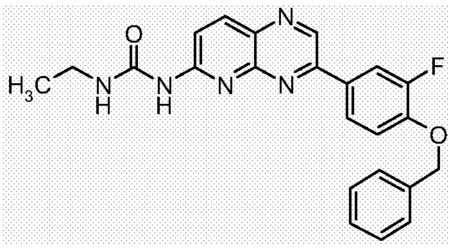

Verbindung 110 1-(2,3-Dihydroxy-propyl)-3-(3-phenyl-pyrido[2,3-b]pyrazin-6-yl)-harnstoff

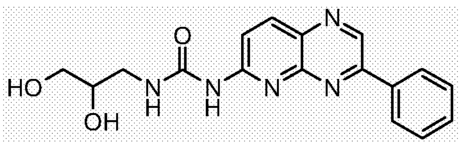

Verbindung 111 1-Ethyl-3-[3-(3-formyl-4-methoxy-phenyl)-pyrido[2,3-b]pyrazin-6-yl]-harnstoff

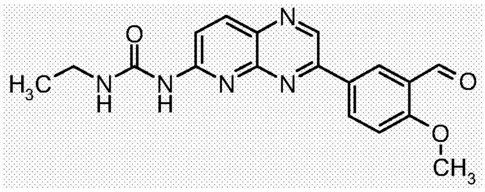

Verbindung 112 1-Ethyl-3-[3-(4-methansulfonyl-phenyl)-pyrido[2,3-b]pyrazin-6-yl]-harnstoff

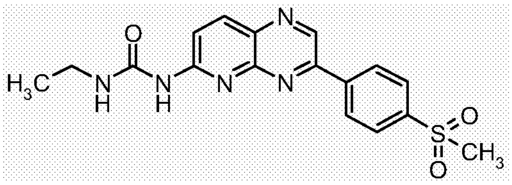

Verbindung 113 N-{4-[6-(3-Ethyl-ureido)-pyrido[2,3-b]pyrazin-3-yl]-phenyl}-succinamidsäure

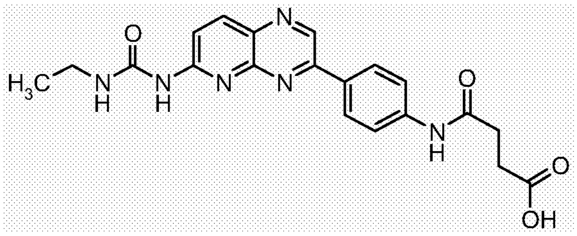

Verbindung 114 1-Ethyl-3-(3-pyridin-4-yl-pyrido[2,3-b]pyrazin-6-yl)-harnstoff Methansulfonsäuresalz (freie Base)

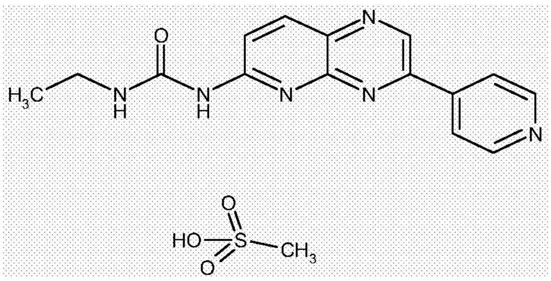

Verbindung 115 1-[3-(2,6-Dimethoxy-pyridin-3-yl)-pyrido[2,3-b]pyrazin-6-yl]-3-ethyl-harnstoff

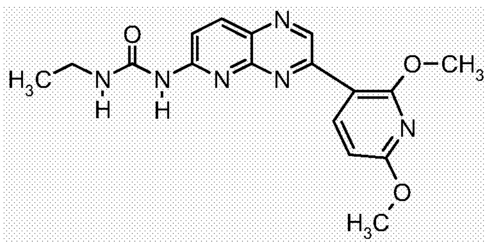

Verbindung 116 1-[3-(2,6-Dimethoxy-pyrimidin-4-yl)-pyrido[2,3-b]pyrazin-6-yl]-3-ethyl-harnstoff

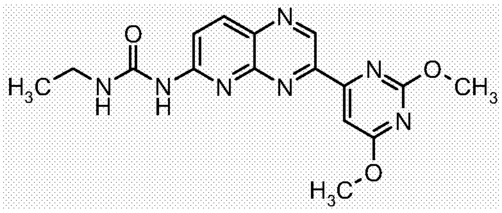

Verbindung 117 1-[3-(2,4-Dioxo-1,2,3,4-tetrahydro-pyrimidin-5-yl)-pyrido[2,3-b]pyrazin-6-yl]-3-ethyl-harnstoff

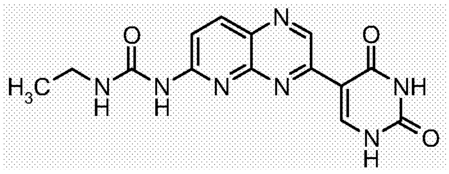

Verbindung 118 1-Ethyl-3-[3-(1H-indol-5-yl)-pyrido[2,3-b]pyrazin-6-yl]-harnstoff

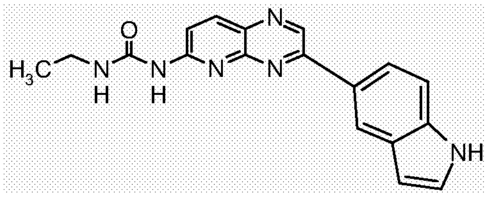

Verbindung 119 1-(3-Chloro-pyrido[2,3-b]pyrazin-6-yl)-3-ethyl-thioharnstoff

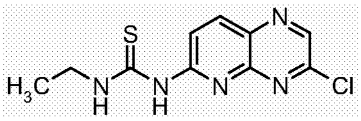

Verbindung 120 1-Ethyl-3-{3-[4-(2-methoxy-ethoxy)-phenyl]-pyrido[2,3-b]pyrazin-6-yl}-harnstoff

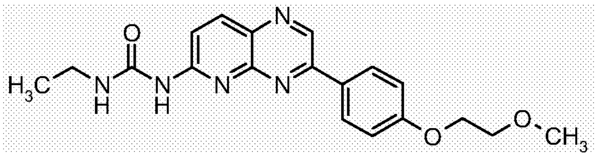

Verbindung 121 Acrylsäure-4-[6-(3-ethyl-ureido)-pyrido[2,3-b]-pyrazin-3-yl]-phenyl-ester

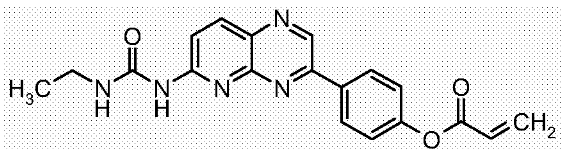

Verbindung 122 1-[3-(4-Cyano-phenyl)-pyrido[2,3-b]pyrazin-6-yl]-3-ethyl-harnstoff

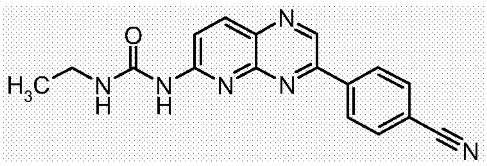

Verbindung 123 1-(3-Benzo[1,2,5]oxadiazol-4-yl-pyrido[2,3-b]pyrazin-6-yl)-3-ethyl-harnstoff

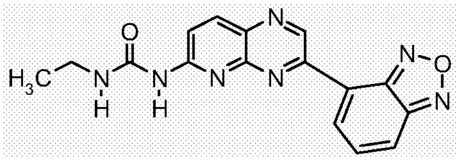

Verbindung 124 1-Ethyl-3-[3-(4-hydroxy-3-methoxy-phenyl)-pyrido[2,3-b]pyrazin-6-yl]-harnstoff

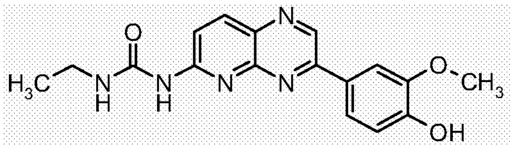

Verbindung 125 1-[3-(2,6-Dimethoxy-phenyl)-pyrido [2,3-b]pyrazin-6-yl]-3-ethyl-harnstoff

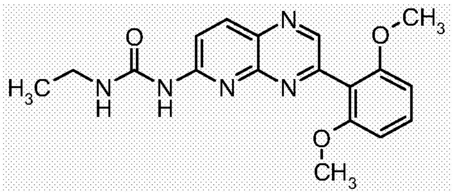

Verbindung 126 1-[3-(3-Acetyl-phenyl)-pyrido[2,3-b]pyrazin-6-yl]-3-ethyl-harnstoff

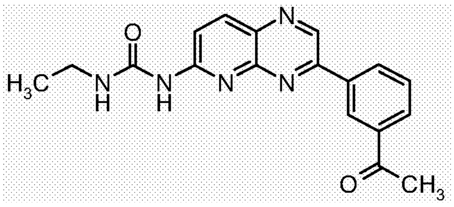

Verbindung 127 1-Ethyl-3-[3-(3-morpholin-4-yl-phenyl)-pyrido[2,3-b]pyrazin-6-yl]-harnstoff

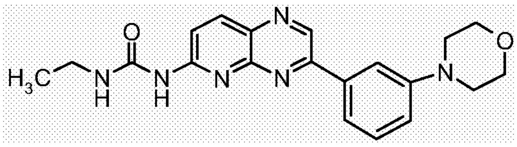

Verbindung 128 1-[3-(6-Amino-pyridin-3-yl)-pyrido[2,3-b]pyrazin-6-yl]-3-ethyl-harnstoff

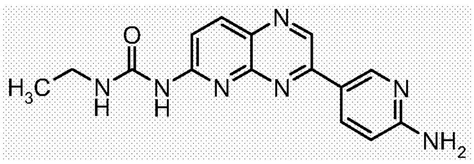

Verbindung 129 3-{4-[6-(3-Ethyl-ureido)-pyrido[2,3-b]-pyrazin-3-yl]-phenoxy}-propionsäure

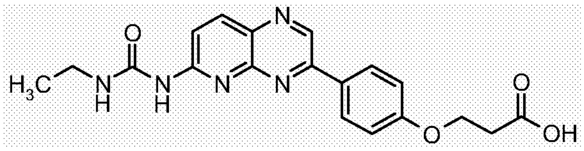

Verbindung 130 1-Isopropyl-3-(3-phenyl-pyrido[2,3-b]pyrazin-6-yl)-harnstoff

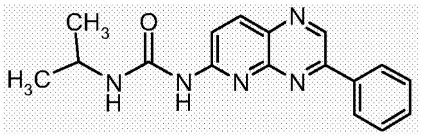

Verbindung 131 1-Cyclopentyl-3-(3-phenyl-pyrido[2,3-b]pyrazin-6-yl)-harnstoff

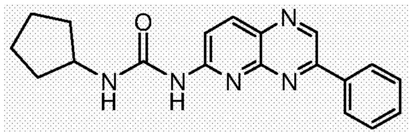

Verbindung 132 1-Pentyl-3-(3-phenyl-pyrido[2,3-b]pyrazin-6-yl)-harnstoff

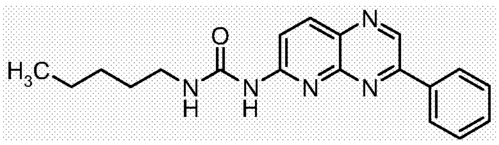

Verbindung 133 N-{4-[6-(3-Ethyl-ureido)-pyrido[2,3-b]pyrazin-3-yl]-phenyl}-acrylamid

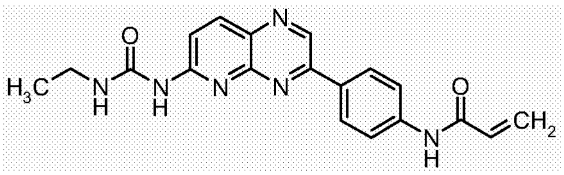

Verbindung 134 1-tert-Butyl-3-(3-phenyl-pyrido[2,3-b]pyrazin-6-yl)-harnstoff

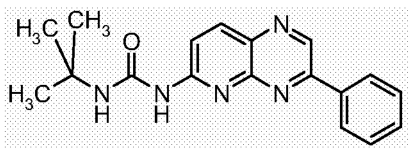

Verbindung 135 1-(2-Hydroxy-ethyl)-3-(3-phenyl-pyrido[2,3-b]pyrazin-6-yl)-harnstoff

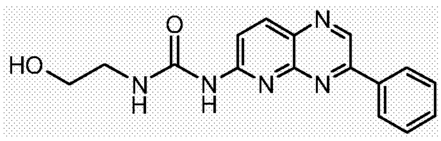

Verbindung 136 1-Cyclobutyl-3-(3-phenyl-pyrido[2,3-b]pyrazin-6-yl)-harnstoff

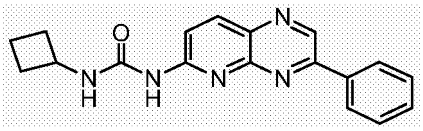

Verbindung 137 1-Allyl-3-[3-(4-nitro-phenyl)-pyrido[2,3-b]pyrazin-7-yl]-thioharnstoff

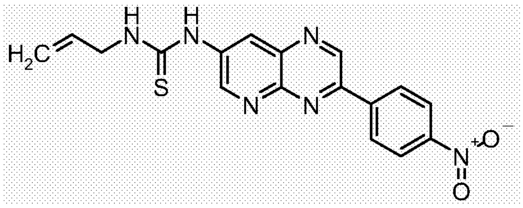

Verbindung 138 1-Ethyl-1-(Ethylcarbamoyl)-3-(3-phenyl-pyrido[2,3-b]pyrazin-6-yl)-harnstoff

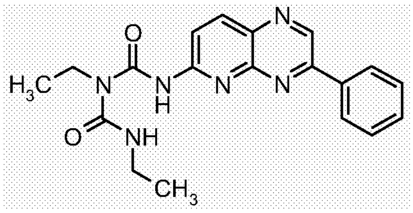

Verbindung 139 [3-(4-Hydroxy-phenyl)-pyrido[2,3-b]pyrazin-6-yl]-carbaminsäure-allyl-ester

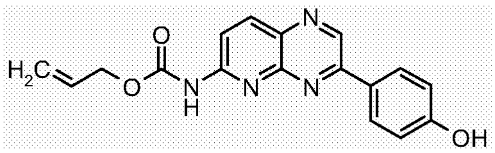

Verbindung 140 (3-Phenyl-pyrido[2,3-b]pyrazin-6-yl)-carbaminsäure-ethyl-ester

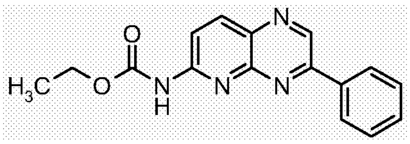

Verbindung 141 1-Ethyl-3-[3-(4-phenyl-piperazin-1-yl)-pyrido[2,3-b]pyrazin-6-yl]-harnstoff

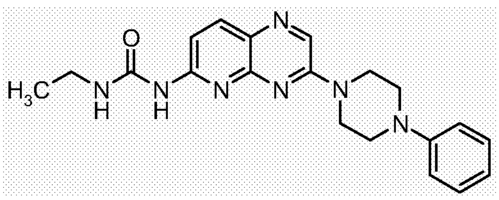

Verbindung 142 1-(2,2-Dimethyl-[1,3]dioxolan-4-ylmethyl)-3-(3-phenyl-pyrido[2,3-b] pyrazin-6-yl)-harnstoff

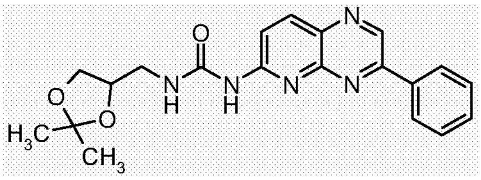

Verbindung 143 1,3-Bis-(3-phenyl-pyrido[2,3-b]pyrazin-6-yl)-harnstoff

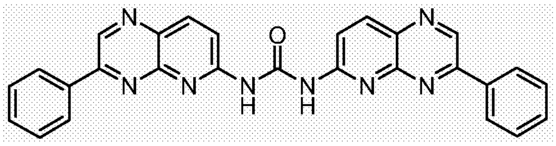

Verbindung 144 N-(3-Phenyl-pyrido[2,3-b]pyrazin-6-yl)-acetamidin

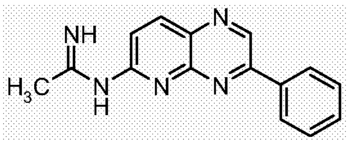

Verbindung 145 1-Ethyl-3-[3-(2-methoxy-pyridin-3-yl)-pyrido[2,3-b]pyrazin-6-yl]-thioharnstoff

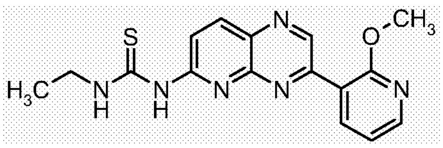

Verbindung 146 1-(4-Hydroxy-butyl)-3-(3-phenyl-pyrido[2,3-b]pyrazin-6-yl)-harnstoff

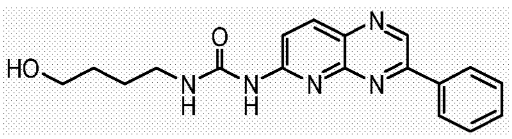

Verbindung 147 1-(3-Hydroxy-propyl)-3-(3-phenyl-pyrido[2,3-b]pyrazin-6-yl)-harnstoff

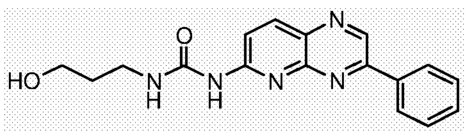

Verbindung 148 1-Ethyl-3-{3-[4-(3-morpholin-4-yl- propoxy)-phenyl]-pyrido[2,3-b]pyrazin-6-yl}-harnstoff

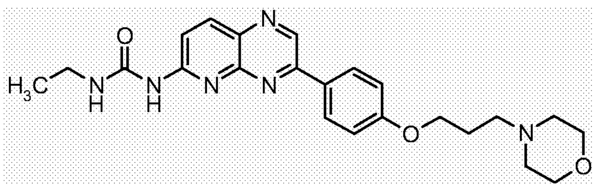

Verbindung 149 1-Ethyl-3-[3-(4-hydroxy-phenyl)-pyrido[2,3-b]pyrazin-6-yl]-thioharnstoff

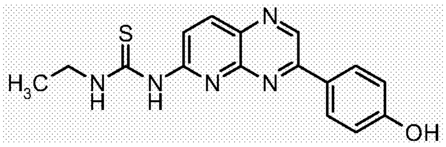

Verbindung 150 1-Ethyl-3-(3-p-tolylamino-pyrido[2,3-b]pyrazin-6-yl)-harnstoff

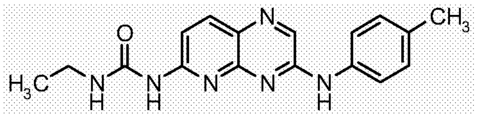

Verbindung 151 1-Ethyl-3-[3-(3-hydroxy-phenyl)-pyrido[2,3-b]pyrazin-6-yl]-harnstoff

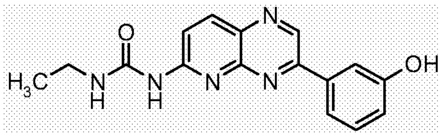

Verbindung 152 3-[6-(3-Ethyl-ureido)-pyrido[2,3-b]pyrazin-3-yl]-benzoesäure

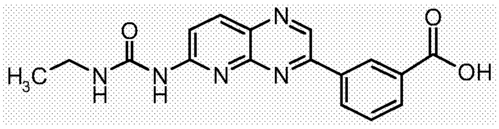

Verbindung 153 1-[3-(3,4-Dimethoxy-phenyl)-pyrido [2,3-b]pyrazin-6-yl]-3-ethyl-harnstoff

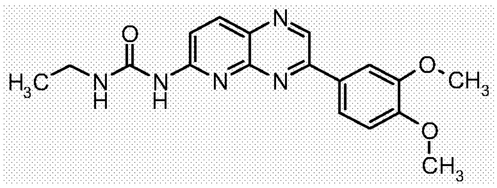

Verbindung 154 1-[3-(4-Amino-3-methoxy-phenyl)-pyrido[2,3-b]pyrazin-6-yl]-3-ethyl-harnstoff

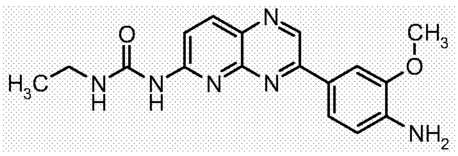

Verbindung 155 3-[6-(3-Ethyl-ureido)-pyrido[2,3-b]-pyrazin-3-yl]-benzamid

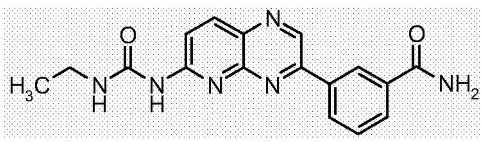

Verbindung 156 1-[3-(3-Chlor-4-hydroxy-5-methoxy-phenyl)-pyrido[2,3-b]pyrazin-6-yl]-3-ethyl-harnstoff

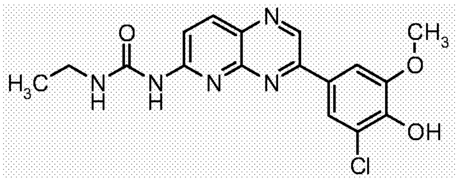

Verbindung 157 1-Ethyl-3-(3-m-tolylamino-pyrido-[2,3-b]pyrazin-6-yl)-harnstoff

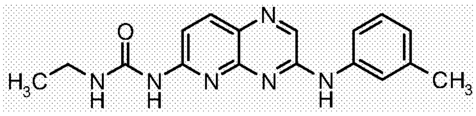

Verbindung 158 1-Ethyl-3-[3-(4-methoxy-phenylamino)-pyrido[2,3-b]pyrazin-6-yl]-harnstoff

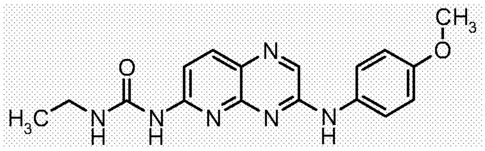

Verbindung 159 1-[3-(4-Chloro-phenylamino)-pyrido-[2,3-b]pyrazin-6-yl]-3-ethyl-harnstoff

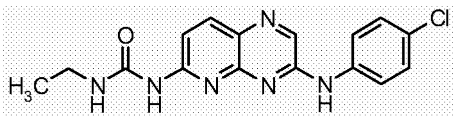

Verbindung 160 1-Ethyl-3-(3-o-tolylamino-pyrido[2,3-b]-pyrazin-6-yl)-harnstoff

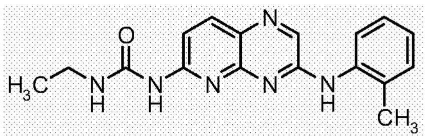

Verbindung 161 1-Ethyl-3-[3-(pyridin-3-ylamino)-pyrido-[2,3-b]pyrazin-6-yl]-harnstoff

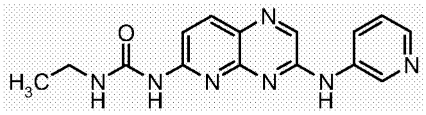

Verbindung 162 1-Ethyl-3-[3-(4-ethyl-phenylamino)-pyrido[2,3-b]pyrazin-6-yl]-harnstoff

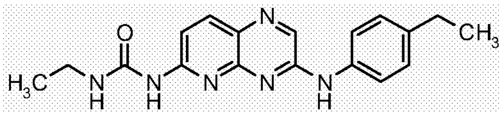

Verbindung 163 1-Ethyl-3-[3-(3-methoxy-4-methyl-phenylamino)-pyrido[2,3-b]pyrazin-6-yl]-harnstoff

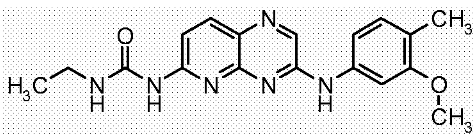

Verbindung 164 1-Ethyl-3-[3-(4-hydroxy-phenylamino)-pyrido[2,3-b]pyrazin-6-yl]-harnstoff

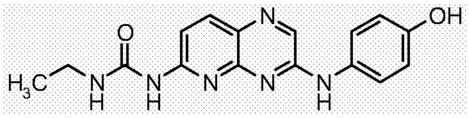

Verbindung 165 1-Ethyl-3-[3-(5-methyl-pyridin-2-yl-amino)-pyrido[2,3-b]pyrazin-6-yl]-harnstoff

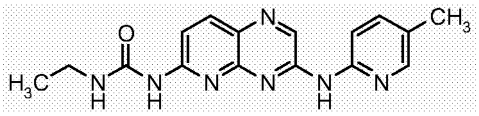

Verbindung 166 1-Ethyl-3-[3-(1-methyl-1H-pyrazol-3-ylamino)-pyrido[2,3-b]pyrazin-6-yl]-harnstoff

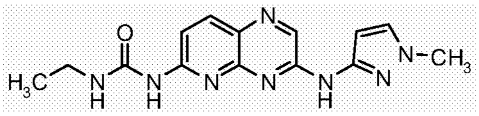

Verbindung 167 1-Ethyl-3-[3-(4-fluor-phenylamino)-pyrido[2,3-b]pyrazin-6-yl]-harnstoff

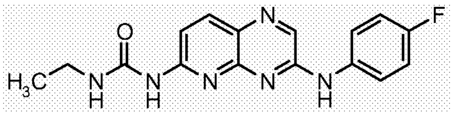

Verbindung 168 1-(4-Hydroxy-butyl)-3-[3-(4-hydroxy-3-methoxy-phenyl)-pyrido[2,3-b]pyrazin-6-yl]-harnstoff

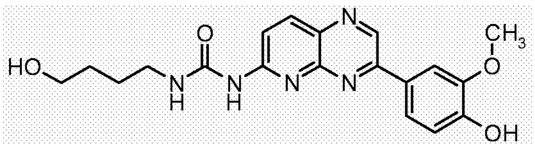

Verbindung 169 Phosphorsäure-mono-{4-[6-(3-ethyl-ureido)-pyrido-[2,3-b]pyrazin-3-yl]-2-methoxy-phenyl}-ester

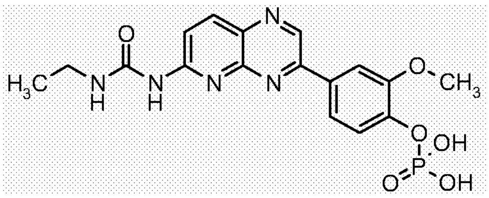

Verbindung 170 1-[3-(2-Chlor-4-hydroxy-phenyl)-pyrido[2,3-b]pyrazin-6-yl]-3-ethyl-harnstoff

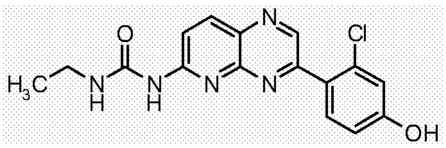

Verbindung 171 1-[3-(4-Chlor-2-methoxy-phenyl)-pyrido[2,3-b]pyrazin-6-yl]-3-ethyl-harnstoff

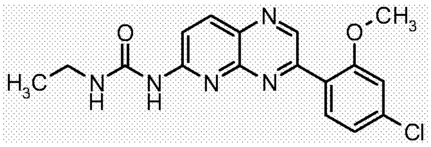

Verbindung 172 1-[3-(3,5-Dimethyl-1 H-pyrazol-4-yl)-pyrido[2,3-b]pyrazin-6-yl]-3-ethyl-harnstoff

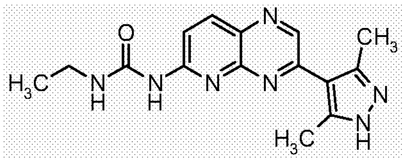

Verbindung 173 1-Ethyl-3-[3-(1-methyl-1H-pyrazol-4-yl)-pyrido[2,3-b]pyrazin-6-yl]-harnstoff

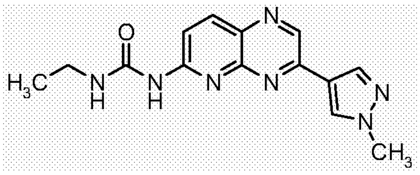

Verbindung 174 1-[3-(5-Cyano-thiophen-2-yl)-pyrido[2,3-b]pyrazin-6-yl]-3-ethyl-harnstoff

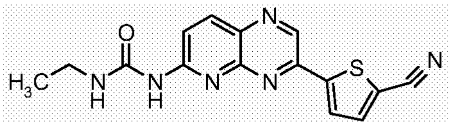

Verbindung 175 Natrium 2-chlor-4-[6-(3-ethyl-ureido)-pyrido[2,3-b]pyrazin-3-yl]-6-methoxy-phenolat

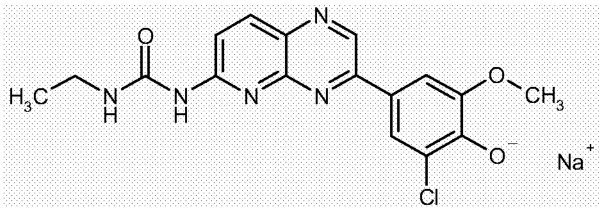

Verbindung 176 1-[3-(4-Hydroxy-3-methoxy-phenyl)-pyrido[2,3-b]pyrazin-6-yl]-3-(4-morpholin-4-yl-butyl)-harnstoff

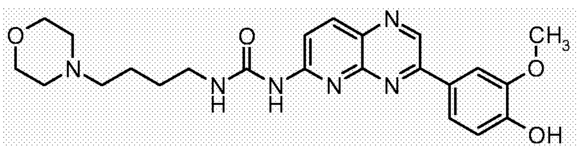

Verbindung 177 1-[3-(3-Chlor-4-methoxy-phenylamino)-pyrido[2,3-b]pyrazin-6-yl]-3-ethyl-harnstoff

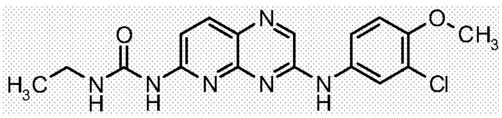

Verbindung 178 1-Ethyl-3-[3-(naphthalin-2-ylamino)-pyrido[2,3-b]pyrazin-6-yl]-harnstoff

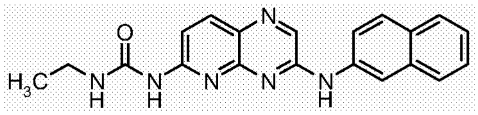

Verbindung 179 1-Ethyl-3-[3-(chinolin-3-ylamino)-pyrido[2,3-b]pyrazin-6-yl]-harnstoff

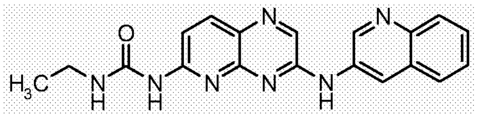

Verbindung 180 1-[3-(3,5-Dimethoxy-phenylamino)-pyrido[2,3-b]pyrazin-6-yl]-3-ethyl-harnstoff

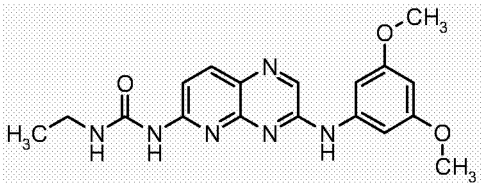

Verbindung 181 1-Ethyl-3-[3-(pyrazin-2-ylamino)-pyrido[2,3-b]pyrazin-6-yl]-harnstoff

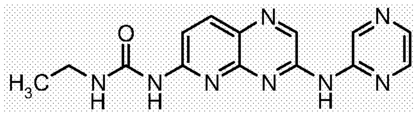

Verbindung 182 1-Ethyl-3-[3-(3-isopropoxy-phenylamino)-pyrido[2,3-b]pyrazin-6-yl]-harnstoff

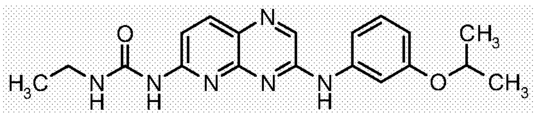

Verbindung 183 1-Ethyl-3-[3-(4-hydroxy-3-methoxy-phenyl)-pyrido[2,3-b]pyrazin-6-yl]-harnstoff p-Toluolsulfonat

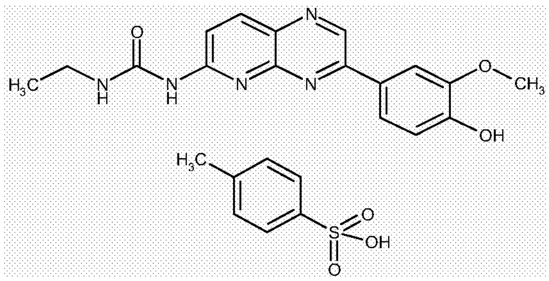

Verbindung 184 1-[3-(2-Chlor-pyridin-4-ylamino)-pyrido[2,3-b]pyrazin-6-yl]-3-ethyl-harnstoff

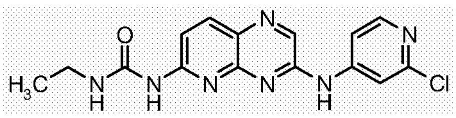

Verbindung 185 1-[3-(3,5-Dichlor-4-hydroxy-phenylamino)-pyrido[2,3-b]pyrazin-6-yl]-3-ethyl-harnstoff

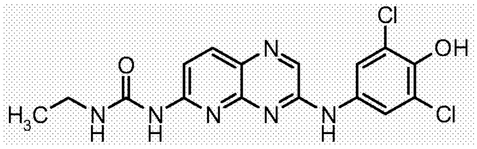

Verbindung 186 1-[3-(3,5-Dichlor-4-hydroxy-phenyl)-pyrido[2,3-b]pyrazin-6-yl]-3-ethyl-harnstoff

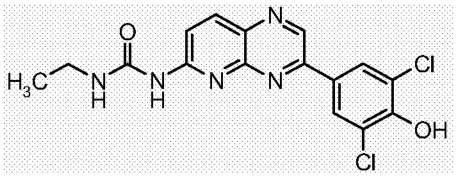

Verbindung 187 1-[3-(3,4-Dimethoxy-phenylamino)-pyrido[2,3-b]pyrazin-6-yl]-3-ethyl-harnstoff

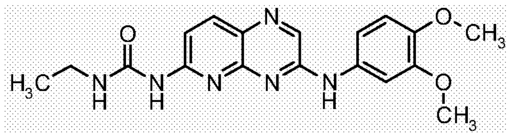

Verbindung 188 1-Ethyl-3-[3-(3-hydroxy-4-methyl-phenylamino)-pyrido[2,3-b]pyrazin-6-yl]-harnstoff

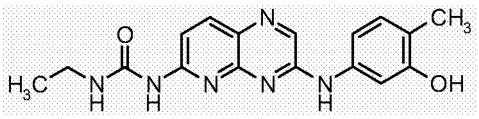

Verbindung 189 3-[6-(3-Ethyl-ureido)-pyrido[2,3-b]-pyrazin-3-ylamino]-5-trifluormethyl-benzoesäure

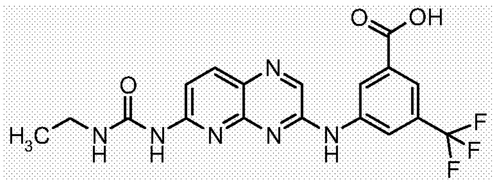

Verbindung 190 1-Ethyl-3-[3-(6-methoxy-pyridin-3-ylamino)-pyrido[2,3-b]pyrazin-6-yl]-harnstoff

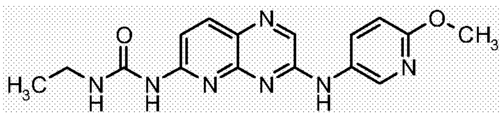

Verbindung 191 1-[3-(3,5-Dimethyl-phenylamino)-pyrido[2,3-b]pyrazin-6-yl]-3-ethyl-harnstoff

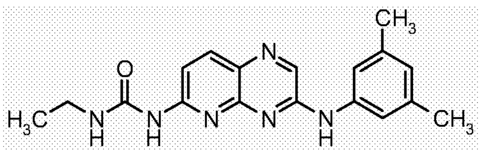

Verbindung 192 1-[3-(4-Cyano-phenylamino)-pyrido-[2,3-b]pyrazin-6-yl]-3-ethyl-harnstoff

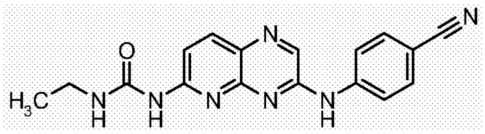

Verbindung 193 1-Ethyl-3-[3-(4-hydroxy-3-methoxy-phenyl)-pyrido[2,3-b]pyrazin-6-yl]-harnstoff (Z)-but-2-endicarbonsäure-Salz

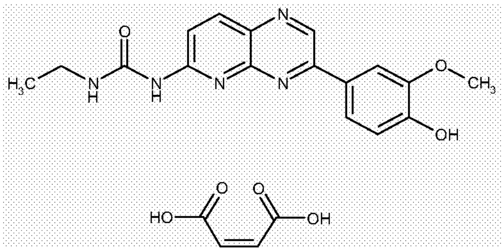

Verbindung 194 1-Ethyl-3-[3-(4-hydroxy-3-methoxy-phenyl)-pyrido[2,3-b]pyrazin-6-yl]-harnstoff Hydrochlorid

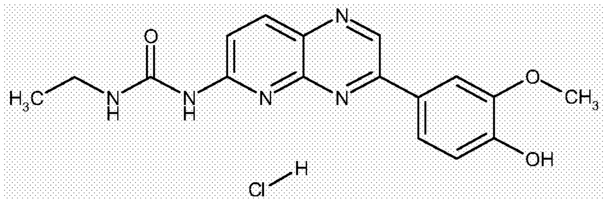

Verbindung 195 1-Ethyl-3-(3-phenyl-pyrido[2,3-b]pyrazin-6-yl)-thioharnstoff

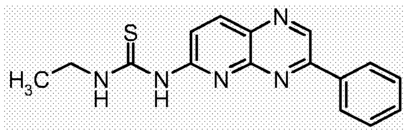

Verbindung 196 1-(3-Chlor-pyrido[2,3-b]pyrazin-6-yl)-3-cyclohexyl-harnstoff

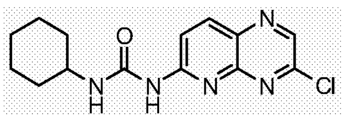

Verbindung 197 1-Ethyl-3-[3-(4-hydroxy-3-methoxy-phenylamino)-pyrido[2,3-b]pyrazin-6-yl]-harnstoff

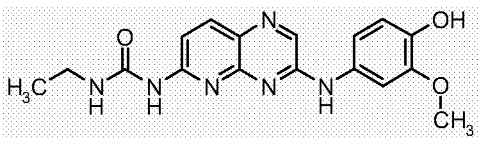

Verbindung 198 1-Ethyl-3-[3-(4-hydroxy-3,5-dimethyl-phenyl)-pyrido[2,3-b]pyrazin-6-yl]-harnstoff

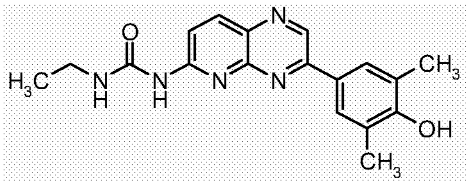

Verbindung 199 3-Ethyl-1-phenethyl-1-(3-phenyl- pyrido[2,3-b]pyrazin-6-yl)-harnstoff

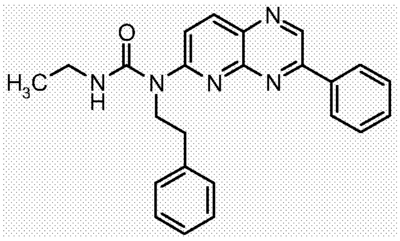

Verbindung 200 1-Allyl-3-{3-[4-(tetrahydro-pyran-2-yloxy)-phenyl]-pyrido[2,3-b]pyrazin-6-yl}-thioharnstoff

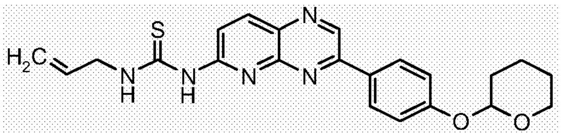

Verbindung 201 3-Ethyl-1-[3-(4-methoxy-phenyl)-pyrido[2,3-b]pyrazin-6-yl]-1-propyl- harnstoff

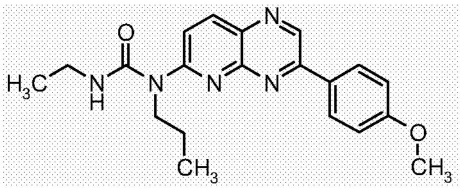

Verbindung 202 3-Ethyl-1-(3-phenyl-pyrido[2,3-b]pyrazin-6-yl)-1-(2-piperidin-1-yl-ethyl)-harnstoff Hydrochlorid

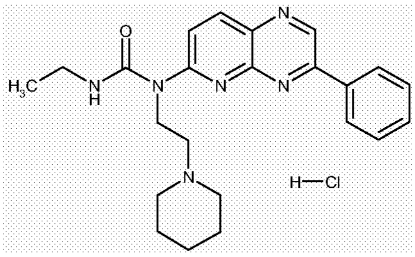

Verbindung 203 N-{4-[6-(3-Ethyl-ureido)-pyrido[2,3-b]-pyrazin-3-yl]-phenyl}-2-phenyl- acetamid

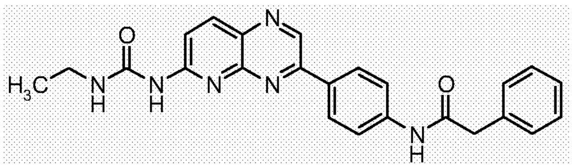

Verbindung 204 1-(4-Hydroxy-butyl)-3-(3-phenyl-pyrido[2,3-b]pyrazin-6-yl)-harnstoff Hydrochlorid

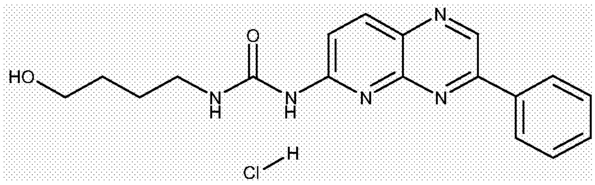

Verbindung 205 Essigsäure-4-[3-(3-phenyl-pyrido[2,3-b]pyrazin-6-yl)-ureido]-butyl ester

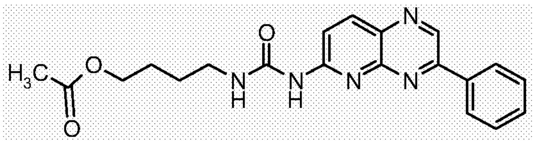

Verbindung 206 1-(4-Amino-butyl)-3-(3-phenyl-pyrido[2,3-b]pyrazin-6-yl)-harnstoff

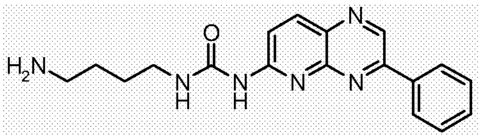

Verbindung 207 1-(5-Hydroxy-pentyl)-3-(3-phenyl-pyrido[2,3-b]pyrazin-6-yl)-harnstoff

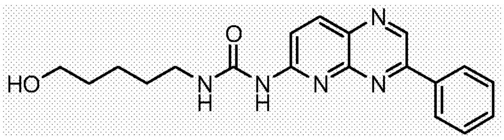

Verbindung 208 3-[6-(3-Ethyl-ureido)-pyrido[2,3-b]-pyrazin-3-yl]-N-methyl-benzamid

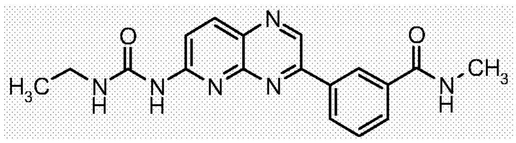

Verbindung 209 1-Ethyl-3-[3-(2-methoxy-5-methyl-phenyl)-pyrido[2,3-b]pyrazin-6-yl]-harnstoff

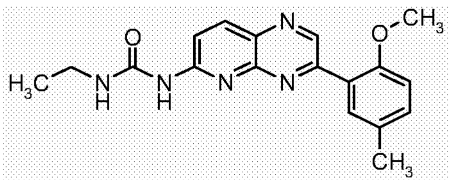

Verbindung 210 1-Ethyl-3-(3-p-tolyl-pyrido[2,3-b] pyrazin-6-yl)-harnstoff

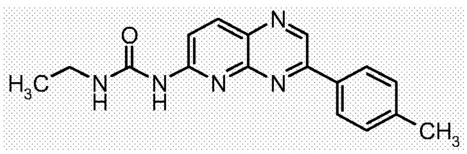

Verbindung 211 1-Ethyl-3-[3-(methyl-p-tolyl-amino)-pyrido[2,3-b]pyrazin-6-yl]-harnstoff

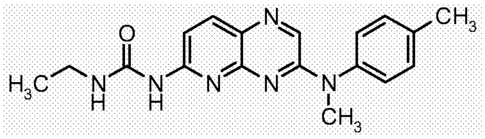

Verbindung 212 1-Ethyl-3-[3-(2-p-tolyl-ethylamino)-pyrido[2,3-b]pyrazin-6-yl]-harnstoff

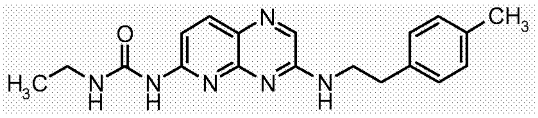

Verbindung 213 1-Ethyl-3-[3-(4-methyl-benzylamino)-pyrido[2,3-b]pyrazin-6-yl]-harnstoff

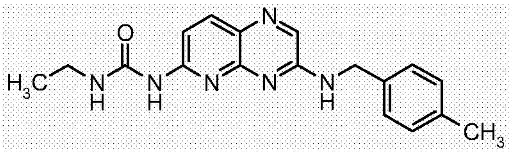

Verbindung 214 1-Ethyl-3-[3-(3-fluor-4-methyl-phenyl-amino)-pyrido[2,3-b]pyrazin-6-yl]-harnstoff

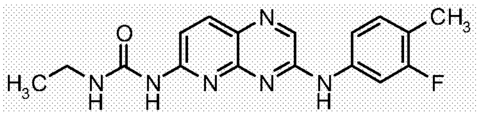

Verbindung 215 1-[3-(3,4-Dimethyl-phenylamino)-pyrido[2,3-b]pyrazin-6-yl]-3-ethyl-harnstoff

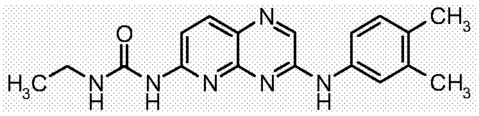

Verbindung 216 1-Ethyl-3-[3-(4-isopropyl-phenylamino)-pyrido[2,3-b]pyrazin-6-yl]-harnstoff

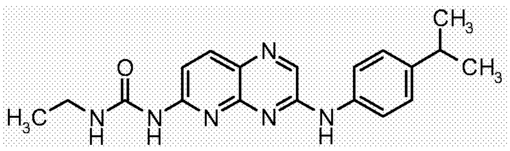

Verbindung 217 1-(4-Morpholin-4-yl-butyl)-3-(3-phenyl-pyrido[2,3-b]pyrazin-6-yl)-harnstoff

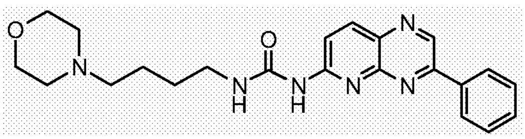

Verbindung 218 N-{4-[3-(3-Phenyl-pyrido[2,3-b]pyrazin-6-yl)-ureido]-butyl}-acetamid

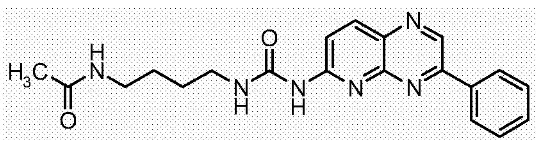

Verbindung 219 1-[3-(3-Amino-4-methyl-phenyl)-pyrido[2,3-b]pyrazin-6-yl]-3-ethyl-harnstoff

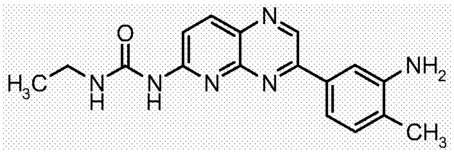

Verbindung 220 1-[3-(3-Acetyl-2-fluor-phenyl)-pyrido[2,3-b]pyrazin-6-yl]-3-ethyl-harnstoff

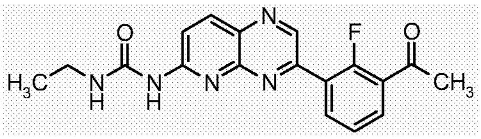

Verbindung 221 1-Ethyl-3-[3-(4-methoxy-3-methyl-phenyl)-pyrido[2,3-b]pyrazin-6-yl]-harnstoff

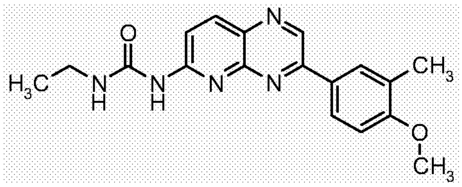

Verbindung 222 1-[3-(6-Ethoxy-pyridin-3-yl)-pyrido[2,3-b]pyrazin-6-yl]-3-ethyl-harnstoff

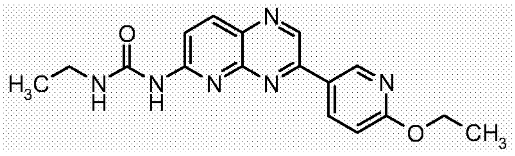

Verbindung 223 1-Ethyl-3-[3-(2-fluor-4-methyl-phenyl)-pyrido[2,3-b]pyrazin-6-yl]-harnstoff

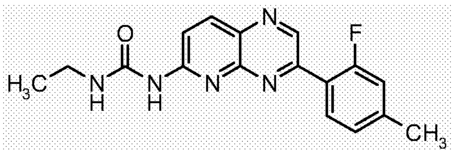

Verbindung 224 1-Ethyl-3-[3-(3-fluor-4-methoxy-phenyl)-pyrido[2,3-b]pyrazin-6-yl]-harnstoff

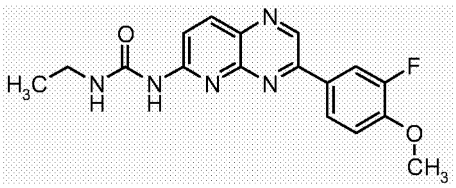

Verbindung 225 1-Ethyl-3-[3-(2-fluor-3-methoxy-phenyl)-pyrido[2,3-b]pyrazin-6-yl]-harnstoff

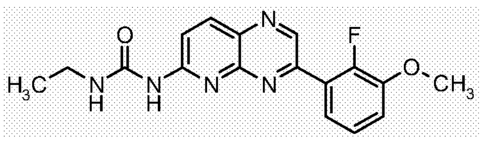

Verbindung 226 1-Ethyl-3-[3-(3,4,5-trimethoxy-phenyl)-pyrido[2,3-b]pyrazin-6-yl]-harnstoff

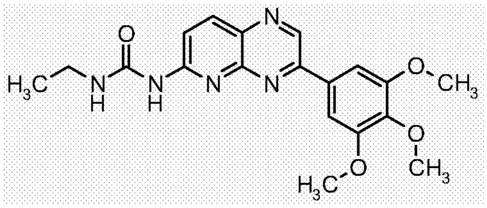

Verbindung 227 1-[3-(3,5-Difluor-2-methoxy-phenyl)-pyrido[2,3-b]pyrazin-6-yl]-3-ethyl-harnstoff

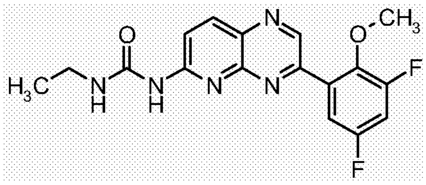

Verbindung 228 1-Ethyl-3-[3-(4-trifluormethyl-phenyl)-pyrido[2,3-b]pyrazin-6-yl]-harnstoff

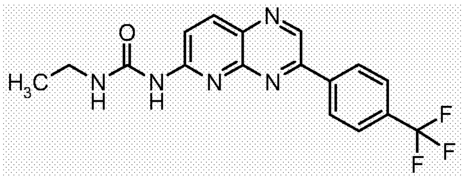

Verbindung 229 1-Ethyl-3-[3-(2,3,4-trimethoxy-phenyl)-pyrido[2,3-b]pyrazin-6-yl]-harnstoff

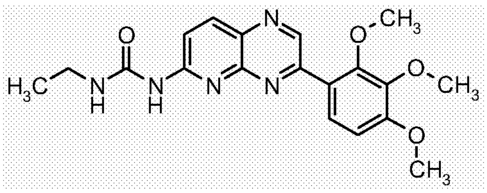

Verbindung 230 1-[3-(3-Chlor-4-methoxy-phenyl)-pyrido[2,3-b]pyrazin-6-yl]-3-ethyl-harnstoff

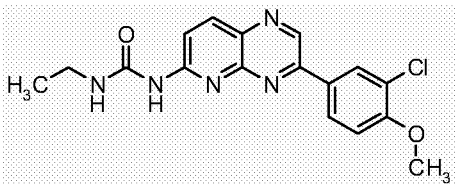

Verbindung 231 1-Ethyl-3-[3-(3-fluor-4-hydroxy-phenyl)-pyrido[2,3-b]pyrazin-6-yl]-harnstoff

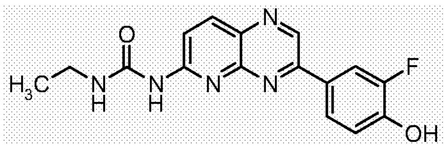

Verbindung 232 1-Ethyl-3-[3-(6-fluor-pyridin-3-yl)-pyrido[2,3-b]pyrazin-6-yl]-harnstoff

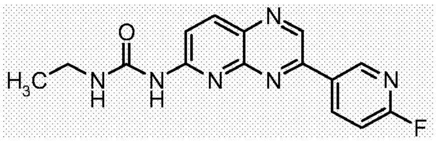

Verbindung 233 1-[3-(2,4-Dimethyl-thiazol-5-yl)-pyrido-[2,3-b]pyrazin-6-yl]-3-ethyl-harnstoff

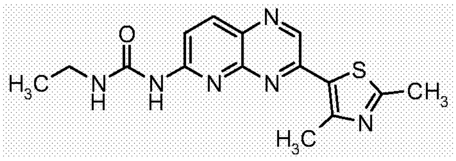

Verbindung 234 1-Ethyl-3-[3-(2-methoxy-phenyl)-pyrido[2,3-b]pyrazin-6-yl]-harnstoff

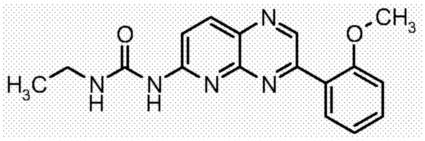

Verbindung 235 1-[3-(2-Chlor-pyridin-4-yl)-pyrido[2,3-b]pyrazin-6-yl]-3-ethyl-harnstoff

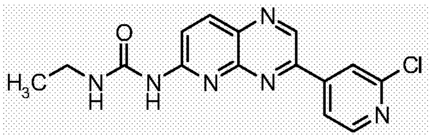

Verbindung 236 1-[3-(5-Acetyl-thiophen-2-yl)-pyrido[2,3-b]pyrazin-6-yl]-3-ethyl-harnstoff

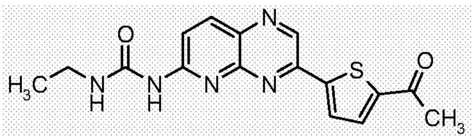

Verbindung 237 1-[3-(5-Chlor-thiophen-2-yl)-pyrido[2,3-b]pyrazin-6-yl]-3-ethyl-harnstoff

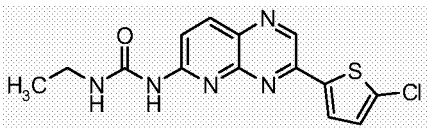

Verbindung 238 1-Ethyl-3-[3-(3-methoxy-phenyl)-pyrido[2,3-b]pyrazin-6-yl]-harnstoff

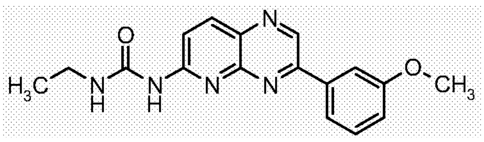

Verbindung 239 1-[3-(3-Brom-5-methoxy-phenyl)-pyrido[2,3-b]pyrazin-6-yl]-3-ethyl-harnstoff

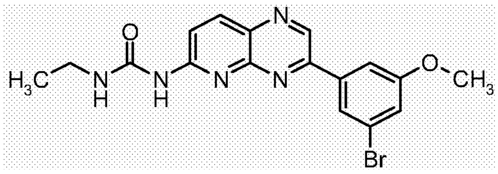

Verbindung 240 1-[3-(Benzothiazol-6-ylamino)-pyrido[2,3-b]pyrazin-6-yl]-3-ethyl-harnstoff

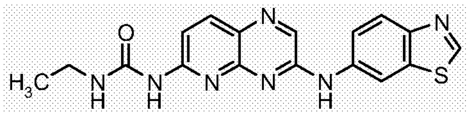

Verbindung 241 1-Ethyl-3-[3-(4-methyl-3-trifluoromethyl-phenylamino)-pyrido[2,3-b]pyrazin-6-yl]-harnstoff

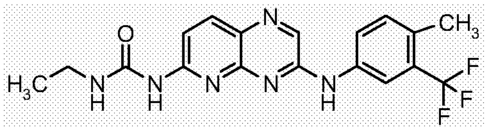

Verbindung 242 1-[3-(3-Cyano-4-methyl-phenylamino)-pyrido[2,3-b]pyrazin-6-yl]-3-ethyl-harnstoff

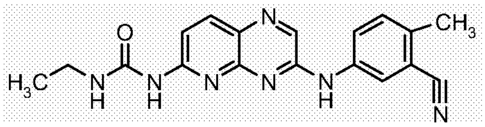

Verbindung 243 1-Ethyl-3-[3-(4-phenoxy-phenylamino)-pyrido[2,3-b]pyrazin-6-yl]-harnstoff

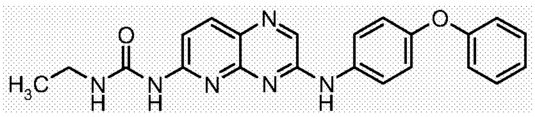

Verbindung 244 1-[3-(4-Chlor-3-methyl-phenylamino)-pyrido[2,3-b]pyrazin-6-yl]-3-ethyl-harnstoff

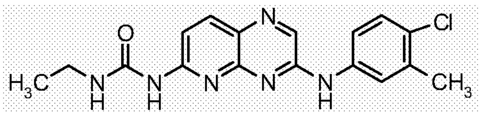

Verbindung 245 1-[3-(2-Chlor-4-methyl-phenylamino)-pyrido[2,3-b]pyrazin-6-yl]-3-ethyl-harnstoff

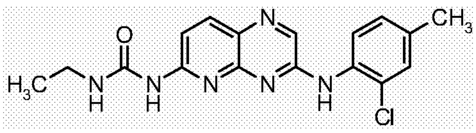

Verbindung 246 1-Ethyl-3-[3-(3-trifluoromethyl-phenylamino)-pyrido[2,3-b]pyrazin-6-yl]-harnstoff

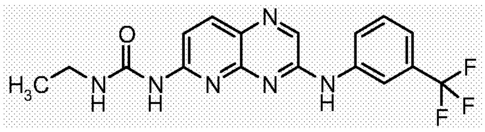

Verbindung 247 1-[3-(2-Chlor-4-trifluormethoxy-phenylamino)-pyrido[2,3-b]pyrazin-6-yl]-3-ethyl-harnstoff

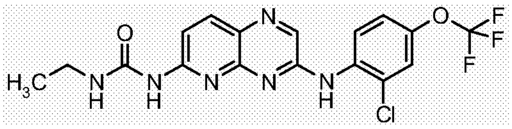

Verbindung 248 1-[3-(4-Chlor-2-methoxy-5-methyl-phenylamino)-pyrido[2,3-b]pyrazin-6-yl]-3-ethyl-harnstoff

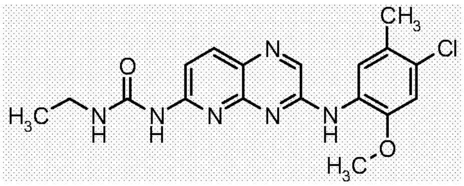

Verbindung 249 1-Ethyl-3-[3-(4-methylsulfanyl-phenyl)-pyrido[2,3-b]pyrazin-6-yl]-harnstoff

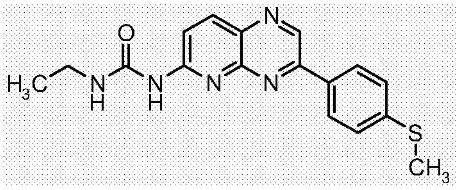

Verbindung 250 3-[6-(3-Ethyl-ureido)-pyrido[2,3-b]pyrazin-3-yl]-benzolsulfonamid

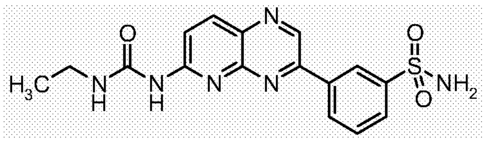

Verbindung 251 N-{4-[3-(3-Phenyl-pyrido[2,3-b]pyrazin-6-yl)-ureido]-butyl}-methansulfonamid

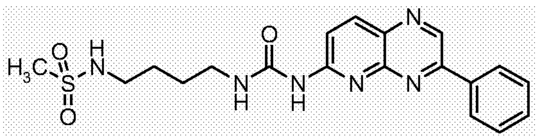

Verbindung 252 1-[3-(Benzo[1,3]dioxol-5-ylamino)-pyrido[2,3-b]pyrazin-6-yl]-3-ethyl-harnstoff

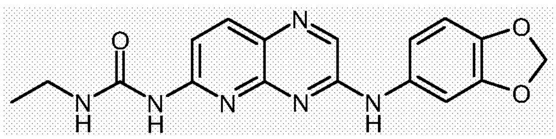

Verbindung 253 3-[6-(3-Ethyl-ureido)-pyrido[2,3-b]pyrazin-3-ylamino]-benzoesäure

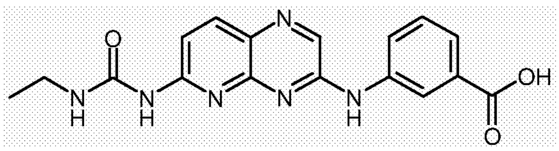

Verbindung 254 2-Chlor-4-[6-(3-ethyl-ureido)-pyrido[2,3-b]pyrazin-3-ylamino]-

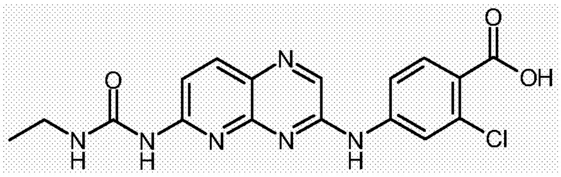

Verbindung 255 1-Ethyl-3-[3-(3-methoxy-5-trifluormethyl-phenylamino)-pyrido[2,3-b]pyrazin-6-yl]-harnstoff

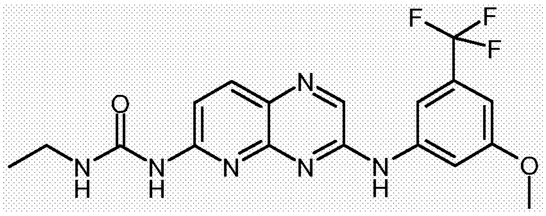

Verbindung 256 1-Ethyl-3-[3-(pyrimidin-2-ylamino)-pyrido[2,3-b]pyrazin-6-yl]-harnstoff

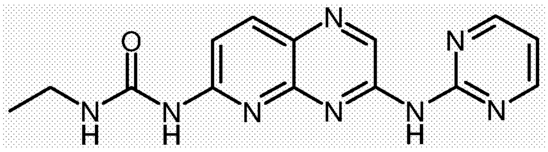

Verbindung 257 6-[6-(3-Ethyl-ureido)-pyrido[2,3-b]pyrazin-3-ylamino]-naphthalin-2-carbonsäure

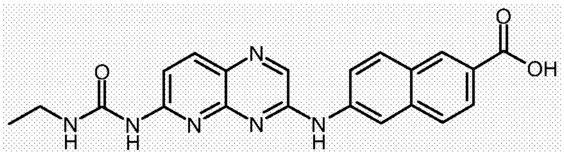

Verbindung 258 1-Ethyl-3-[3-(4-hydroxy-chinolin-2-ylamino)-pyrido[2,3-b]pyrazin-6-yl]-harnstoff

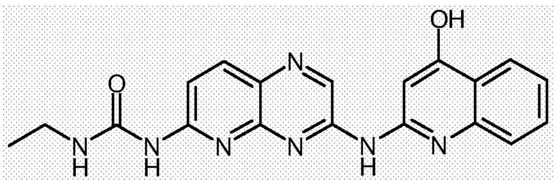

Verbindung 259 1-Ethyl-3-[3-(chinolin-6-ylamino)-pyrido[2,3-b]pyrazin-6-yl]-harnstoff

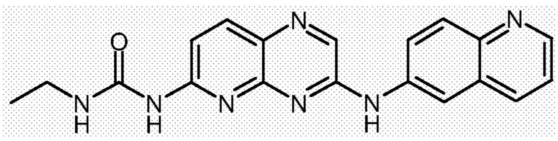

Verbindung 260 1-Ethyl-3-[3-(3,4,5-trimethoxy-phenylamino)-pyrido[2,3-b]pyrazin-6-yl]-harnstoff

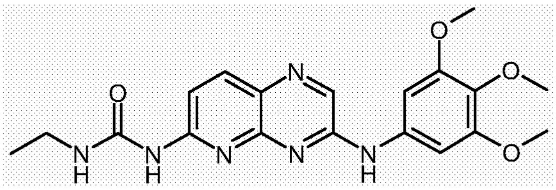

Verbindung 261 1-Ethyl-3-[3-(1H-indol-6-ylamino)-pyrido[2,3-b]pyrazin-6-yl]-harnstoff

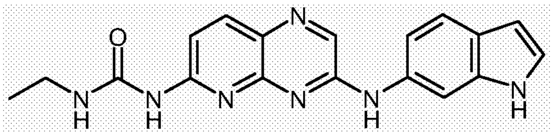

Verbindung 262 1-Ethyl-3-[3-(3-oxo-3,4-dihydro-2H-benzo[1,4]oxazin-7-ylamino)-pyrido[2,3-b]pyrazin-6-yl]-harnstoff

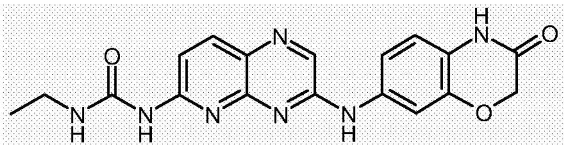

Verbindung 263 1-Ethyl-3-[3-(chinoxalin-6-ylamino)-pyrido[2,3-b]pyrazin-6-yl]-harnstoff

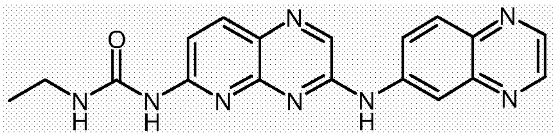

Verbindung 264 1-Ethyl-3-[3-(3-trifluormethoxy-phenylamino)-pyrido[2,3-b]pyrazin-6-yl]-harnstoff

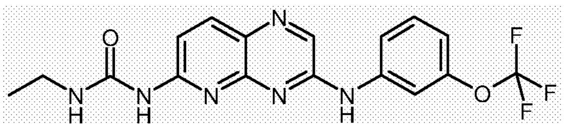

Verbindung 265 1-Ethyl-3-[3-(4-isopropoxy-phenylamino)-pyrido[2,3-b]pyrazin-6-yl]-harnstoff

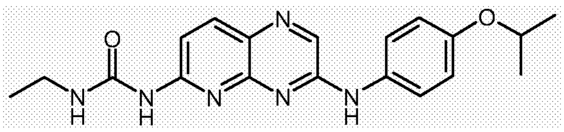

Verbindung 266 1-[3-(Dibenzofuran-2-ylamino)-pyrido[2,3-b]pyrazin-6-yl]-3-ethyl-harnstoff

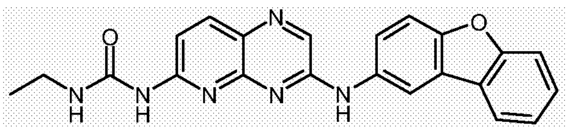

Verbindung 267 1-(6-Dimethylamino-hexyl)-3-[3-(4-hydroxy-3-methoxy-phenyl)-pyrido[2,3-b]pyrazin-6-yl]-harnstoff

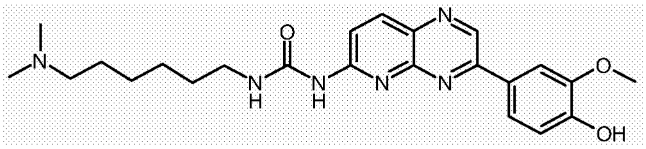

Verbindung 268 1-(4-Dimethylamino-butyl)-3-[3-(4-hydroxy-3-methoxy-phenyl)-pyrido[2,3-b]pyrazin-6-yl]-harnstoff

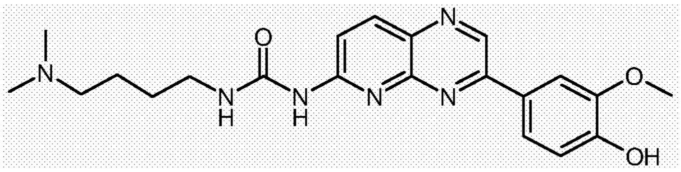

Verbindung 269 1-[3-(4-Hydroxy-3-methoxy-phenyl)-pyrido[2,3-b]pyrazin-6-yl]-3-(5-morpholin-4-yl-pentyl)-harnstoff

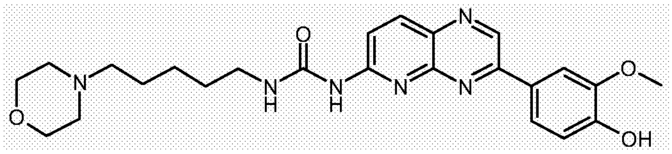

Die orale Verabreichung kann beispielsweise in fester Form als Tablette, Kapsel, Gelkapsel, Dragee, Granulat oder Pulver, jedoch auch in Form einer trinkbaren Lösung erfolgen. Zur oralen Verabreichung können die neuen, erfindungsgemäßen Verbindungen, wie vorstehend definiert, mit bekannten und gewöhnlich verwendeten, physiologisch verträglichen Hilfs- und Trägerstoffen kombiniert werden, wie z.B. Gummiarabikum, Talkum, Stärke, Zucker wie z.B. Mannit, Methylcellulose, Lactose, Gelatine, oberflächenaktive Mittel, Magnesiumstearat, Cyclodextrine, wässrige oder nichtwässrige Trägerstoffe, Verdünnungsmittel, Dispergiermittel, Emulgatoren, Schmiermittel, Konservierungsstoffe und Geschmacksstoffe (z.B. etherische Öle). Die erfindungsgemäßen Verbindungen können auch in einer mikropartikulären, z.B. nanopartikulären Zusammensetzung dispergiert sein.

Die nicht orale Verabreichung kann beispielsweise durch intravenöse, subkutane oder intramuskuläre Injektion steriler wässriger oder öliger Lösungen, Suspensionen oder Emulsionen, mittels Implantaten oder durch Salben, Cremes oder Suppositorien erfolgen. Gegebenenfalls kann auch eine Verabreichung als Retardform erfolgen. Implantate können inerte Materialen enthalten, z.B. biologisch abbaubare Polymere oder synthetische Silicone wie z.B. Silicongummi. Eine intravaginale Verabreichung kann z.B. mittels Vaginalringen erfolgen. Eine intrauterine Verabreichung kann z.B. mittels Diaphragmen oder anderer geeigneter intrauteriner Vorrichtungen erfolgen. Darüber hinaus ist auch eine transdermale Verabreichung, insbesondere mittels einer dazu geeigneten Formulierung und/oder geeigneter Mittel wie z.B. Pflaster, vorgesehen.

Wie bereits vorstehend erläutert, können die neuen, erfindungsgemäßen Verbindungen auch mit weiteren pharmazeutisch aktiven Wirkstoffen kombiniert werden. Im Rahmen einer Kombinationstherapie können die einzelnen wirksamen Bestandteile gleichzeitig oder getrennt verabreicht werden, und zwar entweder auf demselben Wege (z.B. oral) oder auf getrennten Wegen (z.B. oral und als Injektion). Sie können in gleichen oder unterschiedlichen Mengen in einer Einheitsdosis vorliegen bzw. verabreicht werden. Es kann auch ein bestimmtes Dosierungsregime angewendet werden, sofern dies zweckmäßig erscheint. Auf diese Weise können auch mehrere der neuen, erfindungsgemäßen Verbindungen miteinander kombiniert werden.

Die Dosierung kann je nach Art der Indikation, der Schwere der Erkrankung, der Art der Verabreichung, dem Alter, Geschlecht, Körpergewicht und der Sensitivität des zu behandelnden Subjekts in einem breiten Rahmen variieren. Es entspricht den Fähigkeiten eines Fachmanns, eine "pharmakologisch wirksame Menge" der kombinierten pharmazeutischen Zusammensetzung zu bestimmen. Die Verabreichung kann in einer einzigen Dosis oder mehreren getrennten Dosierungen erfolgen.

Eine geeignete Einheitsdosis ist z.B. 0,001 mg bis 100 mg des Wirkstoffs, d.h. mindestens einer erfindungsgemäßen Verbindung und gegebenenfalls eines weiteren pharmazeutisch aktiven Wirkstoffs, pro kg Körpergewicht eines Patienten.

In einem weiteren Aspekt der vorliegenden Erfindung sind demnach auch pharmazeutische Zusammensetzungen umfassend eine pharmakologisch aktive Menge mindestens einer Verbindung ausgewählt aus der Gruppe bestehend aus: "Verbindung 38, 39, 40, 41, 42, 43, 44, 45, 46, 47, 48, 49, 50, 51, 52, 53, 54, 55, 56, 57, 58, 59, 60, 61,

62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131,132, 133, 134, 135, 136, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268 und/oder Verbindung 269" sowie gegebenenfalls pharmazeutisch verträgliche Träger- und/oder Hilfsstoffe, von der vorliegenden Erfindung erfasst.

Bevorzugte und besonders bevorzugte pharmazeutische Zusammensetzungen sind jene, die mindestens eine der vorstehend genannten bevorzugten erfindungsgemäßen Verbindungen umfassen, In pharmazeutischen Zusammensetzungen gemäß der vorliegenden Erfindung können neben mindestens einer erfindungsgemäßen Verbindung, wie vorstehend definiert, auch noch mindestens ein weiterer pharmazeutisch aktiver Wirkstoffe vorliegen, wie vorstehend bereits näher aufgeführt.

In den erfindungsgemäßen pharmazeutischen Zusammensetzungen liegt mindestens eine der neuen, erfindungsgemäßen Verbindungen, wie vorstehend definiert, in einer pharmakologischen aktiven Menge, vorzugsweise in einer Einheitsdosis, z.B. der vorstehend genannten Einheitsdosis, vor, und zwar vorzugsweise in einer Verabreichungsform, die eine orale Verabreichung ermöglicht.

Bezüglich der erfindungsgemäße Verbindungen umfassende pharmazeutische Zusammensetzungen sowie bezüglich der Verwendung der erfindungsgemäßen Verbindungen als Arzneimittel sei hinsichtlich Verwendungs- und Verabreichungsmöglichkeiten auf das bereits im Zusammenhang mit der Verwendung der neuen, erfindungsgemäßen Verbindungen selbst, Gesagte verwiesen.

In einem weiteren Aspekt der vorliegenden Erfindung wurde die erfinderische Aufgabe überraschender Weise gelöst durch die Bereitstellung eines Kits umfassend eine pharmakologisch aktive Menge mindestens einer bevorzugten erfindungsgemäßen Verbindung, wie oben dargestellt, und eine pharmakologisch aktive Menge mindestens eines weiteren pharmakologisch aktiven Wirkstoffs wie vorstehend definiert.

Die Benennung der erfindungsgemäßen Verbindungen der allgemeinen Formel (I) samt bevorzugter Ausführungsbeispiele, und insbesondere der Verbindungen 1 bis 269, erfolgte mit der AutoNom 2000 - Software (ISIS ™/ Draw 2.5; MDL).

<u>Allgemeine Synthesevorschriften für die erfindungsgemäßen Verbindungen</u>

Die Verfahren zur Herstellung erfindungsgemäßer substituierter Pyrido[2,3-b]pyrazine werden nachstehend erläutert.

Die erfindungsgemäßen Verbindungen sind gemäß der folgenden Schemata (Schema 1 - 7) bzw. entsprechenden, dem Fachmann bekannten Verfahren erhältlich. Für die Herstellung der erfindungsgemäßen Verbindungen sei außerdem auf die Patentschriften WO2004/104002 und WO 2004/104003 bzw. auf entsprechende, literaturbekannte Methoden verwiesen.

Die in den folgenden Schemata aufgeführten Reste X1 bis X21 entsprechen in ihrer Bedeutung den weiter oben in Zusammenhang mit der allgemeinen Formel (I) definierten Substituenten, bspw. R-Resten usw. Die jeweilige Zuordnung ist für den Fachmann aufgrund seines durchschnittlichen Fachwissens einfach zu bewerkstelligen.

Schema 1

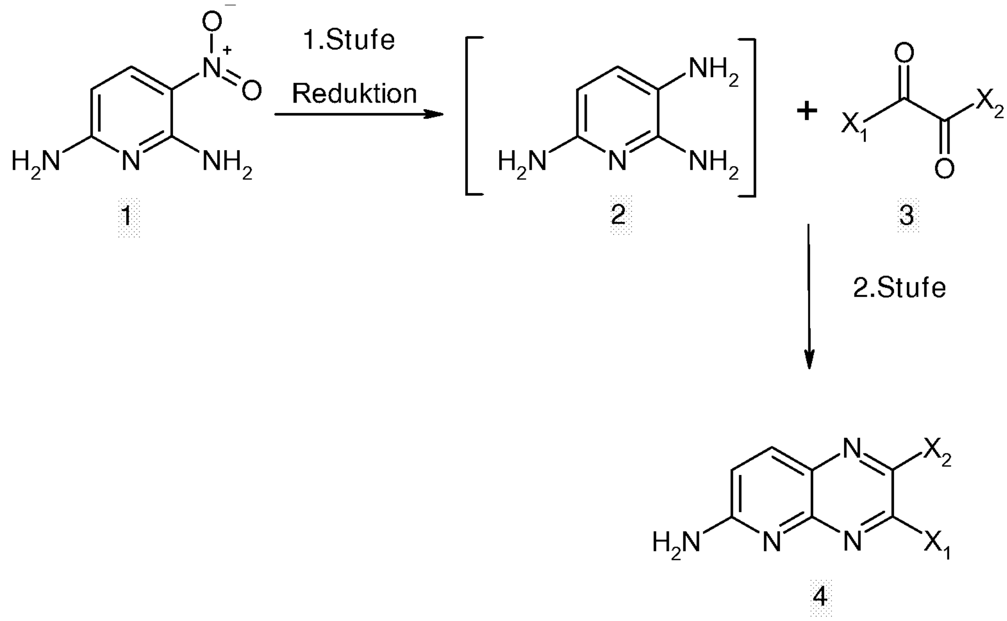


1 2 3 4

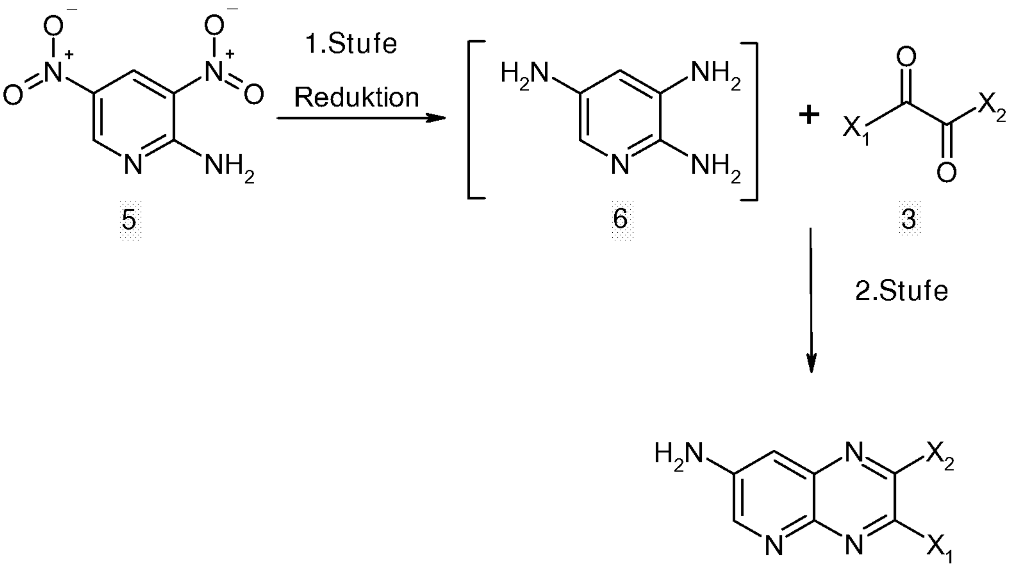


5 6 3 7

Vorstufen für ausgewählte Beispiele der erfindungsgemäßen Pyrido[2,3-b]pyrazine, bei denen die Substituenten R2 und R4 mit Wasserstoff substituiert sein sollen, sind beispielsweise nach dem Verfahren in Schema 2 bzw. einem entsprechenden, dem Fachmann bekannten Verfahren erhältlich.

Schema 2

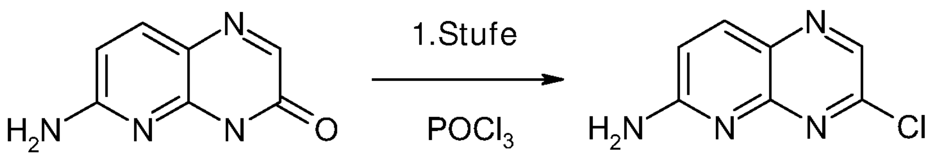


8 9

Vorstufen für ausgewählte Beispiele der erfindungsgemäßen Pyrido[2,3-b]pyrazine, bei denen die Substituenten R3 und/oder R4 die Reste $OX_3$, $SX_4$, $NX_5X_6$ sein sollen, sind beispielsweise nach dem Verfahren in Schema 3 bzw. einem entsprechenden, dem Fachmann bekannten Verfahren erhältlich.

Schema 3

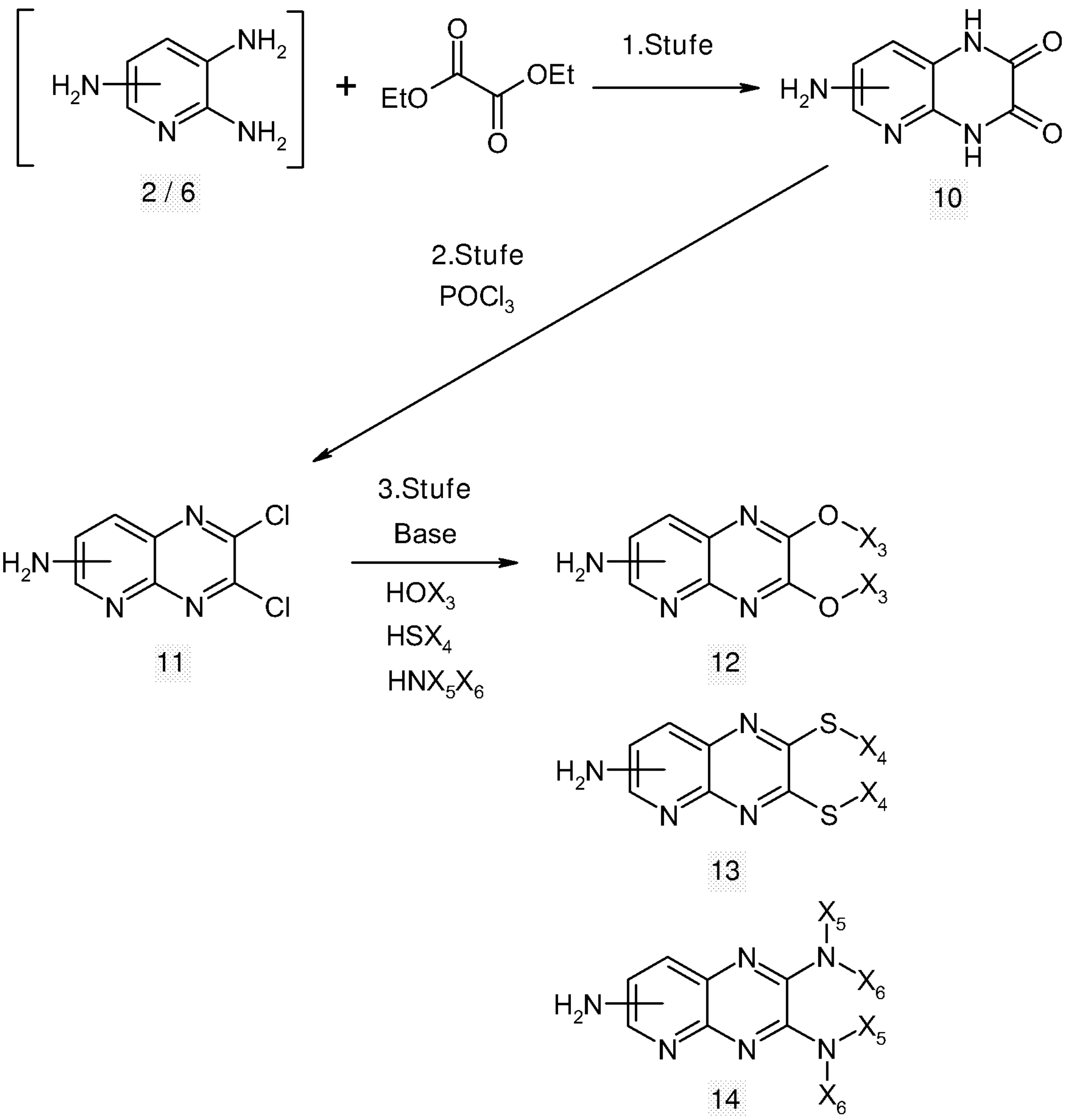


2 / 6 10 11 12 13 14

Für die oben dargestellte Vorstufe 11 können das Zwischenprodukt 9 aus Schema 2 oder auch das Zwischenprodukt 21 aus Schema 6 oder ein entsprechend substituiertes Zwischenprodukt verwendet werden.

Substituierte Vorstufen für ausgewählte Beispiele der erfindungsgemäßen Pyrido[2,3-b]pyrazine sind beispielsweise nach dem Verfahren in Schema 4 bzw. einem entsprechenden, dem Fachmann bekannten Verfahren erhältlich.

Schema 4

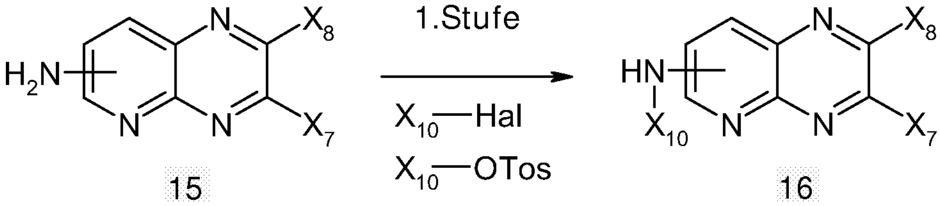


15 16

Die Umsetzung der Vorstufe 16 aus dem Schema 4 zu den erfindungsgemäßen substituierten Pyrido[2,3-b]pyrazinen kann beispielsweise nach den Verfahren in Schema 5 bzw. einem entsprechenden, dem Fachmann bekannten Verfahren erfolgen.

Schema 5

1.Stufe

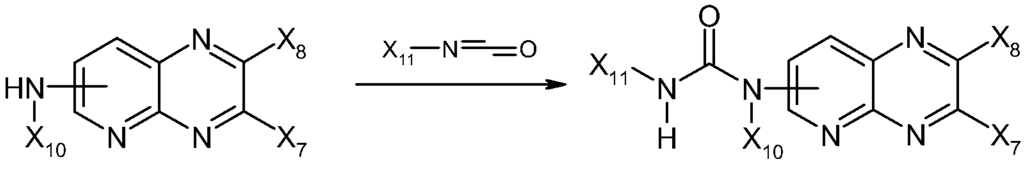

16 17

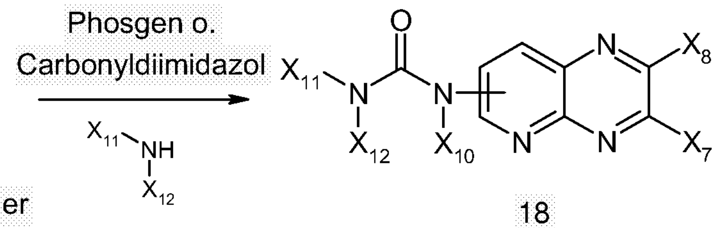


oder

18

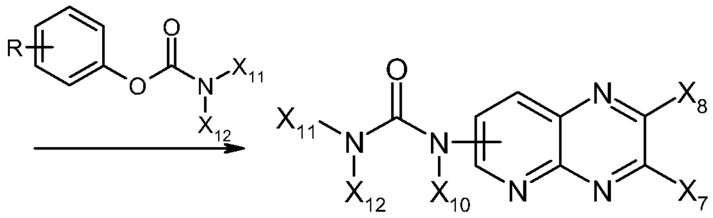

18

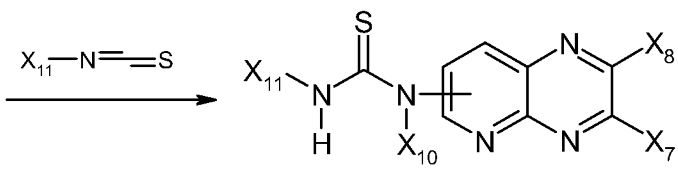

19

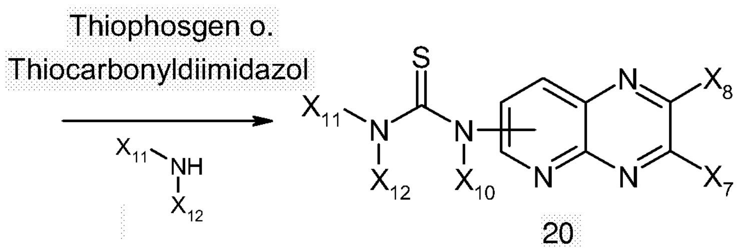


20

Für die oben dargestellte Vorstufe 16 kann auch das Zwischenprodukt 15 aus Schema 4 verwendet werden.

Ausgewählte Beispiele der erfindungsgemäßen Pyrido[2,3-b]pyrazine, bei denen die Substituenten R3 und/oder R4 ausgewählte Alkyl-, Aryl- oder Heteroaryl-Reste sein können, sind beispielsweise nach dem Verfahren in Schema 6 oder entsprechenden, dem Fachmann bekannten Verfahren erhältlich.

Schema 6

1.Stufe

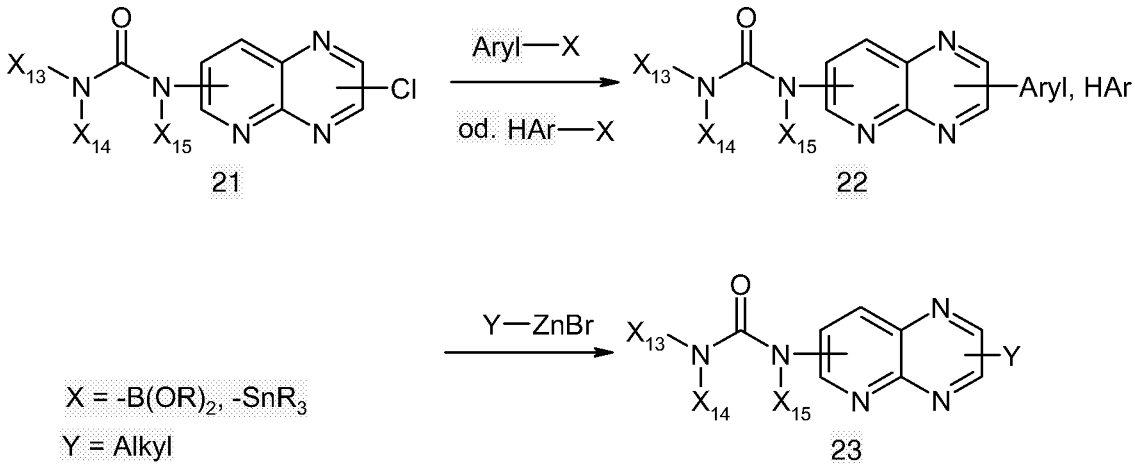


21 22 23

Bei der Herstellung der erfindungsgemäßen Aryl-/Heteroaryl-substituierten Pyridopyrazine kann für die oben dargestellte Vorstufe 21 auch das Zwischenprodukt 9 aus Schema 2 oder auch das Zwischenprodukt 11 aus Schema 3 oder ein entsprechend substituiertes Zwischenprodukt verwendet werden.

Ausgewählte Beispiele der erfindungsgemäßen Pyrido[2,3-b]pyrazine, bei denen die Substituenten R3 und/oder R4 ausgewählte O-, S-, N-substituierte Reste sein können, sind beispielsweise nach dem Verfahren in Schema 7 oder entsprechenden, dem Fachmann bekannten Verfahren erhältlich.

Schema 7

1.Stufe

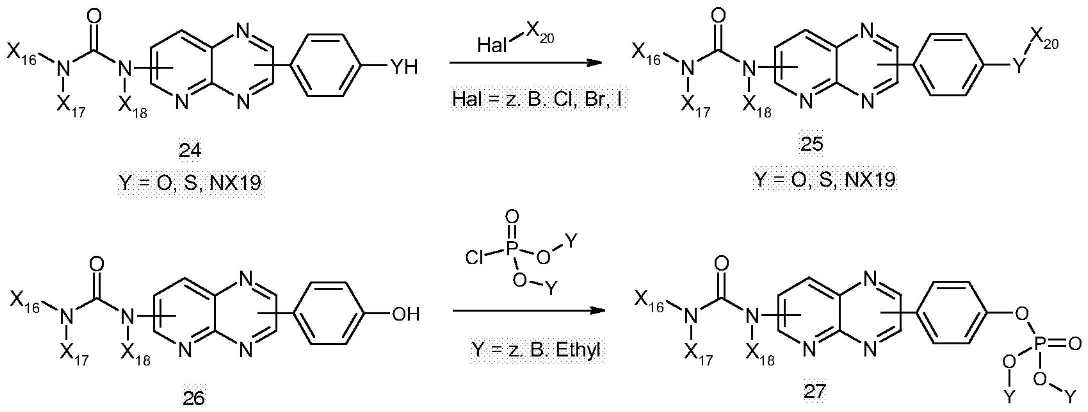


24 25 26 27

Die Spaltung der entsprechenden Phosphorsäurester kann nach an sich oder dem Fachmann bekannten Verfahrensweisen über literaturbekannte Methoden durchgeführt werden.

Die Ausgangsverbindungen und Zwischenstufen sind entweder im Handel erhältlich oder können nach an sich oder dem Fachmann bekannten Verfahrensweisen hergestellt werden. Die Edukte 4, 7, 9-16, 21, 24 und 26 stellen wertvolle Zwischenverbindungen für die Herstellung der erfindungsgemäßen Pyridopyrazine dar.

Für die Herstellung der Ausgangs-, Zwischenverbindungen und der erfindungsgemäßen Pyridopyrazine sei unter anderem auf folgende Primärliteratur verwiesen, deren Inhalt hiermit Bestandteil der Offenbarung der vorliegenden Anmeldung werden soll:

1) Houben-Weyl, Methoden der Organischen Chemie, Band 4/1 a, S. 343-350

2) Houben-Weyl, Methoden der Organischen Chemie, 4.Aufl., Band E 7b (Teil 2), S. 579; Degussa GB 1184848 (1970); S. Seko, et al. EP 735025 (1996)

3) D. Catarzi, et al.; J. Med. Chem. 1996, 1330-1336; J. K. Seydel, et al.; J. Med. Chem. 1994, 3016-3022

4) Houben-Weyl, Methods of Organic Chemistry, Volume E 9c, S.231-235

5) Houben-Weyl/Science of Synthesis, Volume 16, S. 1269

6) C. L. Leese, H. N. Rydon J. Chem. Soc. 1955, 303-309; T. S. Osdene, G. M. Timmis J. Chem. Soc. 1955, 2033-2035

7) W. He, et al. Bioorg. Med. Chem. Lett. 2003, 13, 3097-3100

8) M. S. A. El-Gaby, et al. Indian J. Chem. Sect. B 2001, 40, 195- 200; M. R. Myers, et al. Bioorg. Med. Chem. Lett. 2003, 13, 3091-3096 ; A. R. Renslo, et al. J. Amer. Chem. Soc. 1999, 121, 7459-7460; C. O. Okafor, et al. J. Heterocyclic Chem. 1983, 20, 199-203; C. R. Hopkins, et al. Tet. Lett. 2004, 45, 8631-8633

9) J. Yin, et al. Org. Lett. 2002, 4, 3481-3484 ; O. A. El-Sayed, et al. Arch. Pharm. 2002, 335, 403-410 ; C. Temple, et al. J. Med. Chem. 1992, 35, 988-993

10) A. M. Thompson, et al. J. Med. Chem. 2000, 43, 4200-4211; N. A. Dales, et al. Org. Lett. 2001, 2313-2316; G. Dannhardt, et al. Arch. Pharm. 2000, 267-274; G. S. Poindexter, et al. Bioorg. Med. Chem. 2004, 12, 507-521; J.-M. Receveur, et al. Bioorg. Med. Chem. Lett. 2004, 14, 5075-5080

11) G. Heinisch, et al. Arch. Pharm. 1997, 207-210; K. Matsuno, et al. J. Med. Chem. 2002, 45, 4513-4523; A. M. Papini, et al. J. Med. Chem. 2004, 47, 5224-5229

12) L. Mao, et al. Synthesis 2004, 15, 2535-2539; M. Darabantu, et al. Tetrahedron 2005, 61, 2897-2905; E. Ford, et al. Tet. Lett. 2000, 41, 3197-3198; T. Shiota, et al. J. Org. Chem. 1999, 64, 453-457

13) J. F. Miravet, et al. Org. Lett. 2005, 7, 4791-4794; A. L. Castelhano, et al. Bioorg. Med. Chem. Lett. 2005, 15, 1501-1504.

14) J. W. Huffmann, et al. Bioorg. Med. Chem. 2006, 14, 247-262; T. Liu, et al. Org. & Biomolecular Chem. 2005, 3, 1525-1533

Allgemeine Vorschriften zur Darstellung der erfindungsgemäßen Verbindungen:

Schema 1: 1. Stufe

2,6-Diamino-3-nitropyridin oder 2-Amino-3,5-dinitro-pyridin werden in einem geeigneten, inerten Lösungsmittel, wie beispielsweise Methanol, Ethanol, Dimethylformamid oder Dioxan, gelöst. Nach Zugabe eines Katalysators, beispielsweise Raney-Nickel, Palladium auf Kohle oder Platin(IV)dioxid, setzt man das Reaktionsgemisch unter eine Wasserstoff-Atmosphäre, wobei ein Druck zwischen 1 und 5 bar eingestellt wird. Man läßt das Reaktionsgemisch mehrere Stunden, beispielsweise 1-16 Stunden, in einem Temperaturbereich zwischen 20 °C und 60 °C reagieren. Nach beendeter Umsetzung filtriert man die unlöslichen Rückstände ab, wobei das Filtermedium beispielsweise aus Kieselgel, Celite oder handelsüblichen Glasfaserfiltern bestehen kann, und wäscht mit dem entsprechenden Lösungsmittel nach. Das Rohprodukt wird, in Lösung vorliegend, ohne weitere Aufreinigung für die nächste Umsetzung verwendet.

2. Stufe

Das 1,2-Dion-Derivat wird in einem geeigneten, inerten Lösungsmittel, beispielsweise Methanol, Ethanol, Dioxan, Toluol oder Dimethylformamid, vorgelegt. 2,3,6-Triaminopyridin oder 2,3,5-Triaminopyridin werden direkt nach der Reduktion als Lösung der Rohprodukte in einem der oben genannten Lösungsmittel zum vorgelegten 1,2-Dion gegeben, gegebenenfalls unter Zugabe einer Säure, wie z. B. Essigsäure oder einer Base, beispielsweise Kaliumhydroxid. Das Reaktionsgemisch läßt man in einem Temperaturbereich von 20 °C bis 80 °C einige Zeit, beispielsweise 20 Minuten bis 40 Stunden, reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Bei Verwendung von Dimethylformamid wird das Reaktionsgemisch in eine große Menge Wasser eingerührt und der ausgefallene Niederschlag abfiltriert bzw. die wässrige Phase mit einem

geeigneten organischen Lösungsmittel, wie z. B. Dichlormethan oder Ethylacetat, extrahiert und die organischen Phasen im Vakuum eingeengt. Die Reinigung des zurückbleibenden Rohproduktes erfolgt durch Umkristallisieren aus einem geeigneten Lösungsmittel, beispielsweise Dioxan, oder durch Säulen- bzw. Flash-Chromatographie an Kieselgel oder Aluminiumoxid. Als Laufmittel dient beispielsweise ein Gemisch aus Methanol und Dichlormethan.

Schema 2: 1. Stufe

Das Pyridopyrazin-on-Derivat 8 wird in einem geeigneten, inerten Lösungsmittel, beispielsweise Dimethylformamid, Dioxan oder Toluol, oder ohne Lösungsmittel vorgelegt. Ein Chlorierungsmittel, z. B. Phosphorylchlorid oder Thionylchlorid, wird bei Raumtemperatur zugegeben und das Reaktionsgemisch lässt man in einem Temperaturbereich von 20 °C bis 100 °C einige Zeit, beispielsweise 1 Stunde bis 24 Stunden, reagieren. Nach beendeter Umsetzung wird das Reaktionsgemisch auf Wasser gegossen und mit einer geeigneten wässrigen Base, beispielsweise Natronlauge, neutralisiert. Ein eventuell ausgefallener Niederschlag wird abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, oder die wässrige Phase wird mit einem geeigneten organischen Lösungsmittel, wie z. B. Dichlormethan oder Ethylacetat, extrahiert, und die organischen Phasen im Vakuum eingeengt. Die Reinigung des zurückbleibenden Rohproduktes erfolgt durch Umkristallisieren aus einem geeigneten Lösungsmittel, beispielsweise Dioxan oder Toluol, oder durch Säulen- bzw. Flash-Chromatographie an Kieselgel oder Aluminiumoxid. Als Laufmittel dient beispielsweise ein Gemisch aus Methanol und Dichlormethan.

Schema 3: 1. Stufe

2,3,6-Triaminopyridin oder 2,3,5-Triaminopyridin werden direkt nach der Reduktion als Lösung der Rohprodukte in einem der oben genannten Lösungsmittel vorgelegt. Nach Zugabe eines Oxalsäure-Derivates, wie z. B. Oxalsäurediethylester oder Oxalylchlorid, läßt man das Reaktionsgemisch, gegebenenfalls unter Zugabe einer Säure, wie z. B. Salzsäure, Schwefelsäure oder Eisessig, in einem Temperaturbereich von 20 °C bis 150 °C einige Zeit, beispielsweise 10 Minuten bis 24 Stunden, reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Base, wie z. B. Natronlauge, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Dioxan oder Toluol, oder durch Säulen- bzw. Flash-Chromatographie an Kieselgel oder Aluminiumoxid. Als Laufmittel dient beispielsweise ein Gemisch aus Methanol und Dichlormethan.

2.Stufe

Das Dion-Derivat 10 wird in einem geeigneten, inerten Lösungsmittel, beispielsweise Dimethylformamid, Dioxan oder Toluol, oder ohne Lösungsmittel vorgelegt. Ein Chlorierungsmittel, z. B. Phosphorylchlorid oder Thionylchlorid, wird bei Raumtemperatur zugegeben und das Reaktionsgemisch lässt man in einem Temperaturbereich von 20 °C bis 100 °C einige Zeit, beispielsweise 1 Stunde bis 24 Stunden, reagieren. Nach beendeter Umsetzung wird das Reaktionsgemisch auf Wasser gegossen und mit einer geeigneten wässrigen Base, beispielsweise Natronlauge, neutralisiert. Ein eventuell ausgefallener Niederschlag wird abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, oder die wässrige Phase wird mit einem geeigneten organischen Lösungsmittel, wie z. B. Dichlormethan oder Ethylacetat, extrahiert, und die organischen Phasen im Vakuum eingeengt. Die Reinigung des zurückbleibenden Rohproduktes erfolgt durch Umkristallisieren aus einem geeigneten Lösungsmittel, beispielsweise Dioxan oder Toluol, oder durch Säulen- bzw. Flash-Chromatographie an Kieselgel oder Aluminiumoxid. Als Laufmittel dient beispielsweise ein Gemisch aus Methanol und Dichlormethan.

3. Stufe

Die Zwischenstufe 11 kann mit einem entsprechenden Alkohol, Thiol oder Amin und gegebenenfalls mit einer geeigneten Base, vorzugsweise Natriumhydrid, Pyridin, Triethylamin, Kaliumcarbonat oder Natriummethanolat in Methanol, in einem geeigneten, inerten Lösungsmittel, wie beispielsweise Dimethylformamid, Dimethylsulfoxid, Methanol, Toluol, oder in einer Base als Lösungsmittel, wie z. B. Pyridin oder Triethylamin, oder ohne Lösungsmittel, umgesetzt

werden. Das Reaktionsgemisch lässt man einige Zeit, beispielsweise 30 Minuten bis 2 Tage, in einem Temperaturbereich zwischen 20 °C und 140 °C reagieren. Alternativ kann die Zwischenstufe 11 mit einem entsprechenden Amin und einem geeigneten Katalysator, wie z. B. Tris(dibenzylidenaceton)dipalladium(0) oder Tetrakis(triphenylphosphin)-palladium(0), und einem geeigneten Liganden, wie z. B. 2-(Dicyclohexylphosphanyl)biphenyl oder BINAP, und einer geeigneten Base, beispielsweise Natrium-tert.butanolat oder Kaliumcarbonat, in einem geeignetem Lösungsmittel, wie beispielsweise Toluol, Dioxan oder Dimethylformamid, umgesetzt werden. Das Reaktionsgemisch lässt man einige Zeit, beispielsweise 2 Stunden bis 30 Stunden, in einem Temperaturbereich zwischen 60 °C und 120 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Dioxan, Ethylacetat oder Toluol, oder durch Säulen- bzw. Flash-Chromatographie an Kieselgel oder Aluminium-oxid. Als Laufmittel dient beispielsweise ein Gemisch aus Methanol und Dichlormethan.

Schema 4: 1. Stufe

Die Zwischenstufe 15 kann mit einem entsprechenden, geeigneten Chlorid, Bromid, Iodid oder Tosylat und gegebenenfalls mit einer geeigneten Base, vorzugsweise Natriumhydrid, Pyridin, Triethylamin, Kaliumcarbonat oder Natriummethanolat in Methanol, in einem geeigneten, inerten Lösungsmittel, wie beispielsweise Dimethylformamid, Dimethylsulfoxid, Methanol oder in einer Base als Lösungsmittel, wie z. B. Pyridin oder Triethylamin, oder ohne Lösungsmittel, umgesetzt werden. Das Reaktionsgemisch lässt man einige Zeit, beispielsweise 1 Stunde bis 24 Stunden, in einem Temperaturbereich zwischen 20 °C und 150 °C reagieren. Alternativ kann die Zwischenstufe 15 mit einem entsprechenden Aryl-Bromid oder -Iodid und einem geeigneten Katalysator, wie z. B. Tris(dibenzylidenaceton)-dipalladium(0) oder Tetrakis(triphenylphosphin)-palladium(0), und einem geeigneten Liganden, wie z. B. 2-(Dicyclohexylphosphanyl)biphenyl oder BINAP, und einer geeigneten Base, beispielsweise, Kaliumcarbonat oder Natrium-tert.butanolat, in einem geeignetem Lösungsmittel, wie beispielsweise Toluol, Dioxan oder Dimethylformamid, umgesetzt werden. Das Reaktionsgemisch lässt man einige Zeit, beispielsweise 10 Stunden bis 30 Stunden, in einem Temperaturbereich zwischen 60 °C und 120 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. werden evtl. vorhandene Katalysatorreste abfiltriert mit dem entsprechenden Lösungsmittel nachgewaschen und das Lösungsmittel im Vakuum entfernt, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Toluol, oder durch Säulen- bzw. Flash-Chromatographie an Kieselgel oder Aluminiumoxid. Als Laufmittel dient beispielsweise ein Gemisch aus Methanol und Dichlormethan.

Schema 5: 1. Stufe

Im Anschluß an die Grundverfahren können in Folgereaktionen die nach dem Grundverfahren entstandenen Produkte in einer dem Fachmann bekannten Vorgehensweise zu erfindungsgemässen Folgeprodukten umgesetzt werden.

So kann, wenn das Produkt ein Derivat der Verbindung 17 gemäß Schema 5 sein soll, das Reaktionsprodukt 16 mit einem entsprechenden Isocyanat und gegebenenfalls einer geeigneten Base, vorzugsweise Natriumhydrid, Kaliumhexamethyldisilazid, Pyridin, Triethylamin oder Kaliumcarbonat, in einem geeigneten, inerten Lösungsmittel, wie beispielsweise Dimethylformamid, Dimethylsulfoxid, Acetonitril, Dichlormethan, 1,2-Dichlorethan oder Dioxan, oder in einer Base als Lösungsmittel, wie z. B. Pyridin oder Triethylamin, oder ohne Lösungsmittel, umgesetzt werden. Das Reaktionsgemisch läßt man mehrere Stunden, beispielsweise 1 - 24 Stunden, in einem Temperaturbereich zwischen 0 und 80 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure,

wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminium-oxid. Als Laufmittel dient beispielsweise ein Gemisch aus Methanol und Dichlormethan.

Oder es kann alternativ, wenn das Produkt ein Derivat der Verbindung 18 gemäß Schema 5 sein soll, das Reaktionsprodukt 16 mit Phosgen oder Carbonyldiimidazol und einem entsprechenden Amin in einem geeigneten, inerten Lösungsmittel, wie beispielsweise Dimethylformamid, Tetrahydrofuran, Toluol, Dichlormethan oder Acetonitril umgesetzt werden. Gegebenenfalls wird eine geeignete Base, vorzugsweise Pyridin, Natriumhydrogencarbonat, Triethylamin, N-Methyl-morpholin oder Natriumacetat verwendet. Das Reaktionsgemisch läßt man einige Zeit, beispielsweise 15 Minuten bis 24 Stunden, in einem Temperaturbereich zwischen 0 und 60 °C reagieren. Alternativ kann das Reaktionsprodukt 16 mit einem entsprechenden Amin-Phenyl-Carbamat-Reagenz und gegebenenfalls mit einer geeigneten Base, vorzugsweise Pyridin, Natriumcarbonat, Triethylamin oder Natriumhydrid, in einem geeigneten, inerten Lösungsmittel, wie beispielsweise Tetrahydrofuran, Dioxan, Dichlormethan, Dimethylformamid oder Acetonitril, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 1 Stunde bis 18 Stunden, in einem Temperaturbereich zwischen 0 °C und 120 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung 19 gemäß Schema 5 sein soll, das Reaktionsprodukt 16 mit einem entsprechenden Isothiocyanat und gegebenenfalls einer geeigneten Base, vorzugsweise Natriumhydrid, Triethylamin, Kaliumcarbonat oder Pyridin, in einem geeigneten, inerten Lösungsmittel, wie beispielsweise Dimethylformamid, Tetrahydrofuran, Aceton oder Toluol, oder in einer Base als Lösungsmittel, wie z. B. Pyridin oder Triethylamin, oder ohne Lösungsmittel, umgesetzt werden. Das Reaktionsgemisch läßt man einige Zeit, beispielsweise 30 Minuten bis 90 Stunden, in einem Temperaturbereich zwischen 0 und 115 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

Oder es kann alternativ, wenn das Produkt ein Derivat der Verbindung 20 gemäß Schema 5 sein soll, das Reaktionsprodukt 16 mit Thiophosgen oder Thiocarbonyldiimidazol und einem entsprechenden Amin in einem geeigneten, inerten Lösungsmittel, wie beispielsweise Dimethylformamid, Tetrahydrofuran, Toluol, Dichlormethan, Ethanol oder Acetonitril umgesetzt werden. Gegebenenfalls wird eine geeignete Base, vorzugsweise Pyridin, Natriumhydrogencarbonat, Kaliumcarbonat, Triethylamin oder Imidazol verwendet. Das Reaktionsgemisch läßt man mehrere Stunden, beispielsweise 1 bis 24 Stunden, in einem Temperaturbereich zwischen -10 und 80 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

Schema 6: 1. Stufe

Im Anschluß an die Grundverfahren können in Folgereaktionen die nach dem Grundverfahren entstandenen Produkte in einer dem Fachmann bekannten Vorgehensweise zu erfindungsgemässen Folgeprodukten umgesetzt werden.

So kann, wenn das Produkt ein Derivat der Verbindung 22 gemäß Schema 6 sein soll, das Reaktionsprodukt 21 mit entsprechenden Aryl/Heteroaryl-Boronsäure-Derivaten oder Aryl/Heteroaryl-Organozinn-Verbindungen und einem geeigneten Katalysator, wie z. B. $Pd(PPh_3)_4$, [1,1'-Bis(diphenylphosphino)ferrocen]-dichlor-palladium(II) oder $Pd_2(dba)_3$, und einer geeigneten Base, beispielsweise, Natriumcarbonat, Cäsiumcarbonat oder Triethylamin, in einem geeignetem Lösungsmittel, wie beispielsweise Dimethylformamid, Dimethylformamid/Wasser, Toluol, Acetonitril, Dimethoxyethan oder Dioxan, umgesetzt werden. Das Reaktionsgemisch lässt man einige Zeit, beispielsweise 6 Stunden bis mehrere Tage, in einem Temperaturbereich zwischen 60 °C und 120 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. werden evtl. vorhandene Katalysatorreste abfiltriert mit dem entsprechenden Lösungsmittel nachgewaschen und das Lösungsmittel im Vakuum entfernt, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

So kann, wenn das Produkt ein Derivat der Verbindung 23 gemäß Schema 6 sein soll, das Reaktionsprodukt 21 mit entsprechenden Alkyl-Zink-Halogeniden und einem geeigneten Katalysator, wie z. B. $Pd(PPh_3)_4$, [1,1'-Bis(diphenylphosphino)ferrocen]-dichlor-palladium(II) oder $PdCl_2(PPh_3)_2$ in einem geeignetem Lösungsmittel, wie beispielsweise Dimethylformamid, Tetrahydrofuran, Toluol, Dimethoxyethan oder Dioxan, umgesetzt werden. Das Reaktionsgemisch lässt man einige Zeit, beispielsweise 30 Minuten bis 48 Stunden, in einem Temperaturbereich zwischen Raumtemperatur und 120 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. werden evtl. vorhandene Katalysatorreste abfiltriert mit dem entsprechenden Lösungsmittel nachgewaschen und das Lösungsmittel im Vakuum entfernt, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Ethanol oder Ethylacetat, oder durch Säulen- bzw. Flash-Chromatographie an Kieselgel oder Aluminiumoxid. Als Laufmittel dient beispielsweise ein Gemisch aus Methanol und Dichlormethan.

Schema 7: 1. Stufe

Im Anschluß an die Grundverfahren können in Folgereaktionen die nach dem Grundverfahren entstandenen Produkte in einer dem Fachmann bekannten Vorgehensweise zu erfindungsgemässen Folgeprodukten umgesetzt werden.

So kann, wenn das Produkt ein Derivat der Verbindung 25 gemäß Schema 7 sein soll, das Reaktionsprodukt 24 z. B. mit einem entsprechenden Chlorid, Bromid oder Iodid und gegebenenfalls mit einer geeigneten Base, vorzugsweise Natriumhydrid, Pyridin, Triethylamin, Kaliumcarbonat oder Natriummethanolat in Methanol, in einem geeigneten, inerten Lösungsmittel, wie beispielsweise Dimethylformamid, Dimethylsulfoxid, Methanol, Dioxan, Tetrahydrofuran, Toluol, oder in einer Base als Lösungsmittel, wie z. B. Pyridin oder Triethylamin, oder ohne Lösungsmittel, umgesetzt werden. Das Reaktionsgemisch lässt man einige Zeit, beispielsweise 30 Minuten bis 2 Tage, in einem Temperaturbereich zwischen 0 °C und 140 °C reagieren. Alternativ kann eine A-mino-substituierte Zwischenstufe 24 z. B. mit einem entsprechenden Chlorid, Bromid oder Iodid und einem geeigneten Katalysator, wie z. B. Tris(dibenzylidenaceton)dipalladium(0) oder Tetrakis(triphenylphosphin)-palladium(0), und einem geeigneten Liganden, wie z. B. 2-(Dicyclohexylphosphanyl)biphenyl oder BINAP, und einer geeigneten Base, beispielsweise Natrium-tert.butanolat oder Kaliumcarbonat, in einem geeignetem Lösungsmittel, wie beispielsweise Toluol, Dioxan oder Dimethylformamid, umgesetzt werden. Das Reaktionsgemisch lässt man einige Zeit, beispielsweise 2 Stunden bis 30 Stunden, in einem Temperaturbereich zwischen 60 °C und 120 °C reagieren. Nach beendeter Umsetzung wird ein eventuell ausgefallener Niederschlag abfiltriert, wobei

das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Dioxan, Ethylacetat oder Toluol, oder durch Säulen- bzw. Flash-Chromatographie an Kieselgel oder Aluminium-oxid. Als Laufmittel dient beispielsweise ein Gemisch aus Methanol und Dichlormethan.

So kann, wenn das Produkt ein Derivat der Verbindung 27 gemäß Schema 7 sein soll, das Reaktionsprodukt 26 z. B. mit einem entsprechenden Chlorphosphorsäureester und gegebenenfalls mit einer geeigneten Base, vorzugsweise Natriumhydrid, Pyridin, Triethylamin, Kaliumcarbonat oder Lithiumdiisopropylamid, in einem geeigneten, inerten Lösungsmittel, wie beispielsweise Dimethylformamid, Dimethylsulfoxid, Methanol, Dioxan, Tetrahydrofuran, Toluol, oder in einer Base als Lösungsmittel, wie z. B. Pyridin oder Triethylamin, oder ohne Lösungsmittel, umgesetzt werden. Das Reaktionsgemisch lässt man einige Zeit, beispielsweise 1 Stunde bis 24 Stunden, in einem Temperaturbereich zwischen 0 °C und 100 °C reagieren. Nach beendeter Umsetzung wird ein eventuell ausgefallener Niederschlag abfiltriert, wobei das Filtermedium beispielsweise aus handelsüblichem Filterpapier bestehen kann, mit dem entsprechenden Lösungsmittel nachgewaschen und der zurückbleibende Feststoff im Vakuum getrocknet, bzw. wird das Reaktionsgemisch im Vakuum vom Lösungsmittel befreit. Alternativ kann das Reaktionsgemisch in eine große Menge Wasser eingerührt und der ausgefallene Niederschlag abfiltriert bzw. die wässrige Phase, nach Neutralisation mit einer geeigneten wässrigen Säure, wie z. B. Salzsäure, mit einem geeigneten organischen Lösungsmittel, wie z. B. Dichlormethan oder Ethylacetat, extrahiert und die organische Phase im Vakuum eingeengt werden. Die Reinigung des zurückbleibenden Rohproduktes erfolgt durch Umkristallisieren aus einem geeigneten Lösungsmittel, beispielsweise Dioxan, Ethylacetat oder Ethanol, oder durch Säulen- bzw. Flash-Chromatographie an Kieselgel oder Aluminiumoxid. Als Laufmittel dient beispielsweise ein Gemisch aus Methanol und Dichlormethan.

Unter einigen der genannten Reaktionsbedingungen können OH-, SH- und $NH_2$-Gruppen möglicherweise unerwünschte Nebenreaktionen eingehen. Es ist daher bevorzugt, diese mit Schutzgruppen zu versehen oder im Falle von $NH_2$ durch $NO_2$ zu ersetzen und nachfolgend die Schutzgruppe abzuspalten oder die $NO_2$-Gruppe zu reduzieren. So kann in Abwandlung der oben beschriebenen Verfahren in den Ausgangsverbindungen mindestens eine OH-Gruppe beispielsweise durch eine Benzyloxygruppe und/oder mindestens eine SH-Gruppe beispielsweise durch eine S-Benzylgruppe und/oder mindestens eine $NH_2$-Gruppe durch eine NH-Benzylgruppe oder durch eine $NO_2$-Gruppe ersetzt werden. Nachfolgend kann mindestens eine-vorzugsweise alle - Benzyloxygruppe/n oder NH-Benzylgruppe/n beispielsweise mit Wasserstoff und Palladium auf Kohle und/oder mindestens eine - vorzugsweise alle - S-Benzylgruppe/n beispielsweise mit Natrium in Ammoniak abgespalten und/oder mindestens eine - vorzugsweise alle - $NO_2$-Gruppe/n beispielsweise mit Wasserstoff und Raney-Nickel zu $NH_2$ reduziert werden.

Unter einigen der genannten Reaktionsbedingungen können OH-, $NH_2$- und COOH-Gruppen möglicherweise unerwünschte Nebenreaktionen eingehen. Es ist daher bevorzugt, Ausgangsverbindungen und Zwischenstufen, welche mindestens eine OH- und/oder mindestens eine $NH_2$- und/oder mindestens eine COOH-Gruppe enthalten, in entsprechende Carbonsäureester- und Carbonsäureamid-Derivate zu überführen. In Abwandlung der oben beschriebenen Verfahren können Ausgangsverbindungen und Zwischenstufen, welche mindestens eine OH-Gruppe besitzen, und/oder welche mindestens eine $NH_2$-Gruppe besitzen, durch Umsetzung mit einer aktivierten Carbonsäuregruppe, beispielsweise einer Carbonsäurechloridgruppe, in Carbonsäureester- bzw. Carbonsäureamid-Derivate überführt werden. In Abwandlung der oben beschriebenen Verfahren können Ausgangsverbindungen und Zwischenstufen, welche mindestens eine COOH-Gruppe besitzen, durch Umsetzung mit einem Aktivierungsmittel, wie beispielsweise Thionylchlorid oder Carbonyldiimidazol, und nachfolgender Umsetzung mit einem geeigneten Alkohol oder Amin in Carbonsäureester- bzw. Carbonsäureamid-Derivate überführt werden. Nachfolgend kann mindestens eine - vorzugsweise alle - Carbonsäureester- bzw. Carbonsäureamidgruppe/n in den Ausgangsverbindungen und Zwischenstufen beispielsweise mit verdünnten wässrigen Säuren bzw. Basen gespalten werden, um mindestens eine - vorzugsweise alle - OH-Gruppe/n und/oder $NH_2$-Gruppe/n und/oder COOH-Gruppe/n in Freiheit zu setzen.

Die Erfindung soll anhand der nachfolgenden Beispiele näher erläutert werden, ohne jedoch auf diese Beispiele beschränkt zu sein.

Beispiele

I. Herstellung von erfindungsgemäßen Verbindungen

Gemäß den allgemeinen Synthesevorschriften, denen die Syntheseschemata 1 - 7 zugrundeliegen, wurden die folgenden erfindungsgemäßen Verbindungen synthetisiert.

Die eingesetzten Vorstufen für die Herstellung der erfindungsgemäßen Verbindungen können - soweit nicht anderweitig beschreiben - nach dem Fachmann bekannten Verfahren synthetisiert werden.
Die eingesetzten Chemikalien und Lösungsmittel wurden kommerziell bei den herkömmlichen Anbietern erworben (Acros, Aldrich, Fluka, Lancaster, Maybridge, Merck, Sigma, TCI, etc.) oder synthetisiert.

Beispiel 1:

1-Ethyl-3-[3-(3-hydroxy-phenyl)- pyrido[2,3-b]pyrazin-6-yl]-harnstoff (Verbindung 151)

Herstellung von 1-(3-Chlor-pyrido[2,3-b]pyrazin-6-yl)-3-ethyl-harnstoff (Umsetzung gemäß Schema 5)

100 mg 3-Chlor-pyrido[2,3-b]pyrazin-6-yl-amin (0.55 mmol) wurden in 5 ml vorgetrocknetem Pyridin vorgelegt und 44 μL Ethylisocyanat (0.55 mmol) bei Raumtemperatur zugegeben. Man ließ das Gemisch 3h bei 75 °C rühren und fügte dann nochmal insgesamt 132 μL Ethylisocyanat (1.65 mmol) über 18 h in kleinen Portionen zum Reaktionsgemisch hinzu. Dann wurden die flüchtigen Bestandteile im Vakuum entfernt. Der resultierende Feststoff wurde über Säulenchromatographie an Kieselgel (Laufmittel Dichlormethan/Methanol) gereinigt. Man erhielt einen hellgelben Feststoff.
Für die Herstellung von 3-Chlor-pyrido[2,3-b]pyrazin-6-yl-amin sei hiermit auf folgende Literatur und die dem Fachmann bekannten Verfahren verwiesen:

1) T. S. Osdene, et al. *J. Chem. Soc.* 1955, 2033-2035 ; C. L. Leese, et al. *J. Chem.* Soc. 1955, 303-309

2) C. M. Atkinson, et al. *J. Chem. Soc.* 1956, 26-30 ; S. Goswami, et al. *Molecules* 2005, *10, 929-936*

Herstellung von 1-Ethyl-3-[3-(3-hydroxy-phenyl)-pyrido[2,3-b]pyrazin-6-yl]-harnstoff (Umsetzung gemäß Schema 6)

100 mg 1-(3-Chlor-pyrido[2,3-b]pyrazin-6-yl)-3-ethyl-harnstoff (0.40 mmol) wurden in einem DMF/Wasser-Gemisch unter Stickstoff als Schutzgas vorgelegt. Anschließend wurden 60.3 mg 3-Hydroxyphenyl-boronsäure (0.44 mmol), 126 mg Natriumcarbonat (1.19 mmol) und 23 mg Tetrakis(triphenylphosphin)palladium(0) (0.02 mmol) hinzugefügt. Das Reaktionsgemisch wurde 4h bei 90 °C gerührt. Zur Aufarbeitung wurde das Gemisch mit Wasser versetzt und der ausgefallene Feststoff mit Dichlormethan nachgewaschen. Das resultierende Rohprodukt wurde nochmals in warmen DCM gerührt, abfiltriert und getrocknet. Man erhielt einen gelben Feststoff.
Nach dieser Vorschrift wurden weitere erfindungsgemäße Verbindungen hergestellt: z.B. die Verbindungen 152, 153, 154, 208, 209 und 210.

Beispiel 2:

1-Ethyl-3-(3-o-tolylamino-pyrido[2,3-b]-pyrazin-6-yl)-harnstoff (Verbindung 160)

Herstellung von 1-Ethyl-3-(3-o-tolylamino-pyrido[2,3-b]-pyrazin-6-yl)-harnstoff (Umsetzung gemäß Schema 3)

55 mg 1-(3-Chlor-pyrido[2,3-b]pyrazin-6-yl)-3-ethyl-harnstoff (0.22 mmol), 60 mg o-Toluidin (0.56 mmol), 33 mg Natrium-tert.-butylat (0.33 mmol), 29 mg Tris(dibenzylidenaceton)dipalladium(0) (0.03 mmol) und 78 mg 2-(Dicyclohexylphosphanyl)biphenyl (0.22 mmol) wurden in 1.5 ml vorgetrocknetem Toluol in einem Mikrowellen-Reaktionsgefäß unter Stickstoff als Schutzgas vorgelegt. Das Reaktionsgemisch wurde in der Mikrowelle für 30 Minuten auf 100 °C (100 Watt) erhitzt. Danach wurden die flüchtigen Bestandteile im Vakuum entfernt und das Rohprodukt durch Säulenchromatographie an Kieselgel (Laufmittel Dichlormethan/Methanol) gereinigt. Man erhielt einen gelben Feststoff.
Nach dieser Vorschrift wurden weitere erfindungsgemäße Verbindungen hergestellt: z.B. die Verbindungen 161, 180, 211, 212 und 213.

Beispiel 3:

1-Ethyl-3-(3-phenoxy-pyrido[2,3-b]pyrazin-6-yl)-harnstoff (Verbindung 64)

Herstellung von 1-Ethyl-3-(3-phenoxy-pyrido[2,3-b]pyrazin-6-yl)-harnstoff (Umsetzung gemäß Schema 3)

19 mg Natriumhydrid (0.48 mmol) (60 %ige Suspension in Mineralöl) wurden in 10 ml vorgetrocknetem Dimethylformamid vorgelegt. Bei 0 °C wurden 37 mg Phenol (0.40 mmol) in wenig vorgetrocknetem Dimethylformamid gelöst zugegeben. Das Gemisch wurde bei 0 °C für 1h gerührt. Anschließend wurden 100 mg 1-(3-Chlor-pyrido[2,3-b]pyrazin-6-yl)-3-ethyl-harnstoff (0.40 mmol) in wenig vorgetrocknetem Dimethylformamid gelöst und bei 0 °C zugetropft. Das Reaktionsgemisch wurde über Nacht bei Raumtemperatur gerührt. Nach beendeter Reaktion wurde die Reaktionslösung auf Eiswasser gegeben und mit 1 N HCl neutralisiert. Das ausgefallene Rohprodukt wurde durch Säulenchromatographie an Kieselgel (Laufmittel Dichlormethan/Methanol) gereinigt. Man erhielt einen weißen Feststoff.

Nach dieser Vorschrift wurden weitere erfindungsgemäße Verbindungen hergestellt: z. B. die Verbindungen 67, 69, 73, 80 und 87.

Die Umsetzung mit Aminen kann mit Triethylamin als Base in Dioxan oder ohne Base in Dioxan durchgeführt werden. Nach dieser Methode wurden weitere erfindungsgemäße Verbindungen hergestellt: z. B. die Verbindungen 59, 62, 65, 81 und 88.

II. Physiko-chemische Charakterisierung

II.1 ESI MS-Daten zu ausgewählten Verbindungen

Tabelle 1: Neue Pyrido[2,3-b]pyrazin-Derivate mit zugehörigen MS-Daten gemäß der allgemeinen Formel (I)

| Verb. | Pyridopyrazin-Derivate | MS m/z ($M+H^+$) |
|---|---|---|
| 1 | 1-Allyl-3-(3-phenyl-pyrido[2,3-b]pyrazin-6-yl)-thioharnstoff | 322,0 |
| 17 | 1-(2-Morpholin-4-yl-ethyl)-3-(3-phenyl-pyrido[2,3-b]pyrazin-6-yl)-harnstoff | 379,1 |
| 29 | 1-[3-(4-Hydroxy-phenyl)-pyrido[2,3-b]pyrazin-6-yl]-3-prop-2-inyl-thioharnstoff | 336,2 |
| 31 | 1-[3-(4-Hydroxy-phenyl)-pyrido[2,3-b]pyrazin-6-yl]-3-((E)-propenyl)-thioharnstoff | 338,3 |
| 35 | 1-Phenethyl-3-(3-phenyl-pyrido[2,3-b]pyrazin-6-yl)-harnstoff | 370,2 |
| 36 | 1-(2,3-Di-pyridin-2-yl-pyrido[2,3-b]pyrazin-6-yl)-3-ethyl-harnstoff | 372,1 |
| 37 | 1-(2,3-Dimethyl-pyrido[2,3-b]pyrazin-6-yl)-3-ethyl-harnstoff | 246,2 |
| 38 | 1-[3-(4-Hydroxy-phenyl)-pyrido[2,3-b]pyrazin-6-yl]-3-methyl-harnstoff | 296,1 |
| 39 | 1 -Allyl-3-[3-(4-phenoxy-phenyl)-pyrido[2,3-b]pyrazin-6-yl]-harnstoff | 398,1 |
| 40 | Methansulfonsäure-4-[6-(3-allyl-ureido)-pyrido[2,3-b]pyrazin-3-yl]-phenyl-ester | 400,0 |
| 41 | 4-Dimethylamino-benzoesäure-4-[6-(3-allyl-ureido)-pyrido[2,3-b]pyrazin-3-yl]-phenyl-ester | 469,3 |
| 42 | Essigsäure-4-[6-(3-allyl-ureido)-pyrido[2,3-b]pyrazin-3-yl]-phenyl-ester | 364,1 |
| 43 | 1-Ethyl-3-[3-(4-hydroxy-phenyl)-pyrido[2,3-b]pyrazin-6-yl]-harnstoff | 310,2 |
| 44 | 1-[3-(4-Amino-phenyl)-pyrido[2,3-b]pyrazin-6-yl]-3-methyl-thioharnstoff | 311,1 |
| 45 | 1-Ethyl-3-(3-phenyl-pyrido[2,3-b]pyrazin-6-yl)-harnstoff | 294,2 |
| 46 | 1-Acetyl-1-ethyl-3-(3-phenyl-pyrido[2,3-b]pyrazin-6-yl)-harnstoff | 336,3 |
| 47 | 1-Allyl-3-[3-(4-fluoro-phenyl)-pyrido[2,3-b]pyrazin-6-yl]-harnstoff | 324,0 |
| 48 | 1,1-Diethyl-3-(3-phenyl-pyrido[2,3-b]pyrazin-6-yl)-harnstoff | 322,0 |
| 49 | 1-(2-Chlor-ethyl)-3-[3-(4-fluor-phenyl)-pyrido[2,3-b]pyrazin-6-yl]-harnstoff | 346,3 |
| 50 | 1-Ethyl-3-[3-(4-methoxy-phenyl)-pyrido[2,3-b]pyrazin-6-yl]-harnstoff | 324,2 |
| 51 | 1-(3-Phenyl-pyrido[2,3-b]pyrazin-6-yl)-3-propyl-harnstoff | 308,0 |
| 52 | [3-(3-Phenyl-pyrido[2,3-b]pyrazin-6-yl)-ureido]-essigsäure-ethylester | 352,0 |
| 53 | 1-(3-Chlor-pyrido[2,3-b]pyrazin-6-yl)-3-ethyl-harnstoff | 252,1 |
| 54 | 1-Ethyl-3-[3-(4-fluor-phenyl)-pyrido[2,3-b]pyrazin-6-yl]-harnstoff | 312,2 |

(fortgesetzt)

| Verb. | Pyridopyrazin-Derivate | MS m/z (M+H+) |
|---|---|---|
| 55 | 1-[3-(3-Chlor-4-fluor-phenyl)-pyrido[2,3-b]pyrazin-6-yl]-3-ethyl-harnstoff | 346,2 |
| 56 | 4-[6-(3-Ethyl-ureido)-pyrido[2,3-b]pyrazin-3-yl]-benzoesäure | 338,1 |
| 57 | N-{4-[6-(3-Ethyl-ureido)-pyrido[2,3-b]pyrazin-3-yl]-phenyl}-acetamide | 350,9 |
| 58 | 1-[3-(2,4-Difluor-phenyl)-pyrido[2,3-b]pyrazin-6-yl]-3-ethyl-harnstoff | 329,9 |
| 59 | 1-Ethyl-3-(3-morpholin-4-yl-pyrido[2,3-b]pyrazin-6-yl)-harnstoff | 303,1 |
| 60 | 1-Ethyl-3-[3-(4-methyl-piperazin-1-yl)-pyrido[2,3-b]pyrazin-6-yl]-harnstoff | 316,3 |
| 61 | 1-Ethyl-3-[3-(2-hydroxy-phenyl)-pyrido[2,3-b]pyrazin-6-yl]-harnstoff | 310,2 |
| 62 | 1-Ethyl-3-[3-(2-methoxy-ethylamino)-pyrido[2,3-b]pyrazin-6-yl]-harnstoff | 291,2 |
| 63 | 1-[3-(4-Chlor-3-trifluormethyl-phenyl)-pyrido[2,3-b]pyrazin-6-yl]-3-ethyl-harnstoff | 294,9 |
| 64 | 1-Ethyl-3-(3-phenoxy-pyrido[2,3-b]pyrazin-6-yl)-harnstoff | 310,2 |
| 65 | 1-[3-(Cyclopropylmethyl-amino)-pyrido[2,3-b]pyrazin-6-yl]-3-ethyl-harnstoff | 287,2 |
| 66 | 1-Ethyl-3-{3-[(pyridin-4-ylmethyl)-amino]-pyrido[2,3-b]pyrazin-6-yl}-harnstoff | 324,2 |
| 67 | 1-Ethyl-3-[3-(4-fluor-benzyloxy)-pyrido[2,3-b]pyrazin-6-yl]-harnstoff | 341,9 |
| 68 | 1-Ethyl-3-(3-pyridin-4-yl-pyrido[2,3-b]pyrazin-6-yl)-harnstoff | 295,3 |
| 69 | 1-Ethyl-3-[3-(pyridin-3-yloxy)-pyrido[2,3-b]pyrazin-6-yl]-harnstoff | 311,3 |
| 70 | 1-Ethyl-3-[3-(tetrahydro-furan-2-ylmethoxy)-pyrido[2,3-b]pyrazin-6-yl]-harnstoff | 318,2 |
| 71 | 1-Ethyl-3-[3-(4-morpholin-4-yl-phenyl)-pyrido[2,3-b]pyrazin-6-yl]-harnstoff | 379,4 |
| 72 | 1-Ethyl-3-(3-hydroxy-pyrido[2,3-b]pyrazin-6-yl)-harnstoff | 234,3 |
| 73 | 1-Ethyl-3-[3-(3-methoxy-phenylsulfanyl)-pyrido[2,3-b]pyrazin-6-yl]-harnstoff | 355,9 |
| 74 | 1-Ethyl-3-(3-quinolin-3-yl-pyrido[2,3-b]pyrazin-6-yl)-harnstoff | 345,1 |
| 75 | 1-(3-Benzo[b]thiophen-3-yl-pyrido[2,3-b]pyrazin-6-yl)-3-ethyl-harnstoff | 350,1 |
| 76 | 1-Ethyl-3-[3-(pyridin-2-ylsulfanyl)-pyrido[2,3-b]pyrazin-6-yl]-harnstoff | 327,3 |
| 77 | 1-[3-(4-Dimethylamino-phenyl)-pyrido[2,3-b]pyrazin-6-yl]-3-ethyl-harnstoff | 337,2 |
| 78 | N-{4-[6-(3-Ethyl-ureido)-pyrido[2,3-b]pyrazin-3-yl]-phenyl}-methansulfonamid | 387,3 |
| 79 | 1-Ethyl-3-[3-(1 H-pyrazol-4-yl)-pyrido[2,3-b]pyrazin-6-yl]-harnstoff | 284,2 |
| 80 | 1-(3-Benzylsulfanyl-pyrido[2,3-b]pyrazin-6-yl)-3-ethyl-harnstoff | 340,0 |
| 81 | 1-Ethyl-3-[3-(4-methyl-[1,4]diazepan-1-yl)-pyrido[2,3-b]pyrazin-6-yl]-harnstoff | 330,5 |
| 82 | 1-[3-(4-Amino-phenyl)-pyrido[2,3-b]pyrazin-6-yl]-3-ethyl-harnstoff | 309,4 |
| 83 | 1-(3-Amino-pyrido[2,3-b]pyrazin-6-yl)-3-ethyl-harnstoff | 233,3 |
| 84 | 1-Ethyl-3-pyrido[2,3-b]pyrazin-6-yl-harnstoff | 218,3 |
| 86 | 1-Ethyl-3-[3-(4-fluor-2-hydroxy-phenyl)-pyrido[2,3-b]pyrazin-6-yl]-harnstoff | 328,2 |
| 87 | 1-(3-Cyclopentyloxy-pyrido[2,3-b]pyrazin-6-yl)-3-ethyl-harnstoff | 302,3 |
| 88 | 1-Ethyl-3-[3-(4-hydroxy-piperidin-1-yl)-pyrido[2,3-b]pyrazin-6-yl]-harnstoff | 317,2 |

(fortgesetzt)

| Verb. | Pyridopyrazin-Derivate | MS m/z (M+H$^+$) |
|---|---|---|
| 89 | (3-Phenyl-pyrido[2,3-b]pyrazin-6-yl)-harnstoff | 266,2 |
| 90 | 1-Ethyl-3-(3-pyrimidin-5-yl-pyrido[2,3-b]pyrazin-6-yl)-harnstoff | 296,3 |
| 91 | 1-Ethyl-3-(3-pyridin-3-yl-pyrido[2,3-b]pyrazin-6-yl)-harnstoff | 295,3 |
| 92 | 1-Allyl-3-(3-phenyl-pyrido[2,3-b]pyrazin-6-yl)-harnstoff | 306,3 |
| 93 | 1-Ethyl-3-(3-piperazin-1-yl-pyrido[2,3-b]pyrazin-6-yl)-harnstoff | 302,3 |
| 94 | 1-[3-(3-Chlor-pyridin-4-ylmethyl)-pyrido[2,3-b]pyrazin-6-yl]-3-ethyl-harnstoff | 329,3 |
| 95 | 1-Ethyl-3-[3-(6-methoxy-pyridin-3-yl)-pyrido[2,3-b]pyrazin-6-yl]-harnstoff | 325,3 |
| 96 | 1-[3-(3,5-Dimethyl-isoxazol-4-yl)-pyrido[2,3-b]pyrazin-6-yl]-3-ethyl-harnstoff | 313,3 |
| 97 | 1-Ethyl-3-[3-(4-trifluormethoxy-phenyl)-pyrido[2,3-b]pyrazin-6-yl]-harnstoff | 378,3 |
| 98 | 1-Ethyl-3-(3-furan-2-yl-pyrido[2,3-b]pyrazin-6-yl)-harnstoff | 284,2 |
| 99 | 1-Ethyl-3-[3-(2-methoxy-pyridin-3-yl)-pyrido[2,3-b]pyrazin-6-yl]-harnstoff | 325,3 |
| 100 | 1-[3-(2,4-Dimethoxy-phenyl)-pyrido[2,3-b]pyrazin-6-yl]-3-ethyl-harnstoff | 353,9 |
| 101 | 1-Ethyl-3-[3-(1 H-pyrrol-2-yl)-pyrido[2,3-b]pyrazin-6-yl]-harnstoff | 383,3 |
| 102 | 1-Ethyl-3-[3-(6-morpholin-4-yl-pyridin-3-yl)-pyrido[2,3-b]pyrazin-6-yl]-harnstoff | 380,3 |
| 103 | 1-Benzyl-3-ethyl-1-(3-phenyl-pyrido[2,3-b]pyrazin-6-yl)-harnstoff | 384,0 |
| 104 | 1-[3-(2-Amino-phenyl)-pyrido[2,3-b]pyrazin-6-yl]-3-ethyl-harnstoff | 309,2 |
| 105 | 1-Ethyl-3-[3-(4-hydroxymethyl-phenyl)-pyrido[2,3-b]pyrazin-6-yl]-harnstoff | 324,2 |
| 106 | 1-[3-(3-Amino-phenyl)-pyrido[2,3-b]pyrazin-6-yl]-3-ethyl-harnstoff | 309,2 |
| 107 | 1-[3-(4-Acetyl-phenyl)-pyrido[2,3-b]pyrazin-6-yl]-3-ethyl-harnstoff | 336,1 |
| 108 | 1-[3-(2,3-Dihydro-benzofuran-5-yl)-pyrido[2,3-b]pyrazin-6-yl]-3-ethyl-harnstoff | 336,2 |
| 109 | 1-[3-(4-Benzyloxy-3-fluor-phenyl)-pyrido[2,3-b]pyrazin-6-yl]-3-ethyl-harnstoff | 418,3 |
| 110 | 1-(2,3-Dihydroxy-propyl)-3-(3-phenyl-pyrido[2,3-b]pyrazin-6-yl)-harnstoff | 340,0 |
| 111 | 1-Ethyl-3-[3-(3-formyl-4-methoxy-phenyl)-pyrido[2,3-b]pyrazin-6-yl]-harnstoff | 352,1 |
| 112 | 1-Ethyl-3-[3-(4-methansulfonyl-phenyl)-pyrido[2,3-b]pyrazin-6-yl]-harnstoff | 372,1 |
| 113 | N-{4-[6-(3-Ethyl-ureido)-pyrido[2,3-b]pyrazin-3-yl]-phenyl}-succinamidsäure | 409,3 |
| 114 | 1-Ethyl-3-(3-pyridin-4-yl-pyrido[2,3-b]pyrazin-6-yl)-harnstoff Methansulfonsäuresalz | 295,3 |
| 115 | 1-[3-(2,6-Dimethoxy-pyridin-3-yl)-pyrido[2,3-b]pyrazin-6-yl]-3-ethyl-harnstoff | 354,9 |
| 116 | 1-[3-(2,6-Dimethoxy-pyrimidin-4-yl)-pyrido[2,3-b]pyrazin-6-yl]-3-ethyl-harnstoff | 356,1 |
| 117 | 1-[3-(2,4-Dioxo-1,2,3,4-tetrahydro-pyrimidin-5-yl)-pyrido[2,3-b]pyrazin-6-yl]-3-ethyl-harnstoff | 328,4 |
| 118 | 1-Ethyl-3-[3-(1H-indol-5-yl)-pyrido[2,3-b]pyrazin-6-yl]-harnstoff | 333,0 |

(fortgesetzt)

| Verb. | Pyridopyrazin-Derivate | MS m/z (M+H+) |
|---|---|---|
| 119 | 1-(3-Chloro-pyrido[2,3-b]pyrazin-6-yl)-3-ethyl-thioharnstoff | 268,0 |
| 120 | 1-Ethyl-3-{3-[4-(2-methoxy-ethoxy)-phenyl]-pyrido[2,3-b]pyrazin-6-yl}-harnstoff | 368,4 |
| 121 | Acrylsäure-4-[6-(3-ethyl-ureido)-pyrido[2,3-b]-pyrazin-3-yl]-phenyl-ester | 364,1 |
| 122 | 1-[3-(4-Cyano-phenyl)-pyrido[2,3-b]pyrazin-6-yl]-3-ethyl-harnstoff | 319,1 |
| 123 | 1-(3-Benzo[1,2,5]oxadiazol-4-yl-pyrido[2,3-b]pyrazin-6-yl)-3-ethyl-harnstoff | 336,0 |
| 124 | 1-Ethyl-3-[3-(4-hydroxy-3-methoxy-phenyl)-pyrido[2,3-b]pyrazin-6-yl]-harnstoff | 340,0 |
| 125 | 1-[3-(2,6-Dimethoxy-phenyl)-pyrido [2,3-b]pyrazin-6-yl]-3-ethyl-harnstoff | 354,2 |
| 126 | 1-[3-(3-Acetyl-phenyl)-pyrido[2,3-b]pyrazin-6-yl]-3-ethyl-harnstoff | 336,2 |
| 127 | 1-Ethyl-3-[3-(3-morpholin-4-yl-phenyl)-pyrido[2,3-b]pyrazin-6-yl]-harnstoff | 379,2 |
| 128 | 1-[3-(6-Amino-pyridin-3-yl)-pyrido[2,3-b]pyrazin-6-yl]-3-ethyl-harnstoff | 310,2 |
| 129 | 3-{4-[6-(3-Ethyl-ureido)-pyrido[2,3-b]-pyrazin-3-yl]-phenoxy}-propionsäure | 382,3 |
| 130 | 1-Isopropyl-3-(3-phenyl-pyrido[2,3-b]pyrazin-6-yl)-harnstoff | 308,2 |
| 131 | 1-Cyclopentyl-3-(3-phenyl-pyrido[2,3-b]pyrazin-6-yl)-harnstoff | 333,9 |
| 132 | 1-Pentyl-3-(3-phenyl-pyrido[2,3-b]pyrazin-6-yl)-harnstoff | 336,2 |
| 133 | N-{4-[6-(3-Ethyl-ureido)-pyrido[2,3-b]pyrazin-3-yl]-phenyl}-acrylamid | 363,2 |
| 134 | 1-tert-Butyl-3-(3-phenyl-pyrido[2,3-b]pyrazin-6-yl)-harnstoff | 322,1 |
| 135 | 1-(2-Hydroxy-ethyl)-3-(3-phenyl-pyrido[2,3-b]pyrazin-6-yl)-harnstoff | 310,2 |
| 136 | 1-Cyclobutyl-3-(3-phenyl-pyrido[2,3-b]pyrazin-6-yl)-harnstoff | 319,8 |
| 137 | 1-Allyl-3-[3-(4-nitro-phenyl)-pyrido[2,3-b]pyrazin-7-yl]-thioharnstoff | 367,1 |
| 138 | 1-Ethyl-1-(Ethylcarbamoyl)-3-(3-phenyl-pyrido[2,3-b]pyrazin-6-yl)-harnstoff | 365,1 |
| 139 | [3-(4-Hydroxy-phenyl)-pyrido[2,3-b]pyrazin-6-yl]-carbaminsäure-allyl-ester | 323,0 |
| 140 | (3-Phenyl-pyrido[2,3-b]pyrazin-6-yl)-carbaminsäure-ethyl-ester | 295,3 |
| 141 | 1-Ethyl-3-[3-(4-phenyl-piperazin-1-yl)-pyrido[2,3-b]pyrazin-6-yl]-harnstoff | 378,3 |
| 142 | 1-(2,2-Dimethyl-[1,3]dioxolan-4-ylmethyl)-3-(3-phenyl-pyrido[2,3-b]pyrazin-6-yl)-harnstoff | 380,2 |
| 143 | 1,3-Bis-(3-phenyl-pyrido[2,3-b]pyrazin-6-yl)-harnstoff | 471,3 |
| 144 | N-(3-Phenyl-pyrido[2,3-b]pyrazin-6-yl)-acetamidin | 264,8 |
| 145 | 1-Ethyl-3-[3-(2-methoxy-pyridin-3-yl)-pyrido[2,3-b]pyrazin-6-yl]-thioharnstoff | 341,2 |
| 146 | 1-(4-Hydroxy-butyl)-3-(3-phenyl-pyrido[2,3-b]pyrazin-6-yl)-harnstoff | 338,2 |
| 147 | 1-(3-Hydroxy-propyl)-3-(3-phenyl-pyrido[2,3-b]pyrazin-6-yl)-harnstoff | 324,2 |
| 148 | 1-Ethyl-3-{3-[4-(3-morpholin-4-yl-propoxy)-phenyl]-pyrido[2,3-b]pyrazin-6-yl}-harnstoff | 437,3 |
| 149 | 1-Ethyl-3-[3-(4-hydroxy-phenyl)-pyrido[2,3-b]pyrazin-6-yl]-thioharnstoff | 326,1 |
| 150 | 1-Ethyl-3-(3-p-tolylamino-pyrido[2,3-b]pyrazin-6-yl)-harnstoff | 323,2 |
| 151 | 1-Ethyl-3-[3-(3-hydroxy-phenyl)- pyrido[2,3-b]pyrazin-6-yl]-harnstoff | 310,2 |

(fortgesetzt)

| Verb. | Pyridopyrazin-Derivate | MS m/z (M+H+) |
|---|---|---|
| 152 | 3-[6-(3-Ethyl-ureido)-pyrido[2,3-b]pyrazin-3-yl]-benzoesäure | 338,2 |
| 153 | 1-[3-(3,4-Dimethoxy-phenyl)-pyrido [2,3-b]pyrazin-6-yl]-3-ethyl-harnstoff | 354,1 |
| 154 | 1-[3-(4-Amino-3-methoxy-phenyl)-pyrido[2,3-b]pyrazin-6-yl]-3-ethyl-harnstoff | 339,1 |
| 155 | 3-[6-(3-Ethyl-ureido)-pyrido[2,3-b]-pyrazin-3-yl]-benzamid | 337,1 |
| 156 | 1-[3-(3-Chlor-4-hydroxy-5-methoxy-phenyl)-pyrido[2,3-b]pyrazin-6-yl]-3-ethyl-harnstoff | 374,2 |
| 157 | 1-Ethyl-3-(3-m-tolylamino-pyrido-[2,3-b]pyrazin-6-yl)-harnstoff | 323,3 |
| 158 | 1-Ethyl-3-[3-(4-methoxy-phenylamino)-pyrido[2,3-b]pyrazin-6-yl]-harnstoff | 339,0 |
| 159 | 1-[3-(4-Chloro-phenylamino)-pyrido-[2,3-b]pyrazin-6-yl]-3-ethyl-harnstoff | 343,1 |
| 160 | 1-Ethyl-3-(3-o-tolylamino-pyrido[2,3-b]-pyrazin-6-yl)-harnstoff | 323,2 |
| 161 | 1-Ethyl-3-[3-(pyridin-3-ylamino)-pyrido-[2,3-b]pyrazin-6-yl]-harnstoff | 310,2 |
| 162 | 1-Ethyl-3-[3-(4-ethyl-phenylamino)-pyrido[2,3-b]pyrazin-6-yl]-harnstoff | 337,2 |
| 163 | 1-Ethyl-3-[3-(3-methoxy-4-methyl-phenylamino)-pyrido[2,3-b]pyrazin-6-yl]-harnstoff | 353,3 |
| 164 | 1-Ethyl-3-[3-(4-hydroxy-phenylamino)-pyrido[2,3-b]pyrazin-6-yl]-harnstoff | 325,3 |
| 165 | 1-Ethyl-3-[3-(5-methyl-pyridin-2-yl-amino)-pyrido[2,3-b]pyrazin-6-yl]-harnstoff | 324,3 |
| 166 | 1-Ethyl-3-[3-(1-methyl-1 H-pyrazol-3-ylamino)-pyrido[2,3-b]pyrazin-6-yl]-harnstoff | 313,4 |
| 167 | 1-Ethyl-3-[3-(4-fluor-phenylamino)-pyrido[2,3-b]pyrazin-6-yl]-harnstoff | 327,4 |
| 168 | 1-(4-Hydroxy-butyl)-3-[3-(4-hydroxy-3-methoxy-phenyl)-pyrido[2,3-b]pyrazin-6-yl]-harnstoff | 384,3 |
| 169 | Phosphorsäure-mono-{4-[6-(3-ethyl-ureido)-pyrido-[2,3-b]pyrazin-3-yl]-2-methoxy-phenyl}-ester | 420,2 |
| 170 | 1-[3-(2-Chlor-4-hydroxy-phenyl)-pyrido[2,3-b]pyrazin-6-yl]-3-ethyl-harnstoff | 344,3 |
| 171 | 1-[3-(4-Chlor-2-methoxy-phenyl)-pyrido[2,3-b]pyrazin-6-yl]-3-ethyl-harnstoff | 358,1 |
| 172 | 1-[3-(3,5-Dimethyl-1 H-pyrazol-4-yl)-pyrido[2,3-b]pyrazin-6-yl]-3-ethyl-harnstoff | 312,2 |
| 173 | 1-Ethyl-3-[3-(1-methyl-1 H-pyrazol-4-yl)-pyrido[2,3-b]pyrazin-6-yl]-harnstoff | 298,4 |
| 174 | 1-[3-(5-Cyano-thiophen-2-yl)-pyrido[2,3-b]pyrazin-6-yl]-3-ethyl-harnstoff | 325,3 |
| 176 | 1-[3-(4-Hydroxy-3-methoxy-phenyl)-pyrido[2,3-b]pyrazin-6-yl]-3-(4-morpholin-4-yl-butyl)-harnstoff | 453,4 |
| 177 | 1-[3-(3-Chlor-4-methoxy-phenylamino)-pyrido[2,3-b]pyrazin-6-yl]-3-ethyl-harnstoff | 373,4 |
| 178 | 1-Ethyl-3-[3-(naphthalin-2-ylamino)-pyrido[2,3-b]pyrazin-6-yl]-harnstoff | 359,3 |
| 179 | 1-Ethyl-3-[3-(chinolin-3-ylamino)-pyrido[2,3-b]pyrazin-6-yl]-harnstoff | 360,3 |
| 180 | 1-[3-(3,5-Dimethoxy-phenylamino)-pyrido[2,3-b]pyrazin-6-yl]-3-ethyl-harnstoff | 369,3 |

(fortgesetzt)

| Verb. | Pyridopyrazin-Derivate | MS m/z (M+H⁺) |
|---|---|---|
| 181 | 1-Ethyl-3-[3-(pyrazin-2-ylamino)-pyrido[2,3-b]pyrazin-6-yl]-harnstoff | 311,3 |
| 182 | 1-Ethyl-3-[3-(3-isopropoxy-phenylamino)-pyrido[2,3-b]pyrazin-6-yl]-harnstoff | 367,2 |
| 183 | 1-Ethyl-3-[3-(4-hydroxy-3-methoxy-phenyl)-pyrido[2,3-b]pyrazin-6-yl]-harnstoff p-Toluolsulfonat | 340.3 (freie Base) |
| 184 | 1-[3-(2-Chlor-pyridin-4-ylamino)-pyrido[2,3-b]pyrazin-6-yl]-3-ethyl-harnstoff | 344,3 |
| 185 | 1-[3-(3,5-Dichlor-4-hydroxy-phenylamino)-pyrido[2,3-b]pyrazin-6-yl]-3-ethyl-harnstoff | 393,2 |
| 186 | 1-[3-(3,5-Dichlor-4-hydroxy-phenyl)-pyrido[2,3-b]pyrazin-6-yl]-3-ethyl-harnstoff | 369,2 |
| 187 | 1-[3-(3,4-Dimethoxy-phenylamino)-pyrido[2,3-b]pyrazin-6-yl]-3-ethyl-harnstoff | 369,2 |
| 188 | 1-Ethyl-3-[3-(3-hydroxy-4-methyl-phenylamino)-pyrido[2,3-b]pyrazin-6-yl]-harnstoff | 339,1 |
| 189 | 3-[6-(3-Ethyl-ureido)-pyrido[2,3-b]-pyrazin-3-ylamino]-5-trifluormethyl-benzoesäure | 421,3 |
| 190 | 1-Ethyl-3-[3-(6-methoxy-pyridin-3-ylamino)-pyrido[2,3-b]pyrazin-6-yl]-harnstoff | 340,3 |
| 191 | 1-[3-(3,5-Dimethyl-phenylamino)-pyrido[2,3-b]pyrazin-6-yl]-3-ethyl-harnstoff | 337,3 |
| 192 | 1-[3-(4-Cyano-phenylamino)-pyrido-[2,3-b]pyrazin-6-yl]-3-ethyl-harnstoff | 334,1 |
| 194 | 1-Ethyl-3-[3-(4-hydroxy-3-methoxy-phenyl)-pyrido[2,3-b]pyrazin-6-yl]-harnstoff Hydrochlorid | 340.1 (freie Base) |
| 195 | 1-Ethyl-3-(3-phenyl-pyrido[2,3-b]pyrazin-6-yl)-thioharnstoff | 310,2 |
| 196 | 1-(3-Chlor-pyrido[2,3-b]pyrazin-6-yl)-3-cyclohexyl-harnstoff | 306,2 |
| 197 | 1-Ethyl-3-[3-(4-hydroxy-3-methoxy-phenylamino)-pyrido[2,3-b]pyrazin-6-yl]-harnstoff | 355,3 |
| 198 | 1-Ethyl-3-[3-(4-hydroxy-3,5-dimethyl-phenyl)-pyrido[2,3-b]pyrazin-6-yl]-harnstoff | 338,3 |
| 199 | 3-Ethyl-1-phenethyl-1-(3-phenyl- pyrido[2,3-b]pyrazin-6-yl)-harnstoff | 398,2 |
| 200 | 1-Allyl-3-{3-[4-(tetrahydro-pyran-2-yloxy)-phenyl]-pyrido[2,3-b]pyrazin-6-yl}-thioharnstoff | 422,2 |
| 201 | 3-Ethyl-1-[3-(4-methoxy-phenyl)-pyrido[2,3-b]pyrazin-6-yl]-1-propyl-harnstoff | 366,3 |
| 202 | 3-Ethyl-1-(3-phenyl-pyrido[2,3-b]pyrazin-6-yl)-1-(2-piperidin-1-yl-ethyl)-harnstoff Hydrochlorid | 405.4 (freie Base) |
| 203 | N-{4-[6-(3-Ethyl-ureido)-pyrido[2,3-b]-pyrazin-3-yl]-phenyl}-2-phenyl-acetamid | 427,3 |
| 204 | 1-(4-Hydroxy-butyl)-3-(3-phenyl-pyrido[2,3-b]pyrazin-6-yl)-harnstoff Hydrochlorid | 338.3 (freie Base) |
| 205 | Essigsäure-4-[3-(3-phenyl-pyrido[2,3-b]pyrazin-6-yl)-ureido]-butyl ester | 380,4 |
| 206 | 1-(4-Amino-butyl)-3-(3-phenyl-pyrido[2,3-b]pyrazin-6-yl)-harnstoff | 337,3 |

(fortgesetzt)

| Verb. | Pyridopyrazin-Derivate | MS m/z (M+H+) |
|---|---|---|
| 207 | 1-(5-Hydroxy-pentyl)-3-(3-phenyl-pyrido[2,3-b]pyrazin-6-yl)-harnstoff | 352,3 |
| 208 | 3-[6-(3-Ethyl-ureido)-pyrido[2,3-b]-pyrazin-3-yl]-N-methyl-benzamid | 351,2 |
| 209 | 1-Ethyl-3-[3-(2-methoxy-5-methyl-phenyl)-pyrido[2,3-b]pyrazin-6-yl]-harnstoff | 338,1 |
| 210 | 1-Ethyl-3-(3-p-tolyl-pyrido[2,3-b]pyrazin-6-yl)-harnstoff | 308,2 |
| 211 | 1-Ethyl-3-[3-(methyl-p-tolyl-amino)-pyrido[2,3-b]pyrazin-6-yl]-harnstoff | 337,1 |
| 212 | 1-Ethyl-3-[3-(2-p-tolyl-ethylamino)-pyrido[2,3-b]pyrazin-6-yl]-harnstoff | 351,1 |
| 213 | 1-Ethyl-3-[3-(4-methyl-benzylamino)-pyrido[2,3-b]pyrazin-6-yl]-harnstoff | 337,2 |
| 214 | 1-Ethyl-3-[3-(3-fluor-4-methyl-phenyl-amino)-pyrido[2,3-b]pyrazin-6-yl]-harnstoff | 341,2 |
| 215 | 1-[3-(3,4-Dimethyl-phenylamino)-pyrido[2,3-b]pyrazin-6-yl]-3-ethyl-harnstoff | 337,1 |
| 216 | 1-Ethyl-3-[3-(4-isopropyl-phenylamino)-pyrido[2,3-b]pyrazin-6-yl]-harnstoff | 351,1 |
| 217 | 1-(4-Morpholin-4-yl-butyl)-3-(3-phenyl-pyrido[2,3-b]pyrazin-6-yl)-harnstoff | 407,3 |
| 218 | N-{4-[3-(3-Phenyl-pyrido[2,3-b]pyrazin-6-yl)-ureido]-butyl}-acetamid | 379,3 |
| 219 | 1-[3-(3-Amino-4-methyl-phenyl)-pyrido[2,3-b]pyrazin-6-yl]-3-ethyl-harnstoff | 323,3 |
| 220 | 1-[3-(3-Acetyl-2-fluor-phenyl)-pyrido[2,3-b]pyrazin-6-yl]-3-ethyl-harnstoff | 354,2 |
| 221 | 1-Ethyl-3-[3-(4-methoxy-3-methyl-phenyl)-pyrido[2,3-b]pyrazin-6-yl]-harnstoff | 338,3 |
| 222 | 1-[3-(6-Ethoxy-pyridin-3-yl)-pyrido[2,3-b]pyrazin-6-yl]-3-ethyl-harnstoff | 339,3 |
| 223 | 1-Ethyl-3-[3-(2-fluor-4-methyl-phenyl)-pyrido[2,3-b]pyrazin-6-yl]-harnstoff | 326,2 |
| 224 | 1-Ethyl-3-[3-(3-fluor-4-methoxy-phenyl)-pyrido[2,3-b]pyrazin-6-yl]-harnstoff | 342,1 |
| 225 | 1-Ethyl-3-[3-(2-fluor-3-methoxy-phenyl)-pyrido[2,3-b]pyrazin-6-yl]-harnstoff | 342,1 |
| 226 | 1-Ethyl-3-[3-(3,4,5-trimethoxy-phenyl)-pyrido[2,3-b]pyrazin-6-yl]-harnstoff | 384,2 |
| 227 | 1-[3-(3,5-Difluor-2-methoxy-phenyl)-pyrido[2,3-b]pyrazin-6-yl]-3-ethyl-harnstoff | 360,3 |
| 228 | 1-Ethyl-3-[3-(4-trifluormethyl-phenyl)-pyrido[2,3-b]pyrazin-6-yl]-harnstoff | 362,3 |
| 229 | 1-Ethyl-3-[3-(2,3,4-trimethoxy-phenyl)-pyrido[2,3-b]pyrazin-6-yl]-harnstoff | 384,3 |
| 230 | 1-[3-(3-Chlor-4-methoxy-phenyl)-pyrido[2,3-b]pyrazin-6-yl]-3-ethyl-harnstoff | 358,2 |
| 231 | 1-Ethyl-3-[3-(3-fluor-4-hydroxy-phenyl)-pyrido[2,3-b]pyrazin-6-yl]-harnstoff | 328,3 |
| 232 | 1-Ethyl-3-[3-(6-fluor-pyridin-3-yl)-pyrido[2,3-b]pyrazin-6-yl]-harnstoff | 313,3 |
| 233 | 1-[3-(2,4-Dimethyl-thiazol-5-yl)-pyrido-[2,3-b]pyrazin-6-yl]-3-ethyl-harnstoff | 329,4 |
| 234 | 1-Ethyl-3-[3-(2-methoxy-phenyl)-pyrido[2,3-b]pyrazin-6-yl]-harnstoff | 324,3 |
| 235 | 1-[3-(2-Chlor-pyridin-4-yl)-pyrido[2,3-b]pyrazin-6-yl]-3-ethyl-harnstoff | 329,3 |
| 236 | 1-[3-(5-Acetyl-thiophen-2-yl)-pyrido[2,3-b]pyrazin-6-yl]-3-ethyl-harnstoff | 342,3 |
| 237 | 1-[3-(5-Chlor-thiophen-2-yl)-pyrido[2,3-b]pyrazin-6-yl]-3-ethyl-harnstoff | 334,1 |

(fortgesetzt)

| Verb. | Pyridopyrazin-Derivate | MS m/z (M+H$^+$) |
|---|---|---|
| 238 | 1-Ethyl-3-[3-(3-methoxy-phenyl)-pyrido[2,3-b]pyrazin-6-yl]-harnstoff | 324,1 |
| 239 | 1-[3-(3-Brom-5-methoxy-phenyl)-pyrido[2,3-b]pyrazin-6-yl]-3-ethyl-harnstoff | 402,3 |
| 240 | 1-[3-(Benzothiazol-6-ylamino)-pyrido[2,3-b]pyrazin-6-yl]-3-ethyl-harnstoff | 366,3 |
| 241 | 1-Ethyl-3-[3-(4-methyl-3-trifluoromethyl-phenylamino)-pyrido[2,3-b]pyrazin-6-yl]-harnstoff | 391,4 |
| 242 | 1-[3-(3-Cyano-4-methyl-phenylamino)-pyrido[2,3-b]pyrazin-6-yl]-3-ethyl-harnstoff | 348,3 |
| 243 | 1-Ethyl-3-[3-(4-phenoxy-phenylamino)-pyrido[2,3-b]pyrazin-6-yl]-harnstoff | 401,3 |
| 244 | 1-[3-(4-Chlor-3-methyl-phenylamino)-pyrido[2,3-b]pyrazin-6-yl]-3-ethyl-harnstoff | 357,3 |
| 245 | 1-[3-(2-Chlor-4-methyl-phenylamino)-pyrido[2,3-b]pyrazin-6-yl]-3-ethyl-harnstoff | 357,1 |
| 246 | 1-Ethyl-3-[3-(3-trifluoromethyl-phenylamino)-pyrido[2,3-b]pyrazin-6-yl]-harnstoff | 377,4 |
| 247 | 1-[3-(2-Chlor-4-trifluormethoxy-phenylamino)-pyrido[2,3-b]pyrazin-6-yl]-3-ethyl-harnstoff | 427,3 |
| 248 | 1-[3-(4-Chlor-2-methoxy-5-methyl-phenylamino)-pyrido[2,3-b]pyrazin-6-yl]-3-ethyl-harnstoff | 387,4 |
| 249 | 1-Ethyl-3-[3-(4-methylsulfanyl-phenyl)-pyrido[2,3-b]pyrazin-6-yl]-harnstoff | 340,3 |
| 250 | 3-[6-(3-Ethyl-ureido)-pyrido[2,3-b]pyrazin-3-yl]-benzolsulfonamid | 373,3 |
| 251 | N-{4-[3-(3-Phenyl-pyrido[2,3-b]pyrazin-6-yl)-ureido]-butyl}-methansulfonamid | 415,3 |

11.2 NMR-Spektroskopische Daten und Schmelzpunkte zu ausgewählten Verbindungen

Verbindung 1: 1-Allyl-3-(3-phenyl-pyrido[2,3-b]pyrazin-6-yl)-thioharnstoff Schmelzpunkt: 239-240°C (Zers.)

$^1$H-NMR ($d_6$-DMSO): $\delta$ = 4.40 (m, 2H), 5.30 (d, 1 H), 5.60 (d, 1 H), 6.07-6.17 (m, 1 H), 7.55-7.70 (m, 4H), 8.35 (d, 2H), 8.45 (d, 1 H), 9.50 (s, 1 H), 11.35 (s, 1 H), 12.55 (m, 1 H).

Verbindung 2: 1-(2-Methyl-allyl)-3-(3-naphth-2-yl-pyrido[2,3-b]pyrazin-6-yl)-thioharn-stoff

Smp.: 239-240°C (Zers.)
$^1$H-NMR ($d_6$-DMSO): $\delta$ = 1.94 (s, 3H), 4.32 (m, 2H), 5.07 (s, 1 H), 5.28 (s, 1 H), 7.60-7.69 (m, 3H), 8.00-8.05 (m, 1H), 8.07-8.12 (m, 1H), 8.14 (d, 1H), 8.42-8.51 (m, 2H), 8.98 (s, 1 H), 9.68 (s, 1 H), 11.32 (s, 1 H), 12.78 (m, 1 H).

Verbindung 3: 1-[3-(4-Methoxy-phenyl)-pyrido[2,3-b]pyrazin-6-yl]-3-(2-methyl-allyl)-thio-harnstoff

Smp.: 251-252°C (Zers.)
$^1$H-NMR ($d_6$-DMSO): $\delta$ = 1.92 (s, 3H), 3.85 (s, 3H), 4.27-4.35 (m, 2H), 5.02 (s, 1H), 5.24 (s, 1 H), 7.15 (d, 2H), 7.58 (d, 1 H), 8.31 (d, 2H), 8.41 (d, 1 H), 9.46 (s, 1 H), 11.29 (s, 1 H), 12.68 (m, 1 H).

Verbindung 4: 1-(2-Methyl-allyl)-3-(3-phenyl-pyrido[2,3-b]pyrazin-6-yl)-thioharnstoff

Smp.: 225-226°C (Zers.)
$^1$H-NMR ($d_6$-DMSO): $\delta$ = 1.90 (s, 3H), 4.30-4.35 (m, 2H), 5.01 (s, 1H), 5.22 (s, 1H), 7.55-7.80 (m, 4H), 8.30-8.38 (m, 2H), 8.45 (d, 1 H), 9.52 (s, 1 H), 11.32 (s, 1 H), 12.65 (m, 1 H).

Verbindung 5: 1-Allyl-3-(3-naphth-2-yl-pyrido[2,3-b]pyrazin-6-yl)-thioharnstoff

Smp.: 242-243°C (Zers.)
$^1$H-NMR ($d_6$-DMSO): $\delta$ = 4.42 (m, 2H), 5.37 (d, 1 H), 5.65 (d, 1 H), 6.07-6.19 (m, 1 H), 7.57-7.68 (m, 3H), 7.97-8.05 (m, 1H), 8.07-8.19 (m, 2H), 8.40-8.52 (m, 2H), 8.99 (s, 1 H), 9.70 (s, 1 H), 11.36 (s, 1 H), 12.56 (t, 1 H).

Verbindung 6: 1-Allyl-3-[3-(4-methoxy-phenyl)-pyrido[2,3-b]pyrazin-6-yl]-thioharnstoff

Smp.: 240-241 °C (Zers.)
$^1$H-NMR ($d_6$-DMSO): $\delta$ = 3.87 (s, 3H), 4.36-4.42 (m, 2H), 5.32 (d, 1 H), 5.60 (d, 1 H), 6.06-6.16 (m, 1 H), 7.16 (d, 2H), 7.60 (d, 1 H), 8.32 (d, 2H), 8.42 (d, 1 H), 9.56 (s, 1 H), 11.29 (s, 1 H), 12.56 (m, 1 H).

Verbindung 7: 1-Allyl-3-[3-(4-hydroxy-phenyl)-pyrido[2,3-b]pyrazin-6-yl]-thioharnstoff-Hydrochlorid

Smp.: 160-161 °C (Zers.)
$^1$H-NMR ($d_6$-DMSO): $\delta$ = 4.36-4.43 (m, 2H), 5.31 (d, 1 H), 5.59 (d, 1 H), 6.05-6.16 (m, 1H), 6.97 (d, 2H), 7.57 (d, 1H), 8.20 (d, 2H), 8.40 (d, 1H), 9.41 (s, 1H), 10.17 (bs, 1H), 11.24 (s, 1 H), 12.56 (m, 1 H).

Verbindung 8: 1-(3-Naphth-2-yl-pyrido[2,3-b]pyrazin-6-yl)-3-(4-nitro-phenyl)-thioharnstoff

Smp.: 260-261 °C (Zers.)
$^1$H-NMR ($d_6$-DMSO): $\delta$ = 7.61-7.68 (m, 3H), 7.72 (d, 2H), 7.75 (d, 1H), 8.01-8.06 (m, 1 H), 8.16 (m, 2H), 8.26 (d, 2H), 8.53 (d, 1 H), 8.58 (d, 1 H), 9.04 (s, 1 H), 9.62 (s, 1 H), 9.76 (s, 1H), 11.81 (s, 1H).

Verbindung 9: 1-[3-(4-Methoxy-phenyl)-pyrido[2,3-b]pyrazin-6-yl]-3-(4-nitro-phenyl)-thio-harnstoff

Smp.: 250-251 °C (Zers.)
$^1$H-NMR ($d_6$-DMSO): $\delta$ = 3.85 (s, 3H), 7.17 (d, 2H), 7.71 (d, 2H), 8.21 (d, 2H), 8.22-8.27 (m, 1 H), 8.36-8.42 (m, 3H), 9.53 (s, 1 H), 9.65 (s, 1 H), 11.77 (s, 1 H).

Verbindung 10: 1-*tert*.Butyl-3-(3-phenyl-pyrido[2,3-b]pyrazin-6-yl)-thioharnstoff

Smp.: 227°C (Zers.)
$^1$H-NMR ($d_6$-DMSO): $\delta$ = 1.65 (s, 9H), 7.53-7.69 (m, 4H), 8.34 (d, 2H), 8.41 (d, 1H), 9.51 (s, 1 H), 10.98 (s, 1 H), 12.75 (s, 1 H).

Verbindung 11: 1-Cyclopropyl-3-(3-phenyl-pyrido[2,3-b]pyrazin-6-yl)-thioharnstoff

Smp.: 233-234°C
$^1$H-NMR ($d_6$-DMSO): $\delta$ = 0.70-0.80 (m, 2H), 0.91-1.00 (m, 2H), 3.20-3.28 (m, 1 H), 7.51-7.72 (m, 4H), 8.36 (d, 2H), 8.45 (d, 1 H), 9.52 (s, 1 H), 11.31 (s, 1 H), 12.45 (s, 1 H).

Verbindung 12: 1-Methyl-3-(3-phenyl-pyrido[2,3-b]pyrazin-6-yl)-thioharnstoff

Smp.: 253-254°C
$^1$H-NMR ($d_6$-DMSO): $\delta$ = 3.25 (s, 3H), 7.59-7.67 (m, 4H), 8.38 (d, 2H), 8.46 (d, 1H), 9.52 (s, 1H), 11.31 (s, 1H), 12.10 (s, 1H).

Verbindung 13: 1-Benzyl-3-(3-phenyl-pyrido[2,3-b]pyrazin-6-yl)-thioharnstoff

Smp.: 232-233°C
$^1$H-NMR ($d_6$-DMSO): $\delta$ = 4.96 (m, 2H), 7.37-7.48 (m, 3H), 7.54-7.67 (m, 6H), 8.32 (d, 2H), 8.47 (d, 1 H), 9.52 (s, 1 H), 11.43 (s, 1 H), 12.91 (s, 1 H).

Verbindung 14: 1-(4-Fluoro-phenyl)-3-(3-phenyl-pyrido[2,3-b]pyrazin-6-yl)-thioharnstoff

Smp.: 225-226°C
$^1$H-NMR ($d_6$-DMSO): $\delta$ = 7.33 (m, 2H), 7.57-7.65 (m, 3H), 7.70-7.81 (m, 3H), 8.34 (d, 2H), 8.54 (d, 1 H), 9.57 (s, 1 H), 11.62 (s, 1 H).

Verbindung 15: 1-(3-Phenyl-pyrido[2,3-b]pyrazin-6-yl)-3-p-tolyl-harnstoff

Smp.: 298-299°C
$^1$H-NMR ($d_6$-DMSO): δ = 2.29 (s, 3H), 7.20 (d, 2H), 7.52 (d, 2H), 7.59-7.67 (m, 3H), 7.80 (d, 1 H), 8.38 (d, 2H), 8.44 (d, 1 H), 9.59 (s, 1 H), 10.36 (s, 1 H), 11.46 (s, 1 H).

Verbindung 16: 1-(4-Chloro-3-trifluoromethyl-phenyl)-3-(3-phenyl-pyrido[2,3-b]pyrazin-6-yl)-harnstoff

Smp.: 250°C
$^1$H-NMR ($d_6$-DMSO): δ = 7.58-7.67 (m, 3H), 7.74 (d, 1H), 7.80 (d, 1H), 7.87 (d, 1H), 8.21 (s, 1 H), 8.39 (d, 2H), 8.48 (d, 1 H), 9.53 (s, 1 H), 10.55 (s, 1 H), 11.82 (s, 1 H).

Verbindung 17: 1-(2-Morpholin-4-yl-ethyl)-3-(3-phenyl-pyrido[2,3-b]pyrazin-6-yl)-harnstoff

Smp.: 226°C
$^1$H-NMR ($d_6$-DMSO): δ = 2.45-2.67 (m, 6H), 3.40-3.48 (m, 2H), 3.60-3.69 (m, 4H), 7.55-7.70 (m, 4H), 8.30-8.40 (m, 3H), 9.29 (s, 1 H), 9.42 (s, 1 H), 10.18 (s, 1 H).

Verbindung 18: 1-Cyclohexyl-3-(3-phenyl-pyrido[2,3-b]pyrazin-6-yl)-thioharnstoff

Smp.: 230-232°C

$^1$H-NMR ($d_6$-DMSO): δ = 1.50-1.75 (m, 6H), 1.80-2.00 (m, 4H), 7.55-7.70 (m, 4H), 8.37 (d, 2H), 8.45 (d, 1 H), 9.55 (s, 1 H), 11.20 (s, 1 H), 12.80 (s, 1 H).

Verbindung 19: 1-Isopropyl-3-(3-phenyl-pyrido[2,3-b]pyrazin-6-yl)-thioharnstoff

Smp.: 229-230°C
$^1$H-NMR ($d_6$-DMSO): δ = 1.40 (d, 6H), 4.40-4.50 (m, 1H), 7.58-7.66 (m, 4H), 8.36 (d, 2H), 8.44 (d, 1 H), 9.52 (s, 1 H), 11.20 (s, 1 H), 12.48 (s, 1 H).

Verbindung 20: 1-Furan-2-ylmethyl-3-(3-phenyl-pyrido[2,3-b]pyrazin-6-yl)-thioharnstoff

Smp.: 250°C (Zers.)
$^1$H-NMR ($d_6$-DMSO): δ = 4.95 (s, 2H), 6.55 (m, 1H), 6.68 (d, 1H), 7.59-7.68 (m, 4H), 7.74 (d, 1H), 8.37 (d, 2H), 8.48 (d, 1H), 9.55 (s, 1H), 11.45 (s, 1H), 12.83 (s, 1H).

Verbindung 21: 1-Cyclopropyl-3-(3-phenyl-pyrido[2,3-b]pyrazin-6-yl)-harnstoff

Smp.: 158-160 °C
$^1$H-NMR ($d_6$-DMSO): δ = 0.52-0.60 (m, 2H), 0.72-0.82 (m, 2H), 2.70-2.79 (m, 1 H), 7.57-7.65 (m, 3H), 7.71 (d, 1H), 8.34 (d, 2H), 8.38 (d, 1H), 9.21 (s, 1H), 9.46 (s, 1H), 10.12 (s, 1 H).

Verbindung 22: 1-Methyl-3-[3-(4-nitro-phenyl)-pyrido[2,3-b]pyrazin-6-yl]-thioharnstoff

Smp.: 270°C
$^1$H-NMR ($d_6$-DMSO): δ = 3.25 (s, 3H), 7.70 (d, 1H), 8.44 (d, 2H), 8.50 (d, 1H), 8.64 (d, 2H), 9.64 (s, 1 H), 11.38 (s, 1 H), 12.03 (s, 1 H).

Verbindung 23: 1-[3-(4-Hydroxy-phenyl)-pyrido[2,3-]pyrazin-6-yl]-3-methyl-thioharnstoff

Smp.: 282°C
$^1$H-NMR ($d_6$-DMSO): δ = 3.25 (s, 3H), 6.98 (d, 2H), 7.57 (d, 1 H), 8.26 (d, 2H), 8.40 (d, 1 H), 9.45 (s, 1 H), 10.18 (s, 1 H), 11.25 (s, 1 H), 12.10 (s, 1 H).

Verbindung 24: 1-Allyl-3-[3-(4-nitro-phenyl)-pyrido[2,3-b]pyrazin-6-yl]-thioharnstoff

Smp.: 244°C (Zers.)

$^1$H-NMR ($d_6$-DMSO): δ = 4.40 (s, 2H), 5.36 (d, 1 H), 5.59 (d, 1 H), 6.08-6.15 (m, 1 H), 7.71 (d, 1 H), 8.46 (d, 2H), 8.51 (d, 1 H), 8.60 (d, 2H), 9.64 (s, 1 H), 11.45 (s, 1 H), 12.51 (t, 1H).

Verbindung 25: 1-Allyl-3-[3-(4-hydroxy-phenyl)-pyrido[2,3-b]pyrazin-6-yl]-harnstoff

Smp.: 240 °C (Zers.)
$^1$H-NMR ($d_6$-DMSO): δ = 3.98 (s, 2H), 5.19 (d, 1 H), 5.37 (d, 1 H), 5.96-6.05 (m, 1 H), 6.97 (d, 2H), 7.59 (d, 1H), 8.22 (d, 2H), 8.33 (d, 1H), 9.38 (s, 1H), 9.45 (s, 1H), 10.13 (s, 1 H), 10.18 (s, 1H).

Verbindung 26: 4-[6-(3-Allyl-thioureido)-pyrido[2,3-b]pyrazin-3-yl]-benzoesäureethyl-ester

Smp.: 223-224°C
$^1$H-NMR ($d_6$-DMSO): δ = 1.39 (t, 3H), 4.35-4.42 (m, 4H), 5.35 (d, 1 H), 5.60 (d, 1H), 6.08-6.15 (m, 1H), 7.68 (d, 1H), 8.17 (d, 2H), 8.47 (d, 2H), 8.50 (d, 1H), 9.60 (s, 1H), 11.40 (s, 1 H), 12.52 (t, 1 H).

Verbindung 27: 1-Allyl-3-[3-(3-hydroxy-phenyl)-pyrido[2,3-b]pyrazin-6-yl]-thioharnstoff

Smp.: 205°C (Zers.)
$^1$H-NMR ($d_6$-DMSO): δ = 4.41 (s, 2H), 5.33 (d, 1 H), 5.58 (d, 1 H), 6.07-6.15 (m, 1 H), 6.99 (d, 1 H), 7.42 (t, 1 H), 7.64 (d, 1 H), 7.72 (s, 1 H), 7.77 (d, 1 H), 8.46 (d, 1 H), 9.45 (s, 1 H), 9.80 (s, 1 H), 11.37 (s, 1 H), 12.55 (s, 1 H).

Verbindung 28: 1-Allyl-3-(3-benzo[1,3]dioxol-5-yl-pyrido[2,3-b]pyrazin-6-yl)-thioharnstoff

Smp.: 218-220°C (Zers.)
$^1$H-NMR ($d_6$-DMSO): δ = 4.40 (s, 2H), 5.31 (d, 1 H), 5.60 (d, 1 H), 6.08-6.20 (m, 3H), 7.16 (d, 1 H), 7.61 (d, 1 H), 7.90 (s, 1 H), 7.96 (d, 1 H), 8.43 (d, 1 H), 9.49 (s, 1 H), 11.34 (s, 1 H), 12.58 (s, 1 H).

Verbindung 29: 1-[3-(4-Hydroxy-phenyl)-pyrido[2,3-b]pyrazin-6-yl]-3-prop-2-inylthio-harnstoff

Smp.: 350 °C (Zers.)
$^1$H-NMR ($d_6$-DMSO): δ = 2.09 (s, 1 H), 2.44 (s, 2H), 6.99 (d, 2H), 7.19 (s, 1 H), 7.44 (s, 1 H), 8.24 (d, 2H), 8.26 (d, 1 H), 9.29 (s, 1 H), 10.08 (s, 1 H), 11.81 (s, 1 H).

Verbindung 30: 1-Allyl-3-[3-(4-hydroxy-phenyl)-pyrido[2,3-b]pyrazin-6-yl]-thioharnstoff

Smp.: 230 °C (Zers.)
$^1$H-NMR ($d_6$-DMSO): δ = 4.40 (s, 2H), 5.34 (d, 1 H), 5.60 (d, 1 H), 6.07-6.15 (m, 1 H), 6.98 (d, 2H), 7.58 (d, 1H), 8.24 (d, 2H), 8.42 (d, 1H), 9.45 (s, 1H), 10.19 (s, 1H), 11.34 (s, 1 H), 12.60 (s, 1 H).

Verbindung 31: 1-[3-(4-Hydroxy-phenyl)-pyrido[2,3-b]pyrazin-6-yl]-3-((E)-propenyl)-thio-harnstoff

$^1$H-NMR ($d_6$-DMSO): δ = 2.12 (d, 3H), 5.17 (m, 1H), 6.96 (d, 2H), 7.22-7.26 (m, 1H), 7.59 (d, 1H), 8.25 (d, 2H), 8.45 (d, 1H), 9.48 (s, 1H), 10.20 (s, 1H), 11.56 (s, 1H), 14.67 (s, 1 H).

Verbindung 32: 1-Allyl-3-[2,3-bis-(4-hydroxy-phenyl)-pyrido[2,3-b]pyrazin-6-yl]-thioharn-stoff

Smp.: 270 °C (Zers.)
$^1$H-NMR ($d_6$-DMSO): δ = 4.40 (s, 2H), 5.25 (d, 1 H), 5.50 (d, 1 H), 6.02-6.13 (m, 1 H), 6.74 (d, 2H), 6.76 (d, 2H), 7.31 (d, 2H), 7.36 (d, 2H), 7.62 (d, 1 H), 8.42 (d, 1 H), 9.78 (s, 1 H), 9.85 (s, 1 H), 11.30 (s, 1 H), 12.47 (s, 1 H).

Verbindung 33: 1-[2,3-Bis-(4-hydroxy-phenyl)-pyrido[2,3-b]pyrazin-6-yl]-3-((E)-propenyl)-thioharnstoff

Smp.: 240 °C (Zers.)
$^1$H-NMR ($d_6$-DMSO): δ = 2.05 (d, 3H), 5.10-5.18 (m, 1H), 6.74 (d, 2H), 6.76 (d, 2H), 7.20-7.26 (m, 1 H), 7.34 (d, 2H), 7.39 (d, 2H), 7.63 (d, 1 H), 8.45 (d, 1 H), 9.79 (s, 1 H), 9.89 (s, 1 H), 11.55 (s, 1 H), 14.56 (d, 1 H).

Verbindung 34: 1-Allyl-3-[2-(4-hydroxy-phenyl)-pyrido[2,3-b]pyrazin-6-yl]-thioharnstoff

Smp.: 260 °C (Zers.)
$^1$H-NMR ($d_6$-DMSO): $\delta$ = 4.40 (s, 2H), 5.28 (d, 1H), 5.48 (d, 1H), 6.03-6.12 (m, 1H), 6.96 (d, 2H), 7.66 (d, 1H), 8.16 (d, 2H), 8.43 (d, 1H), 9.52 (s, 1H), 10.06 (s, 1H), 11.31 (s, 1 H), 12.40 (s, 1 H).

Verbindung 35: 1-Phenethyl-3-(3-phenyl-pyrido[2,3-]pyrazin-6-yl)-harnstoff

Smp.: 250 °C (Zers.)
$^1$H-NMR ($d_6$-DMSO): $\delta$ = 2.88-2.95 (m, 2H), 3.52-3.60 (m, 2H), 7.18 (t, 1 H), 7.28 (t, 2H), 7.42 (d, 2H), 7.58-7.68 (m, 4H), 8.37 (d, 3H), 9.25 (s, 1H), 9.48 (s, 1H), 10.18 (s, 1 H).

Verbindung 36: 1-(2,3-Di-pyridin-2-yl-pyrido[2,3-b]pyrazin-6-yl)-3-ethyl-harnstoff

Smp.: 236-237 °C
$^1$H-NMR ($d_6$-DMSO): $\delta$ = 1.13-1.22 (m, 3H), 3.28-3.39 (m, 2H), 3.60-3.69 (m, 4H), 7.31-7.39 (m, 2H), 7.79 (d, 1 H), 7.91-7.99 (m, 4H), 8.26 (d, 1 H), 8.29 (d, 1 H), 8.47 (d, 1 H), 9.08 (s, 1 H), 10.20 (s, 1 H).

Verbindung 37: 1-(2,3-Dimethyl-pyrido[2,3-b]pyrazin-6-yl)-3-ethyl-harnstoff

Smp.: 246-248 °C
$^1$H-NMR ($d_6$-DMSO): $\delta$ = 1.17 (t, 3H), 2.64 (s, 3H), 2.67 (s, 3H), 3.24-3.40 (m, 2H), 7.55 (d, 1 H), 8.24 (d, 1 H), 9.14 (s, 1 H), 9.91 (s, 1 H).

Verbindung 64: 1-Ethyl-3-(3-phenoxy-pyrido[2,3-b]pyrazin-6-yl)-harnstoff

Smp.: 256-258 °C
$^1$H-NMR ($d_6$-DMSO): $\delta$ = 1.09 (t, 3H), 3.15 - 3.22 (m, 2H), 7.31 - 7.38 (m, 3H), 7.48-7.53 (m, 2H), 7.72 (d, 1 H), 8.32 (d, 1 H), 8.34 (bs, 1 H), 8.71 (s, 1 H), 9.90 (s, 1 H).

Verbindung 85: 1-Ethyl-3-(3-imidazol-1-yl-pyrido[2,3-b]pyrazin-6-yl)-harnstoff

$^1$H-NMR ($d_6$-DMSO): $\delta$ = 1.20 (t, 3H), 3.26 - 3.32 (m, 2H), 7.26 (s, 1 H), 7.75 (d, 1 H), 8.20 (s, 1 H), 8.40 (d, 1 H), 8.79 (s, 1 H), 8.84 (s, 1 H), 9.39 (s, 1 H), 10.16 (s, 1 H) ppm.

Verbindung 137: 1-Allyl-3-[3-(4-nitro-phenyl)-pyrido[2,3-b]pyrazin-7-yl]-thioharnstoff

Smp.: 250 °C (Zers.)
$^1$H-NMR ($d_6$-DMSO): $\delta$ = 4.23 (s, 2H), 5.19 (d, 1 H), 5.29 (d, 1 H), 5.90-6.00 (m, 1 H), 8.46 (d, 2H), 8.55 (s, 1H), 8.64 (d, 2H), 8.92 (s, 1H), 9.23 (s, 1H), 9.77 (s, 1H), 10.35 (s, 1 H).

Verbindung 151: 1-Ethyl-3-[3-(3-hydroxy-phenyl)-pyrido[2,3-b]pyrazin-6-yl]-harnstoff

$^1$H-NMR ($d_6$-DMSO): $\delta$ = 1.20 (t, 3H), 3.32 - 3.38 (m, 2H), 6.98 (d, 1 H), 7.40 (t, 1 H), 7.68 (d, 1 H), 7.73 (s, 1 H), 7.76 (d, 1 H), 8.37 (d, 1 H), 9.12 (bs, 1 H), 9.37 (s, 1 H), 9.80 (bs, 1H), 10.12 (s, 1H).

Verbindung 160: 1-Ethyl-3-(3-o-tolylamino-pyrido[2,3-b]-pyrazin-6-yl)-harnstoff

Smp.: 221-223 °C
$^1$H-NMR ($d_6$-DMSO): $\delta$ = 1.12 (t, 3H), 2.31 (s, 3H), 3.20 - 3.26 (m, 2H), 7.12 (t, 1H), 7.23 (t, 1 H), 7.29 (d, 1 H), 7.33 (d, 1 H), 7.86 (d, 1 H), 8.07 (d, 1 H), 8.43 (s, 1 H), 8.81 (bs, 1 H), 9.26 (s, 1 H), 9.75 (s, 1 H).

Verbindung 169: Phosphorsäure-mono-{4-[6-(3-ethyl-ureido)-pyrido-[2,3-b]pyrazin-3-yl]-2-methoxy-phenyl}-ester

$^1$H-NMR ($d_6$-DMSO): $\delta$ = 1.20 (t, 3H), 3.25 - 3.35 (m, 2H), 3.95 (s, 3H), 7.51 (d, 1 H), 7.22 (d, 1H), 7.92 (d, 1H), 7.98 (s, 1H), 8.36 (d, 1H), 8.94 (bs, 1H), 9.47 (s, 1H), 10.09 (s, 1H) ppm.

Verbindung 175: Natrium 2-chlor-4-[6-(3-ethyl-ureido)-pyrido[2,3-b]pyrazin-3-yl]-6-methoxy-phenolat

Smp.: 260 °C (Zers.)
$^1$H-NMR ($d_6$-DMSO): $\delta$ = 1.19 (t, 3H), 3.25 - 3.35 (m, 2H), 3.81 (s, 3H), 7.45 (bs, 1H), 7.64 (bs, 1H) 7.92 (s, 1H), 8.16 (bs, 1H), 9.10 (bs, 1H), 9.23 (bs, 1H), 9.89 (bs, 1 H) ppm.

Verbindung 183: 1-Ethyl-3-[3-(4-hydroxy-3-methoxy-phenyl)-pyrido[2,3-b]pyrazin-6-yl]-harnstoff p-Toluolsulfonat

Smp.: 193-196 °C
$^1$H-NMR ($d_6$-DMSO): $\delta$ = 1.20 (t, 3H), 2.29 (s, 3H), 3.28 - 3.35 (m, 2H), 3.93 (s, 3H), 6.97 (d, 1 H), 7.11 (d, 2H), 7.47 (d, 2H), 7.65 (d, 1 H), 7.85 (d, 1 H), 7.91 (s, 1 H), 8.31 (d, 1H), 9.00 (bs, 1H), 9.41 (s, 1H), 9.71 (bs, 1H), 10.04 (s, 1H) ppm.

Verbindung 193: 1-Ethyl-3-[3-(4-hydroxy-3-methoxy-phenyl)-pyrido[2,3-b]pyrazin-6-yl]-harnstoff (Z)-but-2-endicarbonsäure-Salz

Smp.: 200-204 °C
$^1$H-NMR ($d_6$-DMSO): $\delta$ = 1.20 (t, 3H), 3.25 - 3.35 (m, 2H), 3.92 (s, 3H), 6.24 (s, 2H), 6.98 (d, 1 H), 7.65 (d, 1 H), 7.85 (d, 1 H), 7.91 (s, 1 H), 8.32 (d, 1 H), 9.00 (bs, 1 H), 9.41 (s, 1H), 9.71 (bs, 1H), 10.04 (s, 1H) ppm.

Verbindung 194: 1-Ethyl-3-[3-(4-hydroxy-3-methoxy-phenyl)-pyrido[2,3-b]pyrazin-6-yl]-harnstoff Hydrochlorid

Smp.: 236-239 °C
$^1$H-NMR ($d_6$-DMSO): $\delta$ = 1.20 (t, 3H), 3.32 (quint, 2H), 3.93 (s, 3H), 6.98 (d, 1H), 7.65 (d, 1H), 7.85 (d, 1H), 7.91 (s, 1H), 8.31 (d, 1H), 9.00 (bs, 1H), 9.41 (s, 1H), 9.71 (bs, 1 H), 10.05 (s, 1H) ppm.

Verbindung 202: 3-Ethyl-1-(3-phenyl-pyrido[2,3-b]pyrazin-6-yl)-1-(2-piperidin-1-yl-ethyl)-harnstoff Hydrochlorid

Smp.: 187-190 °C
$^1$H-NMR ($d_6$-DMSO): $\delta$ = 1.19 (t, 3H), 1.46 (m, 1H), 1.76 (d, 1H), 1.89 (m, 4H), 3.06 (m, 2H), 3.28 - 3.35 (m, 4H), 3.75 (d, 2H), 4.43 (t, 2H), 7.60-7.66 (m, 3H), 7.92 (d, 1 H), 8.36 (d, 2H), 8.46 (d, 1 H), 8.59 (t, 1 H), 9.58 (s, 1 H), 9.73 (bs, 1 H) ppm.

Verbindung 204: 1-(4-Hydroxy-butyl)-3-(3-phenyl-pyrido[2,3-b]pyrazin-6-yl)-harnstoff Hydrochlorid

$^1$H-NMR (DMSO-$d_6$) $\delta$ = 1.58-1.64 (m, 4H), 3.30-3.34 (m, 2H), 3.48-3.51 (m, 2H), 7.57-7.63 (m, 3H), 7.66 (d, 1H), 8.34-8.39 (m, 3H), 9.32 (s, 1H), 9.47 (s, 1H), 10.15 (s, 1H) ppm.

III. Nachweis der Kinase-Inhibition erfindungsgemäßer Verbindungen

III.1 Zellfreie Kinaseassays (mittels ALPHA-Technologie)

Die inhibitorische Wirkung der erfindungsgemäßen Verbindungen wurde an diversen humanen Serin/ Threonin-, Tyrosin- und Lipidkinasen in enzymatischen Assays getestet. Dabei wurden rekombinante humane Kinasen wie z.B. Erk2, PI3Kalpha, -beta, - gamma, -delta, p38alpha, p38gamma, Jnk1, Jnk2 und andere eingesetzt, teils als Volllängenkinasen, teils als verkürzte Fragmente - mindestens aber bestehend aus der funktionellen Kinasedomäne. Die käuflichen Kinaseproteine (Proqinase, Upstate) wurden als rekombinante Fusionsproteine mit GST-(Glutathion-S-Transferase) oder His-Tag eingesetzt. Je nach Substrattyp wurden die verschiedenen Kinasereaktionen mittels geeigneter ALPHA™-beads (PerkinElmer) quantifiziert.

<u>Testung</u>

Nachfolgend wird die Substanztestung am Erk-Assay genauer beschrieben. Ausgewählte Testergebnisse der Erk2-, PI3Kalpha-, p38alpha- und Jnk2-Assays sind unten aufgeführt. Zur Bestimmung des $IC_{50}$-Wertes wurden die potenziellen Inhibitorsubstanzen bei 10 halblogaritmisch abgestuften Konzentrationen von 3.16nM-100$\mu$M untersucht.

a) MAPK-ALPHAs (z.B. Erk2): Die Testsubstanz, 0.625ng Erk2 (#14-173, Upstate), 10$\mu$M ATP und 15nM biotinyliertem MBP (myelin basic protein)-Substrat wurden auf einer 384er Optiplate (PerkinElmer) in einem Volumen von 15$\mu$l für 1 h in 25mM Tris, 10mM $MgCl_2$, 0.1% Tween-20, 100$\mu$M $NaVO_4$, 2mM DTT bei pH 7.5 inkubiert. Anschließend wurde die Kinasereaktion durch Zugabe von 10$\mu$l des mit anti-phospho MBP-Antikörpers (320pM, #05-429/ Upstate)

präinkubierten ALPHA-Beadmixes (10µg/ml, #6760617/ PerkinElmer) in 25mM Tris, 200mM NaCl, 100mM EDTA und 0.3% BSA abgestoppt und über Nacht stehen gelassen.

b) PI3K-ALPHAs (z.B. PI3Kalpha): Die Testsubstanz, 1ng PI3Kalpha (#14-602, Upstate), 100µM ATP und 20µM $PIP_2$-Substrat (#P4508, Echelon) auf einer 384er Optiplate (PerkinElmer) für 1h in 50mM Hepes, 50mM NaCl, 5mM $MgCl_2$, 0.05% Chaps, 5mM DTT bei pH 7.4 inkubiert. Anschließend wurde die Kinasereaktion durch Zugabe des mit 1 nM GST:Grp1-Fusionsprotein (Upstate) und 15nM biotinyliertem PIP3 (#C-39B6/ Echelon) präinkubierten ALPHA-Beadmixes (10µg/ml, #6760603/ PerkinElmer) in 50mM Hepes, 50mM NaCl, 50mM EDTA und 0.1% BSA abgestoppt und über Nacht stehen gelassen.

Die Fluoreszenzdetektion erfolgte am nächsten Morgen in einem Fusion™ªα-Gerät (PerlinElmer).

Auswertung

Die Berechnung der %-Inhibitionswerte je Substanz-Konzentration geschah mittels folgender Formel aus den im Fusion™α ermittelten Rohdaten:

$$\%\ \text{Kinase Inhibition}_{(\text{Probe})} = 100 - \left(100x\frac{\text{Mittelwert}_{(\text{Probe})} - \text{Mittelwert}_{(0\%\ \text{Kontrolle})}}{\text{Mittelwert}_{(100\%\ \text{Kontrolle})} - \text{Mittelwert}_{(0\%\ \text{Kontrolle})}}\right)$$

Die Kontrollen wurden jeweils 8fach, die Substanzproben jeweils 2fach bestimmt. Die 0%-Kontrolle (keine Kinase-Aktivität) enthielt entweder kein ATP oder kein Substrat, die 100%-Kontrolle (voll aktive Kinase) enthielt keine Testsubstanz. Die $IC_{50}$-Werte wurden mit GraphPadPrism ermittelt.
Die erfindungsgemäßen Verbindungen zeigten eine effektive Inhibition von Erk, PI3K, p38alpha und Jnk1 + Jnk2 mit $IC_{50}$-Werten bis zu 6 nM (siehe Tabelle 2).

Tabelle 2: MAPK und PI3Kalpha Kinaseassay Versuchsergebnisse (IC50 [µM] bei 10µM bzw. 100nM* ATP)

| Verbindung | Erk2 | PI3Kalpha | p38alpha | Jnk1 + Jnk2 |
|---|---|---|---|---|
| 1 | 1 | 0,9 | >31.6 | >31.6 |
| 25 | 0,076 | 1,5 | >100 | >31.6 |
| 30 | 0,392 | 0,37 | >100 | 21,8 |
| 43 | 0,006 | 6,2 | >100 | 5.5 |
| 44 | 2,5 | 3,8 | >100 | 1,5 |
| 46 | 9,5 | >100 | 0,495 | >100 |
| 82 | 0,944 | 6,4 | >31.6 | 1.1 |
| 124 | 0,062 | 0,49 | >100 | 1.0 |
| 146 | 0.155 | 1.1 | >100 | 3.3 |
| 147 | 0.711 | 4.1 | >100 | 14.2 |
| 148 | 1.8 | 1.4 | >100 | 13.7 |
| 149 | 0.954 | 3.5 | >100 | >31.6 |
| 150 | 18.7 | 0.129 | 18.1 | >100 |
| 151 | 0.104 | 1.5 | >100 | 2.9 |
| 152 | 1.9 | 5.3 | >100 | 12.1 |
| 153 | 0.939 | 0.612 | >100 | 6.7 |
| 154 | 0.592 | 0.984 | >31.6 | 2.8 |
| 155 | >100 | 5 | >100 | 7.8 |
| 156 | 0.138 | 0.425 | >100 | 2.1 |

(fortgesetzt)

| Verbindung | Erk2 | PI3Kalpha | p38alpha | Jnk1 + Jnk2 |
|---|---|---|---|---|
| 157 | >100 | 0.348 | >100 | 28.5 |
| 158 | >100 | 0.186 | >100 | >31.6 |
| 159 | >100 | 0.147 | >100 | 12.6 |
| 160 | 5.8 | 2.9 | >31.6 | >31.6 |
| 161 | >100 | 1.1 | >100 | >100 |
| 162 | >100 | 0.19 | >100 | >31.6 |
| 163 | 26.9 | 0.096 | >100 | >31.6 |
| 164 | 19.6 | 0.444 | >100 | 24.3 |
| 165 | 19.4 | 1.2 | >100 | 27.1 |
| 166 | 13.7 | 1.3 | >100 | 21.9 |
| 167 | >100 | 1.1 | >100 | 27.3 |
| 168 | 0.15 | 0.612 | >100 | 2.5 |
| 169 | 2.7 | 3.7 | >100 | 9.5 |
| 170 | 0.103 | 2.4 | >31.6 | 1.1 |
| 171 | 0.084 | 3 | >100 | 1.4 |
| 172 | 0.41 | 4 | >100 | 4 |
| 173 | 0.107 | 2.4 | >100 | 2.9 |
| 174 | 4.9 | 3 | >31.6 | 4.6 |
| 175 | 0.191 | 0.458 | >100 | 2.5 |
| 177 | >31.6 | 0.295 | >100 | 3.9 |
| 178 | >100 | 0.164 | >31.6 | 2.3 |
| 179 | 8 | 0.202 | 1.4 | 8.1 |
| 180 | 15.7 | 1.1 | >100 | 8.6 |
| 181 | 1.7 | 0.739 | >100 | 4.3 |
| 182 | >31.6 | 1.1 | >100 | 21 |
| 183 | 0.203 | 2.8 | >100 | 4.7 |
| 184 | >100 | 0.133 | >100 | 6.3 |
| 185 | >31.6 | 0.154 | >100 | 4.3 |
| 186 | 0.223 | 3.8 | >100 | 2.5 |
| 187 | 16.6 | 0.712 | >31.6 | >100 |
| 188 | >100 | 2.8 | >100 | >31.6 |
| 189 | 20.3 | 0.237 | >100 | 6.2 |
| 190 | >100 | 0.271 | >100 | 31 |
| 192 | 31.6 | 0.422 | >100 | 1.8 |
| 193 | 0.212 | 2.2 | >100 | 1.5 |

III.2A Zellulärer Assay: Testung auf anti-proliferative Wirkung (XTT-Assay)

Das Prinzip dieses Testes beruht auf der intrazellulären Reduktion des Tetrazolium-Farbstoffes XTT (Natrium

3'-[1-(phenylaminocarbonyl)-3,4-tetrazolium]-bis(4-methoxy-6-nitro)benzensulfonsäure, Sigma) zu einem Formazan-Farbstoff durch mitochondriale Dehydrogenasen. Der Farbstoff wird nur von stoffwechselaktiven Zellen gebildet, seine photometrisch messbare Intensität ist ein quantitativer Indikator für das Vorhandensein lebender Zellen. Die Reduzierung der Farbstoffbildung durch Inkubation der Zellen mit Substanzen dient als Parameter für die anti-proliferative Wirkung.

Testung

Die Tumorzelllinien (ATCC) wurden in 96er Mikrotiterplatten in definierter Zellzahl (5000 Zellen/ Well für BxPC3 und Hct116; 10000 Zellen/ Well für MDA MB468) eingesäat und anschließend über Nacht im Brutschrank bei 37°C, 5% $CO_2$ und 95% Luftfeuchtigkeit inkubiert. Die Testsubstanzen wurden als Stammlösungen (10 mM) in DMSO angesetzt. Zur Bestimmung der $EC_{50}$-Werte wurden die potenziellen Inhibitorsubstanzen in viertellogarithmisch abgestuften Verdünnungen zu den Zellen gegeben, sodass finale Konzentrationen von 0.28μM-50μM resulierten. Die Zellplatten wurden dann für 45h im Brutschrank bei 37°C, 5% $CO_2$ und 95% Luftfeuchtigkeit inkubiert.

Für die Detektionssreaktion wurde das Substrat XTT mit PMS (N-Methyl Dibenzopyrazine Methylsulfat, Sigma) versetzt und zu den Zellen gegeben, sodass eine finale Konzentration von 325μg XTT/ml und 2.5μg PMS/ml resultierte. Es wurde dann für 3h bei 37°C, 95% Luftfeuchte inkubiert. Anschließend konnte das durch zelluläre Dehydrogenasen gebildete Formazan Salz bei einer Adsorption bei 490nm quantifiziert werden.

Auswertung

Die Auswertung der %-Inhibitions-Werte geschah mittels folgender Formel aus den Werten für die jeweils gemessenen optischen Dichten bei 490nm:

$$\%\ \text{Inhibition Zellproliferation}_{(\text{Probe})} = 100 - \left(100x\frac{\text{Mittelwert}_{(\text{Probe})} - \text{Mittelwert}_{(0\%\ \text{Kontrolle})}}{\text{Mittelwert}_{(100\%\ \text{Kontrolle})} - \text{Mittelwert}_{(0\%\ \text{Kontrolle})}}\right)$$

Die Kontrollen wurden jeweils 8fach, die Substanzproben jeweils 2fach bestimmt. Die 0%-Kontrolle enthielt keine Zellen, die 100%-Kontrolle (Proliferationskontrolle) enthielt keine Testsubstanz. Die $EC_{50}$-Werte wurden mit GraphPadPrism ermittelt.

Die erfindungsgemäßen Verbindungen zeigten eine teils effektive Inhibition der Zellproliferation mit $EC_{50}$-Werten bis < 1 μM (siehe Tabelle 3).

Tabelle 3: XTT-Assay Versuchsergebnisse (EC50 [μM])

| Verbindung | BxPC3 | MDA-MB468 | Hct116 |
|---|---|---|---|
| 30 | 12 | 4 | 6 |
| 124 | 10 | 10 | 1,5 |
| 146 | >50 | ca. 20 | 9.3 |
| 150 | 0.9 | 4.3 | 2.3 |
| 153 | >25 | 24 | 6 |
| 156 | 8.9 | 15 | 1.4 |
| 157 | >25 | 3.5 | 4.4 |
| 164 | >50 | 5.2 | >25 |
| 169 | nicht getestet | 5.7 | 2.8 |
| 173 | nicht getestet | >25 | 9 |
| 175 | nicht getestet | >50 | 4.4 |
| 176 | nicht getestet | 17 | 11.8 |
| 183 | nicht getestet | 4.3 | 2.1 |
| 191 | nicht getestet | 8.5 | 5.5 |

(fortgesetzt)

| Verbindung | BxPC3 | MDA-MB468 | Hct116 |
|---|---|---|---|
| 193 | nicht getestet | 4.4 | 2.1 |
| Ly294002 | 25 | 20 | 17 |

Es zeigte sich in überaschender Weise, dass der literatur-bekannte PI3K-Inhibitor Ly294002 eine im Vergleich zu den Ausführungsbeispielen nur schwache anti-proliferative Wirkungen an den eingesetzten Zelllinien aufwies.

III.2B Zellulärer Assay: Testung auf Substrat-Inhibtion (Western Blotting)

Diese Methode ermöglicht eine Aussage darüber, ob der untersuchte Kinasemodulator auch in einem zellulären Kontext die gewünschte Wirkung erzielt, d.h. hierbei wird ein der Targetkinase nachgeschaltetes Substrat-Protein auf seinen Phosphorylierungs-Status untersucht. Dazu werden die mit Substanz inkubierten Zellen lysiert und das Gesamtprotein auf einem reduzierenden Polyacrylamidgel aufgetrennt. Anschließend werden die Proteine mittels Western Blotting auf eine PVDF-Membran transferiert und die gesuchten Substratbanden mit spezifischen Antikörpern und einer geeigneten Detektionsmethode sichtbar gemacht. Die den Targetkinasen nachgeschalteten Substratproteine werden mit einem jeweils speziellen anti-Phospho-Antikörper und gleichzeitig einem total-Antikörper, welcher das Substrat-Totalprotein erkennt, gleichzeitig detektiert. Die Duplex-Technologie des ODYSSEY-Imagers (LiCOR) ermöglicht diese simultane Messung. Die Intensität der Totalsubstrat-Banden wird zur Normalisierung bzw. Quantifizierung der Phosphorylierungs-Inhibition oder Aktivierung heran gezogen.

Testung

Geeignete Tumorzelllinien (z.B. BxPC3, Hct116 oder MDA MB468) wurden in 6-Well Mikrotiterplatten in definierter Zellzahl (z.B. 350 000 Zellen/ Well für BxPC3 und Hct116) in den jeweiligen Standard-Vollmedien eingesäat und anschließend über Nacht im Brutschrank bei 37°C, 5% $CO_2$ und 95% Luftfeuchtigkeit inkubiert. Die Zellen wurden anschließend für weitere 24h unter Serum-reduzierten Bedingungen, d.h. im jeweiligen Medium, jedoch bei nur 0.25% Serum, weiter inkubiert. Die Testsubstanzen wurden als Stammlösungen (10 mM) in DMSO angesetzt und bei finalen Konzentrationen von 5, 15.8 und 50μM für 5h mit den Zellen inkubiert. Daraufhin erfolgte die Zelllyse in 25mM Tris, 150mM NaCl, 10mM Na-Pyrophosphat, 2mM EGTA, 25mM β-Glycerophosphat, 25mM NaF, 10% Glycerin, 0.75% NP-40, 100μM $NaVO_4$-Puffer. Nach Proteinquantifizierung mittels BCA-(Bicinchonic acid protein assay kit, Sigma)Assay wurden Proteinmengen von etwa 20μg pro Laufspur auf einem Lämmli-Polyacrylamidgel aufgetrennt und danach mittels Semi-Dry Western-Blotting bei 0.8mA/$cm^2$ für 1h auf eine PVDF-Membran (Millipore) transferiert. Es folgte eine 1 stündige Prähyridisierung der Membran in I-Block Reagenz (Applied Biosystems) und die Über-Nacht-Inkubation mit den spezifischen Antikörpern. Zur Bestimmung der Erk- und PI3K-Inhibition wurden die jeweils abwärts folgenden Substrate Rsk1 mit dem Total-Antikörper (Rsk #sc-231g C-21, Santa Cruz) und dem Phospho-Antikörper (Phospho-p90RSK (S380) #9341, NEB Cell Signalling) und Akt mit dem Total-Antikörper (Akt1 #sc-1618 C-20, Santa Cruz) und dem Phospho-Antikörper (Phospho-Akt (Ser 473) #9271, NEB Cell Signaling) detektiert. Nach Waschen der Membran erfolgte die Sekundärantikörperinkubation mit anti-Kaninchen IR Dye 800 (#611-732-127, Rockland) für die Phospho-Antikörper und anti-Ziege Alexa Fluor 680 (#A-21081, Molecular Probes) für die Totalprotein-Antikörper. Nach Inkubation für 30min bei Raumtemperatur im Dunkeln wurde die Hybridisierung des Detektionsantikörpers an die Membran durch Scannen im ODYSSEY-Imager (LiCOR) detektiert.

Auswertung

Bei Konzentrationen von 5-50μM zeigten die erfindungsgemäßen Verbindungen eine duale Inhibition der Erk (MAPK1/2) und der PI3K (siehe Tabelle 4), welche durch Hemmung der Bandenintensität beider korrespondierender phospho-Substratproteine Rsk1 und Akt angezeigt wird. Die Abnahme der Fluoreszenzintensität der phospho-Substratbanden (pRsk und pAkt) ist in der Tabelle unten als %Inhibition angegeben und bezieht sich auf folgende Formel:

$$\%\ \text{Inhibition Substratphosphorylierung}_{(\text{Probe})} = 100 - \left(100x \frac{\text{Probe}}{100\%\text{Kontrolle}}\right)$$

Als 100%-Kontrolle wurde die Bandenintensität (Fluoreszenzintensität) der jeweiligen nicht-inhibierten (ohne

Substanz) phospho-Substrate verwendet.

Tabelle 4: Inhibition der zellulären Substratphosphorylierung (bei 50μM)

| Verbindung | Erk →pRsk | PI3K →pAkt |
|---|---|---|
| 30 | 90% | 100% |
| 124 | 90% | 90% |
| 146 | 100% | 10% |
| 150 | 0% | 90% |
| 173 | 90% | 50% |
| 183 | 80% | 80% |
| Ly294002 | 0% | 40% |
| Wortmannin | 0% | 100% |

Der literatur-bekannte PI3K-Inhibitor Ly294002 zeigte eine im Vergleich zu den Pyridopyrazin-Derivaten nur schwache PI3K-Inhibition, d.h. Inhbition des PI3K-Substrates p-Akt und erwartungsgemäß keine Erk-Inhibition bzw. Inhibition des Erk-Substrates p-Rsk. Wortmannin - ein weiterer literatur-bekannter PI3K-Inhibitor - zeigte eine vollständige Inhibition des PI3K-Substrates pAkt, jedoch keine Erk bzw. p-RSK-Inhibition.

Beide hier eingesetzten Referenzsubstanzen zeigten im Gegensatz zu den Ausführungsbeispielen keine duale Inhibition, d.h. von Erk und PI3K gleichzeitg, sondern lediglich eine PI3K-Inhibition.

<u>Abkürzungen</u>

| | |
|---|---|
| Akt | von: murine Akt8 retrovirus oder protein kinase B (PKB) |
| Ask1 | apoptosis signal-regulating kinase |
| ATR | ataxia-telangiectasia and Rad3-related |
| ATM | Ataxia-telangiectasia mutated |
| Bag1 | Bcl-2 associated athanogene-1 |
| Bcl-2 | B-cell leukemia/lymhoma-2 gene |
| DNA-PK | DNA-dependent protein kinase |
| Erk | extracellular signal-regulated kinase |
| Flt-3 | fms like tyrosine kinase 3 |
| GSK-3 | Glycogen synthase kinase-3 |
| hSMG-1 | human ortholog of product of seven nematode gene-1 |
| JAK-3 | Janus kinase 3 |
| JNK | c-jun N-terminal kinase |
| MAPK | mitogen activated protein kinase |
| Mek | MAP or Erk kinase |
| mTOR | mammalian target of rapamycin |
| PDGFR | platelet derived growth factor receptor |
| PI3K | phosphoinositol 3-Kinase |
| PIKK | phosphoinositol 3-Kinase related kinase |
| $PIP_2$ | phosphatidylinositol-biphosphat |
| $PIP_3$ | phosphatidylinositol-triphosphat |
| PtdIns | phosphatidylinositol |
| Raf | rapid accelerated fibrosarcoma |
| Ras | rat sarcoma |
| RTK | receptor tyrosine kinase |
| SAPK | stress-activated protein kinase |
| Ser | Serin |
| Syk | spleen tyrosine kinase |
| Thr | Threonin |

| | |
|---|---|
| Tyr | Tyrosin |
| VEGFR | vascular endothelial growth factor receptor |

Literatur

1. Alessi DR, Andjelkovic M, Caudwell B, Cron P, Morrice N, Cohen P, Hemmings BA. Mechanism of activation of protein kinase B by insulin and IGF-1. EMBO J. 1996 Dec 2;15(23):6541-51.

2. Ali K, Bilancio A, Thomas M, Pearce W, Gilfillan AM, Tkaczyk C, Kuehn N, Gray A, Giddings J, Peskett E, Fox R, Bruce I, Walker C, Sawyer C, Okkenhaug K, Finan P, Vanhaesebroeck B. Essential role for the p110delta phosphoinositide 3-kinase in the allergic response. Nature. 2004 Oct 21;431(7011):1007-11.

3. Bennett BL, Sasaki DT, Murray BW, O'Leary EC, Sakata ST, Xu W, Leisten JC, Motiwala A, Pierce S, Satoh Y, Bhagwat SS, Manning AM, Anderson DW. SP600125, an anthrapyrazolone inhibitor of Jun N-terminal kinase. Proc Natl Acad Sci USA. 2001 Nov 20;98(24):13681-6.

4. Bondev A, Rubio I, Wetzker R. Differential regulation of lipid and protein kinase activities of phosphoinositide 3-kinase gamma in vitro. Biol Chem. 1999 Nov;380(11):1337-40.

5. Bondeva T, Pirola L, Bulgarelli-Leva G, Rubio I, Wetzker R, Wymann MP. Bifurcation of lipid and protein kinase signals of PI3Kgamma to the protein kinases PKB and MAPK. Science. 1998 Oct 9;282(5387):293-6.

6. Campbell IG, Russell SE, Choong DY, Montgomery KG, Ciavarella ML, Hooi CS, Cristiano BE, Pearson RB, Phillips WA. Mutation of the PIK3CA gene in ovarian and breast cancer. Cancer Res. 2004 Nov 1;64(21):7678-81.

7. Chang F, Lee JT, Navolanic PM, Steelman LS, Shelton JG, Blalock WL, Franklin RA, McCubrey JA. Involvement of PI3K/Akt pathway in cell cycle progression, apoptosis, and neoplastic transformation: a target for cancer chemotherapy. Leukemia. 2003 Mar;17(3):590-603. Review

8. Chang F, Steelman LS, Lee JT, Shelton JG, Navolanic PM, Blalock WL, Franklin RA, McCubrey JA. Signal transduction mediated by the Ras/Raf/MEK/ERK pathway from cytokine receptors to transcription factors: potential targeting for therapeutic intervention. Leukemia. 2003 Jul;17(7):1263-93. Review.

9. Chang HW, Aoki M, Fruman D, Auger KR, Bellacosa A, Tsichlis PN, Cantley LC, Roberts TM, Vogt PK. Transformation of chicken cells by the gene encoding the catalytic subunit of PI 3-kinase. Science. 1997 Jun 20;276(5320):1848-50.

10. Chen J, Fujii K, Zhang L, Roberts T, Fu H. Raf-1 promotes cell survival by antagonizing apoptosis signal-regulating kinase 1 through a MEK-ERK independent mechanism. Proc Natl Acad Sci USA. 2001 Jul 3;98(14):7783-8.

11. Chiang GG, Abraham RT. Determination of the catalytic activities of mTOR and other members of the phosphoinositide-3-kinase-related kinase family. Methods Mol Biol. 2004;281:125-41.

12. Crackower MA, Oudit GY, Kozieradzki I, Sarao R, Sun H, Sasaki T, Hirsch E, Suzuki A, Shioi T, Irie-Sasaki J, Sah R, Cheng HY, Rybin VO, Lembo G, Fratta L, Oliveira-dos-Santos AJ, Benovic JL, Kahn CR, Izumo S, Steinberg SF, Wymann MP, Backx PH, Penninger JM. Regulation of myocardial contractility and cell size by distinct PI3K-PTEN signaling pathways. Cell. 2002 Sep 20;110(6):737-49.

13. Davies H, Bignell GR, Cox C, Stephens P, Edkins S, Clegg S, Teague J, Woffendin H, Garnett MJ, Bottomley W, Davis N, Dicks E, Ewing R, Floyd Y, Gray K, Hall S, Hawes R, Hughes J, Kosmidou V, Menzies A, Mould C, Parker A, Stevens C, Watt S, Hooper S, Wilson R, Jayatilake H, Gusterson BA, Cooper C, Shipley J, Hargrave D, Pritchard-Jones K, Maitland N, Chenevix-Trench G, Riggins GJ, Bigner DD, Palmieri G, Cossu A, Flanagan A, Nicholson A, Ho JW, Leung SY, Yuen ST, Weber BL, Seigler HF, Darrow TL, Paterson H, Marais R, Marshall CJ, Wooster R, Stratton MR, Futreal PA. Mutations of the BRAF gene in human cancer. Nature. 2002 Jun 27;417(6892):949-54.

14. Davies SP, Reddy H, Caivano M, Cohen P. Specificity and mechanism of action of some commonly used protein kinase inhibitors. Biochem J. 2000 Oct 1;351 (Pt 1):95-105.

15. Dhand R, Hiles I, Panayotou G, Roche S, Fry MJ, Gout I, Totty NF, Truong O, Vicendo P, Yonezawa K, et al. PI 3-kinase is a dual specificity enzyme: autoregulation by an intrinsic protein-serine kinase activity. EMBO J. 1994 Feb 1;13(3):522-33.

16. Elliott RD, Temple C Jr, Montgomery JA. Potential folic acid antagonists. 3. Deaza analogs of methotrexate. 3. 1- and 3-Deaza analogs of 2,4-diamino-6-[(n-methylanilino)methyl]pteridine. J Org Chem. 1968 Feb;33(2):533-6.

17. Friess H, Berberat P, Schilling M, Kunz J, Korc M, Buchler MW. Pancreatic cancer: the potential clinical relevance of alterations in growth factors and their receptors. J Mol Med. 1996 Jan;74(1):35-42. Review.

18. Gotz R, Wiese S, Takayama S, Camarero GC, Rossoll W, Schweizer U, Troppmair J, Jablonka S, Holtmann B, Reed JC, Rapp UR, Sendtner M. Bag1 is essential for differentiation and survival of hematopoietic and neuronal cells. Nat Neurosci. 2005 Sep;8(9):1169-78.

19. Gum RJ, McLaughlin MM, Kumar S, Wang Z, Bower MJ, Lee JC, Adams JL, Livi GP, Goldsmith EJ, Young PR. Acquisition of sensitivity of stress-activated protein kinases to the p38 inhibitor, SB 203580, by alteration of one or more amino acids within the ATP binding pocket. J Biol Chem. 1998 Jun 19;273(25):15605-10.

20. Hoshino R, Chatani Y, Yamori T, Tsuruo T, Oka H, Yoshida O, Shimada Y, Ari-i S, Wada H, Fujimoto J, Kohno M. Constitutive activation of the 41-/43-kDa mitogen-activated protein kinase signaling pathway in human tumors. Oncogene. 1999 Jan 21;18(3):813-22.

21. Katso R, Okkenhaug K, Ahmadi K, White S, Timms J, Waterfield MD. Cellular function of phosphoinositide 3-kinases: implications for development, homeostasis, and cancer. Annu Rev Cell Dev Biol. 2001;17:615-75. Review.

22. Khleif SN, Abrams SI, Hamilton JM, Bergmann-Leitner E, Chen A, Bastian A, Bernstein S, Chung Y, Allegra CJ, Schlom J. A phase I vaccine trial with peptides reflecting ras oncogene mutations of solid tumors. J Immunother. 1999 Mar;22(2): 155-65.

23. Levine DA, Bogomolniy F, Yee CJ, Lash A, Barakat RR, Borgen PI, Boyd J. Frequent mutation of the PIK3CA gene in ovarian and breast cancers. Clin Cancer Res. 2005 Apr 15;11(8):2875-8.

24. Lewis TS, Shapiro PS, Ahn NG. Signal transduction through MAP kinase cascades. Adv Cancer Res. 1998;74:49-139. Review.

25. Li J, Yen C, Liaw D, Podsypanina K, Bose S, Wang SI, Puc J, Miliaresis C, Rodgers L, McCombie R, Bigner SH, Giovanella BC, Ittmann M, Tycko B, Hibshoosh H, Wigler MH, Parsons R. PTEN, a putative protein tyrosine phosphatase gene mutated in human brain, breast, and prostate cancer. Science. 1997 Mar 28;275(5308):1943-7.

26. Lu Y, Wang H, Mills GB. Targeting PI3K-AKT pathway for cancer therapy. Rev Clin Exp Hematol. 2003 Jun;7(2):205-28. Review.

27. Ma YY, Wei SJ, Lin YC, Lung JC, Chang TC, Whang-Peng J, Liu JM, Yang DM, Yang WK, Shen CY. PIK3CA as an oncogene in cervical cancer. Oncogene. 2000 May 25;19(23):2739-44.

28. Marshall C. How do small GTPase signal transduction pathways regulate cell cycle entry? Curr Opin Cell Biol. 1999 Dec;11(6):732-6. Review.

29. McPhillips F, Mullen P, Monia BP, Ritchie AA, Dorr FA, Smyth JF, Langdon SP. Association of c-Raf expression with survival and its targeting with antisense oligonucleotides in ovarian cancer. Br J Cancer. 2001 Nov 30;85(11):1753-8.

30. Mendelsohn J, Baselga J. The EGF receptor family as targets for cancer therapy. Oncogene. 2000 Dec 27;19(56):6550-65. Review.

31. Moore SM, Rintoul RC, Walker TR, Chilvers ER, Haslett C, Sethi T. The presence of a constitutively active phosphoinositide 3-kinase in small cell lung cancer cells mediates anchorage-independent proliferation via a protein kinase B and p70s6k-dependent pathway. Cancer Res. 1998 Nov 15;58(22):5239-47.

32. Okkenhaug K, Vanhaesebroeck B. PI3K in lymphocyte development, differentiation and activation. Nat Rev Immunol. 2003 Apr;3(4):317-30. Review.

33. Patrucco E, Notte A, Barberis L, Selvetella G, Maffei A, Brancaccio M, Marengo S, Russo G, Azzolino O, Rybalkin SD, Silengo L, Altruda F, Wetzker R, Wymann MP, Lembo G, Hirsch E.. PI3Kgamma modulates the cardiac response to chronic pressure overload by distinct kinase-dependent and -independent effects. Cell. 2004 Aug 6;118(3):375-87.

34. Rapp UR, Rennefahrt U, Troppmair J. Bcl-2 proteins: master switches at the inter-section of death signaling and the survival control by Raf kinases. Biochim Biophys Acta. 2004 Mar 1;1644(2-3):149-58. Review.

35. Rodriguez-Viciana P, Warne PH, Dhand R, Vanhaesebroeck B, Gout I, Fry MJ, Waterfield MD, Downward J. Phosphatidylinositol-3-OH kinase as a direct target of Ras. Nature. 1994 Aug 18;370(6490):527-32.

36. Samuels Y, Wang Z, Bardelli A, Silliman N, Ptak J, Szabo S, Yan H, Gazdar A, Powell SM, Riggins GJ, Willson JK, Markowitz S, Kinzler KW, Vogelstein B, Velculescu VE. High frequency of mutations of the PIK3CA gene in human cancers. Science. 2004 Apr 23;304(5670):554.

37. Sebolt-Leopold JS, Herrera R. Targeting the mitogen-activated protein kinase cascade to treat cancer. Nat Rev Cancer. 2004 Dec;4(12):937-47. Review.

38. Shayesteh L, Lu Y, Kuo WL, Baldocchi R, Godfrey T, Collins C, Pinkel D, Powell B, Mills GB, Gray JW. PIK3CA is implicated as an oncogene in ovarian cancer Nat Genet. 1999 Jan;21(1):99-102.

39. Sirivatanauksorn V, Sirivatanauksorn Y, Lemoine NR. Molecular pattern of ductal pancreatic cancer. Langenbecks Arch Surg. 1998 Apr;383(2):105-15. Review.

40. Steck PA, Pershouse MA, Jasser SA, Yung WK, Lin H, Ligon AH, Langford LA, Baumgard ML, Hattier T, Davis T, Frye C, Hu R, Swedlund B, Teng DH, Tavtigian SV. Identification of a candidate tumour suppressor gene, MMAC1, at chromosome 10q23.3 that is mutated in multiple advanced cancers. Nat Genet. 1997 Apr;15(4):356-62.

41. Sujobert P, Bardet V, Cornillet-Lefebvre P, Hayflick JS, Prie N, Verdier F, Vanhaesebroeck B, Muller O, Pesce F, Ifrah N, Hunault-Berger M, Berthou C, Villemagne B, Jourdan E, Audhuy B, Solary E, Witz B, Harousseau JL, Himberlin C, Lamy T, Lioure B, Cahn JY, Dreyfus F, Mayeux P, Lacombe C, Bouscary D. Essential role for the p110delta isoform in phosphoinositide 3-kinase activation and cell proliferation in acute myeloid leukemia. Blood. 2005 Aug 1;106(3):1063-6.

42. Temple C Jr, Rener GA. Potential antimitotic agents. Synthesis of some ethyl benzopyrazin-7-ylcarbamates, ethyl pyrido[3,4-b]pyrazin-7-ylcarbamates, and ethyl pyrido[3,4-e]-as-triazin-7-ylcarbamates. J Med Chem. 1990 Nov;33(11):3044-50.

43. Troppmair J, Rapp UR. Raf and the road to cell survival: a tale of bad spells, ring bearers and detours. Biochem Pharmacol. 2003 Oct 15;66(8):1341-5. Review.

44. Vanhaesebroeck B, Higashi K, Raven C, Welham M, Anderson S, Brennan P, Ward SG, Waterfield MD. Autophosphorylation of p110delta phosphoinositide 3-kinase: a new paradigm for the regulation of lipid kinases in vitro and in vivo. EMBO J. 1999 Mar 1;18(5):1292-302.

45. Vanhaesebroeck B, Leevers SJ, Ahmadi K, Timms J, Katso R, Driscoll PC, Woscholski R, Parker PJ, Waterfield MD. Synthesis and function of 3-phosphorylated inositol lipids. Annu Rev Biochem. 2001;70:535-602. Review.

46. Weinstein-Oppenheimer CR, Blalock WL, Steelman LS, Chang F, McCubrey JA. The Raf signal transduction cascade as a target for chemotherapeutic intervention in growth factor-responsive tumors. Pharmacol Ther. 2000 Dec;88(3):229-79. Review.

47. Wetzker R, Rommel C. Phosphoinositide 3-kinases as targets for therapeutic intervention. Curr Pharm Des. 2004;10(16):1915-22. Review.

48. Wymann MP, Pirola L. Structure and function of phosphoinositide 3-kinases. Biochim Biophys Acta. 1998 Dec 8;1436(1-2):127-50. Review.

**Patentansprüche**

**1.** Verwendung einer Verbindung gemäß der allgemeinen Formel (I)

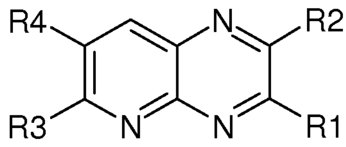

**(I)**

worin die Substituenten R1, R2, R3, R4 folgende Bedeutung haben:

R1 und R2 können unabhängig voneinander:

(i) Wasserstoff
(ii) Hydroxyl
(iii)Halogen
(iv) Alkyl, wobei der Alkylrest gesättigt ist und aus 1 bis 8 C-Atomen bestehen kann,
(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, S03H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, n den Wert 1, 2 oder 3 annehmen kann, und die Alkyl-, Cycloalkyl-, Heterocyclyl-, Aryl-, Heteroaryl-, Alkyl-Cycloalkyl-, Alkyl-Heterocyclyl-, Alkyl-Aryl- und Alkyl-Heteroarylsubstituenten ihrerseits wiederum substituiert sein können,
(vi) unsubstituiertes oder substituiertes Heteroaryl, wobei der Heteroarylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$,

C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2NH_2$, $SO_2NH$-Alkyl, $SO_2NH$-Aryl, $SO_2NH$-Heteroaryl, $SO_2NH$-Alkyl-Aryl, S03H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, und die Alkyl-, Cycloalkyl-, Heterocyclyl-, Aryl- und Heteroarylsubstituenten ihrerseits wiederum substituiert sein können,

(vii) OR5, wobei R5 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl-, Heteroaryl-, Alkyl-Cycloalkyl, Alkyl-Heterocyclyl, Alkyl-Aryl oder Alkyl-Heteroarylsubstituenten ihrerseits wiederum substituiert sein können,

(viii)SR6, wobei R6 Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl-, Alkyl-Heterocyclyl-, Alkyl-Aryl-oder Alkyl-Heteroarylsubstituenten ihrerseits wiederum substituiert sein können,

(ix) NR7R8, wobei R7 und R8 unabhängig voneinander Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein können, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können, oder R7 und R8 zusammen Heterocyclyl bedeuten, wobei Heterocyclyl wiederum substituiert sein kann,

bedeuten.
R3 und R4 können unabhängig voneinander Wasserstoff oder NR9R10 bedeuten, unter der Voraussetzung, dass, wenn R3 = NR9R10 ist, R4 = H ist, und wenn R4 = NR9R10 ist, R3 = H ist, wobei R9 Wasserstoff, Alkyl, Cycloalkyl, Heterocyclyl, Aryl, Heteroaryl, Alkyl-Cycloalkyl, Alkyl-Heterocyclyl, Alkyl-Aryl oder Alkyl-Heteroaryl sein kann, und die Alkyl-, Cycloalkyl-, Heterocyclyl-, Aryl- und Heteroaryl-, Alkyl-Cycloalkyl, Alkyl-Heterocyclyl, Alkyl-Aryl oder Alkyl-Heteroarylsubstituenten ihrerseits wiederum substituiert sein können,
und R10:

- C(Y)NR11 R12 bedeuten kann, wobei Y = O, S und R11 und R12 unabhängig voneinander

(i) Wasserstoff,

(ii) unsubstituiertes oder substituiertes Alkyl, wobei der Alkylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Cycloalkyl, Heterocyclyl, Aryl oder Heteroaryl, ein- oder mehrfach, gleich oder verschieden substituiert sein kann,

(iii)unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, $CO_2H$, $CO_2$-Alkyl, $CO_2$-Cycloalkyl, $CO_2$-Hete-

rocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, Alkyl, oder Aryl ein-oder mehrfach, gleich oder verschieden substituiert sein kann,

(iv) unsubstituiertes oder substituiertes Heterocyclyl, wobei der Heterocyclylrest mit OH, O-Alkyl, O-Aryl, $NH_2$, NH-Alkyl, NH-Aryl, Alkyl, Alkyl-Aryl oder Aryl ein- oder mehrfach, gleich oder verschieden substituiert sein kann,

(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, SO-Alkyl, SO-Aryl, $SO_2$-Alkyl, $SO_2$-Aryl, $SO_2NH_2$, $SO_2NH$-Alkyl, $SO_2NH$-Aryl, $SO_2NH$-Heteroaryl, $SO_2NH$-Alkyl-Aryl, S03H, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(vi) unsubstituiertes oder substituiertes Heteroaryl, wobei der Heteroarylrest F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, $N(Alkyl)_2$, NHC(O)-Alkyl, NHC(O)-Cycloalkyl, NHC(O)-Heterocyclyl, NHC(O)-Aryl, NHC(O)-Heteroaryl, NHC(O)-Alkyl-Aryl, NHC(O)-Alkyl-Heteroaryl, $NHSO_2$-Alkyl, $NHSO_2$-Cycloalkyl, $NHSO_2$-Heterocyclyl, $NHSO_2$-Aryl, $NHSO_2$-Heteroaryl, $NHSO_2$-Alkyl-Aryl, $NHSO_2$-Alkyl-Heteroaryl, $NO_2$, SH, S-Alkyl, S-Aryl, S-Heteroaryl, OH, $OCF_3$, O-Alkyl, O-Cycloalkyl, O-Aryl, O-Heteroaryl, O-Alkyl-Cycloalkyl, O-Alkyl-Heterocyclyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, OC(O)-Alkyl-Aryl, OC(O)-Alkyl-Heteroaryl, $OSO_3H$, $OSO_2$-Alkyl, $OSO_2$-Cycloalkyl, $OSO_2$-Heterocyclyl, $OSO_2$-Aryl, $OSO_2$-Heteroaryl, $OSO_2$-Alkyl-Aryl, $OSO_2$-Alkyl-Heteroaryl, $OP(O)(OH)_2$, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, $C(O)N(Alkyl)_2$, $C(O)N(Cycloalkyl)_2$, $C(O)N(Aryl)_2$, $C(O)N(Heteroaryl)_2$, $SO_2NH_2$, $SO_2NH$-Alkyl, $SO_2NH$-Aryl, $SO_2NH$-Heteroaryl, $SO_2NH$-Alkyl-Aryl, $SO_3H$, $SO_2O$-Alkyl, $SO_2O$-Aryl, $SO_2O$-Alkyl-Aryl, Alkyl, Cycloalkyl, Heterocyclyl, Aryl oder Heteroaryl, ein- oder mehrfach, gleich oder verschieden substituiert sein kann,

(vii) -C(O)-R17, wobei R17 Alkyl, Aryl oder Heteroaryl sein kann, und die Alkyl und Arylsubstituenten ihrerseits wiederum substituiert sein können,

(viii)oder R11 und R12 zusammen Heterocyclyl bedeuten können,

- C(Y)NR13R14 bedeuten kann, wobei Y = NH und R13 und R14 unabhängig voneinander

(i) Wasserstoff,

(ii) unsubstituiertes oder substituiertes Alkyl, wobei der Alkylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, $N(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, SO-Alkyl, SO-Aryl, $SO_2$-Alkyl, $SO_2$-Aryl, $SO_2NH_2$, $SO_3H$, Alkyl, Cycloalkyl, Heterocyclyl, Aryl oder Heteroaryl, ein- oder mehrfach, gleich oder verschieden substituiert sein kann,

(iii)unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, $CO_2H$, $CO_2$-Alkyl, $CO_2$-Cycloalkyl, $CO_2$-Heterocyclyl, $CO_2$-Aryl, $CO_2$-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, C(O)N$(Alkyl)_2$, Alkyl, oder Aryl, ein- oder mehrfach, gleich oder verschieden substituiert sein kann,

(iv) unsubstituiertes oder substituiertes Heterocyclyl, wobei der Heterocyclylrest mit OH, O-Alkyl, O-Aryl, $NH_2$, NH-Alkyl, NH-Aryl, Alkyl, oder Aryl ein- oder mehrfach, gleich oder verschieden substituiert sein kann,

(v) unsubstituiertes oder substituiertes Aryl, wobei der Arylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, SO-Alkyl, SO-Aryl, $SO_2$-Alkyl, $SO_2$-Aryl, $SO_2NH_2$, $SO_2$NH-Alkyl, $SO_2$NH-Aryl, $SO_2$NH-Heteroaryl, S03H, $SO_2$O-Alkyl, $SO_2$O-Aryl, $SO_2$O-Heteroaryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(vi) unsubstituiertes oder substituiertes Heteroaryl, wobei der Heteroarylrest mit F, Cl, Br, I, $CF_3$, CN, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N$(Alkyl)_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Aryl, OH, $OCF_3$, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N$(Alkyl)_2$, C(O)N$(Cycloalkyl)_2$, C(O)N$(Aryl)_2$, C(O)N$(Heteroaryl)_2$, $SO_2$-Alkyl, $SO_2$-Aryl, $SO_2NH_2$, $SO_2$NH-Alkyl, $SO_2$NH-Aryl, $SO_2$NH-Heteroaryl, S03H, $SO_2$O-Alkyl, $SO_2$O-Aryl, $SO_2$O-Heteroaryl, Alkyl, Cycloalkyl, Heterocyclyl, Aryl oder Heteroaryl, ein- oder mehrfach, gleich oder verschieden substituiert sein kann,

(vii) oder R13 und R14 zusammen Heterocyclyl bedeuten können,

- C(NR15)R16 bedeuten kann, wobei R15 = H und R16

(i) unsubstituiertes oder substituiertes Alkyl, wobei der Alkylrest mit F, Cl, Br, I, $CF_3$, $NH_2$, NH-Alkyl,

NH-Cycloalkyl, NH-Heterocyclyl, NH-Aryl, NH-Heteroaryl, NH-Alkyl-Aryl, NH-Alkyl-Heteroaryl, N(Alkyl)$_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-Aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)$_2$, C(O)N(Cycloalkyl)$_2$, C(O)N(Aryl)$_2$, C(O)N(Heteroaryl)$_2$, SO-Alkyl, SO-Aryl, $SO_2$-Alkyl, $SO_2$-Aryl, $SO_2NH_2$, $SO_3H$, Alkyl, Cycloalkyl, Heterocyclyl, Aryl oder Heteroaryl, ein- oder mehrfach, gleich oder verschieden substituiert sein kann,

(ii) unsubstituiertes oder substituiertes Cycloalkyl, wobei der Cycloalkylrest mit F, Cl, Br, I, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)$_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Cycloalkyl, $NHSO_2$-Aryl, $NHSO_2$-Heteroaryl, OH, O-Alkyl, O-Cycloalkyl, O-Heterocyclyl, O-Aryl, O-Heteroaryl, O-Alkyl-Aryl, O-Alkyl-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, $CO_2H$, $CO_2$-Alkyl, $CO_2$-Cycloalkyl, $CO_2$-Heterocyclyl, $CO_2$-Aryl, $CO_2$-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Aryl, C(O)NH-Alkyl-Heteroaryl, C(O)N(Alkyl)$_2$, Alkyl, oder Aryl, ein- oder mehrfach, gleich oder verschieden substituiert sein kann,

(iii)unsubstituiertes oder substituiertes Heterocyclyl, wobei der Heterocyclylrest mit OH, O-Alkyl, O-Aryl, $NH_2$, NH-Alkyl, NH-Aryl, Alkyl oder Aryl ein- oder mehrfach, gleich oder verschieden substituiert sein kann,

(iv) unsubstituiertes oder substituiertes Aryl, wobei der Arylrest mit F, Cl, Br, I, $CF_3$, $NH_2$, NH-Alkyl, NH-Cycloalkyl, NH-Heterocyclyl, NH-Aryl, NH-Heteroaryl, NH-Alkyl-Cycloalkyl, NH-Alkyl-Heterocyclyl, NH-Alkyl-Aryl, NH-Alkyl-Heteroaryl, NH-Alkyl-$NH_2$, NH-Alkyl-OH, N(Alkyl)$_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Cycloalkyl, S-Heterocyclyl, S-Aryl, S-Heteroaryl, OH, $OCF_3$, O-Alkyl, O-Cycloalkyl, O-Heterocyclyl, O-Aryl, O-Heteroaryl, O-Alkyl-Cycloalkyl, O-Alkyl-Heterocyclyl, O-Alkyl-Aryl, O-Alkyl-Heteroaryl, O-Alkyl-OH, O-$(CH_2)_n$-O, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)$_2$, C(O)N(Cycloalkyl)$_2$, C(O)N(Aryl)$_2$, C(O)N(Heteroaryl)$_2$, SO-Alkyl, SO-Aryl, $SO_2$-Alkyl, $SO_2$-Aryl, $SO_2NH_2$, $SO_2$NH-Alkyl, $SO_2$NH-Aryl, $SO_2$NH-Heteroaryl, S03H, $SO_2$O-Alkyl, $SO_2$O-Aryl, $SO_2$O-Heteroaryl, Alkyl, Cycloalkyl, Heterocyclyl, Aryl oder Heteroaryl, ein- oder mehrfach, gleich oder verschieden substituiert sein kann, und n den Wert 1, 2 oder 3 annehmen kann,

(v) unsubstituiertes oder substituiertes Heteroaryl, wobei der Heteroarylrest mit F, Cl, Br, I, $CF_3$, $NH_2$, NH-Alkyl, NH-Cycloalkyl, NH-Heterocyclyl, NH-Aryl, NH-Heteroaryl, NH-Alkyl-Aryl, NH-Alkyl-Heteroaryl, N(Alkyl)$_2$, NHC(O)-Alkyl, NHC(O)-Cycloalkyl, NHC(O)-Heterocyclyl, NHC(O)-Aryl, NHC(O)-Heteroaryl, $NHSO_2$-Alkyl, $NHSO_2$-Aryl, $NHSO_2$-Heteroaryl, $NO_2$, SH, S-Alkyl, S-Aryl, OH, $OCF_3$, O-Alkyl, O-Cycloalkyl, O-Heterocyclyl, O-Aryl, O-Heteroaryl, OC(O)-Alkyl, OC(O)-Cycloalkyl, OC(O)-Heterocyclyl, OC(O)-Aryl, OC(O)-Heteroaryl, $OSO_2$-Alkyl, $OSO_2$-Cycloalkyl, $OSO_2$-Aryl, $OSO_2$-Heteroaryl, C(O)-Alkyl, C(O)-Aryl, C(O)-Heteroaryl, $CO_2H$, $CO_2$-Alkyl, $CO_2$-Cycloalkyl, $CO_2$-Heterocyclyl, $CO_2$-Aryl, $CO_2$-Heteroaryl, $CO_2$-Alkyl-Cycloalkyl, $CO_2$-Alkyl-Heterocyclyl, $CO_2$-Alkyl-aryl, $CO_2$-Alkyl-Heteroaryl, C(O)-$NH_2$, C(O)NH-Alkyl, C(O)NH-Cycloalkyl, C(O)NH-Heterocyclyl, C(O)NH-Aryl, C(O)NH-Heteroaryl, C(O)NH-Alkyl-Cycloalkyl, C(O)NH-Alkyl-Heterocyclyl, C(O)NH-Alkyl-Aryl, C(O)NH-Alkyl-Heteroaryl, C(O)N(Alkyl)$_2$, C(O)N(Cycloalkyl)$_2$, C(O)N(Aryl)$_2$, C(O)N(Heteroaryl)$_2$, $SO_2$-Alkyl, $SO_2$-Aryl, $SO_2NH_2$, $SO_2$NH-Alkyl, $SO_2$NH-Aryl, $SO_2$NH-Heteroaryl, S03H, $SO_2$O-Alkyl, $SO_2$O-Aryl, $SO_2$O-Heteroaryl, Alkyl, Cycloalkyl, Heterocyclyl, Aryl oder Heteroaryl, ein- oder mehrfach, gleich oder verschieden substituiert sein kann, bedeuten können;

zur Herstellung eines Arzneimittels zur Behandlung oder Prophylaxe von physiologischen und/oder pathophysiologischen Zuständen in Säugetieren,
wobei die physiologischen und/oder pathophysiologischen Zustände ausgewählt sind aus der Gruppe bestehend aus:

rheumatische Erkrankungen, arthritische Erkrankungen, HIV Infektionen, Rheuma, Arthritis, AIDS, ARC (AIDS related complex), Kaposi-Sarkom, Tumore ausgehend vom Hirn, Alzheimer, Kolontumor, Darmtumor, Pankreastumor, Leukämie, Lebertumor, Nierentumor, Kopftumor, Halstumor, Gliom, Gebärmutterkrebs, Endometriumkrebs, Gebärmutterhalskrebs, Hirntumor, Adenokanthom, Magentumor, Kolorektaltumor, Speiseröhrenkrebs, Schilddrüsenkrebs, Lymphom, chronische Leukämie, akute Leukämie, Diabetis, rheumatische Arthritis, Allergien, allergische Erkrankungen, Erkrankungen des Herzmuskels, Fettleibigkeit, Übergewicht, Hypertension.

**2.** Verwendung gemäß Anspruch 1, wobei der Alkylrest ausgewählt ist aus der Gruppe bestehend aus: "Methyl, Ethyl, n-Propyl, 2-Propyl, n-Butyl, sec.-Butyl, tert.-Butyl, n-Pentyl, iso-Pentyl, neo-Pentyl, n-Hexyl, 2-Hexyl, n-Octyl, Ethylenyl (Vinyl), Ethinyl, Propenyl ($-CH_2CH=CH_2$; $-CH=CH-CH_3$, $-C(=CH_2)-CH_3$), Propinyl ($-CH_2-C\equiv CH$, $-C\equiv C-CH_3$), Butenyl, Butinyl, Pentenyl, Pentinyl, Hexenyl, Hexinyl, Heptenyl, Heptinyl, Octenyl, Octinyl".

**3.** Verwendung gemäß Anspruch 1, wobei der Heterocyclyl-Rest ausgewählt ist aus der Gruppe bestehend aus: "Tetrahydrofuryl, Tetrahydropyranyl, Pyrrolidinyl, Piperidinyl, Piperazinyl, Morpholinyl".

**4.** Verwendung gemäß Anspruch 1, wobei der Heteroaryl-Rest ausgewählt ist aus der Gruppe bestehend aus: "Pyrrolyl, Furyl, Thienyl, Thiazolyl, Oxazolyl, Isoxazolyl, Pyrazolyl, Imidazolyl, Pyridinyl, Pyrimidinyl, Pyridazinyl, Pyrazinyl, Phthalazinyl, Indolyl, Indazolyl, Indolizinyl, Chinolinyl, Isochinolinyl, Chinoxalinyl, Chinazolinyl, Carbazolyl, Phenazinyl, Phenothiazinyl, Acridinyl".

**5.** Verwendung gemäß Anspruch 1, wobei die Verbindung ausgewählt ist aus der Gruppe bestehend aus:

| | | |
|---|---|---|
| **Verbindung 1** | 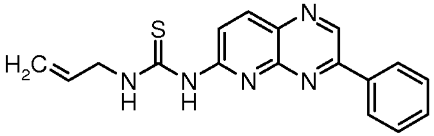 | 1-Allyl-3-(3-phenyl-pyrido[2,3-b]pyrazin-6-yl)-thioharnstoff |
| **Verbindung 2** | 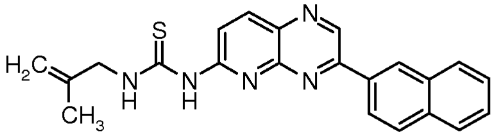 | 1-(2-Methyl-allyl)-3-(3-naphth-2-yl-pyrido[2,3-b]pyrazin-6-yl)-thioharnstoff |
| **Verbindung 3** | 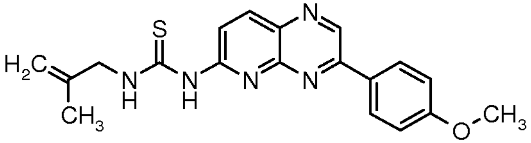 | 1-[3-(4-Methoxy-phenyl)-pyrido[2,3-b]pyrazin-6-yl]-3-(2-methyl-allyl)-thioharnstoff |
| **Verbindung 4** | 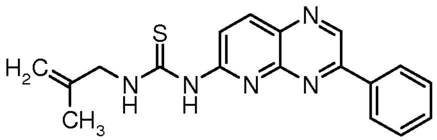 | 1-(2-Methyl-allyl)-3-(3-phenyl-pyrido[2,3-b]pyrazin-6-yl)-thioharnstoff |
| **Verbindung 5** | 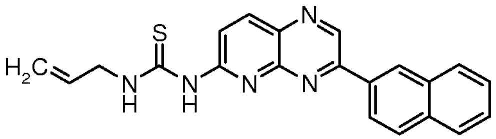 | 1-Allyl-3-(3-naphth-2-yl-pyrido[2,3-b]pyrazin-6-yl)-thioharnstoff |
| **Verbindung 6** | 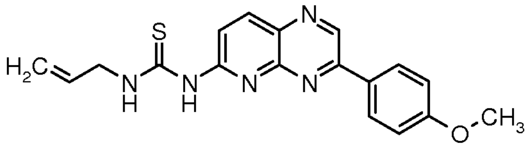 | 1-Allyl-3-[3-(4-methoxy-phenyl)-pyrido[2,3-b]pyrazin-6-yl]-thioharnstoff |
| **Verbindung 7** | 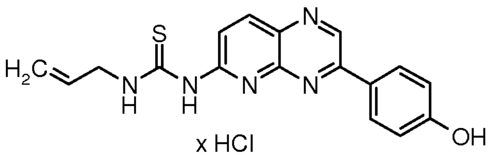 x HCl | 1-Allyl-3-[3-(4-hydroxy-phenyl)-pyrido[2,3-b]pyrazin-6-yl]-thioharnstoff Hydrochlorid |

(fortgesetzt)

| | | |
|---|---|---|
| **Verbindung 8** | 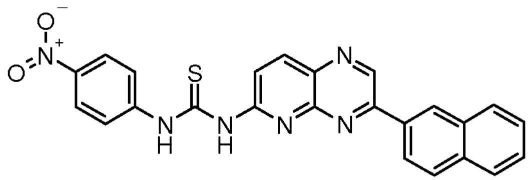 | 1-(3-Naphth-2-yl-pyrido[2,3-b]pyrazin-6-yl)-3-(4-nitro-phenyl)-thioharnstoff |
| **Verbindung 9** | 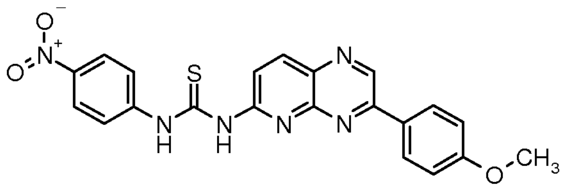 | 1-[3-(4-Methoxy-phenyl)-pyrido[2,3-b]pyrazin-6-yl]-3-(4-nitro-phenyl)-thioharnstoff |
| **Verbindung 10** | 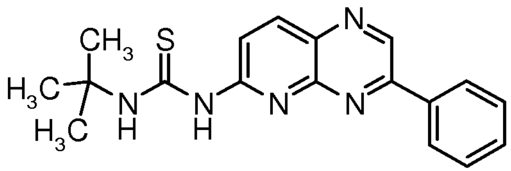 | 1-tert-Butyl-3-(3-phenyl-pyrido[2,3-b]pyrazin-6-yl)-thioharnstoff |
| **Verbindung 11** | 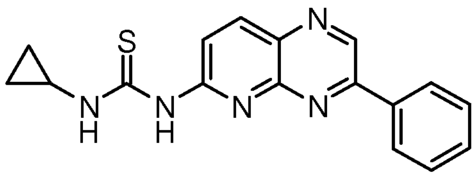 | 1-Cyclopropyl-3-(3-phenyl-pyrido[2,3-b]pyrazin-6-yl)-thioharnstoff |
| **Verbindung 12** | 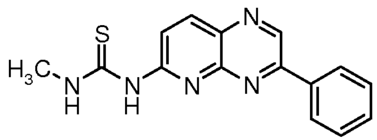 | 1-Methyl-3-(3-phenyl-pyrido[2,3-b]pyrazin-6-yl)-thioharnstoff |
| **Verbindung 13** | 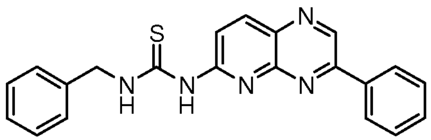 | 1-Benzyl-3-(3-phenyl-pyrido[2,3-b]pyrazin-6-yl)-thioharnstoff |

(fortgesetzt)

| | | |
|---|---|---|
| **Verbindung 14** | 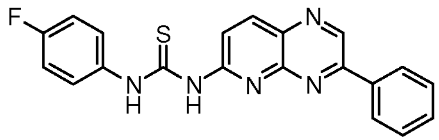 | 1-(4-Fluor-phenyl)-3-(3-phenyl-pyrido[2,3-b]pyrazin-6-yl)-thioharnstoff |
| **Verbindung 15** | 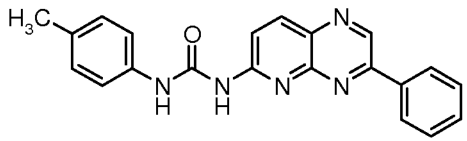 | 1-(3-Phenyl-pyrido[2,3-b]pyrazin-6-yl)-3-p-tolyl-harnstoff |
| **Verbindung 16** | 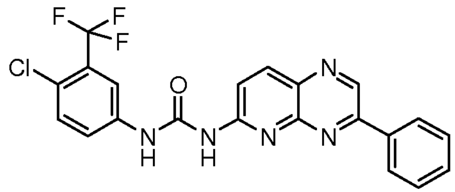 | 1-(4-Chlor-3-trifluormethyl-phenyl)-3-(3-phenyl-pyrido[2,3-b]pyrazin-6-yl)-harnstoff |
| **Verbindung 17** | 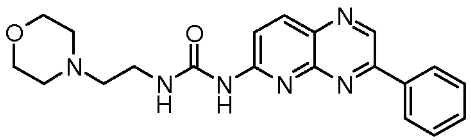 | 1-(2-Morpholin-4-yl-ethyl)-3-(3-phenyl-pyrido[2,3-b]pyrazin-6-yl)-harnstoff |
| **Verbindung 18** | 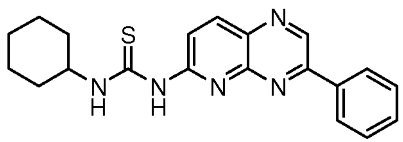 | 1-Cyclohexyl-3-(3-phenyl-pyrido[2,3-b]pyrazin-6-yl)-thioharnstoff |
| **Verbindung 19** | 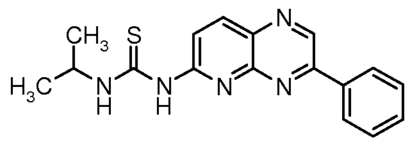 | 1-Isopropyl-3-(3-phenyl-pyrido[2,3-b]pyrazin-6-yl)-thioharnstoff |
| **Verbindung 20** | 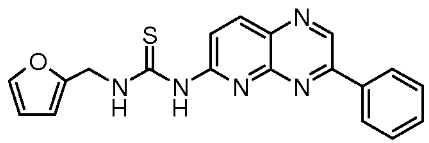 | 1-Furan-2-ylmethyl-3-(3-phenyl-pyrido[2,3-b]pyrazin-6-yl)-thioharnstoff |

(fortgesetzt)

| | | |
|---|---|---|
| **Verbindung 21** | 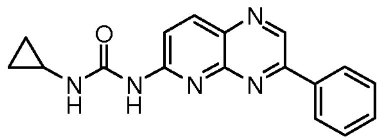 | 1-Cyclopropyl-3-(3-phenyl-pyrido[2,3-b]pyrazin-6-yl)-harnstoff |
| **Verbindung 22** | 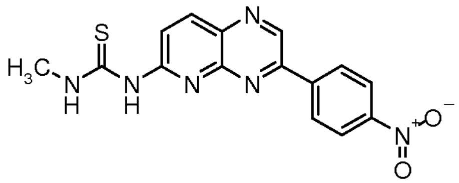 | 1-Methyl-3-[3-(4-nitro-phenyl)-pyrido[2,3-b]pyrazin-6-yl]-thioharnstoff |
| **Verbindung 23** | 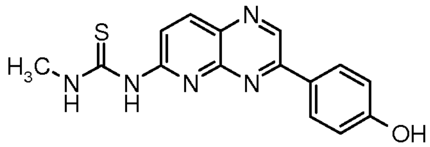 | 1-[3-(4-Hydroxy-phenyl)-pyrido[2,3-b]pyrazin-6-yl]-3-methyl-thioharnstoff |
| **Verbindung 24** | 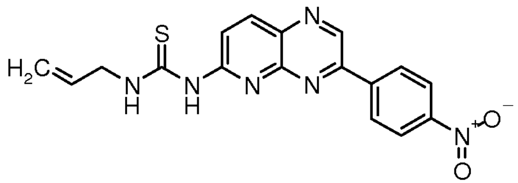 | 1-Allyl-3-[3-(4-nitro-phenyl)-pyrido[2,3-b]pyrazin-6-yl]-thioharnstoff |
| **Verbindung 26** | 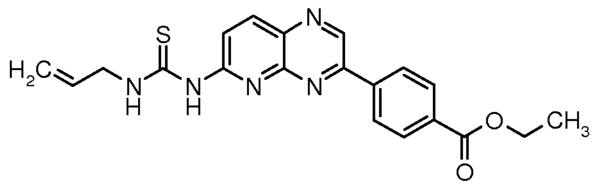 | 4-[6-(3-Allyl-thioureido)-pyrido[2,3-b]pyrazin-3-yl]-benzoesäure-ethylester |
| **Verbindung 27** | 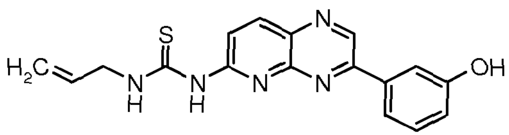 | 1-Allyl-3-[3-(3-hydroxyphenyl)-pyrido[2,3-b]pyrazin-6-yl]-thioharnstoff |

(fortgesetzt)

| | | |
|---|---|---|
| **Verbindung 28** | 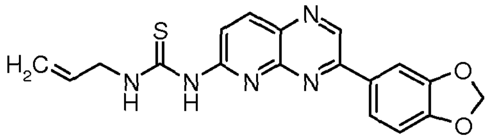 | 1-Allyl-3-(3-benzo[1,3]dioxol-5-yl-pyrido[2,3-b]pyrazin-6-yl)-thioharnstoff |
| **Verbindung 29** | 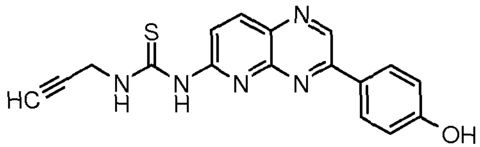 | 1-[3-(4-Hydroxy-phenyl)-pyrido[2,3-b]pyrazin-6-yl]-3-prop-2-inyl-thioharnstoff |
| **Verbindung 31** | 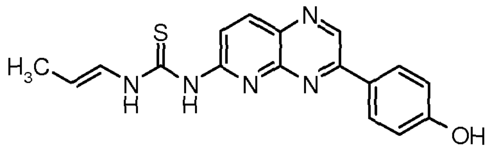 | 1-[3-(4-Hydroxy-phenyl)-pyrido[2,3-b]pyrazin-6-yl]-3-((E)-propenyl)-thioharnstoff |
| **Verbindung 32** | 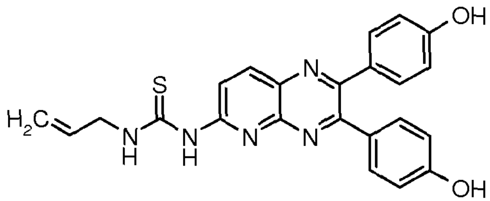 | 1-Allyl-3-[2,3-bis-(4-hydroxy-phenyl)-pyrido[2,3-b]pyrazin-6-yl]-thioharnstoff |
| **Verbindung 33** | 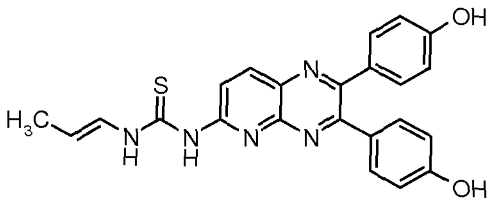 | 1-[2,3-Bis-(4-hydroxyphenyl)-pyrido[2,3-b]pyrazin-6-yl]-3-((E)-propenyl)-thioharnstoff |
| **Verbindung 34** | 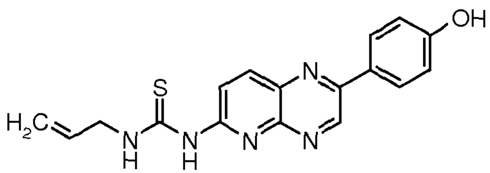 | 1-Allyl-3-[2-(4-hydroxy-phenyl)-pyrido[2,3-b]pyrazin-6-yl]-thioharnstoff |

(fortgesetzt)

| | | |
|---|---|---|
| **Verbindung 35** | 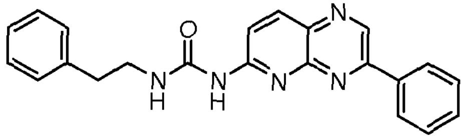 | 1-Phenethyl-3-(3-phenyl-pyrido[2,3-b]pyrazin-6-yl)-harnstoff |
| **Verbindung 36** | 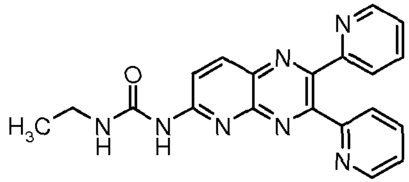 | 1-(2,3-Di-pyridin-2-yl-pyrido[2,3-b]pyrazin-6-yl)-3-ethyl-harnstoff |
| **Verbindung 37** | 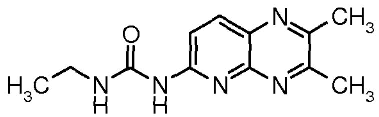 | 1-(2,3-Dimethyl-pyrido[2,3-b]pyrazin-6-yl)-3-ethyl-harnstoff |
| **Verbindung 38** | 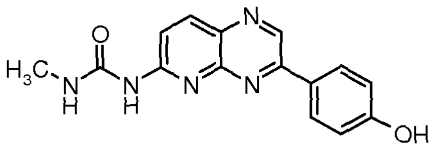 | 1-[3-(4-Hydroxy-phenyl)-pyrido[2,3-b]pyrazin-6-yl]-3-methyl-harnstoff |
| **Verbindung 39** | 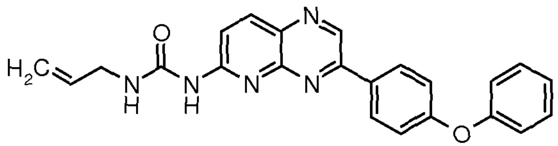 | 1-Allyl-3-[3-(4-phenoxyphenyl)-pyrido[2,3-b]pyrazin-6-yl]-harnstoff |
| **Verbindung 40** | 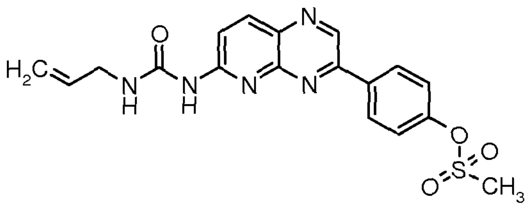 | Methansulfonsäure-4-[6-(3-allyl-ureido)-pyrido[2,3-b]pyrazin-3-yl]-phenyl-ester |

(fortgesetzt)

| | | |
|---|---|---|
| **Verbindung 41** | 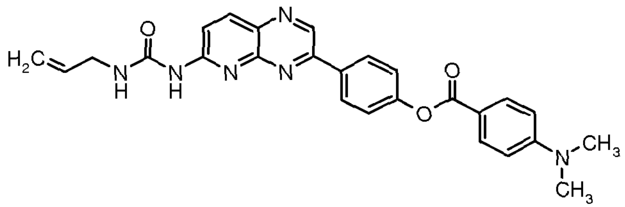 | 4-Dimethylamino-benzoesäure-4-[6-(3-allyl-ureido)-pyrido[2,3-b]pyrazin-3-yl]-phenyl-ester |
| **Verbindung 42** | 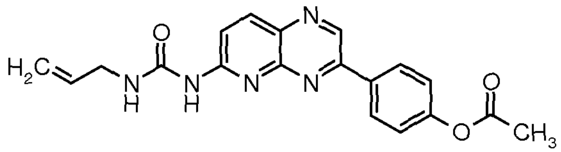 | Essigsäure-4-[6-(3-allyl-ureido)-pyrido[2,3-b]pyrazin-3-yl]-phenyl-ester |
| **Verbindung 43** | 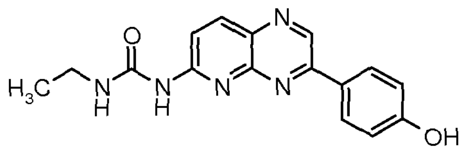 | 1-Ethyl-3-[3-(4-hydroxy-phenyl)-pyrido[2,3-b]pyrazin-6-yl]-harnstoff |
| **Verbindung 44** | 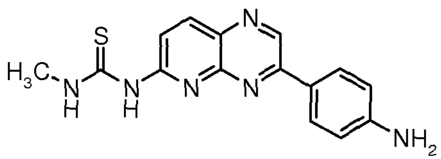 | 1-[3-(4-Amino-phenyl)-pyrido[2,3-b]pyrazin-6-yl]-3-methyl-thioharnstoff |
| **Verbindung 45** | 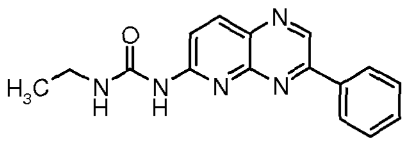 | 1-Ethyl-3-(3-phenyl-pyrido[2,3-b]pyrazin-6-yl)-harnstoff |
| **Verbindung 46** | 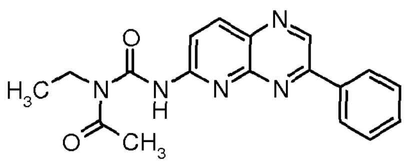 | 1-Acetyl-1-ethyl-3-(3-phenyl-pyrido[2,3-b]pyrazin-6-yl)-harnstoff |

(fortgesetzt)

| | | |
|---|---|---|
| **Verbindung 47** | 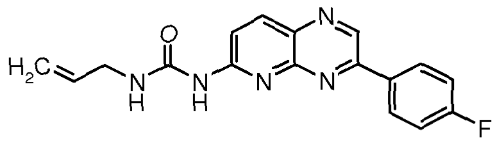 | 1-Allyl-3-[3-(4-fluoro-phenyl)-pyrido[2,3-b]pyrazin-6-yl]-harnstoff |
| **Verbindung 48** | 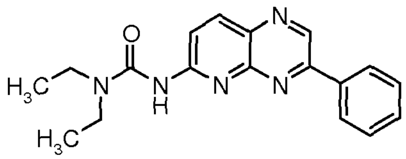 | 1,1-Diethyl-3-(3-phenyl-pyrido[2,3-b]pyrazin-6-yl)-harnstoff |
| **Verbindung 49** | 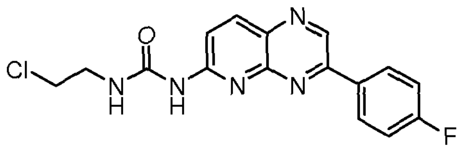 | 1-(2-Chlor-ethyl)-3-[3-(4-fluor-phenyl)-pyrido[2,3-b]pyrazin-6-yl]-harnstoff |
| **Verbindung 50** | 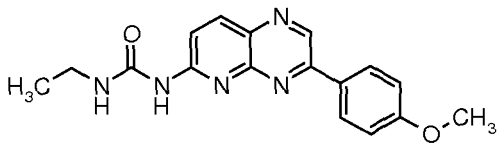 | 1-Ethyl-3-[3-(4-methoxy-phenyl)-pyrido[2,3-b]pyrazin-6-yl]-harnstoff |
| **Verbindung 51** | 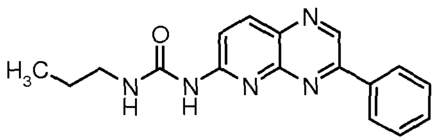 | 1-(3-Phenyl-pyrido[2,3-b]pyrazin-6-yl)-3-propyl-harnstoff |
| **Verbindung 52** | 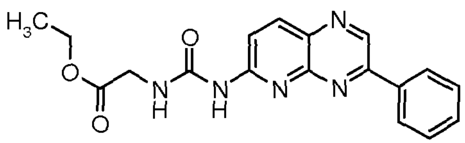 | [3-(3-Phenyl-pyrido[2,3-b]pyrazin-6-yl)-ureido]-essigsäure-ethyl-ester |
| **Verbindung 53** | 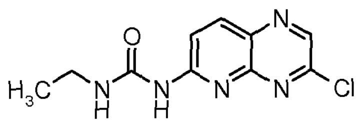 | 1-(3-Chlor-pyrido[2,3-b]pyrazin-6-yl)-3-ethyl-harnstoff |

(fortgesetzt)

| | | |
|---|---|---|
| **Verbindung 54** | 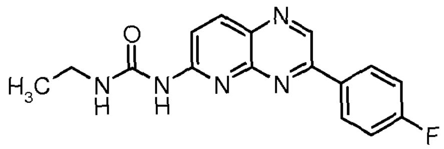 | 1-Ethyl-3-[3-(4-fluor-phenyl)-pyrido[2,3-b]pyrazin-6-yl]-harnstoff |
| **Verbindung 55** | 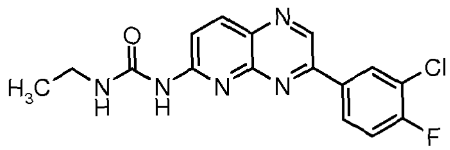 | 1-[3-(3-Chlor-4-fluorphenyl)-pyrido[2,3-b]pyrazin-6-yl]-3-ethyl-harnstoff |
| **Verbindung 56** | 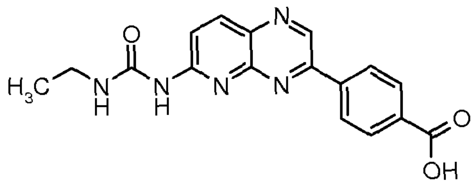 | 4-[6-(3-Ethyl-ureido)-pyrido[2,3-b]pyrazin-3-yl]-benzoesäure |
| **Verbindung 57** | 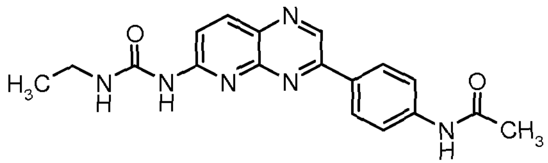 | N-{4-[6-(3-Ethyl-ureido)-pyrido[2,3-b]pyrazin-3-yl]-phenyl}-acetamide |
| **Verbindung 58** | 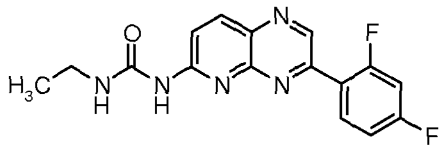 | 1-[3-(2,4-Difluor-phenyl)-pyrido[2,3-b]pyrazin-6-yl]-3-ethyl-harnstoff |
| **Verbindung 59** | 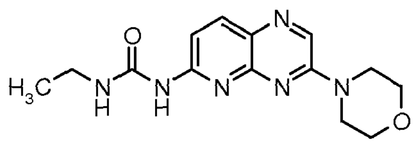 | 1-Ethyl-3-(3-morpholin-4-yl-pyrido[2,3-b]pyrazin-6-yl)-harnstoff |

(fortgesetzt)

| | | |
|---|---|---|
| **Verbindung 60** | 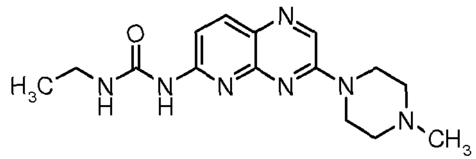 | 1-Ethyl-3-[3-(4-methyl-piperazin-1-yl)-pyrido[2,3-b]pyrazin-6-yl]-harnstoff |
| **Verbindung 61** | 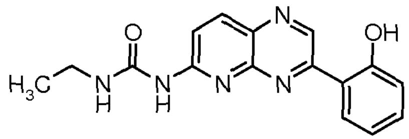 | 1-Ethyl-3-[3-(2-hydroxyphenyl)-pyrido[2,3-b]pyrazin-6-yl]-harnstoff |
| **Verbindung 62** | 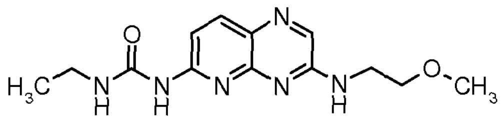 | 1-Ethyl-3-[3-(2-methoxy-ethylamino)-pyrido[2,3-b]pyrazin-6-yl]-harnstoff |
| **Verbindung 63** | 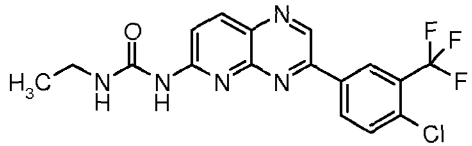 | 1-[3-(4-Chlor-3-trifluormethyl-phenyl)-pyrido[2,3-b]pyrazin-6-yl]-3-ethyl-harnstoff |
| **Verbindung 64** | 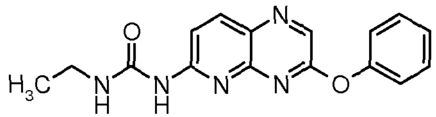 | 1-Ethyl-3-(3-phenoxy-pyrido[2,3-b]pyrazin-6-yl)-harnstoff |
| **Verbindung 65** | 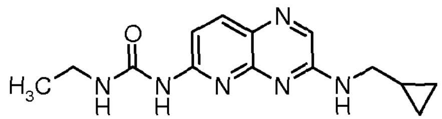 | 1-[3-(Cyclopropylmethyl-amino)-pyrido[2,3-b]pyrazin-6-yl]-3-ethyl-harnstoff |
| **Verbindung 66** | 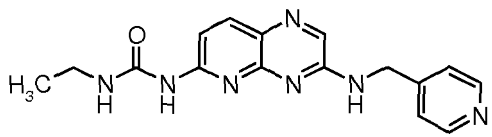 | 1-Ethyl-3-{3-[(pyridin-4-ylmethyl)-amino]- |

(fortgesetzt)

| | | |
|---|---|---|
| **Verbindung 67** | 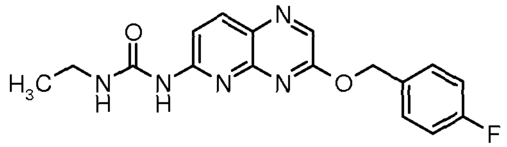 | 1-Ethyl-3-[3-(4-fluor-benzyloxy)-pyrido[2,3-b]pyrazin-6-yl]-harnstoff |
| **Verbindung 68** | 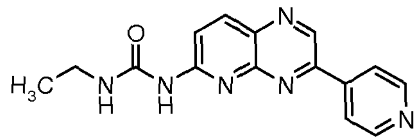 | 1-Ethyl-3-(3-pyridin-4-yl-pyrido[2,3-b]pyrazin-6-yl)-harnstoff |
| **Verbindung 69** | 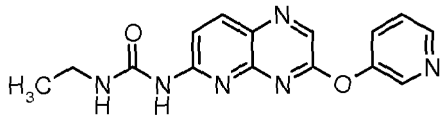 | 1-Ethyl-3-[3-(pyridin-3-yloxy)-pyrido[2,3-b]pyrazin-6-yl]-harnstoff |
| **Verbindung 70** | 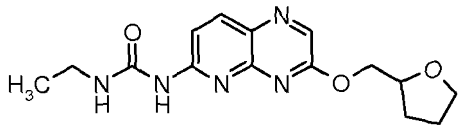 | 1-Ethyl-3-[3-(tetrahydrofuran-2-ylmethoxy)-pyrido[2,3-b]pyrazin-6-yl]-harnstoff |
| **Verbindung 71** | 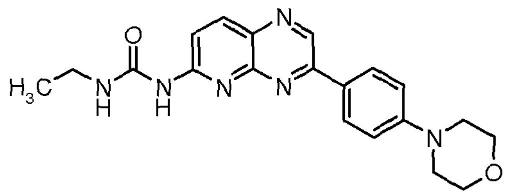 | 1-Ethyl-3-[3-(4-morpholin-4-yl-phenyl)-pyrido[2,3-b]pyrazin-6-yl]-harnstoff |
| **Verbindung 72** | 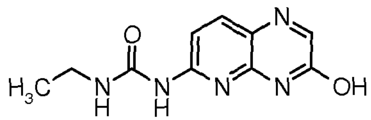 | 1-Ethyl-3-(3-hydroxy-pyrido[2,3-b]pyrazin-6-yl)-harnstoff |
| **Verbindung 73** | 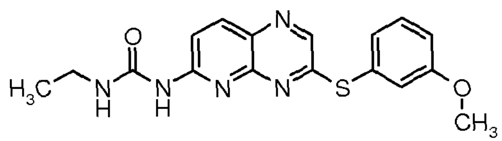 | 1-Ethyl-3-[3-(3-methoxy-phenylsulfanyl)-pyrido[2,3-b]pyrazin-6-yl]-harnstoff |

(fortgesetzt)

| | | |
|---|---|---|
| **Verbindung 74** | 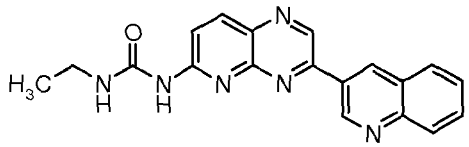 | 1-Ethyl-3-(3-quinolin-3-yl-pyrido[2,3-b]pyrazin-6-yl)-harnstoff |
| **Verbindung 75** | 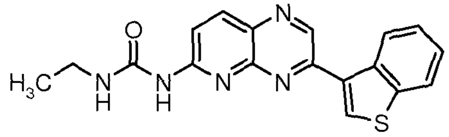 | 1-(3-Benzo[b]thiophen-3-yl-pyrido[2,3-b]pyrazin-6-yl)-3-ethyl-harnstoff |
| **Verbindung 76** | 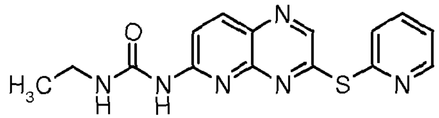 | 1-Ethyl-3-[3-(pyridin-2-ylsulfanyl)-pyrido[2,3-b]pyrazin-6-yl]-harnstoff |
| **Verbindung 77** | 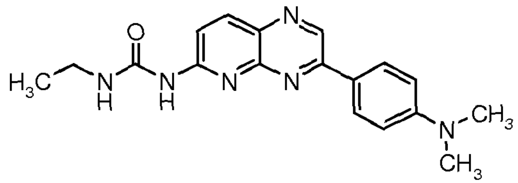 | 1-[3-(4-Dimethylamino-phenyl)-pyrido[2,3-b]pyrazin-6-yl]-3-ethyl-harnstoff |
| **Verbindung 78** | 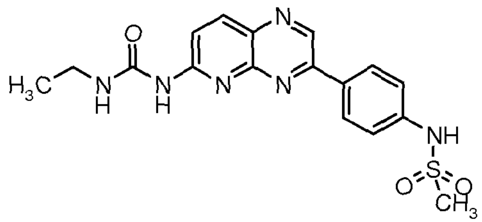 | N-{4-[6-(3-Ethyl-ureido)-pyrido[2,3-b]pyrazin-3-yl]-phenyl}-methansulfonamid |
| **Verbindung 79** | 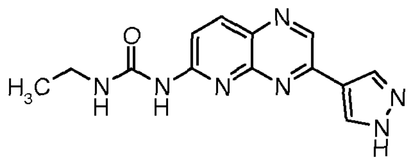 | 1-Ethyl-3-[3-(1 H-pyrazol-4-yl)-pyrido[2,3-b]pyrazin-6-yl]-harnstoff |

(fortgesetzt)

| | | |
|---|---|---|
| **Verbindung 80** | 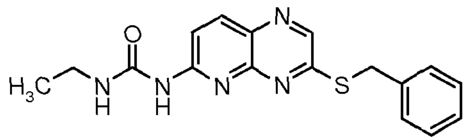 | 1-(3-Benzylsulfanyl-pyrido[2,3-b]pyrazin-6-yl)-3-ethyl-harnstoff |
| **Verbindung 81** | 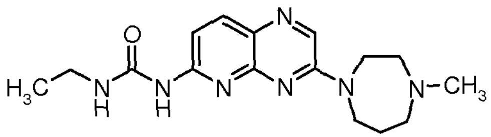 | 1-Ethyl-3-[3-(4-methyl-[1,4]diazepan-1-yl)-pyrido[2,3-b]pyrazin-6-yl]-harnstoff |
| **Verbindung 82** | 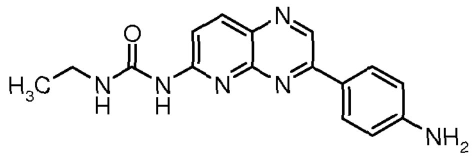 | 1-[3-(4-Amino-phenyl)-pyrido[2,3-b]pyrazin-6-yl]-3-ethyl-harnstoff |
| **Verbindung 83** | 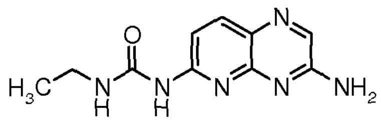 | 1-(3-Amino-pyrido[2,3-b]pyrazin-6-yl)-3-ethyl-harnstoff |
| **Verbindung 84** | 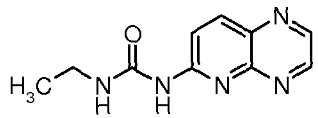 | 1-Ethyl-3-pyrido[2,3-b]pyrazin-6-yl-harnstoff |
| **Verbindung 85** | 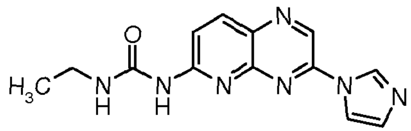 | 1-Ethyl-3-(3-imidazol-1-yl-pyrido[2,3-b]pyrazin-6-yl)-harnstoff |
| **Verbindung 86** | 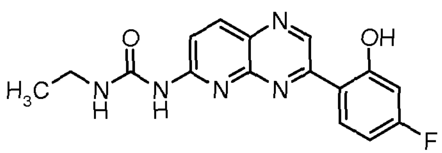 | 1-Ethyl-3-[3-(4-fluor-2-hydroxy-phenyl)-pyrido[2,3-b]pyrazin-6-yl]-harnstoff |

(fortgesetzt)

| | | |
|---|---|---|
| **Verbindung 87** | 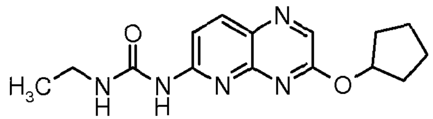 | 1-(3-Cyclopentyloxy-pyrido[2,3-b]pyrazin-6-yl)-3-ethyl-harnstoff |
| **Verbindung 88** | 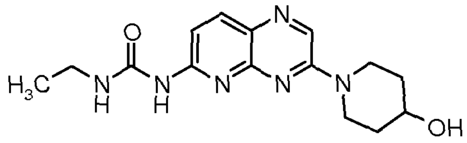 | 1-Ethyl-3-[3-(4-hydroxy-piperidin-1-yl)-pyrido[2,3-b]pyrazin-6-yl]-harnstoff |
| **Verbindung 89** | 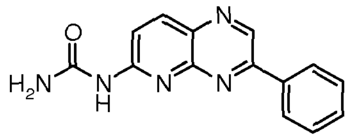 | (3-Phenyl-pyrido[2,3-b]pyrazin-6-yl)-harnstoff |
| **Verbindung 90** | 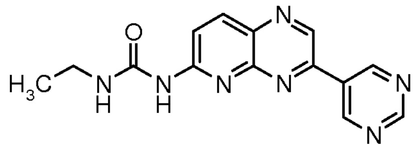 | 1-Ethyl-3-(3-pyrimidin-5-yl-pyrido[2,3-b]pyrazin-6-yl)-harnstoff |
| **Verbindung 91** | 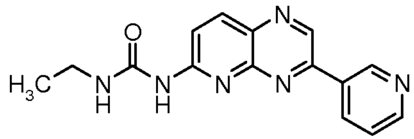 | 1-Ethyl-3-(3-pyridin-3-yl-pyrido[2,3-b]pyrazin-6-yl)-harnstoff |
| **Verbindung 92** | 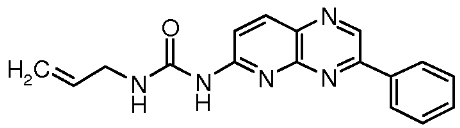 | 1-Allyl-3-(3-phenyl-pyrido[2,3-b]pyrazin-6-yl)-harnstoff |
| **Verbindung 93** | 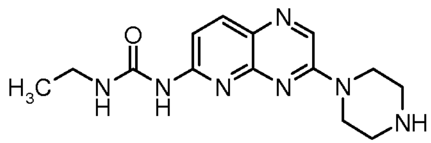 | 1-Ethyl-3-(3-piperazin-1-yl-pyrido[2,3-b]pyrazin-6-yl)-harnstoff |

(fortgesetzt)

| | | |
|---|---|---|
| **Verbindung 94** | 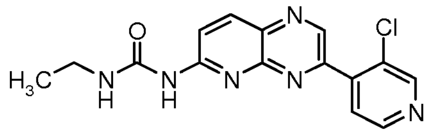 | 1-[3-(3-Chlor-pyridin-4-ylmethyl)-pyrido[2,3-b]pyrazin-6-yl]-3-ethyl-harnstoff |
| **Verbindung 95** | 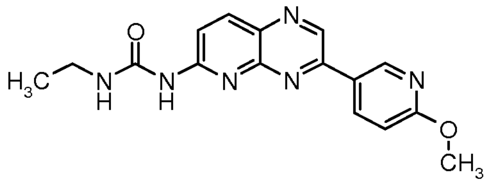 | 1-Ethyl-3-[3-(6-methoxy-pyridin-3-yl)-pyrido[2,3-b]pyrazin-6-yl]-harnstoff |
| **Verbindung 96** | 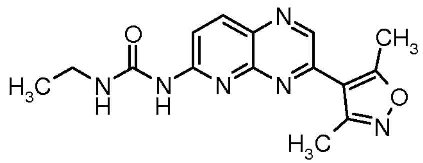 | 1-[3-(3,5-Dimethyl-isoxazol-4-yl)-pyrido[2,3-b]pyrazin-6-yl]-3-ethyl-harnstoff |
| **Verbindung 97** | 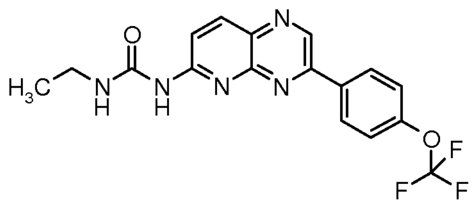 | 1-Ethyl-3-[3-(4-trifluormethoxy-phenyl)-pyrido[2,3-b]pyrazin-6-yl]-harnstoff |
| **Verbindung 98** | 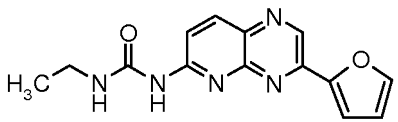 | 1-Ethyl-3-(3-furan-2-yl-pyrido[2,3-b]pyrazin-6-yl)-harnstoff |
| **Verbindung 99** | 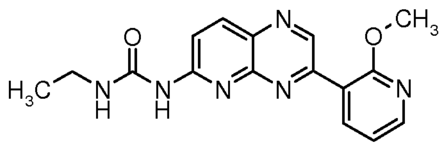 | 1-Ethyl-3-[3-(2-methoxy-pyridin-3-yl)-pyrido[2,3-b]pyrazin-6-yl]-harnstoff |

(fortgesetzt)

| | | |
|---|---|---|
| **Verbindung 100** | 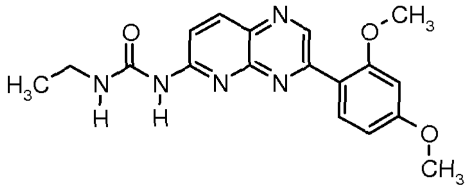 | 1-[3-(2,4-Dimethoxyphenyl)-pyrido[2,3-b]pyrazin-6-yl]-3-ethyl-harnstoff |
| **Verbindung 101** | 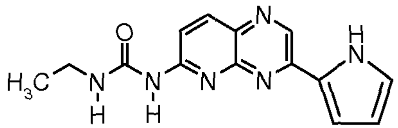 | 1-Ethyl-3-[3-(1H-pyrrol-2-yl)-pyrido[2,3-b]pyrazin-6-yl]-harnstoff |
| **Verbindung 102** | 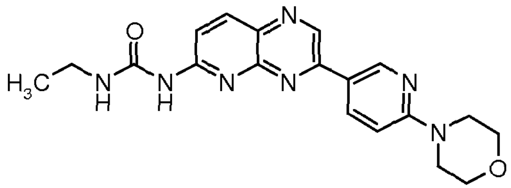 | 1-Ethyl-3-[3-(6-morpholin-4-yl-pyridin-3-yl)-pyrido[2,3-b]pyrazin-6-yl]-harnstoff |
| **Verbindung 103** | 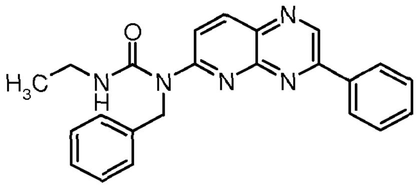 | 1-Benzyl-3-ethyl-1-(3-phenyl-pyrido[2,3-b]pyrazin-6-yl)-harnstoff |
| **Verbindung 104** | 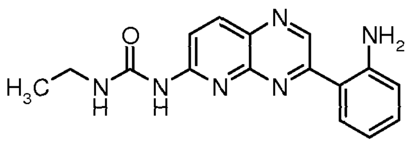 | 1-[3-(2-Amino-phenyl)-pyrido[2,3-b]pyrazin-6-yl]-3-ethyl-harnstoff |
| **Verbindung 105** | 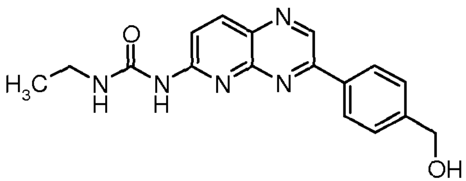 | 1-Ethyl-3-[3-(4-hydroxymethyl-phenyl)-pyrido[2,3-b]pyrazin-6-yl]-harnstoff |

(fortgesetzt)

| | | |
|---|---|---|
| **Verbindung 106** | 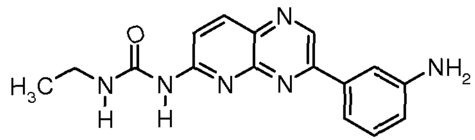 | 1-[3-(3-Amino-phenyl)-pyrido[2,3-b]pyrazin-6-yl]-3-ethyl-harnstoff |
| **Verbindung 107** | 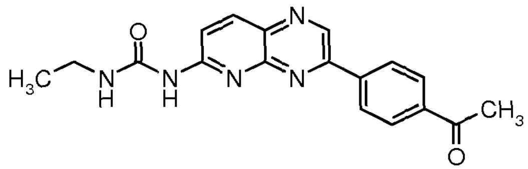 | 1-[3-(4-Acetyl-phenyl)-pyrido[2,3-b]pyrazin-6-yl]-3-ethyl-harnstoff |
| **Verbindung 108** | 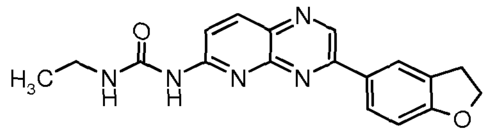 | 1-[3-(2,3-Dihydro-benzofuran-5-yl)-pyrido[2,3-b]pyrazin-6-yl]-3-ethyl-harnstoff |
| **Verbindung 109** | 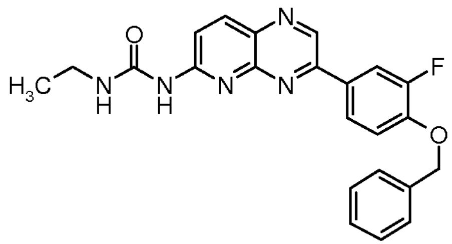 | 1-[3-(4-Benzyloxy-3-fluor-phenyl)-pyrido[2,3-b]pyrazin-6-yl]-3-ethyl-harnstoff |
| **Verbindung 110** | 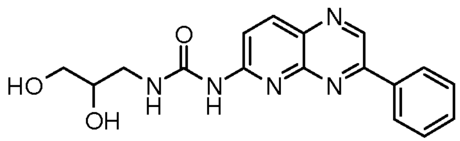 | 1-(2,3-Dihydroxy-propyl)-3-(3-phenyl-pyrido[2,3-b]pyrazin-6-yl)-harnstoff |
| **Verbindung 111** | 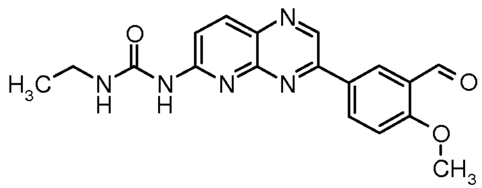 | 1-Ethyl-3-[3-(3-formyl-4-methoxy-phenyl)-pyrido[2,3-b]pyrazin-6-yl]-harnstoff |

(fortgesetzt)

| | | |
|---|---|---|
| **Verbindung 112** | 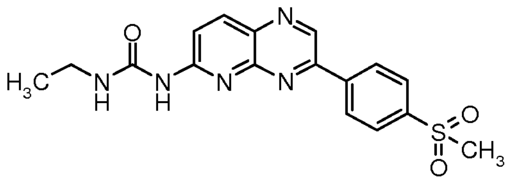 | 1-Ethyl-3-[3-(4-methansulfonyl-phenyl)-pyrido[2,3-b]pyrazin-6-yl]-harnstoff |
| **Verbindung 113** | 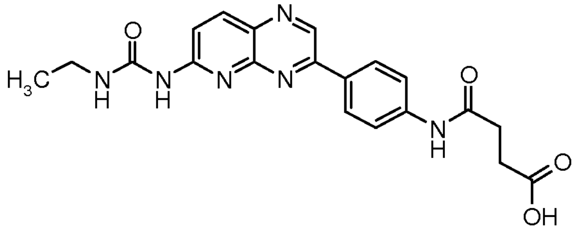 | N-{4-[6-(3-Ethyl-ureido)-pyrido[2,3-b]pyrazin-3-yl]-phenyl}-succinamidsäure |
| **Verbindung 114** | 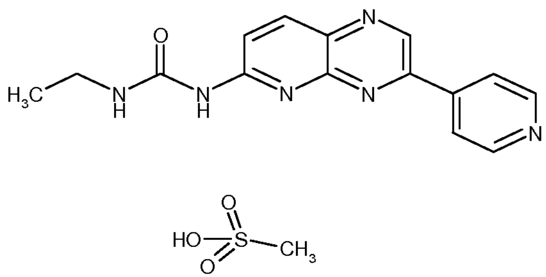 | 1-Ethyl-3-(3-pyridin-4-yl-pyrido[2,3-b]pyrazin-6-yl)-harnstoff Methansulfonsäuresalz (freie Base) |
| **Verbindung 115** | 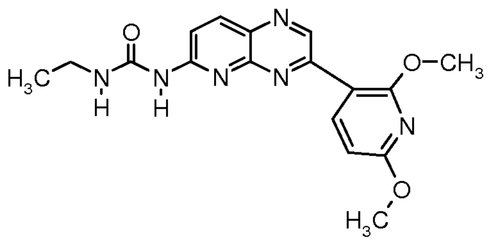 | 1-[3-(2,6-Dimethoxy-pyridin-3-yl)-pyrido[2,3-b]pyrazin-6-yl]-3-ethyl-harnstoff |

(fortgesetzt)

| | | |
|---|---|---|
| **Verbindung 116** | 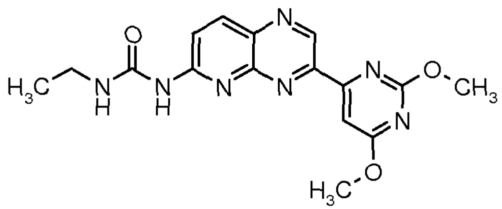 | 1-[3-(2,6-Dimethoxy-pyrimidin-4-yl)-pyrido[2,3-b]pyrazin-6-yl]-3-ethyl-harnstoff |
| **Verbindung 117** | 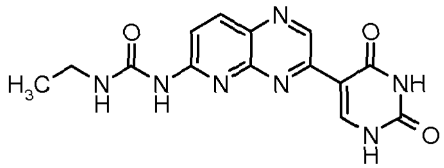 | 1-[3-(2,4-Dioxo-1,2,3,4-tetrahydro-pyrimidin-5-yl)-pyrido[2,3-b]pyrazin-6-yl]-3-ethyl-harnstoff |
| **Verbindung 118** | 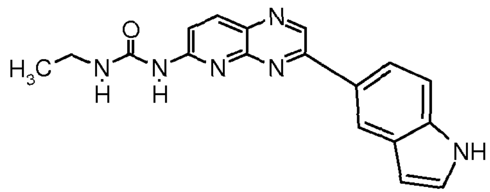 | 1-Ethyl-3-[3-(1H-indol-5-yl)-pyrido[2,3-b]pyrazin-6-yl]-harnstoff |
| **Verbindung 119** | 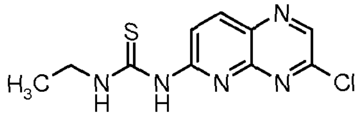 | 1-(3-Chloro-pyrido[2,3-b]pyrazin-6-yl)-3-ethyl-thioharnstoff |
| **Verbindung 120** | 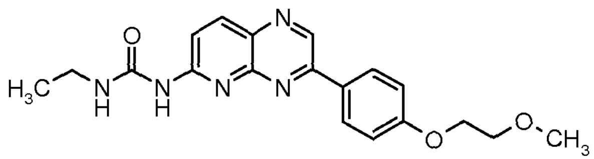 | 1-Ethyl-3-{3-[4-(2-methoxy-ethoxy)-phenyl]-pyrido[2,3-b]pyrazin-6-yl]-harnstoff |
| **Verbindung 121** | 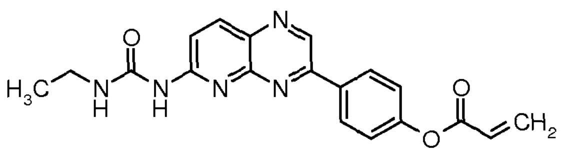 | Acrylsäure-4-[6-(3-ethyl-ureido)-pyrido[2,3-b]-pyrazin-3-yl]-phenylester |

(fortgesetzt)

| | | |
|---|---|---|
| **Verbindung 122** | 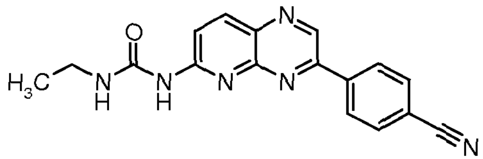 | 1-[3-(4-Cyano-phenyl)-pyrido[2,3-b]pyrazin-6-yl]-3-ethyl-harnstoff |
| **Verbindung 123** | 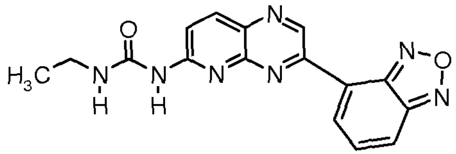 | 1-(3-Benzo[1,2,5]oxadiazol-4-yl-pyrido[2,3-b]pyrazin-6-yl)-3-ethyl-harnstoff |
| **Verbindung 124** | 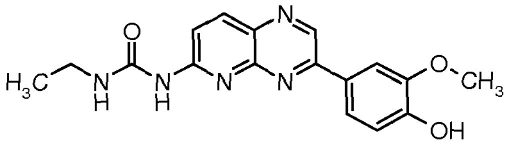 | 1-Ethyl-3-[3-(4-hydroxy-3-methoxy-phenyl)-pyrido[2,3-b]pyrazin-6-yl]-harnstoff |
| **Verbindung 125** | 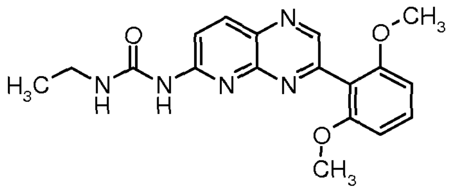 | 1-[3-(2,6-Dimethoxyphenyl)-pyrido [2,3-b]pyrazin-6-yl]-3-ethyl-harnstoff |
| **Verbindung 126** | 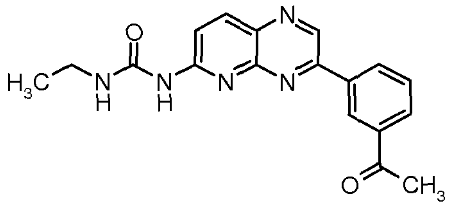 | 1-[3-(3-Acetyl-phenyl)-pyrido[2,3-b]pyrazin-6-yl]-3-ethyl-harnstoff |
| **Verbindung 127** | 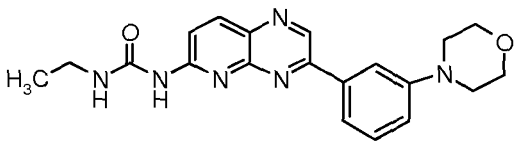 | 1-Ethyl-3-[3-(3-morpholin-4-yl-phenyl)-pyrido[2,3-b]pyrazin-6-yl]-harnstoff |

(fortgesetzt)

| | | |
|---|---|---|
| **Verbindung 128** | 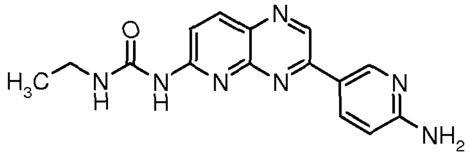 | 1-[3-(6-Amino-pyridin-3-yl)-pyrido[2,3-b]pyrazin-6-yl]-3-ethyl-harnstoff |
| **Verbindung 129** | 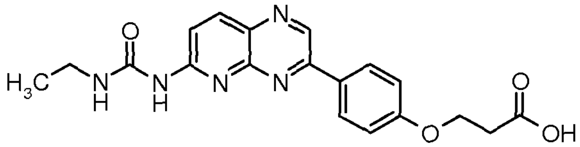 | 3-{4-[6-(3-Ethyl-ureido)-pyrido[2,3-b]-pyrazin-3-yl]-phenoxy}-propionsäure |
| **Verbindung 130** | 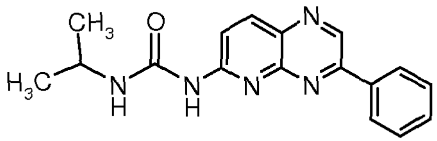 | 1-Isopropyl-3-(3-phenyl-pyrido[2,3-b]pyrazin-6-yl)-harnstoff |
| **Verbindung 131** | 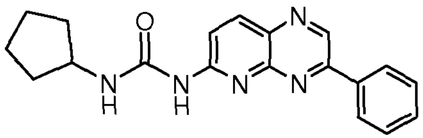 | 1-Cyclopentyl-3-(3-phenyl-pyrido[2,3-b]pyrazin-6-yl)-harnstoff |
| **Verbindung 132** | 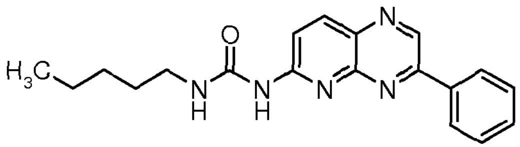 | 1-Pentyl-3-(3-phenyl-pyrido[2,3-b]pyrazin-6-yl)-harnstoff |
| **Verbindung 133** | 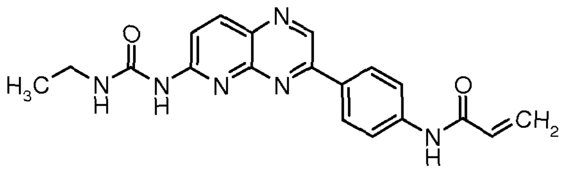 | N-{4-[6-(3-Ethyl-ureido)-pyrido[2,3-b]pyrazin-3-yl]-phenyl}-acrylamid |
| **Verbindung 134** | 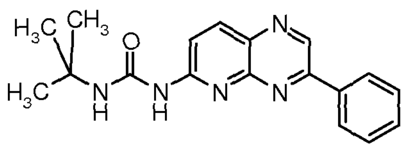 | 1-tert-Butyl-3-(3-phenyl-pyrido[2,3-b]pyrazin-6-yl)-harnstoff |

(fortgesetzt)

| | | |
|---|---|---|
| **Verbindung 135** | 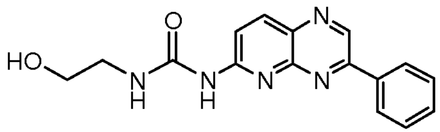 | 1-(2-Hydroxy-ethyl)-3-(3-phenyl-pyrido[2,3-b]pyrazin-6-yl)-harnstoff |
| **Verbindung 136** | 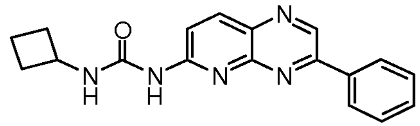 | 1-Cyclobutyl-3-(3-phenyl-pyrido[2,3-b]pyrazin-6-yl)-harnstoff |
| **Verbindung 137** | 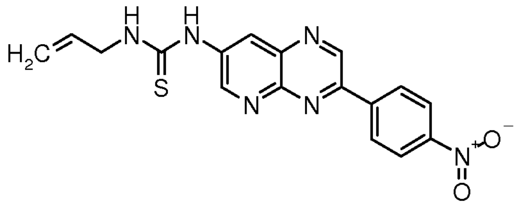 | 1-Allyl-3-[3-(4-nitro-phenyl)-pyrido[2,3-b]pyrazin-7-yl]-thioharnstoff |
| **Verbindung 138** | 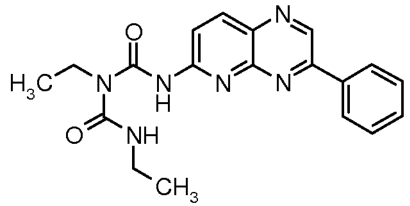 | 1-Ethyl-1-(Ethylcarbamoyl)-3-(3-phenyl-pyrido[2,3-b]pyrazin-6-yl)-harnstoff |
| **Verbindung 139** | 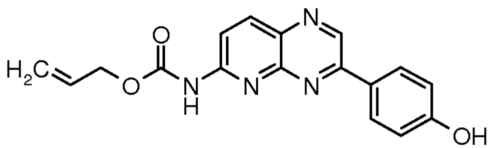 | [3-(4-Hydroxy-phenyl)-pyrido[2,3-b]pyrazin-6-yl]-carbaminsäure-allyl-ester |
| **Verbindung 140** | 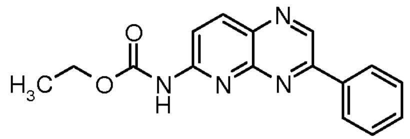 | (3-Phenyl-pyrido[2,3-b]pyrazin-6-yl)-carbaminsäure-ethylester |

(fortgesetzt)

| | | |
|---|---|---|
| **Verbindung 141** | 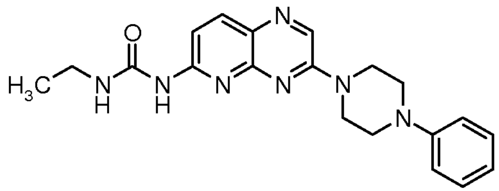 | 1-Ethyl-3-[3-(4-phenyl-piperazin-1-yl)-pyrido[2,3-b]pyrazin-6-yl]-harnstoff |
| **Verbindung 142** | 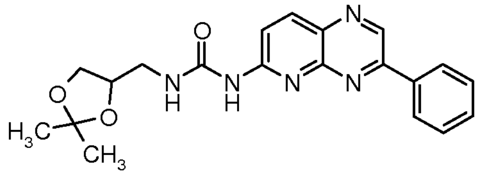 | 1-(2,2-Dimethyl-[1,3]dioxolan-4-ylmethyl)-3-(3-phenyl-pyrido[2,3-b]pyrazin-6-yl)-harnstoff |
| **Verbindung 143** | 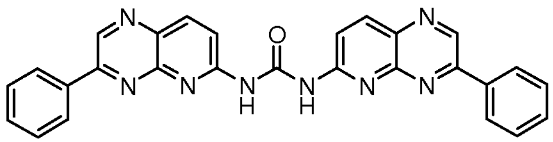 | 1,3-Bis-(3-phenyl-pyrido[2,3-b]pyrazin-6-yl)-harnstoff |
| **Verbindung 144** | 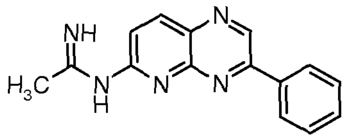 | N-(3-Phenyl-pyrido[2,3-b]pyrazin-6-yl)-acetamidin |
| **Verbindung 145** | 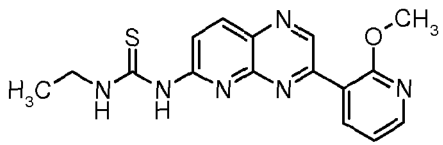 | 1-Ethyl-3-[3-(2-methoxy-pyridin-3-yl)-pyrido[2,3-b]pyrazin-6-yl]-thioharnstoff |
| **Verbindung 146** | 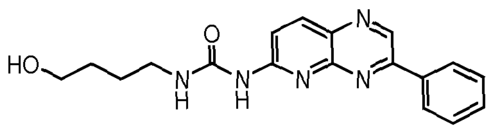 | 1-(4-Hydroxy-butyl)-3-(3-phenyl-pyrido[2,3-b]pyrazin-6-yl)-harnstoff |
| **Verbindung 147** | 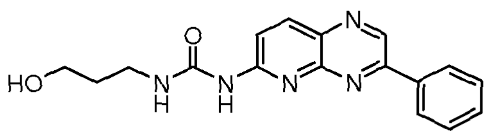 | 1-(3-Hydroxy-propyl)-3-(3-phenyl-py rido[2,3-b]pyrazin-6-yl)-harnstoff |

(fortgesetzt)

| | | |
|---|---|---|
| **Verbindung 148** | 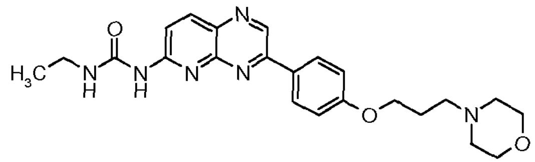 | 1-Ethyl-3-{3-[4-(3-morpholin-4-yl- propoxy)-phenyl]-pyrido[2,3-b]pyrazin-6-yl}-harnstoff |
| **Verbindung 149** | 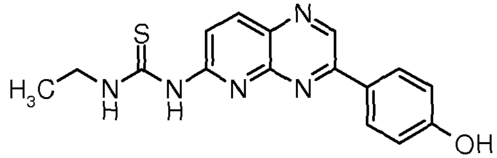 | 1-Ethyl-3-[3-(4-hydroxyphenyl)- pyrido[2,3-b]pyrazin-6-yl]-thioharnstoff |
| **Verbindung 150** | 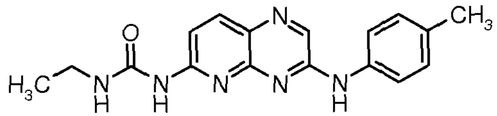 | 1-Ethyl-3-(3-p-tolylamino-pyrido[2,3-b]pyrazin-6-yl)-harnstoff |
| **Verbindung 151** | 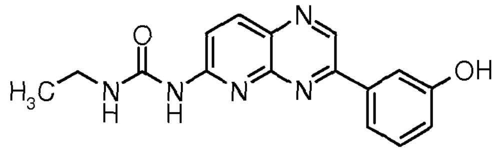 | 1-Ethyl-3-[3-(3-hydroxyphenyl)- pyrido[2,3-b]pyrazin-6-yl]-harnstoff |
| **Verbindung 152** | 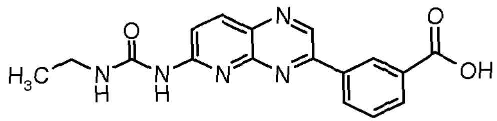 | 3-[6-(3-Ethyl-ureido)-pyrido[2,3-b]pyrazin-3-yl]-benzoesäure |
| **Verbindung 153** | 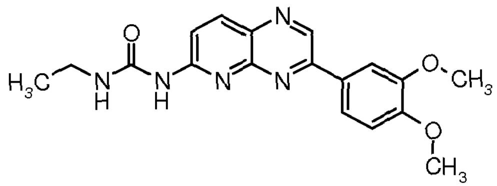 | 1-[3-(3,4-Dimethoxyphenyl)-pyrido [2,3-b]pyrazin-6-yl]-3-ethyl-harnstoff |

(fortgesetzt)

| | | |
|---|---|---|
| **Verbindung 154** | 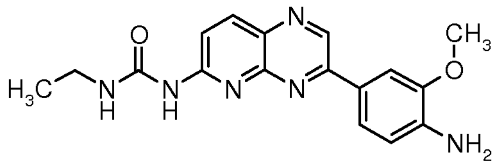 | 1-[3-(4-Amino-3-methoxy-phenyl)-pyrido[2,3-b]pyrazin-6-yl]-3-ethyl-harnstoff |
| **Verbindung 155** | 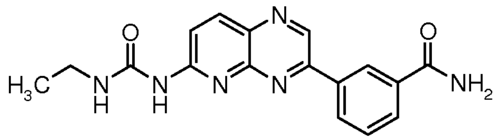 | 3-[6-(3-Ethyl-ureido)-pyrido[2,3-b]-pyrazin-3-yl]-benzamid |
| **Verbindung 156** | 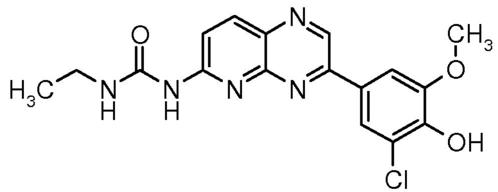 | 1-[3-(3-Chlor-4-hydroxy-5-methoxy-phenyl)-pyrido[2,3-b]pyrazin-6-yl]-3-ethyl-harnstoff |
| **Verbindung 157** | 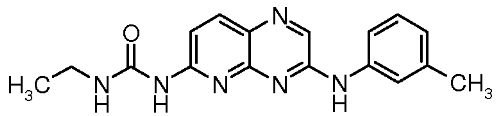 | 1-Ethyl-3-(3-m-tolylamino-pyrido-[2,3-b]pyrazin-6-yl)-harnstoff |
| **Verbindung 158** | 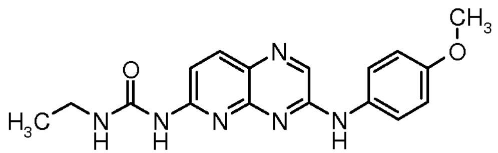 | 1-Ethyl-3-[3-(4-methoxy-phenylamino)-pyrido[2,3-b]pyrazin-6-yl]-harnstoff |
| **Verbindung 159** | 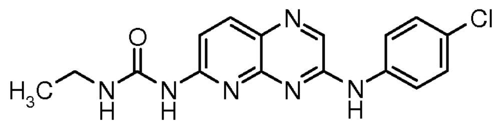 | 1-[3-(4-Chloro-phenylamino)-pyrido-[2,3-b]pyrazin-6-yl]-3-ethyl-harnstoff |
| **Verbindung 160** | 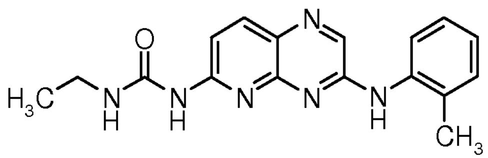 | 1-Ethyl-3-(3-o-tolylamino-pyrido[2,3-b]-pyrazin-6-yl)-harnstoff |

(fortgesetzt)

| | | |
|---|---|---|
| **Verbindung 161** | 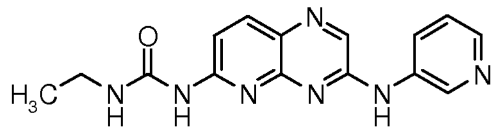 | 1-Ethyl-3-[3-(pyridin-3-ylamino)-pyrido-[2,3-b]pyrazin-6-yl]-harnstoff |
| **Verbindung 162** | 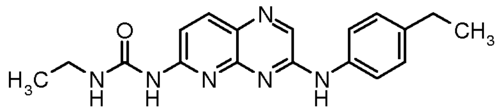 | 1-Ethyl-3-[3-(4-ethyl-phenylamino)-pyrido[2,3-b]pyrazin-6-yl]-harnstoff |
| **Verbindung 163** | 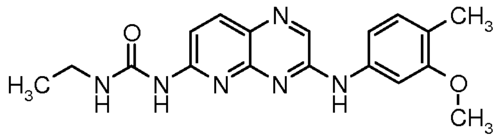 | 1-Ethyl-3-[3-(3-methoxy-4-methyl-phenylamino)-pyrido[2,3-b]pyrazin-6-yl]-harnstoff |
| **Verbindung 164** | 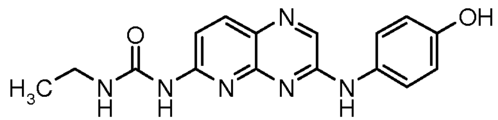 | 1-Ethyl-3-[3-(4-hydroxy-phenylamino)-pyrido[2,3-b]pyrazin-6-yl]-harnstoff |
| **Verbindung 165** | 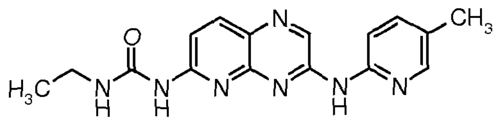 | 1-Ethyl-3-[3-(5-methyl-pyridin-2-yl-amino)-pyrido[2,3-b]pyrazin-6-yl]-harnstoff |
| **Verbindung 166** | 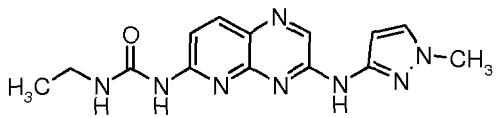 | 1-Ethyl-3-[3-(1-methyl-1H-pyrazol-3-ylamino)-pyrido[2,3-b]pyrazin-6-yl]-harnstoff |
| **Verbindung 167** | 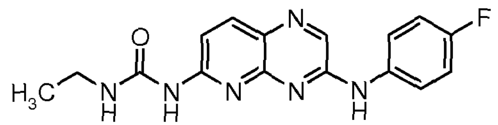 | 1-Ethyl-3-[3-(4-fluor-phenylamino)-pyrido[2,3-b]pyrazin-6-yl]-harnstoff |
| **Verbindung 168** | 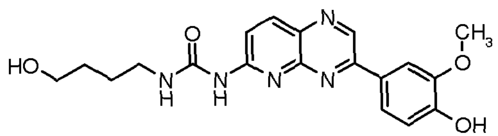 | 1-(4-Hydroxy-butyl)-3-[3-(4-hydroxy-3-methoxy-phenyl)-pyrido[2,3-b]pyrazin-6-yl]-harnstoff |

(fortgesetzt)

| | | |
|---|---|---|
| **Verbindung 169** | 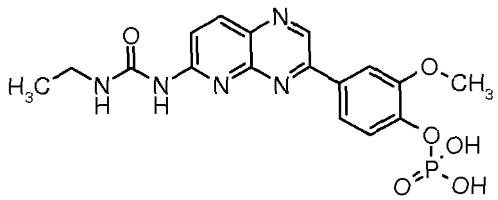 | Phosphorsäure-mono-{4-[6-(3-ethyl-ureido)-pyrido-[2,3-b]pyrazin-3-yl]-2-methoxy-phenyl}-ester |
| **Verbindung 170** | 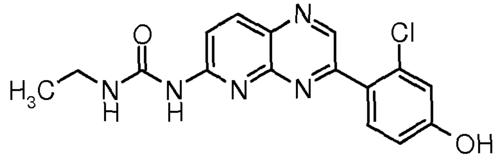 | 1-[3-(2-Chlor-4-hydroxy-phenyl)-pyrido[2,3-b]pyrazin-6-yl]-3-ethyl-harnstoff |
| **Verbindung 171** | 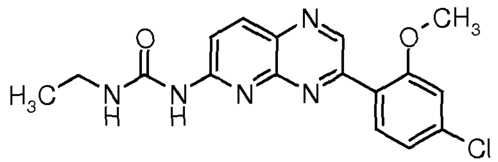 | 1-[3-(4-Chlor-2-methoxy-phenyl)-pyrido[2,3-b]pyrazin-6-yl]-3-ethyl-harnstoff |
| **Verbindung 172** | 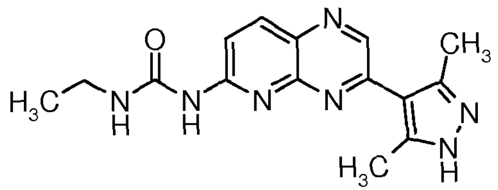 | 1-[3-(3,5-Dimethyl-1H-pyrazol-4-yl)-pyrido[2,3-b]pyrazin-6-yl]-3-ethyl-harnstoff |
| **Verbindung 173** | 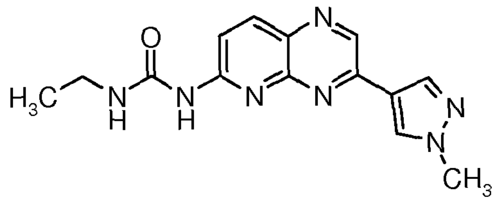 | 1-Ethyl-3-[3-(1-methyl-1H-pyrazol-4-yl)-pyrido[2,3-b]pyrazin-6-yl]-harnstoff |
| **Verbindung 174** | 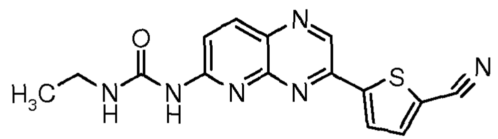 | 1-[3-(5-Cyano-thiophen-2-yl)-pyrido[2,3-b]pyrazin-6-yl]-3-ethyl-harnstoff |

(fortgesetzt)

| | | |
|---|---|---|
| **Verbindung 175** | 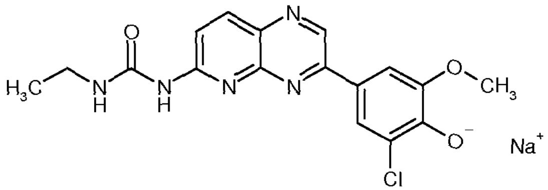 | Natrium 2-chlor-4-[6-(3-ethyl-ureido)-pyrido[2,3-b]pyrazin-3-yl]-6-methoxy-phenolat |
| **Verbindung 176** | 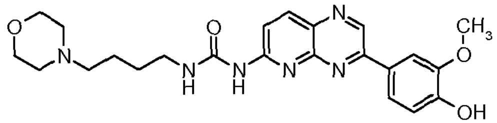 | 1-[3-(4-Hydroxy-3-methoxy-phenyl)-pyrido[2,3-b]pyrazin-6-yl]-3-(4-morpholin-4-yl-butyl)-harnstoff |
| **Verbindung 177** | 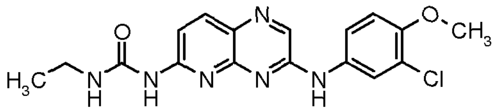 | 1-[3-(3-Chlor-4-methoxy-phenylamino)-pyrido[2,3-b]pyrazin-6-yl]-3-ethyl-harnstoff |
| **Verbindung 178** | 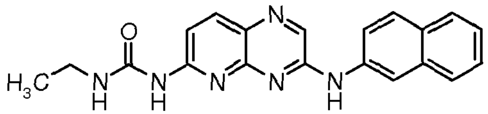 | 1-Ethyl-3-[3-(naphthalin-2-ylamino)-pyrido[2,3-b]pyrazin-6-yl]-harnstoff |
| **Verbindung 179** | 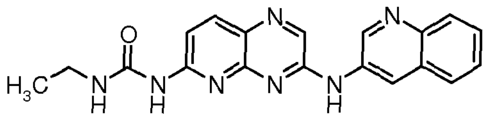 | 1-Ethyl-3-[3-(chinolin-3-ylamino)-pyrido[2,3-b]pyrazin-6-yl]-harnstoff |
| **Verbindung 180** | 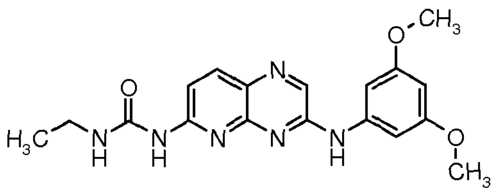 | 1-[3-(3,5-Dimethoxy-phenylamino)-pyrido[2,3-b]pyrazin-6-yl]-3-ethyl-harnstoff |
| **Verbindung 181** | 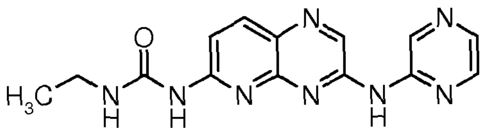 | 1-Ethyl-3-[3-(pyrazin-2-ylamino)-pyrido[2,3-b]pyrazin-6-yl]-harnstoff |

(fortgesetzt)

| | | |
|---|---|---|
| **Verbindung 182** | 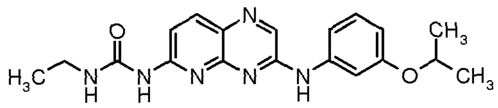 | 1-Ethyl-3-[3-(3-isopropoxy-phenylamino)-pyrido[2,3-b]pyrazin-6-yl]-harnstoff |
| **Verbindung 183** | 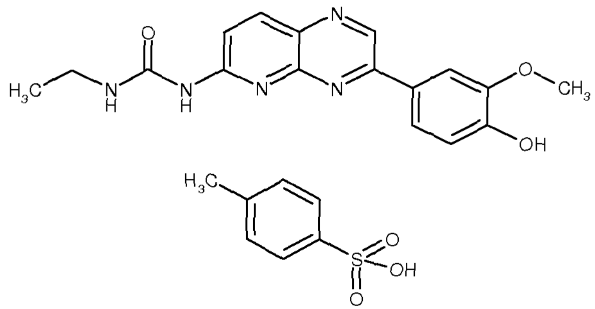 | 1-Ethyl-3-[3-(4-hydroxy-3-methoxy-phenyl)-pyrido[2,3-b]pyrazin-6-yl]-harnstoff p-Toluolsulfonat |
| **Verbindung 184** | 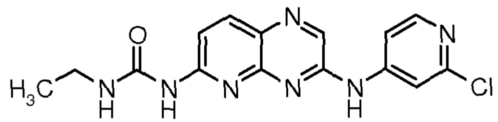 | 1-[3-(2-Chlor-pyridin-4-ylamino)-pyrido[2,3-b]pyrazin-6-yl]-3-ethyl-harnstoff |
| **Verbindung 185** | 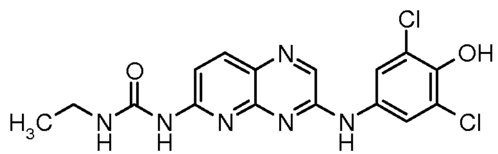 | 1-[3-(3,5-Dichlor-4-hydroxy-phenylamino)-pyrido[2,3-b]pyrazin-6-yl]-3-ethyl-harnstoff |
| **Verbindung 186** | 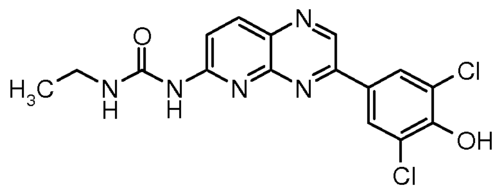 | 1-[3-(3,5-Dichlor-4-hydroxy-phenyl)-pyrido[2,3-b]pyrazin-6-yl]-3-ethyl-harnstoff |
| **Verbindung 187** | 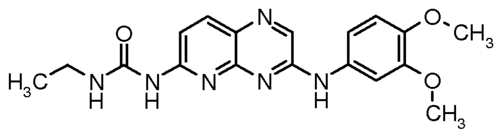 | 1-[3-(3,4-Dimethoxy-phenylamino)-pyrido[2,3-b]pyrazin-6-yl]-3-ethyl-harnstoff |

(fortgesetzt)

| | | |
|---|---|---|
| **Verbindung 188** | 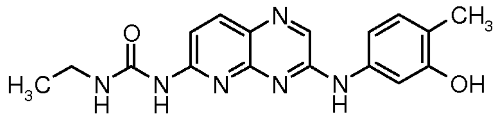 | 1-Ethyl-3-[3-(3-hydroxy-4-methyl-phenylamino)-pyrido[2,3-b]pyrazin-6-yl]-harnstoff |
| **Verbindung 189** | 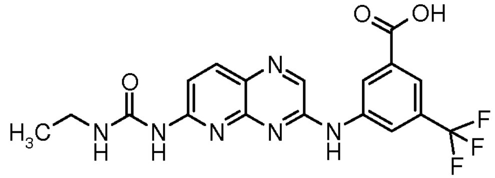 | 3-[6-(3-Ethyl-ureido)-pyrido[2,3-b]-pyrazin-3-ylamino]-5-trifluormethyl-benzoesäure |
| **Verbindung 190** | 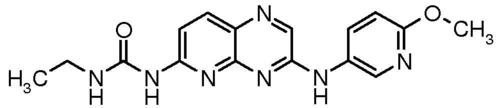 | 1-Ethyl-3-[3-(6-methoxy-pyridin-3-ylamino)-pyrido[2,3-b]pyrazin-6-yl]-harnstoff |
| **Verbindung 191** | 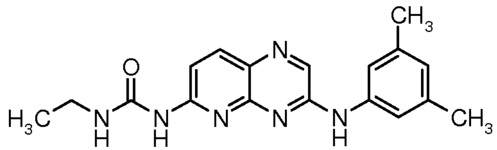 | 1-[3-(3,5-Dimethyl-phenylamino)-pyrido[2,3-b]pyrazin-6-yl]-3-ethyl-harnstoff |
| **Verbindung 192** | 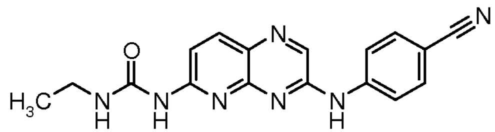 | 1-[3-(4-Cyano-phenylamino)-pyrido-[2,3-b]pyrazin-6-yl]-3-ethyl-harnstoff |
| **Verbindung 193** | 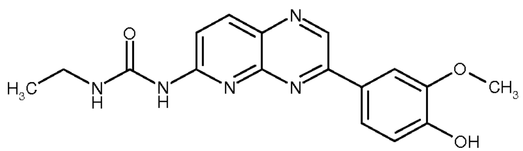 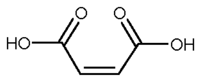 | 1-Ethyl-3-[3-(4-hydroxy-3-methoxy-phenyl)-pyrido[2,3-b]pyrazin-6-yl]-harnstoff (Z)-but-2-endicarbonsäure-Salz |

(fortgesetzt)

| | | |
|---|---|---|
| **Verbindung 194** | 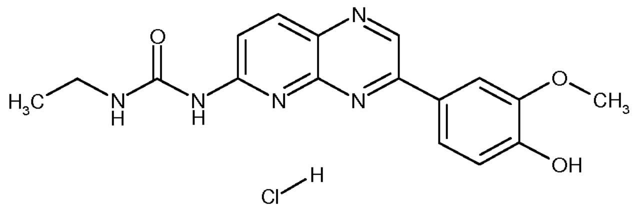 | 1-Ethyl-3-[3-(4-hydroxy-3-methoxy-phenyl)-pyrido[2,3-b]pyrazin-6-yl]-harnstoff Hydrochlorid |
| **Verbindung 195** | 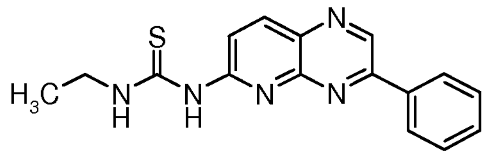 | 1-Ethyl-3-(3-phenyl-pyrido[2,3-b]pyrazin-6-yl)-thioharnstoff |
| **Verbindung 196** | 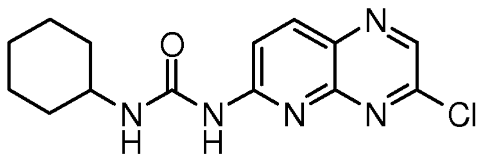 | 1-(3-Chlor-pyrido[2,3-b]pyrazin-6-yl)-3-cyclohexyl-harnstoff |
| **Verbindung 197** | 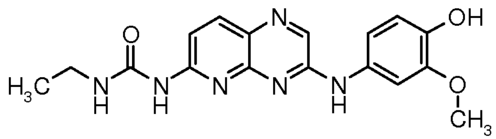 | 1-Ethyl-3-[3-(4-hydroxy-3-methoxy-phenylamino)-pyrido[2,3-b]pyrazin-6-yl]-harnstoff |
| **Verbindung 198** | 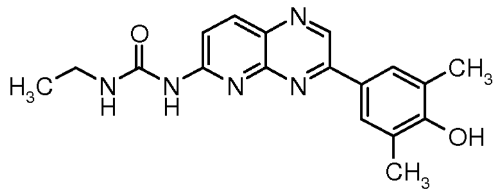 | 1-Ethyl-3-[3-(4-hydroxy-3,5-dimethyl-phenyl)-pyrido[2,3-b]pyrazin-6-yl]-harnstoff |

(fortgesetzt)

| | | |
|---|---|---|
| **Verbindung 199** | 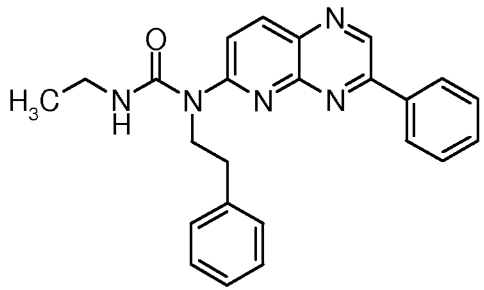 | 3-Ethyl-1-phenethyl-1-(3-phenyl- pyrido[2,3-b]pyrazin-6-yl)-harnstoff |
| **Verbindung 200** | 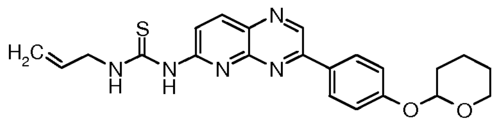 | 1-Allyl-3-{3-[4-(tetrahydro-pyran-2-yloxy)-phenyl]-pyrido[2,3-b]pyrazin-6-yl}-thioharnstoff |
| **Verbindung 201** | 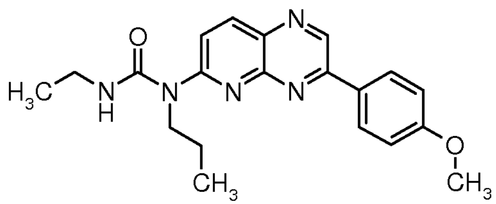 | 3-Ethyl-1-[3-(4-methoxy-phenyl)- pyrido[2,3-b]pyrazin-6-yl]-1-propyl-harnstoff |
| **Verbindung 202** | 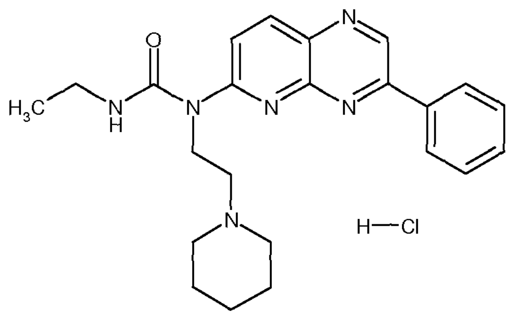 | 3-Ethyl-1-(3-phenyl-pyrido[2,3-b]pyrazin-6-yl)-1-(2-piperidin-1-yl-ethyl)-harnstoff Hydrochlorid |
| **Verbindung 203** | 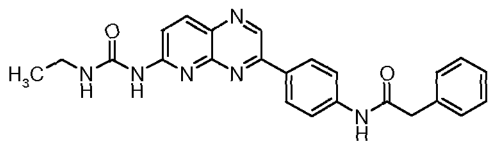 | N-{4-[6-(3-Ethyl-ureido)-pyrido[2,3-b]- pyrazin-3-yl]-phenyl]-2-phenyl-acetamid |

(fortgesetzt)

| | | |
|---|---|---|
| **Verbindung 204** | 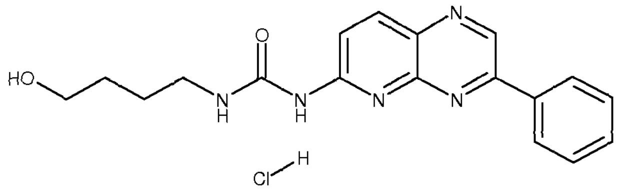 | 1-(4-Hydroxy-butyl)-3-(3-phenyl-pyrido[2,3-b]pyrazin-6-yl)-harnstoff Hydrochlorid |
| **Verbindung 205** | 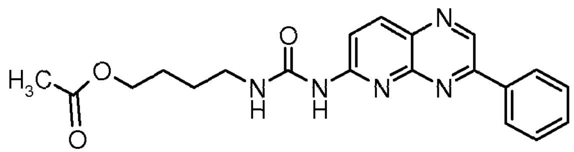 | Essigsäure-4-[3-(3-phenyl-pyrido[2,3-b]pyrazin-6-yl)-ureido]-butyl ester |
| **Verbindung 206** | 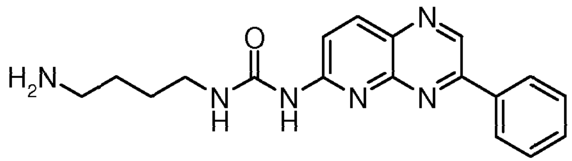 | 1-(4-Amino-butyl)-3-(3-phenyl-pyrido[2,3-b]pyrazin-6-yl)-harnstoff |
| **Verbindung 207** | 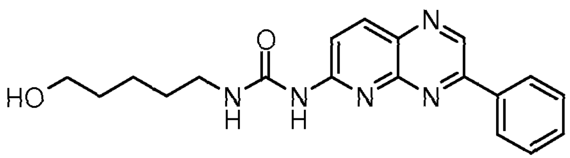 | 1-(5-Hydroxy-pentyl)-3-(3-phenyl-pyrido[2,3-b]pyrazin-6-yl)-harnstoff |
| **Verbindung 208** | 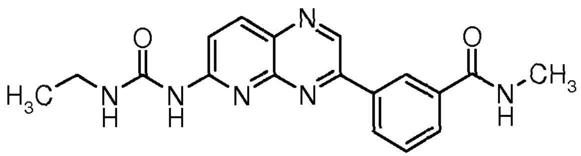 | 3-[6-(3-Ethyl-ureido)-pyrido[2,3-b]-pyrazin-3-yl]-N-methyl-benzamid |
| **Verbindung 209** | 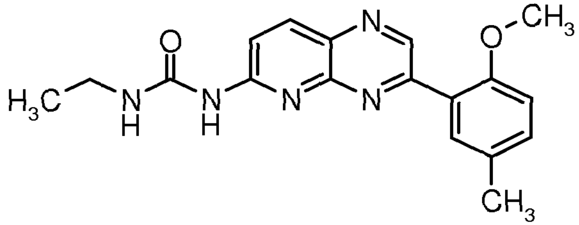 | 1-Ethyl-3-[3-(2-methoxy-5-methyl-phenyl)-pyrido[2,3-b]pyrazin-6-yl]-harnstoff |

(fortgesetzt)

| | | |
|---|---|---|
| **Verbindung 210** | 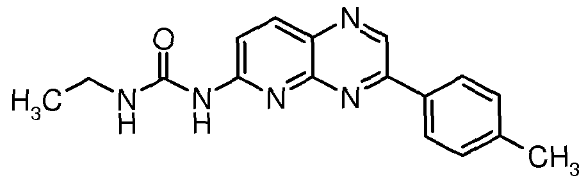 | 1-Ethyl-3-(3-p-tolyl-pyrido[2,3-b]pyrazin-6-yl)-harnstoff |
| **Verbindung 211** | 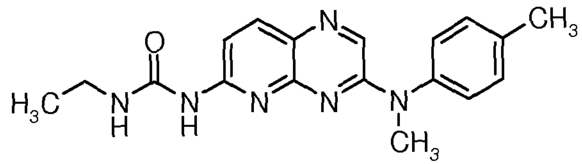 | 1-Ethyl-3-[3-(methyl-p-tolyl-amino)-pyrido[2,3-b]pyrazin-6-yl]-harnstoff |
| **Verbindung 212** | 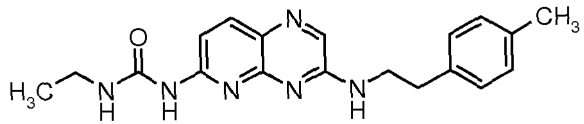 | 1-Ethyl-3-[3-(2-p-tolyl-ethylamino)-pyrido[2,3-b]pyrazin-6-yl]-harnstoff |
| **Verbindung 213** | 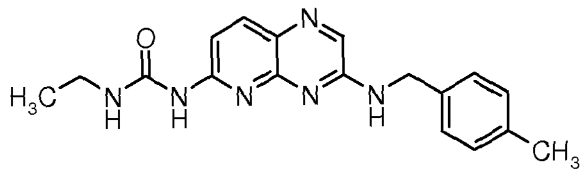 | 1-Ethyl-3-[3-(4-methyl-benzylamino)-pyrido[2,3-b]pyrazin-6-yl]-harnstoff |
| **Verbindung 214** | 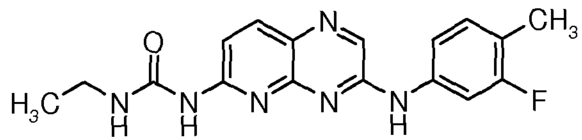 | 1-Ethyl-3-[3-(3-fluor-4-methyl-phenyl-amino)-pyrido[2,3-b]pyrazin-6-yl]-harnstoff |
| **Verbindung 215** | 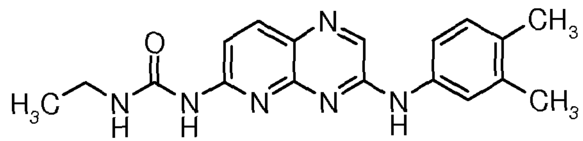 | 1-[3-(3,4-Dimethyl-phenylamino)-pyrido[2,3-b]pyrazin-6-yl]-3-ethyl-harnstoff |

(fortgesetzt)

| | | |
|---|---|---|
| **Verbindung 216** | 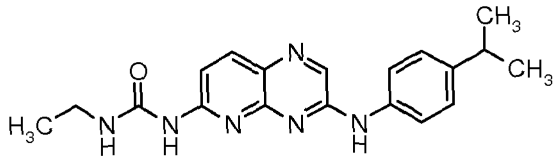 | 1-Ethyl-3-[3-(4-isopropyl-phenylamino)-pyrido[2,3-b]pyrazin-6-yl]-harnstoff |
| **Verbindung 217** | 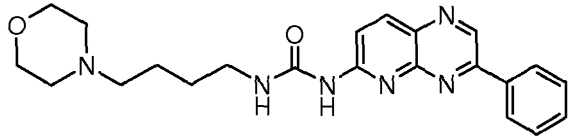 | 1-(4-Morpholin-4-yl-butyl)-3-(3-phenyl-pyrido[2,3-b]pyrazin-6-yl)-harnstoff |
| **Verbindung 218** | 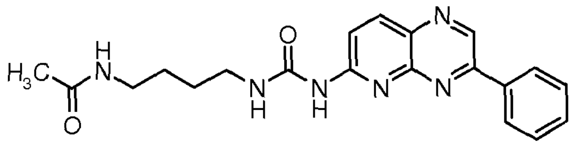 | N-{4-[3-(3-Phenyl-pyrido[2,3-b]pyrazin-6-yl)-ureido]-butyl}-acetamid |
| **Verbindung 219** | 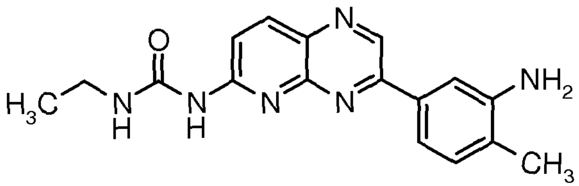 | 1-[3-(3-Amino-4-methylphenyl)-pyrido[2,3-b]pyrazin-6-yl]-3-ethyl-harnstoff |
| **Verbindung 220** | 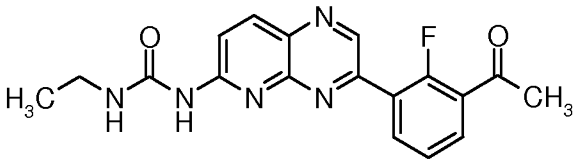 | 1-[3-(3-Acetyl-2-fluorphenyl)-pyrido[2,3-b]pyrazin-6-yl]-3-ethyl-harnstoff |
| **Verbindung 221** | 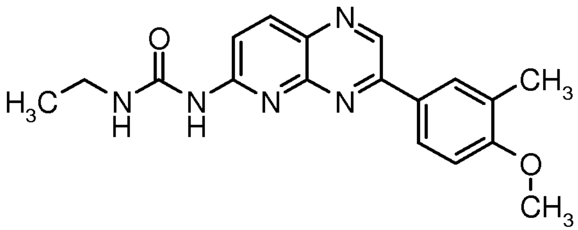 | 1-Ethyl-3-[3-(4-methoxy-3-methyl-phenyl)-pyrido[2,3-b]pyrazin-6-yl]-harnstoff |

(fortgesetzt)

| | | |
|---|---|---|
| **Verbindung 222** | 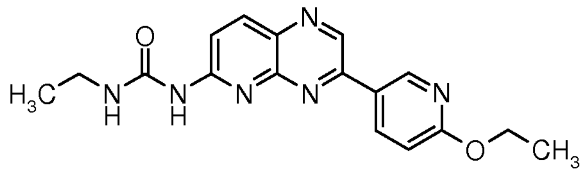 | 1-[3-(6-Ethoxy-pyridin-3-yl)-pyrido[2,3-b]pyrazin-6-yl]-3-ethyl-harnstoff |
| **Verbindung 223** | 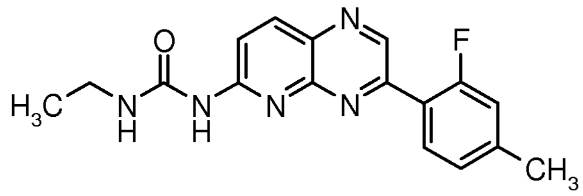 | 1-Ethyl-3-[3-(2-fluor-4-methyl-phenyl)-pyrido[2,3-b]pyrazin-6-yl]-harnstoff |
| **Verbindung 224** | 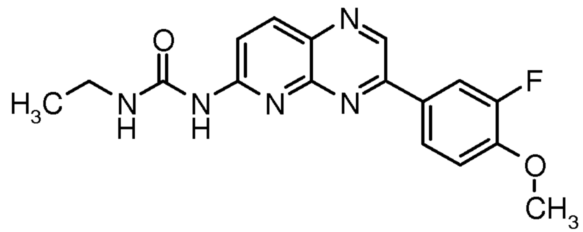 | 1-Ethyl-3-[3-(3-fluor-4-methoxy-phenyl)-pyrido[2,3-b]pyrazin-6-yl]-harnstoff |
| **Verbindung 225** | 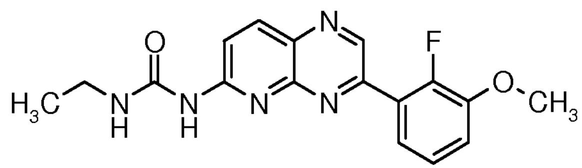 | 1-Ethyl-3-[3-(2-fluor-3-methoxy-phenyl)-pyrido[2,3-b]pyrazin-6-yl]-harnstoff |
| **Verbindung 226** | 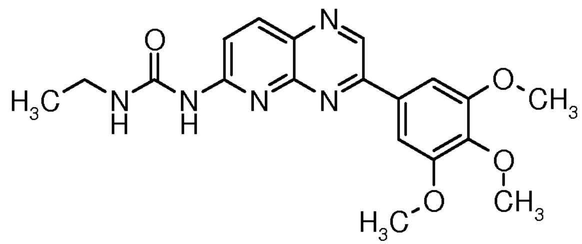 | 1-Ethyl-3-[3-(3,4,5-trimethoxy-phenyl)-pyrido[2,3-b]pyrazin-6-yl]-harnstoff |

(fortgesetzt)

| | | |
|---|---|---|
| **Verbindung 227** | 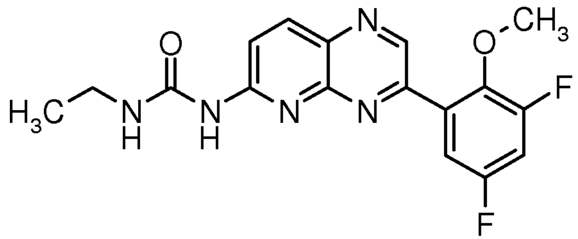 | 1-[3-(3,5-Difluor-2-methoxy-phenyl)-pyrido[2,3-b]pyrazin-6-yl]-3-ethyl-harnstoff |
| **Verbindung 228** | 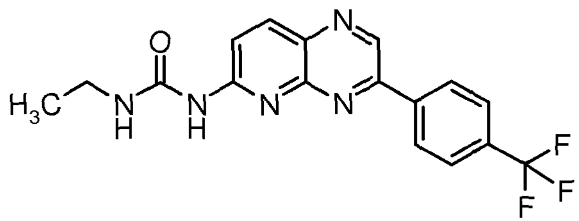 | 1-Ethyl-3-[3-(4-trifluormethyl-phenyl)-pyrido[2,3-b]pyrazin-6-yl]-harnstoff |
| **Verbindung 229** | 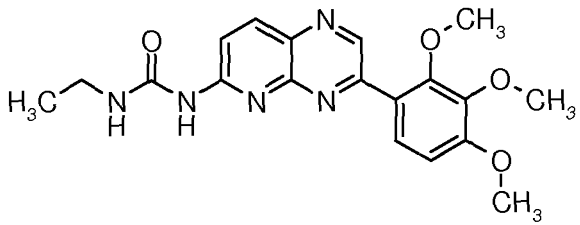 | 1-Ethyl-3-[3-(2,3,4-trimethoxy-phenyl)-pyrido[2,3-b]pyrazin-6-yl]-harnstoff |
| **Verbindung 230** | 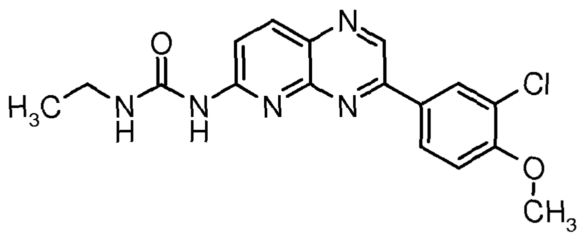 | 1-[3-(3-Chlor-4-methoxy-phenyl)-pyrido[2,3-b]pyrazin-6-yl]-3-ethyl-harnstoff |

(fortgesetzt)

| | | |
|---|---|---|
| **Verbindung 231** | 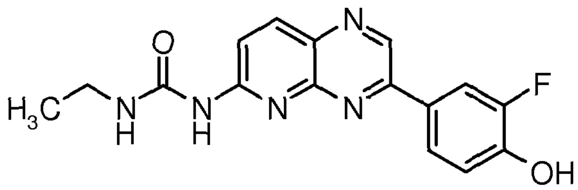 | 1-Ethyl-3-[3-(3-fluor-4-hydroxy-phenyl)-pyrido[2,3-b]pyrazin-6-yl]-harnstoff |
| **Verbindung 232** | 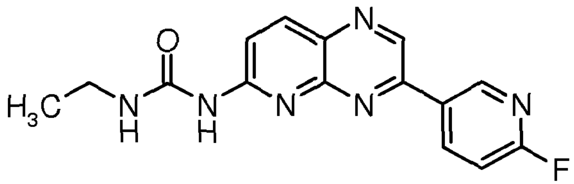 | 1-Ethyl-3-[3-(6-fluor-pyridin-3-yl)-pyrido[2,3-b]pyrazin-6-yl]-harnstoff |
| **Verbindung 233** | 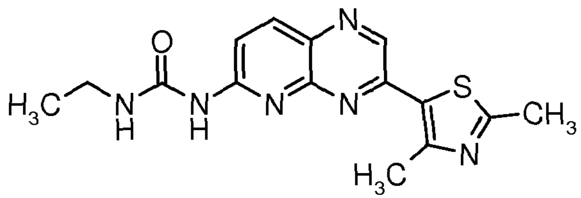 | 1-[3-(2,4-Dimethyl-thiazol-5-yl)-pyrido-[2,3-b]pyrazin-6-yl]-3-ethyl-harnstoff |
| **Verbindung 234** | 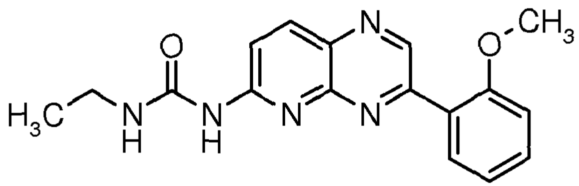 | 1-Ethyl-3-[3-(2-methoxy-phenyl)-pyrido[2,3-b]pyrazin-6-yl]-harnstoff |
| **Verbindung 235** | 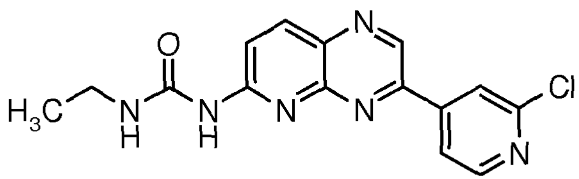 | 1-[3-(2-Chlor-pyridin-4-yl)-pyrido[2,3-b]pyrazin-6-yl]-3-ethyl-harnstoff |

(fortgesetzt)

| | | |
|---|---|---|
| **Verbindung 236** | 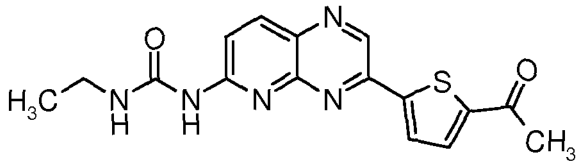 | 1-[3-(5-Acetyl-thiophen-2-yl)-pyrido[2,3-b]pyrazin-6-yl]-3-ethyl-harnstoff |
| **Verbindung 237** | 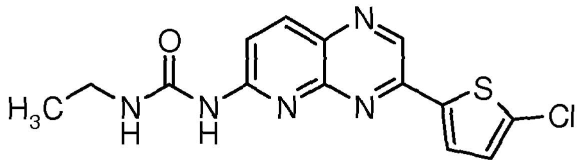 | 1-[3-(5-Chlor-thiophen-2-yl)-pyrido[2,3-b]pyrazin-6-yl]-3-ethyl-harnstoff |
| **Verbindung 238** | 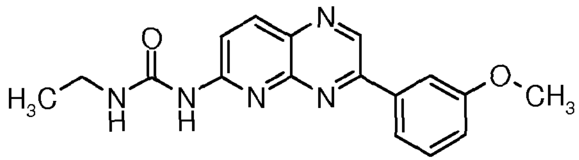 | 1-Ethyl-3-[3-(3-methoxyphenyl)-pyrido[2,3-b]pyrazin-6-yl]-harnstoff |
| **Verbindung 239** | 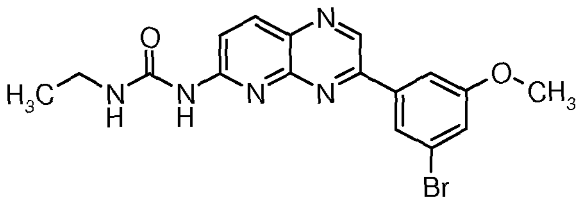 | 1-[3-(3-Brom-5-methoxy-phenyl)-pyrido[2,3-b]pyrazin-6-yl]-3-ethyl-harnstoff |
| **Verbindung 240** | 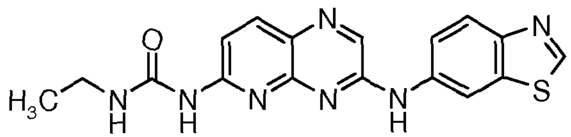 | 1-[3-(Benzothiazol-6-ylamino)-pyrido[2,3-b]pyrazin-6-yl]-3-ethyl-harnstoff |
| **Verbindung 241** | 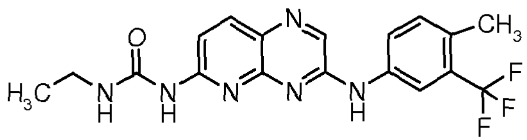 | 1-Ethyl-3-[3-(4-methyl-3-trifluoromethyl-phenylamino)-pyrido[2,3-b]pyrazin-6-yl]-harnstoff |

(fortgesetzt)

| | | |
|---|---|---|
| **Verbindung 242** | 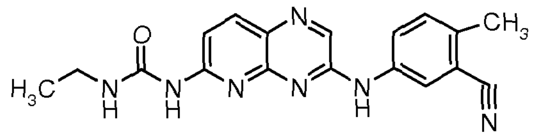 | 1-[3-(3-Cyano-4-methyl-phenylamino)-pyrido[2,3-b]pyrazin-6-yl]-3-ethyl-harnstoff |
| **Verbindung 243** | 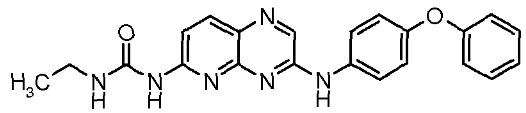 | 1-Ethyl-3-[3-(4-phenoxy-phenylamino)-pyrido[2,3-b]pyrazin-6-yl]-harnstoff |
| **Verbindung 244** | 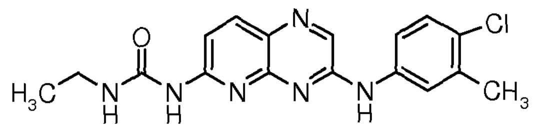 | 1-[3-(4-Chlor-3-methyl-phenylamino)-pyrido[2,3-b]pyrazin-6-yl]-3-ethyl-harnstoff |
| **Verbindung 245** | 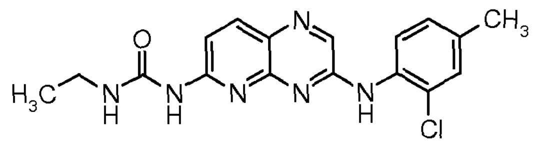 | 1-[3-(2-Chlor-4-methyl-phenylamino)-pyrido[2,3-b]pyrazin-6-yl]-3-ethyl-harnstoff |
| **Verbindung 246** | 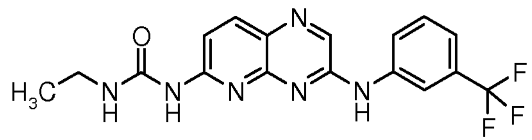 | 1-Ethyl-3-[3-(3-trifluoromethyl-phenylamino)-pyrido[2,3-b]pyrazin-6-yl]-harnstoff |
| **Verbindung 247** | 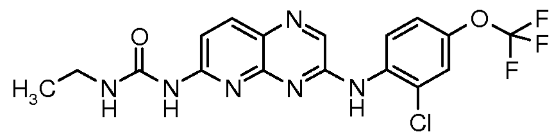 | 1-[3-(2-Chlor-4-trifluormethoxy-phenylamino)-pyrido[2,3-b]pyrazin-6-yl]-3-ethyl-harnstoff |
| **Verbindung 248** | 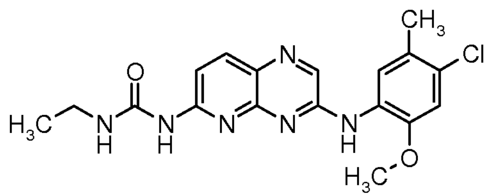 | 1-[3-(4-Chlor-2-methoxy-5-methyl-phenylamino)-pyrido[2,3-b]pyrazin-6-yl]-3-ethyl-harnstoff |

(fortgesetzt)

| | | |
|---|---|---|
| **Verbindung 249** | 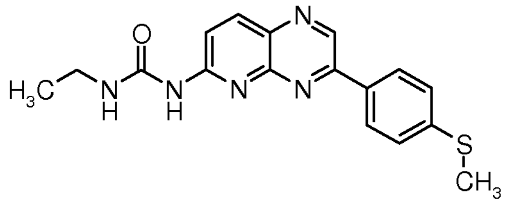 | 1-Ethyl-3-[3-(4-methylsulfanyl-phenyl)-pyrido[2,3-b]pyrazin-6-yl]-harnstoff |
| **Verbindung 250** | 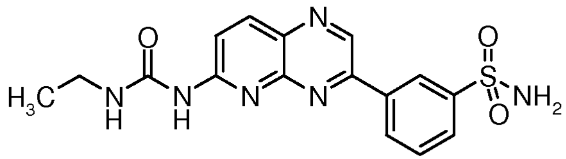 | 3-[6-(3-Ethyl-ureido)-pyrido[2,3-b]pyrazin-3-yl]-benzolsulfonamid |
| **Verbindung 251** | 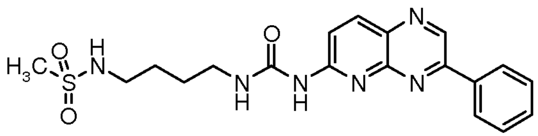 | N-{4-[3-(3-Phenyl-pyrido[2,3-b]pyrazin-6-yl)-ureido]-butyl}-methansulfonamid |
| **Verbindung 252** | 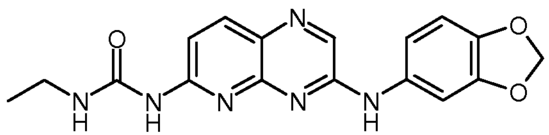 | 1-[3-(Benzo[1,3]dioxol-5-ylamino)-pyrido[2,3-b]pyrazin-6-yl]-3-ethyl-harnstoff |
| **Verbindung 253** | 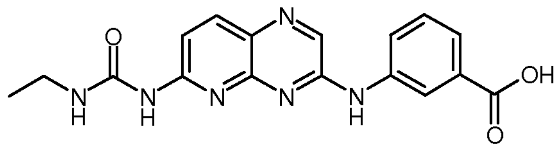 | 3-[6-(3-Ethyl-ureido)-pyrido[2,3-b]pyrazin-3-ylamino]-benzoesäure |
| **Verbindung 254** | 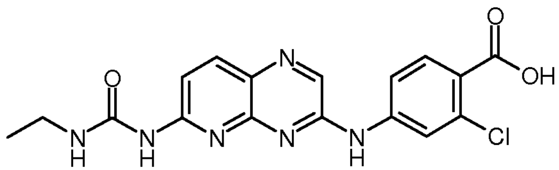 | 2-Chlor-4-[6-(3-ethyl-ureido)-pyrido[2,3-b]pyrazin-3-ylamino]-benzoesäure |

(fortgesetzt)

| | | |
|---|---|---|
| **Verbindung 255** | 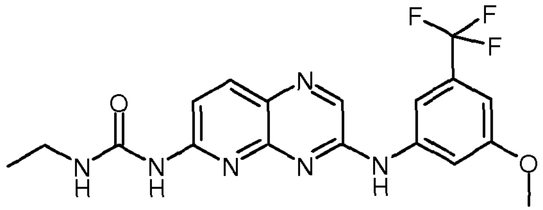 | 1-Ethyl-3-[3-(3-methoxy-5-trifluormethyl-phenylamino)-pyrido[2,3-b]pyrazin-6-yl]-harnstoff |
| **Verbindung 256** | 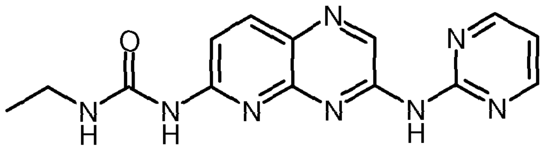 | 1-Ethyl-3-[3-(pyrimidin-2-ylamino)-pyrido[2,3-b]pyrazin-6-yl]-harnstoff |
| **Verbindung 257** | 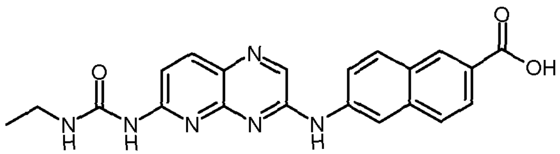 | 6-[6-(3-Ethyl-ureido)-pyrido[2,3-b]pyrazin-3-ylamino]-naphthalin-2-carbonsäure |
| **Verbindung 258** | 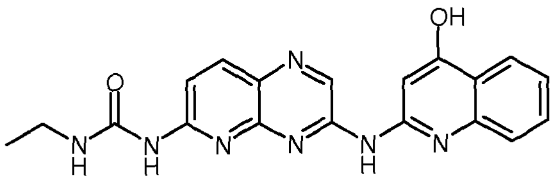 | 1-Ethyl-3-[3-(4-hydroxy-chinolin-2-ylamino)-pyrido[2,3-b]pyrazin-6-yl]-harnstoff |
| **Verbindung 259** | 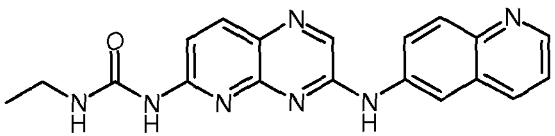 | 1-Ethyl-3-[3-(chinolin-6-ylamino)-pyrido[2,3-b]pyrazin-6-yl]-harnstoff |
| **Verbindung 260** | 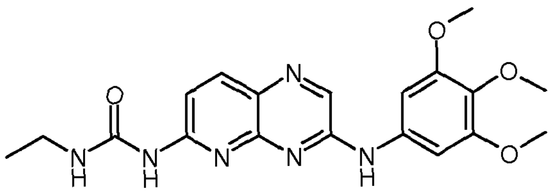 | 1-Ethyl-3-[3-(3,4,5-trimethoxy-phenylamino)-pyrido[2,3-b]pyrazin-6-yl]-harnstoff |

(fortgesetzt)

| | | |
|---|---|---|
| **Verbindung 261** | 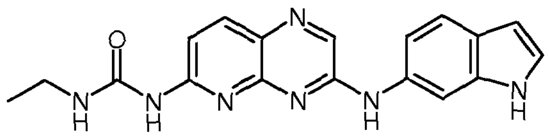 | 1-Ethyl-3-[3-(1H-indol-6-ylamino)-pyrido[2,3-b]pyrazin-6-yl]-harnstoff |
| **Verbindung 262** | 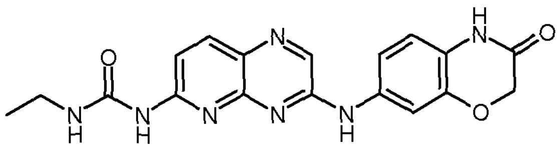 | 1-Ethyl-3-[3-(3-oxo-3,4-dihydro-2H-benzo[1,4]oxazin-7-ylamino)-pyrido[2,3-b]pyrazin-6-yl]-harnstoff |
| **Verbindung 263** | 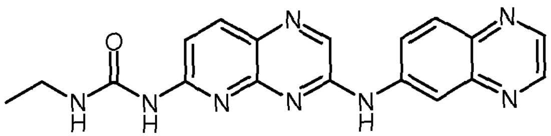 | 1-Ethyl-3-[3-(chinoxalin-6-ylamino)-pyrido[2,3-b]pyrazin-6-yl]-harnstoff |
| **Verbindung 264** | 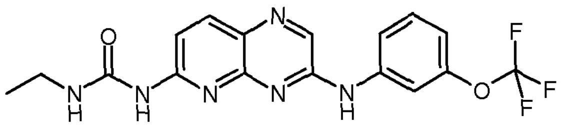 | 1-Ethyl-3-[3-(3-trifluormethoxy-phenylamino)-pyrido[2,3-b]pyrazin-6-yl]-harnstoff |
| **Verbindung 265** | 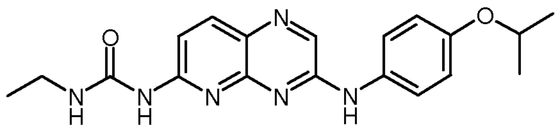 | 1-Ethyl-3-[3-(4-isopropoxy-phenylamino)-pyrido[2,3-b]pyrazin-6-yl]-harnstoff |
| **Verbindung 266** | 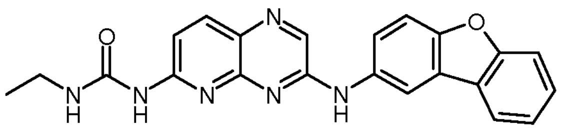 | 1-[3-(Dibenzofuran-2-ylamino)-pyrido[2,3-b]pyrazin-6-yl]-3-ethyl-harnstoff |
| **Verbindung 267** | 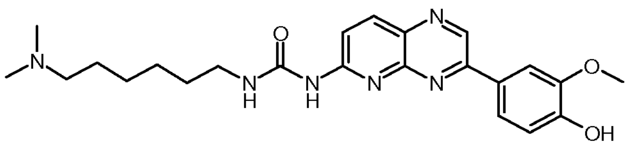 | 1-(6-Dimethylamino-hexyl)-3-[3-(4-hydroxy-3-methoxy-phenyl)-pyrido[2,3-b]pyrazin-6-yl]-harnstoff |

(fortgesetzt)

| | | |
|---|---|---|
| **Verbindung 268** | 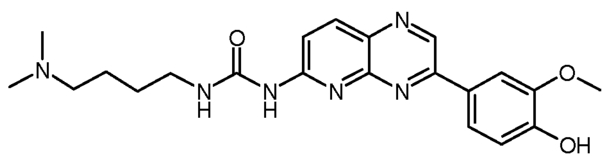 | 1-(4-Dimethylamino-butyl)-3-[3-(4-hydroxy-3-methoxy-phenyl)-pyrido[2,3-b]pyrazin-6-yl]-harnstoff |
| **Verbindung 269** | 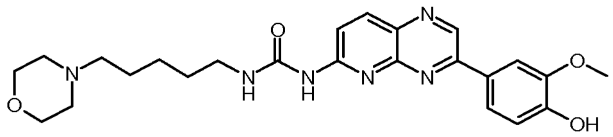 | 1-[3-(4-Hydroxy-3-methoxy-phenyl)-pyrido[2,3-b]pyrazin-6-yl]-3-(5-morpholin-4-yl-pentyl)-harnstoff |

**6.** Verwendung gemäß einem der Ansprüche 1 - 5, wobei das Arzneimittel mindestens eine weitere pharmakologisch aktive Substanz umfasst.

**7.** Verwendung gemäß einem der Ansprüche 1 - 5, wobei das Arzneimittel vor und/oder während und/oder nach der Behandlung mit mindestens einer weiteren pharmakologisch aktiven Substanz verabreicht wird.

**8.** Verwendung gemäß einem der Ansprüche 1 - 5, wobei das Arzneimittel vor und/oder während und/oder nach der Behandlung mit Strahlentherapie und/oder Chirurgie verabreicht wird.

**9.** Verwendung gemäß einem der Ansprüche 1 - 5, wobei die Verbindung gemäß der allgemeinen Formel (I) in einer Einheitsdosis von 0,001 mg bis 100 mg pro kg Körpergewicht eines Patienten vorliegt.

**10.** Pharmazeutische Zusammensetzung, enthaltend eine Verbindung ausgewählt aus der Gruppe der Verbindungen Nr. 38-269:

| | |
|---|---|
| **Verb. 38** | 1-[3-(4-Hydroxy-phenyl)-pyrido[2,3-b]pyrazin-6-yl]-3-methyl-harnstoff |
| **Verb. 39** | 1-Allyl-3-[3-(4-phenoxy-phenyl)-pyrido[2,3-b]pyrazin-6-yl]-harnstoff |
| **Verb. 40** | Methansulfonsäure-4-[6-(3-allyl-ureido)-pyrido[2,3-b]pyrazin-3-yl]-phenyl-ester |
| **Verb. 41** | 4-Dimethylamino-benzoesäure-4-[6-(3-allyl-ureido)-pyrido[2,3-b]pyrazin-3-yl]-phenyl-ester |
| **Verb. 42** | Essigsäure-4-[6-(3-allyl-ureido)-pyrido[2,3-b]pyrazin-3-yl]-phenyl-ester |
| **Verb. 43** | 1 -Ethyl-3-[3-(4-hydroxy-phenyl)-pyrido[2,3-b]pyrazin-6-yl]-harnstoff |
| **Verb. 44** | 1-[3-(4-Amino-phenyl)-pyrido[2,3-b]pyrazin-6-yl]-3-methyl-thioharnstoff |
| **Verb. 45** | 1-Ethyl-3-(3-phenyl-pyrido[2,3-b]pyrazin-6-yl)-harnstoff |
| **Verb. 46** | 1-Acetyl-1-ethyl-3-(3-phenyl-pyrido[2,3-b]pyrazin-6-yl)-harnstoff |
| **Verb. 47** | 1-Allyl-3-[3-(4-fluoro-phenyl)-pyrido[2,3-b]pyrazin-6-yl]-harnstoff |
| **Verb. 48** | 1,1-Diethyl-3-(3-phenyl-pyrido[2,3-b]pyrazin-6-yl)-harnstoff |
| **Verb. 49** | 1-(2-Chlor-ethyl)-3-[3-(4-fluor-phenyl)-pyrido[2,3-b]pyrazin-6-yl]-harnstoff |
| **Verb. 50** | 1-Ethyl-3-[3-(4-methoxy-phenyl)-pyrido[2,3-b]pyrazin-6-yl]-harnstoff |
| **Verb. 51** | 1-(3-Phenyl-pyrido[2,3-b]pyrazin-6-yl)-3-propyl-harnstoff |
| **Verb. 52** | [3-(3-Phenyl-pyrido[2,3-b]pyrazin-6-yl)-ureido]-essigsäure-ethyl-ester |
| **Verb. 53** | 1-(3-Chlor-pyrido[2,3-b]pyrazin-6-yl)-3-ethyl-harnstoff |
| **Verb. 54** | 1-Ethyl-3-[3-(4-fluor-phenyl)-pyrido[2,3-b]pyrazin-6-yl]-harnstoff |
| **Verb. 55** | 1-[3-(3-Chlor-4-fluor-phenyl)-pyrido[2,3-b]pyrazin-6-yl]-3-ethyl-harnstoff |
| **Verb. 56** | 4-[6-(3-Ethyl-ureido)-pyrido[2,3-b]pyrazin-3-yl]-benzoesäure |
| **Verb. 57** | N-{4-[6-(3-Ethyl-ureido)-pyrido[2,3-b]pyrazin-3-yl]-phenyl}-acetamide |
| **Verb. 58** | 1-[3-(2,4-Difluor-phenyl)-pyrido[2,3-b]pyrazin-6-yl]-3-ethyl-harnstoff |
| **Verb. 59** | 1-Ethyl-3-(3-morpholin-4-yl-pyrido[2,3-b]pyrazin-6-yl)-harnstoff |
| **Verb. 60** | 1-Ethyl-3-[3-(4-methyl-piperazin-1-yl)-pyrido[2,3-b]pyrazin-6-yl]-harnstoff |
| **Verb. 61** | 1-Ethyl-3-[3-(2-hydroxy-phenyl)-pyrido[2,3-b]pyrazin-6-yl]-harnstoff |
| **Verb. 62** | 1-Ethyl-3-[3-(2-methoxy-ethylamino)-pyrido[2,3-b]pyrazin-6-yl]-harnstoff |
| **Verb. 63** | 1-[3-(4-Chlor-3-trifluormethyl-phenyl)-pyrido[2,3-b]pyrazin-6-yl]-3-ethyl-harnstoff |
| **Verb. 64** | 1-Ethyl-3-(3-phenoxy-pyrido[2,3-b]pyrazin-6-yl)-harnstoff |
| **Verb. 65** | 1-[3-(Cyclopropylmethyl-amino)-pyrido[2,3-b]pyrazin-6-yl]-3-ethyl-harnstoff |
| **Verb. 66** | 1-Ethyl-3-{3-[(pyridin-4-ylmethyl)-amino]- |
| **Verb. 67** | 1-Ethyl-3-[3-(4-fluor-benzyloxy)-pyrido[2,3-b]pyrazin-6-yl]-harnstoff |
| **Verb. 68** | 1-Ethyl-3-(3-pyridin-4-yl-pyrido[2,3-b]pyrazin-6-yl)-harnstoff |
| **Verb. 69** | 1-Ethyl-3-[3-(pyridin-3-yloxy)-pyrido[2,3-b]pyrazin-6-yl]-harnstoff |
| **Verb. 70** | 1 -Ethyl-3-[3-(tetrahydro-furan-2-ylmethoxy)-pyrido[2,3-b]pyrazin-6-yl]-harnstoff |
| **Verb. 71** | 1 -Ethyl-3-[3-(4-morpholin-4-yl-phenyl)-pyrido[2,3-b]pyrazin-6-yl]-harnstoff |
| **Verb. 72** | 1-Ethyl-3-(3-hydroxy-pyrido[2,3-b]pyrazin-6-yl)-harnstoff |
| **Verb. 73** | 1-Ethyl-3-[3-(3-methoxy-phenylsulfanyl)-pyrido[2,3-b]pyrazin-6-yl]-harnstoff |
| **Verb. 74** | 1-Ethyl-3-(3-quinolin-3-yl-pyrido[2,3-b]pyrazin-6-yl)-harnstoff |
| **Verb. 75** | 1-(3-Benzo[b]thiophen-3-yl-pyrido[2,3-b]pyrazin-6-yl)-3-ethyl-harnstoff |

(fortgesetzt)

| | |
|---|---|
| **Verb. 76** | 1-Ethyl-3-[3-(pyridin-2-ylsulfanyl)-pyrido[2,3-b]pyrazin-6-yl]-harnstoff |
| **Verb. 77** | 1-[3-(4-Dimethylamino-phenyl)-pyrido[2,3-b]pyrazin-6-yl]-3-ethyl-harnstoff |
| **Verb. 78** | N-{4-[6-(3-Ethyl-ureido)-pyrido[2,3-b]pyrazin-3-yl]-phenyl}-methansulfonamid |
| **Verb. 79** | 1-Ethyl-3-[3-(1H-pyrazol-4-yl)-pyrido[2,3-b]pyrazin-6-yl]-harnstoff |
| **Verb. 80** | 1-(3-Benzylsulfanyl-pyrido[2,3-b]pyrazin-6-yl)-3-ethyl-harnstoff |
| **Verb. 81** | 1-Ethyl-3-[3-(4-methyl-[1,4]diazepan-1-yl)-pyrido[2,3-b]pyrazin-6-yl]-harnstoff |
| **Verb. 82** | 1-[3-(4-Amino-phenyl)-pyrido[2,3-b]pyrazin-6-yl]-3-ethyl-harnstoff |
| **Verb. 83** | 1-(3-Amino-pyrido[2,3-b]pyrazin-6-yl)-3-ethyl-harnstoff |
| **Verb. 84** | 1-Ethyl-3-pyrido[2,3-b]pyrazin-6-yl-harnstoff |
| **Verb. 85** | 1-Ethyl-3-(3-imidazol-1-yl-pyrido[2,3-b]pyrazin-6-yl)-harnstoff |
| **Verb. 86** | 1-Ethyl-3-[3-(4-fluor-2-hydroxy-phenyl)-pyrido[2,3-b]pyrazin-6-yl]-harnstoff |
| **Verb. 87** | 1-(3-Cyclopentyloxy-pyrido[2,3-b]pyrazin-6-yl)-3-ethyl-harnstoff |
| **Verb. 88** | 1-Ethyl-3-[3-(4-hydroxy-piperidin-1-yl)-pyrido[2,3-b]pyrazin-6-yl]-harnstoff |
| **Verb. 89** | (3-Phenyl-pyrido[2,3-b]pyrazin-6-yl)-harnstoff |
| **Verb. 90** | 1-Ethyl-3-(3-pyrimidin-5-yl-pyrido[2,3-b]pyrazin-6-yl)-harnstoff |
| **Verb. 91** | 1-Ethyl-3-(3-pyridin-3-yl-pyrido[2,3-b]pyrazin-6-yl)-harnstoff |
| **Verb. 92** | 1-Allyl-3-(3-phenyl-pyrido[2,3-b]pyrazin-6-yl)-harnstoff |
| **Verb. 93** | 1-Ethyl-3-(3-piperazin-1-yl-pyrido[2,3-b]pyrazin-6-yl)-harnstoff |
| **Verb. 94** | 1-[3-(3-Chlor-pyridin-4-ylmethyl)-pyrido[2,3-b]pyrazin-6-yl]-3-ethyl-harnstoff |
| **Verb. 95** | 1-Ethyl-3-[3-(6-methoxy-pyridin-3-yl)-pyrido[2,3-b]pyrazin-6-yl]-harnstoff |
| **Verb. 96** | 1-[3-(3,5-Dimethyl-isoxazol-4-yl)-pyrido[2,3-b]pyrazin-6-yl]-3-ethyl-harnstoff |
| **Verb. 97** | 1-Ethyl-3-[3-(4-trifluormethoxy-phenyl)-pyrido[2,3-b]pyrazin-6-yl]-harnstoff |
| **Verb. 98** | 1-Ethyl-3-(3-furan-2-yl-pyrido[2,3-b]pyrazin-6-yl)-harnstoff |
| **Verb. 99** | 1-Ethyl-3-[3-(2-methoxy-pyridin-3-yl)-pyrido[2,3-b]pyrazin-6-yl]-harnstoff |
| **Verb. 100** | 1-[3-(2,4-Dimethoxy-phenyl)-pyrido[2,3-b]pyrazin-6-yl]-3-ethyl-harnstoff |
| **Verb. 101** | 1-Ethyl-3-[3-(1 H-pyrrol-2-yl)-pyrido[2,3-b]pyrazin-6-yl]-harnstoff |
| **Verb. 102** | 1-Ethyl-3-[3-(6-morpholin-4-yl-pyridin-3-yl)-pyrido[2,3-b]pyrazin-6-yl]-harnstoff |
| **Verb. 103** | 1-Benzyl-3-ethyl-1-(3-phenyl-pyrido[2,3-b]pyrazin-6-yl)-harnstoff |
| **Verb. 104** | 1-[3-(2-Amino-phenyl)-pyrido[2,3-b]pyrazin-6-yl]-3-ethyl-harnstoff |
| **Verb. 105** | 1-Ethyl-3-[3-(4-hydroxymethyl-phenyl)-pyrido[2,3-b]pyrazin-6-yl]-harnstoff |
| **Verb. 106** | 1-[3-(3-Amino-phenyl)-pyrido[2,3-b]pyrazin-6-yl]-3-ethyl-harnstoff |
| **Verb. 107** | 1-[3-(4-Acetyl-phenyl)-pyrido[2,3-b]pyrazin-6-yl]-3-ethyl-harnstoff |
| **Verb. 108** | 1-[3-(2,3-Dihydro-benzofuran-5-yl)-pyrido[2,3-b]pyrazin-6-yl]-3-ethyl-harnstoff |
| **Verb. 109** | 1-[3-(4-Benzyloxy-3-fluor-phenyl)-pyrido[2,3-b]pyrazin-6-yl]-3-ethyl-harnstoff |
| **Verb. 110** | 1-(2,3-Dihydroxy-propyl)-3-(3-phenyl-pyrido[2,3-b]pyrazin-6-yl)-harnstoff |
| **Verb. 111** | 1-Ethyl-3-[3-(3-formyl-4-methoxy-phenyl)-pyrido[2,3-b]pyrazin-6-yl]-harnstoff |
| **Verb. 112** | 1-Ethyl-3-[3-(4-methansulfonyl-phenyl)-pyrido[2,3-b]pyrazin-6-yl]-harnstoff |
| **Verb. 113** | N-{4-[6-(3-Ethyl-ureido)-pyrido[2,3-b]pyrazin-3-yl]-phenyl}-succinamidsäure |
| **Verb. 114** | 1-Ethyl-3-(3-pyridin-4-yl-pyrido[2,3-b]pyrazin-6-yl)-harnstoff Methansulfonsäuresalz (freie Base) |
| **Verb. 115** | 1-[3-(2,6-Dimethoxy-pyridin-3-yl)-pyrido[2,3-b]pyrazin-6-yl]-3-ethyl-harnstoff |
| **Verb. 116** | 1-[3-(2,6-Dimethoxy-pyrimidin-4-yl)-pyrido[2,3-b]pyrazin-6-yl]-3-ethyl-harnstoff |
| **Verb. 117** | 1-[3-(2,4-Dioxo-1,2,3,4-tetrahydro-pyrimidin-5-yl)-pyrido[2,3-b]pyrazin-6-yl]-3-ethyl-harnstoff |
| **Verb. 118** | 1-Ethyl-3-[3-(1H-indol-5-yl)-pyrido[2,3-b]pyrazin-6-yl]-harnstoff |
| **Verb. 119** | 1-(3-Chloro-pyrido[2,3-b]pyrazin-6-yl)-3-ethyl-thioharnstoff |
| **Verb. 120** | 1-Ethyl-3-{3-[4-(2-methoxy-ethoxy)-phenyl]-pyrido[2,3-b]pyrazin-6-yl}-harnstoff |
| **Verb. 121** | Acrylsäure-4-[6-(3-ethyl-ureido)-pyrido[2,3-b]-pyrazin-3-yl]-phenyl-ester |
| **Verb. 122** | 1-[3-(4-Cyano-phenyl)-pyrido[2,3-b]pyrazin-6-yl]-3-ethyl-harnstoff |
| **Verb. 123** | 1-(3-Benzo[1,2,5]oxadiazol-4-yl-pyrido[2,3-b]pyrazin-6-yl)-3-ethyl-harnstoff |
| **Verb. 124** | 1-Ethyl-3-[3-(4-hydroxy-3-methoxy-phenyl)-pyrido[2,3-b]pyrazin-6-yl]-harnstoff |
| **Verb. 125** | 1-[3-(2,6-Dimethoxy-phenyl)-pyrido [2,3-b]pyrazin-6-yl]-3-ethyl-harnstoff |
| **Verb. 126** | 1-[3-(3-Acetyl-phenyl)-pyrido[2,3-b]pyrazin-6-yl]-3-ethyl-harnstoff |

(fortgesetzt)

| | |
|---|---|
| **Verb. 127** | 1-Ethyl-3-[3-(3-morpholin-4-yl-phenyl)-pyrido[2,3-b]pyrazin-6-yl]-harnstoff |
| **Verb. 128** | 1-[3-(6-Amino-pyridin-3-yl)-pyrido[2,3-b]pyrazin-6-yl]-3-ethyl-harnstoff |
| **Verb. 129** | 3-{4-[6-(3-Ethyl-ureido)-pyrido[2,3-b]-pyrazin-3-yl]-phenoxy}-propionsäure |
| **Verb. 130** | 1-Isopropyl-3-(3-phenyl-pyrido[2,3-b]pyrazin-6-yl)-harnstoff |
| **Verb. 131** | 1-Cyclopentyl-3-(3-phenyl-pyrido[2,3-b]pyrazin-6-yl)-harnstoff |
| **Verb. 132** | 1-Pentyl-3-(3-phenyl-pyrido[2,3-b]pyrazin-6-yl)-harnstoff |
| **Verb. 133** | N-{4-[6-(3-Ethyl-ureido)-pyrido[2,3-b]pyrazin-3-yl]-phenyl}-acrylamid |
| **Verb. 134** | 1-tert-Butyl-3-(3-phenyl-pyrido[2,3-b]pyrazin-6-yl)-harnstoff |
| **Verb. 135** | 1-(2-Hydroxy-ethyl)-3-(3-phenyl-pyrido[2,3-b]pyrazin-6-yl)-harnstoff |
| **Verb. 136** | 1-Cyclobutyl-3-(3-phenyl-pyrido[2,3-b]pyrazin-6-yl)-harnstoff |
| **Verb. 137** | 1-Allyl-3-[3-(4-nitro-phenyl)-pyrido[2,3-b]pyrazin-7-yl]-thioharnstoff |
| **Verb. 138** | 1-Ethyl-1-(Ethylcarbamoyl)-3-(3-phenyl-pyrido[2,3-b]pyrazin-6-yl)-harnstoff |
| **Verb. 139** | [3-(4-Hydroxy-phenyl)-pyrido[2,3-b]pyrazin-6-yl]-carbaminsäure-allyl-ester |
| **Verb. 140** | (3-Phenyl-pyrido[2,3-b]pyrazin-6-yl)-carbaminsäure-ethyl-ester |
| **Verb. 141** | 1-Ethyl-3-[3-(4-phenyl-piperazin-1-yl)-pyrido[2,3-b]pyrazin-6-yl]-harnstoff |
| **Verb. 142** | 1-(2,2-Dimethyl-[1,3]dioxolan-4-ylmethyl)-3-(3-phenyl-pyrido[2,3-b]pyrazin-6-yl)-harnstoff |
| **Verb. 143** | 1,3-Bis-(3-phenyl-pyrido[2,3-b]pyrazin-6-yl)-harnstoff |
| **Verb. 144** | N-(3-Phenyl-pyrido[2,3-b]pyrazin-6-yl)-acetamidin |
| **Verb. 145** | 1-Ethyl-3-[3-(2-methoxy-pyridin-3-yl)-pyrido[2,3-b]pyrazin-6-yl]-thioharnstoff |
| **Verb. 146** | 1-(4-Hydroxy-butyl)-3-(3-phenyl-pyrido[2,3-b]pyrazin-6-yl)-harnstoff |
| **Verb. 147** | 1-(3-Hydroxy-propyl)-3-(3-phenyl-py rido[2,3-b]pyrazin-6-yl)-harnstoff |
| **Verb. 148** | 1-Ethyl-3-{3-[4-(3-morpholin-4-yl- propoxy)-phenyl]-pyrido[2,3-b]pyrazin-6-yl}-harnstoff |
| **Verb. 149** | 1-Ethyl-3-[3-(4-hydroxy-phenyl)-pyrido[2,3-b]pyrazin-6-yl]-thioharnstoff |
| **Verb. 150** | 1-Ethyl-3-(3-p-tolylamino-pyrido[2,3-b]pyrazin-6-yl)-harnstoff |
| **Verb. 151** | 1-Ethyl-3-[3-(3-hydroxy-phenyl)-pyrido[2,3-b]pyrazin-6-yl]-harnstoff |
| **Verb. 152** | 3-[6-(3-Ethyl-ureido)-pyrido[2,3-b]pyrazin-3-yl]-benzoesäure |
| **Verb. 153** | 1-[3-(3,4-Dimethoxy-phenyl)-pyrido[2,3-b]pyrazin-6-yl]-3-ethyl-harnstoff |
| **Verb. 154** | 1-[3-(4-Amino-3-methoxy-phenyl)-pyrido[2,3-b]pyrazin-6-yl]-3-ethyl-harnstoff |
| **Verb. 155** | 3-[6-(3-Ethyl-ureido)-pyrido[2,3-b]-pyrazin-3-yl]-benzamid |
| **Verb. 156** | 1-[3-(3-Chlor-4-hydroxy-5-methoxy-phenyl)-pyrido[2,3-b]pyrazin-6-yl]-3-ethyl-harnstoff |
| **Verb. 157** | 1-Ethyl-3-(3-m-tolylamino-pyrido-[2,3-b]pyrazin-6-yl)-harnstoff |
| **Verb. 158** | 1-Ethyl-3-[3-(4-methoxy-phenylamino)-pyrido[2,3-b]pyrazin-6-yl]-harnstoff |
| **Verb. 159** | 1-[3-(4-Chloro-phenylamino)-pyrido-[2,3-b]pyrazin-6-yl]-3-ethyl-harnstoff |
| **Verb. 160** | 1-Ethyl-3-(3-o-tolylamino-pyrido[2,3-b]-pyrazin-6-yl)-harnstoff |
| **Verb. 161** | 1-Ethyl-3-[3-(pyridin-3-ylamino)-pyrido-[2,3-b]pyrazin-6-yl]-harnstoff |
| **Verb. 162** | 1-Ethyl-3-[3-(4-ethyl-phenylamino)-pyrido[2,3-b]pyrazin-6-yl]-harnstoff |
| **Verb. 163** | 1-Ethyl-3-[3-(3-methoxy-4-methyl-phenylamino)-pyrido[2,3-b]pyrazin-6-yl]-harnstoff |
| **Verb. 164** | 1-Ethyl-3-[3-(4-hydroxy-phenylamino)-pyrido[2,3-b]pyrazin-6-yl]-harnstoff |
| **Verb. 165** | 1-Ethyl-3-[3-(5-methyl-pyridin-2-yl-amino)-pyrido[2,3-b]pyrazin-6-yl]-harnstoff |
| **Verb. 166** | 1-Ethyl-3-[3-(1-methyl-1 H-pyrazol-3-ylamino)-pyrido[2,3-b]pyrazin-6-yl]-harnstoff |
| **Verb. 167** | 1-Ethyl-3-[3-(4-fluor-phenylamino)-pyrido[2,3-b]pyrazin-6-yl]-harnstoff |
| **Verb. 168** | 1-(4-Hydroxy-butyl)-3-[3-(4-hydroxy-3-methoxy-phenyl)-pyrido[2,3-b]pyrazin-6-yl]-harnstoff |
| **Verb. 169** | Phosphorsäure-mono-{4-[6-(3-ethyl-ureido)-pyrido-[2,3-b]pyrazin-3-yl]-2-methoxy-phenyl}-ester |
| **Verb. 170** | 1-[3-(2-Chlor-4-hydroxy-phenyl)-pyrido[2,3-b]pyrazin-6-yl]-3-ethyl-harnstoff |
| **Verb. 171** | 1-[3-(4-Chlor-2-methoxy-phenyl)-pyrido[2,3-b]pyrazin-6-yl]-3-ethyl-harnstoff |
| **Verb. 172** | 1-[3-(3,5-Dimethyl-1H-pyrazol-4-yl)-pyrido[2,3-b]pyrazin-6-yl]-3-ethyl-harnstoff |
| **Verb. 173** | 1-Ethyl-3-[3-(1-methyl-1H-pyrazol-4-yl)-pyrido[2,3-b]pyrazin-6-yl]-harnstoff |
| **Verb. 174** | 1-[3-(5-Cyano-thiophen-2-yl)-pyrido[2,3-b]pyrazin-6-yl]-3-ethyl-harnstoff |
| **Verb. 175** | Natrium 2-chlor-4-[6-(3-ethyl-ureido)-pyrido[2,3-b]pyrazin-3-yl]-6-methoxy-phenolat |
| **Verb. 176** | 1-[3-(4-Hydroxy-3-methoxy-phenyl)-pyrido[2,3-b]pyrazin-6-yl]-3-(4-morpholin-4-yl-butyl)-harnstoff |
| **Verb. 177** | 1-[3-(3-Chlor-4-methoxy-phenylamino)-pyrido[2,3-b]pyrazin-6-yl]-3-ethyl-harnstoff |

(fortgesetzt)

| | |
|---|---|
| **Verb. 178** | 1-Ethyl-3-[3-(naphthalin-2-ylamino)-pyrido[2,3-b]pyrazin-6-yl]-harnstoff |
| **Verb. 179** | 1-Ethyl-3-[3-(chinolin-3-ylamino)-pyrido[2,3-b]pyrazin-6-yl]-harnstoff |
| **Verb. 180** | 1-[3-(3,5-Dimethoxy-phenylamino)-pyrido[2,3-b]pyrazin-6-yl]-3-ethyl-harnstoff |
| **Verb. 181** | 1-Ethyl-3-[3-(pyrazin-2-ylamino)-pyrido[2,3-b]pyrazin-6-yl]-harnstoff |
| **Verb. 182** | 1-Ethyl-3-[3-(3-isopropoxy-phenylamino)-pyrido[2,3-b]pyrazin-6-yl]-harnstoff |
| **Verb. 183** | 1-Ethyl-3-[3-(4-hydroxy-3-methoxy-phenyl)-pyrido[2,3-b]pyrazin-6-yl]-harnstoff p-Toluolsulfonat |
| **Verb. 184** | 1-[3-(2-Chlor-pyridin-4-ylamino)-pyrido[2,3-b]pyrazin-6-yl]-3-ethyl-harnstoff |
| **Verb. 185** | 1-[3-(3,5-Dichlor-4-hydroxy-phenylamino)-pyrido[2,3-b]pyrazin-6-yl]-3-ethyl-harnstoff |
| **Verb. 186** | 1-[3-(3,5-Dichlor-4-hydroxy-phenyl)-pyrido[2,3-b]pyrazin-6-yl]-3-ethyl-harnstoff |
| **Verb. 187** | 1-[3-(3,4-Dimethoxy-phenylamino)-pyrido[2,3-b]pyrazin-6-yl]-3-ethyl-harnstoff |
| **Verb. 188** | 1-Ethyl-3-[3-(3-hydroxy-4-methyl-phenylamino)-pyrido[2,3-b]pyrazin-6-yl]-harnstoff |
| **Verb. 189** | 3-[6-(3-Ethyl-ureido)-pyrido[2,3-b]-pyrazin-3-ylamino]-5-trifluormethyl-benzoesäure |
| **Verb. 190** | 1-Ethyl-3-[3-(6-methoxy-pyridin-3-ylamino)-pyrido[2,3-b]pyrazin-6-yl]-harnstoff |
| **Verb. 191** | 1-[3-(3,5-Dimethyl-phenylamino)-pyrido[2,3-b]pyrazin-6-yl]-3-ethyl-harnstoff |
| **Verb. 192** | 1-[3-(4-Cyano-phenylamino)-pyrido-[2,3-b]pyrazin-6-yl]-3-ethyl-harnstoff |
| **Verb. 193** | 1-Ethyl-3-[3-(4-hydroxy-3-methoxy-phenyl)-pyrido[2,3-b]pyrazin-6-yl]-harnstoff (Z)-but-2-endicarbonsäure-Salz |
| **Verb. 194** | 1-Ethyl-3-[3-(4-hydroxy-3-methoxy-phenyl)-pyrido[2,3-b]pyrazin-6-yl]-harnstoff Hydrochlorid |
| **Verb. 195** | 1-Ethyl-3-(3-phenyl-pyrido[2,3-b]pyrazin-6-yl)-thioharnstoff |
| **Verb. 196** | 1-(3-Chlor-pyrido[2,3-b]pyrazin-6-yl)-3-cyclohexyl-harnstoff |
| **Verb. 197** | 1-Ethyl-3-[3-(4-hydroxy-3-methoxy-phenylamino)-pyrido[2,3-b]pyrazin-6-yl]-harnstoff |
| **Verb. 198** | 1-Ethyl-3-[3-(4-hydroxy-3,5-dimethyl-phenyl)-pyrido[2,3-b]pyrazin-6-yl]-harnstoff |
| **Verb. 199** | 3-Ethyl-1-phenethyl-1-(3-phenyl- pyrido[2,3-b]pyrazin-6-yl)-harnstoff |
| **Verb. 200** | 1-Allyl-3-{3-[4-(tetrahydro-pyran-2-yloxy)-phenyl]-pyrido[2,3-b]pyrazin-6-yl}-thioharnstoff |
| **Verb. 201** | 3-Ethyl-1-[3-(4-methoxy-phenyl)- pyrido[2,3-b]pyrazin-6-yl]-1-propyl- harnstoff |
| **Verb. 202** | 3-Ethyl-1-(3-phenyl-pyrido[2,3-b]pyrazin-6-yl)-1-(2-piperidin-1-yl-ethyl)-harnstoff Hydrochlorid |
| **Verb. 203** | N-{4-[6-(3-Ethyl-ureido)-pyrido[2,3-b]-pyrazin-3-yl]-phenyl}-2-phenyl- acetamid |
| **Verb. 204** | 1-(4-Hydroxy-butyl)-3-(3-phenyl-pyrido[2,3-b]pyrazin-6-yl)-harnstoff Hydrochlorid |
| **Verb. 205** | Essigsäure-4-[3-(3-phenyl-pyrido[2,3-b]pyrazin-6-yl)-ureido]-butyl ester |
| **Verb. 206** | 1-(4-Amino-butyl)-3-(3-phenyl-pyrido[2,3-b]pyrazin-6-yl)-harnstoff |
| **Verb. 207** | 1-(5-Hydroxy-pentyl)-3-(3-phenyl-pyrido[2,3-b]pyrazin-6-yl)-harnstoff |
| **Verb. 208** | 3-[6-(3-Ethyl-ureido)-pyrido[2,3-b]-pyrazin-3-yl]-N-methyl-benzamid |
| **Verb. 209** | 1 -Ethyl-3-[3-(2-methoxy-5-methyl-phenyl)-pyrido[2,3-b]pyrazin-6-yl]-harnstoff |
| **Verb. 210** | 1-Ethyl-3-(3-p-tolyl-pyrido[2,3-b]pyrazin-6-yl)-harnstoff |
| **Verb. 211** | 1-Ethyl-3-[3-(methyl-p-tolyl-amino)-pyrido[2,3-b]pyrazin-6-yl]-harnstoff |
| **Verb. 212** | 1-Ethyl-3-[3-(2-p-tolyl-ethylamino)-pyrido[2,3-b]pyrazin-6-yl]-harnstoff |
| **Verb. 213** | 1-Ethyl-3-[3-(4-methyl-benzylamino)-pyrido[2,3-b]pyrazin-6-yl]-harnstoff |
| **Verb. 214** | 1-Ethyl-3-[3-(3-fluor-4-methyl-phenyl-amino)-pyrido[2,3-b]pyrazin-6-yl]-harnstoff |
| **Verb. 215** | 1-[3-(3,4-Dimethyl-phenylamino)-pyrido[2,3-b]pyrazin-6-yl]-3-ethyl-harnstoff |
| **Verb. 216** | 1 -Ethyl-3-[3-(4-isopropyl-phenylamino)-pyrido[2,3-b]pyrazin-6-yl]-harnstoff |
| **Verb. 217** | 1-(4-Morpholin-4-yl-butyl)-3-(3-phenyl-pyrido[2,3-b]pyrazin-6-yl)-harnstoff |
| **Verb. 218** | N-{4-[3-(3-Phenyl-pyrido[2,3-b]pyrazin-6-yl)-ureido]-butyl}-acetamid |
| **Verb. 219** | 1-[3-(3-Amino-4-methyl-phenyl)-pyrido[2,3-b]pyrazin-6-yl]-3-ethyl-harnstoff |
| **Verb. 220** | 1-[3-(3-Acetyl-2-fluor-phenyl)-pyrido[2,3-b]pyrazin-6-yl]-3-ethyl-harnstoff |
| **Verb. 221** | 1-Ethyl-3-[3-(4-methoxy-3-methyl-phenyl)-pyrido[2,3-b]pyrazin-6-yl]-harnstoff |
| **Verb. 222** | 1-[3-(6-Ethoxy-pyridin-3-yl)-pyrido[2,3-b]pyrazin-6-yl]-3-ethyl-harnstoff |
| **Verb. 223** | 1-Ethyl-3-[3-(2-fluor-4-methyl-phenyl)-pyrido[2,3-b]pyrazin-6-yl]-harnstoff |
| **Verb. 224** | 1 -Ethyl-3-[3-(3-fluor-4-methoxy-phenyl)-pyrido[2,3-b]pyrazin-6-yl]-harnstoff |
| **Verb. 225** | 1 -Ethyl-3-[3-(2-fluor-3-methoxy-phenyl)-pyrido[2,3-b]pyrazin-6-yl]-harnstoff |
| **Verb. 226** | 1-Ethyl-3-[3-(3,4,5-trimethoxy-phenyl)-pyrido[2,3-b]pyrazin-6-yl]-harnstoff |
| **Verb. 227** | 1-[3-(3,5-Difluor-2-methoxy-phenyl)-pyrido[2,3-b]pyrazin-6-yl]-3-ethyl-harnstoff |

(fortgesetzt)

| | |
|---|---|
| **Verb. 228** | 1-Ethyl-3-[3-(4-trifluormethyl-phenyl)-pyrido[2,3-b]pyrazin-6-yl]-harnstoff |
| **Verb. 229** | 1-Ethyl-3-[3-(2,3,4-trimethoxy-phenyl)-pyrido[2,3-b]pyrazin-6-yl]-harnstoff |
| **Verb. 230** | 1-[3-(3-Chlor-4-methoxy-phenyl)-pyrido[2,3-b]pyrazin-6-yl]-3-ethyl-harnstoff |
| **Verb. 231** | 1-Ethyl-3-[3-(3-fluor-4-hydroxy-phenyl)-pyrido[2,3-b]pyrazin-6-yl]-harnstoff |
| **Verb. 232** | 1-Ethyl-3-[3-(6-fluor-pyridin-3-yl)-pyrido[2,3-b]pyrazin-6-yl]-harnstoff |
| **Verb. 233** | 1-[3-(2,4-Dimethyl-thiazol-5-yl)-pyrido-[2,3-b]pyrazin-6-yl]-3-ethyl-harnstoff |
| **Verb. 234** | 1-Ethyl-3-[3-(2-methoxy-phenyl)-pyrido[2,3-b]pyrazin-6-yl]-harnstoff |
| **Verb. 235** | 1-[3-(2-Chlor-pyridin-4-yl)-pyrido[2,3-b]pyrazin-6-yl]-3-ethyl-harnstoff |
| **Verb. 236** | 1-[3-(5-Acetyl-thiophen-2-yl)-pyrido[2,3-b]pyrazin-6-yl]-3-ethyl-harnstoff |
| **Verb. 237** | 1-[3-(5-Chlor-thiophen-2-yl)-pyrido[2,3-b]pyrazin-6-yl]-3-ethyl-harnstoff |
| **Verb. 238** | 1-Ethyl-3-[3-(3-methoxy-phenyl)-pyrido[2,3-b]pyrazin-6-yl]-harnstoff |
| **Verb. 239** | 1-[3-(3-Brom-5-methoxy-phenyl)-pyrido[2,3-b]pyrazin-6-yl]-3-ethyl-harnstoff |
| **Verb. 240** | 1-[3-(Benzothiazol-6-ylamino)-pyrido[2,3-b]pyrazin-6-yl]-3-ethyl-harnstoff |
| **Verb. 241** | 1-Ethyl-3-[3-(4-methyl-3-trifluoromethyl-phenylamino)-pyrido[2,3-b]pyrazin-6-yl]-harnstoff |
| **Verb. 242** | 1-[3-(3-Cyano-4-methyl-phenylamino)-pyrido[2,3-b]pyrazin-6-yl]-3-ethyl-harnstoff |
| **Verb. 243** | 1-Ethyl-3-[3-(4-phenoxy-phenylamino)-pyrido[2,3-b]pyrazin-6-yl]-harnstoff |
| **Verb. 244** | 1-[3-(4-Chlor-3-methyl-phenylamino)-pyrido[2,3-b]pyrazin-6-yl]-3-ethyl-harnstoff |
| **Verb. 245** | 1-[3-(2-Chlor-4-methyl-phenylamino)-pyrido[2,3-b]pyrazin-6-yl]-3-ethyl-harnstoff |
| **Verb. 246** | 1-Ethyl-3-[3-(3-trifluoromethyl-phenylamino)-pyrido[2,3-b]pyrazin-6-yl]-harnstoff |
| **Verb. 247** | 1-[3-(2-Chlor-4-trifluormethoxy-phenylamino)-pyrido[2,3-b]pyrazin-6-yl]-3-ethyl-harnstoff |
| **Verb. 248** | 1-[3-(4-Chlor-2-methoxy-5-methyl-phenylamino)-pyrido[2,3-b]pyrazin-6-yl]-3-ethyl-harnstoff |
| **Verb. 249** | 1-Ethyl-3-[3-(4-methylsulfanyl-phenyl)-pyrido[2,3-b]pyrazin-6-yl]-harnstoff |
| **Verb. 250** | 3-[6-(3-Ethyl-ureido)-pyrido[2,3-b]pyrazin-3-yl]-benzolsulfonamid |
| **Verb. 251** | N-{4-[3-(3-Phenyl-pyrido[2,3-b]pyrazin-6-yl)-ureido]-butyl}-methansulfonamid |
| **Verb. 252** | 1-[3-(Benzo[1,3]dioxol-5-ylamino)-pyrido[2,3-b]pyrazin-6-yl]-3-ethyl-harnstoff |
| **Verb. 253** | 3-[6-(3-Ethyl-ureido)-pyrido[2,3-b]pyrazin-3-ylamino]-benzoesäure |
| **Verb. 254** | 2-Chlor-4-[6-(3-ethyl-ureido)-pyrido[2,3-b]pyrazin-3-ylamino]-benzoesäure |
| **Verb. 255** | 1-Ethyl-3-[3-(3-methoxy-5-trifluormethyl-phenylamino)-pyrido[2,3-b]pyrazin-6-yl]-harnstoff |
| **Verb. 256** | 1-Ethyl-3-[3-(pyrimidin-2-ylamino)-pyrido[2,3-b]pyrazin-6-yl]-harnstoff |
| **Verb. 257** | 6-[6-(3-Ethyl-ureido)-pyrido[2,3-b]pyrazin-3-ylamino]-naphthalin-2-carbonsäure |
| **Verb. 258** | 1-Ethyl-3-[3-(4-hydroxy-chinolin-2-ylamino)-pyrido[2,3-b]pyrazin-6-yl]-harnstoff |
| **Verb. 259** | 1-Ethyl-3-[3-(chinolin-6-ylamino)-pyrido[2,3-b]pyrazin-6-yl]-harnstoff |
| **Verb. 260** | 1-Ethyl-3-[3-(3,4,5-trimethoxy-phenylamino)-pyrido[2,3-b]pyrazin-6-yl]-harnstoff |
| **Verb. 261** | 1-Ethyl-3-[3-(1H-indol-6-ylamino)-pyrido[2,3-b]pyrazin-6-yl]-harnstoff |
| **Verb. 262** | 1-Ethyl-3-[3-(3-oxo-3,4-dihydro-2H-benzo[1,4]oxazin-7-ylamino)-pyrido[2,3-b]pyrazin-6-yl]-harnstoff |
| **Verb. 263** | 1-Ethyl-3-[3-(chinoxalin-6-ylamino)-pyrido[2,3-b]pyrazin-6-yl]-harnstoff |
| **Verb. 264** | 1-Ethyl-3-[3-(3-trifluormethoxy-phenylamino)-pyrido[2,3-b]pyrazin-6-yl]-harnstoff |
| **Verb. 265** | 1-Ethyl-3-[3-(4-isopropoxy-phenylamino)-pyrido[2,3-b]pyrazin-6-yl]-harnstoff |
| **Verb. 266** | 1-[3-(Dibenzofuran-2-ylamino)-pyrido[2,3-b]pyrazin-6-yl]-3-ethyl-harnstoff |
| **Verb. 267** | 1-(6-Dimethylamino-hexyl)-3-[3-(4-hydroxy-3-methoxy-phenyl)-pyrido[2,3-b]pyrazin-6-yl]-harnstoff |
| **Verb. 268** | 1-(4-Dimethylamino-butyl)-3-[3-(4-hydroxy-3-methoxy-phenyl)-pyrido[2,3-b]pyrazin-6-yl]-harnstoff |
| **Verb. 269** | 1-[3-(4-Hydroxy-3-methoxy-phenyl)-pyrido[2,3-b]pyrazin-6-yl]-3-(5-morpholin-4-yl-pentyl)-harnstoff. |

## Claims

**1.** Use of a compound according to the general formula (I)

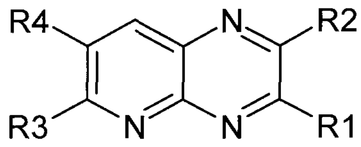

(I)

wherein the substituents R1, R2, R3, R4 have the following meaning:

R1 and R2 can be independently of one another

(i) hydrogen
(ii) hydroxyl
(iii) halogen
(iv) alkyl, wherein the alkyl group is saturated and can consist of 1 to 8 C atoms,
(v) unsubstituted or substituted aryl, wherein the alkyl group can be substituted with one or more, the same or different F, Cl, Br, I, $CF_3$, CN, $NH_2$, NH-alkyl, NH-cycloalkyl, NH-heterocyclyl, NH-aryl, NH-heteroaryl, NH-alkyl-cycloalkyl, NH-alkyl-heterocyclyl, NH-alkyl-aryl, NH-alkyl-heteroaryl, NH-alkyl-$NH_2$, NH-alkyl-OH, $N(alkyl)_2$, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, $NHSO_2$-alkyl, $NHSO_2$-cycloalkyl, $NHSO_2$-heterocyclyl, $NHSO_2$-aryl, $NHSO_2$-heteroaryl, $NHSO_2$-alkyl-aryl, $NHSO_2$-alkyl-heteroaryl, $NO_2$, SH, S-alkyl, S-aryl, S-heteroaryl, OH, $OCF_3$, O-alkyl, O-cycloalkyl, O-heterocyclyl, O-aryl, O-heteroaryl, O-alkyl-cycloalkyl, O-alkyl-heterocyclyl, O-alkyl-aryl, O-alkyl-heteroaryl, O-alkyl-OH, O-$(CH_2)_n$-O, OC(O)-alkyl, OC(O)-cycloalkyl, OC(O)-heterocyclyl, OC(O)-aryl, OC(O)-heteroaryl, OC(O)-alkyl-aryl, OC(O)-alkyl-heteroaryl, $OSO_3H$, $OSO_2$-alkyl, $OSO_2$-cycloalkyl, $OSO_2$-heterocyclyl, $OSO_2$-aryl, $OSO_2$-heteroaryl, $OSO_2$-alkyl-aryl, $OSO_2$-alkyl-heteroaryl, $OP(O)(OH)_2$, C(O)-alkyl, C(O)-aryl, C(O)-heteroaryl, $CO_2H$, $CO_2$-alkyl, $CO_2$-cycloalkyl, $CO_2$-heterocyclyl, $CO_2$-aryl, $CO_2$-heteroaryl, $CO_2$-alkyl-cycloalkyl, $CO_2$-alkyl-heterocyclyl, $CO_2$-alkyl-aryl, $CO_2$-alkyl-heteroaryl, C(O)-$NH_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, $C(O)N(alkyl)_2$, $C(O)N(cycloalkyl)_2$, $C(O)N(aryl)_2$, $C(O)N(heteroaryl)_2$, SO-alkyl, SO-aryl, $SO_2$-alkyl, $SO_2$-aryl, $SO_2NH_2$, $SO_2NH$-alkyl, $SO_2NH$-aryl, $SO_2NH$-heteroaryl, $SO_2NH$-alkyl-aryl, $SO_3H$, $SO_2O$-alkyl, $SO_2O$-aryl, $SO_2O$-alkyl-aryl, alkyl, cycloalkyl, heterocyclyl, aryl or heteroaryl, n can have the value 1, 2 or 3 and the alkyl-, cycloalkyl-, heterocyclyl-, aryl-, heteroaryl-, alkyl-cycloalkyl-, alkyl-heterocyclyl-, alkyl-aryl- and alkyl-heteroaryl substituents for their part can in turn be substituted,
(vi) unsubstituted or substituted heteroaryl, wherein the heteroaryl group can be substituted with one or more, the same or different F, Cl, Br, I, $CF_3$, CN, $NH_2$, NH-alkyl, NH-cycloalkyl, NH-heterocyclyl, NH-aryl, NH-heteroaryl, NH-alkyl-cycloalkyl, NH-alkyl-heterocyclyl, NH-alkyl-aryl, NH-alkyl-heteroaryl, NH-alkyl-$NH_2$, NH-alkyl-OH, $N(alkyl)_2$, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, $NHSO_2$-alkyl, $NHSO_2$-cycloalkyl, $NHSO_2$-heterocyclyl, $NHSO_2$-aryl, $NHSO_2$-heteroaryl, $NHSO_2$-alkyl-aryl, $NHSO_2$-alkyl-heteroaryl, $NO_2$, SH, S-alkyl, S-aryl, S-heteroaryl, OH, $OCF_3$, O-alkyl, O-cycloalkyl, O-aryl, O-heteroaryl, O-alkyl-cycloalkyl, O-alkyl-heterocyclyl, O-alkyl-aryl, O-alkyl-heteroaryl, OC(O)-alkyl, OC(O)-cycloalkyl, OC(O)-heterocyclyl, OC(O)-aryl, OC(O)-heteroaryl, OC(O)-alkyl-aryl, OC(O)-alkyl-heteroaryl, $OSO_3H$, $OSO_2$-alkyl, $OSO_2$-cycloalkyl, $OSO_2$-heterocyclyl, $OSO_2$-aryl, $OSO_2$-heteroaryl, $OSO_2$-alkyl-aryl, $OSO_2$-alkyl-heteroaryl, $OP(O)(OH)_2$, C(O)-alkyl, C(O)-aryl, C(O)-heteroaryl, $CO_2H$, $CO_2$-alkyl, $CO_2$-cycloalkyl, $CO_2$-heterocyclyl, $CO_2$-aryl, $CO_2$-heteroaryl, $CO_2$-alkyl-cycloalkyl, $CO_2$-alkyl-heterocyclyl, $CO_2$-alkyl-aryl, $CO_2$-alkyl-heteroaryl, C(O)-$NH_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, $C(O)N(alkyl)_2$, $C(O)N(cycloalkyl)_2$, $C(O)N(aryl)_2$, $C(O)N(heteroaryl)_2$, $SO_2NH_2$, $SO_2NH$-alkyl, $SO_2NH$-aryl, $SO_2NH$-heteroaryl, $SO_2NH$-alkyl-aryl, S03H, $SO_2O$-alkyl, $SO_2O$-aryl, $SO_2O$-alkyl-aryl, alkyl, cycloalkyl, heterocyclyl, aryl or heteroaryl, and the alkyl-, cycloalkyl-, heterocyclyl-, aryl- and heteroaryl substituents for their part can in turn be substituted,
(vii) OR5, wherein R5 can be alkyl, cycloalkyl, heterocyclyl, aryl, heteroaryl, alkyl-cycloalkyl, alkyl-heterocyclyl, alkyl-aryl or alkyl-heteroaryl, and the alkyl-, cycloalkyl-, heterocyclyl-, aryl-, heteroaryl-, alkyl-cycloalkyl, alkyl-heterocyclyl, alkyl-aryl or alkyl-heteroaryl substituents for their part can in turn be substituted,
(viii) SR6, wherein R6 can be alkyl, cycloalkyl, heterocyclyl, aryl, heteroaryl, alkyl-cycloalkyl, alkyl-heterocyclyl, alkyl-aryl or alkyl-heteroaryl and the alkyl-, cycloalkyl-, heterocyclyl-, aryl- and heteroaryl-, alkyl-cycloalkyl-, alkyl-heterocyclyl-, alkyl-aryl- or alkyl-heteroaryl substituents for their part can in turn be substituted,
(ix) NR7R8, wherein R7 and R8 independently of one another can be hydrogen, alkyl, cycloalkyl, heterocyclyl, aryl, heteroaryl, alkyl-cycloalkyl, alkyl-heterocyclyl, alkyl-aryl or alkyl-heteroaryl and the alkyl-, cycloalkyl-, heterocyclyl-, aryl- and heteroaryl-, alkyl-cycloalkyl, alkyl-heterocyclyl, alkyl-aryl or alkyl-heteroaryl substituents for their part can in turn be substituted,

R3 and R4 can independently of one another mean hydrogen or NR9R10 assuming that if R3 = NR9R10, R4 = H, and if R4 = NR9R10, R3 = H,
wherein R9 can be hydrogen, alkyl, cycloalkyl, heterocyclyl, aryl, heteroaryl, alkyl-cycloalkyl, alkyl-heterocyclyl, alkyl-aryl or alkyl-heteroaryl and the alkyl-, cycloalkyl-, heterocyclyl-, aryl- and heteroaryl-, alkyl-cycloalkyl, alkyl-heterocyclyl, alkyl-aryl or alkyl-heteroaryl substituents for their part can in turn be substituted,
and R10:

can mean -C(Y)NR11R12, wherein Y = O, S and R11 and R12 can be independently of one another

(i) hydrogen,
(ii) unsubstituted or substituted alkyl wherein wherein the alkyl group can be substituted with one or more, the same or different F, Cl, Br, I, $CF_3$, CN, $NH_2$, NH-alkyl, NH-cycloalkyl, NH-heterocyclyl, NH-aryl, NH-heteroaryl, NH-alkyl-cycloalkyl, NH-alkyl-heterocyclyl, NH-alkyl-aryl, NH-alkyl-heteroaryl, $N(alkyl)_2$, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, $NHSO_2$-alkyl, $NHSO_2$-cycloalkyl, $NHSO_2$-heterocyclyl, $NHSO_2$-aryl, $NHSO_2$-heteroaryl, $NHSO_2$-alkyl-aryl, $NHSO_2$-alkyl-heteroaryl, $NO_2$, SH, S-alkyl, S-cycloalkyl, S-heterocyclyl, S-aryl, S-heteroaryl, OH, $OCF_3$, O-alkyl, O-cycloalkyl, O-heterocyclyl, O-aryl, O-heteroaryl, O-alkyl-cycloalkyl, O-alkyl-heterocyclyl, 0-alkyl-aryl, O-alkyl-heteroaryl, OC(O)-alkyl, OC(O)-cycloalkyl, OC(O)-heterocyclyl, OC(O)-aryl, OC(O)-heteroaryl, OC(O)-alkyl-aryl, OC(O)-alkyl-heteroaryl, $OSO_3H$, $OSO_2$-alkyl, $OSO_2$-cycloalkyl, $OSO_2$-heterocyclyl, $OSO_2$-aryl, $OSO_2$-heteroaryl, $0SO_2$-alkyl-aryl, $OSO_2$-alkyl-heteroaryl, $OP(O)(OH)_2$, C(O)-alkyl, C(O)-aryl, C(O)-heteroaryl, $CO_2H$, $CO_2$-alkyl, $CO_2$-cycloalkyl, $CO_2$-heterocyclyl, $CO_2$-aryl, $CO_2$-heteroaryl, $CO_2$-alkyl-cycloalkyl, $CO_2$-alkyl-heterocyclyl, $CO_2$-alkyl-aryl, $CO_2$-alkyl-heteroaryl, C(O)-$NH_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, $C(O)N(alkyl)_2$, $C(O)N(cycloalkyl)_2$, $C(O)N(aryl)_2$, $C(O)N(heteroaryl)_2$, SO-alkyl, SO-aryl, $SO_2$-alkyl, $SO_2$-aryl, $SO_2NH_2$, $SO_2NH$-alkyl, $SO_2NH$-aryl, $SO_2NH$-heteroaryl, $SO_2NH$-alkyl-aryl, $SO_3H$, $SO_2O$-alkyl, $SO_2O$-aryl, $SO_2O$-alkyl-aryl, cycloalkyl, heterocyclyl, aryl or heteroaryl,
(iii) unsubstituted or substituted cycloalkyl, wherein the cycloalkyl group can be substituted with one or more, the same or different F, Cl, Br, I, $NH_2$, NH-alkyl, NH-cycloalkyl, NH-heterocyclyl, NH-aryl, NH-heteroaryl, NH-alkyl-aryl, NH-alkyl-heteroaryl, $N(alkyl)_2$, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, $NHSO_2$-alkyl, $NHSO_2$-cycloalkyl, $NHSO_2$-heterocyclyl, $NHSO_2$-aryl, $NHSO_2$-heteroaryl, $NHSO_2$-alkyl-aryl, $NHSO_2$-alkyl-heteroaryl, OH, O-alkyl, O-cycloalkyl, O-heterocyclyl, O-aryl, O-heteroaryl, O-alkyl-aryl, O-alkyl-heteroaryl, OC(O)-alkyl, OC(O)-cycloalkyl, OC(O)-heterocyclyl, OC(O)-aryl, OC(O)-heteroaryl, OC(O)-alkyl-aryl, OC(O)-alkyl-heteroaryl, $OSO_3H$, $OSO_2$-alkyl, $OSO_2$-cycloalkyl, $OSO_2$-heterocyclyl, $OSO_2$-aryl, $OSO_2$-heteroaryl, $OSO_2$-alkyl-aryl, $OSO_2$-alkyl-heteroaryl, $OP(O)(OH)_2$, $CO_2H$, $CO_2$-alkyl, $CO_2$-cycloalkyl, $CO_2$-heterocyclyl, $CO_2$-aryl, $CO_2$-heteroaryl, $CO_2$-alkyl-cycloalkyl, $CO_2$-alkyl-heterocyclyl, $CO_2$-alkyl-aryl, $CO_2$-alkyl-heteroaryl, C(O)-$NH_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, $C(O)N(alkyl)_2$, $C(O)N(cycloalkyl)_2$, $C(O)N(aryl)_2$, $C(O)N(heteroaryl)_2$, alkyl, or aryl,
(iv) unsubstituted or substituted heterocyclyl, wherein the heterocyclyl group can be substituted with one or more, the same or different OH, O-alkyl, O-aryl, $NH_2$, NH-alkyl, NH-aryl, alkyl, alkyl-aryl or aryl,
(v) unsubstituted or substituted aryl, wherein the aryl group can be substituted with one or more, the same or different F, Cl, Br, I, $CF_3$, CN, $NH_2$, NH-alkyl, NH-cycloalkyl, NH-heterocyclyl, NH-aryl, NH-heteroaryl, NH-alkyl-cycloalkyl, NH-alkyl-heterocyclyl, NH-alkyl-aryl, NH-alkyl-heteroaryl, NH-alkyl-$NH_2$, NH-alkyl-OH, $N(alkyl)_2$, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, $NHSO_2$-alkyl, $NHSO_2$-cycloalkyl, $NHSO_2$-heterocyclyl, $NHSO_2$-aryl, $NHSO_2$-heteroaryl, $NHSO_2$-alkyl-aryl, $NHSO_2$-alkyl-heteroaryl, $NO_2$, SH, S-alkyl, S-cycloalkyl, S-heterocyclyl, S-aryl, S-heteroaryl, OH, $OCF_3$, O-alkyl, O-cycloalkyl, O-heterocyclyl, O-aryl, O-heteroaryl, O-alkyl-cycloalkyl, O-alkyl-heterocyclyl, O-alkyl-aryl, O-alkyl-heteroaryl, O-alkyl-OH, O-$(CH_2)_n$-O, OC(O)-alkyl, OC(O)-cycloalkyl, OC(O)-heterocyclyl, OC(O)-aryl, OC(O)-heteroaryl, OC(O)-alkyl-aryl, OC(O)-alkyl-heteroaryl, $OSO_3H$, $OSO_2$-alkyl, $OSO_2$-cycloalkyl, $OSO_2$-heterocyclyl, $OSO_2$-aryl, $OSO_2$-heteroaryl, $OSO_2$-alkyl-aryl, $OSO_2$-alkyl-heteroaryl, $OP(O)(OH)_2$, C(O)-alkyl, C(O)-aryl, C(O)-heteroaryl, $CO_2H$, $CO_2$-alkyl, $CO_2$-cycloalkyl, $CO_2$-heterocyclyl, $CO_2$-aryl, $CO_2$-heteroaryl, $CO_2$-alkyl-cycloalkyl, $CO_2$-alkyl-heterocyclyl, $CO_2$-alkyl-aryl, $CO_2$-alkyl-heteroaryl, C(O)-$NH_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, $C(O)N(alkyl)_2$, $C(O)N(cycloalkyl)_2$, $C(O)N(aryl)_2$, $C(O)N(heteroaryl)_2$, SO-alkyl, SO-aryl, $SO_2$-alkyl, $SO_2$-aryl, $SO_2NH_2$, $SO_2NH$-alkyl, $SO_2NH$-aryl, $SO_2NH$-heteroaryl, $SO_2NH$-alkyl-aryl, S03H, $SO_2O$-alkyl, $SO_2O$-aryl, $SO_2O$-alkyl-aryl, alkyl, cycloalkyl, heterocyclyl, aryl or heteroaryl, and n can have the value 1, 2 or 3,

(vi) unsubstituted or substituted heteroaryl, wherein the heteroaryl group can be substituted with one or more, the same or different F, Cl, Br, I, $CF_3$, CN, $NH_2$, NH-alkyl, NH-cycloalkyl, NH-heterocyclyl, NH-aryl, NH-heteroaryl, NH-alkyl-cycloalkyl, NH-alkyl-heterocyclyl, NH-alkyl-aryl, NH-alkyl-heteroaryl, NH-alkyl-$NH_2$, NH-alkyl-OH, $N(alkyl)_2$, NHC(O)-alkyl, NHC(O)-cycloalkyl, NHC(O)-heterocyclyl, NHC(O)-aryl, NHC(O)-heteroaryl, NHC(O)-alkyl-aryl, NHC(O)-alkyl-heteroaryl, $NHSO_2$-alkyl, $NHSO_2$-cycloalkyl, $NHSO_2$-heterocyclyl, $NHSO_2$-aryl, $NHSO_2$-heteroaryl, $NHSO_2$-alkyl-aryl, $NHSO_2$-alkyl-heteroaryl, $NO_2$, SH, S-alkyl, S-aryl, S-heteroaryl, OH, $OCF_3$, O-alkyl, O-cycloalkyl, O-aryl, O-heteroaryl, O-alkyl-cycloalkyl, O-alkyl-heterocyclyl, O-alkyl-aryl, O-alkyl-heteroaryl, OC(O)-alkyl, OC(O)-cycloalkyl, OC(O)-heterocyclyl, OC(O)-aryl, OC(O)-heteroaryl, OC(O)-alkyl-aryl, OC(O)-alkyl-heteroaryl, $OSO_3H$, $OSO_2$-alkyl, $OSO_2$-cycloalkyl, $OSO_2$-heterocyclyl, $OSO_2$-aryl, $OSO_2$-heteroaryl, $OSO_2$-alkyl-aryl, $OSO_2$-alkyl-heteroaryl, $OP(O)(OH)_2$, C(O)-alkyl, C(O)-aryl, C(O)-heteroaryl, $CO_2H$, $CO_2$-alkyl, $CO_2$-cycloalkyl, $CO_2$-heterocyclyl, $CO_2$-aryl, $CO_2$-heteroaryl, $CO_2$-alkyl-cycloalkyl, $CO_2$-alkyl-heterocyclyl, $CO_2$-alkyl-aryl, $CO_2$-alkyl-heteroaryl, C(O)-$NH_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, $C(O)N(alkyl)_2$, $C(O)N(cycloalkyl)_2$, $C(O)N(aryl)_2$, $C(O)N(heteroaryl)_2$, $SO_2NH_2$, $SO_2NH$-alkyl, $SO_2NH$-aryl, $SO_2NH$-heteroaryl, $SO_2NH$-alkyl-aryl, $SO_3H$, $SO_2O$-alkyl, $SO_2O$-aryl, $SO_2O$-alkyl-aryl, alkyl, cycloalkyl, heterocyclyl, aryl or heteroaryl,

(vii) -C(O)-R17, wherein R17 can be alkyl, aryl or heteroaryl and the alkyl and aryl substitutents for their part can in turn be substituted,

(viii) or R11 and R12 together can mean cycloalkyl or heterocyclyl;

can mean -C(Y)NR13R14 wherein Y = NH and R13 and R14 can mean independently of one another,

(i) hydrogen,

(ii) unsubstituted or substituted alkyl, wherein the alkyl group can be substituted with one or more, the same or different F, Cl, Br, I, $CF_3$, CN, $NH_2$, NH-alkyl, NH-cycloalkyl, NH-heterocyclyl, NH-aryl, NH-heteroaryl, NH-alkyl-aryl, NH-alkyl-heteroaryl, $N(alkyl)_2$, NHC(O)-alkyl, NHC(O)-cycloalkyl, NHC(O)-heterocyclyl, NHC(O)-aryl, NHC(O)-heteroaryl, $NHSO_2$-alkyl, $NHSO_2$-cycloalkyl, $NHSO_2$-aryl, $NHSO_2$-heteroaryl, $NO_2$, SH, S-alkyl, S-cycloalkyl, S-heterocyclyl, S-aryl, S-heteroaryl, OH, $OCF_3$, O-alkyl, O-cycloalkyl, O-heterocyclyl, O-aryl, O-heteroaryl, O-alkyl-cycloalkyl, O-alkyl-aryl, O-alkyl-heteroaryl, OC(O)-alkyl, OC(O)-cycloalkyl, OC(O)-heterocyclyl, OC(O)-aryl, OC(O)-heteroaryl, $OSO_2$-alkyl, $OSO_2$-cycloalkyl, $OSO_2$-aryl, $OSO_2$-heteroaryl, C(O)-alkyl, C(O)-aryl, $CO_2H$, $CO_2$-alkyl, $CO_2$-cycloalkyl, $CO_2$-heterocyclyl, $CO_2$-aryl, $CO_2$-heteroaryl, $CO_2$-alkyl-cycloalkyl, $CO_2$-alkyl-heterocyclyl, $CO_2$-alkyl-aryl, $CO_2$-alkyl-heteroaryl, C(O)-$NH_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, $C(O)N(alkyl)_2$, $C(O)N(cycloalkyl)_2$, $C(O)N(aryl)_2$, $C(O)N(heteroaryl)_2$, SO-alkyl, SO-aryl, $SO_2$-alkyl, $SO_2$-aryl, $SO_2NH_2$, $SO_3H$, alkyl, cycloalkyl, heterocyclyl, aryl or heteroaryl,

(iii) unsubstituted or substituted cycloalkyl, wherein the cycloalkyl group can be substituted with one or more, the same or different F, Cl, Br, I, $NH_2$, NH-alkyl, NH-cycloalkyl, NH-heterocyclyl, NH-aryl, NH-heteroaryl, NH-alkyl-aryl, NH-alkyl-heteroaryl, $N(alkyl)_2$, NHC(O)-alkyl, NHC(O)-cycloalkyl, NHC(O)-heterocyclyl, NHC(O)-aryl, NHC(O)-heteroaryl, $NHSO_2$-alkyl, $NHSO_2$-cycloalkyl, $NHSO_2$-aryl, $NHSO_2$-heteroaryl, OH, O-alkyl, O-cycloalkyl, 0-heterocyclyl, O-aryl, O-heteroaryl, O-alkyl-aryl, O-alkyl-heteroaryl, OC(O)-alkyl, OC(O)-cycloalkyl, OC(O)-heterocyclyl, OC(O)-aryl, OC(O)-heteroaryl, $OSO_2$-alkyl, $OSO_2$-cycloalkyl, $OSO_2$-aryl, $OSO_2$-heteroaryl, $CO_2H$, $CO_2$-alkyl, $CO_2$-cycloalkyl, $CO_2$-heterocyclyl, $CO_2$-aryl, $CO_2$-heteroaryl, C(O)-$NH_2$, C(O)NH-alkyl, C(O)NH-cycloalkyl, C(O)NH-heterocyclyl, C(O)NH-aryl, C(O)NH-heteroaryl, C(O)NH-alkyl-aryl, C(O)NH-alkyl-heteroaryl, C(O)N(alkyl)$_2$, alkyl, or aryl,

(iv) unsubstituted or substituted heterocyclyl, wherein the heterocyclyl group can be substituted with one or more, the same or different OH, O-alkyl, O-aryl, NH$_2$, NH-alkyl, NH-aryl, alkyl, or aryl,

(v) unsubstituted or substituted aryl, wherein the aryl group can be substituted with one or more, the same or different F, Cl, Br, I, CF$_3$, CN, NH$_2$, NH-alkyl, NH-cycloalkyl, NH-heterocyclyl, NH-aryl, NH-heteroaryl, NH-alkyl-cycloalkyl, NH-alkyl-heterocyclyl, NH-alkyl-aryl, NH-alkyl-heteroaryl, NH-alkyl-NH$_2$, NH-alkyl-OH, N(alkyl)$_2$, NHC(O)-alkyl, NHC(O)-cycloalkyl, NHC(O)-heterocyclyl, NHC(O)-aryl, NHC(O)-heteroaryl, NHSO$_2$-alkyl, NHSO$_2$-aryl, NHSO$_2$-heteroaryl, NO$_2$, SH, S-alkyl, S-cycloalkyl, S-heterocyclyl, S-aryl, S-heteroaryl, OH, OCF$_3$, O-alkyl, O-cycloalkyl, O-heterocyclyl, O-aryl, O-heteroaryl, O-alkyl-cycloalkyl, O-alkyl-heterocyclyl, O-alkyl-aryl, O-alkyl-heteroaryl, O-alkyl-OH, O-(CH$_2$)$_n$-O, OC(O)-alkyl, OC(O)-cycloalkyl, OC(O)-heterocyclyl, OC(O)-aryl, OC(O)-heteroaryl, OSO$_2$-alkyl, OSO$_2$-cycloalkyl, OSO$_2$-aryl, OSO$_2$-heteroaryl, C(O)-alkyl, C(O)-aryl, C(O)-heteroaryl, CO$_2$H, CO$_2$-alkyl, CO$_2$-cycloalkyl, CO$_2$-heterocyclyl, CO$_2$-aryl, CO$_2$-heteroaryl, CO$_2$-alkyl-cycloalkyl, CO$_2$-alkyl-heterocyclyl, CO$_2$-alkyl-aryl, CO$_2$-alkyl-heteroaryl, C(O)-NH$_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)$_2$, C(O)N(cycloalkyl)$_2$, C(O)N(aryl)$_2$, C(O)N(heteroaryl)$_2$, SO-alkyl, SO-aryl, SO$_2$-alkyl, SO$_2$-aryl, SO$_2$NH$_2$, SO$_2$NH-alkyl, SO$_2$NH-aryl, SO$_2$NH-heteroaryl, S03H, SO$_2$O-alkyl, SO$_2$O-aryl, SO$_2$O-heteroaryl, alkyl, cycloalkyl, heterocyclyl, aryl or heteroaryl, and n can have the value 1, 2 or 3,

(vi) unsubstituted or substituted heteroaryl, wherein the heteroaryl group can be substituted with one or more, the same or different F, Cl, Br, I, CF$_3$, CN, NH$_2$, NH-alkyl, NH-cycloalkyl, NH-heterocyclyl, NH-aryl, NH-heteroaryl, NH-alkyl-aryl, NH-alkyl-heteroaryl, N(alkyl)$_2$, NHC(O)-alkyl, NHC(O)-cycloalkyl, NHC(O)-heterocyclyl, NHC(O)-aryl, NHC(O)-heteroaryl, NHSO$_2$-alkyl, NHSO$_2$-aryl, NHSO$_2$-heteroaryl, NO$_2$, SH, S-alkyl, S-aryl, OH, OCF$_3$, O-alkyl, O-cycloalkyl, O-heterocyclyl, O-aryl, O-heteroaryl, OC(O)-alkyl, OC(O)-cycloalkyl, OC(O)-heterocyclyl, OC(O)-aryl, OC(O)-heteroaryl, OSO$_2$-alkyl, OSO$_2$-cycloalkyl, OSO$_2$-aryl, OSO$_2$-heteroaryl, C(O)-alkyl, C(O)-aryl, C(O)-heteroaryl, CO$_2$H, CO$_2$-alkyl, CO$_2$-cycloalkyl, CO$_2$-heterocyclyl, CO$_2$-aryl, CO$_2$-heteroaryl, CO$_2$-alkyl-cycloalkyl, CO$_2$-alkyl-heterocyclyl, CO$_2$-alkyl-aryl, CO$_2$-alkyl-heteroaryl, C(O)-NH$_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)$_2$, C(O)N(cycloalkyl)$_2$, C(O)N(aryl)$_2$, C(O)N(heteroaryl)$_2$, SO$_2$-alkyl, SO$_2$-aryl, SO$_2$NH$_2$, SO$_2$NH-alkyl, SO$_2$NH-aryl, SO$_2$NH-heteroaryl, S03H, SO$_2$O-alkyl, SO$_2$O-aryl, SO$_2$O-heteroaryl, alkyl, cycloalkyl, heterocyclyl, aryl or heteroaryl,

(vii) or R13 and R14 together can mean cycloalkyl or heterocyclyl,

can mean -C(NR15)R16, wherein R15 = H and R16 can be

(i) unsubstituted or substituted alkyl, wherein the alkyl group can be substituted with one or more, the same or different F, Cl, Br, I, CF$_3$, NH$_2$, NH-alkyl, NH-cycloalkyl, NH-heterocyclyl, NH-aryl, NH-heteroaryl, NH-alkyl-aryl, NH-alkyl-heteroaryl, N(alkyl)$_2$, NHC(O)-alkyl, NHC(O)-cycloalkyl, NHC(O)-heterocyclyl, NHC(O)-aryl, NHC(O)-heteroaryl, NHSO$_2$-alkyl, NHSO$_2$-cycloalkyl, NHSO$_2$-aryl, NHSO$_2$-heteroaryl, NO$_2$, SH, S-alkyl, S-cycloalkyl, S-heterocyclyl, S-aryl, S-heteroaryl, OH, OCF$_3$, O-alkyl, O-cycloalkyl, O-heterocyclyl, O-aryl, O-heteroaryl, O-alkyl-cycloalkyl, O-alkyl-aryl, O-alkyl-heteroaryl, OC(O)-alkyl, OC(O)-cycloalkyl, OC(O)-heterocyclyl, OC(O)-aryl, OC(O)-heteroaryl, OSO$_2$-alkyl, OSO$_2$-cycloalkyl, OSO$_2$-aryl, OSO$_2$-heteroaryl, C(O)-alkyl, C(O)-aryl, CO$_2$H, CO$_2$-alkyl, CO$_2$-cycloalkyl, CO$_2$-heterocyclyl, CO$_2$-aryl, CO$_2$-heteroaryl, CO$_2$-alkyl-cycloalkyl, CO$_2$-alkyl-heterocyclyl, CO$_2$-alkyl-aryl, CO$_2$-alkyl-heteroaryl, C(O)-NH$_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N(alkyl)$_2$, C(O)N(cycloalkyl)$_2$, C(O)N(aryl)$_2$, C(O)N(heteroaryl)$_2$, SO-alkyl, SO-aryl, SO$_2$-alkyl, SO$_2$-aryl, SO$_2$NH$_2$, SO$_3$H, alkyl, cycloalkyl, heterocyclyl, aryl or heteroaryl,

(ii) unsubstituted or substituted cycloalkyl, wherein the cycloalkyl group can be substituted with one or more, the same or different F, Cl, Br, I, NH$_2$, NH-alkyl, NH-cycloalkyl, NH-heterocyclyl, NH-aryl, NH-heteroaryl, NH-alkyl-aryl, NH-alkyl-heteroaryl, N(alkyl)$_2$, NHC(O)-alkyl, NHC(O)-cycloalkyl, NHC(O)-heterocyclyl, NHC(O)-aryl, NHC(O)-heteroaryl, NHSO$_2$-alkyl, NHSO$_2$-cycloalkyl, NHSO$_2$-aryl, NHSO$_2$-heteroaryl, OH, O-alkyl, O-cycloalkyl, O-heterocyclyl, O-aryl, O-heteroaryl, O-alkyl-aryl, O-alkyl-heteroaryl, OC(O)-alkyl, OC(O)-cycloalkyl, OC(O)-heterocyclyl, OC(O)-aryl, OC(O)-heteroaryl,

$OSO_2$-alkyl, $OSO_2$-cycloalkyl, $OSO_2$-aryl, $OSO_2$-heteroaryl, $CO_2H$, $CO_2$-alkyl, $CO_2$-cycloalkyl, $CO_2$-heterocyclyl, $CO_2$-aryl, $CO_2$-heteroaryl, C(O)-$NH_2$, C(O)NH-alkyl, C(O)NH-cycloalkyl, C(O)NH-heterocyclyl, C(O)NH-aryl, C(O)NH-heteroaryl, C(O)NH-alkyl-aryl, C(O)NH-alkyl-heteroaryl, C(O)N$(alkyl)_2$, alkyl, or aryl,

(iii) unsubstituted or substituted heterocyclyl, wherein the heterocyclyl group can be substituted with one or more, the same or different OH, O-alkyl, O-aryl, $NH_2$, NH-alkyl, NH-aryl, alkyl or aryl,

(iv) unsubstituted or substituted aryl, wherein the aryl group can be substituted with one or more, the same or different F, Cl, Br, I, $CF_3$, $NH_2$, NH-alkyl, NH-cycloalkyl, NH-heterocyclyl, NH-aryl, NH-heteroaryl, NH-alkyl-cycloalkyl, NH-alkyl-heterocyclyl, NH-alkyl-aryl, NH-alkyl-heteroaryl, NH-alkyl-$NH_2$, NH-alkyl-OH, N$(alkyl)_2$, NHC(O)-alkyl, NHC(O)-cycloalkyl, NHC(O)-heterocyclyl, NHC(O)-aryl, NHC(O)-heteroaryl, $NHSO_2$-alkyl, $NHSO_2$-aryl, $NHSO_2$-heteroaryl, $NO_2$, SH, S-alkyl, S-cycloalkyl, S-heterocyclyl, S-aryl, S-heteroaryl, OH, $OCF_3$, O-alkyl, O-cycloalkyl, O-heterocyclyl, O-aryl, O-heteroaryl, O-alkyl-cycloalkyl, O-alkyl-heterocyclyl, O-alkyl-aryl, O-alkyl-heteroaryl, O-alkyl-OH, O-$(CH_2)_n$-O, OC(O)-alkyl, OC(O)-cycloalkyl, OC(O)-heterocyclyl, OC(O)-aryl, OC(O)-heteroaryl, $OSO_2$-alkyl, $OSO_2$-cycloalkyl, $OSO_2$-aryl, $OSO_2$-heteroaryl, C(O)-alkyl, C(O)-aryl, C(O)-heteroaryl, $CO_2H$, $CO_2$-alkyl, $CO_2$-cycloalkyl, $CO_2$-heterocyclyl, $CO_2$-aryl, $CO_2$-heteroaryl, $CO_2$-alkyl-cycloalkyl, $CO_2$-alkyl-heterocyclyl, $CO_2$-alkyl-aryl, $CO_2$-alkyl-heteroaryl, C(O)-$NH_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N$(alkyl)_2$, C(O)N$(cycloalkyl)_2$, C(O)N$(aryl)_2$, C(O)N$(heteroaryl)_2$, SO-alkyl, SO-aryl, $SO_2$-alkyl, $SO_2$-aryl, $SO_2NH_2$, $SO_2$NH-alkyl, $SO_2$NH-aryl, $SO_2$NH-heteroaryl, S03H, $SO_2$O-alkyl, $SO_2$O-aryl, $SO_2$O-heteroaryl, alkyl, cycloalkyl, heterocyclyl, aryl or heteroaryl, and n can have the vallue 1, 2 or 3,

(v) unsubstituted or substituted heteroaryl, wherein the heteroaryl group can be substituted with one or more, the same or different F, Cl, Br, I, $CF_3$, $NH_2$, NH-alkyl, NH-cycloalkyl, NH-heterocyclyl, NH-aryl, NH-heteroaryl, NH-alkyl-aryl, NH-alkyl-heteroaryl, N$(alkyl)_2$, NHC(O)-alkyl, NHC(O)-cycloalkyl, NHC(O)-heterocyclyl, NHC(O)-aryl, NHC(O)-heteroaryl, $NHSO_2$-alkyl, $NHSO_2$-aryl, $NHSO_2$-heteroaryl, $NO_2$, SH, S-alkyl, S-aryl, OH, $OCF_3$, O-alkyl, O-cycloalkyl, O-heterocyclyl, O-aryl, O-heteroaryl, OC(O)-alkyl, OC(O)-cycloalkyl, OC(O)-heterocyclyl, OC(O)-aryl, OC(O)-heteroaryl, $OSO_2$-alkyl, $OSO_2$-cycloalkyl, $OSO_2$-aryl, $OSO_2$-heteroaryl, C(O)-alkyl, C(O)-aryl, C(O)-heteroaryl, $CO_2H$, $CO_2$-alkyl, $CO_2$-cycloalkyl, $CO_2$-heterocyclyl, $CO_2$-aryl, $CO_2$-heteroaryl, $CO_2$-alkyl-cycloalkyl, $CO_2$-alkyl-heterocyclyl, $CO_2$-alkyl-aryl, $CO_2$-alkyl-heteroaryl, C(O)-$NH_2$, C(O)NH-alkyl, C(O)NH-cycloalkyl, C(O)NH-heterocyclyl, C(O)NH-aryl, C(O)NH-heteroaryl, C(O)NH-alkyl-cycloalkyl, C(O)NH-alkyl-heterocyclyl, C(O)NH-alkyl-aryl, C(O)NH-alkyl-heteroaryl, C(O)N$(alkyl)_2$, C(O)N$(cycloalkyl)_2$, C(O)N$(aryl)_2$, C(O)N$(heteroaryl)_2$, $SO_2$-alkyl, $SO_2$-aryl, $SO_2NH_2$, $SO_2$NH-alkyl, $SO_2$NH-aryl, $SO_2$NH-heteroaryl, S03H, $SO_2$O-alkyl, $SO_2$O-aryl, $SO_2$O-heteroaryl, alkyl, cycloalkyl, heterocyclyl, aryl or heteroaryl;

for the production of a medicament for the treatment or prevention of physiological and/or pathophysiological states in mammals,
wherein the physiological and/or pathophysiological states are selected from the group consisting of:

rheumatic diseases, arthritic diseases, HIV infections, rheumatism, arthritis, AIDS, ARC (AIDS related complex), Kaposi’s sarcoma, tumours originating from the brain Alzheimer’s disease, colon tumour, gastric tumour, pancreatic tumour, leukaemia, hepatic tumour, renal tumour, head tumour, throat tumour, glioma, uterine cancer, endometrial cancer, cervico-uterine carcinoma, brain tumour, adeno-acanthoma, gastric tumour, colorectal tumour, oesophageal cancer, cancer of the thyroid, lymphoma, chronic leukaemia, acute leukaemia, diabetes, rheumatic arthritis, allergies, allergic diseases, heart diseases, obesity, overweight, hypertension.

**2.** Use according to Claim 1, wherein the alkyl group is seleted from the group consisting of: "methyl, ethyl, n-propyl, 2-propyl, n-butyl, sec.-butyl, tert.-butyl, n-pentyl, iso-pentyl, neo-pentyl, n-hexyl, 2-hexyl, n-octyl, ethylenyl (vinyl), ethynyl, propenyl (-CH2CH=CH2; -CH=CH-CH3, -C(=CH2)-CH3), propinyl (-CH2-C≡CH, -C≡C-CH3), butenyl, butinyl, pentenyl, pentinyl, hexenyl, hexinyl, heptenyl, heptinyl, octenyl, octinyl".

**3.** Use according to any one of Claims 1 to 2, wherein the heterocyclyl group is selected from the group consisting of: "tetrahydrofuryl, tetrahydropyranyl, pyrrolidinyl, piperidinyl, piperazinyl, morpholinyl".

**4.** Use according to any one of Claims 1 to 3, wherein the heteroaryl group is selected from the group consisting of:

"pyrrolyl, furyl, thienyl, thiazolyl, oxazolyl, isoxazolyl, pyrazolyl, imidazolyl, pyridinyl, pyrimidinyl, pyridazinyl, pyrazinyl, phthalazinyl, indolyl, indazolyl, indolizinyl, quinolinyl, isoquinolinyl, quinoxalinyl, quinazolinyl, carbazolyl, phenazinyl, phenothiazinyl, acridinyl".

**5.** Use according to any one of Claims 1 to 4, wherein in the compound is selected from the group consisting of:

| | | |
|---|---|---|
| **Compound 1** | 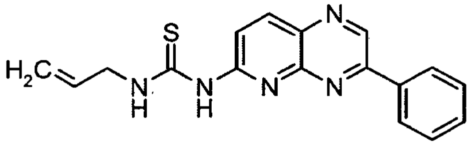 | 1-Allyl-3-(3-phenyl-pyrido[2,3-b]pyrazin-6-yl)-thiourea |
| **Compound 2** | 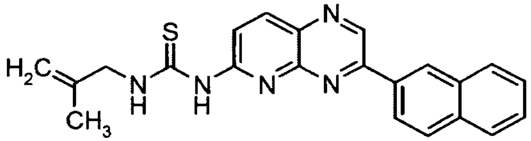 | 1-(2-Methyl-allyl)-3-(3-naphth-2-yl-pyrido[2,3-b]pyrazin-6-yl)-thiourea |
| **Compound 3** | 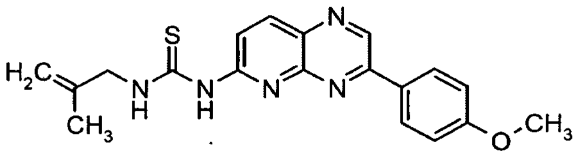 | 1-[3-(4-Methoxy-phenyl)-pyrido[2,3-b]pyrazin-6-yl]-3-(2-methyl-allyl)-thiourea |
| **Compound 4** | 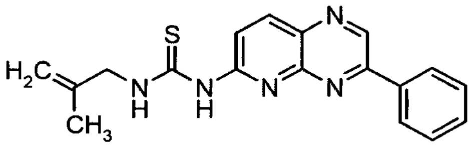 | 1-(2-Methyl-allyl)-3-(3-phenyl-pyrido[2,3-b]pyrazin-6-yl)-thiourea |
| **Compound 5** | 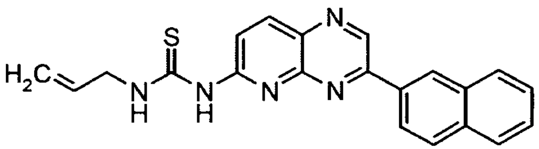 | 1-Allyl-3-(3-naphth-2-yl-pyrido[2,3-b]pyrazin-6-yl)-thiourea |
| **Compound 6** | 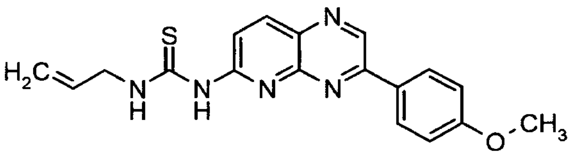 | 1 -Allyl-3-[3-(4-methoxy-phenyl)-pyrido[2,3-b]pyrazin-6-yl]-thiourea |
| **Compound 7** | 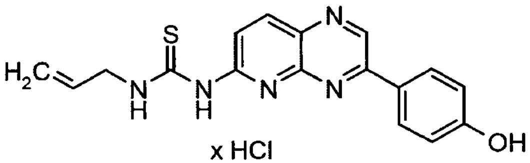 x HCl | 1-Allyl-3-[3-(4-hydroxy-phenyl)-pyrido[2,3-b]pyrazin-6-yl]-thiourea Hydrochloroid |

(continued)

| | | |
|---|---|---|
| **Compound 8** | 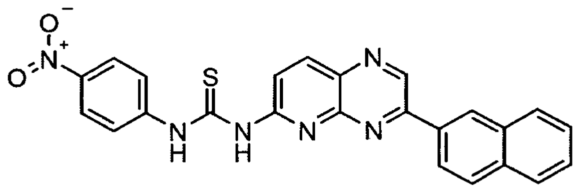 | 1-(3-Naphth-2-yl-pyrido[2,3-b]pyrazin-6-yl)-3-(4-nitrophenyl)-thiourea |
| **Compound 9** | 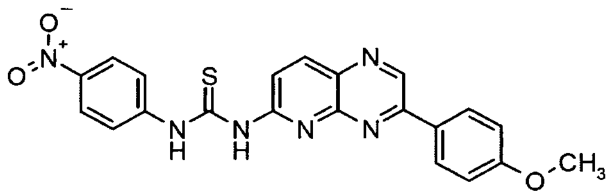 | 1-[3-(4-Methoxy-phenyl)-pyrido[2,3-b]pyrazin-6-yl]-3-(4-nitro-phenyl)-thiourea |
| **Compound 10** | 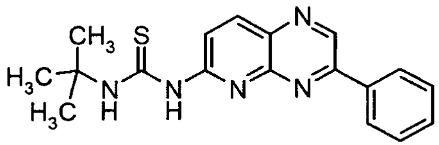 | 1-tert-Butyl-3-(3-phenyl-pyrido[2,3-b]pyrazin-6-yl)-thiourea |
| **Compound 11** | 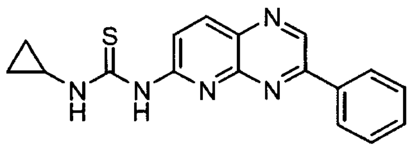 | 1-Cyclopropyl-3-(3-phenyl-pyrido[2,3-b]pyrazin-6-yl)-thiourea |
| **Compound 12** | 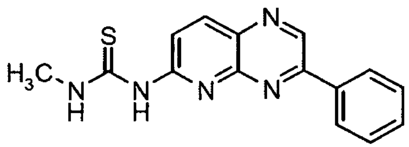 | 1-Methyl-3-(3-phenyl-pyrido[2,3-b]pyrazin-6-yl)-thiourea |
| **Compound 13** | 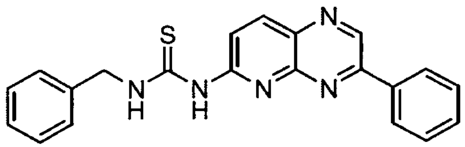 | 1-Benzyl-3-(3-phenyl-pyrido[2,3-b]pyrazin-6-yl)-thiourea |

(continued)

| | | |
|---|---|---|
| **Compound 14** | 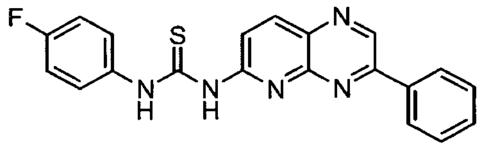 | 1-(4-Fluoro-phenyl)-3-(3-phenyl-pyrido[2,3-b]pyrazin-6-yl)-thiourea |
| **Compound 15** | 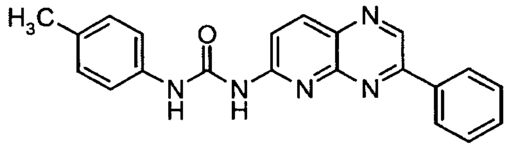 | 1-(3-Phenyl-pyrido[2,3-b]pyrazin-6-yl)-3-p-tolyl-urea |
| **Compound 16** | 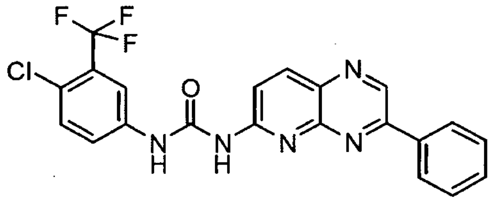 | 1-(4-Chloro-3-trifluoromethyl-phenyl)-3-(3-phenyl-pyrido[2,3-b]pyrazin-6-yl)-urea |
| **Compound 17** | 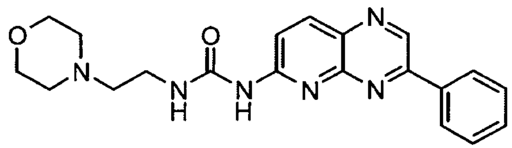 | 1-(2-Morpholin-4-yl-ethyl)-3-(3-phenyl-pyrido[2,3-b]pyrazin-6-yl)-urea |
| **Compound 18** | 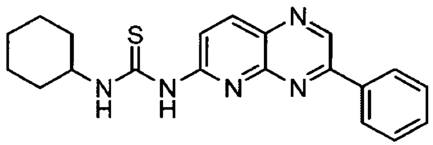 | 1-Cyclohexyl-3-(3-phenyl-pyrido[2,3-b]pyrazin-6-yl)-thiourea |
| **Compound 19** | 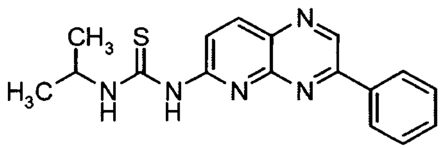 | 1-Isopropyl-3-(3-phenyl-pyrido[2,3-b]pyrazin-6-yl)-thiourea |

(continued)

| | | |
|---|---|---|
| **Compound 20** | 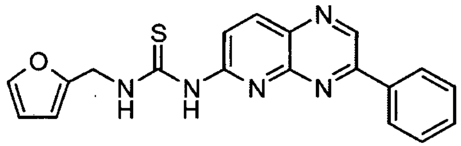 | 1-Furan-2-ylmethyl-3-(3-phenyl-pyrido[2,3-b]pyrazin-6-yl)-thiourea |
| **Compound 21** | 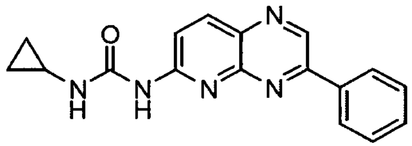 | 1-Cyclopropyl-3-(3-phenyl-pyrido[2,3-b]pyrazin-6-yl)-urea |
| **Compound 22** | 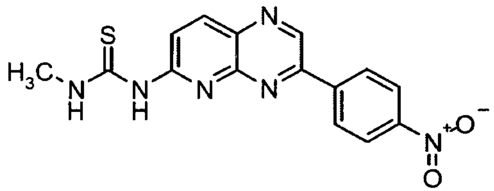 | 1-Methyl-3-[3-(4-nitro-phenyl)-pyrido[2,3-b]pyrazin-6-yl]-thiourea |
| **Compound 23** | 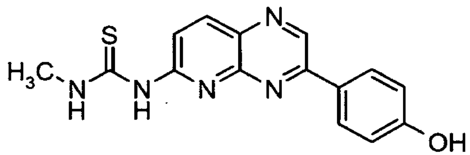 | 1-[3-(4-Hydroxy-phenyl)-pyrido[2,3-b]pyrazin-6-yl]-3-methyl-thiourea |
| **Compound 24** | 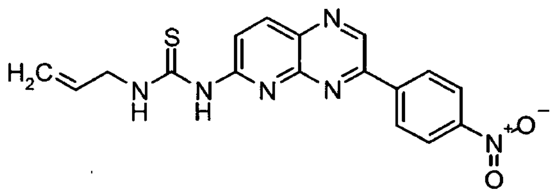 | 1-Allyl-3-[3-(4-nitro-phenyl)-pyrido[2,3-b]pyrazin-6-yl]-thiourea |
| **Compound 26** | 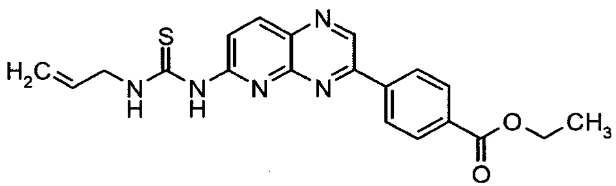 | 4-[6-(3-Allyl-thioureido)-pyrido[2,3-b]pyrazin-3-yl]-benzoic acid-ethyl-ester |

(continued)

| | | |
|---|---|---|
| **Compound 27** | 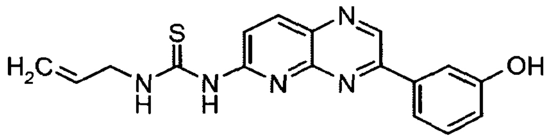 | 1-Allyl-3-[3-(3-hydroxyphenyl)-pyrido[2,3-b]pyrazin-6-yl]-thiourea |
| **Compound 28** | 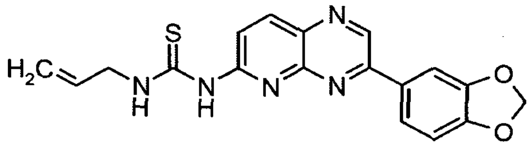 | 1-Alkyl-3-(3-benzo[1,3]dioxol-5-yl-pyrido[2,3-b]pyrazin-6-yl)-thiourea |
| **Compound 29** | 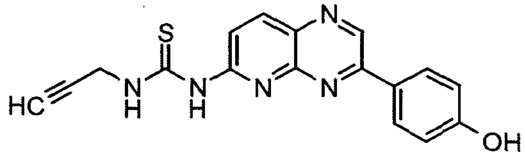 | 1-[3-(4-Hydroxy-phenyl)-pyrido[2,3-b]pyrazin-6-yl]-3-prop-2-inyl-thiourea |
| **Compound 31** | 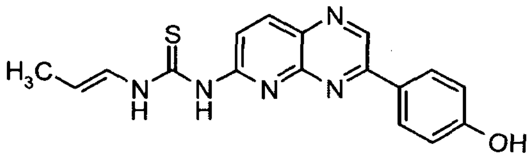 | 1-[3-(4-Hydroxy-phenyl)-pyrido[2,3-b]pyrazin-6-yl]-3-((E)-propenyl)-thiourea |
| **Compound 32** | 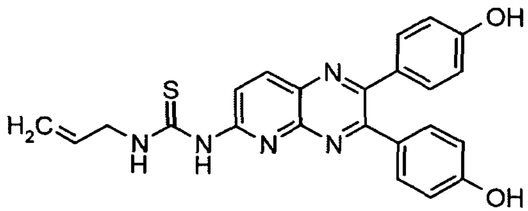 | 1-Allyl-3-[2,3-bis-(4-hydroxy-phenyl)-pyrido[2,3-b]pyrazin-6-yl]-thiourea |
| **Compound 33** | 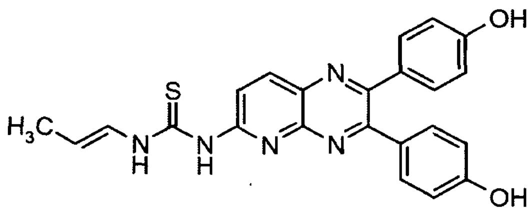 | 1-[2,3-Bis-(4-hydroxy-phenyl)-pyrido[2,3-b]pyrazin-6-yl]-3-((E)-propenyl)-thiourea |

(continued)

| | | |
|---|---|---|
| **Compound 34** | 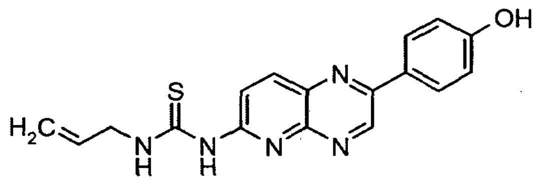 | 1-Allyl-3-[2-(4-hydroxy-phenyl)-pyrido[2,3-b]pyrazin-6-yl]-thiourea |
| **Compound 35** | 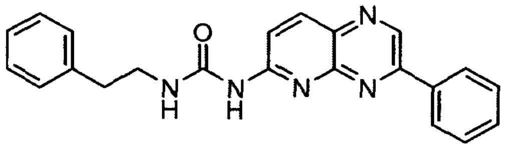 | 1-Phenethyl-3-(3-phenyl-pyrido[2,3-b]pyrazin-6-yl)-urea |
| **Compound 36** | 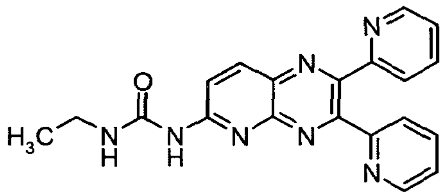 | 1-(2,3-Di-pyridin-2-yl-pyrido[2,3-b]pyrazin-6-yl)-3-ethyl-urea |
| **Compound 37** | 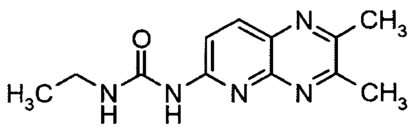 | 1-(2,3-Dimethyl-pyrido[2,3-b]pyrazin-6-yl)-3-ethyl-urea |
| **Compound 38** | 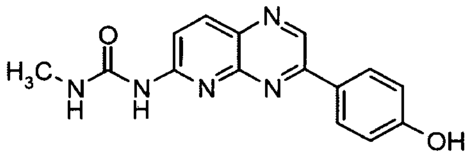 | 1-[3-(4-Hydroxy-phenyl)-pyrido[2,3-b]pyrazin-6-yl]-3-methyl-urea |
| **Compound 39** | 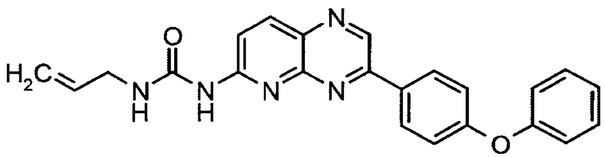 | 1-Allyl-3-[3-(4-phenoxy-phenyl)-pyrido[2,3-b]pyrazin-6-yl]-urea |

(continued)

| | | |
|---|---|---|
| **Compound 40** | 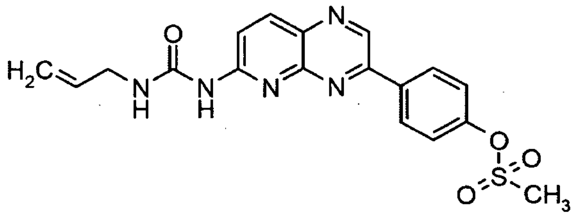 | Methane sulfonic acid-4-[6-(3-allyl-ureido)-pyrido[2,3-b]pyrazin-3-yl]-phenyl-ester |
| **Compound 41** | 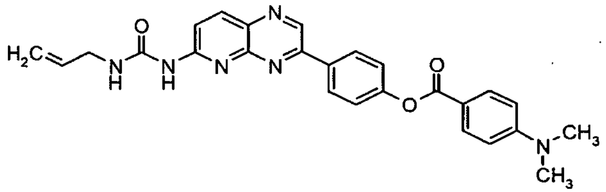 | 4-Dimethylamino-benzoic acid-4-[6-(3-allyl-ureido)-pyrido[2,3-b]pyrazin-3-yl]-phenyl-ester |
| **Compound 42** | 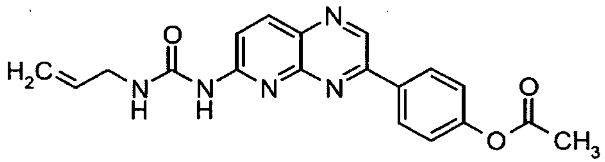 | Acetic acid-4-[6-(3-allyl-ureido)-pyrido[2,3-b]pyrazin-3-yl]-phenyl-ester |
| **Compound 43** | 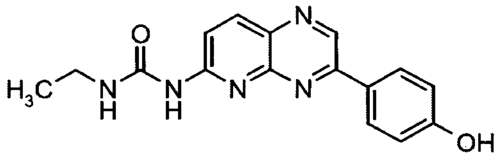 | 1-Ethyl-3-[3-(4-hydroxyphenyl)-pyrido[2,3-b]pyrazin-6-yl]-urea |
| **Compound 44** | 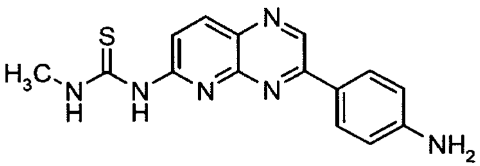 | 1-[3-(4-Amino-phenyl)-pyrido[2,3-b]pyrazin-6-yl]-3-methyl-thiourea |
| **Compound 45** | 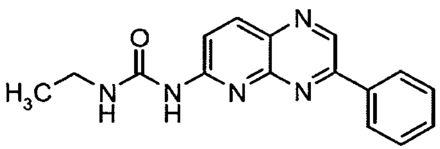 | 1-Ethyl-3-(3-phenyl-pyrido[2,3-b]pyrazin-6-yl)-urea |

(continued)

| | | |
|---|---|---|
| **Compound 46** | 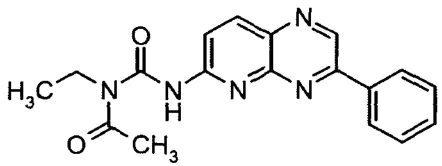 | 1-Acetyl-1-ethyl-3-(3-phenyl-pyrido[2,3-b]pyrazin-6-yl)-urea |
| **Compound 47** | 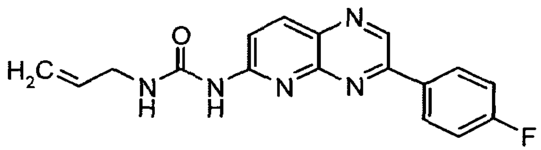 | 1-Allyl-3-[3-(4-fluoro-phenyl)-pyrido[2,3-b]pyrazin-6-yl]-urea |
| **Compound 48** | 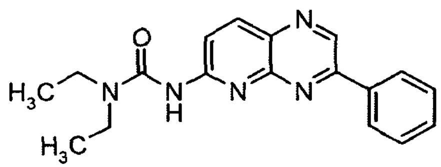 | 1,1-Diethyl-3-(3-phenyl-pyrido[2,3-b]pyrazin-6-yl)-urea |
| **Compound 49** | 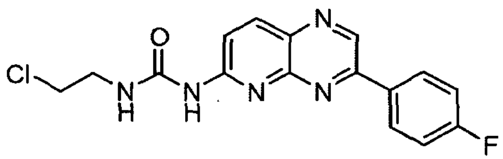 | 1-(2-Chloro-ethyl)-3-[3-(4-fluoro-phenyl)-pyrido[2,3-b]pyrazin-6-yl]-urea |
| **Compound 50** | 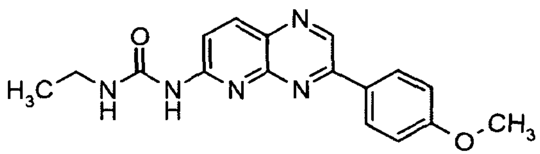 | 1-Ethyl-3-[3-(4-methoxy-phenyl)-pyrido[2,3-b]pyrazin-6-yl]-urea |
| **Compound 51** | 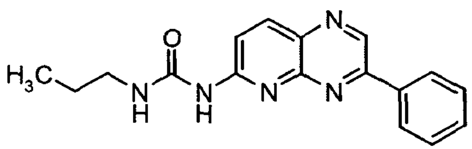 | 1-(3-Phenyl-pyrido[2,3-b]pyrazin-6-yl)-3-propyl-urea |

(continued)

| | | |
|---|---|---|
| **Compound 52** | 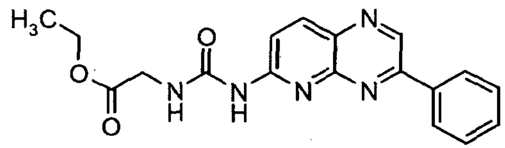 | [3-(3-Phenyl-pyrido[2,3-b]pyrazin-6-yl)-ureido]-acetic acid-ethyl-ester |
| **Compound 53** | 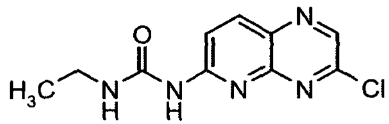 | 1-(3-Chloro-pyrido[2,3-b]pyrazin-6-yl)-3-ethyl-urea |
| **Compound 54** | 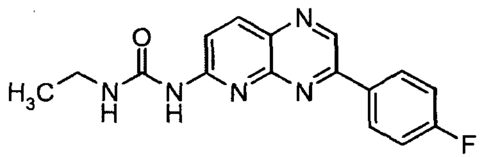 | 1-Ethyl-3-[3-(4-fluorophenyl)-pyrido[2,3-b]pyrazin-6-yl]-urea |
| **Compound 55** | 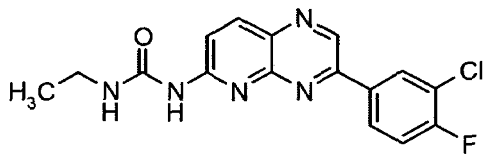 | 1-[3-(3-Chloro-4-fluorophenyl)-pyrido[2,3-b]pyrazin-6-yl]-3-ethyl-urea |
| **Compound 56** | 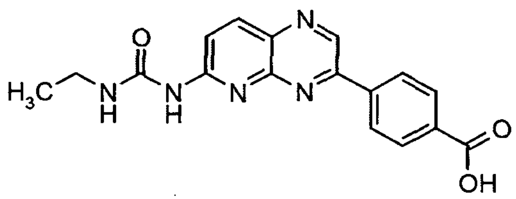 | 4-[6-(3-Ethyl-ureido)-pyrido[2,3-b]pyrazin-3-yl]-benzoic acid |
| **Compound 57** | 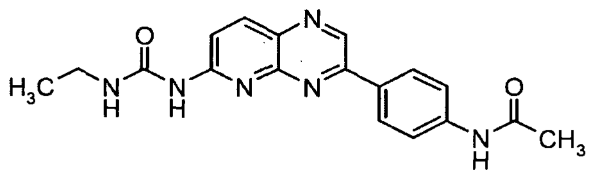 | N-{4-[6-(3-Ethyl-ureido)-pyrido[2,3-b]pyrazin-3-yl]-phenyl}-acetamide |

(continued)

| | | |
|---|---|---|
| **Compound 58** | 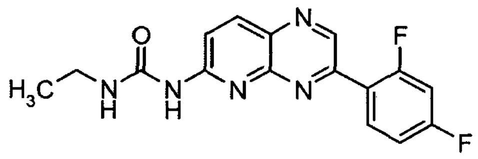 | 1-[3-(2,4-Difluoro-phenyl)-pyrido[2,3-b]pyrazin-6-yl]-3-ethyl-urea |
| **Compound 59** | 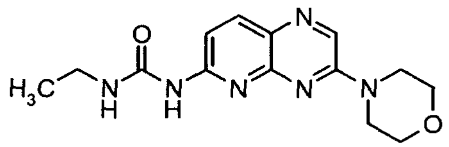 | 1-Ethyl-3-(3-morpholin-4-yl-pyrido[2,3-b]pyrazin-6-yl)-urea |
| **Compound 60** | 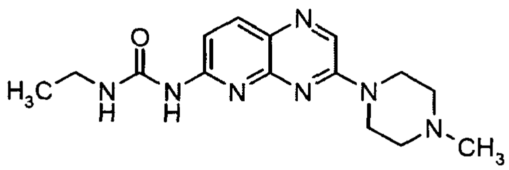 | 1-Ethyl-3-[3-(4-methyl-piperazin-1-yl)-pyrido[2,3-b]pyrazin-6-yl]-urea |
| **Compound 61** | 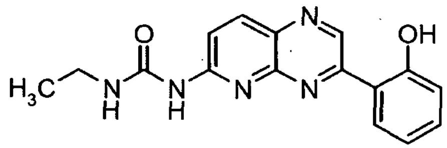 | 1-Ethyl-3-[3-(2-hydroxy-phenyl)-pyrido[2,3-b]pyrazin-6-yl]-urea |
| **Compound 62** | 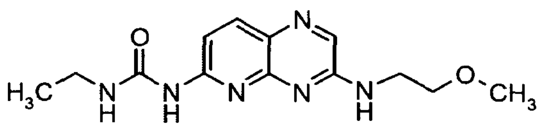 | 1-Ethyl-3-[3-(2-methoxyethylamino)-pyrido[2,3-b]pyrazin-6-yl]-urea |
| **Compound 63** | 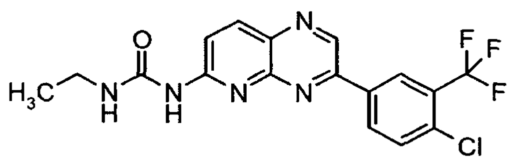 | 1-[3-(4-Chloro-3-trifluoromethyl-phenyl)-pyrido[2,3-b]pyrazin-6-yl]-3-ethyl-urea |
| **Compound 64** | 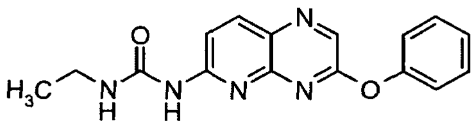 | 1-Ethyl-3-(3-phenoxypyrido[2,3-b]pyrazin-6-yl)-urea |

(continued)

| | | |
|---|---|---|
| **Compound 65** | 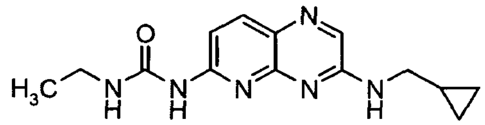 | 1-[3-(Cyclopropylmethyl-amino)-pyrido[2,3-b]pyrazin6-yl]-3-ethyl-urea |
| **Compound 66** | 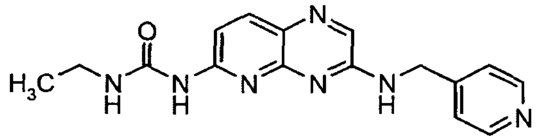 | 1-Ethyl-3-{3-[(pyridin-4-ylmethyl)-amino]-pyrido[2,3-b]pyrazin-6-yl}-urea |
| **Compound 67** | 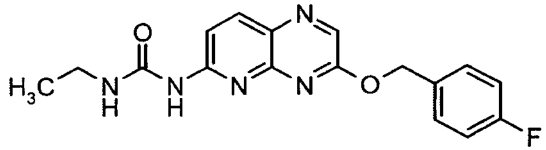 | 1-Ethyl-3-[3-(4-fluoro-benzyloxy)-pyrido[2,3-b]pyrazin-6-yl]-urea |
| **Compound 68** | 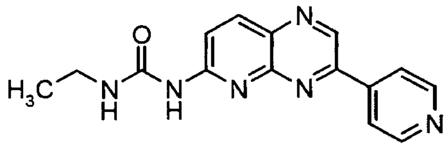 | 1-Ethyl-3-(3-pyridin-4-yl-pyrido[2,3-b]pyrazin-6-yl)-urea |
| **Compound 69** | 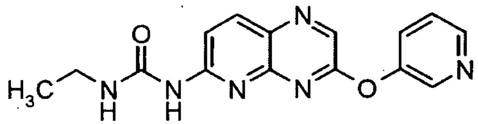 | 1-Ethyl-3-[3-(pyridin-3-yloxy)-pyrido[2,3-b]pyrazin-6-yl]-urea |
| **Compound 70** | 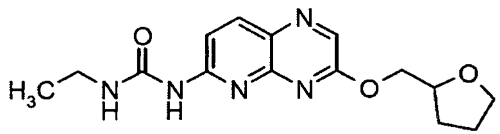 | 1-Ethyl-3-[3-(tetrahydro-furan-2-ylmethoxy)-pyrido[2,3-b]pyrazin-6-yl]-urea |
| **Compound 71** | 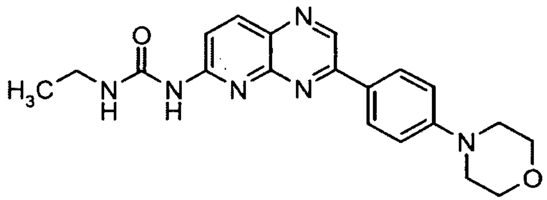 | 1-Ethyl-3-[3-(4-morpholin-4-yl-phenyl)-pyrido[2,3-b]pyrazin-6-yl]-urea |

(continued)

| | | |
|---|---|---|
| **Compound 72** | 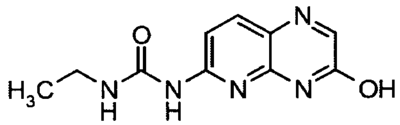 | 1-Ethyl-3-(3-hydroxypyrido[2,3-b]pyrazin-6-yl)-urea |
| **Compound 73** | 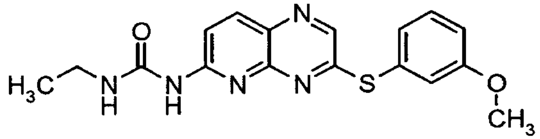 | 1-Ethyl-3-[3-(3-methoxy-phenylsulfanyl)-pyrido[2,3-b]pyrazin-6-yl]-urea |
| **Compound 74** | 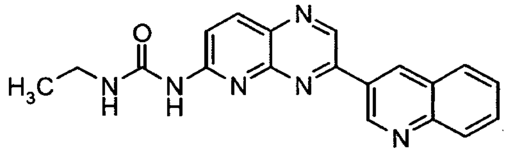 | 1-Ethyl-3-(3-quinolin-3-yl-pyrido[2,3-b]pyrazin-6-yl)-urea |
| **Compound 75** | 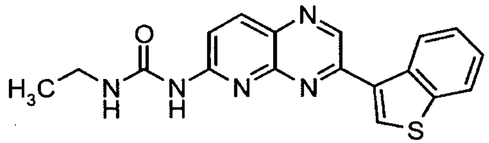 | 1-(3-Benzo[b]thiophen-3-yl-pyrido[2,3-b]pyrazin-6-yl)-3-ethyl-urea |
| **Compound 76** | 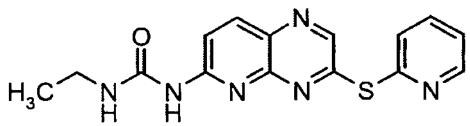 | 1-Ethyl-3-[3-(pyridin-2-ylsulfanyl)-pyrido[2,3-b]pyrazin-6-yl]-urea |
| **Compound 77** | 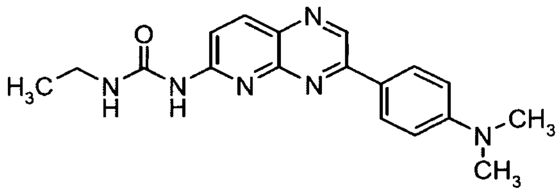 | 1-[3-(4-Dimethylaminophenyl)-pyrido[2,3-b]pyrazin-6-yl]-3-ethyl-urea |

(continued)

| | | |
|---|---|---|
| **Compound 78** | 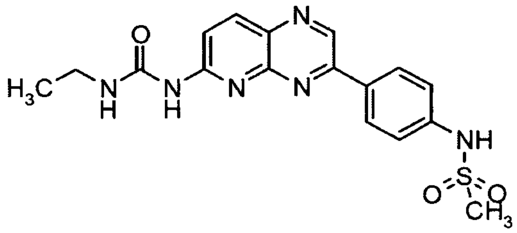 | N-{4-[6-(3-Ethyl-ureido)-pyrido[2,3-b]pyrazin-3-yl]-phenyl}-methansulfonamide |
| **Compound 79** | 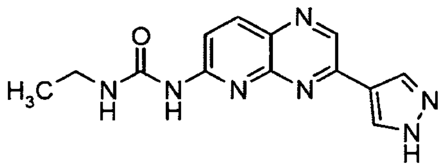 | 1-Ethyl-3-[3-(1H-pyrazol-4-yl)-pyrido[2,3-b]pyrazin-6-yl]-urea |
| **Compound 80** | 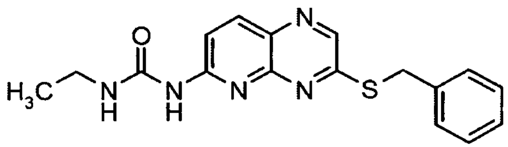 | 1-(3-Benzylsulfanyl-pyrido[2,3-b]pyrazin-6-yl)-3-ethyl-urea |
| **Compound 81** | 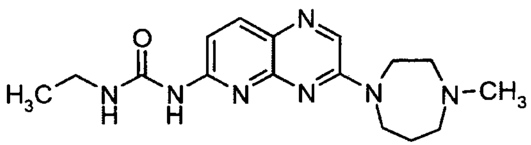 | 1-Ethyl-3-[3-(4-methyl-[1,4]diazepan-1-yl)-pyrido[2,3-b]pyrazin-6-yl]-urea |
| **Compound 82** | 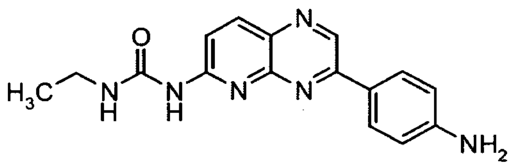 | 1-[3-(4-Amino-phenyl)-pyrido[2,3-b]pyrazin-6-yl]-3-ethyl-urea |
| **Compound 83** | 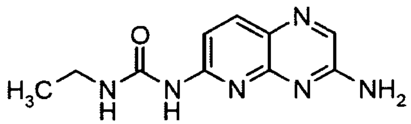 | 1-(3-Amino-pyrido[2,3-b]pyrazin-6-yl)-3-ethyl-urea |

(continued)

| | | |
|---|---|---|
| **Compound 84** | 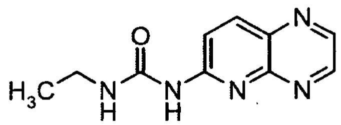 | 1-Ethyl-3-pyrido[2,3-b]pyrazin-6-yl-urea |
| **Compound 85** | 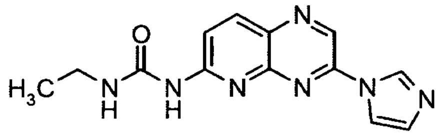 | 1-Ethyl-3-(3-imidazol-1-yl-pyrido[2,3-b]pyrazin-6-yl)-urea |
| **Compound 86** | 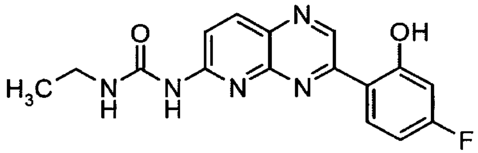 | 1-Ethyl-3-[3-(4-fluoro-2-hydroxy-phenyl)-pyrido[2,3-b]pyrazin-6-yl]-urea |
| **Compound 87** | 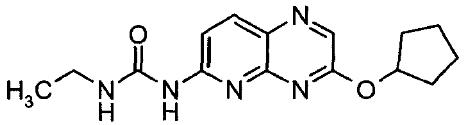 | 1-(3-Cyclopentyloxy-pyrido[2,3-b]pyrazin-6-yl)-3-ethyl-urea |
| **Compound 88** | 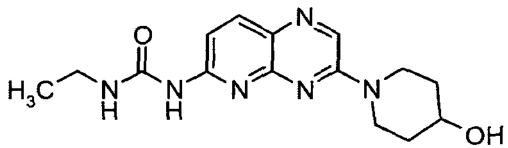 | 1-Ethyl-3-[3-(4-hydroxy-piperidin-1-yl)-pyrido[2,3-b]pyrazin-6-yl]-urea |
| **Compound 89** | 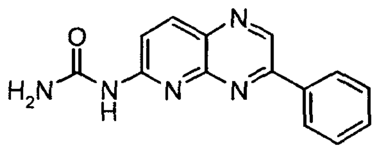 | (3-Phenyl-pyrido[2,3-b]pyrazin-6-yl)-urea |
| **Compound 90** | 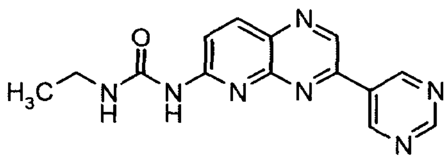 | 1-Ethyl-3-(3-pyrimidin-5-yl-pyrido[2,3-b]pyrazin-6-yl)-urea |

(continued)

| | | |
|---|---|---|
| **Compound 91** | 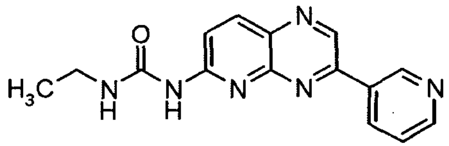 | 1-Ethyl-3-(3-pyridin-3-yl-pyrido[2,3-b]pyrazin-6-yl)-urea |
| **Compound 92** | 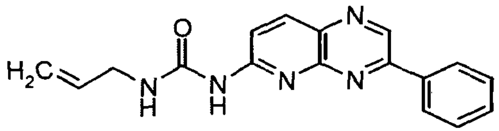 | 1-Allyl-3-(3-phenyl-pyrido[2,3-b]pyrazin-6-yl)-urea |
| **Compound 93** | 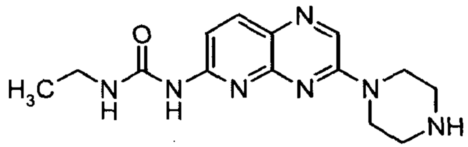 | 1-Ethyl-3-(3-piperazin-1-yl-pyrido[2,3-b]pyrazin-6-yl)-urea |
| **Compound 94** | 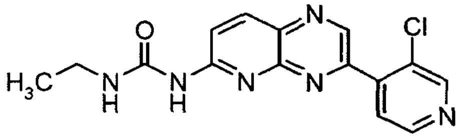 | 1-[3-(3-Chloro-pyridin-4-ylmethyl)-pyrido[2,3-b]pyrazin-6-yl]-3-ethyl-urea |
| **Compound 95** | 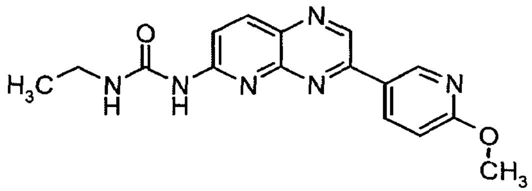 | 1-Ethyl-3-[3-(6-methoxy-pyridin-3-yl)-pyrido[2,3-b]pyrazin-6-yl]-urea |
| **Compound 96** | 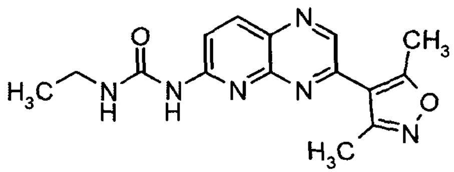 | 1-[3-(3,5-Dimethyl-isoxazol-4-yl)-pyrido[2,3-b]pyrazin-6-yl]-3-ethyl-urea |

(continued)

| | | |
|---|---|---|
| **Compound 97** | 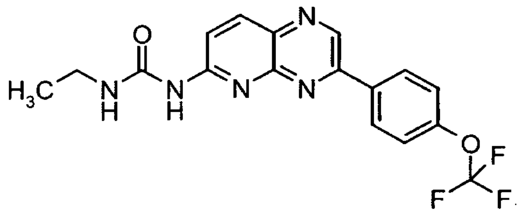 | 1-Ethyl-3-[3-(4-trifluoromethoxy-phenyl)-pyrido[2,3-b]pyrazin-6-yl]-urea |
| **Compound 98** | 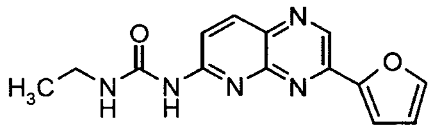 | 1-Ethyl-3-(3-furan-2-yl-pyrido[2,3-b]pyrazin-6-yl)-urea |
| **Compound 99** | 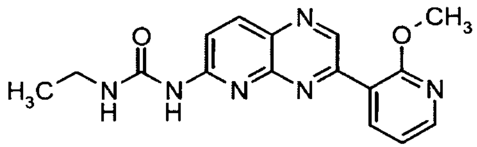 | 1-Ethyl-3-[3-(2-methoxy-pyridin-3-yl)-pyrido[2,3-b]pyrazin-6-yl]-urea |
| **Compound 100** | 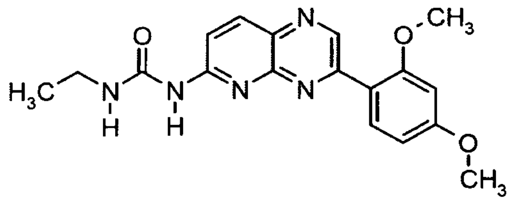 | 1-[3-(2,4-Dimethoxy-phenyl)-pyrido[2,3-b]pyrazin-6-yl]-3-ethyl-urea |
| **Compound 101** | 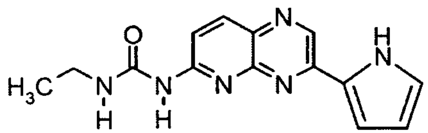 | 1-Ethyl-3-[3-(1H-pyrrol-2-yl)-pyrido[2,3-b]pyrazin-6-yl]-urea |
| **Compound 102** | 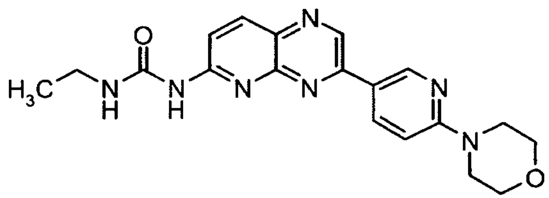 | 1-Ethyl-3-[3-(6-morpholin-4-yl-pyridin-3-yl)-pyrido[2,3-b]pyrazin-6-yl]-urea |

(continued)

| | | |
|---|---|---|
| **Compound 103** | 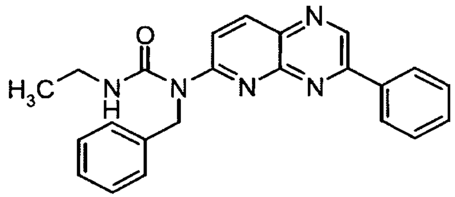 | 1-Benzyl-3-ethyl-1-(3-phenyl-pyrido[2,3-b]pyrazin-6-yl)-urea |
| **Compound 104** | 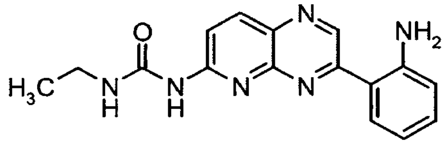 | 1-[3-(2-Amino-phenyl)-pyrido[2,3-b]pyrazin-6-yl]-3-ethyl-urea |
| **Compound 105** | 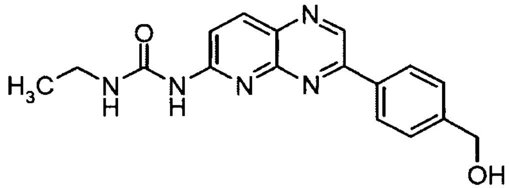 | 1-Ethyl-3-[3-(4-hydroxymethyl-phenyl)-pyrido[2,3-b]pyrazin-6-yl]-urea |
| **Compound 106** | 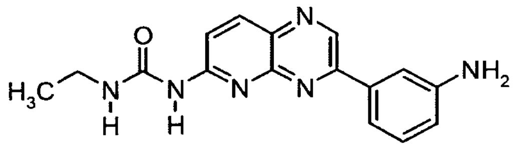 | 1-[3-(3-Amino-phenyl)-pyrido[2,3-b]pyrazin-6-yl]-3-ethyl-urea |
| **Compound 107** | 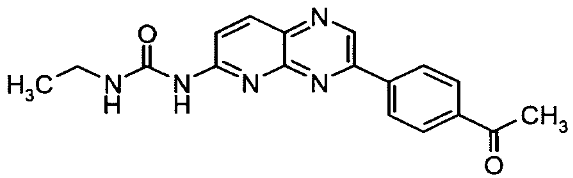 | 1-[3-(4-Acetyl-phenyl)-pyrido[2,3-b]pyrazin-6-yl]-3-ethyl-urea |
| **Compound 108** | 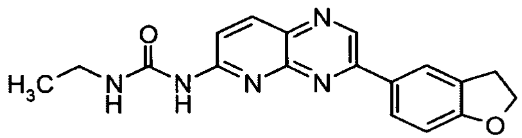 | 1-[3-(2,3-Dihydro-benzofuran-5-yl)-pyrido[2,3-b]pyrazin-6-yl]-3-ethyl-urea |

(continued)

| | | |
|---|---|---|
| **Compound 109** | 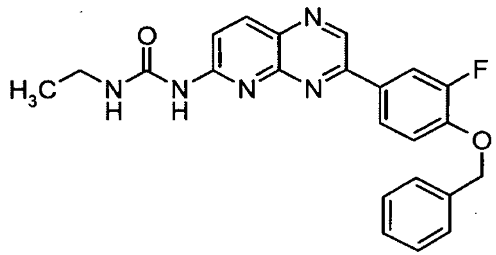 | 1-[3-(4-Benzyloxy-3-fluorophenyl)-pyrido[2,3-b]pyrazin-6-yl]-3-ethyl-urea |
| **Compound 110** | 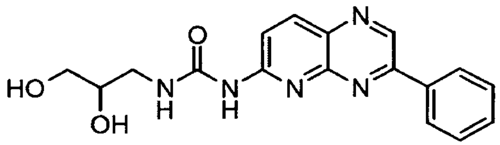 | 1-(2,3-Dihydroxy-propyl)-3-(3-phenyl-pyrido[2,3-b]pyrazin-6-yl)-urea |
| **Compound 111** | 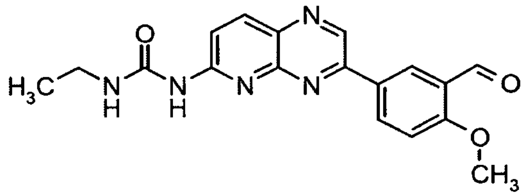 | 1-Ethyl-3-[3-(3-formyl-4-methoxy-phenyl)-pyrido[2,3-b]pyrazin-6-yl]-urea |
| **Compound 112** | 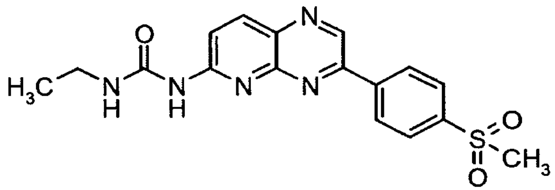 | 1-Ethyl-3-[3-(4-methansulfonyl-phenyl)-pyrido[2,3-b]pyrazin-6-yl]-urea |
| **Compound 113** | 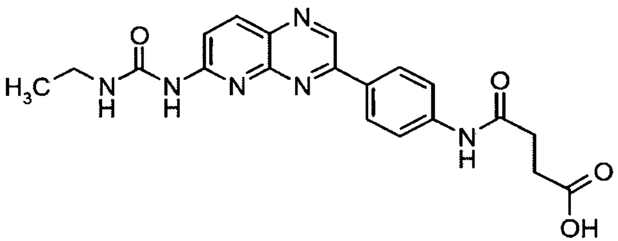 | N-{4-[6-(3-Ethyl-ureido)-pyrido[2,3-b]pyrazin-3-yl]-phenyl}-succinamid acid |

(continued)

| | | |
|---|---|---|
| **Compound 114** | 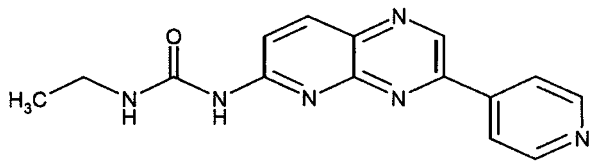 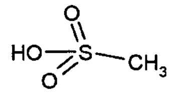 | 1-Ethyl-3-(3-pyridin-4-yl-pyrido[2,3-b]pyrazin-6-yl)-urea Methane sulfonic acid salt (free base) |
| **Compound 115** | 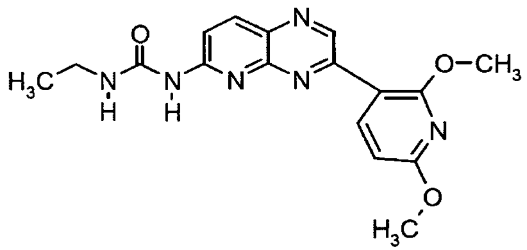 | 1-[3-(2,6-Dimethoxy-pyridin-3-yl)-pyrido[2,3-b]pyrazin-6-yl]-3-ethyl-urea |
| **Compound 116** | 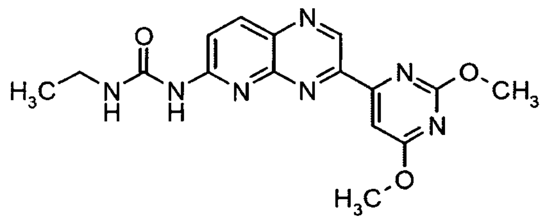 | 1-[3-(2,6-Dimethoxy-pyrimidin-4-yl)-pyrido[2,3-b]pyrazin-6-yl]-3-ethyl-urea |
| **Compound 117** | 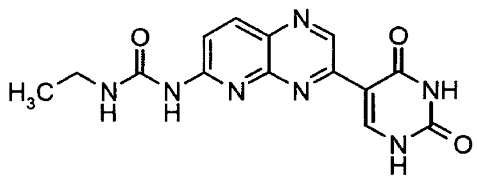 | 1-[3-(2,4-Dioxo-1,2,3,4-tetrahydro-pyrimidin-5-yl)-pyrido[2,3-b]pyrazin-6-yl]-3-ethyl-urea |

(continued)

| | | |
|---|---|---|
| **Compound 118** | 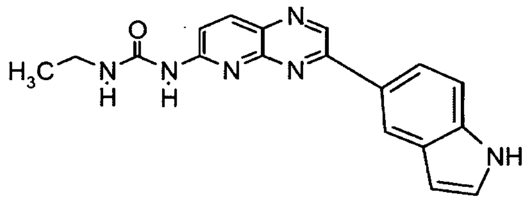 | 1-Ethyl-3-[3-(1H-indol-5-yl)-pyrido[2,3-b]pyrazin-6-yl]-urea |
| **Compound 119** | 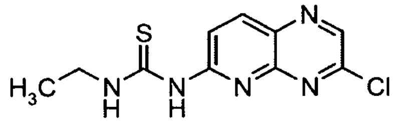 | 1-(3-Chloro-pyrido[2,3-b]pyrazin-6-yl)-3-ethyl-thiourea |
| **Compound 120** | 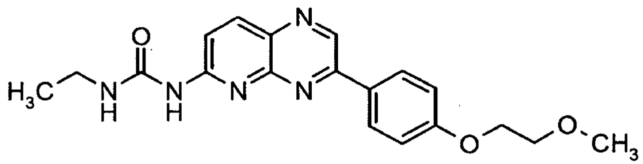 | 1-Ethyl-3-{3-[4-(2-methoxy-ethoxy)-phenyl]-pyrido[2,3-b]pyrazin-6-yl}-urea |
| **Compound 121** | 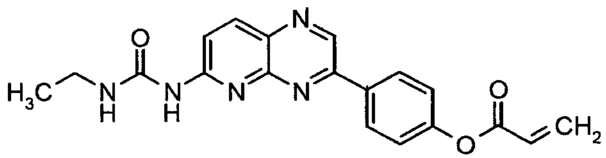 | Acrylic acid-4-[6-(3-ethyl-ureido)-pyrido[2,3-b]-pyrazin-3-yl]-phenyl-ester |
| **Compound 122** | 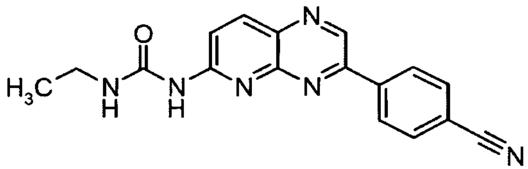 | 1-[3-(4-Cyano-phenyl)-pyrido[2,3-b]pyrazin-6-yl]-3-ethylurea |
| **Compound 123** | 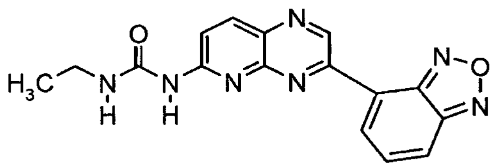 | 1-(3-Benzo[1,2,5]oxadiazol-4-yl-pyrido[2,3-b]pyrazin-6-yl)-3-ethyl-urea |

(continued)

| | | |
|---|---|---|
| **Compound 124** | 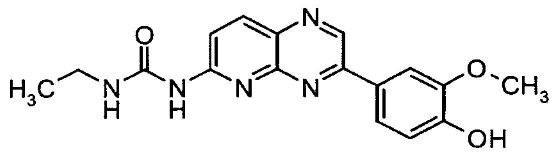 | 1-Ethyl-3-[3-(4-hydroxy-3-methoxy-phenyl)-pyrido[2,3-b]pyrazin-6-yl]-urea |
| **Compound 125** | 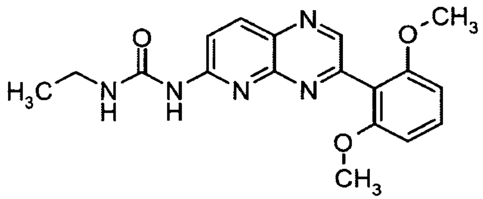 | 1-[3-(2,6-Dimethoxyphenyl)-pyrido [2,3-b]pyrazin-6-yl]-3-ethyl-urea |
| **Compound 126** | 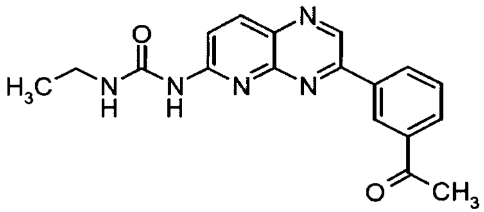 | 1-[3-(3-Acetyl-phenyl)-pyrido[2,3-b]pyrazin-6-yl]-3-ethyl-urea |
| **Compound 127** | 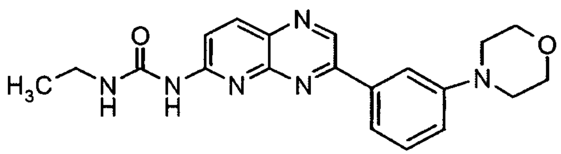 | 1-Ethyl-3-[3-(3-morpholin-4-yl-phenyl)-pyrido[2,3-b]pyrazin-6-yl]-urea |
| **Compound 128** | 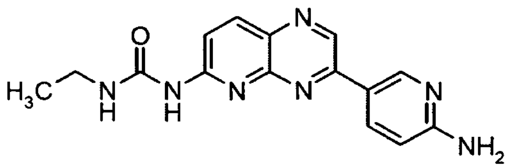 | 1-[3-(6-Amino-pyridin-3-yl)-pyrido[2,3-b]pyrazin-6-yl]-3-ethyl-urea |
| **Compound 129** | 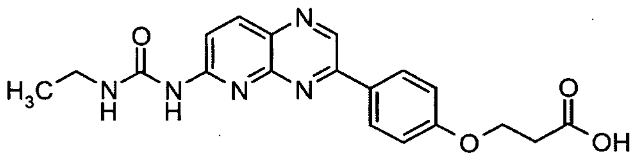 | 3-{4-[6-(3-Ethyl-ureido)-pyrido[2,3-b]-pyrazin-3-yl]-phenoxy}-propionic acid |

(continued)

| | | |
|---|---|---|
| **Compound 130** | 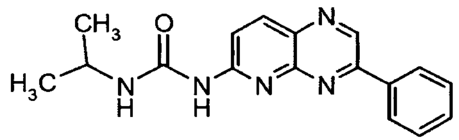 | 1-Isopropyl-3-(3-phenyl-pyrido[2,3-b]pyrazin-6-yl)-urea |
| **Compound 131** | 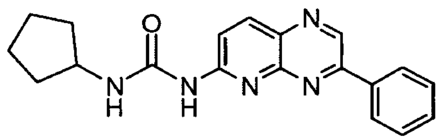 | 1-Cyclopentyl-3-(3-phenyl-pyrido[2,3-b]pyrazin-6-yl)-urea |
| **Compound 132** | 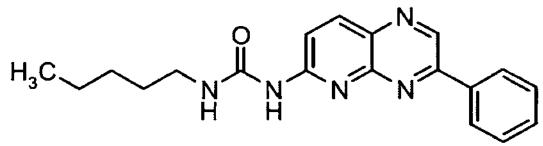 | 1-Pentyl-3-(3-phenyl-pyrido[2,3-b]pyrazin-6-yl)-urea |
| **Compound 133** | 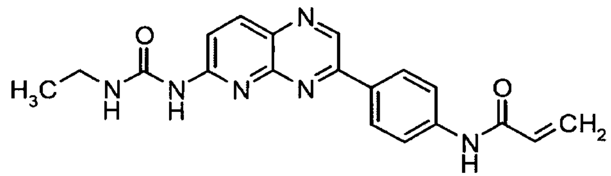 | N-{4-[6-(3-Ethyl-ureido)-pyrido[2,3-b]pyrazin-3-yl]-phenyl}-acrylamide |
| **Compound 134** | 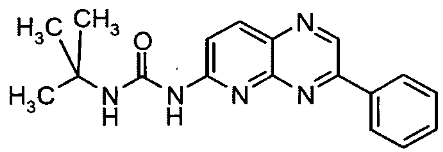 | 1-tert-Butyl-3-(3-phenyl-pyrido[2,3-b]pyrazin-6-yl)-urea |
| **Compound 135** | 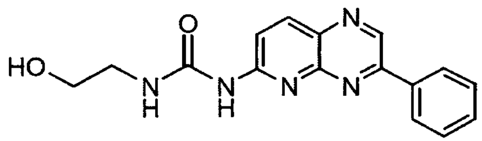 | 1-(2-Hydroxy-ethyl)-3-(3-phenyl-pyrido[2,3-b]pyrazin-6-yl)-urea |
| **Compound 136** | 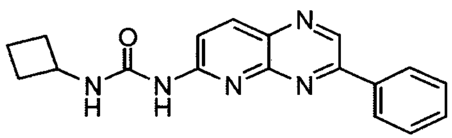 | 1-Cyclobutyl-3-(3-phenyl-pyrido[2,3-b]pyrazin-6-yl)-urea |

(continued)

| | | |
|---|---|---|
| **Compound 137** | 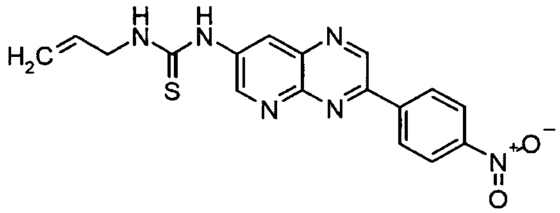 | 1-Allyl-3-[3-(4-nitro-phenyl)-pyrido[2,3-b]pyrazin-7-yl]-thiourea |
| **Compound 138** | 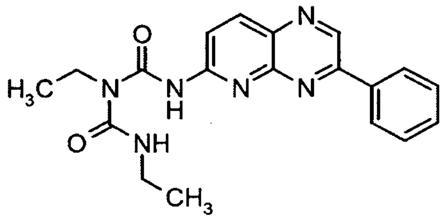 | 1-Ethyl-1-(Ethylcarbamoyl)-3-(3-phenyl-pyrido[2,3-b]pyrazin-6-yl)-urea |
| **Compound 139** | 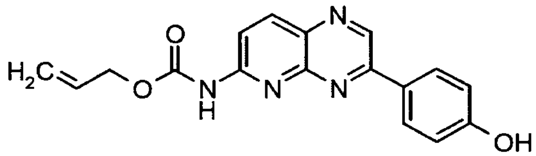 | [3-(4-Hydroxy-phenyl)-pyrido[2,3-b]pyrazin-6-yl]-carbamic acid-allyl-ester |
| **Compound 140** | 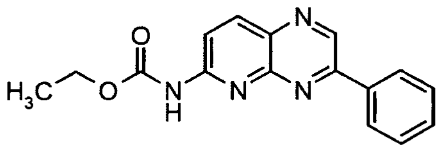 | (3-Phenyl-pyrido[2,3-b]pyrazin-6-yl)-carbamic acid-ethyl-ester |
| **Compound 141** | 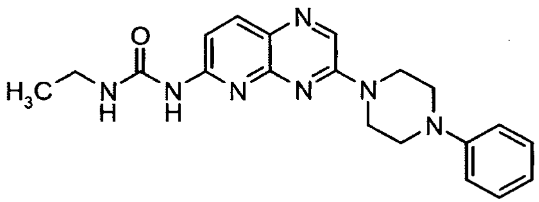 | 1-Ethyl-3-[3-(4-phenyl-piperazin-1-yl)-pyrido[2,3-b]pyrazin-6-yl]-urea |

(continued)

| | | |
|---|---|---|
| **Compound 142** | 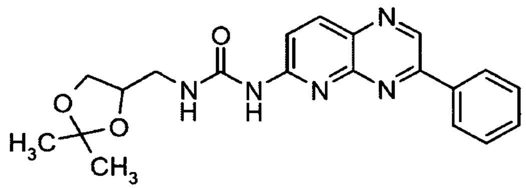 | 1-(2,2-Dimethyl-[1,3]dioxolan-4-ylmethyl)-3-(3-phenyl-pyrido[2,3-b]pyrazin-6-yl)-urea |
| **Compound 143** | 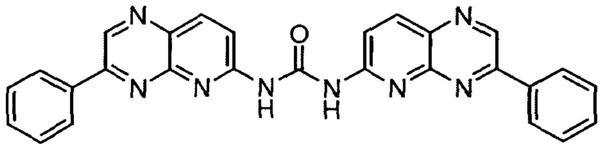 | 1,3-Bis-(3-phenyl-pyrido[2,3-b]pyrazin-6-yl)-urea |
| **Compound 144** | 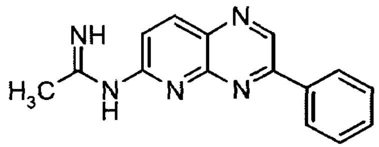 | N-(3-Phenyl-pyrido[2,3-b]pyrazin-6-yl)-acetamidine |
| **Compound 145** | 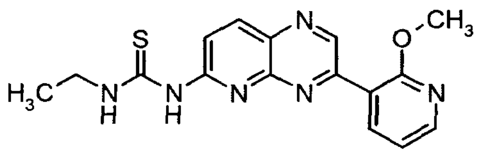 | 1-Ethyl-3-[3-(2-methoxy-pyridin-3-yl)-pyrido[2,3-b]pyrazin-6-yl]-thiourea |
| **Compound 146** | 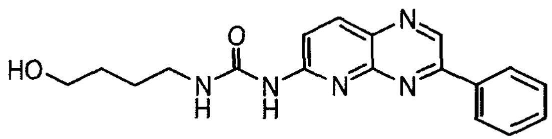 | 1-(4-Hydroxy-butyl)-3-(3-phenyl-pyrido[2,3-b]pyrazin-6-yl)-urea |
| **Compound 147** | 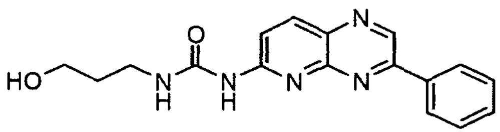 | 1-(3-Hydroxy-propyl)-3-(3-phenyl-pyrido[2,3-b]pyrazin-6-yl)-urea |

(continued)

| | | |
|---|---|---|
| **Compound 148** | 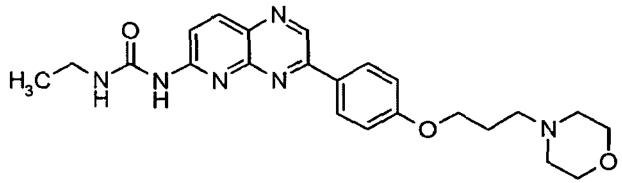 | 1-Ethyl-3-{3-[4-(3-morpholin-4-yl-propoxy)-phenyl]-pyrido[2,3-b]pyrazin-6-yl}-urea |
| **Compound 149** | 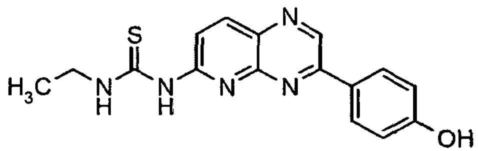 | 1-Ethyl-3-[3-(4-hydroxy-phenyl)-pyrido[2,3-b]pyrazin-6-yl]-thiourea |
| **Compound 150** | 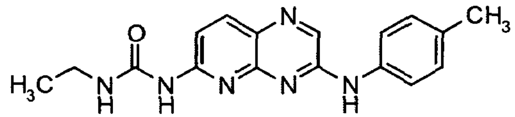 | 1-Ethyl-3-(3-p-tolylamino-pyrido[2,3-b]pyrazin-6-yl)-urea |
| **Compound 151** | 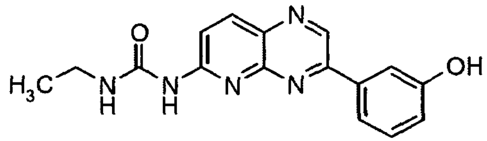 | 1-Ethyl-3-[3-(3-hydroxy-phenyl)-pyrido[2,3-b]pyrazin-6-yl]-urea |
| **Compound 152** | 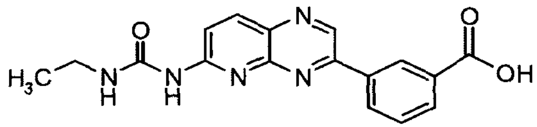 | 3-[6-(3-Ethyl-ureido)-pyrido[2,3-b]pyrazin-3-yl]-benzoic acid |
| **Compound 153** | 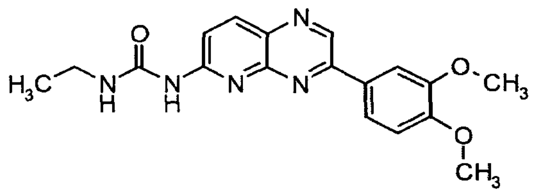 | 1-[3-(3,4-Dimethoxyphenyl)-pyrido[2,3-b]pyrazin-6-yl]-3-ethyl-urea |

(continued)

| | | |
|---|---|---|
| **Compound 154** | 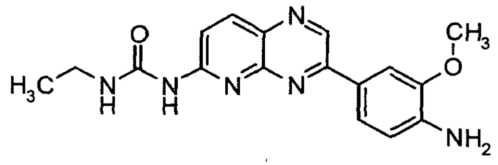 | 1-[3-(4-Amino-3-methoxy-phenyl)-pyrido[2,3-b]pyrazin-6-yl]-3-ethyl-urea |
| **Compound 155** | 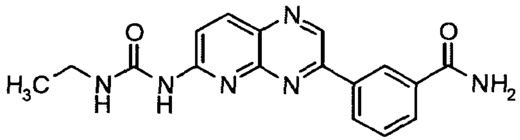 | 3-[6-(3-Ethyl-ureido)-pyrido[2,3-b]-pyrazin-3-yl]-benzamide |
| **Compound 156** | 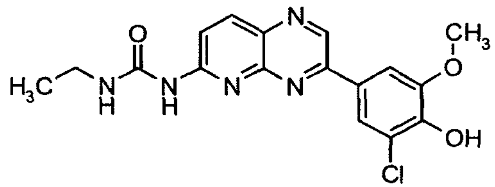 | 1-[3-(3-Chloro-4-hydroxy-5-methoxy-phenyl)-pyrido[2,3-b]pyrazin-6-yl]-3-ethyl-urea |
| **Compound 157** | 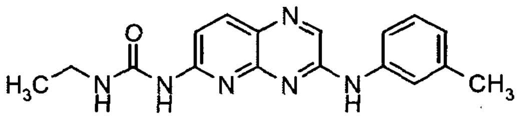 | 1-Ethyl-3-(3-m-tolylamino-pyrido-[2,3-b]pyrazin-6-yl)-urea |
| **Compound 158** | 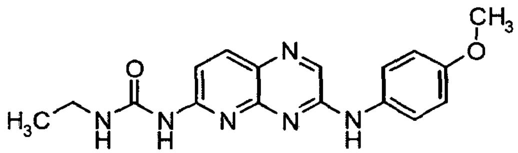 | 1-Ethyl-3-[3-(4-methoxy-phenylamino)-pyrido[2,3-b]pyrazin-6-yl]-urea |
| **Compound 159** | 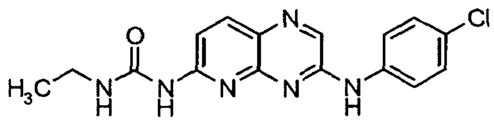 | 1-[3-(4-Chloro-phenylamino)-pyrido-[2,3-b]pyrazin-6-yl]-3-ethyl-urea |
| **Compound 160** | 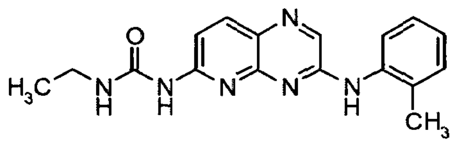 | 1-Ethyl-3-(3-o-tolylamino-pyrido[2,3-b]-pyrazin-6-yl)-urea |

(continued)

| | | |
|---|---|---|
| **Compound 161** | 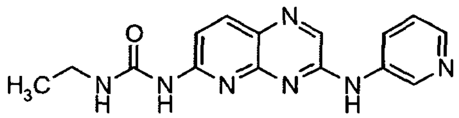 | 1-Ethyl-3-[3-(pyridin-3-ylamino)-pyrido-[2,3-b]pyrazin-6-yl]-urea |
| **Compound 162** | 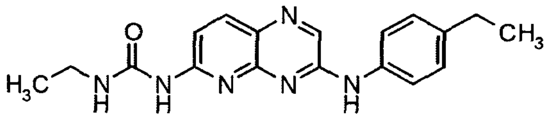 | Ethyl-3-[3-(4-ethyl-phenylamino)-pyrido[2,3-b]pyrazin-6-yl]-urea |
| **Compound 163** | 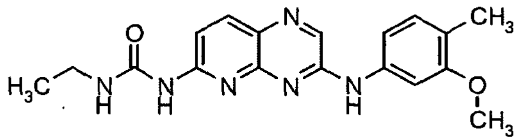 | 1-Ethyl-3-[3-(3-methoxy-4-methyl-phenylamino)-pyrido[2,3-b]pyrazin-6-yl]-urea |
| **Compound 164** | 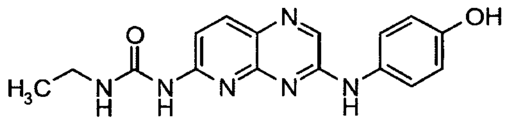 | 1-Ethyl-3-[3-(4-hyd roxy-phenylamino)-pyrido[2,3-b]pyrazin-6-yl]-urea |
| **Compound 165** | 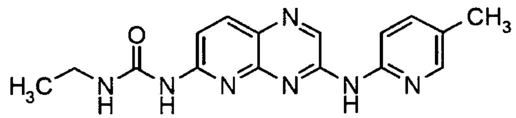 | 1-Ethyl-3-[3-(5-methyl-pyridin-2-yl-amino)-pyrido[2,3-b]pyrazin-6-yl]-urea |
| **Compound 166** | 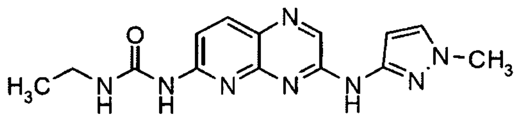 | 1-Ethyl-3-[3-(1-methyl-1H-pyrazol-3-ylamino)-pyrido[2,3-b]pyrazin-6-yl]-urea |
| **Compound 167** | 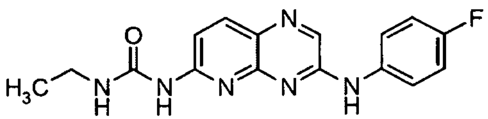 | 1-Ethyl-3-[3-(4-fl uoro-phenylamino)-pyrido[2,3-b]pyrazin-6-yl]-urea |
| **Compound 168** | 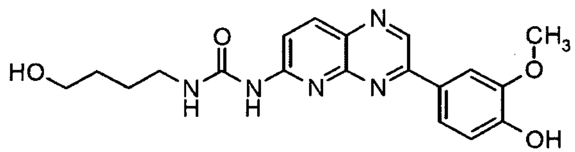 | 1-(4-Hydroxy-butyl)-3-[3-(4-hydroxy-3-methoxy-phenyl)-pyrido[2,3-b]pyrazin-6-yl]-urea |

(continued)

| | | |
|---|---|---|
| **Compound 169** | 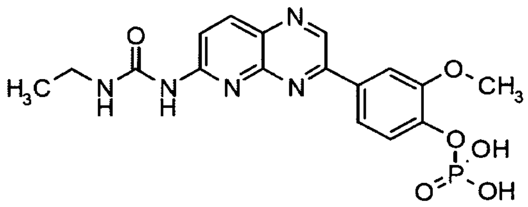 | Phosphoric acid-mono-{4-[6-(3-ethyl-ureido)-pyrido-[2,3-b]pyrazin-3-yl]-2-methoxy-phenyl}-ester |
| **Compound 170** | 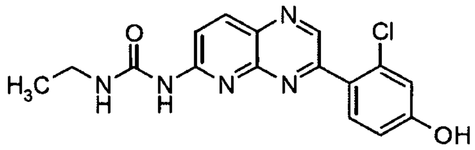 | 1-[3-(2-Chloro-4-hydroxy-phenyl)-pyrido[2,3-b]pyrazin-6-yl]-3-ethyl-urea |
| **Compound 171** | 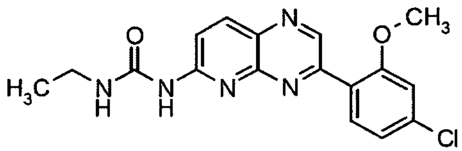 | 1-[3-(4-Chloro-2-methoxyphenyl)-pyrido[2,3-b]pyrazin-6-yl]-3-ethyl-urea |
| **Compound 172** | 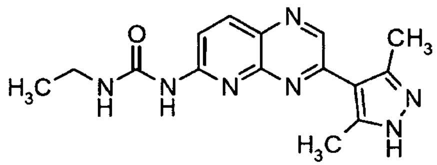 | 1-[3-(3,5-Dimethyl-1 H-pyrazol-4-yl)-pyrido[2,3-b]pyrazin-6-yl]-3-ethyl-urea |
| **Compound 173** | 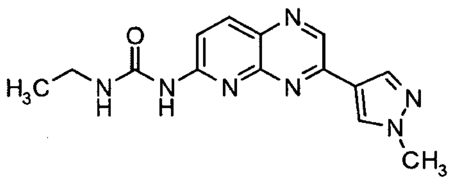 | 1-Ethyl-3-[3-(1-methyl-1H-pyrazol-4-yl)-pyrido[2,3-b]pyrazin-6-yl]-urea |
| **Compound 174** | 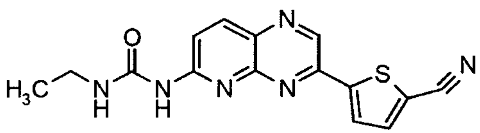 | 1-[3-(5-Cyano-thiophen-2-yl)-pyrido[2,3-b]pyrazin-6-yl]-3-ethyl-urea |

(continued)

| | | |
|---|---|---|
| **Compound 175** | 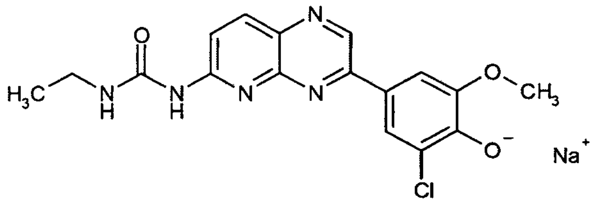 | Sodium 2-chloro-4-[6-(3-ethyl-ureido)-pyrido[2,3-b]pyrazin-3-yl]-6-methoxy-phenolate |
| **Compound 176** | 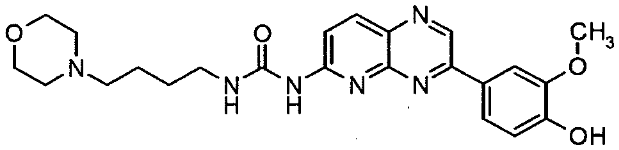 | 1-[3-(4-Hyd roxy-3-methoxyphenyl)-pyrido[2,3-b]pyrazin-6-yl]-3-(4-morpholin-4-yl-butyl)-urea |
| **Compound 177** | 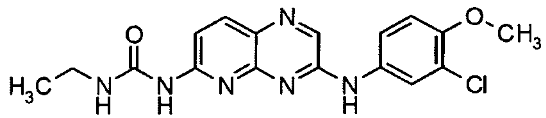 | 1-[3-(3-Chloro-4-methoxy-phenylamino)-pyrido[2,3-b]pyrazin-6-yl]-3-ethyl-urea |
| **Compound 178** | 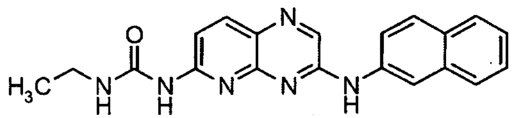 | 1-Ethyl-3-[3-(naphthalin-2-ylamino)-pyrido[2,3-b]pyrazin-6-yl]-urea |
| **Compound 179** | 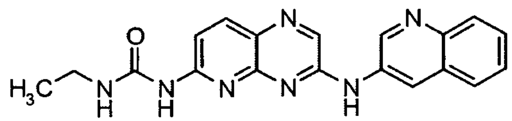 | 1-Ethyl-3-[3-(quinolin-3-ylamino)-pyrido[2,3-b]pyrazin-6-yl]-urea |
| **Compound 180** | 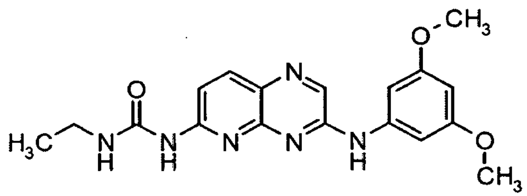 | 1-[3-(3,5-Dimethoxy-phenylamino)-pyrido[2,3-b]pyrazin-6-yl]-3-ethyl-urea |
| **Compound 181** | 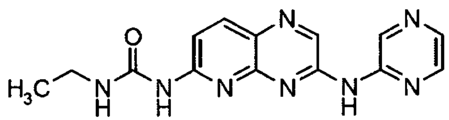 | 1-Ethyl-3-[3-(pyrazin-2-ylamino)-pyrido[2,3-b]pyrazin-6-yl]-urea |

(continued)

| | | |
|---|---|---|
| **Compound 182** | 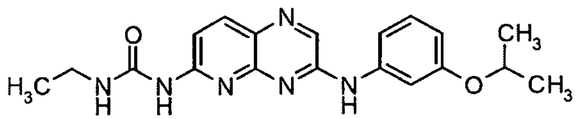 | 1-Ethyl-3-[3-(3-isopropoxy-phenylamino)-pyrido[2,3-b]pyrazin-6-yl]-urea |
| **Compound 183** | 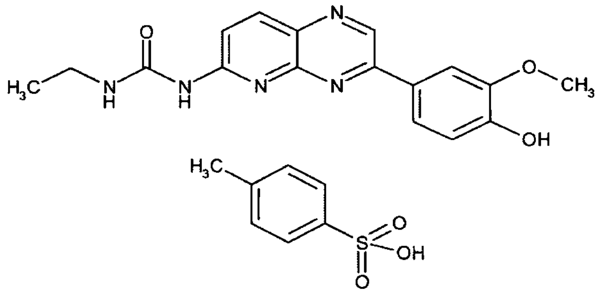 | 1-Ethyl-3-[3-(4-hydroxy-3-methoxy-phenyl)-pyrido[2,3-b]pyrazin-6-yl]-urea p-Toluolsulfonat |
| **Compound 184** | 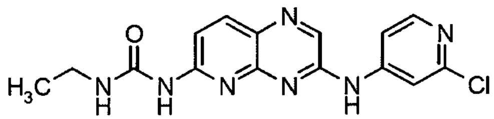 | 1-[3-(2-Chloro-pyridin-4-ylamino)-pyrido[2,3-b]pyrazin-6-yl]-3-ethyl-urea |
| **Compound 185** | 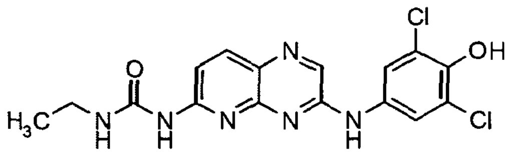 | 1-[3-(3,5-Dichloro-4-hydroxy-phenylamino)-pyrido[2,3-b]pyrazin-6-yl]-3-ethyl-urea |
| **Compound 186** | 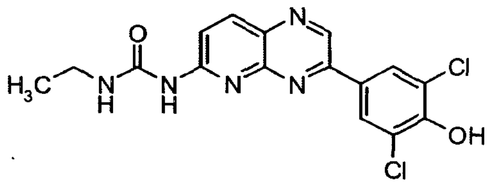 | 1-[3-(3,5-Dichloro-4-hydroxy-phenyl)-pyrido[2,3-b]pyrazin-6-yl]-3-ethyl-urea |
| **Compound 187** | 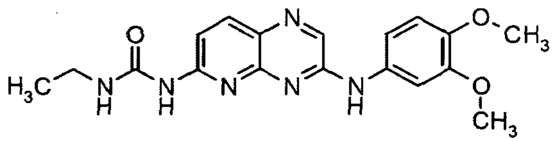 | 1-[3-(3,4-Dimethoxy-phenylamino)-pyrido[2,3-b]pyrazin-6-yl]-3-ethyl-urea |

(continued)

| | | |
|---|---|---|
| **Compound 188** | 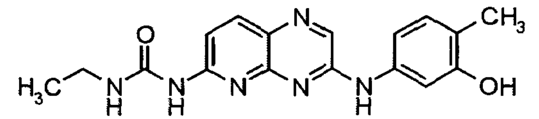 | 1-Ethyl-3-[3-(3-hydroxy-4-methyl-phenylamino)-pyrido[2,3-b]pyrazin-6-yl]-urea |
| **Compound 189** | 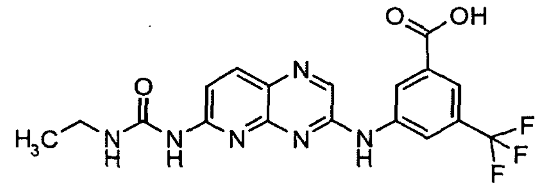 | 3-[6-(3-Ethyl-ureido)-pyrido[2,3-b]-pyrazin-3-ylamino]-5-trifluoromethyl-benzoic acid |
| **Compound 190** | 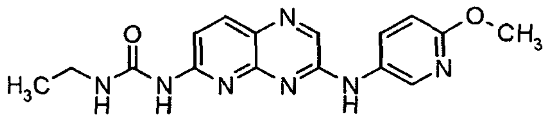 | 1-Ethyl-3-[3-(6-methoxy-pyridin-3-ylamino)-pyrido[2,3-b]pyrazin-6-yl]-urea |
| **Compound 191** | 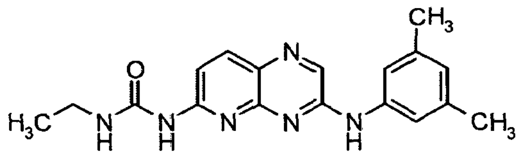 | 1-[3-(3,5-Dimethyl-phenylamino)-pyrido[2,3-b]pyrazin-6-yl]-3-ethyl-urea |
| **Compound 192** | 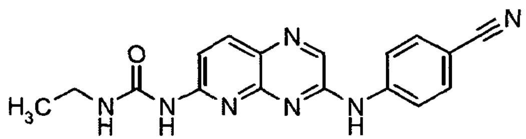 | 1-[3-(4-Cyano-phenylamino)-pyrido-[2,3-b]pyrazin-6-yl]-3-ethyl-u rea |
| **Compound 193** | 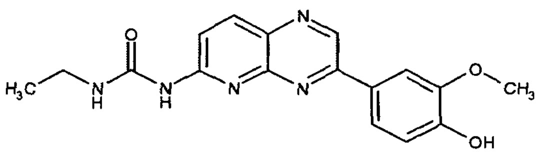 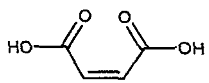 | 1-Ethyl-3-[3-(4-hydroxy-3-methoxy-phenyl)-pyrido[2,3-b]pyrazin-6-yl]-urea (Z)-but-2-endicarbonic acid salt |

(continued)

| | | |
|---|---|---|
| **Compound 194** | 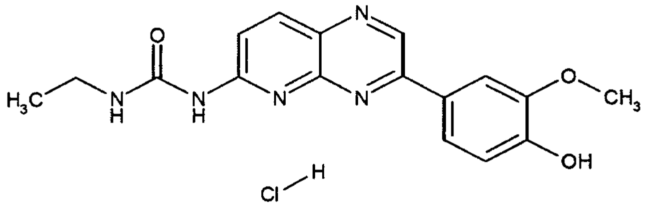 | 1-Ethyl-3-[3-(4-hydroxy-3-methoxy-phenyl)-pyrido[2,3-b]pyrazin-6-yl]-urea Hydrochloride |
| **Compound 195** | 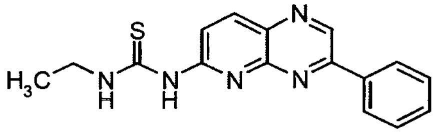 | 1-Ethyl-3-(3-phenyl-pyrido[2,3-b]pyrazin-6-yl)-thiourea |
| **Compound 196** | 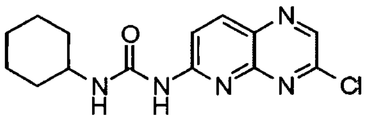 | 1-(3-Chloro-pyrido[2,3-b]pyrazin-6-yl)-3-cyclohexyl-urea |
| **Compound 197** | 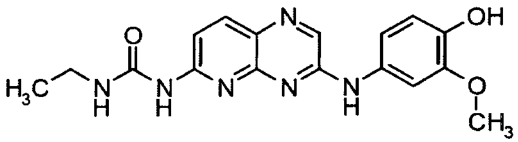 | 1-Ethyl-3-[3-(4-hydroxy-3-methoxy-phenylamino)-pyrido[2,3-b]pyrazin-6-yl]-urea |
| **Compound 198** | 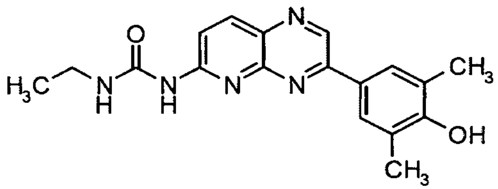 | 1-Ethyl-3-[3-(4-hydroxy-3,5-dimethyl-phenyl)-pyrido[2,3-b]pyrazin-6-yl]-urea |
| **Compound 199** | 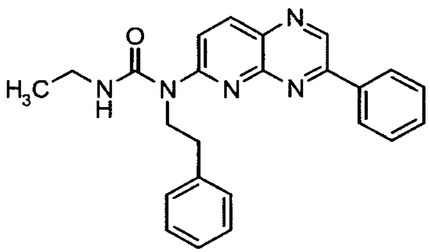 | 3-Ethyl-1-phenethyl-1-(3-phenyl-pyrido[2,3-b]pyrazin-6-yl)-urea |

(continued)

| | | |
|---|---|---|
| **Compound 200** | 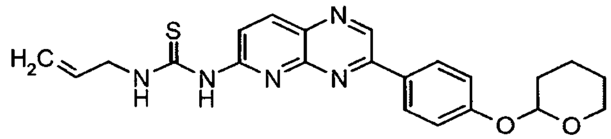 | 1-Allyl-3-{3-[4-(tetrahydro-pyran-2-yloxy)-phenyl]-pyrido[2,3-b]pyrazin-6-yl}-thiourea |
| **Compound 201** | 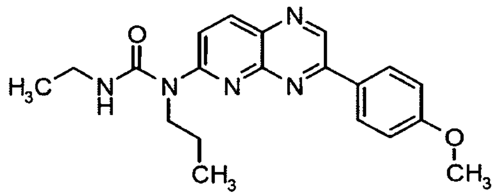 | 3-Ethyl-1-[3-(4-methoxy-phenyl)-pyrido[2,3-b]pyrazin-6-yl]-1-propyl-urea |
| **Compound 202** | 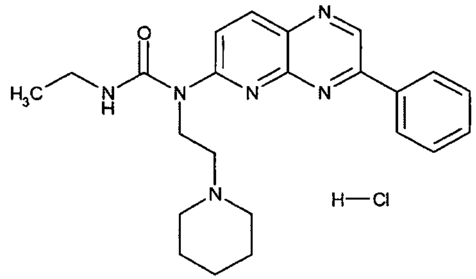 | 3-Ethyl-1-(3-phenyl-pyrido[2,3-b]pyrazin-6-yl)-1-(2-piperidin-1-yl-ethyl)-urea Hydrochloride |
| **Compound 203** | 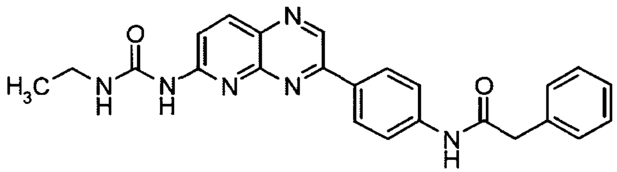 | N-{4-[6-(3-Ethyl-ureido)-pyrido[2,3-b]-pyrazin-3-yl]-phenyl}-2-phenyl acetamide |
| **Compound 204** | 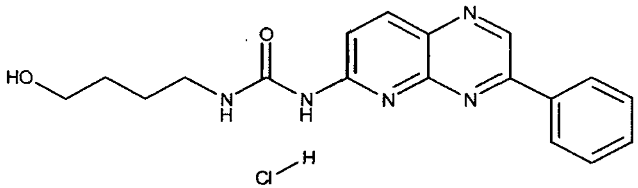 | 1-(4-Hydroxy-butyl)-3-(3-phenyl-pyrido[2,3-b]pyrazin-6-yl)-urea Hydrochloride |

(continued)

| | | |
|---|---|---|
| **Compound 205** | 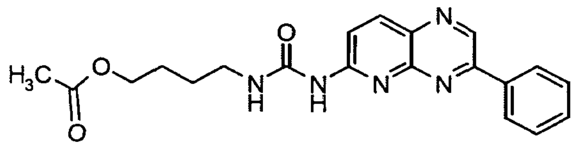 | Acetic acid-4-[3-(3-phenyl-pyrido[2,3-b]pyrazin-6-yl)-ureido]-butyl ester |
| **Compound 206** | 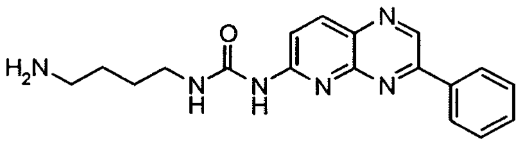 | 1-(4-Amino-butyl)-3-(3-phenyl-pyrido[2,3-b]pyrazin-6-yl)-urea |
| **Compound 207** | 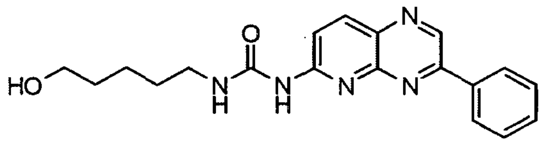 | 1-(5-Hydroxy-pentyl)-3-(3-phenyl-pyrido[2,3-b]pyrazin-6-yl)-urea |
| **Compound 208** | 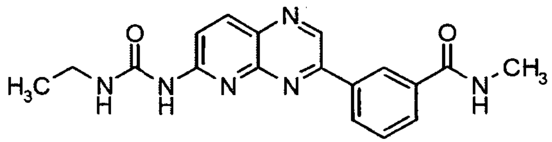 | 3-[6-(3-Ethyl-ureido)-pyrido[2,3-b]-pyrazin-3-yl]-N-methyl-benzamide |
| **Compound 209** | 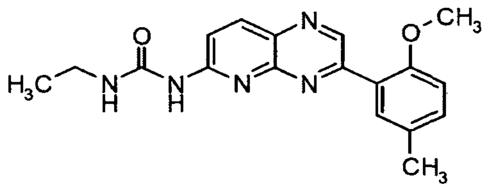 | 1-Ethyl-3-[3-(2-methoxy-5-methyl-phenyl)-pyrido[2,3-b]pyrazin-6-yl]-urea |
| **Compound 210** | 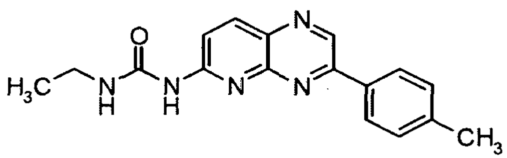 | 1-Ethyl-3-(3-p-tolyl-pyrido[2,3-b]pyrazin-6-yl)-urea |

(continued)

| | | |
|---|---|---|
| **Compound 211** | 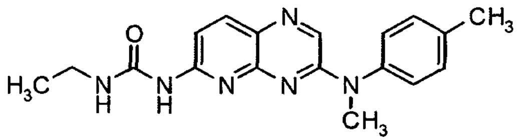 | 1-Ethyl-3-[3-(methyl-p-tolyl-amino)-pyrido[2,3-b]pyrazin-6-yl]-urea |
| **Compound 212** | 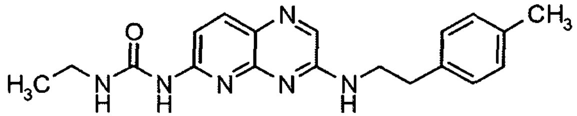 | 1-Ethyl-3-[3-(2-p-tolyl-ethylamino)-pyrido[2,3-b]pyrazin-6-yl]-urea |
| **Compound 213** | 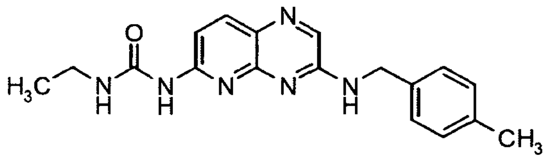 | 1-Ethyl-3-[3-(4-methyl-benzylamino)-pyrido[2,3-b]pyrazin-6-yl]-urea |
| **Compound 214** | 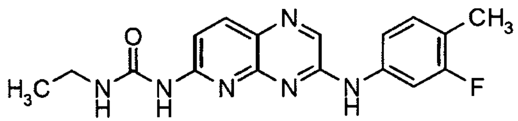 | 1-Ethyl-3-[3-(3-fluoro-4-methyl-phenyl-amino)-pyrido[2,3-b]pyrazin-6-yl]-urea |
| **Compound 215** | 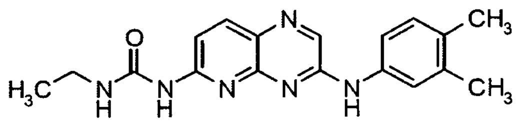 | 1-[3-(3,4-Dimethyl-phenylamino)-pyrido[2,3-b]pyrazin-6-yl]-3-ethyl-urea |
| **Compound 216** | 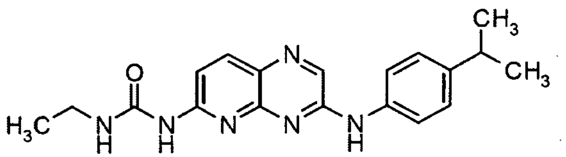 | 1-Ethyl-3-[3-(4-isopropyl-phenylamino)-pyrido[2,3-b]pyrazin-6-yl]-urea |
| **Compound 217** | 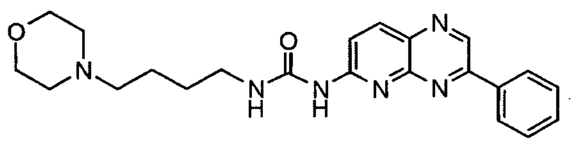 | 1-(4-Morpholin-4-yl-butyl)-3-(3-phenyl-pyrido[2,3-b]pyrazin-6-yl)-urea |

(continued)

| | | |
|---|---|---|
| **Compound 218** | 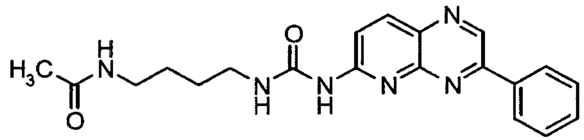 | N-{4-[3-(3-Phenyl-pyrido[2,3-b]pyrazin-6-yl)-ureido]-butyl}-acetamide |
| **Compound 219** | 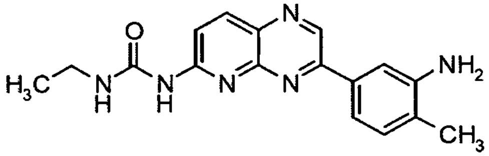 | 1-[3-(3-Amino-4-methyl-phenyl)-pyrido[2,3-b]pyrazin-6-yl]-3-ethyl-urea |
| **Compound 220** | 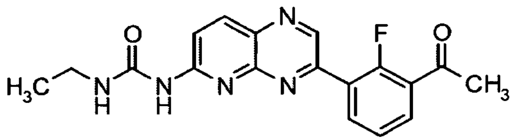 | 1-[3-(3-Acetyl-2-fluorophenyl)-pyrido[2,3-b]pyrazin-6-yl]-3-ethyl-urea |
| **Compound 221** | 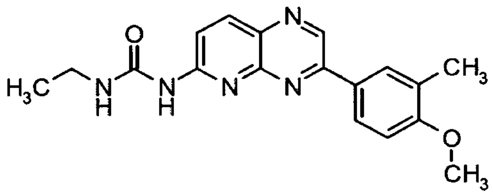 | 1-Ethyl-3-[3-(4-methoxy-3-methyl-phenyl)-pyrido[2,3-b]pyrazin-6-yl]-urea |
| **Compound 222** | 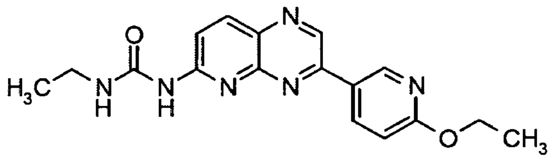 | 1-[3-(6-Ethoxy-pyridin-3-yl)-pyrido[2,3-b]pyrazin-6-yl]-3-ethyl-urea |
| **Compound 223** | 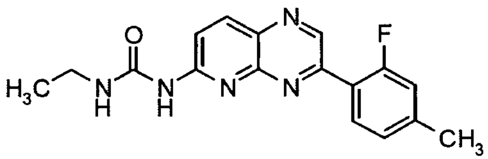 | 1-Ethyl-3-[3-(2-fluoro-4-methyl-phenyl)-pyrido[2,3-b]pyrazin-6-yl]-urea |

(continued)

| | | |
|---|---|---|
| **Compound 224** | 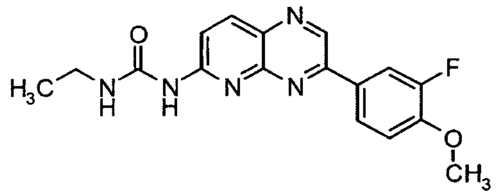 | 1-Ethyl-3-[3-(3-fluoro-4-methoxy-phenyl)-pyrido[2,3-b]pyrazin-6-yl]-urea |
| **Compound 225** | 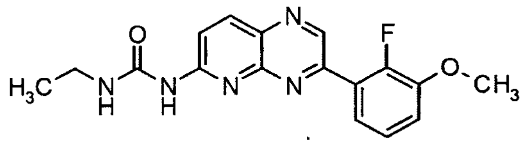 | 1-Ethyl-3-[3-(2-fluoro-3-methoxy-phenyl)-pyrido[2,3-b]pyrazin-6-yl]-urea |
| **Compound 226** | 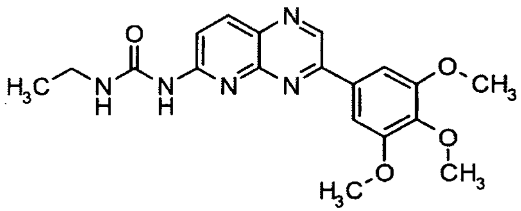 | 1-Ethyl-3-[3-(3,4,5-trimethoxy-phenyl)-pyrido[2,3-b]pyrazin-6-yl]-urea |
| **Compound 227** | 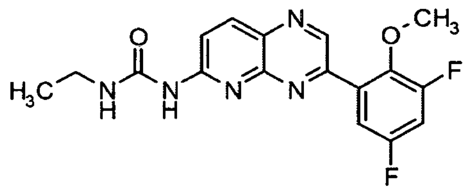 | 1-[3-(3,5-Difluoro-2-methoxy-phenyl)-pyrido[2,3-b]pyrazin-6-yl]-3-ethyl-urea |
| **Compound 228** | 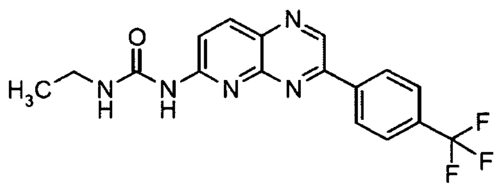 | 1-Ethyl-3-[3-(4-trifluoromethyl-phenyl)-pyrido[2,3-b]pyrazin-6-yl]-urea |

(continued)

| | | |
|---|---|---|
| **Compound 229** | 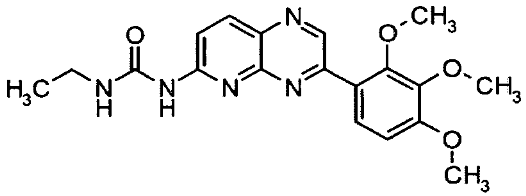 | 1-Ethyl-3-[3-(2,3,4-trimethoxy-phenyl)-pyrido[2,3-b]pyrazin-6-yl]-urea |
| **Compound 230** | 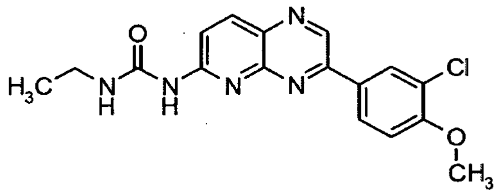 | 1-[3-(3-Chloro-4-methoxy-phenyl)-pyrido[2,3-b]pyrazin-6-yl]-3-ethyl-urea |
| **Compound 231** | 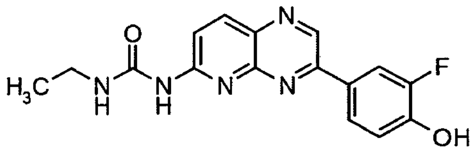 | 1-Ethyl-3-[3-(3-fluoro-4-hydroxy-phenyl)-pyrido[2,3-b]pyrazin-6-yl]-urea |
| **Compound 232** | 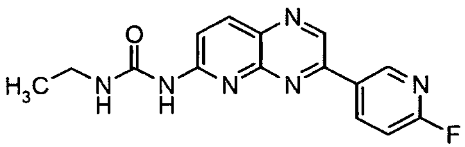 | 1-Ethyl-3-[3-(6-fluoro-pyridin-3-yl)-pyrido[2,3-b]pyrazin-6-yl]-urea |
| **Compound 233** | 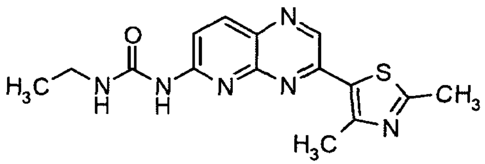 | 1-[3-(2,4-Dimethyl-thiazol-5-yl)-pyrido-[2,3-b]pyrazin-6-yl]-3-ethyl-urea |
| **Compound 234** | 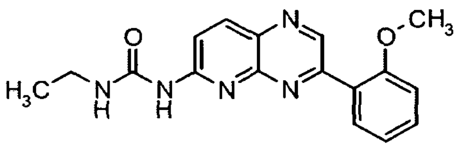 | 1-Ethyl-3-[3-(2-methoxy-phenyl)-pyrido[2,3-b]pyrazin-6-yl]-urea |

(continued)

| | | |
|---|---|---|
| **Compound 235** | 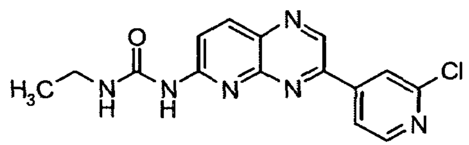 | 1-[3-(2-Chloro-pyridin-4-yl)-pyrido[2,3-b]pyrazin-6-yl]-3-ethyl-urea |
| **Compound 236** | 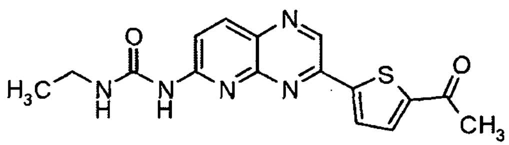 | 1-[3-(5-Acetyl-thiophen-2-yl)-pyrido[2,3-b]pyrazin-6-yl]-3-ethyl-urea |
| **Compound 237** | 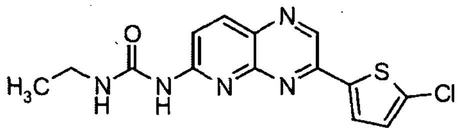 | 1-[3-(5-Chloro-thiophen-2-yl)-pyrido[2,3-b]pyrazin-6-yl]-3-ethyl-urea |
| **Compound 238** | 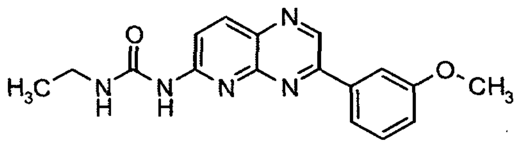 | 1-Ethyl-3-[3-(3-methoxyphenyl)-pyrido[2,3-b]pyrazin-6-yl]-urea |
| **Compound 239** | 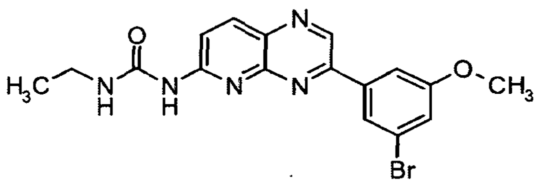 | 1-[3-(3-Bromo-5-methoxy-phenyl)-pyrido[2,3-b]pyrazin-6-yl]-3-ethyl-urea |
| **Compound 240** | 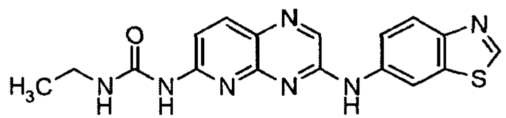 | 1-[3-(Benzothiazol-6-ylamino)-pyrido[2,3-b]pyrazin-6-yl]-3-eth yl-u rea |
| **Compound 241** | 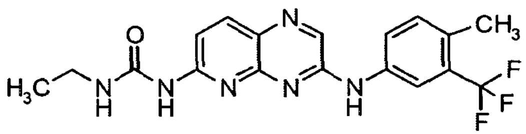 | 1-Ethyl-3-[3-(4-methyl-3-trifluoromethyl-phenylamino)-pyrido[2,3-b]pyrazin-6-yl]-urea |

(continued)

| | | |
|---|---|---|
| **Compound 242** | 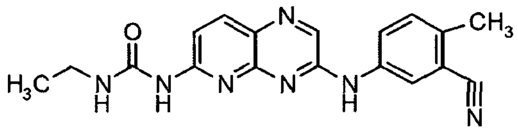 | 1-[3-(3-Cyano-4-methyl-phenylamino)-pyrido[2,3-b]pyrazin-6-yl]-3-ethyl-urea |
| **Compound 243** | 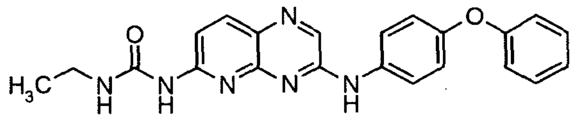 | 1-Ethyl-3-[3-(4-phenoxy-phenylamino)-pyrido[2,3-b]pyrazin-6-yl]-urea |
| **Compound 244** | 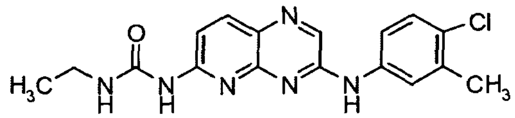 | 1-[3-(4-Chloro-3-methyl-phenylamino)-pyrido[2,3-b]pyrazin-6-yl]-3-ethyl-urea |
| **Compound 245** | 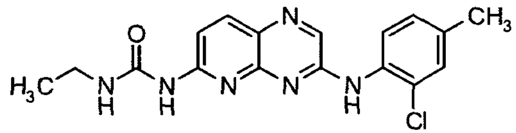 | 1-[3-(2-Chloro-4-methyl-phenylamino)-pyrido[2,3-b]pyrazin-6-yl]-3-ethyl-urea |
| **Compound 246** | 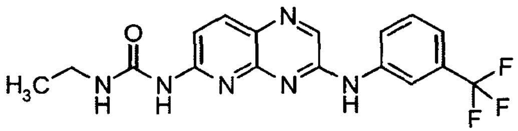 | 1-Ethyl-3-[3-(3-trifluoromethyl-phenylamino)-pyrido[2,3-b]pyrazin-6-yl]-urea |
| **Compound 247** | 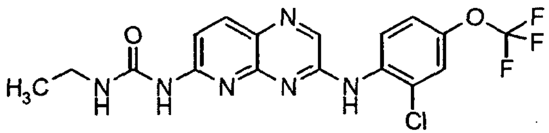 | 1-[3-(2-Chloro-4-trifluoromethoxy-phenylamino)-pyrido[2,3-b]pyrazin-6-yl]-3-ethyl-urea |
| **Compound 248** | 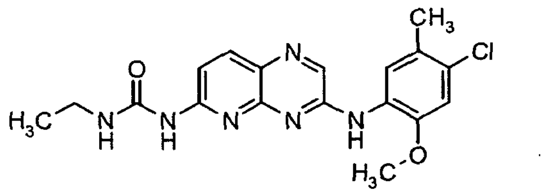 | 1-[3-(4-Chloro-2-methoxy-5-methyl-phenylamino)-pyrido[2,3-b]pyrazin-6-yl]-3-ethyl-urea |

(continued)

| | | |
|---|---|---|
| **Compound 249** | 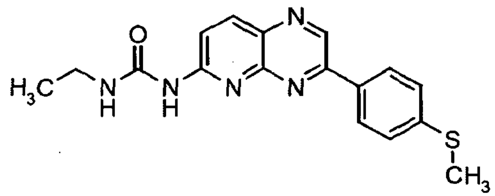 | 1-Ethyl-3-[3-(4-methylsulfanyl-phenyl)-pyrido[2,3-b]pyrazin-6-yl]-urea |
| **Compound 250** | 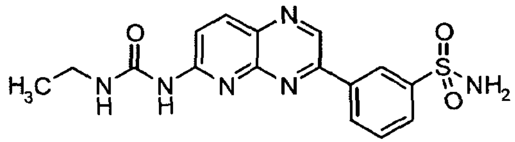 | 3-[6-(3-Ethyl-ureido)-pyrido[2,3-b]pyrazin-3-yl]-benzenesulfonamide |
| **Compound 251** | 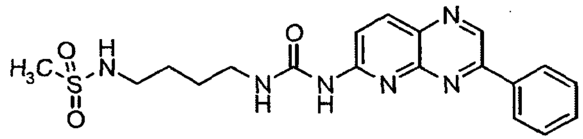 | N-{4-[3-(3-Phenyl-pyrido[2,3-b]pyrazin-6-yl)-ureido]-butyl}-methansulfonamide |
| **Compound 252** | 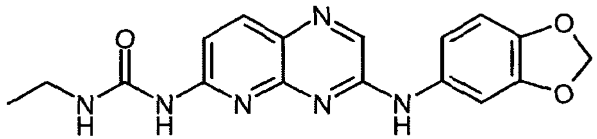 | 1-[3-(Benzo[1,3]dioxol-5-ylamino)-pyrido[2,3-b]pyrazin-6-yl]-3-ethyl-urea |
| **Compound 253** | 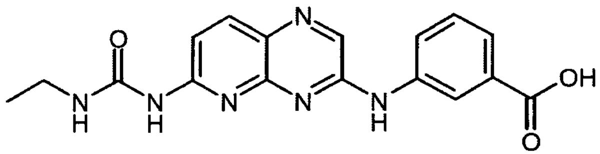 | 3-[6-(3-Ethyl-ureido)-pyrido[2,3-b]pyrazin-3-ylamino]-benzoic acid |
| **Compound 254** | 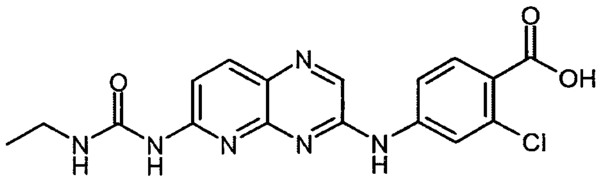 | 2-Chloro-4-[6-(3-ethyl-ureido)-pyrido[2,3-b]pyrazin-3-ylamino]-benzoic acid |

(continued)

| | | |
|---|---|---|
| **Compound 255** | 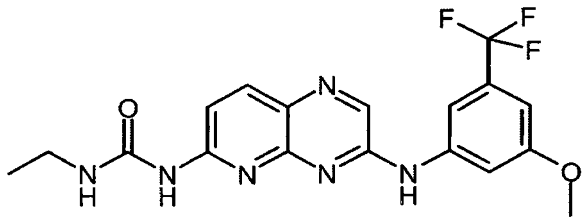 | 1-Ethyl-3-[3-(3-methoxy-5-trifluoromethyl-phenylamino)-pyrido[2,3-b]pyrazin-6-yl]-urea |
| **Compound 256** | 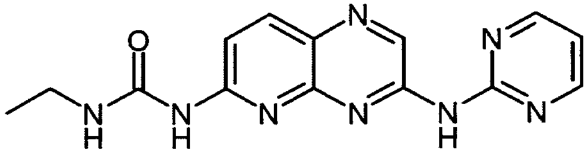 | 1-Ethyl-3-[3-(pyrimidin-2-ylamino)-pyrido[2,3-b]pyrazin-6-yl]-urea |
| **Compound 257** | 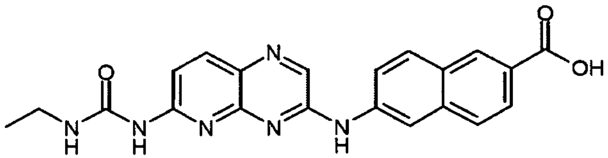 | 6-[6-(3-Ethyl-ureido)-pyrido[2,3-b]pyrazin-3-ylamino]-naphthalin-2-carbonic acid |
| **Compound 258** | 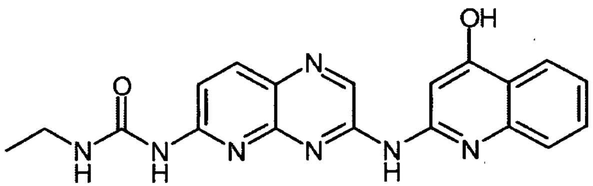 | 1-Ethyl-3-[3-(4-hydroxy-quinolin-2-ylamino)-pyrido[2,3-b]pyrazin-6-yl]-urea |
| **Compound 259** | 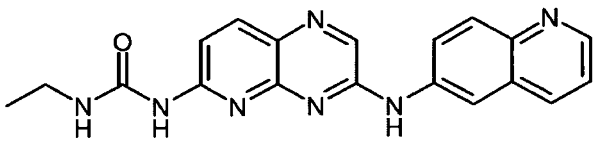 | 1-Ethyl-3-[3-(quinolin-6-ylamino)-pyrido[2,3-b]pyrazin-6-yl]-urea |
| **Compound 260** | 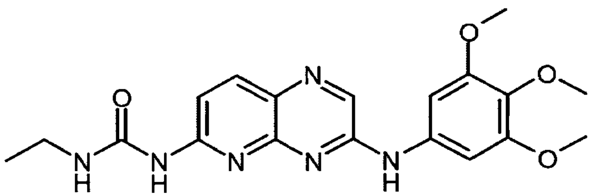 | 1-Ethyl-3-[3-(3,4,5-trimethoxy-phenylamino)-pyrido[2,3-b]pyrazin-6-yl]-urea |

(continued)

| | | |
|---|---|---|
| **Compound 261** | 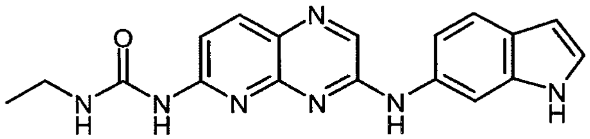 | 1-Ethyl-3-[3-(1H-indol-6-ylamino)-pyrido[2,3-b]pyrazin-6-yl]-urea |
| **Compound 262** | 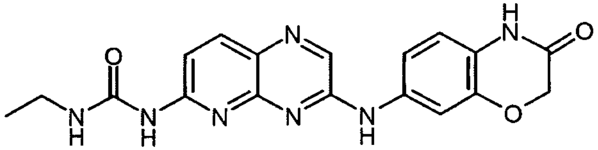 | 1-Ethyl-3-[3-(3-oxo-3,4-dihydro-2H-benzo[1,4]oxazin-7-ylamino)-pyrido[2,3-b]pyrazin-6-yl]-urea |
| **Compound 263** | 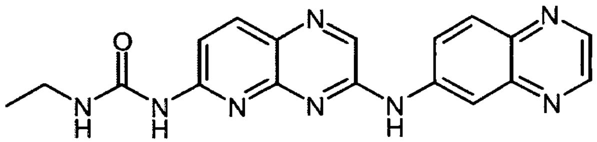 | 1-Ethyl-3-[3-(quinoxalin-6-ylamino)-pyrido[2,3-b]pyrazin-6-yl]-urea |
| **Compound 264** | 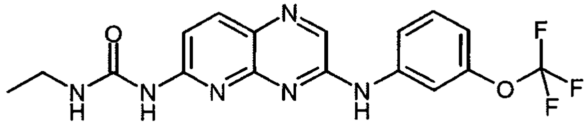 | 1-Ethyl-3-[3-(3-trifluoromethoxy-phenylamino)-pyrido[2,3-b]pyrazin-6-yl]-urea |
| **Compound 265** | 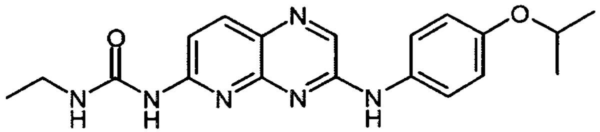 | 1-Ethyl-3-[3-(4-isopropoxy-phenylamino)-pyrido[2,3-b]pyrazin-6-yl]-urea |
| **Compound 266** | 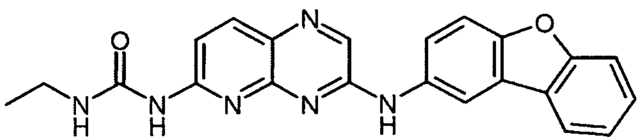 | 1-[3-(Dibenzofuran-2-ylamino)-pyrido[2,3-b]pyrazin-6-yl]-3-ethyl-urea |
| **Compound 267** | 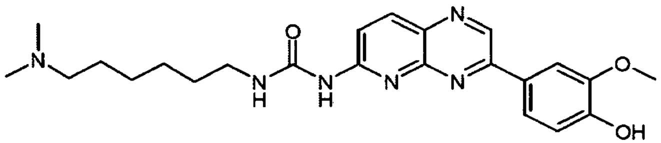 | 1-(6-Dimethylamino-hexyl)-3-[3-(4-hydroxy-3-methoxy-phenyl)-pyrido[2,3-b]pyrazin-6-yl]-urea |

(continued)

| | | |
|---|---|---|
| **Compound 268** | 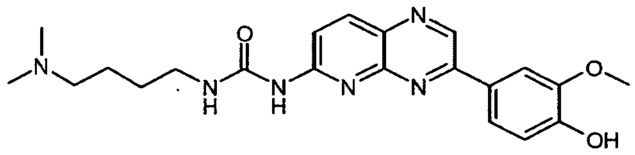 | 1-(4-Dimethylamino-butyl)-3-[3-(4-hydroxy-3-methoxy-phenyl)-pyrido[2,3-b]pyrazin-6-yl]-urea |
| **Compound 269** | 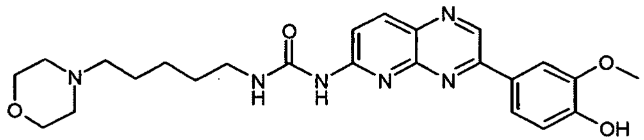 | 1-[3-(4-Hydroxy-3-methoxy-phenyl)-pyrido[2,3-b]pyrazin-6-yl]-3-(5-morpholin-4-yl-pentyl)-urea |

6. Use according to any one of Claims 1 to 5, wherein the medicament is administered with at least one further pharmacologically active substance.

7. Use according to any one of Claims 1 to 5, wherein the medicament is administered before and/or during and/or after treatment with at least one further pharmacologically active substance.

8. Use according to any one of Claims 1 to 5, wherein the medicament is administered before and/or during and/or after treatment with radiation therapy and/or surgery.

9. Use according to any one of Claims 1 to 5, wherein the compound according to formula (I) is present in a unit dose of 0.001 mg to 100 mg per kg body weight of a patient.

10. Pharmaceutical composition, comprising a compound selected from the group of compounds No. 38 - 269:

| | |
|---|---|
| **Cpd. 38** | 1-[3-(4-Hydroxy-phenyl)-pyrido[2,3-b]pyrazin-6-yl]-3-methyl-urea |
| **Cpd. 39** | 1-Allyl-3-[3-(4-phenoxy-phenyl)-pyrido[2,3-b]pyrazin-6-yl]-urea |
| **Cpd. 40** | Methane sulfonic acid-4-[6-(3-allyl-ureido)-pyrido[2,3-b]pyrazin-3-yl]-phenyl-ester |
| **Cpd. 41** | 4-Dimethylamino-benzoic acid-4-[6-(3-allyl-ureido)-pyrido[2,3-b]pyrazin-3-yl]-phenyl-ester |
| **Cpd. 42** | Acetic acid-4-[6-(3-allyl-ureido)-pyrido[2,3-b]pyrazin-3-yl]-phenyl-ester |
| **Cpd. 43** | 1-Ethyl-3-[3-(4-hydroxy-phenyl)-pyrido[2,3-b]pyrazin-6-yl]-urea |
| **Cpd. 44** | 1-[3-(4-Amino-phenyl)-pyrido[2,3-b]pyrazin-6-yl]-3-methyl-thiourea |
| **Cpd. 45** | 1-Ethyl-3-(3-phenyl-pyrido[2,3-b]pyrazin-6-yl)-urea |
| **Cpd. 46** | 1-Acetyl-1-ethyl-3-(3-phenyl-pyrido[2,3-b]pyrazin-6-yl)-urea |
| **Cpd. 47** | 1-Allyl-3-[3-(4-fluoro-phenyl)-pyrido[2,3-b]pyrazin-6-yl]-urea |
| **Cpd. 48** | 1,1-Diethyl-3-(3-phenyl-pyrido[2,3-b]pyrazin-6-yl)-urea |
| **Cpd. 49** | 1-(2-Chloro-ethyl)-3-[3-(4-fluoro-phenyl)-pyrido[2,3-b]pyrazin-6-yl]-urea |
| **Cpd. 50** | 1-Ethyl-3-[3-(4-methoxy-phenyl)-pyrido[2,3-b]pyrazin-6-yl]-urea |
| **Cpd. 51** | 1-(3-Phenyl-pyrido[2,3-b]pyrazin-6-yl)-3-propyl-urea |
| **Cpd. 52** | [3-(3-Phenyl-pyrido[2,3-b]pyrazin-6-yl)-ureido]-acetic acid-ethyl-ester |
| **Cpd. 53** | 1-(3-Chloro-pyrido[2,3-b]pyrazin-6-yl)-3-ethyl-urea |
| **Cpd. 54** | 1-Ethyl-3-[3-(4-fluoro-phenyl)-pyrido[2,3-b]pyrazin-6-yl]-urea |
| **Cpd. 55** | 1-[3-(3-Chloro-4-fluoro-phenyl)-pyrido[2,3-b]pyrazin-6-yl]-3-ethyl-urea |
| **Cpd. 56** | 4-[6-(3-Ethyl-ureido)-pyrido[2,3-b]pyrazin-3-yl]-benzoic acid |
| **Cpd. 57** | N-{4-[6-(3-Ethyl-ureido)-pyrido[2,3-b]pyrazin-3-yl]-phenyl}-acetamide |
| **Cpd. 58** | 1-[3-(2,4-Difluoro-phenyl)-pyrido[2,3-b]pyrazin-6-yl]-3-ethyl-urea |
| **Cpd. 59** | 1-Ethyl-3-(3-morpholin-4-yl-pyrido[2,3-b]pyrazin-6-yl)-urea |
| **Cpd. 60** | 1-Ethyl-3-[3-(4-methyl-piperazin-1-yl)-pyrido[2,3-b]pyrazin-6-yl]-urea |
| **Cpd. 61** | 1-Ethyl-3-[3-(2-hydroxy-phenyl)-pyrido[2,3-b]pyrazin-6-yl]-urea |
| **Cpd. 62** | 1-Ethyl-3-[3-(2-methoxy-ethylamino)-pyrido[2,3-b]pyrazin-6-yl]-urea |
| **Cpd. 63** | 1-[3-(4-Chloro-3-trifluoromethyl-phenyl)-pyrido[2,3-b]pyrazin-6-yl]-3-ethyl-urea |
| **Cpd. 64** | 1-Ethyl-3-(3-phenoxy-pyrido[2,3-b]pyrazin-6-yl)-urea |
| **Cpd. 65** | 1-[3-(Cyclopropylmethyl-amino)-pyrido[2,3-b]pyrazin-6-yl]-3-ethyl-urea |
| **Cpd. 66** | 1-Ethyl-3-{3-[(pyridin-4-ylmethyl)-amino]-pyrido[2,3-b]pyrazin-6-yl}-urea |

(continued)

| | |
|---|---|
| **Cpd. 67** | 1-Ethyl-3-[3-(4-fluoro-benzyloxy)-pyrido[2,3-b]pyrazin-6-yl]-urea |
| **Cpd. 68** | 1-Ethyl-3-(3-pyridin-4-yl-pyrido[2,3-b]pyrazin-6-yl)-urea |
| **Cpd. 69** | 1-Ethyl-3-[3-(pyridin-3-yloxy)-pyrido[2,3-b]pyrazin-6-yl]-urea |
| **Cpd. 70** | 1-Ethyl-3-[3-(tetrahydro-furan-2-ylmethoxy)-pyrido[2,3-b]pyrazin-6-yl]-urea |
| **Cpd. 71** | 1-Ethyl-3-[3-(4-morpholin-4-yl-phenyl)-pyrido[2,3-b]pyrazin-6-yl]-urea |
| **Cpd. 72** | 1-Ethyl-3-(3-hydroxy-pyrido[2,3-b]pyrazin-6-yl)-urea |
| **Cpd. 73** | 1-Ethyl-3-[3-(3-methoxy-phenylsulfanyl)-pyrido[2,3-b]pyrazin-6-yl]-urea |
| **Cpd. 74** | 1-Ethyl-3-(3-quinolin-3-yl-pyrido[2,3-b]pyrazin-6-yl)-urea |
| **Cpd. 75** | 1-(3-Benzo[b]thiophen-3-yl-pyrido[2,3-b]pyrazin-6-yl)-3-ethyl-urea |
| **Cpd. 76** | 1-Ethyl-3-[3-(pyridin-2-ylsulfanyl)-pyrido[2,3-b]pyrazin-6-yl]-urea |
| **Cpd. 77** | 1-[3-(4-Dimethylamino-phenyl)-pyrido[2,3-b]pyrazin-6-yl]-3-ethyl-urea |
| **Cpd. 78** | N-{4-[6-(3-Ethyl-ureido)-pyrido[2,3-b]pyrazin-3-yl]-phenyl}-methansulfonamide |
| **Cpd. 79** | 1-Ethyl-3-[3-(1H-pyrazol-4-yl)-pyrido[2,3-b]pyrazin-6-yl]-urea |
| **Cpd. 80** | 1-(3-Benzylsulfanyl-pyrido[2,3-b]pyrazin-6-yl)-3-ethyl-urea |
| **Cpd. 81** | 1-Ethyl-3-[3-(4-methyl-[1,4]diazepan-1-yl)-pyrido[2,3-b]pyrazin-6-yl]-urea |
| **Cpd. 82** | 1-[3-(4-Amino-phenyl)-pyrido[2,3-b]pyrazin-6-yl]-3-ethyl-urea |
| **Cpd. 83** | 1-(3-Amino-pyrido[2,3-b]pyrazin-6-yl)-3-ethyl-urea |
| **Cpd. 84** | 1-Ethyl-3-pyrido[2,3-b]pyrazin-6-yl-urea |
| **Cpd. 85** | 1-Ethyl-3-(3-imidazol-1-yl-pyrido[2,3-b]pyrazin-6-yl)-urea |
| **Cpd. 86** | 1-Ethyl-3-[3-(4-fluoro-2-hydroxy-phenyl)-pyrido[2,3-b]pyrazin-6-yl]-urea |
| **Cpd. 87** | 1-(3-Cyclopentyloxy-pyrido[2,3-b]pyrazin-6-yl)-3-ethyl-urea |
| **Cpd. 88** | 1-Ethyl-3-[3-(4-hydroxy-piperidin-1-yl)-pyrido[2,3-b]pyrazin-6-yl]-urea |
| **Cpd. 89** | (3-Phenyl-pyrido[2,3-b]pyrazin-6-yl)-urea |
| **Cpd. 90** | 1-Ethyl-3-(3-pyrimidin-5-yl-pyrido[2,3-b]pyrazin-6-yl)-urea |
| **Cpd. 91** | 1-Ethyl-3-(3-pyridin-3-yl-pyrido[2,3-b]pyrazin-6-yl)-urea |
| **Cpd. 92** | 1-Allyl-3-(3-phenyl-pyrido[2,3-b]pyrazin-6-yl)-urea |
| **Cpd. 93** | 1-Ethyl-3-(3-piperazin-1-yl-pyrido[2,3-b]pyrazin-6-yl)-urea |
| **Cpd. 94** | 1-[3-(3-Chloro-pyridin-4-ylmethyl)-pyrido[2,3-b]pyrazin-6-yl]-3-ethyl-urea |
| **Cpd. 95** | 1-Ethyl-3-[3-(6-methoxy-pyridin-3-yl)-pyrido[2,3-b]pyrazin-6-yl]-urea |
| **Cpd. 96** | 1-[3-(3,5-Dimethyl-isoxazol-4-yl)-pyrido[2,3-b]pyrazin-6-yl]-3-ethyl-urea |
| **Cpd. 97** | 1-Ethyl-3-[3-(4-trifluoromethoxy-phenyl)-pyrido[2,3-b]pyrazin-6-yl]-urea |
| **Cpd. 98** | 1-Ethyl-3-(3-furan-2-yl-pyrido[2,3-b]pyrazin-6-yl)-urea |
| **Cpd. 99** | 1-Ethyl-3-[3-(2-methoxy-pyridin-3-yl)-pyrido[2,3-b]pyrazin-6-yl]-urea |
| **Cpd. 100** | 1-[3-(2,4-Dimethoxy-phenyl)-pyrido[2,3-b]pyrazin-6-yl]-3-ethyl-urea |
| **Cpd. 101** | 1-Ethyl-3-[3-(1H-pyrrol-2-yl)-pyrido[2,3-b]pyrazin-6-yl]-urea |
| **Cpd. 102** | 1-Ethyl-3-[3-(6-morpholin-4-yl-pyridin-3-yl)-pyrido[2,3-b]pyrazin-6-yl]-urea |
| **Cpd. 103** | 1-Benzyl-3-ethyl-1-(3-phenyl-pyrido[2,3-b]pyrazin-6-yl)-urea |
| **Cpd. 104** | 1-[3-(2-Amino-phenyl)-pyrido[2,3-b]pyrazin-6-yl]-3-ethyl-urea |
| **Cpd. 105** | 1-Ethyl-3-[3-(4-hydroxymethyl-phenyl)-pyrido[2,3-b]pyrazin-6-yl]-urea |

(continued)

| | |
|---|---|
| **Cpd. 106** | 1-[3-(3-Amino-phenyl)-pyrido[2,3-b]pyrazin-6-yl]-3-ethyl-urea |
| **Cpd. 107** | 1-[3-(4-Acetyl-phenyl)-pyrido[2,3-b]pyrazin-6-yl]-3-ethyl-urea |
| **Cpd. 108** | 1-[3-(2,3-Dihydro-benzofuran-5-yl)-pyrido[2,3-b]pyrazin-6-yl]-3-ethyl-urea |
| **Cpd. 109** | 1-[3-(4-Benzyloxy-3-fluoro-phenyl)-pyrido[2,3-b]pyrazin-6-yl]-3-ethyl-urea |
| **Cpd. 110** | 1-(2,3-Dihydroxy-propyl)-3-(3-phenyl-pyrido[2,3-b]pyrazin-6-yl)-urea |
| **Cpd. 111** | 1-Ethyl-3-[3-(3-formyl-4-methoxy-phenyl)-pyrido[2,3-b]pyrazin-6-yl]-urea |
| **Cpd. 112** | 1-Ethyl-3-[3-(4-methansulfonyl-phenyl)-pyrido[2,3-b]pyrazin-6-yl]-urea |
| **Cpd. 113** | N-{4-[6-(3-Ethyl-ureido)-pyrido[2,3-b]pyrazin-3-yl]-phenyl}-succinamid acid |
| **Cpd. 114** | 1-Ethyl-3-(3-pyridin-4-yl-pyrido[2,3-b]pyrazin-6-yl)-urea Methane sulfonic acid salt (free base) |
| **Cpd. 115** | 1-[3-(2,6-Dimethoxy-pyridin-3-yl)-pyrido[2,3-b]pyrazin-6-yl]-3-ethyl-urea |
| **Cpd. 116** | 1-[3-(2,6-Dimethoxy-pyrimidin-4-yl)-pyrido[2,3-b]pyrazin-6-yl]-3-ethyl-urea |
| **Cpd. 117** | 1-[3-(2,4-Dioxo-1,2,3,4-tetrahydro-pyrimidin-5-yl)-pyrido[2,3-b]pyrazin-6-yl]-3-ethyl-urea |
| **Cpd. 118** | 1-Ethyl-3-[3-(1H-indol-5-yl)-pyrido[2,3-b]pyrazin-6-yl]-urea |
| **Cpd. 119** | 1-(3-Chloro-pyrido[2,3-b]pyrazin-6-yl)-3-ethyl-thiourea |
| **Cpd. 120** | 1-Ethyl-3-{3-[4-(2-methoxy-ethoxy)-phenyl]-pyrido[2,3-b]pyrazin-6-yl}-urea |
| **Cpd. 121** | Acrylic acid-4-[6-(3-ethyl-ureido)-pyrido[2,3-b]-pyrazin-3-yl]-phenyl-ester |
| **Cpd. 122** | 1-[3-(4-Cyano-phenyl)-pyrido[2,3-b]pyrazin-6-yl]-3-ethylurea |
| **Cpd. 123** | 1-(3-Benzo[1,2,5]oxadiazol-4-yl-pyrido[2,3-b]pyrazin-6-yl)-3-ethyl-urea |
| **Cpd. 124** | 1-Ethyl-3-[3-(4-hydroxy-3-methoxy-phenyl)-pyrido[2,3-b]pyrazin-6-yl]-urea |
| **Cpd. 125** | 1-[3-(2,6-Dimethoxy-phenyl)-pyrido[2,3-b]pyrazin-6-yl]-3-ethyl-urea |
| **Cpd. 126** | 1-[3-(3-Acetyl-phenyl)-pyrido[2,3-b]pyrazin-6-yl]-3-ethyl-urea |
| **Cpd. 127** | 1-Ethyl-3-[3-(3-morpholin-4-yl-phenyl)-pyrido[2,3-b]pyrazin-6-yl]-urea |
| **Cpd. 128** | 1-[3-(6-Amino-pyridin-3-yl)-pyrido[2,3-b]pyrazin-6-yl]-3-ethyl-urea |
| **Cpd. 129** | 3-{4-[6-(3-Ethyl-ureido)-pyrido[2,3-b]-pyrazin-3-yl]-phenoxy}-propionic acid |
| **Cpd. 130** | 1-isopropyl-3-(3-phenyl-pyrido[2,3-b]pyrazin-6-yl)-urea |
| **Cpd. 131** | 1-Cyclopentyl-3-(3-phenyl-pyrido[2,3-b]pyrazin-6-yl)-urea |
| **Cpd. 132** | 1-Pentyl-3-(3-phenyl-pyrido[2,3-b]pyrazin-6-yl)-urea |
| **Cpd. 133** | N-{4-[6-(3-Ethyl-ureido)-pyrido[2,3-b]pyrazin-3-yl]-phenyl}-acrylamide |
| **Cpd. 134** | 1-tert-Butyl-3-(3-phenyl-pyrido[2,3-b]pyrazin-6-yl)-urea |
| **Cpd. 135** | 1-(2-Hydroxy-ethyl)-3-(3-phenyl-pyrido[2,3-b]pyrazin-6-yl)-urea |
| **Cpd. 136** | 1-Cyclobutyl-3-(3-phenyl-pyrido[2,3-b]pyrazin-6-yl)-urea |
| **Cpd. 137** | 1-Allyl-3-[3-(4-nitro-phenyl)-pyrido[2,3-b]pyrazin-7-yl]-thiourea |
| **Cpd. 138** | 1-Ethyl-1-(Ethylcarbamoyl)-3-(3-phenyl-pyrido[2,3-b]pyrazin-6-yl)-urea |
| **Cpd. 139** | [3-(4-Hydroxy-phenyl)-pyrido[2,3-b]pyrazin-6-yl]-carbamic acid-allyl-ester |
| **Cpd. 140** | (3-Phenyl-pyrido[2,3-b]pyrazin-6-yl)-carbamic acid-ethyl-ester |
| **Cpd. 141** | 1-Ethyl-3-[3-(4-phenyl-piperazin-1-yl)-pyrido[2,3-b]pyrazin-6-yl]-urea |
| **Cpd. 142** | 1-(2,2-Dimethyl-[1,3]dioxolan-4-ylmethyl)-3-(3-phenyl-pyrido[2,3-b]pyrazin-6-yl)-urea |
| **Cpd. 143** | 1,3-Bis-(3-phenyl-pyrido[2,3-b]pyrazin-6-yl)-urea |
| **Cpd. 144** | N-(3-Phenyl-pyrido[2,3-b]pyrazin-6-yl)-acetamidine |

(continued)

| | |
|---|---|
| **Cpd. 145** | 1-Ethyl-3-[3-(2-methoxy-pyridin-3-yl)-pyrido[2,3-b]pyrazin-6-yl]-thiourea |
| **Cpd. 146** | 1-(4-Hydroxy-butyl)-3-(3-phenyl-pyrido[2,3-b]pyrazin-6-yl)-urea |
| **Cpd. 147** | 1-(3-Hydroxy-propyl)-3-(3-phenyl-pyrido[2,3-b]pyrazin-6-yl)-urea |
| **Cpd. 148** | 1-Ethyl-3-{3-[4-(3-morpholin-4-yl-propoxy)-phenyl]-pyrido[2,3-b]pyrazin-6-yl}-urea |
| **Cpd. 149** | 1-Ethyl-3-[3-(4-hydroxy-phenyl)-pyrido[2,3-b]pyrazin-6-yl]-thiourea |
| **Cpd. 150** | 1-Ethyl-3-(3-p-tolylamino-pyrido[2,3-b]pyrazin-6-yl)-urea |
| **Cpd. 151** | 1-Ethyl-3-[3-(3-hydroxy-phenyl)-pyrido[2,3-b]pyrazin-6-yl]-urea |
| **Cpd. 152** | 3-[6-(3-Ethyl-ureido)-pyrido[2,3-b]pyrazin-3-yl]-benzoic acid |
| **Cpd. 153** | 1-[3-(3,4-Dimethoxy-phenyl)-pyrido[2,3-b]pyrazin-6-yl]-3-ethyl-urea |
| **Cpd. 154** | 1-[3-(4-Amino-3-methoxy-phenyl)-pyrido[2,3-b]pyrazin-6-yl]-3-ethyl-urea |
| **Cpd. 155** | 3-[6-(3-Ethyl-ureido)-pyrido[2,3-b]-pyrazin-3-yl]-benzamide |
| **Cpd. 156** | 1-[3-(3-Chloro-4-hydroxy-5-methoxy-phenyl)-pyrido[2,3-b]pyrazin-6-yl]-3-ethyl-urea |
| **Cpd. 157** | 1-Ethyl-3-(3-m-tolylamino-pyrido-[2,3-b]pyrazin-6-yl)-urea |
| **Cpd. 158** | 1-Ethyl-3-[3-(4-methoxy-phenylamino)-pyrido[2,3-b]pyrazin-6-yl]-urea |
| **Cpd. 159** | 1-[3-(4-Chloro-phenylamino)-pyrido-[2,3-b]pyrazin-6-yl]-3-ethyl-urea |
| **Cpd. 160** | 1-Ethyl-3-(3-o-tolylamino-pyrido[2,3-b]-pyrazin-6-yl)-urea |
| **Cpd. 161** | 1-Ethyl-3-[3-(pyridin-3-ylamino)-pyrido-[2,3-b]pyrazin-6-yl]-urea |
| **Cpd. 162** | Ethyl-3-[3-(4-ethyl-phenylamino)-pyrido[2,3-b]pyrazin-6-yl]-urea |
| **Cpd. 163** | 1-Ethyl-3-[3-(3-methoxy-4-methyl-phenylamino)-pyrido[2,3-b]pyrazin-6-yl]-urea |
| **Cpd. 164** | 1-Ethyl-3-[3-(4-hydroxy-phenylamino)-pyrido[2,3-b]pyrazin-6-yl]-urea |
| **Cpd. 165** | 1-Ethyl-3-[3-(5-methyl-pyridin-2-yl-amino)-pyrido[2,3-b]pyrazin-6-yl]-urea |
| **Cpd. 166** | 1-Ethyl-3-[3-(1-methyl-1H-pyrazol-3-ylamino)-pyrido[2,3-b]pyrazin-6-yl]-urea |
| **Cpd. 167** | 1-Ethyl-3-[3-(4-fluoro-phenylamino)-pyrido[2,3-b]pyrazin-6-yl]-urea |
| **Cpd. 168** | 1-(4-Hydroxy-butyl)-3-[3-(4-hydroxy-3-methoxy-phenyl)-pyrido[2,3-b]pyrazin-6-yl]-urea |
| **Cpd. 169** | Phosphoric acid-mono-{4-[6-(3-ethyl-ureido)-pyrido-[2,3-b]pyrazin-3-yl]-2-methoxy-phenyl}-ester |
| **Cpd. 170** | 1-[3-(2-Chloro-4-hydroxy-phenyl)-pyrido[2,3-b]pyrazin-6-yl]-3-ethyl-urea |
| **Cpd. 171** | 1-[3-(4-Chloro-2-methoxy-phenyl)-pyrido[2,3-b]pyrazin-6-yl]-3-ethyl-urea |
| **Cpd. 172** | 1-[3-(3,5-Dimethyl-1H-pyrazol-4-yl)-pyrido[2,3-b]pyrazin-6-yl]-3-ethyl-urea |
| **Cpd. 173** | 1-Ethyl-3-[3-(1-methyl-1H-pyrazol-4-yl)-pyrido[2,3-b]pyrazin-6-yl]-urea |
| **Cpd. 174** | 1-[3-(5-Cyano-thiophen-2-yl)-pyrido[2,3-b]pyrazin-6-yl]-3-ethyl-urea |
| **Cpd. 175** | Sodium 2-chloro-4-[6-(3-ethyl-ureido)-pyrido[2,3-b]pyrazin-3-yl]-6-methoxy-phenolate |
| **Cpd. 176** | 1-[3-(4-Hydroxy-3-methoxy-phenyl)-pyrido[2,3-b]pyrazin-6-yl]-3-(4-morpholin-4-yl-butyl)-urea |
| **Cpd. 177** | 1-[3-(3-Chloro-4-methoxy-phenylamino)-pyrido[2,3-b]pyrazin-6-yl]-3-ethyl-urea |
| **Cpd. 178** | 1-Ethyl-3-[3-(naphthalin-2-ylamino)-pyrido[2,3-b]pyrazin-6-yl]-urea |
| **Cpd. 179** | 1-Ethyl-3-[3-(quinolin-3-ylamino)-pyrido[2,3-b]pyrazin-6-yl]-urea |
| **Cpd. 180** | 1-[3-(3,5-Dimethoxy-phenylamino)-pyrido[2,3-b]pyrazin-6-yl]-3-ethyl-urea |
| **Cpd. 181** | 1-Ethyl-3-[3-(pyrazin-2-ylamino)-pyrido[2,3-b]pyrazin-6-yl]-urea |
| **Cpd. 182** | 1-Ethyl-3-[3-(3-isopropoxy-phenylamino)-pyrido[2,3-b]pyrazin-6-yl]-urea |
| **Cpd. 183** | 1-Ethyl-3-[3-(4-hydroxy-3-methoxy-phenyl)-pyrido[2,3-b]pyrazin-6-yl]-urea p-Toluolsulfonate |

(continued)

| | |
|---|---|
| **Cpd. 184** | 1-[3-(2-Chloro-pyridin-4-ylamino)-pyrido[2,3-b]pyrazin-6-yl]-3-ethyl-urea |
| **Cpd. 185** | 1-[3-(3,5-Dichloro-4-hydroxy-phenylamino)-pyrido[2,3-b]pyrazin-6-yl]-3-ethyl-urea |
| **Cpd. 186** | 1-[3-(3,5-Dichloro-4-hydroxy-phenyl)-pyrido[2,3-b]pyrazin-6-yl]-3-ethyl-urea |
| **Cpd. 187** | 1-[3-(3,4-Dimethoxy-phenylamino)-pyrido[2,3-b]pyrazin-6-yl]-3-ethyl-urea |
| **Cpd. 188** | 1-Ethyl-3-[3-(3-hydroxy-4-methyl-phenylamino)-pyrido[2,3-b]pyrazin-6-yl]-urea |
| **Cpd. 189** | 3-[6-(3-Ethyl-ureido)-pyrido[2,3-b]-pyrazin-3-ylamino]-5-trifluoromethylbenzoic acid |
| **Cpd. 190** | 1-Ethyl-3-[3-(6-methoxy-pyridin-3-ylamino)-pyrido[2,3-b]pyrazin-6-yl]-urea |
| **Cpd. 191** | 1-[3-(3,5-Dimethyl-phenylamino)-pyrido[2,3-b]pyrazin-6-yl]-3-ethyl-urea |
| **Cpd. 192** | 1-[3-(4-Cyano-phenylamino)-pyrido-[2,3-b]pyrazin-6-yl]-3-ethyl-urea |
| **Cpd. 193** | 1-Ethyl-3-[3-(4-hydroxy-3-methoxy-phenyl)-pyrido[2,3-b]pyrazin-6-yl]-urea (Z)-but-2-endicarbonic acid salt |
| **Cpd. 194** | 1-Ethyl-3-[3-(4-hydroxy-3-methoxy-phenyl)-pyrido[2,3-b]pyrazin-6-yl]-urea Hydrochloride |
| **Cpd. 195** | 1-Ethyl-3-(3-phenyl-pyrido[2,3-b]pyrazin-6-yl)-thiourea |
| **Cpd. 196** | 1-(3-Chloro-pyrido[2,3-b]pyrazin-6-yl)-3-cyclohexyl-urea |
| **Cpd. 197** | 1-Ethyl-3-[3-(4-hydroxy-3-methoxy-phenylamino)-pyrido[2,3-b]pyrazin-6-yl]-urea |
| **Cpd. 198** | 1-Ethyl-3-[3-(4-hydroxy-3,5-dimethyl-phenyl)-pyrido[2,3-b]pyrazin-6-yl]-urea |
| **Cpd. 199** | 3-Ethyl-1-phenethyl-1-(3-phenyl-pyrido[2,3-b]pyrazin-6-yl)-urea |
| **Cpd. 200** | 1-Allyl-3-{3-[4-(tetrahydro-pyran-2-yloxy)-phenyl]-pyrido[2,3-b]pyrazin-6-yl}-thiourea |
| **Cpd. 201** | 3-Ethyl-1-[3-(4-methoxy-phenyl)-pyrido[2,3-b]pyrazin-6-yl]-1-propyl- urea |
| **Cpd. 202** | 3-Ethyl-1-(3-phenyl-pyrido[2,3-b]pyrazin-6-yl)-1-(2-piperidin-1-yl-ethyl)-urea Hydrochloride |
| **Cpd. 203** | N-{4-[6-(3-Ethyl-ureido)-pyrido[2,3-b]-pyrazin-3-yl]-phenyl}-2-phenyl acetamide |
| **Cpd. 204** | 1-(4-Hydroxy-butyl)-3-(3-phenyl-pyrido[2,3-b]pyrazin-6-yl)-urea Hydrochloride |
| **Cpd. 205** | Acetic acid-4-[3-(3-phenyl-pyrido[2,3-b]pyrazin-6-yl)-ureido]-butyl ester |
| **Cpd. 206** | 1-(4-Amino-butyl)-3-(3-phenyl-pyrido[2,3-b]pyrazin-6-yl)-urea |
| **Cpd. 207** | 1-(5-Hydroxy-pentyl)-3-(3-phenyl-pyrido[2,3-b]pyrazin-6-yl)-urea |
| **Cpd. 208** | 3-[6-(3-Ethyl-ureido)-pyrido[2,3-b]-pyrazin-3-yl]-N-methyl-benzamide |
| **Cpd. 209** | 1-Ethyl-3-[3-(2-methoxy-5-methyl-phenyl)-pyrido[2,3-b]pyrazin-6-yl]-urea |
| **Cpd. 210** | 1-Ethyl-3-(3-p-tolyl-pyrido[2,3-b]pyrazin-6-yl)-urea |
| **Cpd. 211** | 1-Ethyl-3-[3-(methyl-p-tolyl-amino)-pyrido[2,3-b]pyrazin-6-yl]-urea |
| **Cpd. 212** | 1-Ethyl-3-[3-(2-p-tolyl-ethylamino)-pyrido[2,3-b]pyrazin-6-yl]-urea |
| **Cpd. 213** | 1-Ethyl-3-[3-(4-methyl-benzylamino)-pyrido[2,3-b]pyrazin-6-yl]-urea |
| **Cpd. 214** | 1-Ethyl-3-[3-(3-fluoro-4-methyl-phenyl-amino)-pyrido[2,3-b]pyrazin-6-yl]-urea |
| **Cpd. 215** | 1-[3-(3,4-Dimethyl-phenylamino)-pyrido[2,3-b]pyrazin-6-yl]-3-ethyl-urea |
| **Cpd. 216** | 1-Ethyl-3-[3-(4-isopropyl-phenylamino)-pyrido[2,3-b]pyrazin-6-yl]-urea |
| **Cpd. 217** | 1-(4-Morpholin-4-yl-butyl)-3-(3-phenyl-pyrido[2,3-b]pyrazin-6-yl)-urea |
| **Cpd. 218** | N-{4-[3-(3-Phenyl-pyrido[2,3-b]pyrazin-6-yl)-ureido]-butyl}-acetamide |
| **Cpd. 219** | 1-[3-(3-Amino-4-methyl-phenyl)-pyrido[2,3-b]pyrazin-6-yl]-3-ethyl-urea |
| **Cpd. 220** | 1-[3-(3-Acetyl-2-fluoro-phenyl)-pyrido[2,3-b]pyrazin-6-yl]-3-ethyl-urea |
| **Cpd. 221** | 1-Ethyl-3-[3-(4-methoxy-3-methyl-phenyl)-pyrido[2,3-b]pyrazin-6-yl]-urea |

(continued)

| | |
|---|---|
| **Cpd. 222** | 1-[3-(6-Ethoxy-pyridin-3-yl)-pyrido[2,3-b]pyrazin-6-yl]-3-ethyl-urea |
| **Cpd. 223** | 1-Ethyl-3-[3-(2-fluoro-4-methyl-phenyl)-pyrido[2,3-b]pyrazin-6-yl]-urea |
| **Cpd. 224** | 1-Ethyl-3-[3-(3-fluoro-4-methoxy-phenyl)-pyrido[2,3-b]pyrazin-6-yl]-urea |
| **Cpd. 225** | 1-Ethyl-3-[3-(2-fluoro-3-methoxy-phenyl)-pyrido[2,3-b]pyrazin-6-yl]-urea |
| **Cpd. 226** | 1-Ethyl-3-[3-(3,4,5-trimethoxy-phenyl)-pyrido[2,3-b]pyrazin-6-yl]-urea |
| **Cpd. 227** | 1-[3-(3,5-Difluoro-2-methoxy-phenyl)-pyrido[2,3-b]pyrazin-6-yl]-3-ethyl-urea |
| **Cpd. 228** | 1-Ethyl-3-[3-(4-trifluoromethyl-phenyl)-pyrido[2,3-b]pyrazin-6-yl]-urea |
| **Cpd. 229** | 1-Ethyl-3-[3-(2,3,4-trimethoxy-phenyl)-pyrido[2,3-b]pyrazin-6-yl]-urea |
| **Cpd. 230** | 1-[3-(3-Chloro-4-methoxy-phenyl)-pyrido[2,3-b]pyrazin-6-yl]-3-ethyl-urea |
| **Cpd. 231** | 1-Ethyl-3-[3-(3-fluoro-4-hydroxy-phenyl)-pyrido[2,3-b]pyrazin-6-yl]-urea |
| **Cpd. 232** | 1-Ethyl-3-[3-(6-fluoro-pyridin-3-yl)-pyrido[2,3-b]pyrazin-6-yl]-urea |
| **Cpd. 233** | 1-[3-(2,4-Dimethyl-thiazol-5-yl)-pyrido-[2,3-b]pyrazin-6-yl]-3-ethyl-urea |
| **Cpd. 234** | 1-Ethyl-3-[3-(2-methoxy-phenyl)-pyrido[2,3-b]pyrazin-6-yl]-urea |
| **Cpd. 235** | 1-[3-(2-Chloro-pyridin-4-yl)-pyrido[2,3-b]pyrazin-6-yl]-3-ethyl-urea |
| **Cpd. 236** | 1-[3-(5-Acetyl-thiophen-2-yl)-pyrido[2,3-b]pyrazin-6-yl]-3-ethyl-urea |
| **Cpd. 237** | 1-[3-(5-Chloro-thiophen-2-yl)-pyrido[2,3-b]pyrazin-6-yl]-3-ethyl-urea |
| **Cpd. 238** | 1-Ethyl-3-[3-(3-methoxy-phenyl)-pyrido[2,3-b]pyrazin-6-yl]-urea |
| **Cpd. 239** | 1-[3-(3-Bromo-5-methoxy-phenyl)-pyrido[2,3-b]pyrazin-6-yl]-3-ethyl-urea |
| **Cpd. 240** | 1-[3-(Benzothiazol-6-ylamino)-pyrido[2,3-b]pyrazin-6-yl]-3-ethyl-urea |
| **Cpd. 241** | 1-Ethyl-3-[3-(4-methyl-3-trifluoromethyl-phenylamino)-pyrido[2,3-b]pyrazin-6-yl]-urea |
| **Cpd. 242** | 1-[3-(3-Cyano-4-methyl-phenylamino)-pyrido[2,3-b]pyrazin-6-yl]-3-ethyl-urea |
| **Cpd. 243** | 1-Ethyl-3-[3-(4-phenoxy-phenylamino)-pyrido[2,3-b]pyrazin-6-yl]-urea |
| **Cpd. 244** | 1-[3-(4-Chloro-3-methyl-phenylamino)-pyrido[2,3-b]pyrazin-6-yl]-3-ethyl-urea |
| **Cpd. 245** | 1-[3-(2-Chloro-4-methyl-phenylamino)-pyrido[2,3-b]pyrazin-6-yl]-3-ethyl-urea |
| **Cpd. 246** | 1-Ethyl-3-[3-(3-trifluoromethyl-phenylamino)-pyrido[2,3-b]pyrazin-6-yl]-urea |
| **Cpd. 247** | 1-[3-(2-Chloro-4-trifluoromethoxy-phenylamino)-pyrido[2,3-b]pyrazin-6-yl]-3-ethyl-urea |
| **Cpd. 248** | 1-[3-(4-Chloro-2-methoxy-5-methyl-phenylamino)-pyrido[2,3-b]pyrazin-6-yl]-3-ethyl-urea |
| **Cpd. 249** | 1-Ethyl-3-[3-(4-methylsulfanyl-phenyl)-pyrido[2,3-b]pyrazin-6-yl]-urea |
| **Cpd. 250** | 3-[6-(3-Ethyl-ureido)-pyrido[2,3-b]pyrazin-3-yl]-benzenesulfonamide |
| **Cpd. 251** | N-{4-[3-(3-Phenyl-pyrido[2,3-b]pyrazin-6-yl)-ureido]-butyl}-methansulfonamide |
| **Cpd. 252** | 1-[3-(Benzo[1,3]dioxol-5-ylamino)-pyrido[2,3-b]pyrazin-6-yl]-3-ethyl-urea |
| **Cpd. 253** | 3-[6-(3-Ethyl-ureido)-pyrido[2,3-b]pvrazin-3-ylamino]-benzoic acid |
| **Cpd. 254** | 2-Chloro-4-[6-(3-ethyl-ureido)-pyrido[2,3-b]pyrazin-3-ylamino]-benzoic acid |
| **Cpd. 255** | 1-Ethyl-3-[3-(3-methoxy-5-trifluoromethyl-phenylamino)-pyrido[2,3-b]pyrazin-6-yl]-urea |
| **Cpd. 256** | 1-Ethyl-3-[3-(pyrimidin-2-ylamino)-pyrido[2,3-b]pyrazin-6-yl]-urea |
| **Cpd. 257** | 6-[6-(3-Ethyl-ureido)-pyrido[2,3-b]pyrazin-3-ylamino]-naphthalin-2-carbonic acid |
| **Cpd. 258** | 1-Ethyl-3-[3-(4-hydroxy-quinolin-2-ylamino)-pyrido[2,3-b]pyrazin-6-yl]-urea |
| **Cpd. 259** | 1-Ethyl-3-[3-(quinolin-6-ylamino)-pyrido[2,3-b]pyrazin-6-yl]-urea |
| **Cpd. 260** | 1-Ethyl-3-[3-(3,4,5-trimethoxy-phenylamino)-pyrido[2,3-b]pyrazin-6-yl]-urea |

(continued)

| | |
|---|---|
| **Cpd. 261** | 1-Ethyl-3-[3-(1H-indol-6-ylamino)-pyrido[2,3-b]pyrazin-6-yl]-urea |
| **Cpd. 262** | 1-Ethyl-3-[3-(3-oxo-3,4-dihydro-2H-benzo[1,4]oxazin-7-ylamino)-pyrido[2,3-b]pyrazin-6-yl]-urea |
| **Cpd. 263** | 1-Ethyl-3-[3-(quinoxalin-6-ylamino)-pyrido[2,3-b]pyrazin-6-yl]-urea |
| **Cpd. 264** | 1-Ethyl-3-[3-(3-trifluoromethoxy-phenylamino)-pyrido[2,3-b]pyrazin-6-yl]-urea |
| **Cpd. 265** | 1-Ethyl-3-[3-(4-isopropoxy-phenylamino)-pyrido[2,3-b]pyrazin-6-yl]-urea |
| **Cpd. 266** | 1-[3-(Dibenzofuran-2-ylamino)-pyrido[2,3-b]pyrazin-6-yl]-3-ethyl-urea |
| **Cpd. 267** | 1-(6-Dimethylamino-hexyl)-3-[3-(4-hydroxy-3-methoxy-phenyl)-pyrido[2,3-b]pyrazin-6-yl]-urea |
| **Cpd. 268** | 1-(4-Dimethylamino-butyl)-3-[3-(4-hydroxy-3-methoxy-phenyl)-pyrido[2,3-b]pyrazin-6-yl]-urea |
| **Cpd. 269** | 1-[3-(4-Hydroxy-3-methoxy-phenyl)-pyrido[2,3-b]pyrazin-6-yl]-3-(5-morpholin-4-yl-pentyl)-urea |

## Revendications

1. Utilisation d'un composé selon la formule générale **(I)**

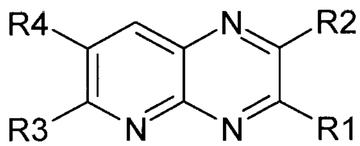

**(I)**

dans laquelle les substituants R1, R2, R3, R4 ont la signification suivante :

R1 et R2 peuvent représenter indépendamment l'un de l'autre :

(i) un atome d'hydrogène
(ii) un groupe hydroxy
(iii) un atome d'halogène
(iv) un radical alkyle, le radical alkyle étant saturé et pouvant être constitué de 1 à 8 atomes de carbone,
(v) un radical aryle substitué ou non substitué, le radical aryle pouvant porter un ou plusieurs substituants, identiques ou différents, choisis parmi F, Cl, Br, I, $CF_3$, CN, $NH_2$, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-alkyl-$NH_2$, NH-alkyl-OH, N(alkyle)$_2$, NHC(O)-alkyle, NHC(O)-cycloalkyle, NHC(O)-hétérocyclyle, NHC(O)-aryle, NHC(O)-hétéroaryle, NHC(O)-alkyl-aryle, NHC(O)-alkyl-hétéroaryle, $NHSO_2$-alkyle, $NHSO_2$-cycloalkyle, $NHSO_2$-hétérocyclyle, $NHSO_2$-aryle, $NHSO_2$-hétéroaryle, $NHSO_2$-alkyl-aryle, $NHSO_2$-alkyl-hétéroaryle, $NO_2$, SH, S-alkyle, S-aryle, S-hétéroaryle, OH, $OCF_3$, O-alkyle, O-cycloalkyle, O-hétérocyclyle, O-aryle, O-hétéroaryle, O-alkyl-cycloalkyle, O-alkyl-hétérocyclyle, O-alkyl-aryle, O-alkyl-hétéroaryle, O-alkyl-OH, O-$(CH_2)_n$-O, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, $OSO_3H$, $OSO_2$-alkyle, $OSO_2$-cycloalkyle, $OSO_2$-hétérocyclyle, $OSO_2$-aryle, $OSO_2$-hétéroaryle, $OSO_2$-alkyl-aryle, $OSO_2$-alkyl-hétéroaryle, OP(O)(OH)$_2$, C(O)-alkyle, C(O)-aryle, C(O)-hétéroaryle, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyle)$_2$, C(O)N(cycloalkyle)$_2$, C(O)N(aryle)$_2$, C(O)N(hétéroaryle)$_2$, SO-alkyle, SO-aryle, $SO_2$-alkyle, $SO_2$-aryle, $SO_2NH_2$, $SO_2NH$-alkyle, $SO_2NH$-aryle, $SO_2NH$-hétéroaryle, $SO_2NH$-alkyl-aryle, $SO_3H$, $SO_2O$-alkyle, $SO_2O$-aryle, $SO_2O$-alkyl-aryle, alkyle, cycloalkyle, hétérocyclyle, aryle ou hétéroaryle, n pouvant prendre la valeur 1, 2 ou 3, et les substituants alkyle, cycloalkyle, hétérocyclyle, aryle, hétéroaryle, alkyl-cycloalkyle, alkyl-hétérocyclyle, alkyl-aryle et alkyl-hétéroaryle pouvant à leur tour être substitués,
(vi) un radical hétéroaryle substitué ou non substitué, le radical hétéroaryle pouvant porter un ou plusieurs

substituants, identiques ou différents, choisis parmi F, Cl, Br, I, $CF_3$, CN, $NH_2$, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-alkyl-$NH_2$, NH-alkyl-OH, $N(alkyle)_2$, NHC(O)-alkyle, NHC(O)-cycloalkyle, NHC(O)-hétérocyclyle, NHC(O)-aryle, NHC(O)-hétéroaryle, NHC(O)-alkyl-aryle, NHC(O)-alkyl-hétéroaryle, $NHSO_2$-alkyle, $NHSO_2$-cycloalkyle, $NHSO_2$-hétérocyclyle, $NHSO_2$-aryle, $NHSO_2$-hétéroaryle, $NHSO_2$-alkyl-aryle, $NHSO_2$-alkyl-hétéroaryle, $NO_2$, SH, S-alkyle, S-aryle, S-hétéroaryle, OH, $OCF_3$, O-alkyle, O-cycloalkyle, O-aryle, O-hétéroaryle, O-alkyl-cycloalkyle, O-alkyl-hétérocyclyle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, $OSO_3H$, $OSO_2$-alkyle, $OSO_2$-cycloalkyle, $OSO_2$-hétérocyclyle, $OSO_2$-aryle, $OSO_2$-hétéroaryle, $OSO_2$-alkyl-aryle, $OSO_2$-alkyl-hétéroaryle, $OP(O)(OH)_2$, C(O)-alkyle, C(O)-aryle, C(O)-hétéroaryle, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, $C(O)N(alkyle)_2$, $C(O)N(cycloalkyle)_2$, $C(O)N(aryle)_2$, $C(O)N(hétéroaryle)_2$, $SO_2NH_2$, $SO_2NH$-alkyle, $SO_2NH$-aryle, $SO_2NH$-hétéroaryle, $SO_2NH$-alkyl-aryle, $SO_3H$, $SO_2O$-alkyle, $SO_2O$-aryle, $SO_2O$-alkyl-aryle, alkyle, cycloalkyle, hétérocyclyle, aryle ou hétéroaryle, et les substituants alkyle, cycloalkyle, hétérocyclyle, aryle et hétéroaryle pouvant à leur tour être substitués,

(vii) OR5, R5 pouvant être un substituant alkyle, cycloalkyle, hétérocyclyle, aryle, hétéroaryle, alkyl-cycloalkyle, alkyl-hétérocyclyle, alkyl-aryle ou alkyl-hétéroaryle, et les substituants alkyle, cycloalkyle, hétérocyclyle, aryle, hétéroaryle, alkyl-cycloalkyle, alkyl-hétérocyclyle, alkyl-aryle ou alkyl-hétéroaryle pouvant à leur tour être substitués,

(viii) SR6, R6 pouvant être un substituant alkyle, cycloalkyle, hétérocyclyle, aryle, hétéroaryle, alkyl-cycloalkyle, alkyl-hétérocyclyle, alkyl-aryle ou alkyl-hétéroaryle, et les substituants alkyle, cycloalkyle, hétérocyclyle, aryle et hétéroaryle, alkyl-cycloalkyle, alkyl-hétérocyclyle, alkyl-aryle ou alkyl-hétéroaryle pouvant à leur tour être substitués,

(ix) NR7R8, R7 et R8 pouvant représenter indépendamment l'un de l'autre un atome d'hydrogène, un substituant alkyle, cycloalkyle, hétérocyclyle, aryle, hétéroaryle, alkyl-cycloalkyle, alkyl-hétérocyclyle, alkyl-aryle ou alkyl-hétéroaryle, et les substituants alkyle, cycloalkyle, hétérocyclyle, aryle et hétéroaryle, alkyl-cycloalkyle, alkyl-hétérocyclyle, alkyl-aryle ou alkyl-hétéroaryle pouvant à leur tour être substitués, ou R7 et R8 représentent ensemble un groupe hétérocyclyle, le groupe hétérocyclyle pouvant à son tour être substitué,

R3 et R4 peuvent représenter indépendamment l'un de l'autre un atome d'hydrogène ou NR9R10, étant entendu que lorsque R3 = NR9R10, R4 est un atome d'hydrogène, et lorsque R4 = NR9R10, R3 est un atome d'hydrogène, R9 pouvant être un atome d'hydrogène, un substituant alkyle, cycloalkyle, hétérocyclyle, aryle, hétéroaryle, alkyl-cycloalkyle, alkyl-hétérocyclyle, alkyl-aryle ou alkyl-hétéroaryle, et les substituants alkyle, cycloalkyle, hétérocyclyle, aryle et hétéroaryle, alkyl-cycloalkyle, alkyl-hétérocyclyle, alkyl-aryle ou alkyl-hétéroaryle pouvant à leur tour être substitués,
etR10:

peut représenter -C(Y)NR11R12, Y étant O, S et R11 et R12 représentant indépendamment l'un de l'autre

(i) un atome d'hydrogène,

(ii) un radical alkyle substitué ou non substitué, le radical alkyle pouvant porter un ou plusieurs substituants identiques ou différents, choisis parmi F, Cl, Br, I, $CF_3$, CN, $NH_2$, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, $N(alkyle)_2$, NHC(O)-alkyle, NHC(O)-cycloalkyle, NHC(O)-hétérocyclyle, NHC(O)-aryle, NHC(O)-hétéroaryle, NHC(O)-alkyl-aryle, NHC(O)-alkyl-hétéroaryle, $NHSO_2$-alkyle, $NHSO_2$-cycloalkyle, $NHSO_2$-hétérocyclyle, $NHSO_2$-aryle, $NHSO_2$-hétéroaryle, $NHSO_2$-alkyl-aryle, $NHSO_2$-alkyl-hétéroaryle, $NO_2$, SH, S-alkyle, S-cycloalkyle, S-hétérocyclyle, S-aryle, S-hétéroaryle, OH, $OCF_3$, O-alkyle, O-cycloalkyle, O-hétérocyclyle, O-aryle, O-hétéroaryle, O-alkyl-cycloalkyle, O-alkyl-hétérocyclyle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, $OSO_3H$, $OSO_2$-alkyle, $OSO_2$-cycloalkyle, $OSO_2$-hétérocyclyle, $OSO_2$-aryle, $OSO_2$-hétéroaryle, $OSO_2$-alkyl-aryle, $OSO_2$-alkyl-hétéroaryle, $OP(O)(OH)_2$, C(O)-alkyle, C(O)-aryle, C(O)-hétéroaryle, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, $C(O)N(alkyle)_2$, $C(O)N(cycloalkyle)_2$, $C(O)N(aryle)_2$, $C(O)N(hétéroaryle)_2$, SO-alkyle, SO-aryle, $SO_2$-alkyle, $SO_2$-aryle, $SO_2NH_2$, $SO_2NH$-alkyle, $SO_2NH$-aryle, $SO_2NH$-hétéroaryle, $SO_2NH$-alkyl-aryle, $SO_3H$, $SO_2O$-alkyle, $SO_2O$-aryle, $SO_2O$-alkyl-aryle, cycloalkyle, hétérocyclyle, aryle ou hétéroaryle,

(iii) un radical cycloalkyle substitué ou non substitué, le radical cycloalkyle pouvant porter un ou plusieurs substituants identiques ou différents, choisis parmi F, Cl, Br, I, $NH_2$, NH-alkyle, NH-cycloalkyle, NH-hétérocyclyle, NH-aryle, NH-hétéroaryle, NH-alkyl-aryle, NH-alkyl-hétéroaryle, $N(alkyle)_2$, NHC(O)-alkyle, NHC(O)-cycloalkyle, NHC(O)-hétérocyclyle, NHC(O)-aryle, NHC(O)-hétéroaryle, NHC(O)-alkyl-aryle, NHC(O)-alkyl-hétéroaryle, $NHSO_2$-alkyle, $NHSO_2$-cycloalkyle, $NHSO_2$-hétérocyclyle, $NHSO_2$-aryle, $NHSO_2$-hétéroaryle, $NHSO_2$-alkyl-aryle, $NHSO_2$-alkyl-hétéroaryle, OH, O-alkyle, O-cycloalkyle, O-hétérocyclyle, O-aryle, O-hétéroaryle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, $OSO_3H$, $OSO_2$-alkyle, $OSO_2$-cycloalkyle, $OSO_2$-hétérocyclyle, $OSO_2$-aryle, $OSO_2$-hétéroaryle, $OSO_2$-alkyl-aryle, $OSO_2$-alkyl-hétéroaryle, $OP(O)(OH)_2$, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, $C(O)N(alkyle)_2$, $C(O)N(cycloalkyle)_2$, $C(O)N(aryle)_2$, $C(O)N(hétéroaryle)_2$, alkyle, ou aryle,

(iv) un radical hétérocyclyle substitué ou non substitué, le radical hétérocyclyle pouvant porter un ou plusieurs substituants, identiques ou différents, choisis parmi OH, O-alkyle, O-aryle, $NH_2$, NH-alkyle, NH-aryle, alkyle, alkyl-aryle ou aryle,

(v) un radical aryle substitué ou non substitué, le radical aryle pouvant porter un ou plusieurs substituants, identiques ou différents, choisis parmi F, Cl, Br, I, $CF_3$, CN, $NH_2$, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-alkyl-$NH_2$, NH-alkyl-OH, $N(alkyle)_2$, NHC(O)-alkyle, NHC(O)-cycloalkyle, NHC(O)-hétérocyclyle, NHC(O)-aryle, NHC(O)-hétéroaryle, NHC(O)-alkyl-aryle, NHC(O)-alkyl-hétéroaryle, $NHSO_2$-alkyle, $NHSO_2$-cycloalkyle, $NHSO_2$-hétérocyclyle, $NHSO_2$-aryle, $NHSO_2$-hétéroaryle, $NHSO_2$-alkyl-aryle, $NHSO_2$-alkyl-hétéroaryle, $NO_2$, SH, S-alkyle, S-cycloalkyle, S-hétérocyclyle, S-aryle, S-hétéroaryle, OH, $OCF_3$, O-alkyle, O-cycloalkyle, O-hétérocyclyle, O-aryle, O-hétéroaryle, O-alkyl-cycloalkyle, O-alkyl-hétérocyclyle, O-alkyl-aryle, O-alkyl-hétéroaryle, O-alkyl-OH, O-$(CH_2)_n$-O, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, $OSO_3H$, $OSO_2$-alkyle, $OSO_2$-cycloalkyle, $OSO_2$-hétérocyclyle, $OSO_2$-aryle, $OSO_2$-hétéroaryle, $OSO_2$-alkyl-aryle, $OSO_2$-alkyl-hétéroaryle, $OP(O)(OH)_2$, C(O)-alkyle, C(O)-aryle, C(O)-hétéroaryle, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, $C(O)N(alkyle)_2$, $C(O)N(cycloalkyle)_2$, $C(O)N(aryle)_2$, $C(O)N(hétéroaryle)_2$, SO-alkyle, SO-aryle, $SO_2$-alkyle, $SO_2$-aryle, $SO_2NH_2$, $SO_2NH$-alkyle, $SO_2NH$-aryle, $SO_2NH$-hétéroaryle, $SO_2NH$-alkyl-aryle, $SO_3H$, $SO_2O$-alkyle, $SO_2O$-aryle, $SO_2O$-alkyl-aryle, alkyle, cycloalkyle, hétérocyclyle, aryle ou hétéroaryle, et n pouvant prendre la valeur 1, 2 ou 3,

(vi) un radical hétéroaryle substitué ou non substitué, le radical hétéroaryle pouvant porter un ou plusieurs substituants, identiques ou différents, choisis parmi F, Cl, Br, I, $CF_3$, CN, $NH_2$, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-alkyl-$NH_2$, NH-alkyl-OH, $N(alkyle)_2$, NHC(O)-alkyle, NHC(O)-cycloalkyle, NHC(O)-hétérocyclyle, NHC(O)-aryle, NHC(O)-hétéroaryle, NHC(O)-alkyl-aryle, NHC(O)-alkyl-hétéroaryle, $NHSO_2$-alkyle, $NHSO_2$-cycloalkyle, $NHSO_2$-hétérocyclyle, $NHSO_2$-aryle, $NHSO_2$-hétéroaryle, $NHSO_2$-alkyl-aryle, $NHSO_2$-alkyl-hétéroaryle, $NO_2$, SH, S-alkyle, S-aryle, S-hétéroaryle, OH, $OCF_3$, O-alkyle, O-cycloalkyle, O-aryle, O-hétéroaryle, O-alkyl-cycloalkyle, O-alkyl-hétérocyclyle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, OC(O)-alkyl-aryle, OC(O)-alkyl-hétéroaryle, $OSO_3H$, $OSO_2$-alkyle, $OSO_2$-cycloalkyle, $OSO_2$-hétérocyclyle, $OSO_2$-aryle, $OSO_2$-hétéroaryle, $OSO_2$-alkyl-aryle, $OSO_2$-alkyl-hétéroaryle, $OP(O)(OH)_2$, C(O)-alkyle, C(O)-aryle, C(O)-hétéroaryle, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyle)$_2$, C(O)N(cycloalkyle)$_2$, C(O)N(aryle)$_2$, C(O)N(hétéroaryle)$_2$, $SO_2NH_2$, $SO_2$NH-alkyle, $SO_2$NH-aryle, $SO_2$NH-hétéroaryle, $SO_2$NH-alkyl-aryle, $SO_3H$, $SO_2$O-alkyle, $SO_2$O-aryle, $SO_2$O-alkyl-aryle, alkyle, cycloalkyle, hétérocyclyle, aryle ou hétéroaryle,

(vii) -C(O)-R17, R17 pouvant être un substituant alkyle, aryle ou hétéroaryle, et les substituants alkyle et aryle pouvant à leur tour être substitués,

(viii) ou R11 et R12 pouvant représenter ensemble un groupe hétérocyclyle,

peut représenter -C(Y)NR13R14, Y représentant NH et R13 et R14 pouvant représenter indépendamment l'un de l'autre

(i) un atome d'hydrogène,

(ii) un radical alkyle substitué ou non substitué, le radical pouvant porter un ou plusieurs substituants, identiques ou différents, choisis parmi F, Cl, Br, I, $CF_3$, CN, $NH_2$, NH-alkyle, NH-cycloalkyle, NH-hétérocyclyle, NH-aryle, NH-hétéroaryle, NH-alkyl-aryle, NH-alkyl-hétéroaryle, N(alkyle)$_2$, NHC(O)-alkyle, NHC(O)-cycloalkyle, NHC(O)-hétérocyclyle, NHC(O)-aryle, NHC(O)-hétéroaryle, $NHSO_2$-alkyle,$NHSO_2$-cycloalkyle, $NHSO_2$-aryle, $NHSO_2$-hétéroaryle, $NO_2$, SH, S-alkyle, S-cycloalkyle, S-hétérocyclyle, S-aryle, S-hétéroaryle, OH, $OCF_3$, O-alkyle, O-cycloalkyle, O-hétérocyclyle, O-aryle, O-hétéroaryle, O-alkyl-cycloalkyle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, $OSO_2$-alkyle, $OSO_2$-cycloalkyle, $OSO_2$-aryle, $OSO_2$-hétéroaryle, C(O)-alkyle, C(O)-aryle, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyle)$_2$, C(O)N(cycloalkyle)$_2$, C(O)N(aryle)$_2$, C(O)N(hétéroaryle)$_2$, SO-alkyle, SO-aryle, $SO_2$-alkyle, $SO_1$-aryle, $SO_2NH_2$, $SO_3H$, alkyle, cycloalkyle, hétérocyclyle, aryle ou hétéroaryle,

(iii) un radical cycloalkyle substitué ou non substitué, le radical cycloalkyle pouvant porter un ou plusieurs substituants, identiques ou différents, choisis parmi F, Cl, Br, I, $NH_2$, NH-alkyle, NH-cycloalkyle, NH-hétérocyclyle, NH-aryle, NH-hétéroaryle, NH-alkyl-aryle, NH-alkyl-hétéroaryle, N(alkyle)$_2$, NHC(O)-alkyle, NHC(O)-cycloalkyle, NHC(O)-hétérocyclyle, NHC(O)-aryle, NHC(O)-hétéroaryle, $NHSO_2$-alkyle,$NHSO_2$-cycloalkyle, $NHSO_2$-aryle, $NHSO_2$-hétéroaryle, OH, O-alkyle, O-cycloalkyle, O-hétérocyclyle, O-aryle, O-hétéroaryle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, $OSO_2$-alkyle, $OSO_2$-cycloalkyle, $OSO_2$-aryle, $OSO_2$-hétéroaryle, $CO_2H$, $CO_2$-alkyle, $CO_2$-cycloalkyle, $CO_2$-hétérocyclyle, $CO_2$-aryle, $CO_2$-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-aryle, C(O)NH-alkyl-hétéroaryle, C(O)N(alkyle)$_2$, alkyle, ou aryle,

(iv) un radical hétérocyclyle substitué ou non substitué, le radical hétérocyclyle pouvant porter un ou plusieurs substituants, identiques ou différents, choisis parmi OH, O-alkyle, O-aryle, $NH_2$, NH-alkyle, NH-aryle, alkyle, ou aryle,

(v) un radical aryle substitué ou non substitué, le radical aryle pouvant porter un ou plusieurs substituants, identiques ou différents, choisis parmi F, Cl, Br, I, $CF_3$, CN, $NH_2$, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-alkyl-$NH_2$, NH-alkyl-OH, N(alkyle)$_2$, NHC(O)-alkyle, NHC(O)-cycloalkyle, NHC(O)-hétérocyclyle, NHC(O)-aryle, NHC(O)-hétéroaryle, $NHSO_2$-alkyle,$NHSO_2$-aryle, $NHSO_2$-hétéroaryle, $NO_2$, SH, S-alkyle, S-cycloalkyle, S-hétérocyclyle, S-aryle, S-hétéroaryle, OH, $OCF_3$, O-alkyle, O-cycloalkyle, O-hétérocyclyle, O-aryle, O-hétéroaryle, O-alkyl-cycloalkyle, O-alkyl-hétérocyclyle, O-alkyl-aryle, O-alkyl-hétéroaryle, O-alkyl-OH, O-$(CH_2)_n$-O, OC(O)-alkyle, OC(O)-cycloalkyle, OC(O)-hétérocyclyle, OC(O)-aryle, OC(O)-hétéroaryle, $OSO_2$-alkyle, $OSO_2$-cycloalkyle, $OSO_2$-aryle, $OSO_2$-hétéroaryle, C(O)-alkyle, C(O)-aryle, C(O)-hétéroaryle, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyle)$_2$, C(O)N(cycloalkyle)$_2$, C(O)N(aryle)$_2$, C(O)N(hétéroaryle)$_2$, SO-alkyle, SO-aryle, $SO_2$-alkyle, $SO_2$-aryle, $SO_2NH_2$, $SO_2$NH-alkyle, $SO_2$NH-aryle, $SO_2$NH-hétéroaryle, $SO_3H$, $SO_2$O-alkyle, $SO_2$O-aryle, $SO_2$O-hétéroaryle, alkyle, cycloalkyle, hétérocyclyle, aryle ou hétéroaryle, et n pouvant prendre la valeur 1, 2 ou 3,

(vi) un radical hétéroaryle substitué ou non substitué, le radical hétéroaryle pouvant porter un ou plusieurs substituants, identiques ou différents, choisis parmi F, Cl, Br, I, $CF_3$, CN, $NH_2$, NH-alkyle, NH-cycloalkyle, NH-hétérocyclyle, NH-aryle, NH-hétéroaryle, NH-alkyl-aryle, NH-alkyl-hétéroaryle, N(alkyle)$_2$, NHC(O)-alkyle, NHC(O)-cycloalkyle, NHC(O)-hétérocyclyle, NHC(O)-aryle, NHC(O)-hétéroaryle, $NHSO_2$-alkyle,$NHSO_2$-aryle, $NHSO_2$-hétéroaryle, $NO_2$, SH, S-alkyle, S-aryle, OH, $OCF_3$, O-alkyle, O-cycloalkyle, O-hétérocyclyle, O-aryle, O-hétéroaryle, OC(O)-alkyle, OC(O)-cycloalkyle, OC(O)-hétérocyclyle, OC(O)-aryle, OC(O)-hétéroaryle, $OSO_2$-alkyle, $OSO_2$-cycloalkyle, $OSO_2$-aryle, $OSO_2$-hétéroaryle, C(O)-alkyle, C(O)-aryle, C(O)-hétéroaryle, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyle)$_2$, C(O)N(cycloalkyle)$_2$, C(O)N(aryle)$_2$, C(O)N(hétéroaryle)$_2$, $SO_2$-alkyle, $SO_2$-aryle, $SO_2NH_2$, $SO_2$NH-alkyle, $SO_2$NH-aryle, $SO_2$NH-hétéroaryle, $SO_3H$, $SO_2$O-alkyle, $SO_2$O-aryle, $SO_2$O-hétéroaryle, alkyle, cycloalkyle, hétérocyclyle, aryle ou hétéroaryle, ou R13 et R14 peuvent représenter ensemble un groupe hétérocyclyle,

peut représenter-C(NR15)R16, R15 représentant H et R16 pouvant représenter

(i) un radical alkyle substitué ou non substitué, le radical alkyle pouvant porter un ou plusieurs substituants, identiques ou différents, choisis parmi F, Cl, Br, I, $CF_3$, $NH_2$, NH-alkyle, NH-cycloalkyle, NH-hétérocyclyle, NH-aryle, NH-hétéroaryle, NH-alkyl-aryle, NH-alkyl-hétéroaryle, N(alkyle)$_2$, NHC(O)-alkyle, NHC(O)-cycloalkyle, NHC(O)-hétérocyclyle, NHC(O)-aryle, NHC(O)-hétéroaryle, $NHSO_2$-alkyle, $NHSO_2$-cycloalkyle, $NHSO_2$-aryle, $NHSO_2$-hétéroaryle, $NO_2$, SH, S-alkyle, S-cycloalkyle, S-hétérocyclyle, S-aryle, S-hétéroaryle, OH, $OCF_3$, O-alkyle, O-cycloalkyle, O-hétérocyclyle, O-aryle, O-hétéroaryle, O-alkyl-cycloalkyle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, $OSO_2$-alkyle, $OSO_2$-cycloalkyle, $OSO_2$-aryle, $OSO_2$-hétéroaryle, C(O)-alkyle, C(O)-aryle, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyle)$_2$, C(O)N(cycloalkyle)$_2$, C(O)N(aryle)$_2$, C(O)N(hétéroaryle)$_2$, SO-alkyle, SO-aryle, $SO_2$-alkyle, $SO_2$-aryle, $SO_2NH_2$, $SO_3H$, alkyle, cycloalkyle, hétérocyclyle, aryle ou hétéroaryle,
(ii) un radical cycloalkyle substitué ou non substitué, le radical cycloalkyle pouvant porter un ou plusieurs substituants, identiques ou différents, choisis parmi F, Cl, Br, I, $NH_2$, NH-alkyle, NH-cycloalkyle, NH-hétérocyclyle, NH-aryle, NH-hétéroaryle, NH-alkyl-aryle, NH-alkyl-hétéroaryle, N(alkyle)$_2$, NHC(O)-alkyle, NHC(O)-cycloalkyle, NHC(O)-hétérocyclyle, NHC(O)-aryle, NHC(O)-hétéroaryle, $NHSO_2$-alkyle,$NHSO_2$-cycloalkyle, $NHSO_2$-aryle, $NHSO_2$-hétéroaryle, OH, O-alkyle, O-cycloalkyle, O-hétérocyclyle, O-aryle, O-hétéroaryle, O-alkyl-aryle, O-alkyl-hétéroaryle, OC(O)-alkyle, OC(O)-cycloalkyle, OC(O)-hétérocyclyle, OC(O)-aryle, OC(O)-hétéroaryle, $OSO_2$-alkyle, $OSO_2$-cycloalkyle, $OSO_2$-aryle, $OSO_2$-hétéroaryle, $CO_2H$, $CO_2$-alkyle, $CO_2$-cycloalkyle, $CO_2$-hétérocyclyle, $CO_2$-aryle, $CO_2$-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-aryle, C(O)NH-alkyl-hétéroaryle, C(O)N(alkyle)$_2$, alkyle, ou aryle,
(iii) un radical hétérocyclyle substitué ou non substitué, le radical hétérocyclyle pouvant porter un ou plusieurs substituants, identiques ou différents, choisis parmi OH, O-alkyle, O-aryle, $NH_2$, NH-alkyle, NH-aryle, alkyle ou aryle,
(iv) un radical aryle substitué ou non substitué, le radical aryle pouvant porter un ou plusieurs substituants, identiques ou différents, choisis parmi F, Cl, Br, I, $CF_3$, $NH_2$, NH-alkyle, NH-cycloalkyle, NH-hétérocyclyle, NH-aryle, NH-hétéroaryle, NH-alkyl-cycloalkyle, NH-alkyl-hétérocyclyle, NH-alkyl-aryle, NH-alkyl-hétéroaryle, NH-alkyl-$NH_2$, NH-alkyl-OH, N(alkyle)$_2$, NHC(O)-alkyle, NHC(O)-cycloalkyle, NHC(O)-hétérocyclyle, NHC(O)-aryle, NHC(O)-hétéroaryle, $NHSO_2$-alkyle, $NHSO_2$-aryle, $NHSO_2$-hétéroaryle, $NO_2$, SH, S-alkyle, S-cycloalkyle, S-hétérocyclyle, S-aryle, S-hétéroaryle, OH, $OCF_3$, 0-alkyle, O-cycloalkyle, O-hétérocyclyle, O-aryle, O-hétéroaryle, O-alkyl-cycloalkyle, O-alkyl-hétérocyclyle, O-alkyl-aryle, O-alkyl-hétéroaryle, O-alkyl-OH, O-$(CH_2)_n$-O, OC(O)-alkyle, OC(O)-cycloalkyle, OC(O)-hétérocyclyle, OC(O)-aryle, OC(O)-hétéroaryle, $OSO_2$-alkyle, $OSO_2$-cycloalkyle, $OSO_2$-aryle, $OSO_2$-hétéroaryle, C(O)-alkyle, C(O)-aryle, C(O)-hétéroaryle, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyle)$_2$, C(O)N(cycloalkyle)$_2$, C(O)N(aryle)$_2$, C(O)N(hétéroaryle)$_2$, SO-alkyle, SO-aryle, $SO_2$-alkyle, $SO_2$-aryle, $SO_2NH_2$, $SO_2$NH-alkyle, $SO_2$NH-aryle, $SO_2$NH-hétéroaryle, $SO_3H$, $SO_2$O-alkyle, $SO_2$O-aryle, $SO_2$O-hétéroaryle, alkyle, cycloalkyle, hétérocyclyle, aryle ou hétéroaryle, et n pouvant prendre la valeur 1, 2 ou 3,
(v) un radical hétéroaryle substitué ou non substitué, le radical hétéroaryle pouvant porter un ou plusieurs substituants, identiques ou différents, choisis parmi F, Cl, Br, I, $CF_3$, $NH_2$, NH-alkyle, NH-cycloalkyle, NH-hétérocyclyle, NH-aryle, NH-hétéroaryle, NH-alkyl-aryle, NH-alkyl-hétéroaryle, N(alkyle)$_2$, NHC(O)-alkyle, NHC(O)-cycloalkyle, NHC(O)-hétérocyclyle, NHC(O)-aryle, NHC(O)-hétéroaryle, $NHSO_2$-alkyle, $NHSO_2$-aryle, $NHSO_2$-hétéroaryle, $NO_2$, SH, S-alkyle, S-aryle, OH, $OCF_3$, O-alkyle, O-cycloalkyle, O-hétérocyclyle, O-aryle, O-hétéroaryle, OC(O)-alkyle, OC(O)-cycloalkyle, OC(O)-hétérocyclyle, OC(O)-aryle, OC(O)-hétéroaryle, $OSO_2$-alkyle, $OSO_2$-cycloalkyle, $OSO_2$-aryle, $OSO_2$-hétéroaryle, C(O)-alkyle, C(O)-aryle, C(O)-hétéroaryle, $CO_2H$, $CO_2$-alkyle, $CO_2$-cycloalkyle, $CO_2$-hétérocyclyle, $CO_2$-aryle, $CO_2$-hétéroaryle, $CO_2$-alkyl-cycloalkyle, $CO_2$-alkyl-hétérocyclyle, $CO_2$-alkyl-aryle, $CO_2$-alkyl-hétéroaryle, C(O)-$NH_2$, C(O)NH-alkyle, C(O)NH-cycloalkyle, C(O)NH-hétérocyclyle, C(O)NH-aryle, C(O)NH-hétéroaryle, C(O)NH-alkyl-cycloalkyle, C(O)NH-alkyl-hétérocyclyle, C(O)NH-alkyl-aryle, C(O)NH-alkyl-hétéroaryle, C(O)N(alkyle)$_2$, C(O)N(cycloalkyle)$_2$, C(O)N(aryle)$_2$, C(O)N(hétéroaryle)$_2$, $SO_2$-alkyle, $SO_2$-aryle, $SO_2NH_2$, $SO_2$NH-alkyle, $SO_2$NH-aryle, $SO_2$NH-hétéroaryle, $SO_3H$, $SO_2$O-alkyle, $SO_2$O-aryle, $SO_2$O-hétéroaryle, alkyle, cycloalkyle, hétérocyclyle, aryle ou hétéroaryle ;

pour la fabrication d'un médicament destiné au traitement ou à la prophylaxie d'états physiologiques et/ou pathophysiologiques chez des mammifères les états physiologiques et/ou pathophysiologiques étant choisis parmi :

des affections rhumatismales, des affections arthritiques, des infections à VIH, le rhumatisme, l'arthrite, le SIDA, l'ARC (complexe associé au SIDA), le sarcome de Kaposi, des tumeurs partant du cerveau, la maladie d'Alzheimer, une tumeur du côlon, une tumeur de l'intestin, une tumeur du pancréas, la leucémie, une tumeur du foie, une tumeur du rein, une tumeur de la tête, une tumeur du cou, un gliome, le cancer de l'utérus, le cancer de l'endomètre, le cancer du col utérin, une tumeur cérébrale, un adénocanthome, une tumeur gastrique, une tumeur colorectale, le cancer de l'oesophage, le cancer de la thyroïde, un lymphome, la leucémie chronique, la leucémie aiguë, le diabète, la polyarthrite rhumatoïde, les allergies, les maladies allergiques, les maladies du myocarde, l'obésité, la surcharge pondérale, l'hypertension.

**2.** Utilisation selon la revendication 1, dans laquelle le radical alkyle est choisi dans le groupe constitué par : « méthyle, éthyle, n-propyle, 2-propyle, n-butyle, sec-butyle, tert-butyle, n-pentyle, isopentyle, néopentyle, n-hexyle, 2-hexyle, n-octyle, éthylényle (vinyle), éthynyle, propényle ($-CH_2CH=CH_2$, $-CH=CH-CH_3$, $-C(=CH_2)-CH_3$), propynyle ($-CH_2-C\equiv CH$, $-C\equiv C-CH_3$), butényle, butynyle, pentényle, pentynyle, hexényle, hexynyle, heptényle, heptynyle, octényle, octynyle ».

**3.** Utilisation selon la revendication 1, dans lequel le radical hétérocyclyle est choisi dans le groupe constitué par : « tétrahydrofuryle, tétrahydropyranyle, pyrrolidinyle, pipéridinyle, pipérazinyle, morpholinyle ».

**4.** Utilisation selon la revendication 1, dans lequel le radical hétéroaryle est choisi dans le groupe constitué par : « pyrrolyle, furyle, thiényle, thiazolyle, oxazolyle, isoxazolyle, pyrazolyle, imidazolyle, pyridinyle, pyrimidinyle, pyridazinyle, pyrazinyle, phtalazinyle, indolyle, indazolyle, indolizinyle, quinolinyle, isoquinolinyle, quinoxalinyle, quinazolinyle, carbazolyle, phénazinyle, phénothiazinyle, acridinyle ».

**5.** Utilisation selon la revendication 1, dans laquelle le composé est choisi dans le groupe constitué par :

| | | |
|---|---|---|
| **Composé 1** | 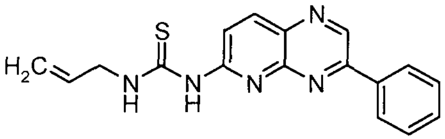 | 1-allyl-3-(3-phényl-pyrido[2,3-b] pyrazin-6-yl)-thiourée |
| **Composé 2** | 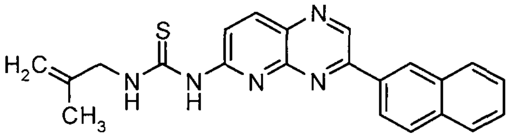 | 1-(2-méthyl-allyl)-3-(3-napht-2-yl-pyrido[2,3-b] pyrazin-6-yl)-thiourée |
| **Composé 3** | 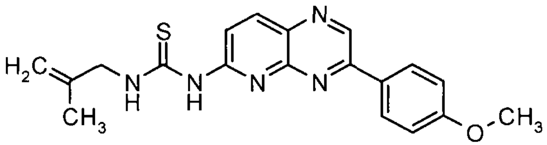 | 1-[3-(4-méthoxy-phényl)-pyrido[2,3-b] pyrazin-6-yl]-3-(2-méthyl-allyl)-thiourée |
| **Composé 4** | 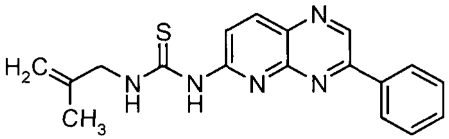 | 1-(2-méthyl-allyl)-3-(3-phényl-pyrido[2,3-b] pyrazin-6-yl)-thiourée |
| **Composé 5** | 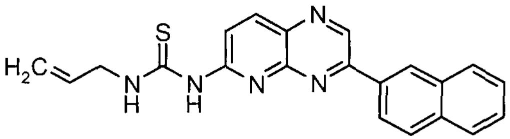 | 1-allyl-3-(3-napht-2-yl-pyrido[2,3-b] pyrazin-6-yl)-thiourée |
| **Composé 6** | 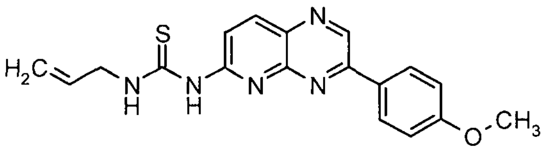 | 1-allyl-3-[3-(4-méthoxy-phényl)-pyrido[2,3-b]pyrazin-6-yl]-thiourée |
| **Composé 7** | 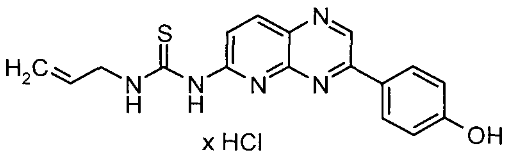 x HCl | 1-allyl-3-[3-(4-hydroxy-phényl)-pyrido[2,3-b] pyrazin-6-yl]-thiourée chlorhydrate |

(suite)

| | | |
|---|---|---|
| **Composé 8** | 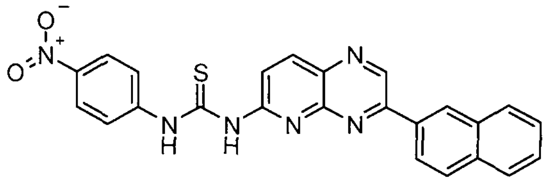 | 1-(3-napht-2-yl-pyrido[2,3-b]pyrazin-6-yl)-3-(4-nitro-phényl)-thiourée |
| **Composé 9** | 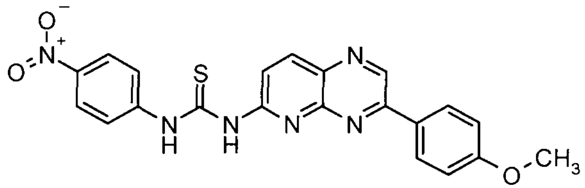 | 1-[3-(4-méthoxy-phényl)-pyrido[2,3-b]pyrazin-6-yl]-3-(4-nitro-phényl)-thiourée |
| **Composé 10** | 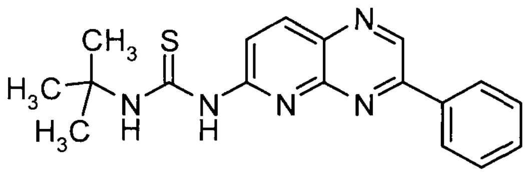 | 1-tert-butyl-3-(3-phényl-pyrido[2,3-b]pyrazin-6-yl)-thiourée |
| **Composé 11** | 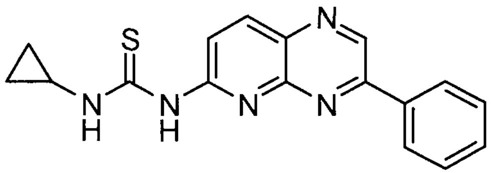 | 1-cyclopropyl-3-(3-phényl-pyrido[2,3-b]pyrazin-6-yl)-thiourée |
| **Composé 12** | 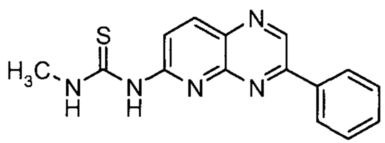 | 1-méthyl-3-(3-phényl-pyrido[2,3-b]pyrazin-6-yl)-thiourée |
| **Composé 13** | 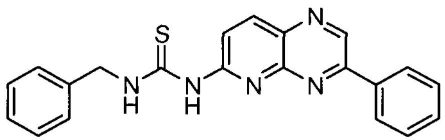 | 1-benzyl-3-(3-phényl-pyrido[2,3-b]pyrazin-6-yl)-thiourée |

(suite)

| | | |
|---|---|---|
| **Composé 14** | 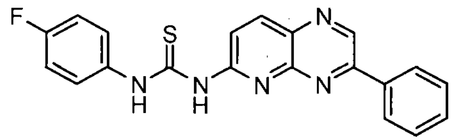 | 1-(4-fluoro-phényl)-3-(3-phényl-pyrido[2,3-b]pyrazin-6-yl)-thiourée |
| **Composé 15** | 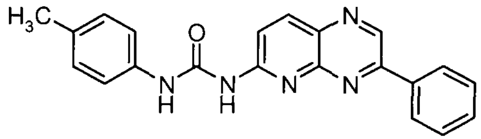 | 1-(3-phényl-pyrido[2,3-b]-pyrazin-6-yl)-3-p-tolyl-urée |
| **Composé 16** | 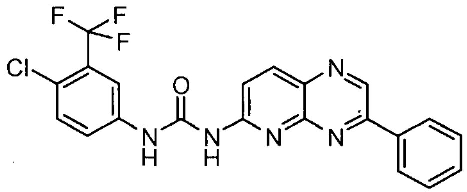 | 1-(4-chloro-3-trifluorométhyl-phényl)-3-(3-phényl-pyrido[2,3-b]pyrazin-6-yl)-urée |
| **Composé 17** | 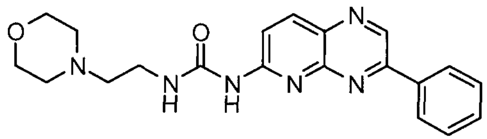 | 1-(2-morpholin-4-yl-éthyl)-3-(3-phényl-pyrido[2,3-b]pyrazin-6-yl)-urée |
| **Composé 18** | 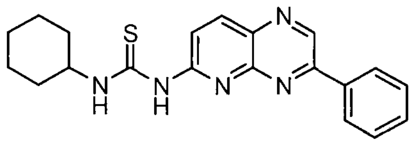 | 1-cyclohexyl-3-(3-phényl-pyrido[2,3-b]pyrazin-6-yl)-thiourée |
| **Composé 19** | 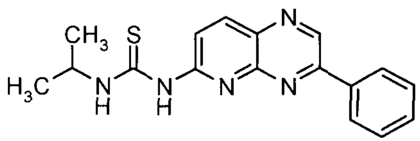 | 1-isopropyl-3-(3-phényl-pyrido[2,3-b]pyrazin-6-yl)-thiourée |
| **Composé 20** | 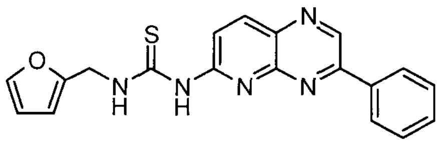 | 1-furan-2-ylméthyl-3-(3-phényl-pyrido[2,3-b]pyrazin-6-yl)-thiourée |

(suite)

| | | |
|---|---|---|
| **Composé 21** | 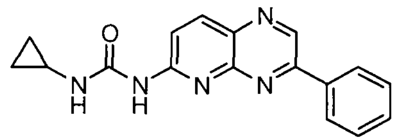 | 1-cyclopropyl-3-(3-phényl-pyrido[2,3-b]pyrazin-6-yl)-urée |
| **Composé 22** | 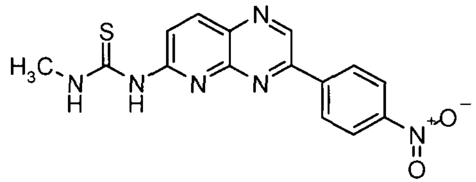 | 1-méthyl-3-[3-(4-nitro-phényl)-pyrido[2,3-b]pyrazin-6-yl]-thiourée |
| **Composé 23** | 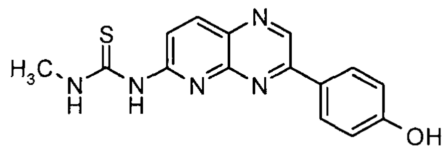 | 1-[3-(4-hydroxy-phényl)-pyrido[2,3-b]pyrazin-6-yl]-3-méthyl-thiourée |
| **Composé 24** | 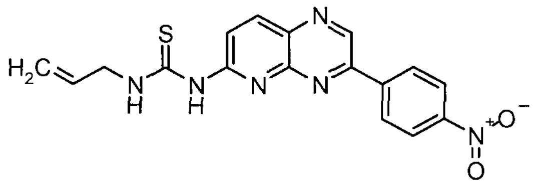 | 1-allyl-3-[3-(4-nitro-phényl)-pyrido[2,3-b]-pyrazin-6-yl]-thiourée |
| **Composé 26** | 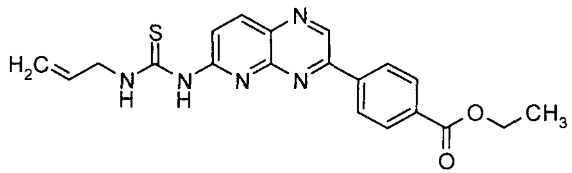 | 4-[6-(3-allyl-thiouréido)-pyrido[2,3-b]pyrazin-3-yl]-benzoate d'éthyle |
| **Composé 27** | 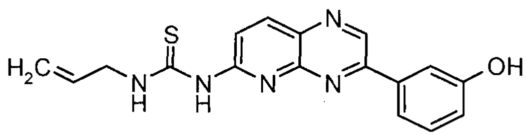 | 1-allyl-3-[3-(3-hydroxy-phényl)-pyrido[2,3-b]pyrazin-6-yl]-thiourée |

(suite)

| | | |
|---|---|---|
| **Composé 28** | 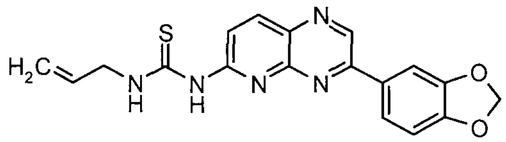 | 1-allyl-3-(3-benzo[1,3]dioxol-5-yl-pyrido[2,3-b]pyrazin-6-yl)-thiourée |
| **Composé 29** | 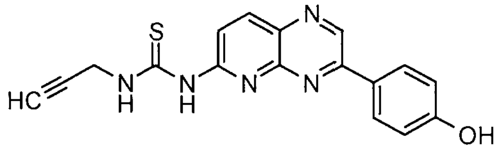 | 1-[3-(4-hydroxy-phényl)-pyrido[2,3-b]-pyrazin-6-yl]-3-prop-2-ynyl-thiourée |
| **Composé 31** | 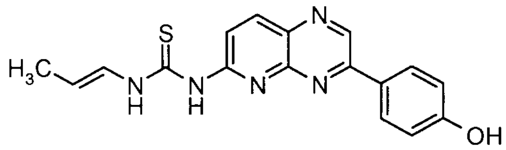 | 1-[3-(4-hydroxy-phényl)-pyrido[2,3-b]-pyrazin-6-yl]-3-((E)-propényl)-thiourée |
| **Composé 32** | 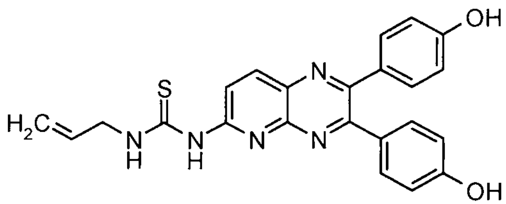 | 1-allyl-3-[2,3-bis-(4-hydroxy-phényl)-pyrido[2,3-b]pyrazin-6-yl]-thiourée |
| **Composé 33** | 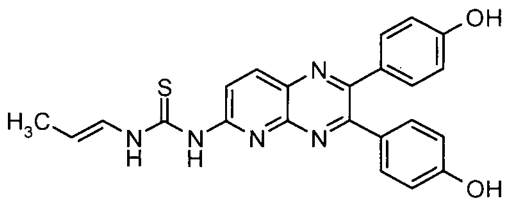 | 1-[2,3-bis-(4-hydroxy-phényl)-pyrido[2, 3-b]-pyrazin-6-yl]-3-((E)-propényl)-thiourée |
| **Composé 34** | 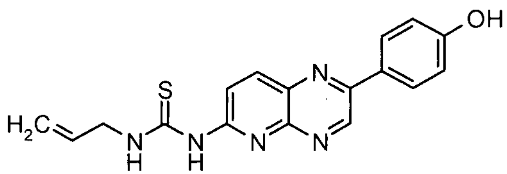 | 1-allyl-3-[2-(4-hydroxy-phényl)-pyrido[2,3-b]pyrazin-6-yl]-thiourée |

(suite)

| | | |
|---|---|---|
| **Composé 35** | 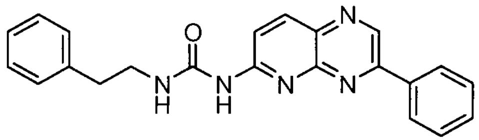 | 1-phénéthyl-3-(3-phényl-pyrido[2,3-b]-pyrazin-6-yl)-urée |
| **Composé 36** | 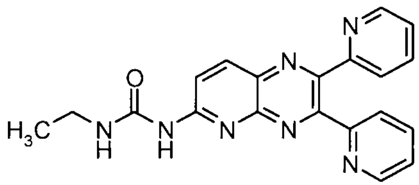 | 1-(2,3-di-pyridin-2-yl-pyrido[2,3-b]pyrazin-6-yl)-3-éthyl-urée |
| **Composé 37** | 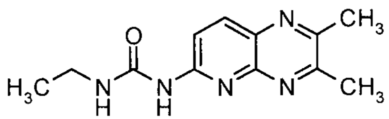 | 1-(2,3-diméthyl-pyrido[2,3-b]pyrazin-6-yl)-3-éthyl-urée |
| **Composé 38** | 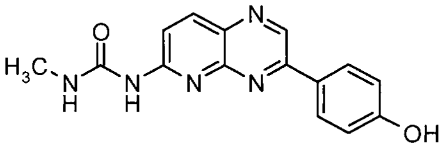 | 1-[3-(4-hydroxy-phényl)-pyrido[2,3-b]-pyrazin-6-yl]-3-méthyl-urée |
| **Composé 39** | 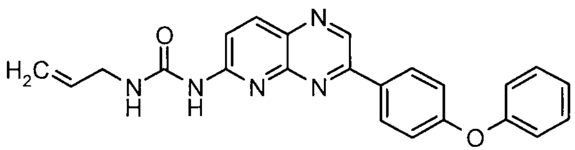 | 1-allyl-3-[3-(4-phénoxy-phényl)-pyrido[2,3-b]pyrazin-6-yl]-urée |
| **Composé 40** | 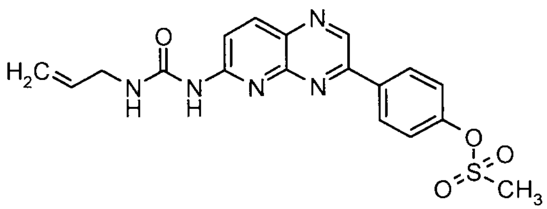 | méthanesulfonate de 4-[6-(3-allyl-uréido)-pyrido[2,3-b] pyrazin-3-yl]-phényle |

(suite)

| | | |
|---|---|---|
| **Composé 41** | 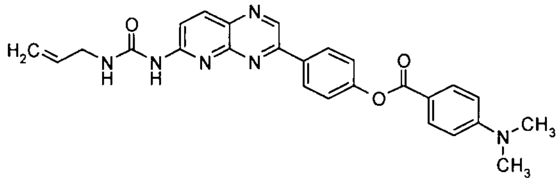 | 4-diméthyl-aminobenzoate de 4-[6-(3-allyl-uréido)-pyrido[2,3-b]pyrazin-3-yl]-phényle |
| **Composé 42** | 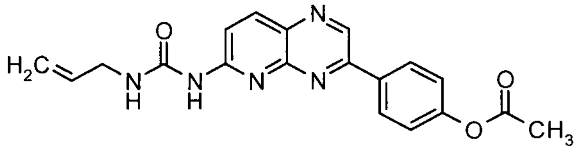 | acétate de 4-[6-(3-allyl-uréido)-pyrido[2,3-b]pyrazin-3-yl]-phényle |
| **Composé 43** | 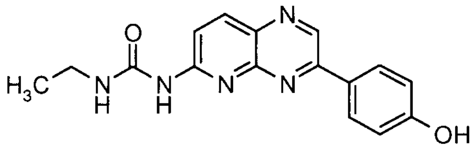 | 1-éthyl-3-[3-(4-hydroxy-phényl)-pyrido[2,3-b]pyrazin-6-yl]-urée |
| **Composé 44** | 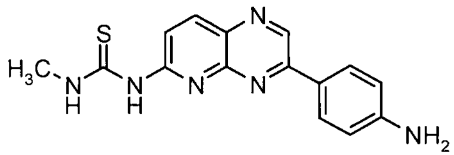 | 1-[3-(4-amino-phényl)-pyrido[2,3-b]-pyrazin-6-yl]-3-méthyl-thiourée |
| **Composé 45** | 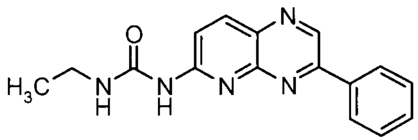 | 1-éthyl-3-(3-phényl-pyrido[2,3-b]pyrazin-6-yl)-urée |
| **Composé 46** | 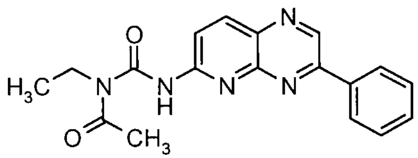 | 1-acétyl-1-éthyl-3-(3-phényl-pyrido[2,3-b]-pyrazin-6-yl)-urée |

(suite)

| | | |
|---|---|---|
| **Composé 47** | 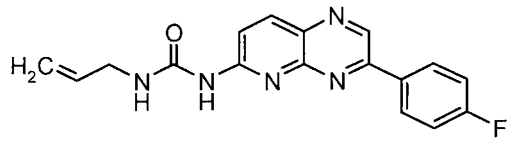 | 1-allyl-3-[3-(4-fluoro-phényl)-pyrido[2,3-b]-pyrazin-6-yl]-urée |
| **Composé 48** | 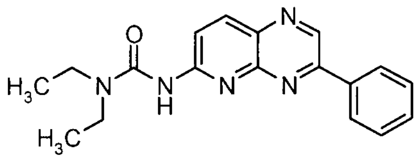 | 1,1-diéthyl-3-(3-phényl-pyrido[2,3-b]-pyrazin-6-yl)-urée |
| **Composé 49** | 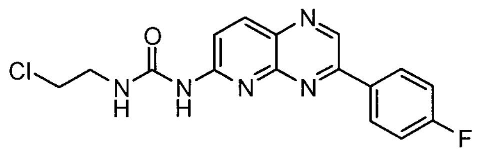 | 1-(2-chloro-éthyl)-3-[3-(4-fluoro-phényl)-pyrido[2,3-b]pyrazin-6-yl]-urée |
| **Composé 50** | 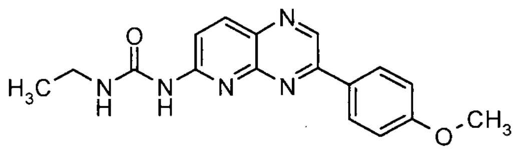 | 1-éthyl-3-[3-(4-méthoxy-phényl)-pyrido[2,3-b]pyrazin-6-yl]-urée |
| **Composé 51** | 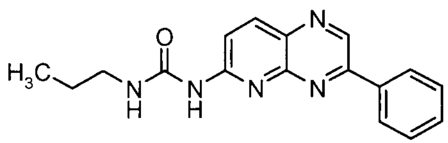 | 1-(3-phényl-pyrido[2,3-b] pyrazin-6-yl)-3-propyl-urée |
| **Composé 52** | 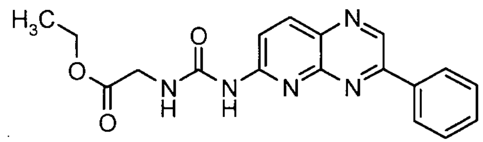 | [3-(3-phényl-pyrido[2,3-b]pyrazin-6-yl)-uréido]-acétate d'éthyle |
| **Composé 53** | 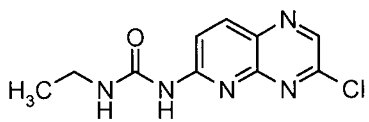 | 1-(3-chloro-pyrido[2,3-b]pyrazin-6-yl)-3-éthyl-urée |

(suite)

| | | |
|---|---|---|
| **Composé 54** | 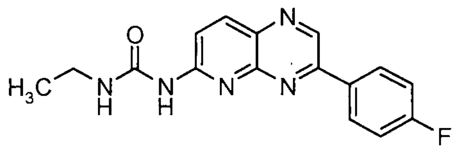 | 1-éthyl-3-[3-(4-fluoro-phényl)-pyrido[2,3-b]-pyrazin-6-yl]-urée |
| **Composé 55** | 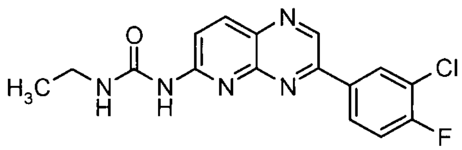 | 1-[3-(3-chloro-4-fluoro-phényl)-pyrido[2,3-b]pyrazin-6-yl]-3-éthyl-urée |
| **Composé 56** | 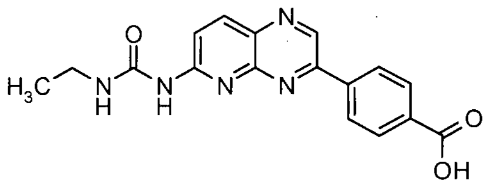 | acide 4-[6-(3-éthyl-uréido)-pyrido[2,3-b]-pyrazin-3-yl]-benzoïque |
| **Composé 57** | 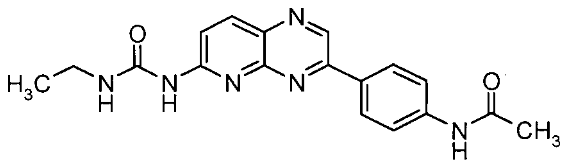 | N-{4-[6-(3-éthyl-uréido)-pyrido[2,3-b]-pyrazin-3-yl]-phényl}-acétamide |
| **Composé 58** | 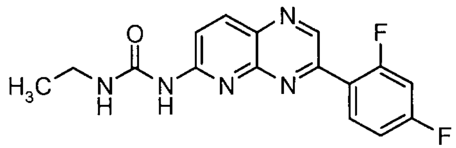 | 1-[3-(2,4-difluoro-phényl)-pyrido[2,3-b]-pyrazin-6-yl]-3-éthyl-urée |
| **Composé 59** | 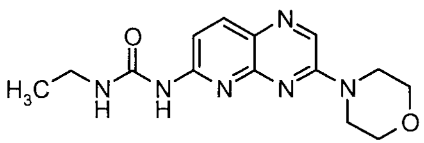 | 1-éthyl-3-(3-morpholin-4-yl-pyrido[2,3-b]pyrazin-6-yl)-urée |

(suite)

| | | |
|---|---|---|
| **Composé 60** | 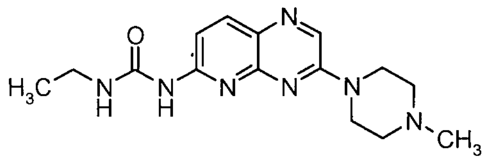 | 1-éthyl-3-[3-(4-méthyl-pipérazin-1-yl)-pyrido[2,3-b]-pyrazin-6-yl]-urée |
| **Composé 61** | 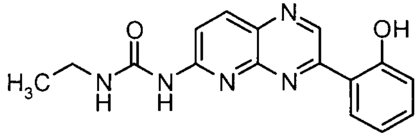 | 1-éthyl-3-[3-(2-hydroxy-phényl)-pyrido[2,3-b] pyrazin-6-yl]-urée |
| **Composé 62** | 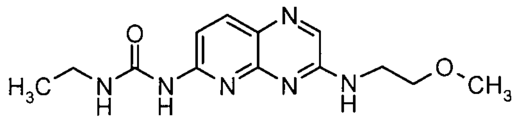 | 1-éthyl-3-[3-(2-méthoxy-éthylamino)-pyrido[2,3-b]pyrazin-6-yl]-urée |
| **Composé 63** | 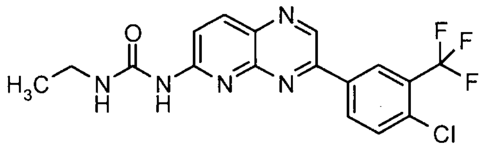 | 1-[3-(4-chloro-3-trifluorométhyl-phényl)-pyrido[2,3-b]-pyrazin-6-yl]-3-éthyl-urée |
| **Composé 64** | 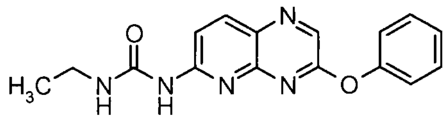 | 1-éthyl-3-(3-phénoxy-pyrido[2,3-b]pyrazin-6-yl)-urée |
| **Composé 65** | 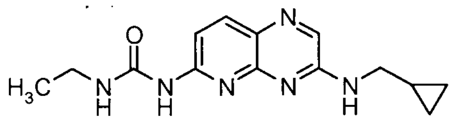 | 1-[3-(cyclopropyl-méthyl-amino)-pyrido[2,3-b]pyrazin-6-yl]-3-éthyl-urée |
| **Composé 66** | 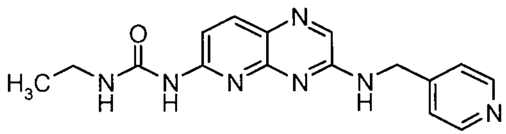 | 1-éthyl-3-{3-[(pyridin-4-ylméthyl)-amino]- |

(suite)

| | | |
|---|---|---|
| **Composé 67** | 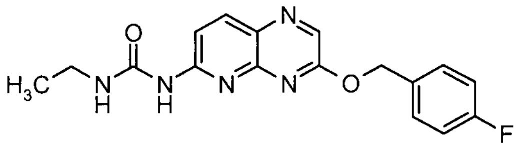 | 1-éthyl-3-[3-(4-fluoro-benzyloxy)-pyrido[2,3-b]pyrazin-6-yl]-urée |
| **Composé 68** | 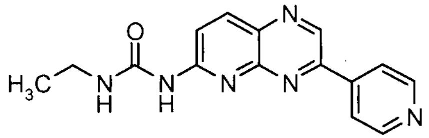 | 1-éthyl-3-(3-pyridin-4-yl-pyrido[2,3-b]-pyrazin-6-yl)-urée |
| **Composé 69** | 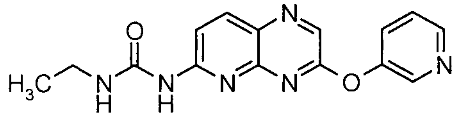 | 1-éthyl-3-[3-(pyridin-3-yloxy)-pyrido[2,3-b]-pyrazin-6-yl]-urée |
| **Composé 70** | 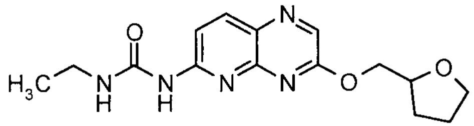 | 1-éthyl-3-[3-(tétrahydro-furan-2-ylméthoxy)-pyrido[2,3-b]pyrazin-6-yl]-urée |
| **Composé 71** | 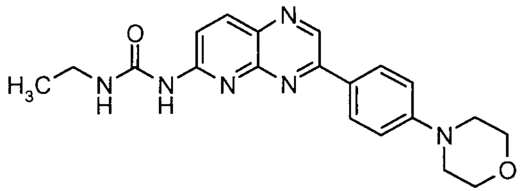 | 1-éthyl-3-[3-(4-morpholin-4-yl-phényl)-pyrido[2,3-b] pyrazin-6-yl]-urée |
| **Composé 72** | 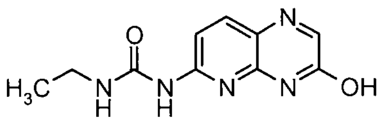 | 1-éthyl-3-(3-hydroxy-pyrido[2,3-b]pyrazin-6-yl)-urée |
| **Composé 73** | 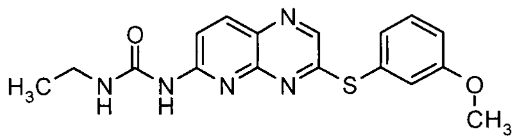 | 1-éthyl-3-[3-(3-méthoxy-phényl-sulfanyl)-pyrido[2,3-b]pyrazin-6-yl]-urée |

(suite)

| | | |
|---|---|---|
| **Composé 74** | 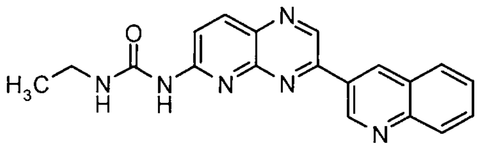 | 1-éthyl-3-(3-quinolin-3-yl-pyrido[2, 3-b]-pyrazin-6-yl)-urée |
| **Composé 75** | 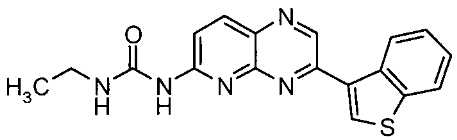 | 1-(3-benzo[b]-thiophén-3-yl-pyrido[2,3-b] pyrazin-6-yl)-3-éthyl-urée |
| **Composé 76** | 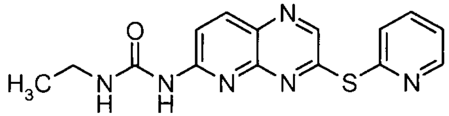 | 1-éthyl-3-[3-(pyridin-2-ylsulfanyl)-pyrido[2,3-b]pyrazin-6-yl]-urée |
| **Composé 77** | 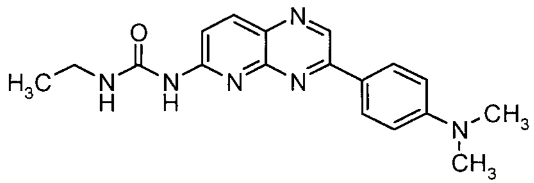 | 1-[3-(4-diméthylamino-phényl)-pyrido[2,3-b]-pyrazin-6-yl]-3-éthyl-urée |
| **Composé 78** | 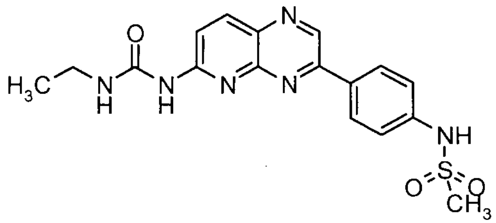 | N-{4-[6-(3-éthyl-uréido)-pyrido[2,3-b]-pyrazin-3-yl]-phényl}-méthanesulfonamide |
| **Composé 79** | 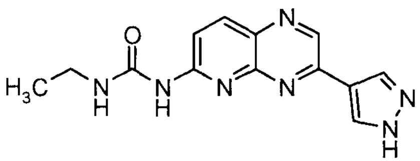 | 1-éthyl-3-[3-(1H-pyrazol-4-yl)-pyrido[2,3-b]pyrazin-6-yl]-urée |

(suite)

| | | |
|---|---|---|
| **Composé 80** | 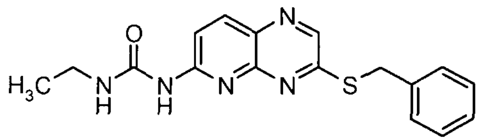 | 1-(3-benzylsulfanyl-pyrido[2,3-b]pyrazin-6-yl)-3-éthyl-urée |
| **Composé 81** | 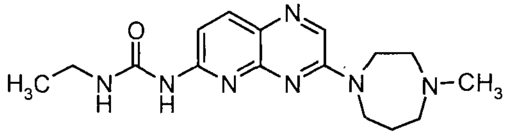 | 1-éthyl-3-[3-(4-méthyl-[1,4]diazépan-1-yl)-pyrido[2,3-b]pyrazin-6-yl]-urée |
| **Composé 82** | 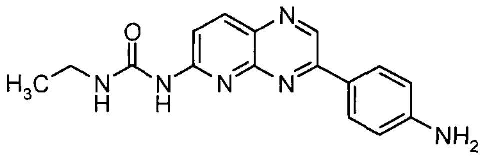 | 1-[3-(4-amino-phényl)-pyrido[2,3-b]pyrazin-6-yl]-3-éthyl-urée |
| **Composé 83** | 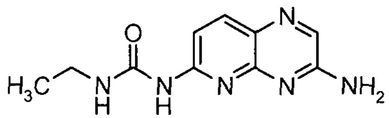 | 1-(3-amino-pyrido[2,3-b]pyrazin-6-yl)-3-éthyl-urée |
| **Composé 84** | 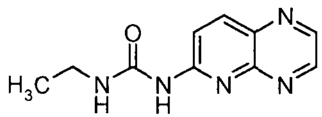 | 1-éthyl-3-pyrido[2,3-b]pyrazin-6-yl-urée |
| **Composé 85** | 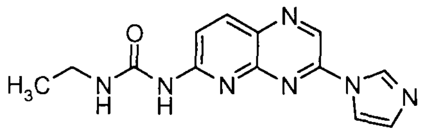 | 1-éthyl-3-(3-imidazol-1-yl-pyrido[2,3-b]pyrazin-6-yl)-urée |
| **Composé 86** | 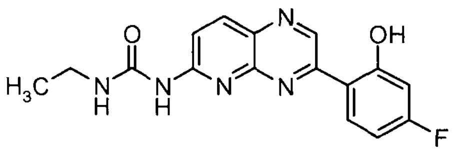 | 1-éthyl-3-[3-(4-fluoro-2-hydroxy-phényl)-pyrido[2,3-b]pyrazin-6-yl]-urée |

(suite)

| | | |
|---|---|---|
| **Composé 87** | 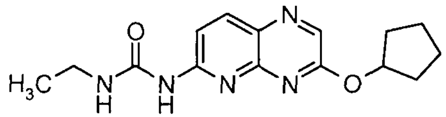 | 1-(3-cyclopentyloxy-pyrido[2,3-b]pyrazin-6-yl)-3-éthyl-urée |
| **Composé 88** | 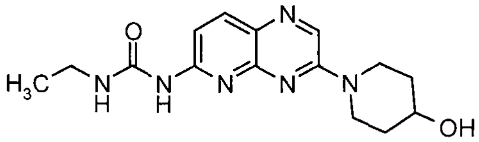 | 1-éthyl-3-[3-(4-hydroxy-pipéridin-1-yl)-pyrido[2,3-b]pyrazin-6-yl]-urée |
| **Composé 89** | 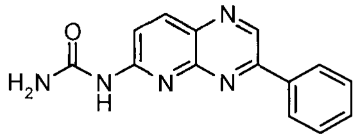 | (3-phényl-pyrido[2,3-b] pyrazin-6-yl)-urée |
| **Composé 90** | 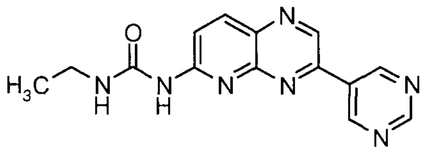 | 1-éthyl-3-(3-pyrimidin-5-yl-pyrido[2,3-b]pyrazin-6-yl)-urée |
| **Composé 91** | 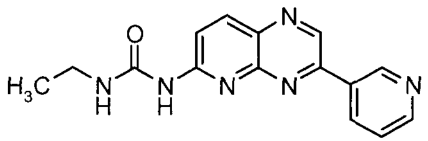 | 1-éthyl-3-(3-pyridin-3-yl-pyrido[2,3-b]pyrazin-6-yl)-urée |
| **Composé 92** | 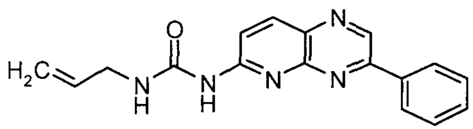 | 1-allyl-3-(3-phényl-pyrido[2,3-b]-pyrazin-6-yl)-urée |
| **Composé 93** | 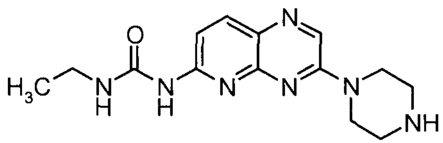 | 1-éthyl-3-(3-pipérazin-1-yl-pyrido[2,3-b]pyrazin-6-yl)-urée |

(suite)

| | | |
|---|---|---|
| **Composé 94** | 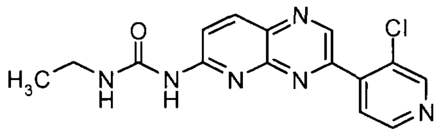 | 1-[3-(3-chloro-pyridin-4-ylméthyl)-pyrido[2,3-b]pyrazin-6-yl]-3-éthyl-urée |
| **Composé 95** | 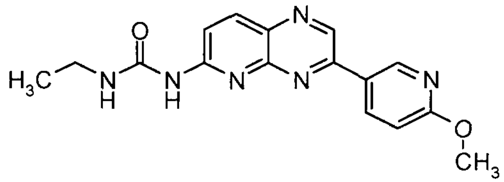 | 1-éthyl-3-[3-(6-méthoxy-pyridin-3-yl)-pyrido[2,3-b]pyrazin-6-yl]-urée |
| **Composé 96** | 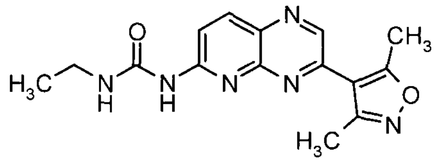 | 1-[3-(3,5-diméthyl-isoxazol-4-yl)-pyrido[2,3-b]pyrazin-6-yl]-3-éthyl-urée |
| **Composé 97** | 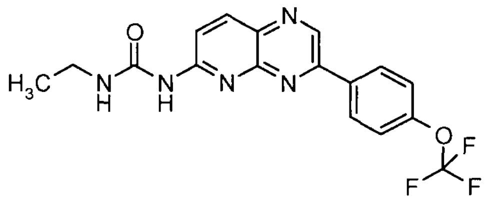 | 1-éthyl-3-[3-(4-trifluoro-méthoxyphényl)-pyrido[2, 3-b]-pyrazin-6-yl]-urée |
| **Composé 98** | 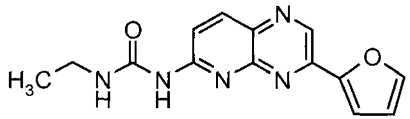 | 1-éthyl-3-(3-furan-2-yl-pyrido[2,3-b]pyrazin-6-yl)-urée |
| **Composé 99** | 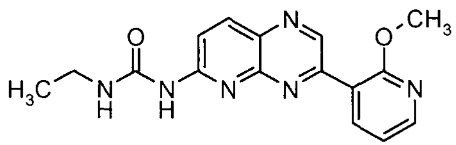 | 1-éthyl-3-[3-(2-méthoxy-pyridin-3-yl)-pyrido[2,3-b]pyrazin-6-yl]-urée |

(suite)

| | | |
|---|---|---|
| **Composé 100** | 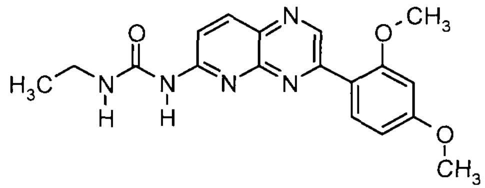 | 1-[3-(2,4-diméthoxy-phényl)-pyrido[2,3-b]pyrazin-6-yl]-3-éthyl-urée |
| **Composé 101** | 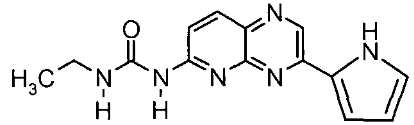 | 1-éthyl-3-[3-(1H-pyrrol-2-yl)-pyrido[2,3-b]pyrazin-6-yl]-urée |
| **Composé 102** | 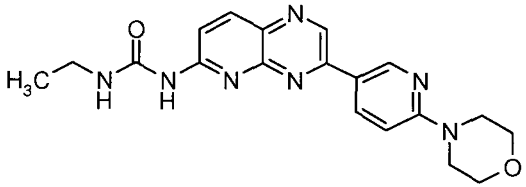 | 1-éthyl-3-[3-(6-morpholin-4-yl-pyridin-3-yl)-pyrido[2,3-b]pyrazin-6-yl]-urée |
| **Composé 103** | 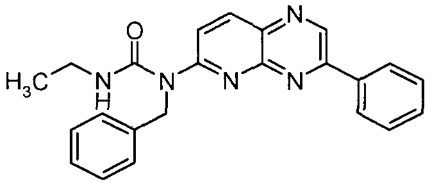 | 1-benzyl-3-éthyl-1-(3-phényl-pyrido[2,3-b]pyrazin-6-yl)-urée |
| **Composé 104** | 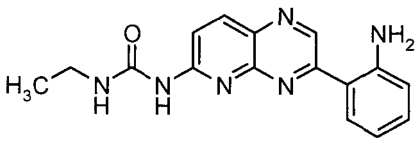 | 1-[3-(2-amino-phényl)-pyrido[2,3-b]pyrazin-6-yl]-3-éthyl-urée |
| **Composé 105** | 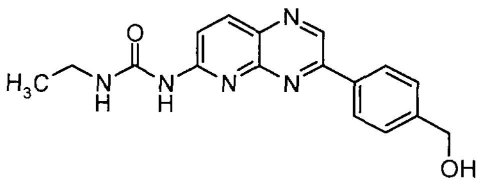 | 1-éthyl-3-[3-(4-hydroxyméthyl-phényl)-pyrido[2,3-b]pyrazin-6-yl]-urée |

(suite)

| | | |
|---|---|---|
| **Composé 106** | 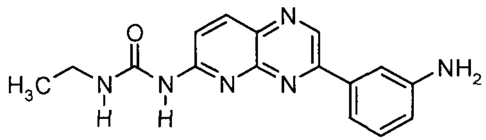 | 1-[3-(3-amino-phényl)-pyrido[2,3-b]pyrazin-6-yl]-3-éthyl-urée |
| **Composé 107** | 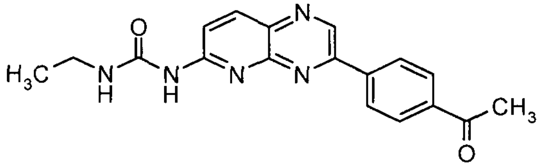 | 1-[3-(4-acétyl-phényl)-pyrido[2,3-b]pyrazin-6-yl]-3-éthyl-urée |
| **Composé 108** | 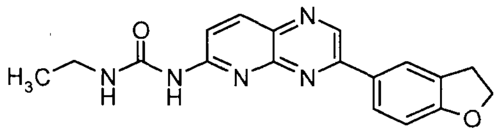 | 1-[3-(2,3-dihydro-benzofuran-5-yl)-pyrido[2,3-b]pyrazin-6-yl]-3-éthyl-urée |
| **Composé 109** | 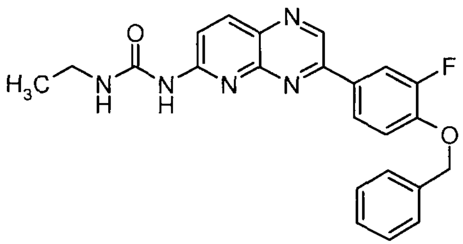 | 1-[3-(4-benzyloxy-3-fluoro-phényl)-pyrido[2,3-b]pyrazin-6-yl]-3-éthyl-urée |
| **Composé 110** | 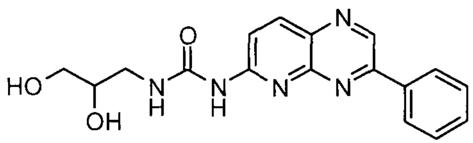 | 1-(2,3-dihydroxy-propyl)-3-(3-phényl-pyrido[2,3-b]pyrazin-6-yl)-urée |
| **Composé 111** | 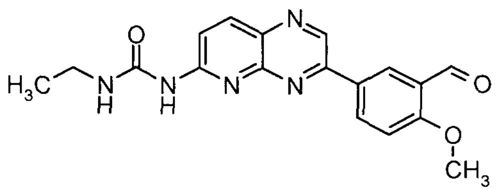 | 1-éthyl-3-[3-(3-formyl-4-méthoxy-phényl)-pyrido[2, 3-b]-pyrazin-6-yl]-urée |

(suite)

| | | |
|---|---|---|
| **Composé 112** | 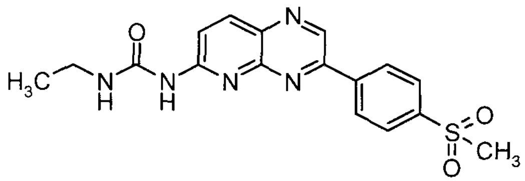 | 1-éthyl-3-[3-(4-méthanesulfonyl-phényl)-pyrido[2,3-b]pyrazin-6-yl]-urée |
| **Composé 113** | 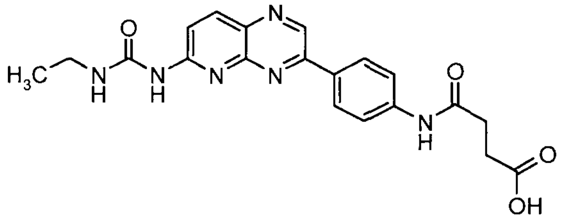 | acide N-{4-[6-(3-éthyl-uréido)-pyrido[2,3-b]pyrazin-3-yl]-phényl}-succinamidique |
| **Composé 114** | 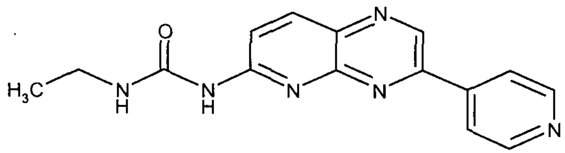 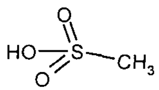 | 1-éthyl-3-(3-pyridin-4-yl-pyrido[2,3-b]pyrazin-6-yl)-urée sel d'acide méthanesulfonique (base libre) |
| **Composé 115** | 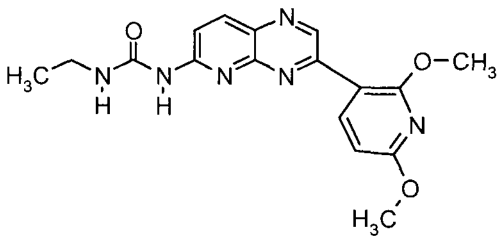 | 1-[3-(2,6-diméthoxy-pyridin-3-yl)-pyrido[2,3-b]-pyrazin-6-yl]-3-éthyl-urée |

(suite)

| | | |
|---|---|---|
| **Composé 116** | 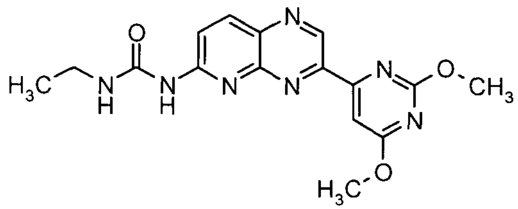 | 1-[3-(2,6-diméthoxy-pyrimidin-4-yl)-pyrido[2,3-b]pyrazin-6-yl]-3-éthyl-urée |
| **Composé 117** | 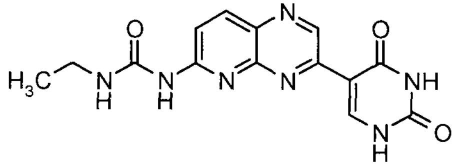 | 1-[3-(2,4-dioxo-1,2,3,4-tetrahydro-pyrimidin-5-yl)-pyrido[2,3-b]pyrazin-6-yl]-3-éthyl-urée |
| **Composé 118** | 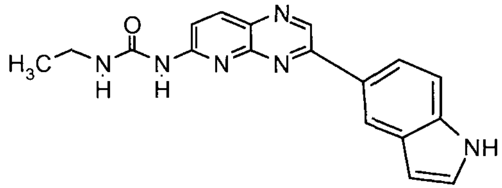 | 1-éthyl-3-[3-(1H-indol-5-yl)-pyrido[2,3-b]pyrazin-6-yl]-urée |
| **Composé 119** | 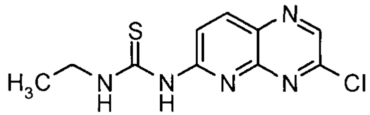 | 1-(3-chloro-pyrido[2,3-b] pyrazin-6-yl)-3-éthyl-thiourée |
| **Composé 120** | 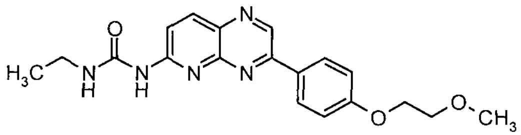 | 1-éthyl-3-{3-[4-(2-méthoxy-éthoxy)-phényl]-pyrido[2,3-b]-pyrazin-6-yl}-urée |
| **Composé 121** | 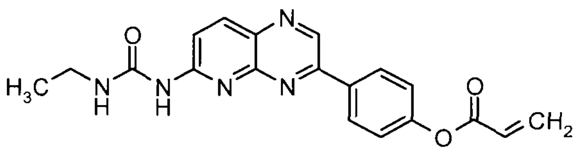 | acrylate de 4-[6-(3-éthyl-uréido)-pyrido[2,3-b]pyrazin-3-yl]-phényle |

(suite)

| | | |
|---|---|---|
| **Composé 122** | 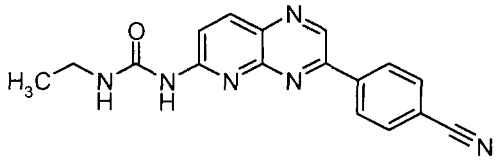 | 1-[3-(4-cyano-phényl)-pyrido[2,3-b]pyrazin-6-yl]-3-éthyl-urée |
| **Composé 123** | 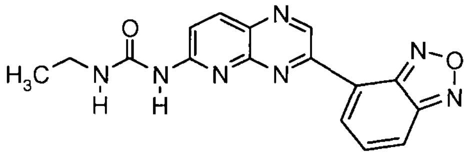 | 1-(3-benzo[1,2,5]oxadiazol-4-yl-pyrido[2,3-b]pyrazin-6-yl)-3-éthyl-urée |
| **Composé 124** | 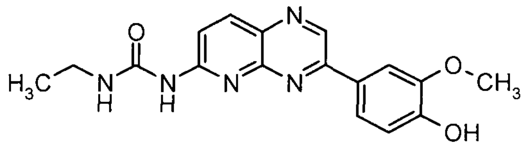 | 1-éthyl-3-[3-(4-hydroxy-3-méthoxyphényl)-pyrido[2, 3-b]-pyrazin-6-yl]-urée |
| **Composé 125** | 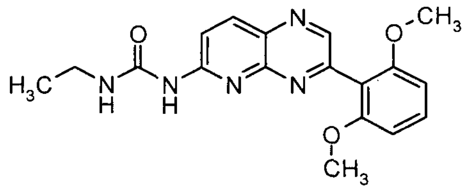 | 1-[3-(2,6-diméthoxy-phényl)-pyrido [2,3-b]pyrazin-6-yl]-3-éthyl-urée |
| **Composé 126** | 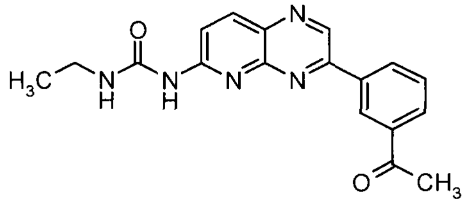 | 1-[3-(3-acétyl-phényl)-pyrido[2,3-b]pyrazin-6-yl]-3-éthyl-urée |
| **Composé 127** | 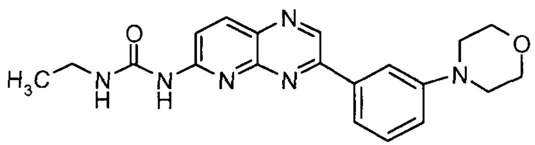 | 1-éthyl-3-[3-(3-morpholin-4-yl-phényl)-pyrido[2,3-b]pyrazin-6-yl]-urée |

(suite)

| | | |
|---|---|---|
| **Composé 128** | 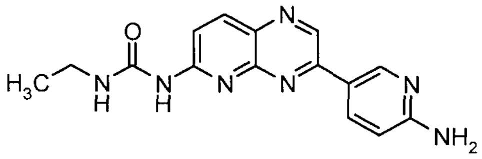 | 1-[3-(6-amino-pyridin-3-yl)-pyrido[2,3-b]pyrazin-6-yl]-3-éthyl-urée |
| **Composé 129** | 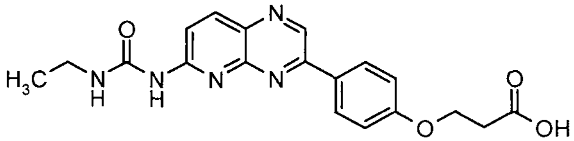 | acide 3-{4-[6-(3-éthyl-uréido)-pyrido[2,3-b]pyrazin-3-yl]-phénoxy}-propionique |
| **Composé 130** | 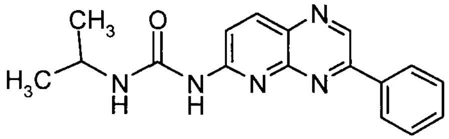 | 1-isopropyl-3-(3-phényl-pyrido[2,3-b]pyrazin-6-yl)-urée |
| **Composé 131** | 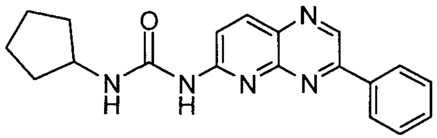 | 1-cyclopentyl-3-(3-phényl-pyrido[2,3-b] pyrazin-6-yl)-urée |
| **Composé 132** | 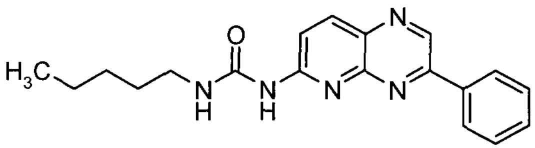 | 1-pentyl-3-(3-phényl-pyrido[2,3-b]pyrazin-6-yl)-urée |
| **Composé 133** | 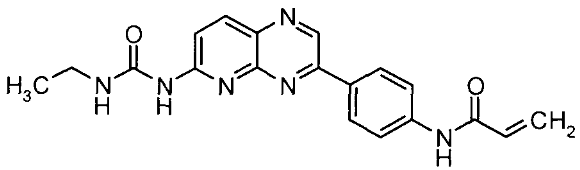 | N-{4-[6-(3-éthyl-uréido)-pyrido[2,3-b]pyrazin-3-yl]-phényl}-acrylamide |
| **Composé 134** | 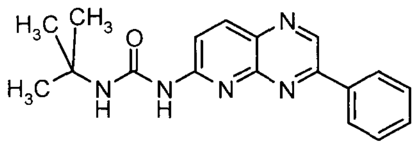 | 1-tert-butyl-3-(3-phényl-pyrido[2,3-b]pyrazin-6-yl)-urée |

(suite)

| | | |
|---|---|---|
| **Composé 135** | 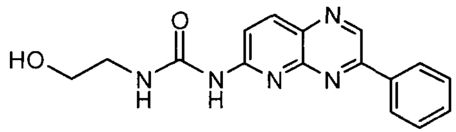 | 1-(2-hydroxy-éthyl)-3-(3-phényl-pyrido[2,3-b]pyrazin-6-yl)-urée |
| **Composé 136** | 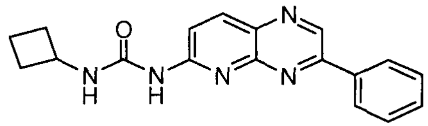 | 1-cyclobutyl-3-(3-phényl-pyrido[2,3-b]pyrazin-6-yl)-urée |
| **Composé 137** | 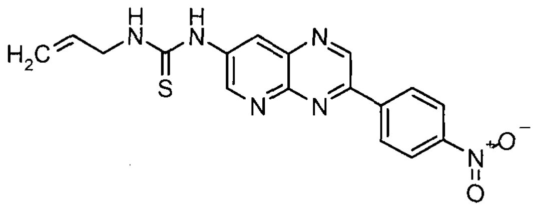 | 1-allyl-3-[3-(4-nitro-phényl)-pyrido[2,3-b]pyrazin-7-yl]-thiourée |
| **Composé 138** | 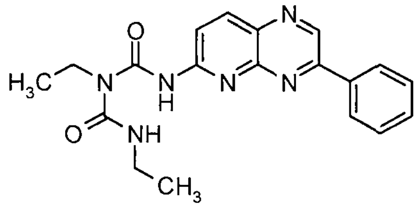 | 1-éthyl-1-(éthylcarbamoyl)-3-(3-phényl-pyrido[2,3-b]pyrazin-6-yl)-urée |
| **Composé 139** | 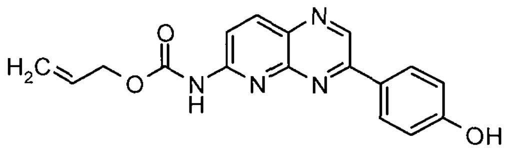 | [3-(4-hydroxy-phényl)-pyrido[2,3-b]pyrazin-6-yl]-carbamate d'allyle |
| **Composé 140** | 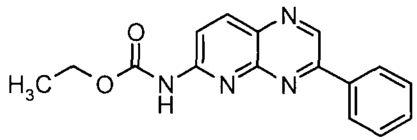 | (3-phényl-pyrido[2,3-b]pyrazin-6-yl)-carbamate d'éthyle |

(suite)

| | | |
|---|---|---|
| **Composé 141** | 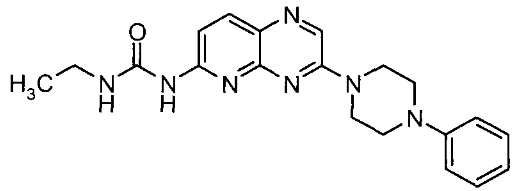 | 1-éthyl-3-[3-(4-phényl-pipérazin-1-yl)-pyrido[2,3-b]pyrazin-6-yl]-urée |
| **Composé 142** | 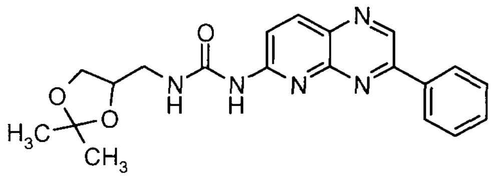 | 1-(2,2-diméthyl-[1,3]dioxolan-4-ylméthyl)-3-(3-phényl-pyrido[2,3-b]pyrazin-6-yl)-urée |
| **Composé 143** | 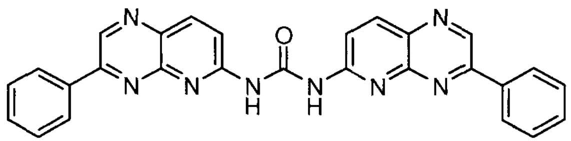 | 1,3-bis-(3-phényl-pyrido[2,3-b]-pyrazin-6-yl)-urée |
| **Composé 144** | 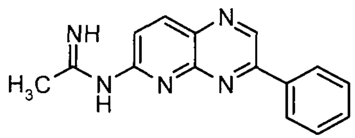 | N-(3-phényl-pyrido[2,3-b] pyrazin-6-yl)-acétamidine |
| **Composé 145** | 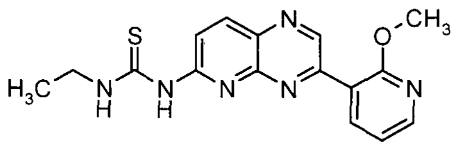 | 1-éthyl-3-[3-(2-méthoxy-pyridin-3-yl)-pyrido[2,3-b]pyrazin-6-yl]-thiourée |
| **Composé 146** | 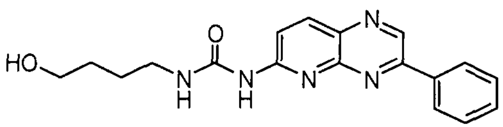 | 1-(4-hydroxy-butyl)-3-(3-phényl-pyrido[2,3-b]pyrazin-6-yl)-urée |
| **Composé 147** | 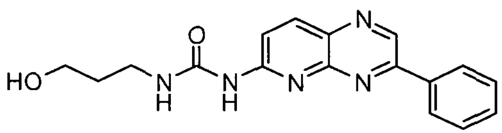 | 1-(3-hydroxy-propyl)-3-(3-phényl-pyrido[2,3-b]pyrazin-6-yl)-urée |

(suite)

| | | |
|---|---|---|
| **Composé 148** | 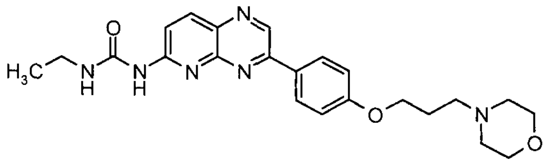 | 1-éthyl-3-{3-[4-(3-morpholin-4-yl-propoxy)-phényl]-pyrido[2,3-b]pyrazin-6-yl}-urée |
| **Composé 149** | 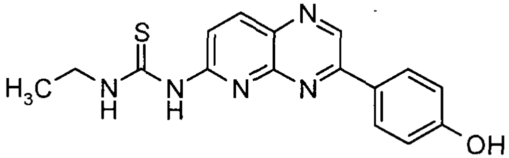 | 1-éthyl-3-[3-(4-hydroxy-phényl)-pyrido[2,3-b]pyrazin-6-yl]-thiourée |
| **Composé 150** | 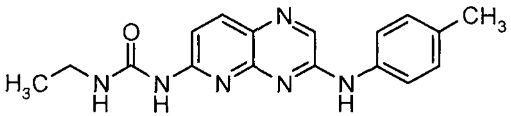 | 1-éthyl-3-(3-p-tolylamino-pyrido[2,3-b]pyrazin-6-yl)-urée |
| **Composé 151** | 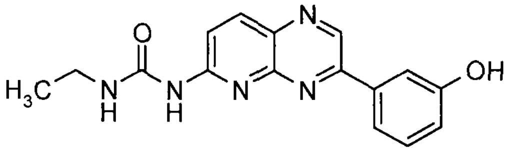 | 1-éthyl-3-[3-(3-hydroxy-phényl)-pyrido[2,3-b]pyrazin-6-yl]-urée |
| **Composé 152** | 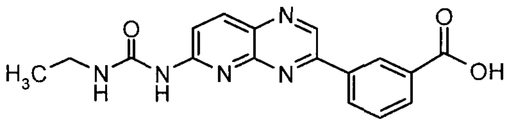 | acide 3-[6-(3-éthyl-uréido)-pyrido[2,3-b]pyrazin-3-yl]-benzoïque |
| **Composé 153** | 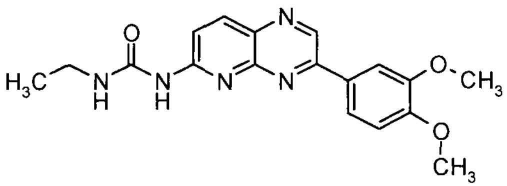 | 1-[3-(3,4-diméthoxy-phényl)-pyrido [2,3-b]pyrazin-6-yl]-3-éthyl-urée |

(suite)

| | | |
|---|---|---|
| **Composé 154** | 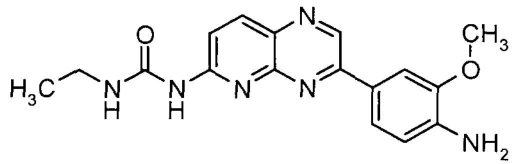 | 1-[3-(4-amino-3-méthoxy-phényl)-pyrido[2,3-b]pyrazin-6-yl]-3-éthyl-urée |
| **Composé 155** | 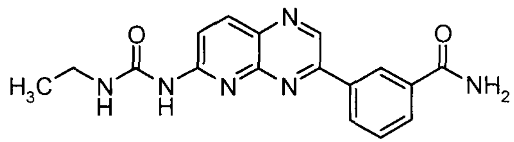 | 3-[6-(3-éthyl-uréido)-pyrido[2,3-b]pyrazin-3-yl]-benzamide |
| **Composé 156** | 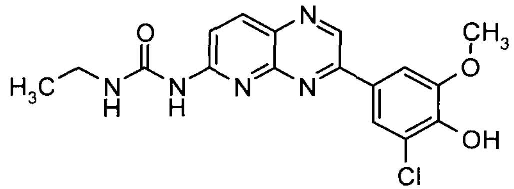 | 1-[3-(3-chloro-4-hydroxy-5-méthoxy-phényl)-pyrido[2,3-b]-pyrazin-6-yl]-3-éthyl-urée |
| **Composé 157** | 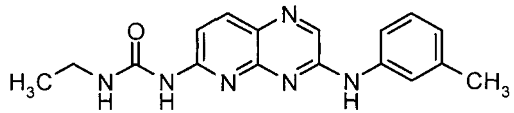 | 1-éthyl-3-(3-m-tolylam inopyrido-[2,3-b]pyrazin-6-yl)-urée |
| **Composé 158** | 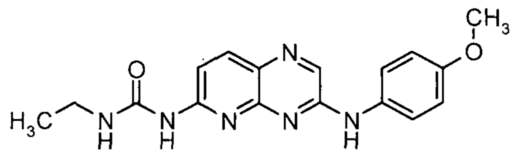 | 1-éthyl-3-[3-(4-méthoxy-phénylamino)-pyrido[2,3-b]-pyrazin-6-yl]-urée |
| **Composé 159** | 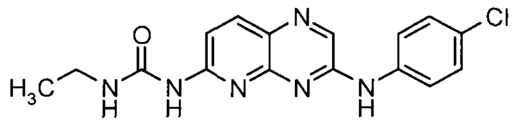 | 1-[3-(4-chloro-phénylamino)-pyrido-[2,3-b]pyrazin-6-yl]-3-éthyl-urée |
| **Composé 160** | 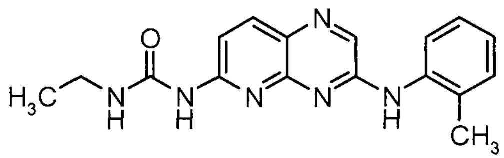 | 1-éthyl-3-(3-o-tolylamino-pyrido[2,3-b]-pyrazin-6-yl)-urée |

(suite)

| | | |
|---|---|---|
| **Composé 161** | 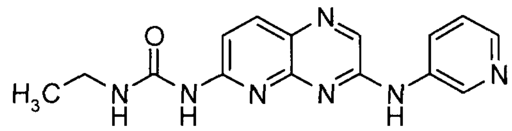 | 1-éthyl-3-[3-(pyridin-3-ylamino)-pyrido-[2,3-b] pyrazin-6-yl]-urée |
| **Composé 162** | 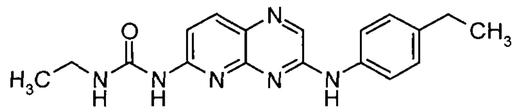 | 1-éthyl-3-[3-(4-éthyl-phénylamino)-pyrido[2,3-b]-pyrazin-6-yl]-urée |
| **Composé 163** | 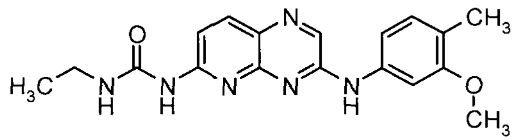 | 1-éthyl-3-[3-(3-méthoxy-4-méthyl-phénylamino)-pyrido[2,3-b]pyrazin-6-yl]-urée |
| **Composé 164** | 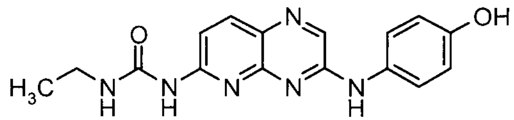 | 1-éthyl-3-[3-(4-hydroxy-phénylamino)-pyrido[2,3-b]-pyrazin-6-yl]-urée |
| **Composé 165** | 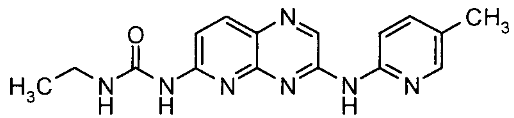 | 1-éthyl-3-[3-(5-méthyl-pyridin-2-yl-amino)-pyrido[2,3-b]pyrazin-6-yl]-urée |
| **Composé 166** | 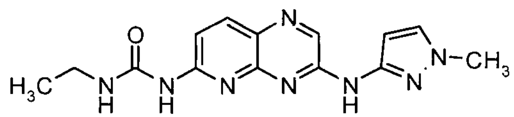 | 1-éthyl-3-[3-(1-méthyl-1H-pyrazol-3-ylamino)-pyrido[2,3-b]pyrazin-6-yl]-urée |
| **Composé 167** | 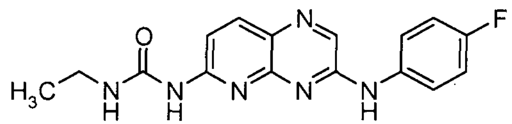 | 1-éthyl-3-[3-(4-fluoro-phénylamino)-pyrido[2,3-b]-pyrazin-6-yl]-urée |
| **Composé 168** | 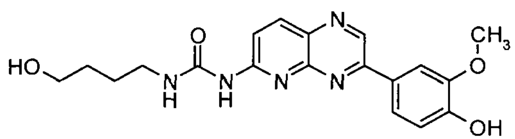 | 1-(4-hydroxy-butyl)-3-[3-(4-hydroxy-3-méthoxyphényl)-pyrido[2,3-b]pyrazin-6-yl]-urée |

(suite)

| | | |
|---|---|---|
| **Composé 169** | 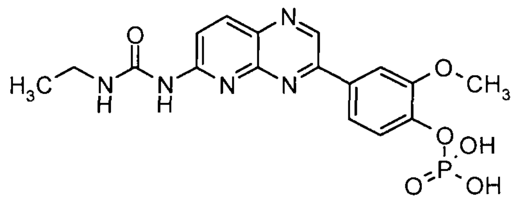 | phosphate de mono-{4-[6-(3-éthyl-uréido)-pyrido-[2,3-b] pyrazin-3-yl]-2-méthoxy-phényle} |
| **Composé 170** | 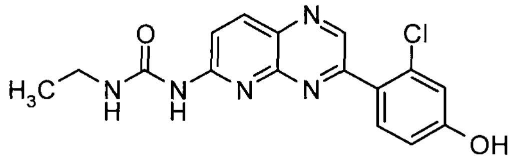 | 1-[3-(2-chloro-4-hydroxy-phényl)-pyrido[2,3-b]pyrazin-6-yl]-3-éthyl-urée |
| **Composé 171** | 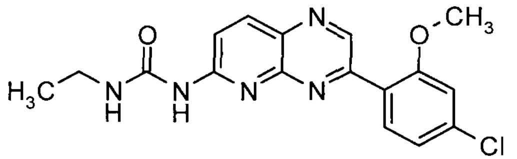 | 1-[3-(4-chloro-2-méthoxy-phényl)-pyrido[2,3-b]pyrazin-6-yl]-3-éthyl-urée |
| **Composé 172** | 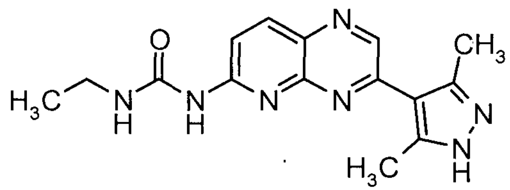 | 1-[3-(3,5-diméthyl-1 H-pyrazol-4-yl)-pyrido[2,3-b]pyrazin-6-yl]-3-éthyl-urée |
| **Composé 173** | 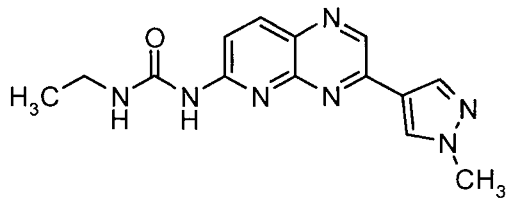 | 1-éthyl-3-[3-(1-méthyl-1H-pyrazol-4-yl)-pyrido[2,3-b]-pyrazin-6-yl]-urée |
| **Composé 174** | 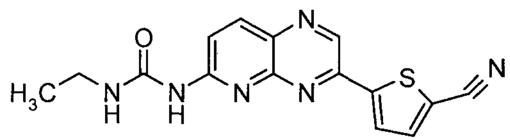 | 1-[3-(5-cyano-thiophén-2-yl)-pyrido[2,3-b]pyrazin-6-yl]-3-éthyl-urée |

(suite)

| | | |
|---|---|---|
| **Composé 175** | 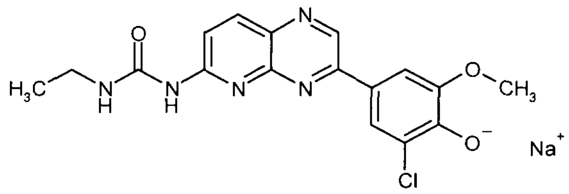 | 2-chloro-4-[6-(3-éthyl-uréido)-pyrido[2,3-b]pyrazin-3-yl]-6-méthoxy-phénolate de sodium |
| **Composé 176** | 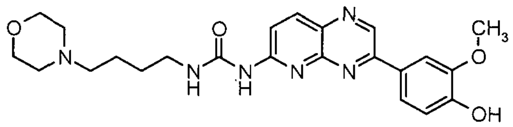 | 1-[3-(4-hydroxy-3-méthoxy-phényl)-pyrido[2,3-b]pyrazin-6-yl]-3-(4-morpholin-4-yl-butyl)-urée |
| **Composé 177** | 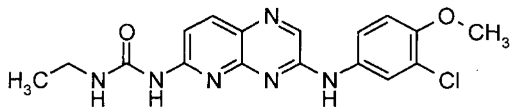 | 1-[3-(3-chloro-4-méthoxy-phénylamino)-pyrido[2,3-b]-pyrazin-6-yl]-3-éthyl-urée |
| **Composé 178** | 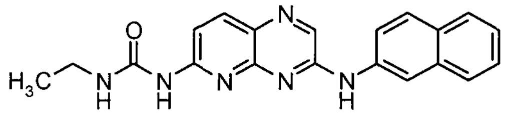 | 1-éthyl-3-[3-(naphtalén-2-ylamino)-pyrido[2,3-b]pyrazin-6-yl]-urée |
| **Composé 179** | 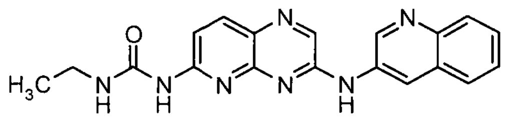 | 1-éthyl-3-[3-(quinolin-3-ylamino)-pyrido[2,3-b]pyrazin-6-yl]-urée |
| **Composé 180** | 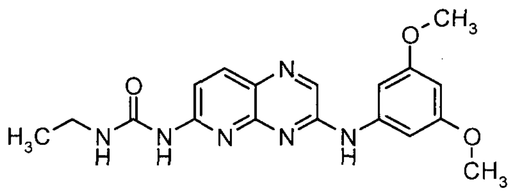 | 1-[3-(3,5-diméthoxy-phénylamino)-pyrido[2,3-b]-pyrazin-6-yl]-3-éthyl-urée |
| **Composé 181** | 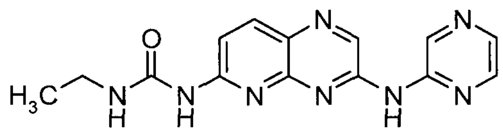 | 1-éthyl-3-[3-(pyrazin-2-ylamino)-pyrido[2,3-b]pyrazin-6-yl]-urée |

(suite)

| | | |
|---|---|---|
| **Composé 182** | 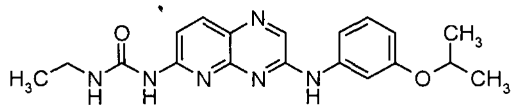 | 1-éthyl-3-[3-(3-isopropoxy-phénylamino)-pyrido[2,3-b]-pyrazin-6-yl]-urée |
| **Composé 183** | 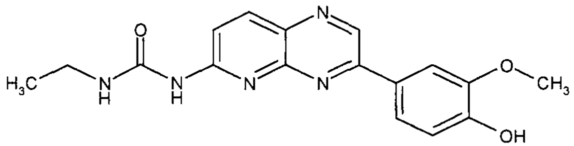 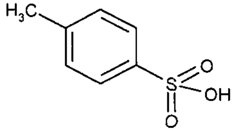 | 1-éthyl-3-[3-(4-hydroxy-3-méthoxy-phényl)-pyrido[2,3-b]-pyrazin-6-yl]-urée p-toluènesulfonate |
| **Composé 184** | 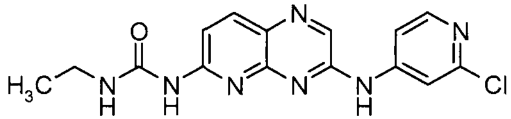 | 1-[3-(2-chloro-pyridin-4-ylamino)-pyrido[2,3-b]pyrazin-6-yl]-3-éthyl-urée |
| **Composé 185** | 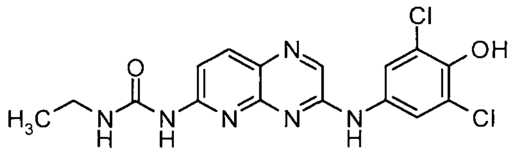 | 1-[3-(3,5-dichloro-4-hydroxy-phénylamino)-pyrido[2,3-b]-pyrazin-6-yl]-3-éthyl-urée |
| **Composé 186** | 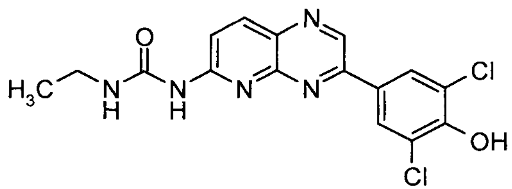 | 1-[3-(3,5-dichloro-4-hydroxy-phényl)-pyrido[2,3-b]pyrazin-6-yl]-3-éthyl-urée |
| **Composé 187** | 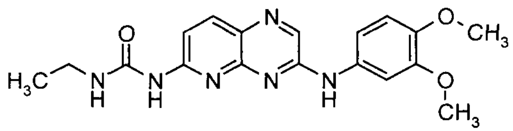 | 1-[3-(3,4-diméthoxy-phénylamino)-pyrido[2,3-b]-pyrazin-6-yl]-3-éthyl-urée |

(suite)

| | | |
|---|---|---|
| **Composé 188** | 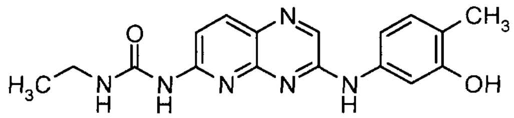 | 1-éthyl-3-[3-(3-hydroxy-4-méthyl-phénylamino)-pyrido[2,3-b]pyrazin-6-yl]-urée |
| **Composé 189** | 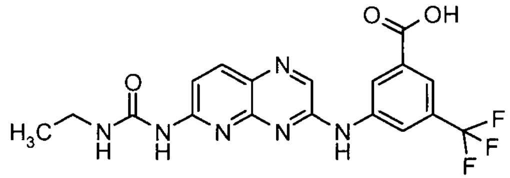 | acide 3-[6-(3-éthyl-uréido)-pyrido[2,3-b]pyrazin-3-ylamino]-5-trifluorométhylbenzoïque |
| **Composé 190** | 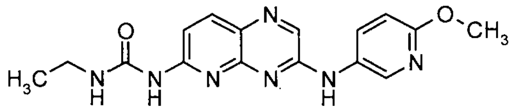 | 1-éthyl-3-[3-(6-méthoxy-pyridin-3-ylamino)-pyrido[2,3-b]pyrazin-6-yl]-urée |
| **Composé 191** | 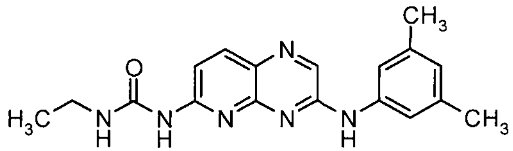 | 1-[3-(3,5-diméthyl-phénylamino)-pyrido[2,3-b]-pyrazin-6-yl]-3-éthyl-urée |
| **Composé 192** | 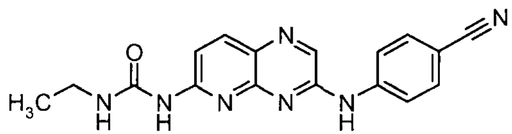 | 1-[3-(4-cyano-phénylamino)-pyrido-[2,3-b] pyrazin-6-yl]-3-éthyl-urée |
| **Composé 193** | 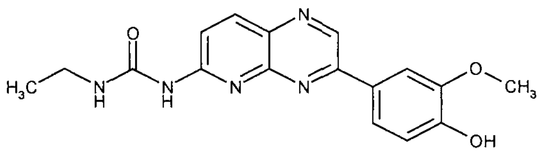 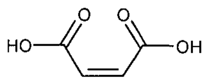 | 1-éthyl-3-[3-(4-hydroxy-3-méthoxy-phényl)-pyrido[2,3-b]-pyrazin-6-yl]-urée sel d'acide (Z)-but-2-ène-dicarboxylique |

(suite)

| | | |
|---|---|---|
| **Composé 194** | 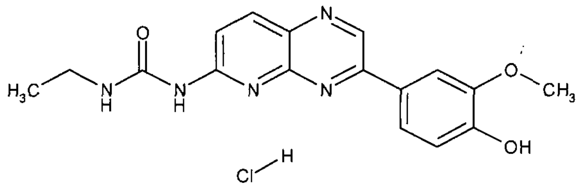 | 1-éthyl-3-[3-(4-hydroxy-3-méthoxy-phényl)-pyrido[2,3-b]-pyrazin-6-yl]-urée chlorhydrate |
| **Composé 195** | 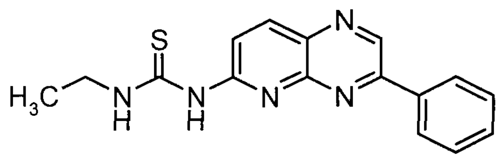 | 1-éthyl-3-(3-phényl-pyrido[2,3-b]pyrazin-6-yl)-thiourée |
| **Composé 196** | 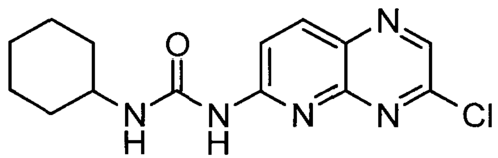 | 1-(3-chloro-pyrido[2,3-b]pyrazin-6-yl)-3-cyclohexyl-urée |
| **Composé 197** | 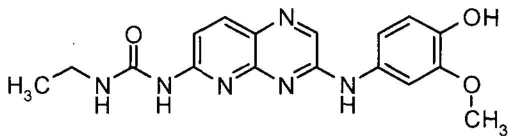 | 1-éthyl-3-[3-(4-hydroxy-3-méthoxy-phénylamino)-pyrido[2,3-b]pyrazin-6-yl]-urée |
| **Composé 198** | 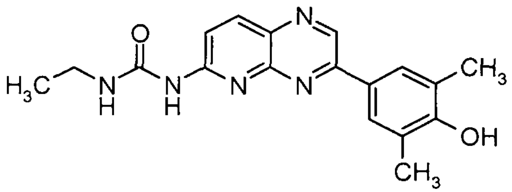 | 1-éthyl-3-[3-(4-hydroxy-3,5-diméthyl-phényl)-pyrido[2, 3-b]-pyrazin-6-yl]-urée |

(suite)

| | | |
|---|---|---|
| **Composé 199** | 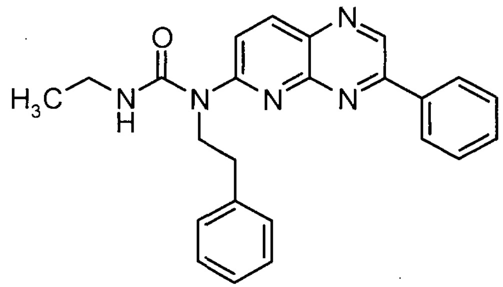 | 3-éthyl-1-phénéthyl-1-(3-phényl-pyrido[2,3-b]pyrazin-6-yl)-urée |
| **Composé 200** | 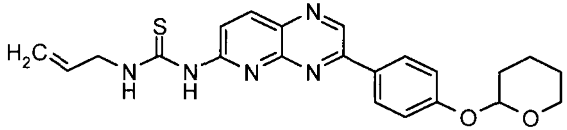 | 1-allyl-3-{3-[4-(tétrahydro-pyran-2-yloxy)-phényl]-pyrido[2,3-b]-pyrazin-6-yl}-thiourée |
| **Composé 201** | 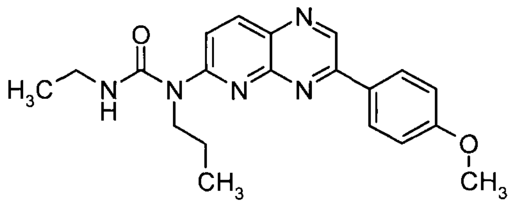 | 3-éthyl-1-[3-(4-méthoxy-phényl)-pyrido[2,3-b]pyrazin-6-yl]-1-propyl-urée |
| **Composé 202** | 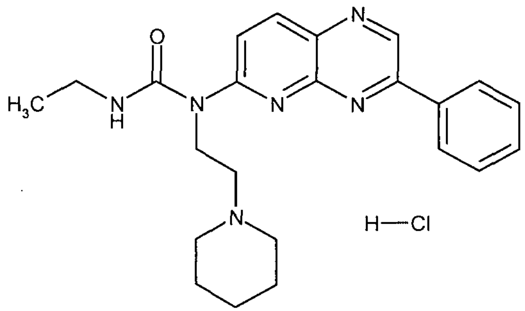 | 3-éthyl-1-(3-phényl-pyrido[2,3-b]pyrazin-6-yl)-1-(2-piperidin-1-yl-éthyl)-urée chlorhydrate |

(suite)

| | | |
|---|---|---|
| **Composé 203** | 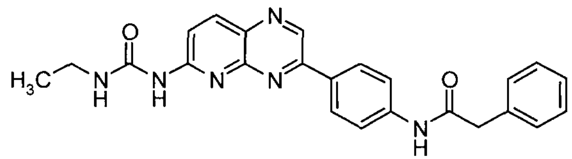 | N-{4-[6-(3-éthyl-uréido)-pyrido[2,3-b]pyrazin-3-yl]-phényl}-2-phényl- acétamide |
| **Composé 204** | 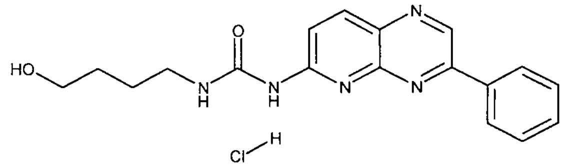 | 1-(4-hydroxy-butyl)-3-(3-phényl-pyrido[2,3-b]pyrazin-6-yl)-urée chlorhydrate |
| **Composé 205** | 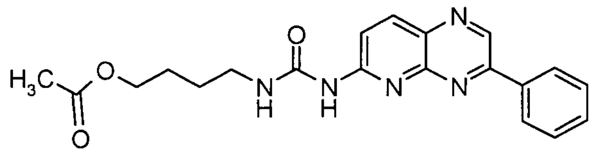 | acétate de 4-[3-(3-phényl-pyrido[2,3-b]pyrazin-6-yl)-uréido]-butyle |
| **Composé 206** | 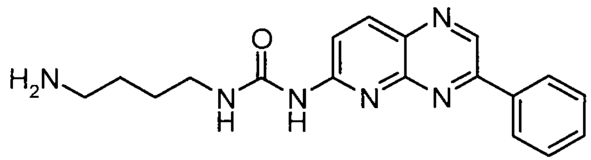 | 1-(4-amino-butyl)-3-(3-phényl-pyrido[2,3-b]pyrazin-6-yl)-urée |
| **Composé 207** | 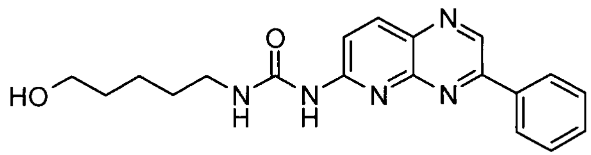 | 1-(5-hydroxy-pentyl)-3-(3-phényl-pyrido[2,3-b]pyrazin-6-yl)-urée |
| **Composé 208** | 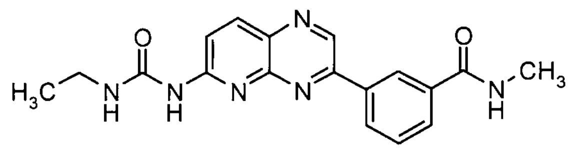 | 3-[6-(3-éthyl-uréido)-pyrido[2,3-b]pyrazin-3-yl]-N-méthyl-benzamide |

(suite)

| | | |
|---|---|---|
| **Composé 209** | 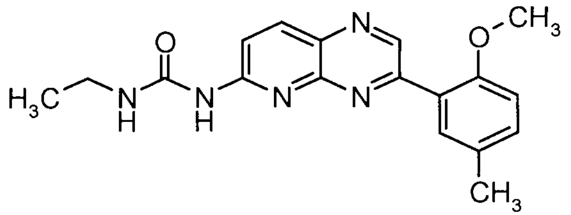 | 1-éthyl-3-[3-(2-méthoxy-5-méthyl-phényl)-pyrido[2,3-b]-pyrazin-6-yl]-urée |
| **Composé 210** | 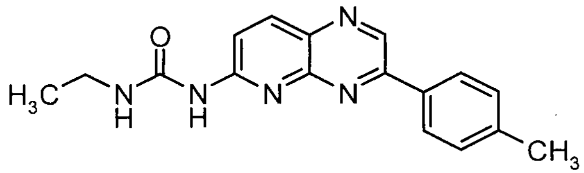 | 1-éthyl-3-(3-p-tolyl-pyrido[2, 3-b] pyrazin-6-yl)-urée |
| **Composé 211** | 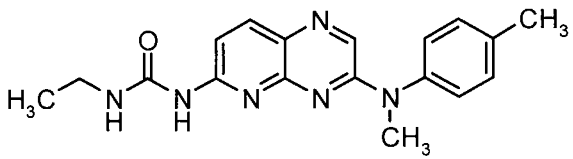 | 1-éthyl-3-[3-(méthyl-p-tolyl-amino)-pyrido[2,3-b]pyrazin-6-yl]-urée |
| **Composé 212** | 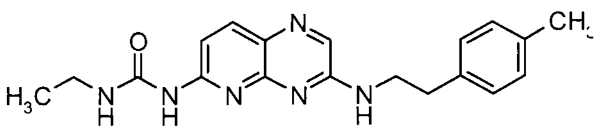 | 1-éthyl-3-[3-(2-p-tolyl-éthylamino)-pyrido[2,3-b]-pyrazin-6-yl]-urée |
| **Composé 213** | 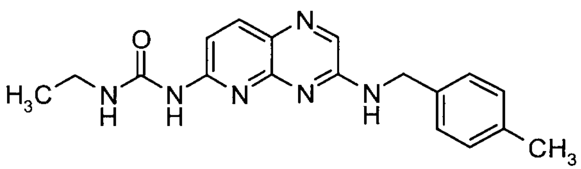 | 1-éthyl-3-[3-(4-méthyl-benzylamino)-pyrido[2,3-b]-pyrazin-6-yl]-urée |
| **Composé 214** | 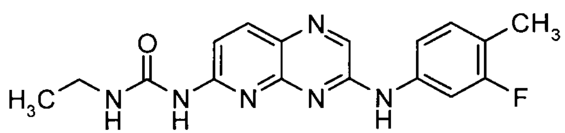 | 1-éthyl-3-[3-(3-fluoro-4-méthyl-phényl-amino)-pyrido[2,3-b]-pyrazin-6-yl]-urée |

(suite)

| | | |
|---|---|---|
| **Composé 215** | 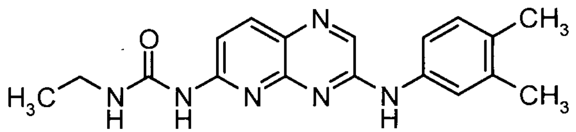 | 1-[3-(3,4-diméthyl-phénylamino)-pyrido[2,3-b]-pyrazin-6-yl]-3-éthyl-urée |
| **Composé 216** | 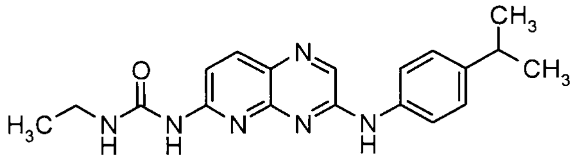 | 1-éthyl-3-[3-(4-isopropyl-phénylamino)-pyrido[2,3-b]-pyrazin-6-yl]-urée |
| **Composé 217** | 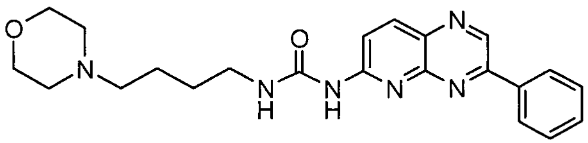 | 1-(4-morpholin-4-yl-butyl)-3-(3-phényl-pyrido[2,3-b]pyrazin-6-yl)-urée |
| **Composé 218** | 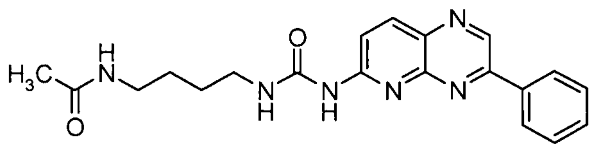 | N-{4-[3-(3-phényl-pyrido[2,3-b] pyrazin-6-yl)-uréido]-butyl}-acétamide |
| **Composé 219** | 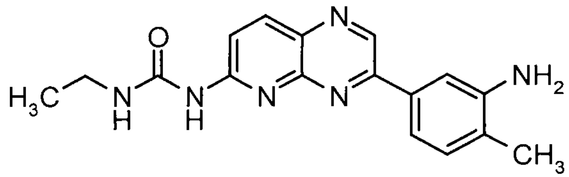 | 1-[3-(3-amino-4-méthyl-phényl)-pyrido[2,3-b]pyrazin-6-yl]-3-éthyl-urée |
| **Composé 220** | 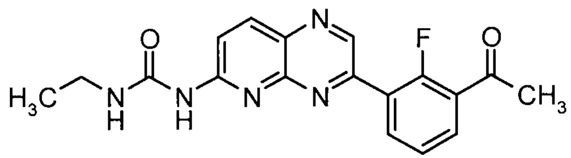 | 1-[3-(3-acétyl-2-fluoro-phényl)-pyrido[2,3-b]pyrazin-6-yl]-3-éthyl-urée |

(suite)

| | | |
|---|---|---|
| **Composé 221** | 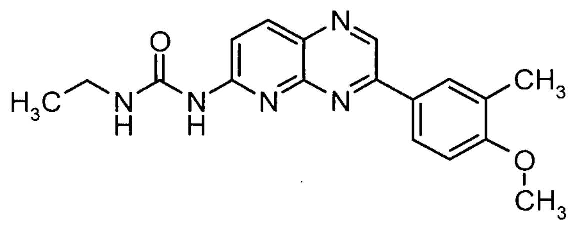 | 1-éthyl-3-[3-(4-méthoxy-3-méthyl-phényl)-pyrido[2,3-b]-pyrazin-6-yl]-urée |
| **Composé 222** | 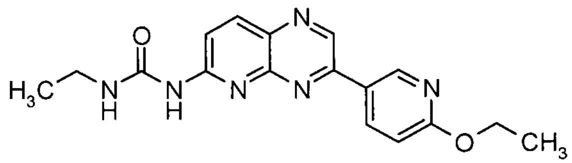 | 1-[3-(6-éthoxy-pyridin-3-yl)-pyrido[2,3-b]pyrazin-6-yl]-3-éthyl-urée |
| **Composé 223** | 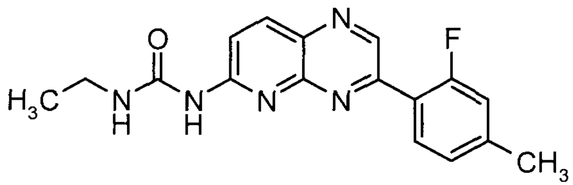 | 1-éthyl-3-[3-(2-fluoro-4-méthyl-phényl)-pyrido[2,3-b]pyrazin-6-yl]-urée |
| **Composé 224** | 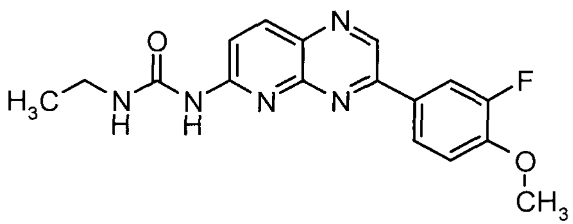 | 1-éthyl-3-[3-(3-fluoro-4-méthoxy-phényl)-pyrido[2,3-b]-pyrazin-6-yl]-urée |
| **Composé 225** | 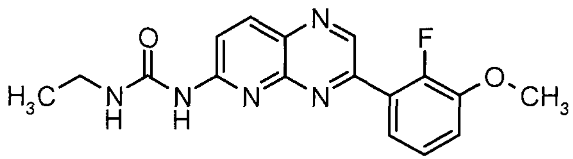 | 1-éthyl-3-[3-(2-fluoro-3-méthoxy-phényl)-pyrido[2,3-b]-pyrazin-6-yl]-urée |

(suite)

| | | |
|---|---|---|
| **Composé 226** | 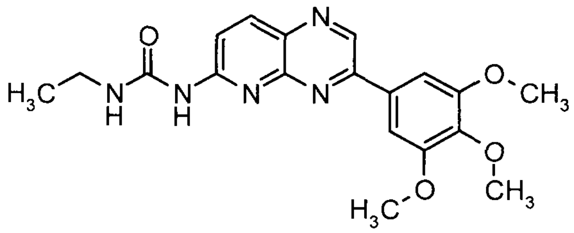 | 1-éthyl-3-[3-(3,4,5-triméthoxy-phényl)-pyrido[2,3-b]pyrazin-6-yl]-urée |
| **Composé 227** | 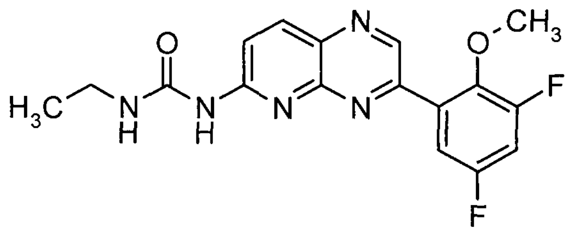 | 1-[3-(3,5-difluoro-2-méthoxy-phényl)-pyrido[2,3-b]pyrazin-6-yl]-3-éthyl-urée |
| **Composé 228** | 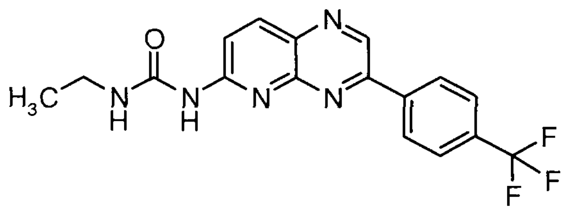 | 1-éthyl-3-[3-(4-trifluorométhyl-phényl)-pyrido[2,3-b]pyrazin-6-yl]-urée |
| **Composé 229** | 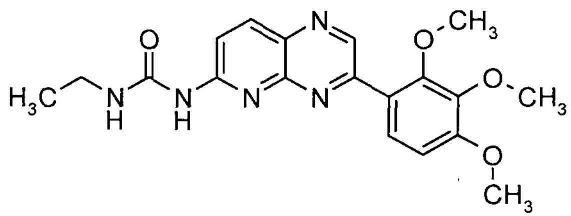 | 1-éthyl-3-[3-(2,3,4-triméthoxy-phényl)-pyrido[2,3-b]pyrazin-6-yl]-urée |

(suite)

| | | |
|---|---|---|
| **Composé 230** | 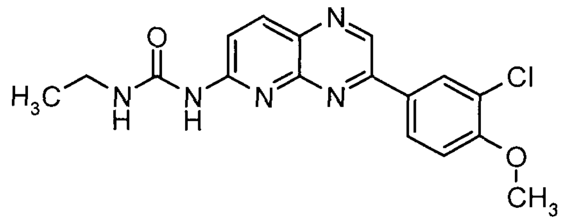 | 1-[3-(3-chloro-4-méthoxy-phényl)-pyrido[2,3-b]pyrazin-6-yl]-3-éthyl-urée |
| **Composé 231** | 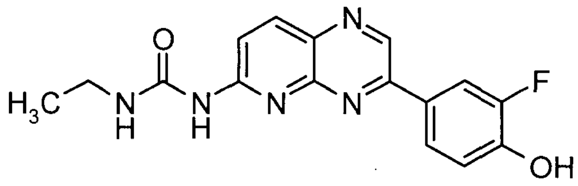 | 1-éthyl-3-[3-(3-fluoro-4-hydroxy-phényl)-pyrido[2,3-b]pyrazin-6-yl]-urée |
| **Composé 232** | 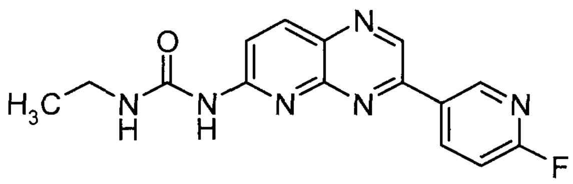 | 1-éthyl-3-[3-(6-fluoro-pyridin-3-yl)-pyrido[2,3-b]pyrazin-6-yl]-urée |
| **Composé 233** | 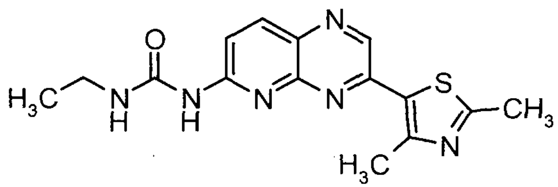 | 1-[3-(2,4-diméthyl-thiazol-5-yl)-pyrido-[2,3-b] pyrazin-6-yl]-3-éthyl-urée |
| **Composé 234** | 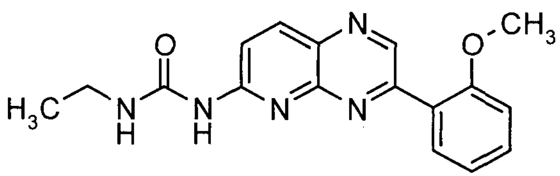 | 1-éthyl-3-[3-(2-méthoxy-phényl)-pyrido[2,3-b]pyrazin-6-yl]-urée |

(suite)

| | | |
|---|---|---|
| **Composé 235** | 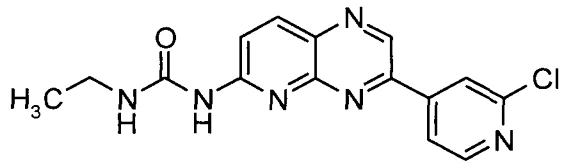 | 1-[3-(2-chloro-pyridin-4-yl)-pyrido[2,3-b]pyrazin-6-yl]-3-éthyl-urée |
| **Composé 236** | 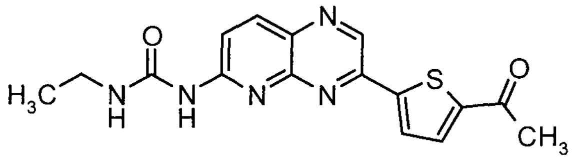 | 1-[3-(5-acétyl-thiophén-2-yl)-pyrido[2,3-b]pyrazin-6-yl]-3-éthyl-urée |
| **Composé 237** | 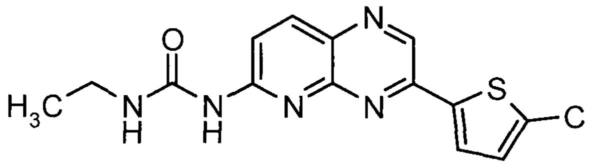 | 1-[3-(5-chloro-thiophén-2-yl)-pyrido[2,3-b]pyrazin-6-yl]-3-éthyl-urée |
| **Composé 238** | 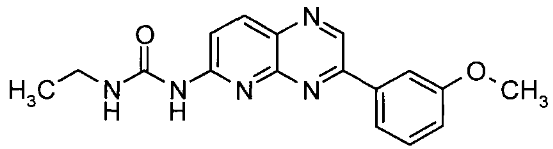 | 1-éthyl-3-[3-(3-méthoxy-phényl)-pyrido[2,3-b]pyrazin-6-yl]-urée |
| **Composé 239** | 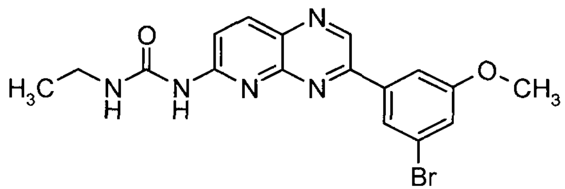 | 1-[3-(3-bromo-5-méthoxy-phényl)-pyrido[2,3-b]pyrazin-6-yl]-3-éthyl-urée |
| **Composé 240** | 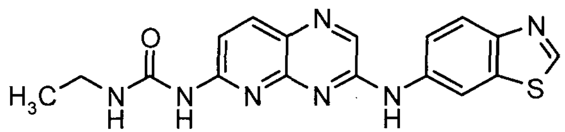 | 1-[3-(benzothiazol-6-ylamino)-pyrido[2,3-b]pyrazin-6-yl]-3-éthyl-urée |

(suite)

| | | |
|---|---|---|
| **Composé 241** | 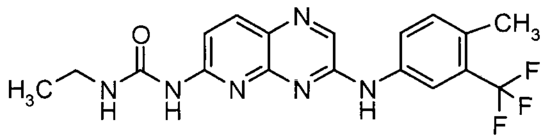 | 1-éthyl-3-[3-(4-méthyl-3-trifluorométhyl-phénylamino)-pyrido[2,3-b]pyrazin-6-yl]-urée |
| **Composé 242** | 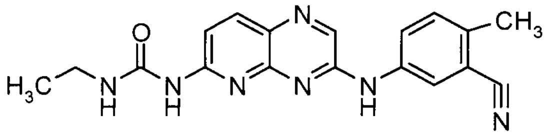 | 1-[3-(3-cyano-4-méthyl-phénylamino)-pyrido[2,3-b]-pyrazin-6-yl]-3-éthyl-urée |
| **Composé 243** | 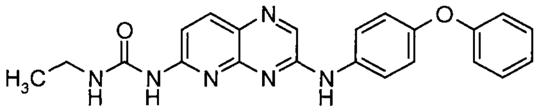 | 1-éthyl-3-[3-(4-phénoxy-phénylamino)-pyrido[2,3-b]-pyrazin-6-yl]-urée |
| **Composé 244** | 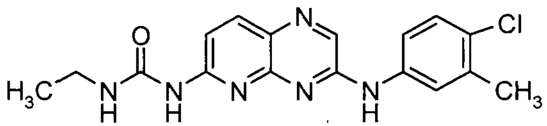 | 1-[3-(4-chloro-3-méthyl-phénylamino)-pyrido[2,3-b]-pyrazin-6-yl]-3-éthyl-urée |
| **Composé 245** | 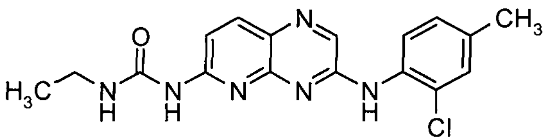 | 1-[3-(2-chloro-4-méthyl-phénylamino)-pyrido[2,3-b]-pyrazin-6-yl]-3-éthyl-urée |
| **Composé 246** | 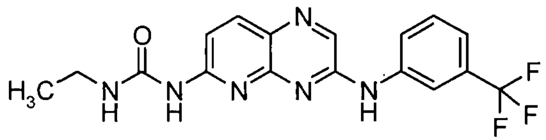 | 1-éthyl-3-[3-(3-trifluorométhyl-phénylamino)-pyrido[2,3-b]-pyrazin-6-yl]-urée |
| **Composé 247** | 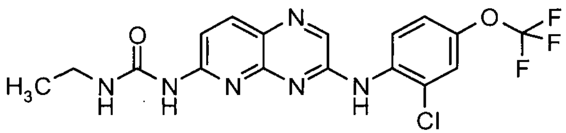 | 1-[3-(2-chloro-4-trifluoro-méthoxy-phénylamino)-pyrido[2,3-b]pyrazin-6-yl]-3-éthyl-urée |

(suite)

| | | |
|---|---|---|
| **Composé 248** | 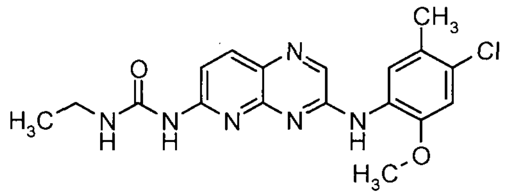 | 1-[3-(4-chloro-2-méthoxy-5-méthyl-phénylamino)-pyrido[2,3-b]pyrazin-6-yl]-3-éthyl-urée |
| **Composé 249** | 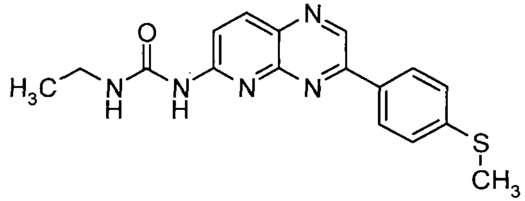 | 1-éthyl-3-[3-(4-méthylsulfanyl-phényl)-pyrido[2,3-b]pyrazin-6-yl]-urée |
| **Composé 250** | 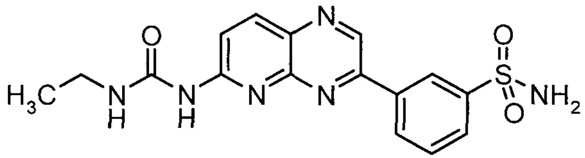 | 3-[6-(3-éthyl-uréido)-pyrido[2,3-b]pyrazin-3-yl]-benzène-sulfonamide |
| **Composé 251** | 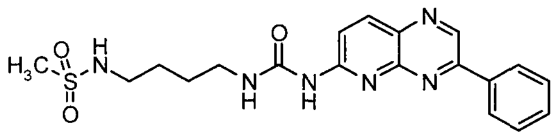 | N-{4-[3-(3-phényl-pyrido[2,3-b]-pyrazin-6-yl)-uréido]-butyl}-méthanesulfonamide |
| **Composé 252** | 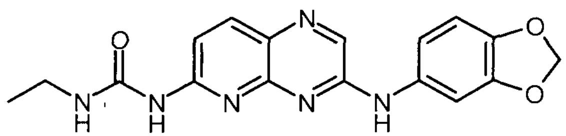 | 1-[3-(benzo[1,3]dioxol-5-ylamino)-pyrido[2,3-b]pyrazin-6-yl]-3-éthyl-urée |
| **Composé 253** | 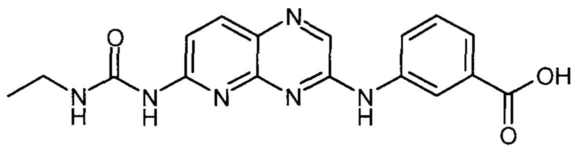 | acide 3-[6-(3-éthyl-uréido)-pyrido[2,3-b]pyrazin-3-ylamino]-benzoïque |

(suite)

| | | |
|---|---|---|
| **Composé 254** | 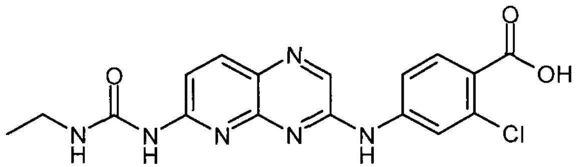 | acide 2-chloro-4-[6-(3-éthyl-uréido)-pyrido[2,3-b]pyrazin-3-ylamino]-benzoïque |
| **Composé 255** | 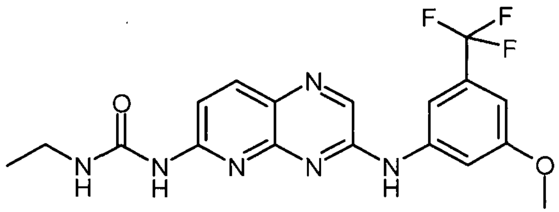 | 1-éthyl-3-[3-(3-méthoxy-5-trifluorométhyl-phénylamino)-pyrido[2,3-b]pyrazin-6-yl]-urée |
| **Composé 256** | 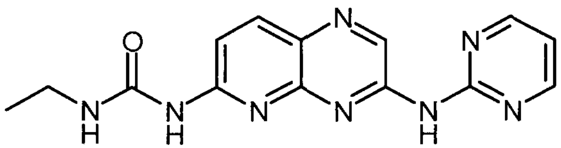 | 1-éthyl-3-[3-(pyrimidin-2-ylamino)-pyrido[2,3-b]pyrazin-6-yl]-urée |
| **Composé 257** | 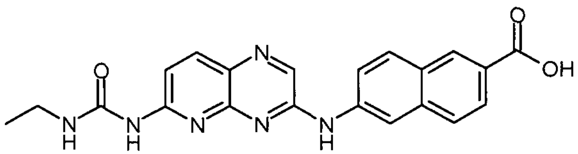 | acide 6-[6-(3-éthyl-uréido)-pyrido[2,3-b]pyrazin-3-ylamino]-naphtalène-2-carboxylique |
| **Composé 258** | 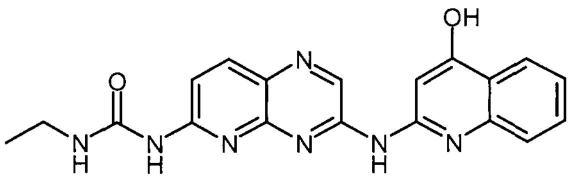 | 1-éthyl-3-[3-(4-hydroxy-quinolin-2-ylamino)-pyrido[2,3-b]pyrazin-6-yl]-urée |
| **Composé 259** | 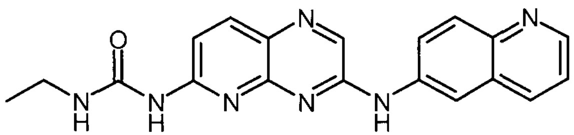 | 1-éthyl-3-[3-(quinolin-6-ylamino)-pyrido[2,3-b]pyrazin-6-yl]-urée |

(suite)

| | | |
|---|---|---|
| **Composé 260** | 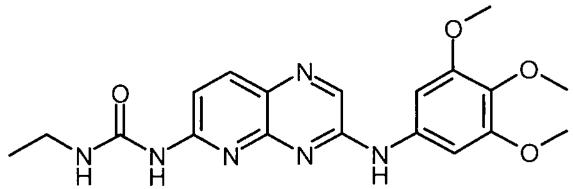 | 1-éthyl-3-[3-(3,4,5-triméthoxy-phénylamino)-pyrido[2,3-b]-pyrazin-6-yl]-urée |
| **Composé 261** | 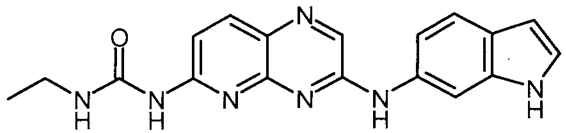 | 1-éthyl-3-[3-(1 H-indol-6-ylamino)-pyrido[2,3-b]pyrazin-6-yl]-urée |
| **Composé 262** | 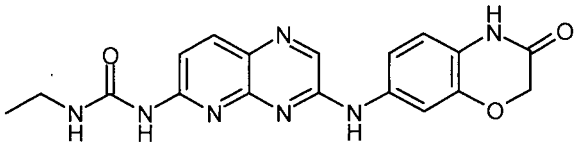 | 1-éthyl-3-[3-(3-oxo-3,4-dihydro-2H-benzo[1,4]oxazin-7-ylamino)-pyrido[2,3-b]pyrazin-6-yl]-urée |
| **Composé 263** | 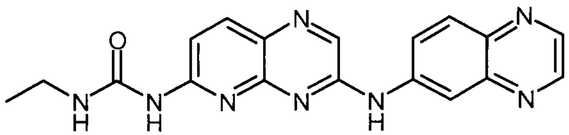 | 1-éthyl-3-[3-(quinoxalin-6-ylamino)-pyrido[2,3-b]pyrazin-6-yl]-urée |
| **Composé 264** | 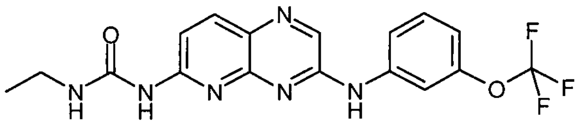 | 1-éthyl-3-[3-(3-trifluorométhoxy-phénylamino)-pyrido[2,3-b]-pyrazin-6-yl]-urée |
| **Composé 265** | 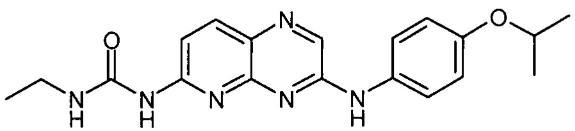 | 1-éthyl-3-[3-(4-isopropoxy-phénylamino)-pyrido[2,3-b]-pyrazin-6-yl]-urée |
| **Composé 266** | 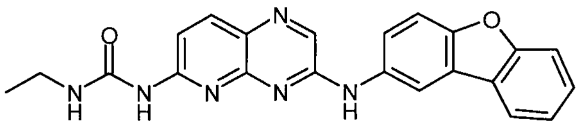 | 1-[3-(dibenzofuran-2-ylamino)-pyrido[2,3-b]pyrazin-6-yl]-3-éthyl-urée |

(suite)

| | | |
|---|---|---|
| **Composé 267** | 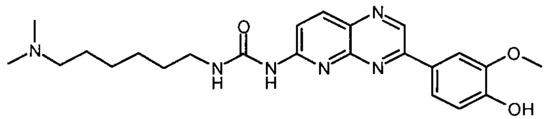 | 1-(6-diméthylamino-hexyl)-3-[3-(4-hydroxy-3-méthoxy-phényl)-pyrido[2,3-b]pyrazin-6-yl]-urée |
| **Composé 268** | 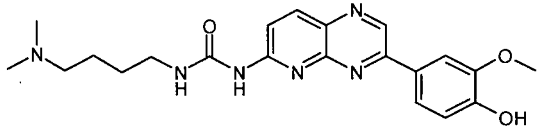 | 1-(4-diméthylamino-butyl)-3-[3-(4-hydroxy-3-méthoxy-phényl)-pyrido[2,3-b]pyrazin-6-yl]-urée |
| **Composé 269** | 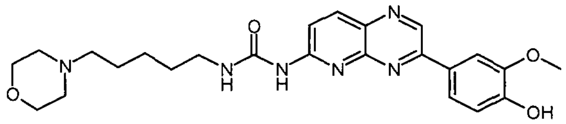 | 1-[3-(4-hydroxy-3-méthoxy-phényl)-pyrido[2,3-b]pyrazin-6-yl]-3-(5-morpholin-4-yl-pentyl)-urée |

**6.** Utilisation selon l'une quelconque des revendications 1 à 5, dans laquelle le médicament comprend au moins une autre substance pharmacologiquement active.

**7.** Utilisation selon l'une quelconque des revendications 1 à 5, dans laquelle on administre le médicament avant et/ou pendant et/ou après le traitement par au moins une autre substance pharmacologiquement active.

**8.** Utilisation selon l'une quelconque des revendications 1 à 5, dans laquelle on administre le médicament avant et/ou pendant et/ou après le traitement par radiothérapie et/ou chirurgie.

**9.** Utilisation selon l'une quelconque des revendications 1 à 5, dans laquelle le composé selon la formule générale (I) se trouve en une dose unitaire de 0,001 mg à 100 mg par kg de poids corporel d'un patient.

**10.** Composition pharmaceutique, contenant un composé choisi dans le groupe des composés n° 38-269.

| | |
|---|---|
| **Comp. 38** | 1-[3-(4-hydroxy-phényl)-pyrido[2,3-b]pyrazin-6-yl]-3-méthyl-urée |
| **Comp. 39** | 1-allyl-3-[3-(4-phénoxy-phényl)-pyrido[2,3-b]pyrazin-6-yl]-urée |
| **Comp. 40** | méthanesulfonate de 4-[6-(3-allyl-uréido)-pyrido[2,3-b]pyrazin-3-yl]-phényle |
| **Comp. 41** | 4-diméthylaminobenzoate de 4-[6-(3-allyl-uréido)-pyrido[2,3-b]pyrazin-3-yl]-phényle |
| **Comp. 42** | acétate de 4-[6-(3-allyl-uréido)-pyrido[2,3-b]pyrazin-3-yl]-phényle |
| **Comp. 43** | 1-éthyl-3-[3-(4-hydroxy-phényl)-pyrido[2,3-b]pyrazin-6-yl]-urée |
| **Comp. 44** | 1-[3-(4-amino-phényl)-pyrido[2,3-b]pyrazin-6-yl]-3-méthyl-thiourée |
| **Comp. 45** | 1-éthyl-3-(3-phényl-pyrido[2,3-b]pyrazin-6-yl)-urée |
| **Comp. 46** | 1-acétyl-1-éthyl-3-(3-phényl-pyrido[2,3-b]pyrazin-6-yl)-urée |
| **Comp. 47** | 1-allyl-3-[3-(4-fluoro-phényl)-pyrido[2,3-b]pyrazin-6-yl]-urée |
| **Comp. 48** | 1,1-diéthyl-3-(3-phényl-pyrido[2,3-b]pyrazin-6-yl)-urée |
| **Comp. 49** | 1-(2-chloro-éthyl)-3-[3-(4-fluoro-phényl)-pyrido[2,3-b]pyrazin-6-yl]-urée |
| **Comp. 50** | 1-éthyl-3-[3-(4-méthoxy-phényl)-pyrido[2,3-b]pyrazin-6-yl]-urée |
| **Comp. 51** | 1-(3-phényl-pyrido[2,3-b]pyrazin-6-yl)-3-propyl-urée |
| **Comp. 52** | [3-(3-phényl-pyrido[2,3-b]pyrazin-6-yl)-uréido]-acétate d'éthyle |
| **Comp. 53** | 1-(3-chloro-pyrido[2,3-b]pyrazin-6-yl)-3-éthyl-urée |
| **Comp. 54** | 1-éthyl-3-[3-(4-fluoro-phényl)-pyrido[2,3-b]pyrazin-6-yl]-urée |
| **Comp. 55** | 1-[3-(3-chloro-4-fluoro-phényl)-pyrido[2,3-b]pyrazin-6-yl]-3-éthyl-urée |
| **Comp. 56** | acide 4-[6-(3-éthyl-uréido)-pyrido[2,3-b]pyrazin-3-yl]-benzoïque |
| **Comp. 57** | N-{4-[6-(3-éthyl-uréido)-pyrido[2,3-b]pyrazin-3-yl]-phényl}-acétamide |
| **Comp. 58** | 1-[3-(2,4-difluoro-phényl)-pyrido[2,3-b]pyrazin-6-yl]-3-éthyl-urée |
| **Comp. 59** | 1-éthyl-3-(3-morpholin-4-yl-pyrido[2,3-b]pyrazin-6-yl)-urée |
| **Comp. 60** | 1-éthyl-3-[3-(4-méthyl-pipérazin-1-yl)-pyrido[2,3-b]pyrazin-6-yl]-urée |
| **Comp. 61** | 1-éthyl-3-[3-(2-hydroxy-phényl)-pyrido[2,3-b] pyrazin-6-yl]-urée |
| **Comp. 62** | 1-éthyl-3-[3-(2-méthoxy-éthylamino)-pyrido[2,3-b]pyrazin-6-yl]-urée |
| **Comp. 63** | 1-[3-(4-chloro-3-trifluorométhyl-phényl)-pyrido[2,3-b]pyrazin-6-yl]-3-éthyl-urée |
| **Comp. 64** | 1-éthyl-3-(3-phénoxy-pyrido[2,3-b]pyrazin-6-yl)-urée |
| **Comp. 65** | 1-[3-(cyclopropylméthyl-amino)-pyrido[2,3-b]pyrazin-6-yl]-3-éthyl-urée |
| **Comp. 66** | 1-éthyl-3-{3-[(pyridin-4-ylméthyl)-amino]- |

(suite)

| | |
|---|---|
| **Comp. 67** | 1-éthyl-3-[3-(4-fluoro-benzyloxy)-pyrido[2,3-b]pyrazin-6-yl]-urée |
| **Comp. 68** | 1-éthyl-3-(3-pyridin-4-yl-pyrido[2,3-b]pyrazin-6-yl)-urée |
| **Comp. 69** | 1-éthyl-3-[3-(pyridin-3-yloxy)-pyrido[2,3-b]pyrazin-6-yl]-urée |
| **Comp. 70** | 1-éthyl-3-[3-(tétrahydro-furan-2-ylméthoxy)-pyrido[2,3-b]pyrazin-6-yl]-urée |
| **Comp. 71** | 1-éthyl-3-[3-(4-morpholin-4-yl-phényl)-pyrido[2,3-b] pyrazin-6-yl]-urée |
| **Comp. 72** | 1-éthyl-3-(3-hydroxy-pyrido[2,3-b]pyrazin-6-yl)-urée |
| **Comp. 73** | 1-éthyl-3-[3-(3-méthoxy-phénylsulfanyl)-pyrido[2,3-b]pyrazin-6-yl]-urée |
| **Comp. 74** | 1-éthyl-3-(3-quinolin-3-yl-pyrido[2,3-b]pyrazin-6-yl)-urée |
| **Comp. 75** | 1-(3-benzo[b]thiophén-3-yl-pyrido[2,3-b] pyrazin-6-yl)-3-éthyl-urée |
| **Comp. 76** | 1-éthyl-3-[3-(pyridin-2-ylsulfanyl)-pyrido[2,3-b]pyrazin-6-yl]-urée |
| **Comp. 77** | 1-[3-(4-diméthylamino-phényl)-pyrido[2,3-b]pyrazin-6-yl]-3-éthyl-urée |
| **Comp. 78** | N-{4-[6-(3-éthyl-uréido)-pyrido[2,3-b]pyrazin-3-yl]-phényl}-méthanesulfonamide |
| **Comp. 79** | 1-éthyl-3-[3-(1H-pyrazol-4-yl)-pyrido[2,3-b]pyrazin-6-yl]-urée |
| **Comp. 80** | 1-(3-benzylsulfanyl-pyrido[2,3-b]pyrazin-6-yl)-3-éthyl-urée |
| **Comp. 81** | 1-éthyl-3-[3-(4-méthyl-[1,4]diazépan-1-yl)-pyrido[2,3-b]pyrazin-6-yl]-urée |
| **Comp. 82** | 1-[3-(4-amino-phényl)-pyrido[2,3-b]pyrazin-6-yl]-3-éthyl-urée |
| **Comp. 83** | 1-(3-amino-pyrido[2,3-b]pyrazin-6-yl)-3-éthyl-urée |
| **Comp. 84** | 1-éthyl-3-pyrido[2,3-b]pyrazin-6-yl-urée |
| **Comp. 85** | 1-éthyl-3-(3-imidazol-1-yl-pyrido[2,3-b]pyrazin-6-yl)-urée |
| **Comp. 86** | 1-éthyl-3-[3-(4-fluoro-2-hydroxy-phényl)-pyrido[2,3-b]pyrazin-6-yl]-urée |
| **Comp. 87** | 1-(3-cyclopentyloxy-pyrido[2,3-b]pyrazin-6-yl)-3-éthyl-urée |
| **Comp. 88** | 1-éthyl-3-[3-(4-hydroxy-pipéridin-1-yl)-pyrido[2,3-b]pyrazin-6-yl]-urée |
| **Comp. 89** | (3-phényl-pyrido[2,3-b]pyrazin-6-yl)-urée |
| **Comp. 90** | 1-éthyl-3-(3-pyrimidin-5-yl-pyrido[2,3-b]pyrazin-6-yl)-urée |
| **Comp. 91** | 1-éthyl-3-(3-pyridin-3-yl-pyrido[2,3-b]pyrazin-6-yl)-urée |
| **Comp. 92** | 1-allyl-3-(3-phényl-pyrido[2,3-b]pyrazin-6-yl)-urée |
| **Comp. 93** | 1-éthyl-3-(3-pipérazin-1-yl-pyrido[2,3-b]pyrazin-6-yl)-urée |
| **Comp. 94** | 1-[3-(3-chloro-pyridin-4-ylméthyl)-pyrido[2,3-b]pyrazin-6-yl]-3-éthyl-urée |
| **Comp. 95** | 1-éthyl-3-[3-(6-méthoxy-pyridin-3-yl)-pyrido[2,3-b]pyrazin-6-yl]-urée |
| **Comp. 96** | 1-[3-(3,5-diméthyl-isoxazol-4-yl)-pyrido[2,3-b]pyrazin-6-yl]-3-éthyl-urée |
| **Comp. 97** | 1-éthyl-3-[3-(4-trifluorométhoxyphényl)-pyrido[2,3-b]pyrazin-6-yl]-urée |
| **Comp. 98** | 1-éthyl-3-(3-furan-2-yl-pyrido[2,3-b]pyrazin-6-yl)-urée |
| **Comp. 99** | 1-éthyl-3-[3-(2-méthoxy-pyridin-3-yl)-pyrido[2,3-b]pyrazin-6-yl]-urée |
| **Comp. 100** | 1-[3-(2,4-diméthoxy-phényl)-pyrido[2,3-b]pyrazin-6-yl]-3-éthyl-urée |
| **Comp. 101** | 1-éthyl-3-[3-(1H-pyrrol-2-yl)-pyrido[2,3-b]pyrazin-6-yl]-urée |
| **Comp. 102** | 1-éthyl-3-[3-(6-morpholin-4-yl-pyridin-3-yl)-pyrido[2,3-b]pyrazin-6-yl]-urée |
| **Comp. 103** | 1-benzyl-3-éthyl-1-(3-phényl-pyrido[2,3-b]pyrazin-6-yl)-urée |
| **Comp. 104** | 1-[3-(2-amino-phényl)-pyrido[2,3-b]pyrazin-6-yl]-3-éthyl-urée |
| **Comp. 105** | 1-éthyl-3-[3-(4-hydroxyméthyl-phényl)-pyrido[2,3-b]pyrazin-6-yl]-urée |

(suite)

| | |
|---|---|
| **Comp. 106** | 1-[3-(3-amino-phényl)-pyrido[2,3-b]pyrazin-6-yl]-3-éthyl-urée |
| **Comp. 107** | 1-[3-(4-acétyl-phényl)-pyrido[2,3-b]pyrazin-6-yl]-3-éthyl-urée |
| **Comp. 108** | 1-[3-(2,3-dihydro-benzofuran-5-yl)-pyrido[2,3-b]pyrazin-6-yl]-3-éthyl-urée |
| **Comp. 109** | 1-[3-(4-benzyloxy-3-fluoro-phényl)-pyrido[2,3-b]pyrazin-6-yl]-3-éthyl-urée |
| **Comp. 110** | 1-(2,3-dihydroxy-propyl)-3-(3-phényl-pyrido[2,3-b]pyrazin-6-yl)-urée |
| **Comp. 111** | 1-éthyl-3-[3-(3-formyl-4-méthoxy-phényl)-pyrido[2,3-b]pyrazin-6-yl]-urée |
| **Comp. 112** | 1-éthyl-3-[3-(4-méthanesulfonylphényl)-pyrido[2,3-b]pyrazin-6-yl]-urée |
| **Comp. 113** | acide N-{4-[6-(3-éthyl-uréido)-pyrido[2,3-b]pyrazin-3-yl]-phényl}-succinamidique |
| **Comp. 114** | 1-éthyl-3-(3-pyridin-4-yl-pyrido[2,3-b]pyrazin-6-yl)-urée sel d'acide méthane-sulfonique (base libre) |
| **Comp. 115** | 1-[3-(2,6-diméthoxy-pyridin-3-yl)-pyrido[2,3-b]-pyrazin-6-yl]-3-éthyl-urée |
| **Comp. 116** | 1-[3-(2,6-diméthoxy-pyrimidin-4-yl)-pyrido[2,3-b]pyrazin-6-yl]-3-éthyl-urée |
| **Comp. 117** | 1-[3-(2,4-dioxo-1,2,3,4-tetrahydro-pyrimidin-5-yl)-pyrido[2,3-b]pyrazin-6-yl]-3-éthyl-urée |
| **Comp. 118** | 1-éthyl-3-[3-(1H-indol-5-yl)-pyrido[2,3-b]pyrazin-6-yl]-urée |
| **Comp. 119** | 1-(3-chloro-pyrido[2,3-b]pyrazin-6-yl)-3-éthyl-thiourée |
| **Comp. 120** | 1-éthyl-3-{3-[4-(2-méthoxy-éthoxy)-phényl]-pyrido[2,3-b]pyrazin-6-yl}-urée |
| **Comp. 121** | acrylate de 4-[6-(3-éthyl-uréido)-pyrido[2,3-b]pyrazin-3-yl]-phényle |
| **Comp. 122** | 1-[3-(4-cyano-phényl)-pyrido[2,3-b]pyrazin-6-yl]-3-éthyl-urée |
| **Comp. 123** | 1-(3-benzo[1,2,5]oxadiazol-4-yl-pyrido[2,3-b]pyrazin-6-yl)-3-éthyl-urée |
| **Comp. 124** | 1-éthyl-3-[3-(4-hydroxy-3-méthoxyphényl)-pyrido[2,3-b]pyrazin-6-yl]-urée |
| **Comp. 125** | 1-[3-(2,6-diméthoxy-phényl)-pyrido[2,3-b]pyrazin-6-yl]-3-éthyl-urée |
| **Comp. 126** | 1-[3-(3-acétyl-phényl)-pyrido[2,3-b]pyrazin-6-yl]-3-éthyl-urée |
| **Comp. 127** | 1-éthyl-3-[3-(3-morpholin-4-yl-phényl)-pyrido[2,3-b]pyrazin-6-yl]-urée |
| **Comp. 128** | 1-[3-(6-amino-pyridin-3-yl)-pyrido[2,3-b]pyrazin-6-yl]-3-éthyl-urée |
| **Comp. 129** | acide 3-{4-[6-(3-éthyl-uréido)-pyrido[2,3-b]pyrazin-3-yl]-phénoxy}-propionique |
| **Comp. 130** | 1-isopropyl-3-(3-phényl-pyrido[2,3-b]pyrazin-6-yl)-urée |
| **Comp. 131** | 1-cyclopentyl-3-(3-phényl-pyrido[2,3-b]pyrazin-6-yl)-urée |
| **Comp. 132** | 1-pentyl-3-(3-phényl-pyrido[2,3-b]pyrazin-6-yl)-urée |
| **Comp. 133** | N-{4-[6-(3-éthyl-uréido)-pyrido[2,3-b]pyrazin-3-yl]-phényl}-acrylamide |
| **Comp. 134** | 1-tert-butyl-3-(3-phényl-pyrido[2,3-b]pyrazin-6-yl)-urée |
| **Comp. 135** | 1-(2-hydroxy-éthyl)-3-(3-phényl-pyrido[2,3-b]pyrazin-6-yl)-urée |
| **Comp. 136** | 1-cyclobutyl-3-(3-phényl-pyrido[2,3-b]pyrazin-6-yl)-urée |
| **Comp. 137** | 1-allyl-3-[3-(4-nitro-phényl)-pyrido[2,3-b]pyrazin-7-yl]-thiourée |
| **Comp. 138** | 1-éthyl-1-(éthylcarbamoyl)-3-(3-phényl-pyrido[2,3-b]pyrazin-6-yl)-urée |
| **Comp. 139** | [3-(4-hydroxy-phényl)-pyrido[2,3-b]pyrazin-6-yl]-carbamate d'allyle |
| **Comp. 140** | (3-phényl-pyrido[2,3-b]pyrazin-6-yl)-carbamate d'éthyle |
| **Comp. 141** | 1-éthyl-3-[3-(4-phényl-pipérazin-1-yl)-pyrido[2,3-b]pyrazin-6-yl]-urée |
| **Comp. 142** | 1-(2,2-diméthyl-[1,3]dioxolan-4-ylméthyl)-3-(3-phényl-pyrido[2,3-b]pyrazin-6-yl)-urée |
| **Comp. 143** | 1,3-bis-(3-phényl-pyrido[2,3-b]pyrazin-6-yl)-urée |
| **Comp. 144** | N-(3-phényl-pvrido[2,3-b]pyrazin-6-yl)-acétamidine |

(suite)

| | |
|---|---|
| **Comp. 145** | 1-éthyl-3-[3-(2-méthoxy-pyridin-3-yl)-pyrido[2,3-b]pyrazin-6-yl]-thiourée |
| **Comp. 146** | 1-(4-hydroxy-butyl)-3-(3-phényl-pyrido[2,3-b]pyrazin-6-yl)-urée |
| **Comp. 147** | 1-(3-hydroxy-propyl)-3-(3-phényl-pyrido[2,3-b]pyrazin-6-yl)-urée |
| **Comp. 148** | 1-éthyl-3-{3-[4-(3-morpholin-4-yl-propoxy)-phényl]-pyrido[2,3-b]pyrazin-6-yl}-urée |
| **Comp. 149** | 1-éthyl-3-[3-(4-hydroxy-phényl)-pyrido[2,3-b]pyrazin-6-yl]-thiourée |
| **Comp. 150** | 1-éthyl-3-(3-p-tolylamino-pyrido[2,3-b]pyrazin-6-yl)-urée |
| **Comp. 151** | 1-éthyl-3-[3-(3-hydroxy-phényl)-pyrido[2,3-b]pyrazin-6-yl]-urée |
| **Comp. 152** | acide 3-[6-(3-éthyl-uréido)-pyrido[2,3-b]pyrazin-3-yl]-benzoïque |
| **Comp. 153** | 1-[3-(3,4-diméthoxy-phényl)-pyrido[2,3-b]pyrazin-6-yl]-3-éthyl-urée |
| **Comp. 154** | 1-[3-(4-amino-3-méthoxy-phényl)-pyrido[2,3-b]pyrazin-6-yl]-3-éthyl-urée |
| **Comp. 155** | 3-[6-(3-éthyl-uréido)-pyrido[2,3-b]pyrazin-3-yl]-benzamide |
| **Comp. 156** | 1-[3-(3-chloro-4-hydroxy-5-méthoxy-phényl)-pyrido[2,3-b]pyrazin-6-yl]-3-éthyl-urée |
| **Comp. 157** | 1-éthyl-3-(3-m-tolylamino-pyrido-[2,3-b]pyrazin-6-yl)-urée |
| **Comp. 158** | 1-éthyl-3-[3-(4-méthoxy-phénylamino)-pyrido[2,3-b]pyrazin-6-yl]-urée |
| **Comp. 159** | 1-[3-(4-chloro-phénylamino)-pyrido-[2,3-b]pyrazin-6-yl]-3-éthyl-urée |
| **Comp. 160** | 1-éthyl-3-(3-o-tolylamino-pyrido[2,3-b]pyrazin-6-yl)-urée |
| **Comp. 161** | 1-éthyl-3-[3-(pyridin-3-ylamino)-pyrido-[2,3-b]pyrazin-6-yl]-urée |
| **Comp. 162** | 1-éthyl-3-[3-(4-éthyl-phénylamino)-pyrido[2,3-b]pyrazin-6-yl]-urée |
| **Comp. 163** | 1-éthyl-3-[3-(3-méthoxy-4-méthyl-phénylamino)-pyrido[2,3-b]pyrazin-6-yl]-urée |
| **Comp. 164** | 1-éthyl-3-[3-(4-hydroxy-phénylamino)-pyrido[2,3-b]pyrazin-6-yl]-urée |
| **Comp. 165** | 1-éthyl-3-[3-(5-méthyl-pyridin-2-yl-amino)-pyridof2,3-b]pyrazin-6-yl]-urée |
| **Comp. 166** | 1-éthyl-3-[3-(1-méthyl-1H-pyrazol-3-ylamino)-pyrido[2,3-b]pyrazin-6-yl]-urée |
| **Comp. 167** | 1-éthyl-3-[3-(4-fluoro-phénylamino)-pyrido[2,3-b]pyrazin-6-yl]-urée |
| **Comp. 168** | 1-(4-hydroxy-butyl)-3-[3-(4-hydroxy-3-méthoxyphényl)-pyrido[2,3-b]pyrazin-6-yl]-urée |
| **Comp. 169** | phosphate de mono-{4-[6-(3-éthyl-uréido)-pyrido-[2,3-b] pyrazin-3-yl]-2-méthoxy-phényle} |
| **Comp. 170** | 1-[3-(2-chloro-4-hydroxy-phényl)-pyrido[2,3-b]pyrazin-6-yl]-3-éthyl-urée |
| **Comp. 171** | 1-[3-(4-chloro-2-méthoxy-phényl)-pyrido[2,3-b]pyrazin-6-yl]-3-éthyl-urée |
| **Comp. 172** | 1-[3-(3,5-diméthyl-1H-pyrazol-4-yl)-pyrido[2,3-b]pyrazin-6-yl]-3-éthyl-urée |
| **Comp. 173** | 1-éthyl-3-[3-(1-méthyl-1H-pyrazol-4-yl)-pyrido[2,3-b]pyrazin-6-yl]-urée |
| **Comp. 174** | 1-[3-(5-cyano-thiophén-2-yl)-pyrido[2,3-b]pyrazin-6-yl]-3-éthyl-urée |
| **Comp. 175** | 2-chloro-4-[6-(3-éthyl-uréido)-pyrido[2,3-b]pyrazin-3-yl]-6-méthoxy-phénolate de sodium |
| **Comp. 176** | 1-[3-(4-hydroxy-3-méthoxy-phényl)-pyrido[2,3-b]pyrazin-6-yl]-3-(4-morpholin-4-yl-butyl)-urée |
| **Comp. 177** | 1-[3-(3-chloro-4-méthoxy-phénylamino)-pyrido[2,3-b]pyrazin-6-yl]-3-éthyl-urée |
| **Comp. 178** | 1-éthyl-3-[3-(naphtalén-2-ylamino)-pyrido[2,3-b]pyrazin-6-yl]-urée |
| **Comp. 179** | 1-éthyl-3-[3-(quinolin-3-ylamino)-pyrido[2,3-b]pyrazin-6-yl]-urée |
| **Comp. 180** | 1-[3-(3,5-diméthoxy-phénylamino)-pyrido[2,3-b]pyrazin-6-yl]-3-éthyl-urée |
| **Comp. 181** | 1-éthyl-3-[3-(pyrazin-2-ylamino)-pyrido[2,3-b]pyrazin-6-yl]-urée |
| **Comp. 182** | 1-éthyl-3-[3-(3-isopropoxy-phénylamino)-pyrido[2,3-b]pyrazin-6-yl]-urée |
| **Comp. 183** | 1-éthyl-3-[3-(4-hydroxy-3-méthoxy-phényl)-pyrido[2,3-b]pyrazin-6-yl]-urée p-toluènesulfonate |

(suite)

| | |
|---|---|
| **Comp. 184** | 1-[3-(2-chloro-pyridin-4-ylamino)-pyrido[2,3-b]pyrazin-6-yl]-3-éthyl-urée |
| **Comp. 185** | 1-[3-(3,5-dichloro-4-hydroxy-phénylamino)-pyrido[2,3-b]pyrazin-6-yl]-3-éthyl-urée |
| **Comp. 186** | 1-[3-(3,5-dichloro-4-hydroxy-phényl)-pyrido[2,3-b]pyrazin-6-yl]-3-éthyl-urée |
| **Comp. 187** | 1-[3-(3,4-diméthoxy-phénylamino)-pyrido[2,3-b]pyrazin-6-yl]-3-éthyl-urée |
| **Comp. 188** | 1-éthyl-3-[3-(3-hydroxy-4-méthyl-phénylamino)-pyrido[2,3-b]pyrazin-6-yl]-urée |
| **Comp. 189** | acide 3-[6-(3-éthyl-uréido)-pyrido[2,3-b]pyrazin-3-ylamino]-5-trifluoro-méthylbenzoïque |
| **Comp. 190** | 1-éthyl-3-[3-(6-méthoxy-pyridin-3-ylamino)-pyrido[2,3-b]pyrazin-6-yl]-urée |
| **Comp. 191** | 1-[3-(3,5-diméthyl-phénylamino)-pyrido[2,3-b]pyrazin-6-yl]-3-éthyl-urée |
| **Comp. 192** | 1-[3-(4-cyano-phénylamino)-pyrido-[2,3-b] pyrazin-6-yl]-3-éthyl-urée |
| **Comp. 193** | 1-éthyl-3-[3-(4-hydroxy-3-méthoxy-phényl)-pyrido[2,3-b]pyrazin-6-yl]-urée sel d'acide (Z)-but-2-ène-dicarboxylique |
| **Comp. 194** | 1-éthyl-3-[3-(4-hydroxy-3-méthoxy-phényl)-pyrido[2,3-b]pyrazin-6-yl]-urée chlorhydrate |
| **Comp. 195** | 1-éthyl-3-(3-phényl-pyrido[2,3-b]pyrazin-6-yl)-thiourée |
| **Comp. 196** | 1-(3-chloro-pyrido[2,3-b]pyrazin-6-yl)-3-cyclohexyl-urée |
| **Comp. 197** | 1-éthyl-3-[3-(4-hydroxy-3-méthoxy-phénylamino)-pyrido[2,3-b]pyrazin-6-yl]-urée |
| **Comp. 198** | 1-éthyl-3-[3-(4-hydroxy-3,5-diméthyl-phényl)-pyrido[2,3-b]pyrazin-6-yl]-urée |
| **Comp. 199** | 3-éthyl-1-phénéthyl-1-(3-phényl-pyrido[2,3-b]pyrazin-6-yl)-urée |
| **Comp. 200** | 1-allyl-3-{3-[4-(tétrahydro-pyran-2-yloxy)-phényl]-pyrido[2,3-b]pyrazin-6-yl}-thiourée |
| **Comp. 201** | 3-éthyl-1-[3-(4-méthoxy-phényl)-pyrido[2,3-b]pyrazin-6-yl]-1-propyl-urée |
| **Comp. 202** | 3-éthyl-1-(3-phényl-pyrido[2,3-b]pyrazin-6-yl)-1-(2-piperidin-1-yl-éthyl)-urée chlorhydrate |
| **Comp 203** | N-{4-[6-(3-éthyl-uréido)-pyrido[2,3-b]pyrazin-3-yl]-phényl}-2-phényl-acétamide |
| **Comp. 204** | 1-(4-hydroxy-butyl)-3-(3-phényl-pyrido[2,3-b]pyrazin-6-yl)-urée chlorhydrate |
| **Comp. 205** | acétate de 4-[3-(3-phényl-pyrido[2,3-b]pyrazin-6-yl)-uréido]-butyle |
| **Comp. 206** | 1-(4-amino-butyl)-3-(3-phényl-pyrido[2,3-b]pyrazin-6-yl)-urée |
| **Comp. 207** | 1-(5-hydroxy-pentyl)-3-(3-phényl-pyrido[2,3-b]pyrazin-6-yl)-urée |
| **Comp. 208** | 3-[6-(3-éthyl-uréido)-pyrido[2,3-b]pyrazin-3-yl]-N-méthyl-benzamide |
| **Comp. 209** | 1-éthyl-3-[3-(2-méthoxy-5-méthyl-phényl)-pyrido[2,3-b]pyrazin-6-yl]-urée |
| **Comp. 210** | 1-éthyl-3-(3-p-tolyl-pyrido[2,3-b]pyrazin-6-yl)-urée |
| **Comp. 211** | 1-éthyl-3-[3-(méthyl-p-tolyl-amino)-pyrido[2,3-b]pyrazin-6-yl]-urée |
| **Comp. 212** | 1-éthyl-3-[3-(2-p-tolyl-éthylamino)-pyrido[2,3-b]pyrazin-6-yl]-urée |
| **Comp. 213** | 1-éthyl-3-[3-(4-méthyl-benzylamino)-pyrido[2,3-b]pyrazin-6-yl]-urée |
| **Comp. 214** | 1-éthyl-3-[3-(3-fluoro-4-méthyl-phényl-amino)-pyrido[2,3-b]pyrazin-6-yl]-urée |
| **Comp. 215** | 1-[3-(3,4-diméthyl-phénylamino)-pyrido[2,3-b]pyrazin-6-yl]-3-éthyl-urée |
| **Comp. 216** | 1-éthyl-3-[3-(4-isopropyl-phénylamino)-pyrido[2,3-b]pyrazin-6-yl]-urée |
| **Comp. 217** | 1-(4-morpholin-4-yl-butyl)-3-(3-phényl-pyrido[2,3-b]pyrazin-6-yl)-urée |
| **Comp. 218** | N-{4-[3-(3-phényl-pyrido[2,3-b] pyrazin-6-yl)-uréido]-butyl}-acétamide |
| **Comp. 219** | 1-[3-(3-amino-4-méthyl-phényl)-pyrido[2,3-b]pyrazin-6-yl]-3-éthyl-urée |
| **Comp. 220** | 1-[3-(3-acétyl-2-fluoro-phényl)-pyrido[2,3-b]pyrazin-6-yl]-3-éthyl-urée |
| **Comp. 221** | 1-éthyl-3-[3-(4-méthoxy-3-méthyl-phényl)-pyrido[2,3-b]pyrazin-6-yl]-urée |

(suite)

| | |
|---|---|
| **Comp. 222** | 1-[3-(6-éthoxy-pyridin-3-yl)-pyrido[2,3-b]pyrazin-6-yl]-3-éthyl-urée |
| **Comp. 223** | 1-éthyl-3-[3-(2-fluoro-4-méthyl-phényl)-pyrido[2,3-b]pyrazin-6-yl]-urée |
| **Comp. 224** | 1-éthyl-3-[3-(3-fluoro-4-méthoxy-phényl)-pyrido[2,3-b]pyrazin-6-yl]-urée |
| **Comp. 225** | 1-éthyl-3-[3-(2-fluoro-3-méthoxy-phényl)-pyrido[2,3-b]pyrazin-6-yl]-urée |
| **Comp. 226** | 1-éthyl-3-[3-(3,4,5-triméthoxy-phényl)-pyrido[2,3-b]pyrazin-6-yl]-urée |
| **Comp. 227** | 1-[3-(3,5-difluoro-2-méthoxy-phényl)-pyrido[2,3-b]pyrazin-6-yl]-3-éthyl-urée |
| **Comp. 228** | 1-éthyl-3-[3-(4-trifluorométhyl-phényl)-pyrido[2,3-b]pyrazin-6-yl]-urée |
| **Comp. 229** | 1-éthyl-3-[3-(2,3,4-triméthoxy-phényl)-pyrido[2,3-b]pyrazin-6-yl]-urée |
| **Comp. 230** | 1-[3-(3-chloro-4-méthoxy-phényl)-pyrido[2,3-b]pyrazin-6-yl]-3-éthyl-urée |
| **Comp. 231** | 1-éthyl-3-[3-(3-fluoro-4-hydroxy-phényl)-pyrido[2,3-b]pyrazin-6-yl]-urée |
| **Comp. 232** | 1-éthyl-3-[3-(6-fluoro-pyridin-3-yl)-pyrido[2,3-b]pyrazin-6-yl]-urée |
| **Comp. 233** | 1-[3-(2,4-diméthyl-thiazol-5-yl)-pyrido-[2,3-b] pyrazin-6-yl]-3-éthyl-urée |
| **Comp. 234** | 1-éthyl-3-[3-(2-méthoxy-phényl)-pyrido[2,3-b]pyrazin-6-yl]-urée |
| **Comp. 235** | 1-[3-(2-chloro-pyridin-4-yl)-pyrido[2,3-b]pyrazin-6-yl]-3-éthyl-urée |
| **Comp. 236** | 1-[3-(5-acétyl-thiophén-2-yl)-pyrido[2,3-b]pyrazin-6-yl]-3-éthyl-urée |
| **Comp. 237** | 1-[3-(5-chloro-thiophén-2-yl)-pyrido[2,3-b]pyrazin-6-yl]-3-éthyl-urée |
| **Comp. 238** | 1-éthyl-3-[3-(3-méthoxy-phényl)-pyrido[2,3-b]pyrazin-6-yl]-urée |
| **Comp. 239** | 1-[3-(3-bromo-5-méthoxy-phényl)-pyrido[2,3-b]pyrazin-6-yl]-3-éthyl-urée |
| **Comp. 240** | 1-[3-(benzothiazol-6-ylamino)-pyrido[2,3-b]pyrazin-6-yl]-3-éthyl-urée |
| **Comp. 241** | 1-éthyl-3-[3-(4-méthyl-3-trifluorométhyl-phénylamino)-pyrido[2,3-b]pyrazin-6-yl]-urée |
| **Comp. 242** | 1-[3-(3-cyano-4-méthyl-phénylamino)-pyrido[2,3-b]pyrazin-6-yl]-3-éthyl-urée |
| **Comp. 243** | 1-éthyl-3-[3-(4-phénoxy-phénylamino)-pyrido[2,3-b]pyrazin-6-yl]-urée |
| **Comp. 244** | 1-[3-(4-chloro-3-méthyl-phénylamino)-pyrido[2,3-b]pyrazin-6-yl]-3-éthyl-urée |
| **Comp. 245** | 1-[3-(2-chloro-4-méthyl-phénylamino)-pyrido[2,3-b]pyrazin-6-yl]-3-éthyl-urée |
| **Comp. 246** | 1-éthyl-3-[3-(3-trifluorométhyl-phénylamino)-pyrido[2,3-b]pyrazin-6-yl]-urée |
| **Comp. 247** | 1-[3-(2-chloro-4-trifluorométhoxy-phénylamino)-pyrido[2,3-b]pyrazin-6-yl]-3-éthyl-urée |
| **Comp. 248** | 1-[3-(4-chloro-2-méthoxy-5-méthyl-phénylamino)-pyrido[2,3-b]pyrazin-6-yl]-3-éthyl-urée |
| **Comp. 249** | 1-éthyl-3-[3-(4-méthylsulfanylphényl)-pyrido[2,3-b]pyrazin-6-yl]-urée |
| **Comp. 250** | 3-[6-(3-éthyl-uréido)-pyrido[2,3-b]pyrazin-3-yl]-benzènesulfonamide |
| **Comp. 251** | N-{4-[3-(3-phényl-pyrido[2,3-b]pyrazin-6-yl)-uréido]-butyl}-méthane-sulfonamide |
| **Comp. 252** | 1-[3-(benzo[1,3]dioxol-5-ylamino)-pyrido[2,3-b]pyrazin-6-yl]-3-éthyl-urée |
| **Comp. 253** | acide 3-[6-(3-éthyl-uréido)-pyrido[2,3-b]pyrazin-3-ylamino]-benzoïque |
| **Comp. 254** | acide 2-chloro-4-[6-(3-éthyl-uréido)-pyrido[2,3-b]pyrazin-3-ylamino]-benzoïque |
| **Comp. 255** | 1-éthyl-3-[3-(3-méthoxy-5-trifluorométhyl-phénylamino)-pyrido[2,3-b]pyrazin-6-yl]-urée |
| **Comp. 256** | 1-éthyl-3-[3-(pyrimidin-2-ylamino)-pyrido[2,3-b]pyrazin-6-yl]-urée |
| **Comp. 257** | acide 6-[6-(3-éthyl-uréido)-pyrido[2,3-b]pyrazin-3-ylamino]-naphtalène-2-carboxylique |
| **Comp. 258** | 1-éthyl-3-[3-(4-hydroxy-quinolin-2-ylamino)-pyrido[2,3-b]pyrazin-6-yl]-urée |
| **Comp. 259** | 1-éthyl-3-[3-(quinolin-6-ylamino)-pyrido[2,3-b]pyrazin-6-yl]-urée |
| **Comp. 260** | 1-éthyl-3-[3-(3,4,5-triméthoxy-phénylamino)-pyrido[2,3-b]pyrazin-6-yl]-urée |

(suite)

| | |
|---|---|
| **Comp. 261** | 1-éthyl-3-[3-(1H-indol-6-ylamino)-pyrido[2,3-b]pyrazin-6-yl]-urée |
| **Comp. 262** | 1-éthyl-3-[3-(3-oxo-3,4-dihydro-2H-benzo[1,4]oxazin-7-ylamino)-pyrido[2,3-b]pyrazin-6-yl]-urée |
| **Comp. 263** | 1-éthyl-3-[3-(quinoxalin-6-ylamino)-pyrido[2,3-b]pyrazin-6-yl]-urée |
| **Comp. 264** | 1-éthyl-3-[3-(3-trifluorométhoxy-phénylamino)-pyrido[2,3-b]pyrazin-6-yl]-urée |
| **Comp. 265** | 1-éthyl-3-[3-(4-isopropoxy-phénylamino)-pyrido[2,3-b]pyrazin-6-yl]-urée |
| **Comp. 266** | 1-[3-(dibenzofuran-2-ylamino)-pyrido[2,3-b]pyrazin-6-yl]-3-éthyl-urée |
| **Comp. 267** | 1-(6-diméthylamino-hexyl)-3-[3-(4-hydroxy-3-méthoxy-phényl)-pyrido[2,3-b]-pyrazin-6-yl]-urée |
| **Comp. 268** | 1-(4-diméthylamino-butyl)-3-[3-(4-hydroxy-3-méthoxy-phényl)-pyrido[2,3-b]-pyrazin-6-yl]-urée |
| **Comp. 269** | 1-[3-(4-hydroxy-3-méthoxy-phényl)-pyrido[2,3-b]pyrazin-6-yl]-3-(5-morpholin-4-yl-pentyl)-urée |

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9932111 A
- WO 03068223 A
- WO 0206213 A
- WO 0035435 A
- WO 0037141 A
- WO 04104002 A
- WO 04104003 A
- WO 9917759 A
- WO 05007099 A
- WO 05056547 A
- WO 04005472 A
- WO 03084473 A
- WO 03086394 A
- WO 03086403 A
- WO 03024448 A
- WO 0181346 A
- WO 04017950 A
- WO 2004104002 A
- WO 2004104003 A
- GB 1184848 A, Degussa
- EP 735025 A, S. Seko


### In der Beschreibung aufgeführte Nicht-Patentliteratur


- **R. D. ELLIOTT.** *J. Org. Chem.,* 1968
- **HOUBEN-WEYL.** *Methoden der Organischen Chemie,* vol. 4/1 a, 343-350
- **HOUBEN-WEYL.** *Methoden der Organischen Chemie,* vol. E 7b, 579
- **D. CATARZI et al.** *J. Med. Chem.,* 1996, 1330-1336
- **J. K. SEYDEL et al.** *J. Med. Chem.,* 1994, 3016-3022
- **HOUBEN-WEYL.** *Methods of Organic Chemistry,* vol. E 9c, 231-235
- **HOUBEN-WEYL.** *Science of Synthesis,* vol. 16, 1269
- **C. L. LEESE ; H. N. RYDON.** *J. Chem. Soc.,* 1955, 303-309
- **T. S. OSDENE ; G. M. TIMMIS.** *J. Chem. Soc.,* 1955, 2033-2035
- **W. HE et al.** *Bioorg. Med. Chem. Lett.,* 2003, vol. 13, 3097-3100
- **M. S. A. EL-GABY et al.** *Indian J. Chem. Sect. B,* 2001, vol. 40, 195-200
- **M. R. MYERS et al.** *Bioorg. Med. Chem. Lett.,* 2003, vol. 13, 3091-3096
- **A. R. RENSLO et al.** *J. Amer. Chem. Soc.,* 1999, vol. 121, 7459-7460
- **C. O. OKAFOR et al.** *J. Heterocyclic Chem.,* 1983, vol. 20, 199-203
- **C. R. HOPKINS et al.** *Tet. Lett.,* 2004, vol. 45, 8631-8633
- **J. YIN et al.** *Org. Lett.,* 2002, vol. 4, 3481-3484
- **O. A. EL-SAYED et al.** *Arch. Pharm.,* 2002, vol. 335, 403-410
- **C. TEMPLE et al.** *J. Med. Chem.,* 1992, vol. 35, 988-993
- **A. M. THOMPSON et al.** *J. Med. Chem.,* 2000, vol. 43, 4200-4211
- **N. A. DALES et al.** *Org. Lett.,* 2001, 2313-2316
- **G. DANNHARDT et al.** *Arch. Pharm.,* 2000, 267-274
- **G. S. POINDEXTER et al.** *Bioorg. Med. Chem.,* 2004, vol. 12, 507-521
- **J.-M. RECEVEUR et al.** *Bioorg. Med. Chem. Lett.,* 2004, vol. 14, 5075-5080
- **G. HEINISCH et al.** *Arch. Pharm.,* 1997, 207-210
- **K. MATSUNO et al.** *J. Med. Chem.,* 2002, vol. 45, 4513-4523
- **A. M. PAPINI et al.** *J. Med. Chem.,* 2004, vol. 47, 5224-5229
- **L. MAO et al.** *Synthesis,* 2004, vol. 15, 2535-2539
- **M. DARABANTU et al.** *Tetrahedron,* 2005, vol. 61, 2897-2905
- **E. FORD et al.** *Tet. Lett.,* 2000, vol. 41, 3197-3198
- **T. SHIOTA et al.** *J. Org. Chem.,* 1999, vol. 64, 453-457
- **J. F. MIRAVET et al.** *Org. Lett.,* 2005, vol. 7, 4791-4794
- **A. L. CASTELHANO et al.** *Bioorg. Med. Chem. Lett.,* 2005, vol. 15, 1501-1504
- **J. W. HUFFMANN et al.** *Bioorg. Med. Chem.,* 2006, vol. 14, 247-262
- **T. LIU et al.** *Org. & Biomolecular Chem.,* 2005, vol. 3, 1525-1533

- **ALESSI DR ; ANDJELKOVIC M ; CAUDWELL B ; CRON P ; MORRICE N ; COHEN P ; HEMMINGS BA.** Mechanism of activation of protein kinase B by insulin and IGF-1. *EMBO J.,* 02. Dezember 1996, vol. 15 (23), 6541-51
- **ALI K ; BILANCIO A ; THOMAS M ; PEARCE W ; GILFILLAN AM ; TKACZYK C ; KUEHN N ; GRAY A ; GIDDINGS J ; PESKETT E.** Essential role for the p110delta phosphoinositide 3-kinase in the allergic response. *Nature,* 21. Oktober 2004, vol. 431 (7011), 1007-11
- **BENNETT BL ; SASAKI DT ; MURRAY BW ; O'LEARY EC ; SAKATA ST ; XU W ; LEISTEN JC ; MOTIWALA A ; PIERCE S ; SATOH Y.** SP600125, an anthrapyrazolone inhibitor of Jun N-terminal kinase. *Proc Natl Acad Sci USA.,* 20. November 2001, vol. 98 (24), 13681-6
- **BONDEV A ; RUBIO I ; WETZKER R.** Differential regulation of lipid and protein kinase activities of phosphoinositide 3-kinase gamma in vitro. *Biol Chem.,* November 1999, vol. 380 (11), 1337-40
- **BONDEVA T ; PIROLA L ; BULGARELLI-LEVA G ; RUBIO I ; WETZKER R ; WYMANN MP.** Bifurcation of lipid and protein kinase signals of PI3Kgamma to the protein kinases PKB and MAPK. *Science,* 09. Oktober 1998, vol. 282 (5387), 293-6
- **CAMPBELL IG ; RUSSELL SE ; CHOONG DY ; MONTGOMERY KG ; CIAVARELLA ML ; HOOI CS ; CRISTIANO BE ; PEARSON RB ; PHILLIPS WA.** Mutation of the PIK3CA gene in ovarian and breast cancer. *Cancer Res.,* 01. November 2004, vol. 64 (21), 7678-81
- **CHANG F ; LEE JT ; NAVOLANIC PM ; STEELMAN LS ; SHELTON JG ; BLALOCK WL ; FRANKLIN RA ; MCCUBREY JA.** Involvement of PI3K/Akt pathway in cell cycle progression, apoptosis, and neoplastic transformation: a target for cancer chemotherapy. *Leukemia,* Marz 2003, vol. 17 (3), 590-603
- **CHANG F ; STEELMAN LS ; LEE JT ; SHELTON JG ; NAVOLANIC PM ; BLALOCK WL ; FRANKLIN RA ; MCCUBREY JA.** Signal transduction mediated by the Ras/Raf/MEK/ERK pathway from cytokine receptors to transcription factors: potential targeting for therapeutic intervention. *Leukemia,* Juli 2003, vol. 17 (7), 1263-93
- **CHANG HW ; AOKI M ; FRUMAN D ; AUGER KR ; BELLACOSA A ; TSICHLIS PN ; CANTLEY LC ; ROBERTS TM ; VOGT PK.** Transformation of chicken cells by the gene encoding the catalytic subunit of PI 3-kinase. *Science,* 20. Juni 1997, vol. 276 (5320), 1848-50
- **CHEN J ; FUJII K ; ZHANG L ; ROBERTS T ; FU H.** Raf-1 promotes cell survival by antagonizing apoptosis signal-regulating kinase 1 through a MEK-ERK independent mechanism. *Proc Natl Acad Sci USA.,* 03. Juli 2001, vol. 98 (14), 7783-8
- **CHIANG GG ; ABRAHAM RT.** Determination of the catalytic activities of mTOR and other members of the phosphoinositide-3-kinase-related kinase family. *Methods Mol Biol.,* 2004, vol. 281, 125-41
- **CRACKOWER MA ; OUDIT GY ; KOZIERADZKI I ; SARAO R ; SUN H ; SASAKI T ; HIRSCH E ; SUZUKI A ; SHIOI T ; IRIE-SASAKI J.** Regulation of myocardial contractility and cell size by distinct PI3K-PTEN signaling pathways. *Cell,* 20. September 2002, vol. 110 (6), 737-49
- **DAVIES H ; BIGNELL GR ; COX C ; STEPHENS P ; EDKINS S ; CLEGG S ; TEAGUE J ; WOFFENDIN H ; GARNETT MJ ; BOTTOMLEY W.** Mutations of the BRAF gene in human cancer. *Nature,* 27. Juni 2002, vol. 417 (6892), 949-54
- **DAVIES SP ; REDDY H ; CAIVANO M ; COHEN P.** Specificity and mechanism of action of some commonly used protein kinase inhibitors. *Biochem J.,* 01. Oktober 2000, vol. 351, 95-105
- **DHAND R ; HILES I ; PANAYOTOU G ; ROCHE S ; FRY MJ ; GOUT I ; TOTTY NF ; TRUONG O ; VICENDO P ; YONEZAWA K et al.** PI 3-kinase is a dual specificity enzyme: autoregulation by an intrinsic protein-serine kinase activity. *EMBO J.,* 01. Februar 1994, vol. 13 (3), 522-33
- **ELLIOTT RD ; TEMPLE C JR ; MONTGOMERY JA.** Potential folic acid antagonists. 3. Deaza analogs of methotrexate. 3. 1- and 3-Deaza analogs of 2,4-diamino-6-[(n-methylanilino)methyl]pteridine. *J Org Chem.,* Februar 1968, vol. 33 (2), 533-6
- **FRIESS H ; BERBERAT P ; SCHILLING M ; KUNZ J ; KORC M ; BUCHLER MW.** Pancreatic cancer: the potential clinical relevance of alterations in growth factors and their receptors. *J Mol Med.,* Januar 1996, vol. 74 (1), 35-42
- **GOTZ R ; WIESE S ; TAKAYAMA S ; CAMARERO GC ; ROSSOLL W ; SCHWEIZER U ; TROPPMAIR J ; JABLONKA S ; HOLTMANN B ; REED JC.** Bag1 is essential for differentiation and survival of hematopoietic and neuronal cells. *Nat Neurosci.,* September 2005, vol. 8 (9), 1169-78
- **GUM RJ ; MCLAUGHLIN MM ; KUMAR S ; WANG Z ; BOWER MJ ; LEE JC ; ADAMS JL ; LIVI GP ; GOLDSMITH EJ ; YOUNG PR.** Acquisition of sensitivity of stress-activated protein kinases to the p38 inhibitor, SB 203580, by alteration of one or more amino acids within the ATP binding pocket. *J Biol Chem.,* 19. Juni 1998, vol. 273 (25), 15605-10
- **HOSHINO R ; CHATANI Y ; YAMORI T ; TSURUO T ; OKA H ; YOSHIDA O ; SHIMADA Y ; ARI-I S ; WADA H ; FUJIMOTO J.** Constitutive activation of the 41-/43-kDa mitogen-activated protein kinase signaling pathway in human tumors. *Oncogene,* 21. Januar 1999, vol. 18 (3), 813-22

- **KATSO R ; OKKENHAUG K ; AHMADI K ; WHITE S ; TIMMS J ; WATERFIELD MD.** Cellular function of phosphoinositide 3-kinases: implications for development, homeostasis, and cancer. *Annu Rev Cell Dev Biol.,* 2001, vol. 17, 615-75
- **KHLEIF SN ; ABRAMS SI ; HAMILTON JM ; BERGMANN-LEITNER E ; CHEN A ; BASTIAN A ; BERNSTEIN S ; CHUNG Y ; ALLEGRA CJ ; SCHLOM J.** A phase I vaccine trial with peptides reflecting ras oncogene mutations of solid tumors. *J Immunother.,* Marz 1999, vol. 22 (2), 155-65
- **LEVINE DA ; BOGOMOLNIY F ; YEE CJ ; LASH A ; BARAKAT RR ; BORGEN PI ; BOYD J.** Frequent mutation of the PIK3CA gene in ovarian and breast cancers. *Clin Cancer Res.,* 15. April 2005, vol. 11 (8), 2875-8
- **LEWIS TS ; SHAPIRO PS ; AHN NG.** Signal transduction through MAP kinase cascades. *Adv Cancer Res.,* 1998, vol. 74, 49-139
- **LI J ; YEN C ; LIAW D ; PODSYPANINA K ; BOSE S ; WANG SI ; PUC J ; MILIARESIS C ; RODGERS L ; MCCOMBIE R.** PTEN, a putative protein tyrosine phosphatase gene mutated in human brain, breast, and prostate cancer. *Science,* 28. Marz 1997, vol. 275 (5308), 1943-7
- **LU Y ; WANG H ; MILLS GB.** Targeting PI3K-AKT pathway for cancer therapy. *Rev Clin Exp Hematol.,* Juni 2003, vol. 7 (2), 205-28
- **MA YY ; WEI SJ ; LIN YC ; LUNG JC ; CHANG TC ; WHANG-PENG J ; LIU JM ; YANG DM ; YANG WK ; SHEN CY.** PIK3CA as an oncogene in cervical cancer. *Oncogene,* 25. Mai 2000, vol. 19 (23), 2739-44
- **MARSHALL C.** How do small GTPase signal transduction pathways regulate cell cycle entry?. *Curr Opin Cell Biol.,* Dezember 1999, vol. 11 (6), 732-6
- **MCPHILLIPS F ; MULLEN P ; MONIA BP ; RITCHIE AA ; DORR FA ; SMYTH JF ; LANGDON SP.** Association of c-Raf expression with survival and its targeting with antisense oligonucleotides in ovarian cancer. *Br J Cancer,* 30. November 2001, vol. 85 (11), 1753-8
- **MENDELSOHN J ; BASELGA J.** The EGF receptor family as targets for cancer therapy. *Oncogene,* 27. Dezember 2000, vol. 19 (56), 6550-65
- **MOORE SM ; RINTOUL RC ; WALKER TR ; CHILVERS ER ; HASLETT C ; SETHI T.** The presence of a constitutively active phosphoinositide 3-kinase in small cell lung cancer cells mediates anchorage-independent proliferation via a protein kinase B and p70s6k-dependent pathway. *Cancer Res.,* 15. November 1998, vol. 58 (22), 5239-47
- **OKKENHAUG K ; VANHAESEBROECK B.** PI3K in lymphocyte development, differentiation and activation. *Nat Rev Immunol.,* April 2003, vol. 3 (4), 317-30
- **PATRUCCO E ; NOTTE A ; BARBERIS L ; SELVETELLA G ; MAFFEI A ; BRANCACCIO M ; MARENGO S ; RUSSO G ; AZZOLINO O ; RYBALKIN SD.** PI3Kgamma modulates the cardiac response to chronic pressure overload by distinct kinase-dependent and -independent effects. *Cell,* 06. August 2004, vol. 118 (3), 375-87
- **RAPP UR ; RENNEFAHRT U ; TROPPMAIR J.** Bcl-2 proteins: master switches at the inter-section of death signaling and the survival control by Raf kinases. *Biochim Biophys Acta,* 01. Marz 2004, vol. 1644 (2-3), 149-58
- **RODRIGUEZ-VICIANA P ; WARNE PH ; DHAND R ; VANHAESEBROECK B ; GOUT I ; FRY MJ ; WATERFIELD MD ; DOWNWARD J.** Phosphatidylinositol-3-OH kinase as a direct target of Ras. *Nature,* 18. August 1994, vol. 370 (6490), 527-32
- **SAMUELS Y ; WANG Z ; BARDELLI A ; SILLIMAN N ; PTAK J ; SZABO S ; YAN H ; GAZDAR A ; POWELL SM ; RIGGINS GJ.** High frequency of mutations of the PIK3CA gene in human cancers. *Science,* 23. April 2004, vol. 304 (5670), 554
- **SEBOLT-LEOPOLD JS ; HERRERA R.** Targeting the mitogen-activated protein kinase cascade to treat cancer. *Nat Rev Cancer,* Dezember 2004, vol. 4 (12), 937-47
- **SHAYESTEH L ; LU Y ; KUO WL ; BALDOCCHI R ; GODFREY T ; COLLINS C ; PINKEL D ; POWELL B ; MILLS GB ; GRAY JW.** PIK3CA is implicated as an oncogene in ovarian cancer. *Nat Genet,* Januar 1999, vol. 21 (1), 99-102
- **SIRIVATANAUKSORN V ; SIRIVATANAUKSORN Y ; LEMOINE NR.** Molecular pattern of ductal pancreatic cancer. *Langenbecks Arch Surg,* April 1998, vol. 383 (2), 105-15
- **STECK PA ; PERSHOUSE MA ; JASSER SA ; YUNG WK ; LIN H ; LIGON AH ; LANGFORD LA ; BAUMGARD ML ; HATTIER T ; DAVIS T.** Identification of a candidate tumour suppressor gene, MMAC1, at chromosome 10q23.3 that is mutated in multiple advanced cancers. *Nat Genet.,* April 1997, vol. 15 (4), 356-62
- **SUJOBERT P ; BARDET V ; CORNILLET-LEFEBVRE P ; HAYFLICK JS ; PRIE N ; VERDIER F ; VANHAESEBROECK B ; MULLER O ; PESCE F ; IFRAH N.** Essential role for the p110delta isoform in phosphoinositide 3-kinase activation and cell proliferation in acute myeloid leukemia. *Blood,* 01. August 2005, vol. 106 (3), 1063-6
- **TEMPLE C JR ; RENER GA.** Potential antimitotic agents. Synthesis of some ethyl benzopyrazin-7-ylcarbamates, ethyl pyrido[3,4-b]pyrazin-7-ylcarbamates, and ethyl pyrido[3,4-e]-as-triazin-7-ylcarbamates. *J Med Chem.,* November 1990, vol. 33 (11), 3044-50

- **TROPPMAIR J ; RAPP UR.** Raf and the road to cell survival: a tale of bad spells, ring bearers and detours. *Biochem Pharmacol.,* 15. Oktober 2003, vol. 66 (8), 1341-5
- **VANHAESEBROECK B ; HIGASHI K ; RAVEN C ; WELHAM M ; ANDERSON S ; BRENNAN P ; WARD SG ; WATERFIELD MD.** Autophosphorylation of p110delta phosphoinositide 3-kinase: a new paradigm for the regulation of lipid kinases in vitro and in vivo. *EMBO J.,* 01. Marz 1999, vol. 18 (5), 1292-302
- **VANHAESEBROECK B ; LEEVERS SJ ; AHMADI K ; TIMMS J ; KATSO R ; DRISCOLL PC ; WOSCHOLSKI R ; PARKER PJ ; WATERFIELD MD.** Synthesis and function of 3-phosphorylated inositol lipids. *Annu Rev Biochem.,* 2001, vol. 70, 535-602
- **WEINSTEIN-OPPENHEIMER CR ; BLALOCK WL ; STEELMAN LS ; CHANG F ; MCCUBREY JA.** The Raf signal transduction cascade as a target for chemotherapeutic intervention in growth factor-responsive tumors. *Pharmacol Ther.,* Dezember 2000, vol. 88 (3), 229-79
- **WETZKER R ; ROMMEL C.** Phosphoinositide 3-kinases as targets for therapeutic intervention. *Curr Pharm Des.,* 2004, vol. 10 (16), 1915-22
- **WYMANN MP ; PIROLA L.** Structure and function of phosphoinositide 3-kinases. *Biochim Biophys Acta.,* 08. Dezember 1998, vol. 1436 (1-2), 127-50